(19) Europäisches Patentamt
European Patent Office
Office européen des brevets

(11) EP 4 257 587 B1

(12) **EUROPEAN PATENT SPECIFICATION**

(45) Date of publication and mention of the grant of the patent:
**03.06.2026 Bulletin 2026/23**

(21) Application number: **21900042.9**

(22) Date of filing: **02.12.2021**

(51) International Patent Classification (IPC):
***C07D 417/14*** (2006.01) ***C07D 401/12*** (2006.01)
***C07D 213/82*** (2006.01) ***A61K 31/455*** (2006.01)
***A61P 29/00*** (2006.01) ***A61P 35/00*** (2006.01)
***A61P 37/00*** (2006.01)

(52) Cooperative Patent Classification (CPC):
**A61P 29/00; C07D 213/78; C07D 213/82; C07D 401/12; C07D 401/14; C07D 403/14; C07D 405/12; C07D 405/14; C07D 413/12; C07D 417/12; C07D 417/14; C07F 9/58; C07F 9/65583**

(86) International application number:
**PCT/CN2021/134929**

(87) International publication number:
**WO 2022/117016 (09.06.2022 Gazette 2022/23)**

(54) **HYDROXAMATE COMPOUND, PREPARATION METHOD THEREFOR AND APPLICATION THEREOF**

HYDROXAMATVERBINDUNG, HERSTELLUNGSVERFAHREN DAFÜR UND ANWENDUNG DAVON

COMPOSÉ HYDROXAMATE, SON PROCÉDÉ DE PRÉPARATION ET SON APPLICATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.12.2020 CN 202011389148**
**05.07.2021 CN 202110758372**

(43) Date of publication of application:
**11.10.2023 Bulletin 2023/41**

(73) Proprietor: **Shenzhen Chipscreen Biosciences Co., Ltd.**
**Guangdong 518057 (CN)**

(72) Inventors:
- **WANG, Wei**
  **Shenzhen, Guangdong 518057 (CN)**
- **ZHOU, You**
  **Shenzhen, Guangdong 518057 (CN)**
- **SHAN, Song**
  **Shenzhen, Guangdong 518057 (CN)**
- **PAN, Desi**
  **Shenzhen, Guangdong 518057 (CN)**
- **LI, Zhibin**
  **Shenzhen, Guangdong 518057 (CN)**
- **LU, Xianping**
  **Shenzhen, Guangdong 518057 (CN)**

(74) Representative: **Merli, Silvia**
**Marchi & Partners S.r.l.**
**Via Vittor Pisani, 13**
**20124 Milano (IT)**

(56) References cited:

| | |
|---|---|
| EP-A1- 1 518 855 | EP-A1- 2 004 632 |
| WO-A1-2013/091011 | WO-A1-2015/168079 |
| WO-A1-2020/086616 | WO-A1-2020/086616 |
| WO-A2-2008/080965 | CN-A- 1 665 789 |
| CN-A- 101 405 283 | CN-A- 102 227 409 |
| CN-A- 103 998 442 | CN-A- 113 563 309 |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 257 587 B1

## Description

### Technical field

The present invention relates to the field of pharmaceutical chemistry, and particularly relates to hydroxamate compounds, preparation method therefor, and application thereof.

### Background art

Janus Kinase (JAK) family is a group of non-receptor tyrosine kinase. Four members of the family have been found, including JAK1, JAK2, JAK3 and TYK2. Signal transducer and activator of transcription (STAT) is the direct substrate of JAK, and seven members have been found, including STAT1, STAT2, STAT3, STAT4, STAT5a, STAT5b and STAT6. Many cytokines and growth factors transmit signals through the JAK-STAT signal pathway. These cytokines and growth factors have corresponding receptors on the cell membrane, and these receptors do not have kinase activity by themselves but instead have JAK binding sites in the intracellular segment. The binding of the receptor to the ligand causes dimerization of the receptor molecules, making the JAK coupled to the receptor approach to each other and activated through the interaction of tyrosine residues phosphorylation. Activated JAK catalyzes the phosphorylation of the tyrosine residues of the receptor itself to form the corresponding STAT docking sites, so that STAT is allowed to couple with the receptor and activated by phosphorylation under the action JAK. After entering the nucleus in the form of dimer, STAT couples with the corresponding target gene promoter to activate the corresponding gene transcription and expression process. Although one kind of JAK can participate in the signal transduction processes of multiple cytokines, and one kind of cytokine signal pathway can also activate multiple JAKs, cytokines have certain selectivity for activated STAT molecules.

JAK-STAT signal pathway is closely related to autoimmune diseases and inflammatory diseases. Therefore, the JAK family has become a hot target for new drug research and development. Small molecular drugs that inhibit JAK1, JAK2 and JAK3 have been approved for the treatment of multiple related diseases, such as pan-JAK inhibitor tofacitinib and specifictinib, and JAK1/JAK2 inhibitor baricitinib, which have been approved for the treatment of rheumatoid arthritis, and JAK1/JAK2 inhibitor ruxolitinib, which has been approved for the treatment of bone marrow fibrosis. However, these drugs have been box-warned by the FDA of the United States because of the potential risk of infection and thrombosis, especially in the circumstances of high-dose and/or long-term uses. Such first generation of JAK inhibitors fail to achieve high selective inhibition of different subtypes of JAK kinase, which might be the reason for those substantial side effects. Therefore, the development of the next generation of JAK inhibitors focuses on high selectivity. WO2020/086616 discloses compounds that are useful in treating a TYK2-mediated disorder.

As a member of the JAK family, Tyrosine Kinase 2 (TYK2) is widely distributed in a variety of tissues and cells, and capable of coupling with receptors such as IFNAR1, IL10R2, EL12R- β1, and gp130, etc. It plays roles in coupled forms such as TYK2/JAK1, TYK2/JAK2, TYK2/JAK1/JAK2, to mediate signal pathways related to factors such as type I interferon, interleukin 10 (IL-10), IL-12, IL-23, etc. However, they do not participate in any cytokine response mediated by any other kinase. Although no TYK2 inhibitor has been approved for marketing yet, it is considered that TYK2 inhibitor might be a promising target with less side effects when providing the same efficacy, due to its unique molecular mechanism of action in diseases. By inhibiting the TYK2 signal pathway, TYK2 inhibitors might block the signal pathways induced by factors including type I interferon, IL-10, IL-12, IL-23, etc., and provide a positive effect on diseases including but not limited to those diseases closely related to these factors.

### Summary of the invention

The invention is set out in the appended set of claims 1-15.

Problems to be solved by the invention:

Although several patent applications relating to TYK2 selective inhibitors have been published, due to the excellent prospects of TYK2 specific inhibitors in the treatment of inflammatory diseases, autoimmune diseases and cancers, there still is a need for new compounds. After continuous efforts, the inventors of this application have designed compounds represented by formula (I), formula (I') or formula (I"), which are found to exhibit excellent effects, better druggability, stronger drug efficacy and higher TYK2 kinase selectivity.

Solutions to solve the problems:

Aiming to solve the above problems, the inventors of this application have carried out intensive researches and

found that some particular hydroxamate compounds can achieve the desired purpose, thereby completing the invention.

The present invention relates to the following hydroxamate compounds.

Embodiments that the present invention seek to protect:

A compound represented by formula (I), or a pharmaceutically acceptable salt thereof:

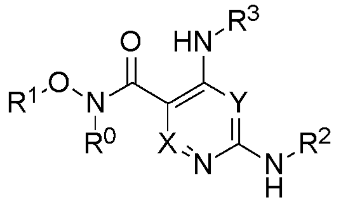

(I)

wherein:

X is $CR^a$;
Y is CH;
$R^a$ is hydrogen;
$R^0$ is hydrogen;
$R^1$ is selected from the group consisting of $C_{1-6}$ alkyl optionally substituted by from one to seven $R^{1a}$ groups;
$R^{1a}$ is selected from the group consisting of deuterium, F and Cl;
$R^2$ is selected from the group consisting of $C_{3-6}$ cycloalkyl-C(O)-, $C_{3-6}$ heterocyclyl-C(O)- wherein the $C_{3-6}$ heterocyclyl contains from one to two heteroatoms selected from N, O and S, phenyl, and 5-6 membered heteroaryl containing from one to two heteroatoms selected from N, O and S, wherein the $C_{3-6}$ cycloalkyl, $C_{3-6}$ heterocyclyl containing from one to two heteroatoms selected from N, O and S, phenyl, and 5-6 membered heteroaryl containing from one to two heteroatoms selected from N, O and S are respectively and optionally substituted by from one to two $R^{2a}$ groups;
most preferably, $R^2$ is selected from the group consisting of $C_{3-6}$ cycloalkyl-C(O)-, $C_{3-6}$ heterocyclyl-C(O)- wherein the $C_{3-6}$ heterocyclyl contains from one to two heteroatoms selected from N, O and S, phenyl, and 5-6 membered heteroaryl containing from one to two heteroatoms selected from N and S, wherein the 5-6 membered heteroaryl containing from one to two heteroatoms selected from N and S is selected from the group consisting of pyridinyl, pyrimidinyl, pyrazinyl, pyridazinyl, thiazolyl and pyrazolyl, wherein the $C_{3-6}$ cycloalkyl, $C_{3-6}$ heterocyclyl containing from one to two heteroatoms selected from N, O and S, phenyl, and 5-6 membered heteroaryl containing from one to two heteroatoms selected from N and S are respectively and optionally substituted by from one to two $R^{2a}$ groups;
preferably, the $C_{3-6}$ heterocyclyl containing from one to two heteroatoms selected from N, O and S is azetidinyl;
preferably, the heterocyclyl in the $C_{3-8}$ heterocyclyl-C(O)- or $C_{3-6}$ heterocyclyl-C(O)- is connected to -C(O)- through the heteroatom in the heterocyclic ring; preferably, the heteroatom is N atom;
$R^{2a}$ is selected from the group consisting of deuterium, =O (oxo), F, Cl, Br, I, CN, $OCF_3$, $-NO_2$, $-(CH_2)_r-OR^b$, $-(CH_2)_r-SR^b$, $-(CH_2)_r-C(O)R^b$, $-(CH_2)_r-C(O)OR^b$, $-(CH_2)_r-NR^bR^c$, $-(CH_2)_r-C(O)NR^bR^c$, $-(CH_2)_r-NR^bC(O)R^c$, $-(CH_2)_r-NR^bC(O)OR^c$, $-NR^bC(O)NR^cR^c$, $-NR^bS(O)_pR^c$, $-S(O)_pR^c$, $-P(O)R^bR^c$, $C_{1-6}$ alkyl optionally substituted by from one to three $R^d$ groups, $C_{2-6}$ alkenyl optionally substituted by from one to three $R^d$ groups, $C_{2-6}$ alkynyl optionally substituted by from one to three $R^d$ groups, $-(CH_2)_r$-3-10 membered carbocyclyl optionally substituted by from one to three $R^d$ groups, and $-(CH_2)_r$-5-10 membered heterocyclyl optionally substituted by from one to three $R^d$ groups; wherein the heterocyclyl contains from one to four heteroatoms selected from O, N and $S(O)_p$;
preferably $R^{2a}$ is selected from the group consisting of $C_{1-6}$ alkyl, halogen, $C_{1-6}$ alkoxy, $C_{3-6}$ cycloalkyl, cyano, halo $C_{1-6}$ alkyl, $C_{1-6}$ alkyl substituted by hydroxyl group, and $C_{1-6}$ alkyl-$S(O)_2$-;
$R^3$ is selected from the group consisting of phenyl, pyridinyl, 2-pyridinonyl and indazolyl, wherein the phenyl, pyridinyl, 2-pyridinonyl and indazolyl are optionally substituted by from one to three $R^{3a}$ groups;
$R^{3a}$ is selected from the group consisting of F, Cl, Br, I, CN, $-NO_2$, $-OR^b$, $-SR^b$, $-C(O)OR^b$, $-C(O)NR^bR^c$, $-NR^bC(O)R^c$, $-NR^bC(O)OR^c$, $-NR^bC(O)NR^cR^c$, $-NR^bS(O)_2R^c$, $-S(O)_2R^c$, $-S(O)_2NR^cR^c$, $-P(O)R^bR^c$, $C_{1-6}$ alkyl optionally substituted by from one to three $R^d$ groups, $C_{2-6}$ alkenyl, $C_{2-6}$ alkynyl, $C_{3-6}$ cycloalkyl, 5-9 membered heteroaryl containing from one to three heteroatoms selected from N, O and S and optionally substituted by from one to three $R^d$ groups, and 5-6 membered saturated heterocyclyl containing from one to two heteroatoms selected from N, O and S;
$R^b$ is selected from the group consisting of hydrogen, $C_{1-6}$ alkyl optionally substituted by from one to three $R^d$ groups, and 4-6 membered saturated heterocyclyl containing from one to two oxygen atoms;
$R^c$ is selected from the group consisting of hydrogen, $C_{1-6}$ alkyl and $C_{3-6}$ cycloalkyl;
$R^d$ is selected from the group consisting of hydrogen, deuterium, F, Cl, Br, I,$-OCF_3$, $-CF_3$, $-(CH_2)_r-CN$, $-NO_2$, $-OR^e$,

$-(CH_2)_r-COR^c$, $-NR^eR^e$, $-NR^eC(O)OR^c$, $C_1$-$C_6$ alkyl, $C_{3-6}$ cycloalkyl, and $-(CH_2)_r$-phenyl;
$R^e$ is hydrogen;
p is 0, 1 or 2, and
r is 0, 1, 2, 3 or 4.

According to a preferred embodiment, when:

X is $CR^a$;
$R^a$ is hydrogen;
$R^0$ is hydrogen;
$R^1$ is selected from the group consisting of methyl, ethyl, isopropyl, t-butyl, trideuterated methyl and 2,2,2-trifluoroethyl;
$R^2$ is selected from the group consisting of cyclopropylcarbonyl,

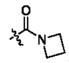,

phenyl, pyrazolyl, thiazolyl, pyridinyl, pyrazinyl, pyrimidinyl and pyridazinyl, wherein the cyclopropylcarbonyl,

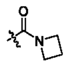,

phenyl, pyrazolyl, thiazolyl, pyridinyl, pyrazinyl, pyrimidinyl and pyridazinyl are optionally substituted by from one to two $R^{2a}$ groups;
$R^{2a}$ is selected from the group consisting of methyl, F, methoxy, cyclopropyl, cyano, $-CF_3$, hydroxymethyl and methanesulfonyl;
$R^3$ is selected from the group consisting of phenyl, pyridinyl, 2-pyridinonyl and indazolyl, wherein the phenyl, pyridinyl, 2-pyridinonyl and indazolyl are optionally substituted by from one to three $R^{3a}$ groups;
$R^{3a}$ is selected from the group consisting of F, Cl, Br, I, CN, $-OR^b$, $-C(O)NR^bR^c$,$-NR^bS(O)_2R^c$, $-S(O)_2R^c$, $-S(O)_2NR^cR^c$, $-P(O)R^bR^c$, $C_{1-6}$ alkyl optionally substituted by from one to three $R^d$ groups, $C_{2-6}$ alkynyl, $C_{3-6}$ cycloalkyl, 5-9 membered heteroaryl containing from one to three heteroatoms selected from N, O and S and optionally substituted by from one to three $R^d$ groups, and 5-6 membered saturated heterocyclyl containing from one to two heteroatoms selected from N, O and S;
$R^b$ is selected from the group consisting of hydrogen; methyl, ethyl, and isopropyl, each optionally substituted by from one to three $R^d$ groups; and

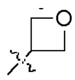;

$R^c$ is selected from the group consisting of hydrogen, methyl and cyclopropyl;
$R^d$ is selected from the group consisting of deuterium, F, Cl, hydroxyl, methyl, cyclopropyl and isopropyl;

In some embodiments, when:

X is CH;
$R^1$ is selected from the group consisting of methyl, ethyl, isopropyl, t-butyl, trideuterated methyl and 2,2,2-trifluoroethyl;
$R^{2a}$ is selected from the group consisting of methyl, F, methoxy, cyclopropyl, cyano, $-CF_3$, hydroxymethyl and methanesulfonyl;
$R^{3a}$ is selected from the group consisting of F, Cl, Br, I, CN, $-OR^b$, $-C(O)NR^bR^c$,$-NR^bS(O)_2R^c$, $-S(O)_2R^c$, $-S(O)_2NR^cR^c$, $-P(O)R^bR^c$, $C_1$-$C_3$ alkyl optionally substituted by from one to three $R^d$ groups, $C_{2-4}$ alkynyl, $C_{3-5}$ cycloalkyl, 5-9 membered heteroaryl containing from one to three heteroatoms selected from N, O and S and optionally substituted by $R^d$ group, and 5-6 membered saturated heterocyclyl containing from one to two heteroatoms selected from N, O and S, wherein the 5-9 membered heteroaryl containing from one to three heteroatoms selected from N, O and S is selected from the group consisting of thiazolyl, benzimidazolyl, pyrazolyl, oxadiazolyl, triazolyl, pyridinyl, pyrimidinyl and pyrazinyl, wherein the 5-6 membered saturated heterocyclyl containing from one to two heteroatoms selected

from N, O and S is selected from the group consisting of morpholinyl and piperidinyl;

$R^b$ is selected from the group consisting of hydrogen; methyl, ethyl, and isopropyl, each optionally substituted by from one to three $R^d$ groups; and

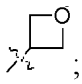;

$R^c$ is selected from the group consisting of hydrogen, methyl and cyclopropyl;

$R^d$ is selected from the group consisting of deuterium, F, Cl, hydroxyl, methyl, cyclopropyl and isopropyl.

In some preferred embodiments, $R^2$ is selected from the group consisting of

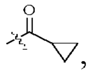,

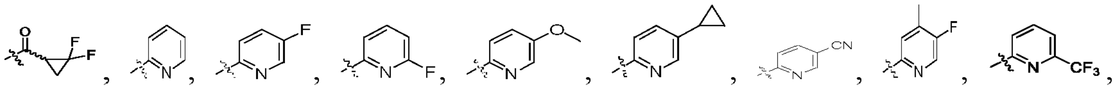,

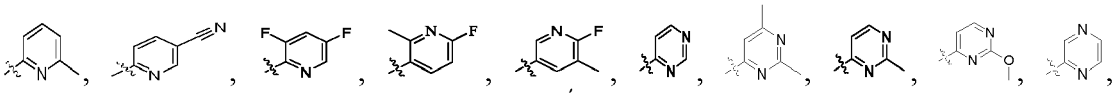,

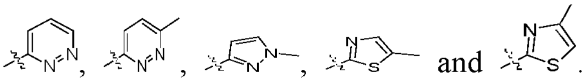.

According to some of the above embodiments, $R^3$ is selected from the group consisting of:

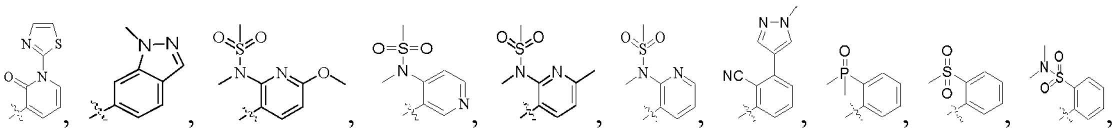,

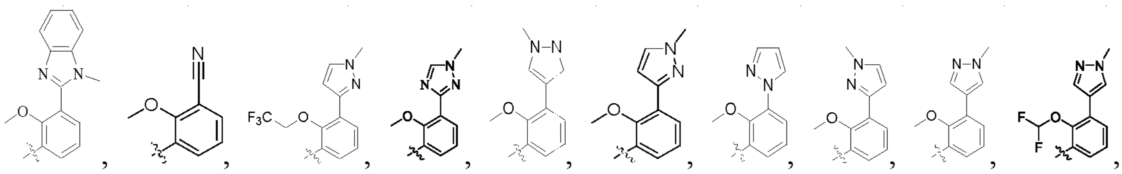,

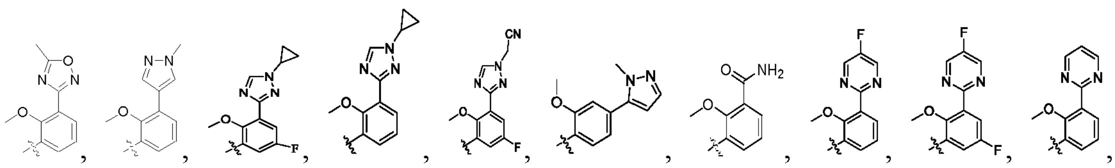,

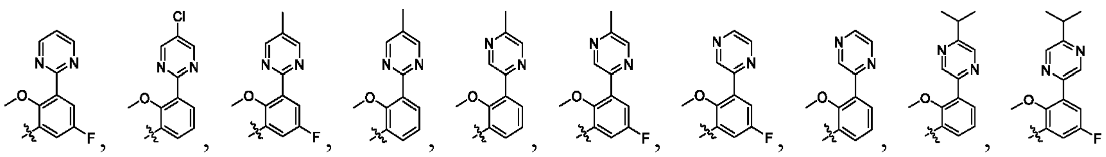,

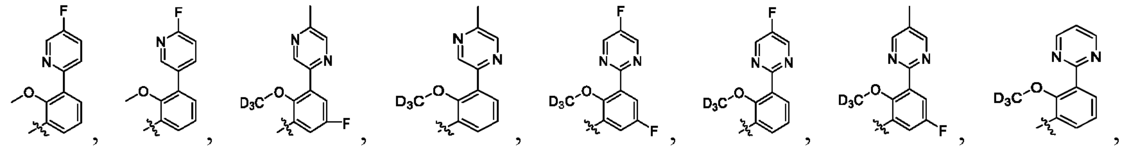
, , , , , , , ,

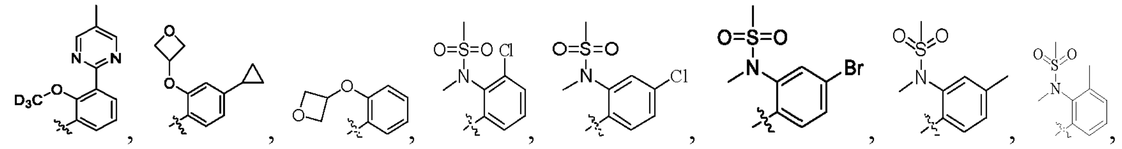
, , , , , , , ,

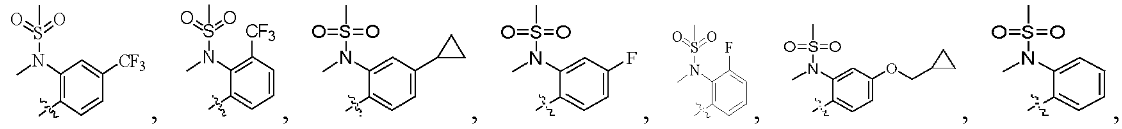
, , , , , , ,

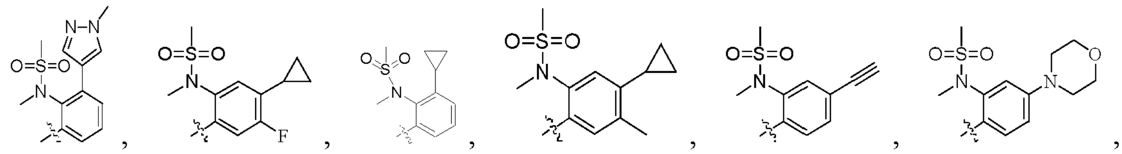
, , , , , ,

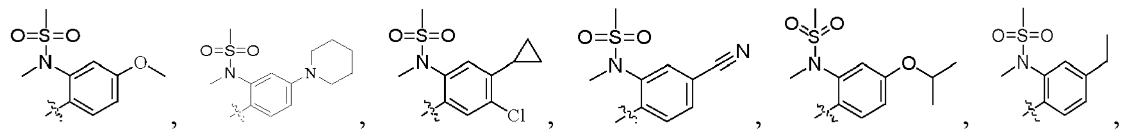
, , , , , ,

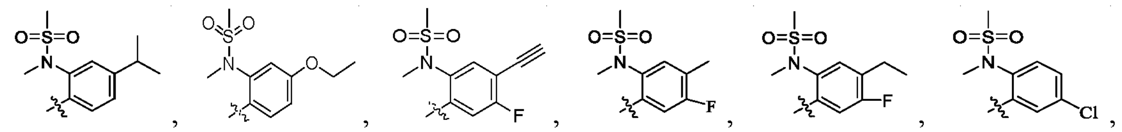
, , , , , ,

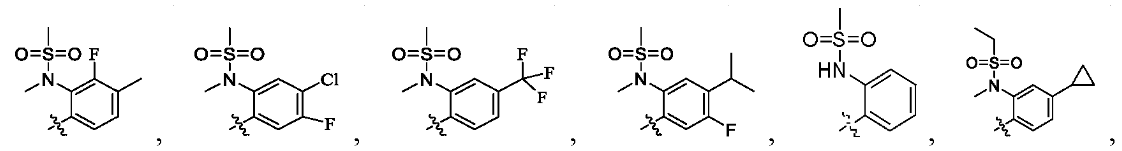
, , , , , ,

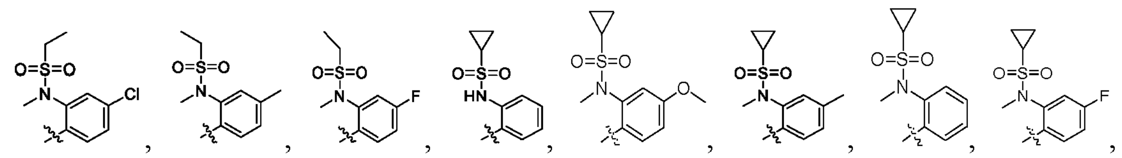
, , , , , , , ,

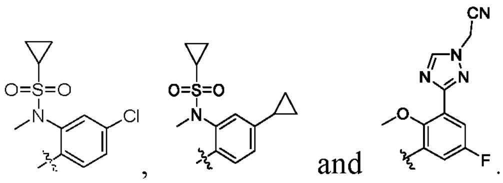
, and .

Additionally, according to the above embodiments the present invention further provides a compound represented by formula (I") or a pharmaceutically acceptable salt thereof:

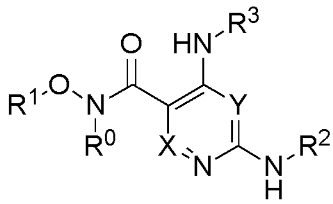

(I'')

wherein:

X is CH;
Y is CH;
$R^0$ is hydrogen;
$R^1$ is ethyl;
$R^2$ is selected from the group consisting of pyridinyl, pyrimidinyl, -C(O)-$C_{3-6}$ cycloalkyl and -C(O)-$C_{3-6}$ heterocyclyl, wherein the $C_{3-6}$ heterocyclyl contains from one to two heteroatoms selected from N, O and S; wherein the $C_{3-6}$ cycloalkyl and $C_{3-6}$ heterocyclyl are respectively and optionally substituted by from one to three $R^{2a}$ groups;$R^{2a}$ is selected from the group consisting of $C_{1-6}$ alkyl, halogen, $C_{1-6}$ alkoxy, $C_{3-6}$ cycloalkyl, cyano, halo $C_{1-6}$ alkyl, $C_{1-6}$ alkyl substituted by hydroxyl group, and $C_{1-6}$ alkyl-$S(O)_2$-;
$R^3$ is phenyl optionally substituted by from one to three $R^{3a}$ groups;
$R^{3a}$ is selected from the group consisting of $-(CH_2)_r-OR^b$, $-NR^bS(O)_pR^c$, F, Cl, $C_{1-6}$ alkyl, $C_{3-6}$ cycloalkyl, triazolyl, pyrazolyl, pyrimidinyl and pyrazinyl; wherein the $C_{1-6}$ alkyl, $C_{3-6}$ cycloalkyl, triazolyl, pyrazolyl, pyrimidinyl and pyrazinyl are, respectively and independently, optionally substituted by from one to three $R^d$ groups.
$R^b$ is selected from the group consisting of hydrogen, $C_{1-6}$ alkyl optionally substituted by from one to three $R^d$ groups, and 4-6 membered saturated heterocyclyl containing from one to two oxygen atoms;
$R^c$ is selected from the group consisting of hydrogen, $C_{1-6}$ alkyl and $C_{3-6}$ cycloalkyl;
$R^d$ is selected from the group consisting of hydrogen, deuterium, F, Cl, Br, I, $OCF_3$, $CF_3$, $-(CH_2)_r-CN$, $NO_2$, $OR^e$, $-(CH_2)_r-COR^c$, $-NR^eR^e$, $-NR^eC(O)OR^c$, $C_1$-$C_6$ alkyl, $C_{3-6}$ cycloalkyl, and $-(CH_2)_r-$;
$R^e$ is hydrogen;
p is 0, 1 or 2, and
r is 0, 1, 2, 3 or 4.

The invention is further described by claims 7-12.

The compounds represented by Formula I or Formula I' or Formula I" in the present invention include the following exemplary compounds:

| Compound # | Structure | nomenclature |
|---|---|---|
| 1* | 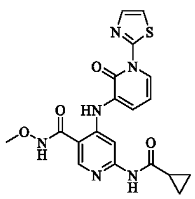 | 6-(cyclopropylcarboxamido)-N-methoxy-4-((2-oxo-1-(thiazol-2-yl)-1,2-dihydropyridin-3-yl)amino)nicotinamide |
| 2 | 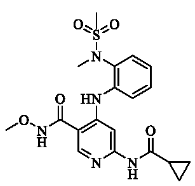 | 6-(cyclopropylcarboxamido)-N-methoxy-4-((2-(N-methyl methanesulfonamido)phenyl)amino)nicoti namide |

(continued)

| Compound # | Structure | nomenclature |
|---|---|---|
| 3* | 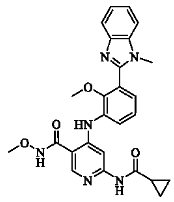 | 6-(cyclopropylcarboxamido)-N-methoxy-4-((2-methoxy-3-(1-methyl-1H-benzo[d]imidazol-2-yl)phenyl)amino)nicotinamide |
| 4 | 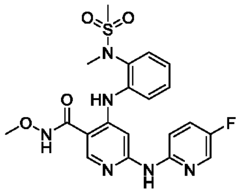 | 6-((5-fluoropyridin-2-yl)amino)-N-methoxy-4-((2-(N-methyl methanesulfonamido)phenyl)amino)nicoti namide |
| 5* | 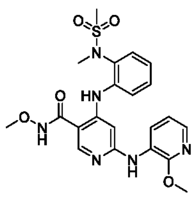 | N-methoxy-6-(((2-methoxy pyridin-3-yl)amino)-4-((2-(N-methyl methanesulfonamido)phenyl)amino)nicoti namide |
| 6* | 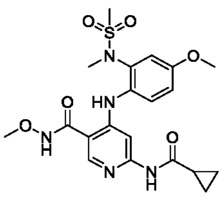 | 6-(cyclopropylcarboxamido)-N-methoxy-4-((4-methoxy-2-(N-methyl methanesulfonamido)phenyl)amino)nicoti namide |
| 7* | 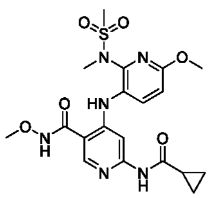 | 6-(cyclopropylcarboxamido)-N-methoxy-4-((6-methoxy-2-(N-methyl methanesulfonamido)pyridin-3-yl)amino)nicotinamide |
| 8* | 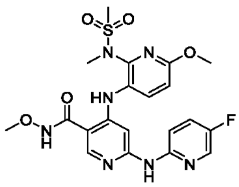 | 6-((5-fluoropyridin-2-yl)amino)-N-methoxy-4-((6-methoxy-2-(N-methyl methanesulfonamido)pyridin-3-yl)amino)nicotinamide |
| 9* | 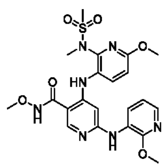 | N-methoxy-4-((6-methoxy-2-(N-methyl methanesulfonamido)pyridin-3-yl)amino)-6-((2-methoxy pyridin-3-yl)amino)nicotinamide |
| 10 | 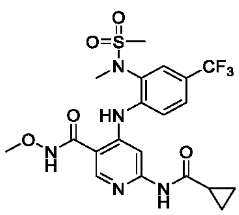 | 6-(cyclopropylcarboxamido)-N-methoxy-4-((2-(N-methyl methanesulfonamido)-4-(trifluoromethyl)phenyl)amino)nicotinamide |

(continued)

| Compound # | Structure | nomenclature |
|---|---|---|
| 11* | 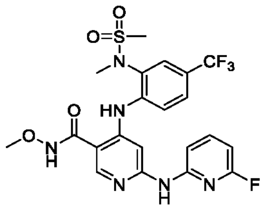 | 6-((6-fluoropyridin-2-yl)amino)-N-methoxy-4-((2-(N-methyl methanesulfonamido)-4-(trifluoromethyl)phenyl)amino)nicotinamide |
| 12 | 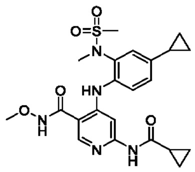 | 6-(cyclopropylcarboxamido)-4-((4-cyclopropyl-2-(N-methyl methanesulfonamido)phenyl)amino)-N-methoxy nicotinamide |
| 13 | 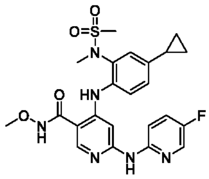 | 4-((4-cyclopropyl-2-(N-methyl methanesulfonamido)phenyl)amino)-6-((5-fluoropyridin-2-yl)amino)-N-methoxy nicotinamide |
| 14 | 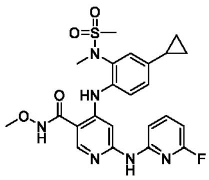 | 4-((4-cyclopropyl-2-(N-methyl methanesulfonamido)phenyl)amino)-6-((6-fluoropyridin-2-yl)amino)-N-methoxy nicotinamide |
| 15* | 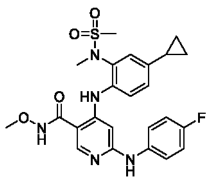 | 4-((4-cyclopropyl-2-(N-methyl methanesulfonamido)phenyl)amino)-6-((4-fluorophenyl)amino)-N-methoxy nicotinamide |
| 16 | 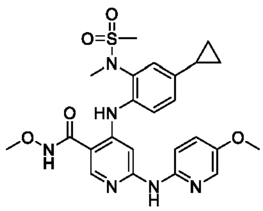 | 4-((4-cyclopropyl-2-(N-methyl methanesulfonamido)phenyl)amino)-N-methoxy-6-((5-methoxy pyridin-2-yl)amino)nicotinamide |
| 17* | 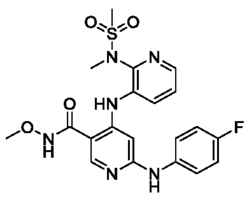 | 6-((4-fluorophenyl)amino)-N-methoxy-4-((2-(N-methyl methanesulfonamido)pyridin-3-yl)amino)nicotinamide |
| 18 | 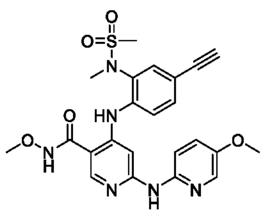 | 4-((4-ethynyl-2-(N-methyl methanesulfonamido)phenyl)amino)-N-methoxy-6-((5-methoxy pyridin-2-yl)amino)nicotinamide |

(continued)

| Compound # | Structure | nomenclature |
|---|---|---|
| 19 | 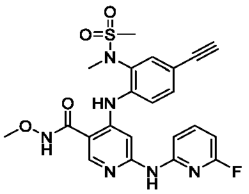 | 4-((4-ethynyl-2-(N-methyl methanesulfonamido)phenyl)amino)-6-(((6-fluoropyridin-2-yl)amino)-N-methoxy nicotinamide |
| 20 | 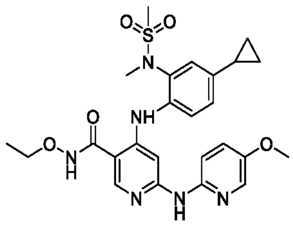 | 4-((4-cyclopropyl-2-(N-methyl methanesulfonamido)phenyl)amino)-N-ethoxy-6-((5-methoxy pyridin-2-yl)amino)nicotinamide |
| 21 | 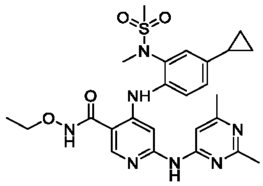 | 4-((4-cyclopropyl-2-(N-methyl methanesulfonamido)phenyl)amino)-6-((2, 6-dimethyl pyrimidin-4-yl)amino)-N-ethoxy nicotinamide |
| 22 | 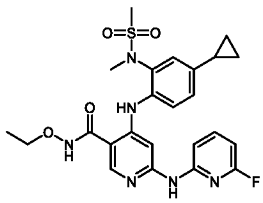 | 4-((4-cyclopropyl-2-(N-methyl methanesulfonamido)phenyl)amino)-N-ethoxy-6-((6-fluoropyridin-2-yl)amino)nicotinamide |
| 23* | 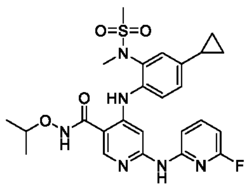 | 4-((4-cyclopropyl-2-(N-methyl methanesulfonamido)phenyl)amino)-6-((6-fluoropyridin-2-yl)amino)-N-isopropoxy nicotinamide |
| 24* | 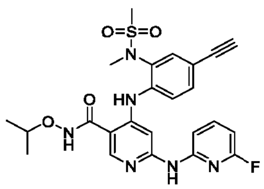 | 4-((4-ethynyl-2-(N-methyl methanesulfonamido)phenyl)amino)-6-(((6-fluoropyridin-2-yl)amino)-N-isopropoxy nicotinamide |
| 25* | 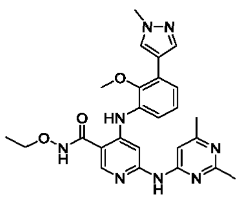 | 6-((2, 6-dimethyl pyrimidin-4-yl)amino)-N-ethoxy-4-((2-methoxy-3-(1-methyl-1H-pyrazol-4-yl)phenyl)amino)nicotinamide |

(continued)

| Compound # | Structure | nomenclature |
|---|---|---|
| 26* | 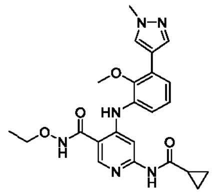 | 6-(cyclopropylcarboxamido)-N-ethoxy-4-((2-methoxy-3-(1-methyl-1H-pyrazol-4-yl)phenyl)amino)nicotinamide |
| 27* | 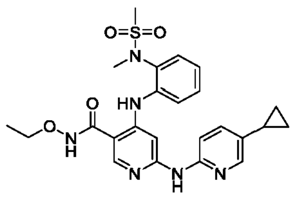 | 6-((5-cyclopropyl pyridin-2-yl)amino)-N-ethoxy-4-((2-(N-methyl methanesulfonamido)phenyl)amino)nicoti namide |
| 28* | 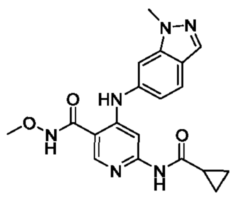 | 6-(cyclopropylcarboxamido)-N-methoxy-4-((1-methyl-1H-indazol-6-yl)amino)nicotinamide |
| 29 | 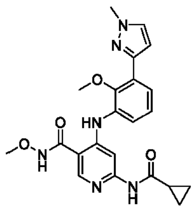 | 6-(cyclopropylcarboxamido)-N-methoxy-4-((2-methoxy-3-(1-methyl-1H-pyrazol-3-yl)phenyl)amino)nicotinamide |
| 30* | 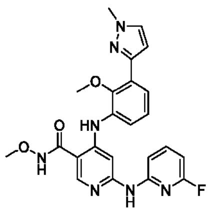 | 6-((6-fluoropyridin-2-yl)amino)-N-methoxy-4-((2-methoxy-3-(1-methyl-1H-pyrazol-3-yl)phenyl)amino)nicotinamide |
| 31* | 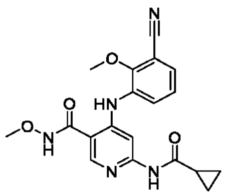 | 4-((3-cyano-2-methoxy phenyl)amino)-6-(cyclopropylcarboxamido)-N-methoxy nicotinamide |
| 32* | 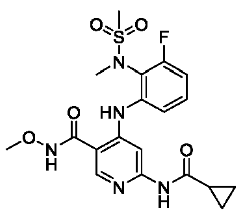 | 6-(cyclopropylcarboxamido)-4-((3-fluoro-2-(N-methyl methanesulfonamido)phenyl)amino)-N-methoxy nicotinamide |

(continued)

| Compound # | Structure | nomenclature |
|---|---|---|
| 33* | 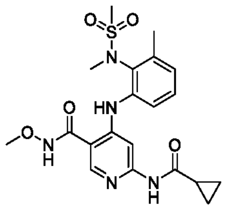 | 6-(cyclopropylcarboxamido)-N-methoxy-4-((3-methyl-2-(N-methyl methanesulfonamido)phenyl)amino)nicoti namide |
| 34* | 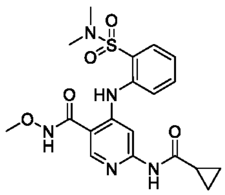 | 6-(cyclopropylcarboxamido)-4-((2-(N, N-dimethyl aminosulfonyl) phenyl)amino)-N-methoxy nicotinamide |
| 35* | 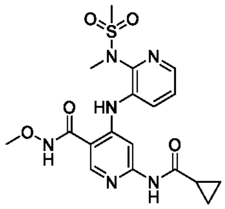 | 6-(cyclopropylcarboxamido)-N-methoxy-4-((2-(N-methyl methanesulfonamido)pyridin-3-yl)amino)nicotinamide |
| 36 | 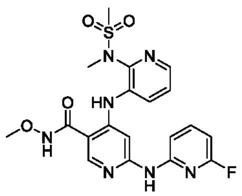 | 6-((6-fluoropyridin-2-yl)amino)-N-methoxy-4-((2-(N-methyl methanesulfonamido)pyridin-3-yl)amino)nicotinamide |
| 37* | 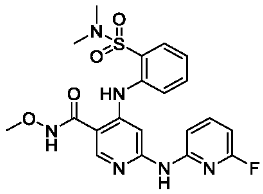 | 4-((2-(N, N-dimethyl aminosulfonyl)phenyl)amino)-6-((6-fluoropyridin-2-yl)amino)-N-methoxy nicotinamide |
| 38 | 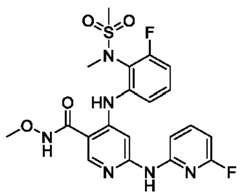 | 4-((3-fluoro-2-(N-methyl methanesulfonamido)phenyl)amino)-6-((6-fluoropyridin-2-yl)amino)-N-methoxy nicotinamide |
| 39* | 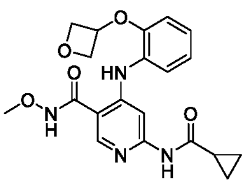 | 6-(cyclopropylcarboxamido)-N-methoxy-4-((2-(oxetan-3-yloxy)phenyl)amino)nicotinamide |
| 40* | 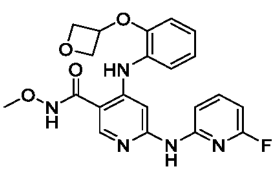 | 6-(((6-fluoropyridin-2-yl)amino)-*N*-methoxy-4-((2-(oxetan-3-yloxy) phenyl)amino)nicotinamide |

(continued)

| Compound # | Structure | nomenclature |
|---|---|---|
| 41 | 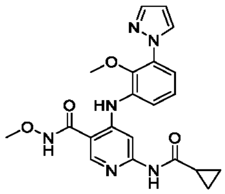 | 6-(cyclopropylcarboxamido)-N-methoxy-4-((2-methoxy-3-(1H-pyrazol-1-yl)phenyl)amino)nicotinamide |
| 42 | 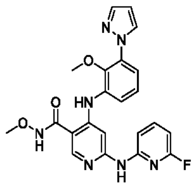 | 6-((6-fluoropyridin-2-yl)amino)-N-methoxy-4-((2-methoxy-3-(1H-pyrazol-1-yl)phenyl)amino)nicotinamide |
| 43 | 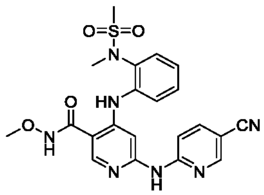 | 6-((5-cyano pyridin-2-yl)amino)-N-methoxy-4-((2-(N-methyl methanesulfonamido)phenyl)amino)nicoti namide |
| 44 | 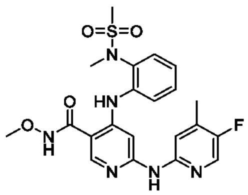 | 6-((5-fluoro-4-methyl pyridin-2-yl)amino)-N-methoxy-4-((2-(N-methyl methanesulfonamido)phenyl)amino)nicoti namide |
| 45 | 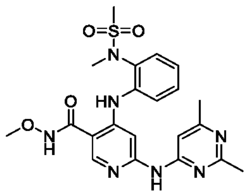 | 6-(((2, 6-dimethyl pyrimidin-4-yl)amino)-N-methoxy-4-((2-(N-methyl methanesulfonamido)phenyl)amino)nicoti namide |
| 46 | 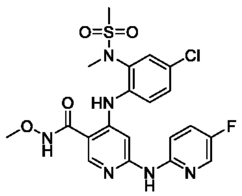 | 4-((4-chloro-2-(N-methyl methanesulfonamido)phenyl)amino)-6-((5-fluoropyridin-2-yl)amino)-N-methoxy nicotinamide |
| 47 | 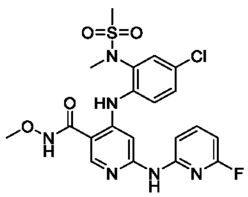 | 4-((4-chloro-2-(N-methyl methanesulfonamido)phenyl)amino)-6-((6-fluoropyridin-2-yl)amino)-N-methoxy nicotinamide |

(continued)

| Compound # | Structure | nomenclature |
|---|---|---|
| 48* | 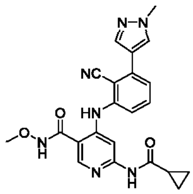 | 4-((2-cyano-3-(1-methyl-1H-pyrazol-4-yl)phenyl)amino)-6-(cyclopropylcarboxamido)-N-methoxy nicotinamide |
| 49* | 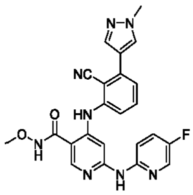 | 4-((2-cyano-3-(1-methyl-1H-pyrazol-4-yl)phenyl)amino)-6-((5-fluoropyridin-2-yl)amino)-N-methoxy nicotinamide |
| 50* | 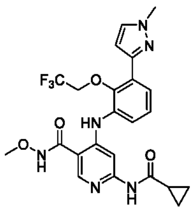 | 6-(cyclopropylcarboxamido)-N-methoxy-4-((3-(1-methyl-1H-pyrazol-3-yl)-2-(2,2,2-trifluoroethoxy)phenyl)amino)nicotinamide |
| 51* | 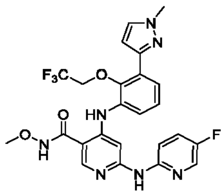 | 6-((5-fluoropyridin-2-yl)amino)-N-methoxy-4-((3-(1-methyl-1H-pyrazol-3-yl)-2-(2,2,2-trifluoroethoxy)phenyl)amino)nicotinamide |
| 52* | 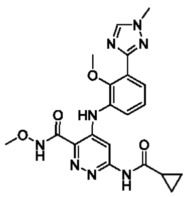 | 6-(cyclopropylcarboxamido)-N-methoxy-4-((2-methoxy-3-(1-methyl-1H-1, 2, 4-triazol-3-yl)phenyl)amino)pyridazine-3-carboxamide |
| 53* | 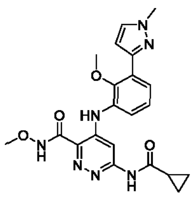 | 6-(cyclopropylcarboxamido)-N-methoxy-4-((2-methoxy-3-(1-methyl-1H-pyrazol-3-yl)phenyl)amino)pyridazine-3-carboxamide |
| 54* | 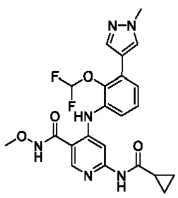 | 6-(cyclopropylcarboxamido)-4-((2-(difluoromethoxy)-3-(1-methyl-1H-pyrazol-4-yl)phenyl)amino)-N-methoxy nicotinamide |

(continued)

| Compound # | Structure | nomenclature |
|---|---|---|
| 55* | 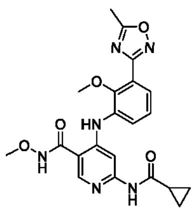 | 6-(cyclopropylcarboxamido)-N-methoxy-4-((2-methoxy-3-(5-methyl-1, 2, 4-oxadiazol-3-yl)phenyl)amino)nicotinamide |
| 56 | 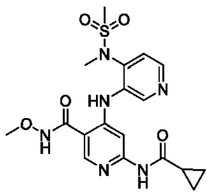 | 6-(cyclopropylcarboxamido)-N-methoxy-4-((4-(N-methyl methanesulfonamido)pyridin-3-yl)amino)nicotinamide |
| 57 | 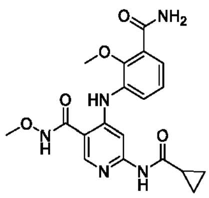 | 4-((3-carbamoyl-2-methoxy phenyl)amino)-6-(cyclopropylcarboxamido)-N-methoxy nicotinamide |
| 58 | 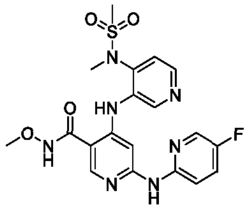 | 6-((5-fluoropyridin-2-yl)amino)-N-methoxy-4-((4-(N-methyl methanesulfonamido)pyridin-3-yl)amino)nicotinamide |
| 59* | 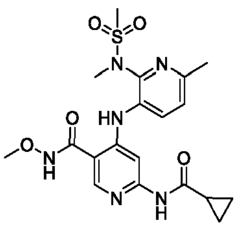 | 6-(cyclopropylcarboxamido)-N-methoxy-4-((6-methyl-2-(N-methyl methanesulfonamido)pyridin-3-yl)amino)nicotinamide |
| 60 | 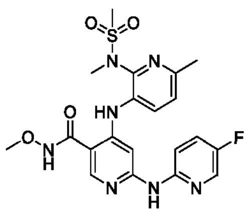 | 6-(((5-fluoropyridin-2-yl)amino)-N-methoxy-4-((6-methyl-2-(N-methyl methanesulfonamido)pyridin-3-yl)amino)nicotinamide |
| 61 | 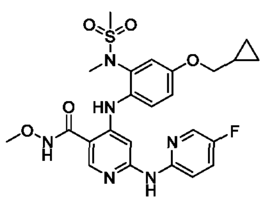 | 4-((4-(cyclopropyl methoxy)-2-(N-methyl methanesulfonamido) phenyl)amino)-6-((5-fluoropyridin-2-yl)amino)-N-methoxy nicotina-mide |

(continued)

| Compound # | Structure | nomenclature |
|---|---|---|
| 62 | 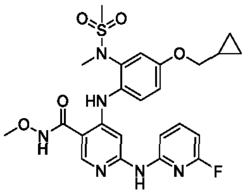 | 4-((4-(cyclopropyl methoxy)-2-(N-methyl methanesulfonamido) phenyl)amino)-6-((6-fluoropyridin-2-yl)amino)-N-methoxy nicotinamide |
| 63 | 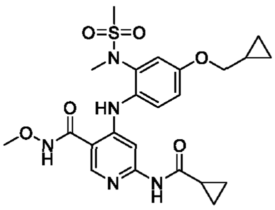 | 6-(cyclopropylcarboxamido)-4-((4-(cyclopropyl methoxy)-2-(N-methyl methanesulfonamido)phenyl)amino)-N-methoxy nicotinamide |
| 64* | 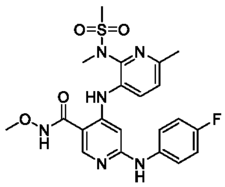 | 6-((4-fluorophenyl)amino)-N-methoxy-4-((6-methyl-2-(N-methyl methanesulfonamido)pyridin-3-yl)amino)nicotinamide |
| 65* | 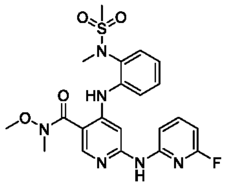 | 6-((6-fluoropyridin-2-yl)amino)-N-methoxy-N-methyl-4-((2-(N-methyl methanesulfonamido)phenyl)amino) nicotinamide |
| 66* | 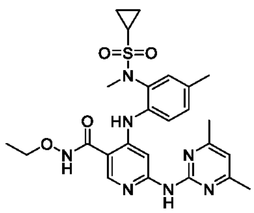 | 6-((4, 6-dimethyl pyrimidin-2-yl)amino)-N-ethoxy-4-((4-methyl-2-(N-methyl cyclopropylsulfonamido)phenyl)amino) nicotinamide |
| 67* | 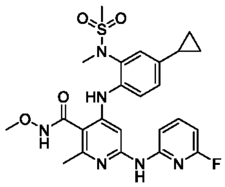 | 4-((4-cyclopropyl-2-(N-methyl methanesulfonamido)phenyl)amino)-6-((6-fluoropyridin-2-yl)amino)-N-methoxy-2-methyl nicotinamide |
| 68 | 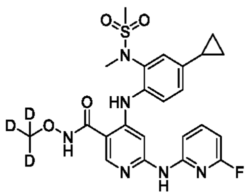 | 4-((4-cyclopropyl-2-(N-methyl methanesulfonamido)phenyl)amino)-6-((6-fluoropyridin-2-yl)amino)-N-(methoxy-d3)nicotinamide |

(continued)

| Compound # | Structure | nomenclature |
|---|---|---|
| 69 | 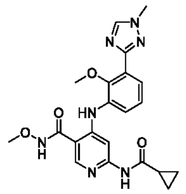 | 6-(cyclopropylcarboxamido)-N-methoxy-4-((2-methoxy-3-(1-methyl-1H-1, 2, 4-triazol-3-yl)phenyl)amino)-nicotinamide |
| 70 | 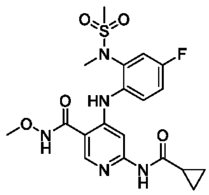 | 6-(cyclopropylcarboxamido)-4-((4-fluoro-2-(N-methyl methanesulfonamido)phenyl)amino)-N-methoxy nicotinamide |
| 71* (control) | 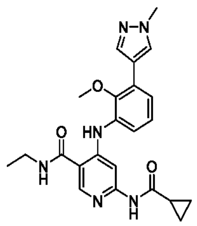 | 6-(cyclopropylcarboxamido)-N-ethyl-4-((2-methoxy-3-(1-methyl-1H-pyrazol-4-yl)phenyl)amino)nicotinamide |
| 72* | 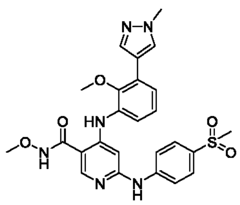 | N-methoxy-4-((2-methoxy-3-(1-methyl-1H-pyrazol-4-yl)phenyl)amino)-6-((4-(methanesulfonyl)phenyl)amino)nicotinamide |
| 73* | 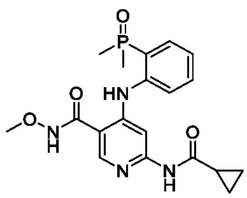 | 6-(cyclopropylcarboxamido)-4-((2-(dimethyl phosphoryl)phenyl) amino)-N-methoxy nicotinamide |
| 74* | 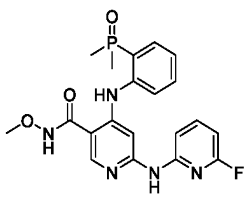 | 4-((2-(dimethyl phosphoryl)phenyl)amino)-6-((6-fluoropyridin-2-yl) amino)-N-methoxy nicotinamide |
| 75* | 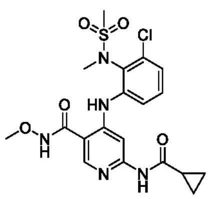 | 4-((3-chloro-2-(N-methyl methanesulfonamido)phenyl)amino)-6-(cyclopropylcarboxamido)-N-methoxy nicotinamide |

(continued)

| Compound # | Structure | nomenclature |
|---|---|---|
| 76* | 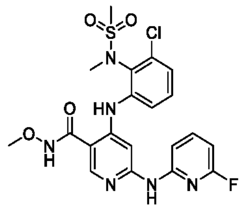 | 4-((3-chloro-2-(N-methyl methanesulfonamido)phenyl)amino)-6-((6-fluoropyridin-2-yl)amino)-N-methoxy nicotinamide |
| 77* | 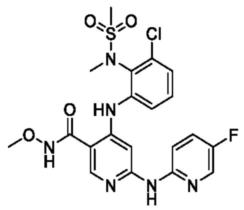 | 4-((3-chloro-2-(N-methyl methanesulfonamido)phenyl)amino)-6-((5-fluoropyridin-2-yl)amino)-N-methoxy nicotinamide |
| 78* | 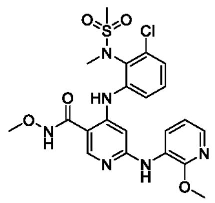 | 4-((3-chloro-2-(N-methyl methanesulfonamido)phenyl)amino)-N-methoxy-6-((2-methoxy pyridin-3-yl)amino)nicotinamide |
| 79 | 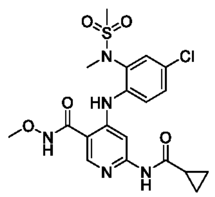 | 4-((4-chloro-2-(N-methyl methanesulfonamido)phenyl)amino)-6-(cyclopropylcarboxamido)-N-methoxy nicotinamide |
| 80 | 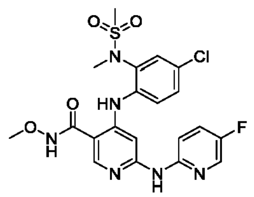 | 4-((4-chloro-2-(N-methyl methanesulfonamido)phenyl)amino)-6-((5-fluoropyridin-2-yl)amino)-N-methoxy nicotinamide |
| 81 | 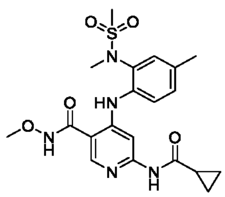 | 6-(cyclopropylcarboxamido)-N-methoxy-4-((4-methyl-2-(N-methyl methanesulfonamido)phenyl)amino) nicotinamide |
| 82* | 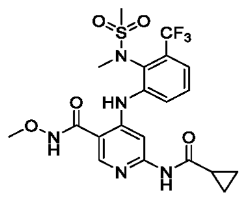 | 6-(cyclopropylcarboxamido)-N-methoxy-4-((2-(N-methyl methanesulfonamido)-3-(trifluoromethyl)phenyl)amino)nicotinamide |

(continued)

| Compound # | Structure | nomenclature |
|---|---|---|
| 83* | 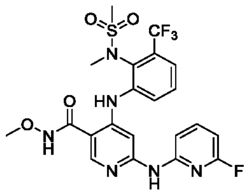 | 6-((6-fluoropyridin-2-yl)amino)-N-methoxy-4-((2-(N-methyl methanesulfonamido)-3-(trifluoromethyl)phenyl)amino)nicotinamide |
| 84* | 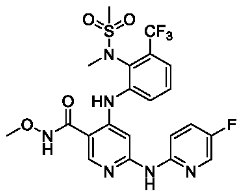 | 6-((5-fluoropyridin-2-yl)amino)-N-methoxy-4-((2-(N-methyl methanesulfonamido)-3-(trifluoromethyl)phenyl)amino)nicotinamide |
| 85* | 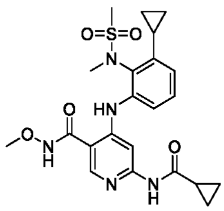 | 6-(cyclopropylcarboxamido)-4-((3-cyclopropyl-2-(N-methyl methanesulfonamido)phenyl)amino)-N-methoxy nicotinamide |
| 86* | 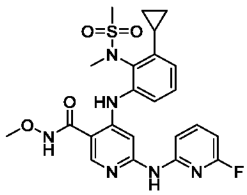 | 4-((3-cyclopropyl-2-(N-methyl methanesulfonamido)phenyl)amino)-6-(6-fluoropyridin-2-yl)-N-methoxy nicotinamide |
| 87* | 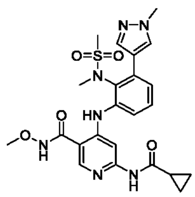 | 6-(cyclopropylcarboxamido)-N-methoxy-4-((3-(1-methyl-1H-pyrazol-4-yl)-2-(N-methyl methanesulfonamido)phenyl)amino) nicotinamide |
| 88* | 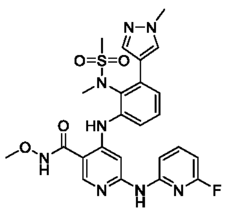 | 6-((6-fluoropyridin-2-yl)amino)-N-methoxy-4-((3-(1-methyl-1H-pyrazol-4-yl)-2-(N-methyl methanesulfonamido)phenyl)amino) nicotinamide |
| 89* | 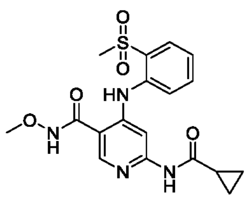 | 6-(cyclopropylcarboxamido)-N-methoxy-4-((2-methanesulfonyl) phenyl)amino) nicotinamide |

(continued)

| Compound # | Structure | nomenclature |
|---|---|---|
| 90 | 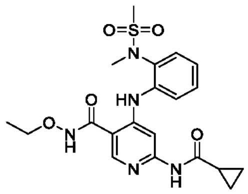 | 6-(cyclopropylcarboxamido)-N-ethoxy-4-((2-(N-methyl methanesulfonamido)phenyl)amino) nicotinamide |
| 91* | 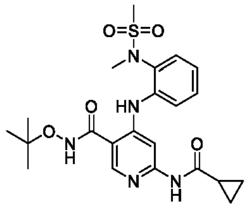 | N-(t-butoxy)-6-(cyclopropylcarboxamido)-4-((2-(N-methyl methanesulfonamido)phenyl)amino) nicotinamide |
| 92* | 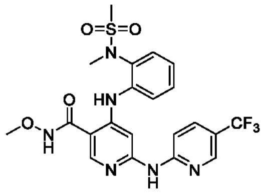 | N-methoxy-4-((2-(N-methyl methanesulfonamido)phenyl)amino)-6-((5-(trifluoromethyl)pyridin-2-yl)amino)nicotinamide |
| 93* (control) | 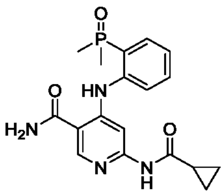 | 6-(cyclopropylcarboxamido)-4-((2-(dimethyl phosphoryl)phenyl) amino)nicotinamide |
| 94* | 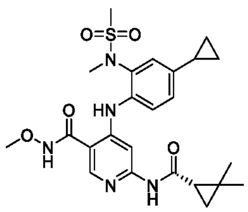 | (S)-4-((4-cyclopropyl-2-(N-methyl methanesulfonamido)phenyl) amino)-6-(2, 2-dimethyl cyclopropyl-1-carboxamido)-N-methoxy nicotinamide |
| 95 | 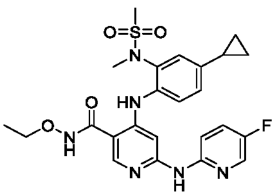 | 4-((4-cyclopropyl-2-(N-methyl methanesulfonamido)phenyl)amino)-N-ethoxy-6-((5-fluoropyridin-2-yl)amino)nicotinamide |
| 96* | 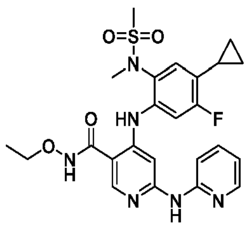 | 4-((4-cyclopropyl-5-fluoro-2-(N-methyl methanesulfonamido)phenyl)amino)-N-ethoxy-6-(pyridin-2-ylamino)nicotinamide |

(continued)

| Compound # | Structure | nomenclature |
|---|---|---|
| 97 | 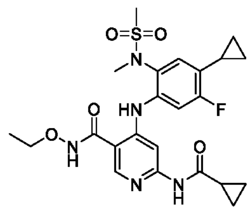 | 6-(cyclopropylcarboxamido)-4-((4-cyclopropyl-5-fluoro-2-(N-methyl methanesulfonamido)phenyl)amino)-N-ethoxy nicotinamide |
| 98 | 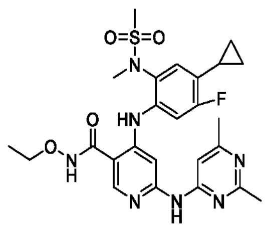 | 4-((4-cyclopropyl-5-fluoro-2-(N-methyl methanesulfonamido)phenyl)amino)-6-(((2, 6-dimethyl pyrimidin-4-yl)amino)-N-ethoxy nicotinamide |
| 99* | 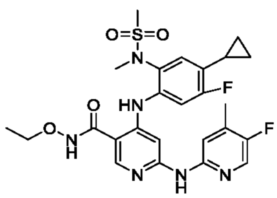 | 4-((4-cyclopropyl-5-fluoro-2-(N-methyl methanesulfonamido)phenyl)amino)-N-ethoxy-6-((5-fluoro-4-methyl pyridin-2-yl)amino)nicotinamide |
| 100* (control) | 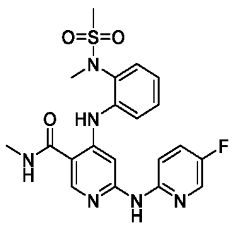 | 6-((5-fluoropyridin-2-yl)amino)-N-methyl-4-((2-(N-methyl methanesulfonamido)phenyl)amino)nicotinamide |
| 101 | 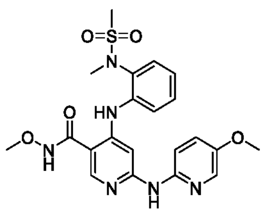 | N-methoxy-6-(((5-methoxy pyridin-2-yl)amino)-4-((2-(N-methyl methanesulfonamido)phenyl)amino)nicotinamide |
| 102 | 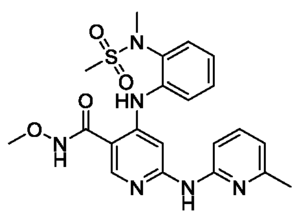 | N-methoxy-4-((2-(N-methyl methanesulfonamido)phenyl)amino)-6-((6-methyl pyridin-2-yl)amino)nicotinamide |
| 103* | 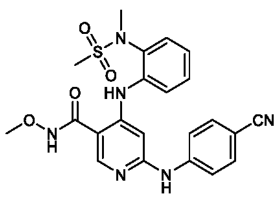 | 6-((4-cyano phenyl)amino)-N-methoxy-4-((2-(N-methyl methanesulfonamido)phenyl)amino)nicotinamide |

(continued)

| Compound # | Structure | nomenclature |
|---|---|---|
| 104* | 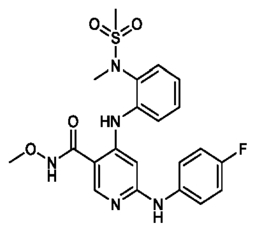 | 6-((4-fluorophenyl)amino)-N-methoxy-4-((2-(N-methyl methanesulfonamido)phenyl)amino) nicotinamide |
| 105* | 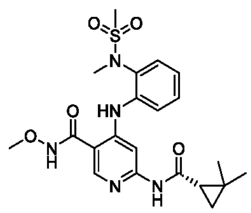 | (S)-6-(2, 2-dimethyl cyclopropyl-1-carboxamido)-N-methoxy-4-((2-(N-methyl methanesulfonamido)phenyl)amino) nicotinamide |
| 106 | 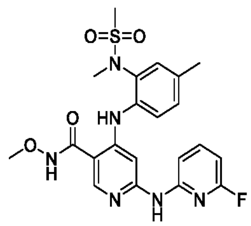 | 6-((6-fluoropyridin-2-yl)amino)-N-methoxy-4-((4-methyl-2-(N-methyl methanesulfonamido)phenyl)amino) nicotinamide |
| 107* | 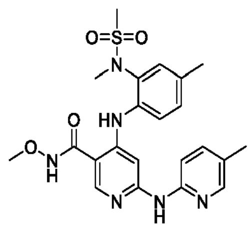 | N-methoxy-4-((4-methyl-2-(N-methyl methanesulfonamido)phenyl) amino)-6-((5-methyl pyridin-2-yl)amino)nicotinamide |
| 108 | 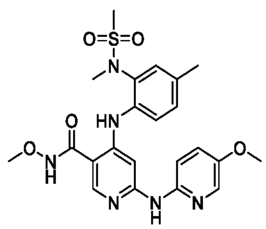 | N-methoxy-6-(((5-methoxy pyridin-2-yl)amino)-4-((4-methyl-2-(N-methyl methanesulfonamido)phenyl)amino) nicotinamide |
| 109 | 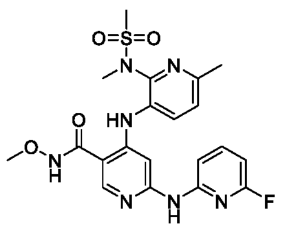 | 6-((6-fluoropyridin-2-yl)amino)-N-methoxy-4-((6-methyl-2-(N-methyl methanesulfonamido)pyridin-3-yl)amino)nicotinamide |
| 110 | 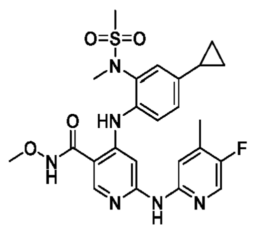 | 4-((6-cyclopropyl-2-(N-methyl methanesulfonamido)phenyl)amino)-6-((5-fluoro-4-methyl pyridin-2-yl)amino)-N-methoxy nicotinamide |

(continued)

| Compound # | Structure | nomenclature |
|---|---|---|
| 111 | 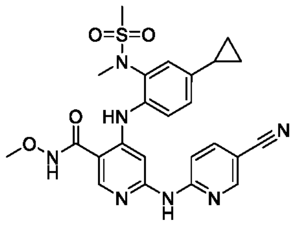 | 4-((4-cyclopropyl-2-(N-methyl methanesulfonamido)phenyl)amino)-6-((5-cyano pyridin-2-yl)amino)-N-methoxy nicotinamide |
| 112 | 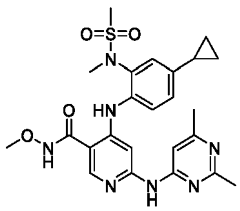 | 4-((4-cyclopropyl-2-(N-methyl methanesulfonamido)phenyl)amino)-6-(((2, 6-dimethyl pyrimidin-4-yl)amino)-N-methoxy nicotinamide |
| 113 | 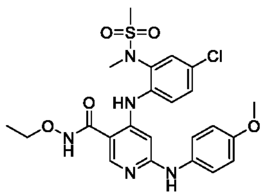 | 4-((4-chloro-2-(N-methyl methanesulfonamido)phenyl)amino)-N-ethoxy-6-((4-methoxy phenyl) amino)nicotinamide |
| 114 | 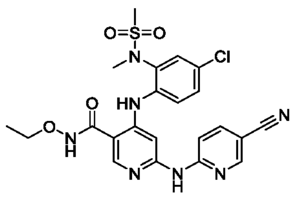 | 4-((4-chloro-2-(N-methyl methanesulfonamido)phenyl)amino)-N-ethoxy-6-((5-cyano pyridin-2-yl)amino)nicotinamide |
| 115 | 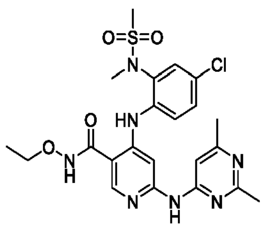 | 4-((4-chloro-2-(N-methyl methanesulfonamido)phenyl)amino)-6-((2, 6-dimethyl pyrimidin-4-yl)amino)-N-ethoxy nicotinamide |
| 116 | 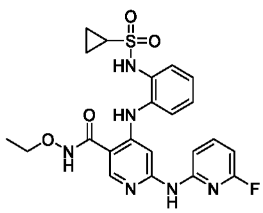 | 4-((2-(cyclopropyl sulfonamido)phenyl)amino)-N-ethoxy-6-((6-fluoropyridin-2-yl)amino)nicotinamide |
| 117* | 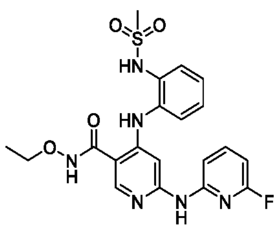 | N-ethoxy-6-(((6-fluoropyridin-2-yl)amino)-2-((2-(methyl sulfonamido)phenyl)amino)nicotinamide |

(continued)

| Compound # | Structure | nomenclature |
|---|---|---|
| 118 | 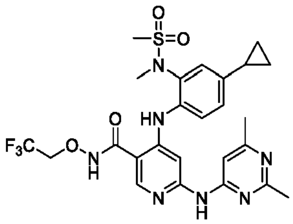 | 4-((4-cyclopropyl-2-(N-methyl methanesulfonamido)phenyl)amino)-6-(((2, 6-dimethyl pyrimidin-4-yl)amino)-N-(2,2,2-trifluoroethoxy)nicotinamide |
| 119 | 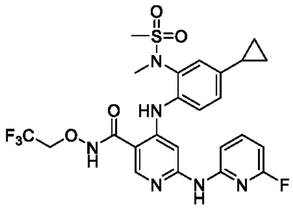 | 4-((4-cyclopropyl-2-(N-methyl methanesulfonamido)phenyl)amino)-6-((6-fluoropyridin-2-yl)amino)-N-(2,2,2-trifluoroethoxy)nicotinamide |
| 120* | 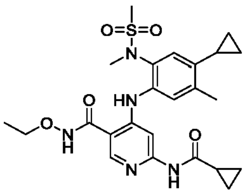 | 6-(cyclopropylcarboxamido)-4-((4-cyclopropyl-5-methyl-2-(N-methyl methanesulfonamido)phenyl)amino)-N-ethoxy nicotinamide |
| 121* | 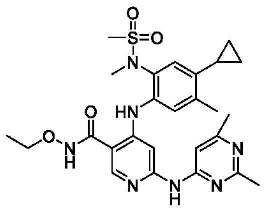 | 4-((4-cyclopropyl-5-methyl-2-(N-methyl methanesulfonamido)phenyl)amino)-6-(((2, 6-dimethyl pyrimidin-4-yl)amino)-N-ethoxy nicotinamide |
| 122* | 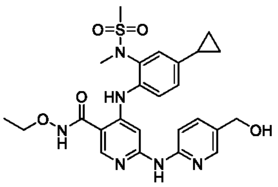 | 4-((4-cyclopropyl-2-(N-methyl methanesulfonamido)phenyl)amino)-N-ethoxy-6-((5-(hydroxymethyl)pyridin-2-yl)amino)nicotinamide |
| 123 * | 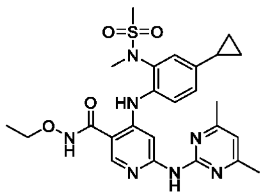 | 4-((4-cyclopropyl-2-(N-methyl methanesulfonamido)phenyl)amino)-6-((4, 6-dimethyl pyrimidin-2-yl)amino)-N-ethoxy nicotinamide |
| 124* | 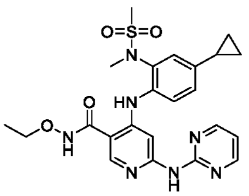 | 4-((4-cyclopropyl-2-(N-methyl methanesulfonamido)phenyl)amino)-N-ethoxy-6-(pyrimidin-2-ylamino)nicotinamide |

(continued)

| Compound # | Structure | nomenclature |
|---|---|---|
| 125 | 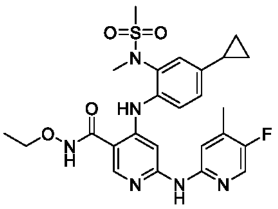 | 4-((4-cyclopropyl-2-(N-methyl methanesulfonamido)phenyl)amino)-N-ethoxy-6-((5-fluoro-4-methyl pyridin-2-yl)amino)nicotinamide |
| 126 | 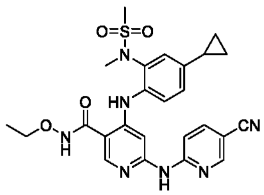 | 6-((5-cyano pyridin-2-yl)amino)-4-((4-cyclopropyl-2-(N-methyl methanesulfonamido)phenyl)amino)-N-ethoxy nicotinamide |
| 127 | 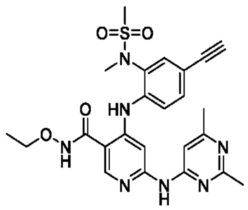 | 6-((2, 6-dimethyl pyrimidin-4-yl)amino)-N-ethoxy-4-((4-ethynyl-2-(N-methyl methanesulfonamido)phenyl)amino) nicotinamide |
| 128 | 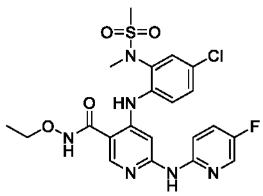 | 4-((4-chloro-2-(N-methyl methanesulfonamido)phenyl)amino)-N-ethoxy-6-((5-fluoropyridin-2-yl)amino)nicotinamide |
| 129 | 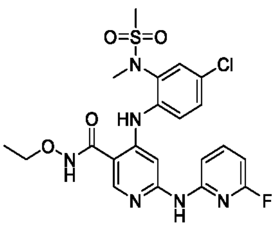 | 4-((4-chloro-2-(N-methyl methanesulfonamido)phenyl)amino)-N-ethoxy-6-((6-fluoropyridin-2-yl)amino)nicotinamide |
| 130 | 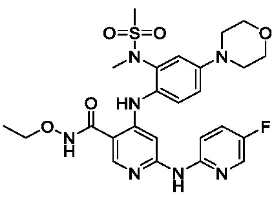 | N-ethoxy-6-(((5-fluoropyridin-2-yl)amino)-4-((2-(N-methyl methanesulfonamido)-4-morpholinophenyl)amino)nicotinamide |
| 131 | 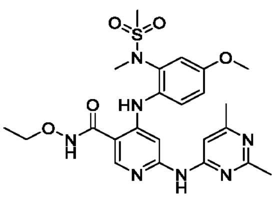 | 6-((2, 6-dimethyl pyrimidin-4-yl)amino)-N-ethoxy-4-((4-methoxy-2-(N-methyl methanesulfonamido)phenyl)amino) nicotinamide |

(continued)

| Compound # | Structure | nomenclature |
|---|---|---|
| 132 | 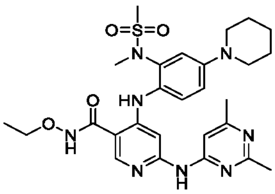 | 6-((2, 6-dimethyl pyrimidin-4-yl)amino)-N-ethoxy-4-((2-(N-methyl methanesulfonamido)-4-(piperidin-1-yl)phenyl)amino)nicotinamide |
| 133 | 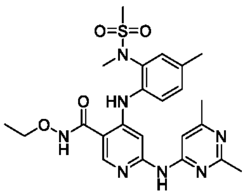 | 6-((2, 6-dimethyl pyrimidin-4-yl)amino)-N-ethoxy-4-((4-methyl-2-(N-methyl methanesulfonamido)phenyl)amino) nicotinamide |
| 134 | 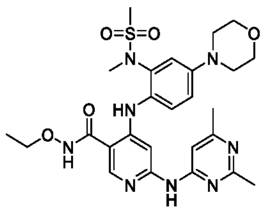 | 6-((2, 6-dimethyl pyrimidin-4-yl)amino)-N-ethoxy-4-((2-(N-methyl methanesulfonamido)-4-morpholinophenyl)amino)nicotinamide |
| 135* | 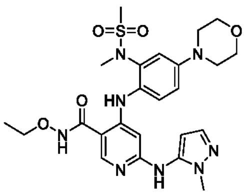 | N-ethoxy-6-(((1-methyl-1H-pyrazol-5-yl)amino)-4-((2-(N-methyl methanesulfonamido)-4-morpholinophenyl)amino)nicotinamide |
| 136 | 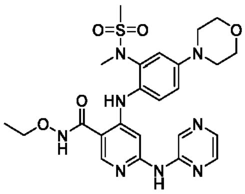 | N-ethoxy-4-((2-(N-methyl methanesulfonamido)-4-morpholinophenyl)amino)-6-(pyrazin-2-ylamino)nicotinamide |
| 137* | 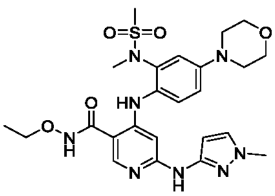 | N-ethoxy-6-(((1-methyl-1H-pyrazol-3-yl)amino)-4-((2-(N-methyl methanesulfonamido)-4-morpholinophenyl)amino)nicotinamide |
| 138 | 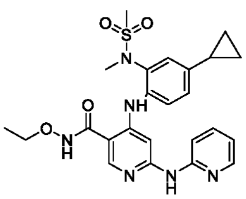 | 4-((4-cyclopropyl-2-(N-methyl methanesulfonamido)phenyl)amino)-N-ethoxy-6-(pyridin-2-ylamino)nicotinamide |

(continued)

| Compound # | Structure | nomenclature |
|---|---|---|
| 139 | 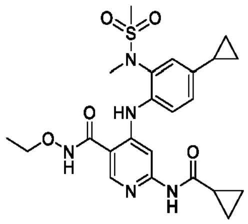 | 6-(cyclopropylcarboxamido)-4-((4-cyclopropyl-2-(N-methyl methanesulfonamido)phenyl)amino)-N-ethoxy nicotinamide |
| 140* | 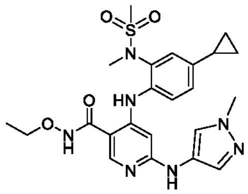 | 4-((4-cyclopropyl-2-(N-methyl methanesulfonamido)phenyl)amino)-N-ethoxy-6-((1-methyl-1H-pyrazol-4-ylamino)nicotinamide |
| 141 | 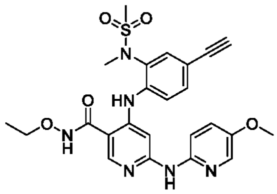 | N-ethoxy-4-((4-ethynyl-2-(N-methyl methanesulfonamido)phenyl) amino)-6-(5-methoxy pyridin-2-ylamino)nicotinamide |
| 142 | 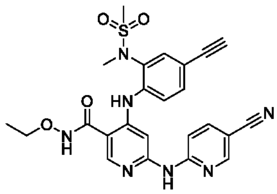 | 6-(5-cyano pyridin-2-ylamino)-N-ethoxy-4-((4-ethynyl-2-(N-methyl methanesulfonamido)phenyl)amino) nicotinamide |
| 143 | 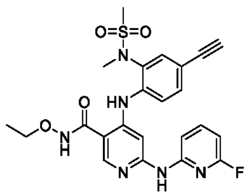 | N-ethoxy-4-((4-ethynyl-2-(N-methyl methanesulfonamido)phenyl) amino)-6-(6-fluoropyridin-2-ylamino)nicotinamide |
| 144 | 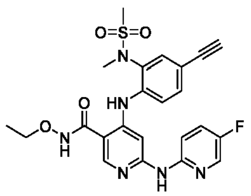 | N-ethoxy-4-((4-ethynyl-2-(N-methyl methanesulfonamido)phenyl) amino)-6-(5-fluoropyridin-2-ylamino)nicotinamide |
| 145 | 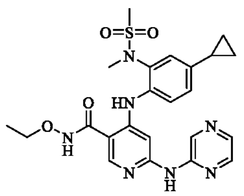 | 4-((4-cyclopropyl-2-(N-methyl methanesulfonamido)phenyl)amino)-N-ethoxy-6-(pyrazin-2-ylamino)nicotinamide |

(continued)

| Compound # | Structure | nomenclature |
|---|---|---|
| 146 | 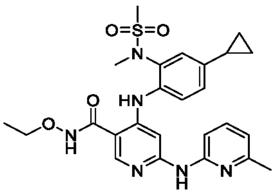 | 4-((4-cyclopropyl-2-(N-methyl methanesulfonamido)phenyl)amino)-N-ethoxy-6-((6-methyl pyridin-2-yl)amino)nicotinamide |
| 147* | 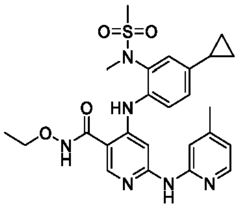 | 4-((4-cyclopropyl-2-(N-methyl methanesulfonamido)phenyl)amino)-N-ethoxy-6-((4-methyl pyridin-2-yl)amino)nicotinamide |
| 148* | 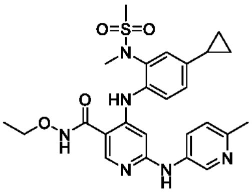 | 4-((4-cyclopropyl-2-(N-methyl methanesulfonamido)phenyl)amino)-N-ethoxy-6-((6-methyl pyridin-3-yl)amino)nicotinamide |
| 149* | 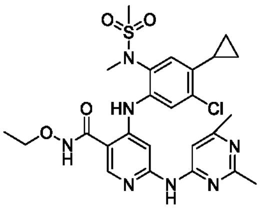 | 4-((5-chloro-4-cyclopropyl-2-(N-methyl methanesulfonamido)phenyl)amino)-6-(((2, 6-dimethyl pyrimidin-4-yl)amino)-N-ethoxy nicotinamide |
| 150* | 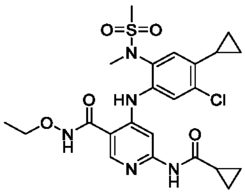 | 4-((5-chloro-4-cyclopropyl-2-(N-methyl methanesulfonamido)phenyl)amino)-6-(cyclopropylcarboxamido)-N-ethoxy nicotinamide |
| 151 | 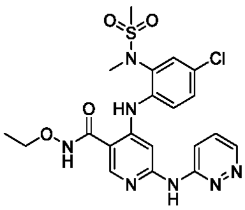 | 4-((4-chloro-2-(N-methyl methanesulfonamido)phenyl)amino)-N-ethoxy-6-(pyridazin-3-ylamino)nicotinamide |
| 152* | 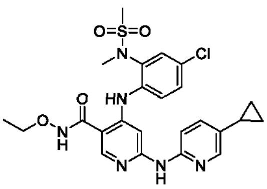 | 4-((4-chloro-2-(N-methyl methanesulfonamido)phenyl)amino)-6-((5-cyclopropyl pyridin-2-yl)amino)-N-ethoxy nicotinamide |

(continued)

| Compound # | Structure | nomenclature |
|---|---|---|
| 153 | 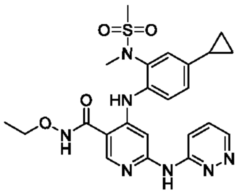 | 4-((4-cyclopropyl-2-(N-methyl methanesulfonamido)phenyl)amino)-N-ethoxy-6-(pyridazin-3-ylamino)nicotinamide |
| 154 | 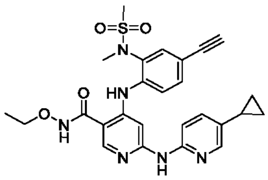 | 6-((5-cyclopropyl pyridin-2-yl)amino)-N-ethoxy-4-((4-ethynyl-2-(N-methyl methanesulfonamido)phenyl)amino)nicoti namide |
| 155 | 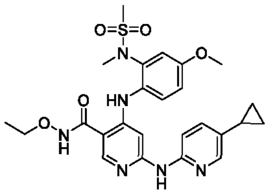 | 6-((5-cyclopropyl pyridin-2-yl)amino)-N-ethoxy-4-((4-methoxy-2-(N-methyl methanesulfonamido)phenyl)amino)nicoti namide |
| 156 | 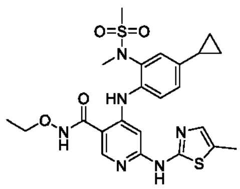 | 4-((4-cyclopropyl-2-(N-methyl methanesulfonamido)phenyl)amino)-N-ethoxy-6-((5-methyl thiazol-2-yl)amino)nicotinamide |
| 157 | 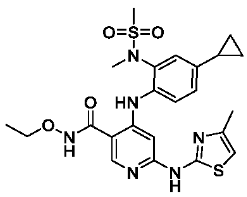 | 4-((4-cyclopropyl-2-(N-methyl methanesulfonamido)phenyl)amino)-N-ethoxy-6-((4-methyl thiazol-2-yl)amino)nicotinamide |
| 158* | 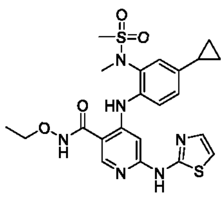 | 4-((4-cyclopropyl-2-(N-methyl methanesulfonamido)phenyl)amino)-N-ethoxy-6-(thiazol-2-ylamino)nicotinamide |
| 159 | 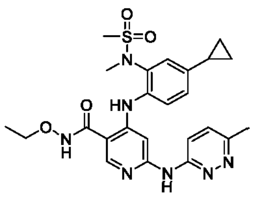 | 4-((4-cyclopropyl-2-(N-methyl methanesulfonamido)phenyl)amino)-N-ethoxy-6-((6-methyl pyridazin-3-yl)amino)nicotinamide |
| 160 | 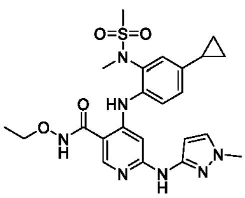 | 4-((4-cyclopropyl-2-(N-methyl methanesulfonamido)phenyl)amino)-N-ethoxy-6-((1-methyl-1H-pyrazol-3-yl)amino)nicotinamide |

(continued)

| Compound # | Structure | nomenclature |
|---|---|---|
| 161* | 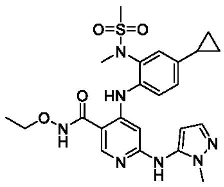 | 4-((4-cyclopropyl-2-(N-methyl methanesulfonamido)phenyl)amino)-N-ethoxy-6-((1-methyl-1H-pyrazol-5-yl)amino)nicotinamide |
| 162 | 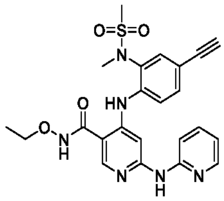 | N-ethoxy-4-((4-ethynyl-2-(N-methyl methanesulfonamido)phenyl) amino)-6-(pyridin-2-ylamino)nicotinamide |
| 163* | 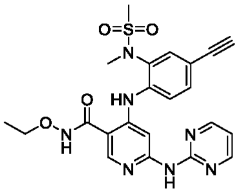 | N-ethoxy-4-((4-ethynyl-2-(N-methyl methanesulfonamido)phenyl) amino)-6-(pyrimidin-2-ylamino)nicotinamide |
| 164* | 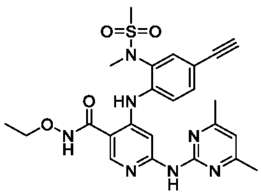 | 6-((4, 6-dimethyl pyrimidin-2-yl)amino)-N-ethoxy-4-((4-ethynyl-2-(N-methyl methanesulfonamido)phenyl)amino) nicotinamide |
| 165* | 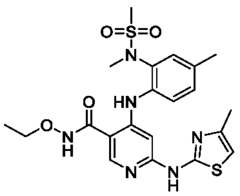 | N-ethoxy-4-((4-methyl-2-(N-methyl methanesulfonamido)phenyl) amino)-6-((4-methyl thiazol-2-yl)amino)nicotinamide |
| 166 | 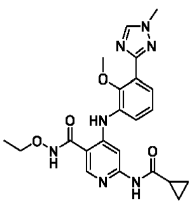 | 6-(cyclopropylcarboxamido)-N-ethoxy-4-((2-methoxy-3-(1-methyl-1H-1, 2, 4-triazol-3-yl)phenyl)amino)nicotinamide |
| 167 | 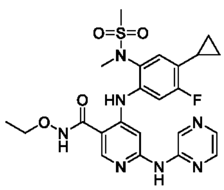 | 4-((4-cyclopropyl-5-fluoro-2-(N-methyl methanesulfonamido)phenyl)amino)-N-ethoxy-6-(pyrazin-2-ylamino)nicotinamide |

(continued)

| Compound # | Structure | nomenclature |
|---|---|---|
| 168* | 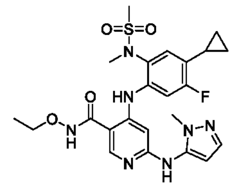 | 4-((4-cyclopropyl-5-fluoro-2-(N-methyl methanesulfonamido)phenyl)amino)-N-ethoxy-6-((1-methyl-1H-pyrazol-5-yl)amino)nicotinamide |
| 169* | 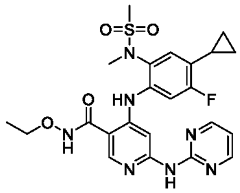 | 4-((4-cyclopropyl-5-fluoro-2-(N-methyl methanesulfonamido)phenyl)amino)-N-ethoxy-6-(pyrimidin-2-ylamino)nicotinamide |
| 170 | 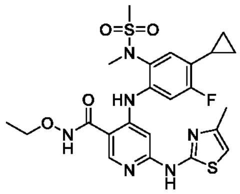 | 4-((4-cyclopropyl-5-fluoro-2-(N-methyl methanesulfonamido)phenyl)amino)-N-ethoxy-6-((4-methyl thiazol-2-yl)amino)nicotinamide |
| 171 | 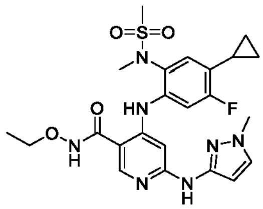 | 4-((4-cyclopropyl-5-fluoro-2-(N-methyl methanesulfonamido)phenyl)amino)-N-ethoxy-6-((1-methyl-1H-pyrazol-3-yl)amino)nicotinamide |
| 172* | 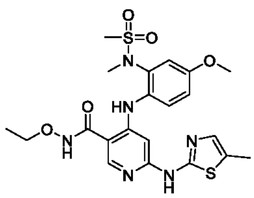 | N-ethoxy-4-((4-methoxy-2-(N-methyl methanesulfonamido)phenyl) amino)-6-(5-methyl thiazol-2-ylamino)nicotinamide |
| 173 | 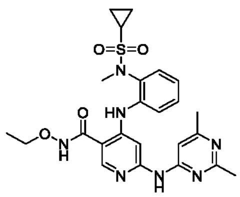 | 6-((2, 6-dimethyl pyrimidin-4-yl)amino)-N-ethoxy-4-((2-(N-methyl cyclopropyl sulfonamido)phenyl)amino)nicotinamide |
| 174 | 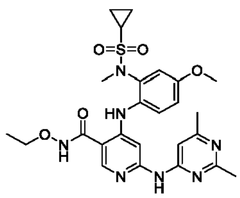 | 6-((2, 6-dimethyl pyrimidin-4-yl)amino)-N-ethoxy-4-((4-methoxy-2-(N-methyl cyclopropylsulfonamido)phenyl)amino) nicotinamide |

(continued)

| Compound # | Structure | nomenclature |
|---|---|---|
| 175 | 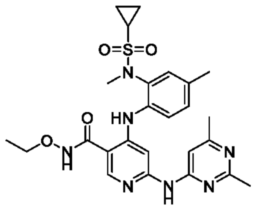 | 6-((2, 6-dimethyl pyrimidin-4-yl)amino)-N-ethoxy-4-((4-methyl-2-(N-methyl cyclopropylsulfonamido)phenyl)amino) nicotinamide |
| 176 | 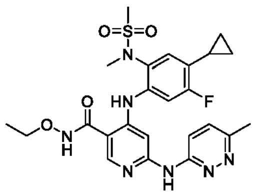 | 4-((4-cyclopropyl-5-fluoro-2-(N-methyl methanesulfonamido)phenyl)amino)-N-ethoxy-6-((6-methyl pyridazin-3-yl)amino)nicotinamide |
| 177 | 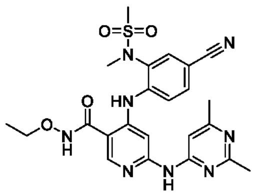 | 4-((4-cyano-2-(N-methyl methanesulfonamido)phenyl)amino)-6-(((2, 6-dimethyl pyrimidin-4-yl)amino)-N-ethoxy nicotinamide |
| 178 | 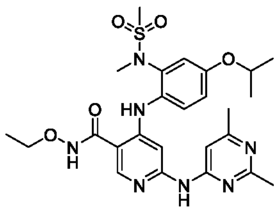 | 6-((2, 6-dimethyl pyrimidin-4-yl)amino)-N-ethoxy-4-((4-isopropoxy-2-(N-methyl methanesulfonamido)phenyl)amino)nicotinamide |
| 179 | 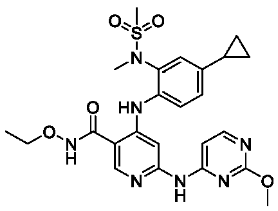 | 4-((4-cyclopropyl-2-(N-methyl methanesulfonamido)phenyl)amino)-N-ethoxy-6-((2-methoxy pyrimidin-4-yl)amino)nicotinamide |
| 180 | 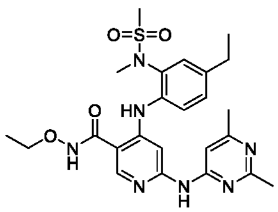 | 6-(((2, 6-dimethyl pyrimidin-4-yl)amino)-N-ethoxy-4-((4-ethyl-2-(N-methyl methanesulfonamido)phenyl)amino)nicotinamide |
| 181 | 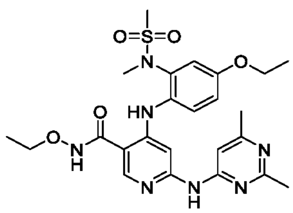 | 6-(((2, 6-dimethyl pyrimidin-4-yl)amino)-N-ethoxy-4-((4-ethoxy-2-(N-methyl methanesulfonamido)phenyl)amino)nicotinamide |

(continued)

| Compound # | Structure | nomenclature |
|---|---|---|
| 182 | 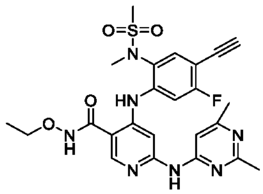 | 6-(((2, 6-dimethyl pyrimidin-4-yl)amino)-N-ethoxy-4-((4-ethynyl-5-fluoro-2-(N-methyl methanesulfonamido)phenyl)amino) nicotinamide |
| 183 | 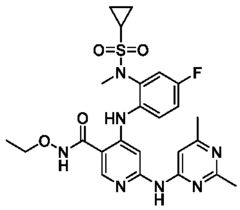 | 6-(((2, 6-dimethyl pyrimidin-4-yl)amino)-N-ethoxy-4-((4-fluoro-2-(N-methyl cyclopropylsulfonamido)phenyl)amino) nicotinamide |
| 184* | 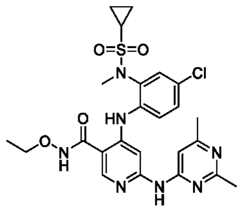 | 6-((4, 6-dimethyl pyrimidin-2-yl)amino)-N-ethoxy-4-((4-methyl-2-(N-methyl cyclopropylsulfonamido)phenyl)amino) nicotinamide |
| 185 | 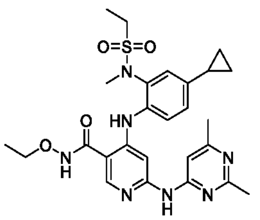 | 4-((4-cyclopropyl-2-(N-methyl ethyl sulfonamido)phenyl)amino)-6-(((2, 6-dimethyl pyrimidin-4-yl)amino)-N-ethoxy nicotinamide |
| 186 | 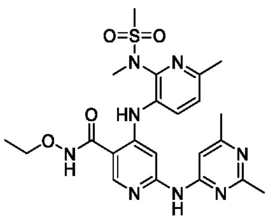 | 6-(((2, 6-dimethyl pyrimidin-4-yl)amino)-N-ethoxy-4-((6-methyl-2-(N-methyl methanesulfonamido)pyridin-3-yl)amino)nicotinamide |
| 187 | 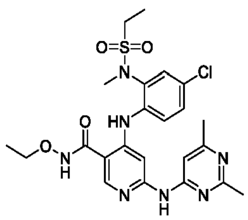 | 4-((4-chloro-2-(N-methyl ethyl sulfonamido)phenyl)amino)-6-((2, 6-dimethyl pyrimidin-4-yl)amino)-N-ethoxynicotinamide |
| 188 | 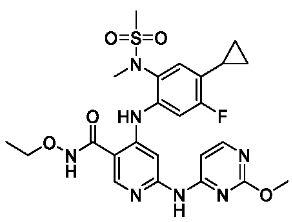 | 4-((4-cyclopropyl-5-fluoro-2-(N-methyl methanesulfonamido)phenyl)amino)-N-ethoxy-6-((2-methoxy pyrimidin-4-yl)amino)nicotinamide |

(continued)

| Compound # | Structure | nomenclature |
|---|---|---|
| 189 | 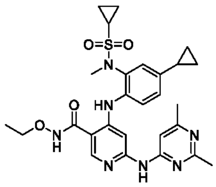 | 4-((4-cyclopropyl-2-(N-methyl cyclopropylsulfonamido)phenyl)amino)-6-(((2, 6-dimethyl pyrimidin-4-yl)amino)-N-ethoxy nicotinamide |
| 190 | 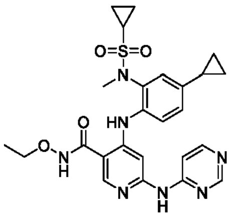 | 4-((4-cyclopropyl-2-(N-methyl cyclopropylsulfonamido)phenyl)amino)-N-ethoxy-6-(pyrimidin-4-ylamino)nicotinamide |
| 195* | 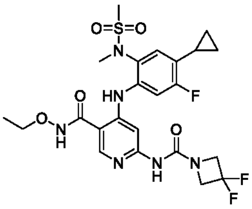 | 4-((4-cyclopropyl-5-fluoro-2-(N-methyl methanesulfonamido)phenyl)amino)-6-(3, 3-difluoroazetidin-1-ylcarboxamido)-N-ethoxy nicotinamide |
| 196* | 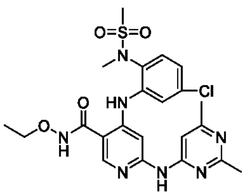 | 4-((5-chloro-2-(N-methyl methanesulfonamido)phenyl)amino)-6-((2, 6-dimethyl pyrimidin-4-yl)amino)-N-ethoxy nicotinamide |
| 197 | 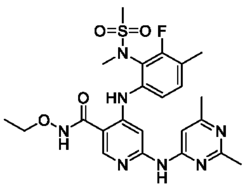 | 6-(((2, 6-dimethyl pyrimidin-4-yl)amino)-N-ethoxy-4-((3-fluoro-4-methyl-2-(N-methyl methanesulfonamido)phenyl)amino) nicotinamide |
| 198 | 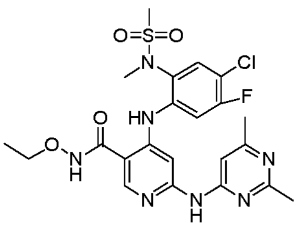 | 4-((4-chloro-5-fluoro-2-(N-methyl methanesulfonamido)phenyl) amino)-6-((2, 6-dimethyl pyrimidin-4-yl)amino)-N-ethoxy nicotinamide |
| 199* | 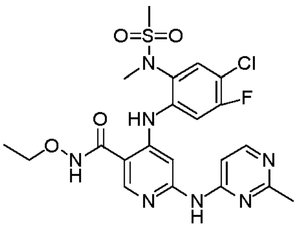 | 4-((4-chloro-5-fluoro-2-(N-methyl methanesulfonamido)phenyl) amino)-N-ethoxy-6-((2-methyl pyrimidin-4-yl)amino)nicotinamide |

(continued)

| Compound # | Structure | nomenclature |
|---|---|---|
| 200 | 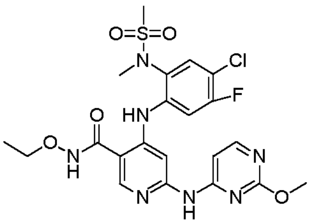 | 4-((4-chloro-5-fluoro-2-(N-methyl methanesulfonamido)phenyl) amino)-N-ethoxy-6-((2-methoxy pyrimidin-4-yl)amino)nicotinamide |
| 202 | 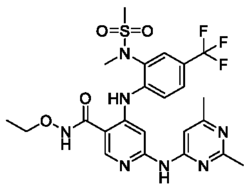 | 6-((2, 6-dimethyl pyrimidin-4-yl)amino)-N-ethoxy-4-((2-(N-methyl methanesulfonamido)-4-(trifluoromethyl)phenyl)amino)nicotinamide |
| 204 | 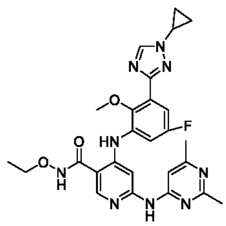 | 4-((3-(1-cyclopropyl-1H-1, 2, 4-triazol-3-yl)-5-fluoro-2-methoxy phenyl)amino)-6-((2, 6-dimethyl pyridin-4-yl)amino)-N-ethyl nicotinamide |
| 291* | 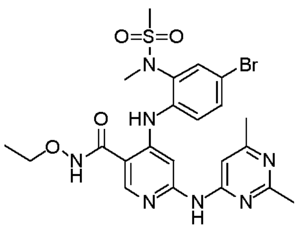 | 4-((4-bromo-5-fluoro-2-(N-methyl methanesulfonamido)phenyl) amino)-6-((2, 6-dimethyl pyrimidin-4-yl)amino)-N-ethoxy nicotinamide |
| 194 | 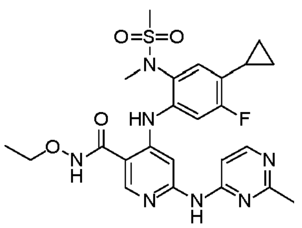 | 4-((4-cyclopropyl-5-fluoro-2-(N-methyl methanesulfonamido)phenyl)amino)-N-ethoxy-6-((2-methyl pyrimidin-4-yl)amino)nicotinamide |
| 292* | 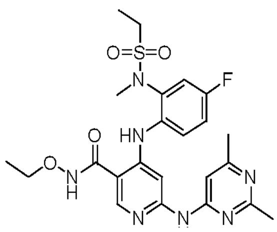 | 6-((2, 6-dimethyl pyrimidin-4-yl)amino)-N-ethoxy-4-((4-fluoro-2-(N-methyl ethyl sulfonamido)phenyl)amino)nicotinamide |
| 293* | 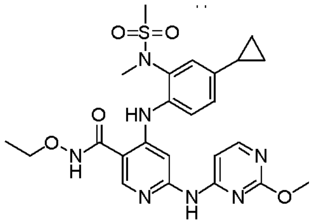 | 4-((4-cyclopropyl-2-(N-methyl methanesulfonamido)phenyl)amino)-N-ethoxy-6-((2-methoxy pyrimidin-4-yl)amino)nicotinamide |

(continued)

| Compound # | Structure | nomenclature |
|---|---|---|
| 192 | 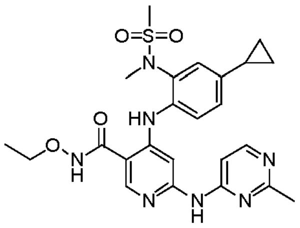 | 4-((4-cyclopropyl-2-(N-methyl methanesulfonamido)phenyl)amino)-N-ethoxy-6-((2-methyl pyrimidin-4-yl)amino)nicotinamide |
| 193 | 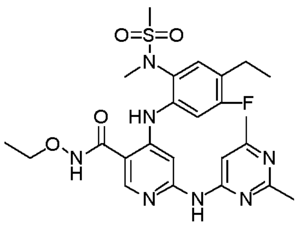 | 6-((2, 6-dimethyl pyrimidin-4-yl)amino)-N-ethoxy-4-((4-ethyl-5-fluoro-2-(N-methyl methanesulfonamido)phenyl)amino)nicotinamide |
| 203 | 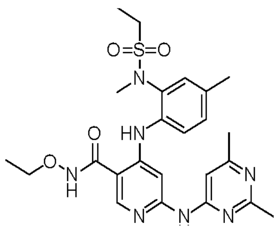 | 6-((2, 6-dimethyl pyrimidin-4-yl)amino)-N-ethoxy-4-((4-methyl-2-(N-methyl ethyl sulfonamido)phenyl)amino)nicotinamide |
| 230 | 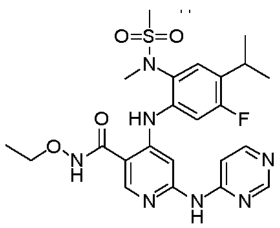 | N-ethoxy-4-((5-fluoro-4-isopropyl-2-(N-methyl methanesulfonamido)phenyl)amino)-6-(pyrimidin-4-ylamino)nicotinamide |
| 229 | 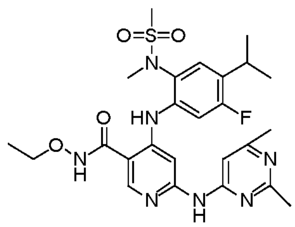 | 6-((2, 6-dimethyl pyrimidin-4-yl)amino)-N-ethoxy-4-((5-fluoro-4-isopropyl-2-(N-methyl methanesulfonamido)phenyl)amino)nicotinamide |
| 201 | 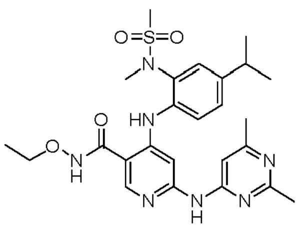 | 6-((2, 6-dimethyl pyrimidin-4-yl)amino)-N-ethoxy-4-((4-isopropyl-2-(N-methyl methanesulfonamido)phenyl)amino)nicotinamide |

(continued)

| Compound # | Structure | nomenclature |
|---|---|---|
| 294* | 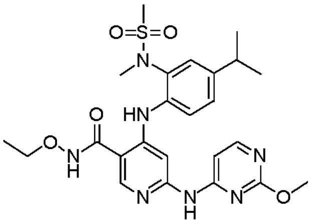 | N-ethoxy-4-((4-isopropyl-2-(N-methyl methanesulfonamido)phenyl) amino)-6-((2-methoxy pyridin-4-yl)amino)nicotinamide |
| 191 | 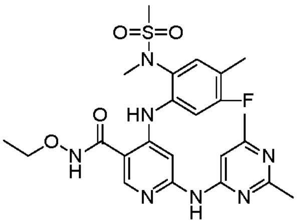 | 6-((2, 6-dimethyl pyrimidin-4-yl)amino)-N-ethoxy-4-((5-fluoro-4-methyl-2-(N-methyl methanesulfonamido)phenyl)amino)nicotinamide |
| 295* | 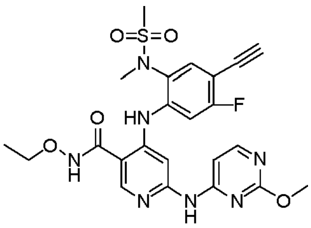 | N-ethoxy-4-((4-ethynyl-5-fluoro-2-(N-methyl methanesulfonamido) phenyl)amino)-6-((2-methoxy pyrimidin-4-yl)amino)nicotinamide |
| 205 | 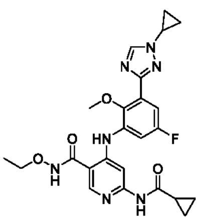 | 6-(cyclopropylcarboxamido)-4-((3-(1-cyclopropyl-1H-1, 2, 4-triazol-3-yl)-5-fluoro-2-methoxy phenyl)amino)-N-ethyl nicotinamide |
| 206* | 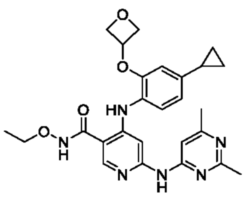 | 4-((4-cyclopropyl-2-(oxetan-3-yloxy)phenyl)amino)-6-(((2, 6-dimethyl pyrimidin-4-yl)amino)-N-ethoxy nicotinamide |
| 207 | 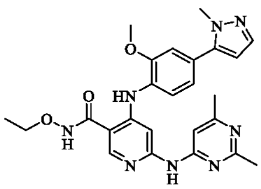 | 6-(((2, 6-dimethyl pyrimidin-4-yl)amino)-N-ethoxy-4-((2-methoxy-4-(1-methyl-1H-pyrazol-5-yl)phenyl)amino)nicotinamide |
| 208 | 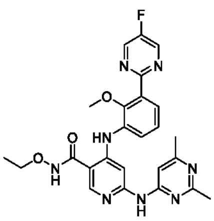 | 6-((2, 6-dimethyl pyrimidin-4-yl)amino)-N-ethoxy-4-((3-(5-fluoropyrimidin-2-yl)-2-methoxy phenyl)amino)nicotinamide |

(continued)

| Compound # | Structure | nomenclature |
|---|---|---|
| 209 | 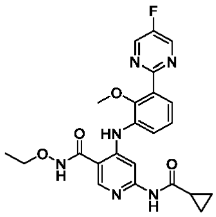 | 6-(cyclopropylcarboxamido)-N-ethoxy-4-((3-(5-fluoropyrimidin-2-yl)-2-methoxy phenyl)amino)nicotinamide |
| 210 | 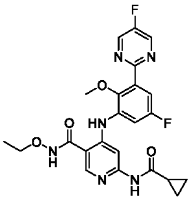 | 6-(cyclopropylcarboxamido)-N-ethoxy-4-((5-fluoro-3-(5-fluoropyrimidin-2-yl)-2-methoxy phenyl)amino)nicotinamide |
| 211 | 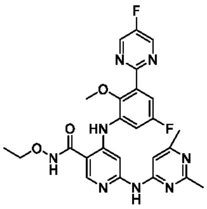 | 6-(((2, 6-dimethyl pyrimidin-4-yl)amino)-N-ethoxy-4-((5-fluoro-3-(5-fluoropyrimidin-2-yl)-2-methoxy phenyl)amino)nicotinamide |
| 212 | 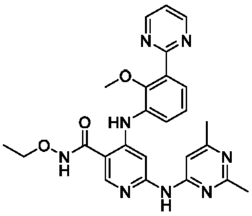 | 6-(((2, 6-dimethyl pyrimidin-4-yl)amino)-N-ethoxy-4-((2-methoxy-3-(pyrimidin-2-yl)phenyl)amino)nicotinamide |
| 213 | 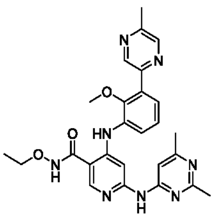 | 6-(((2, 6-dimethyl pyrimidin-4-yl)amino)-N-ethoxy-4-((2-methoxy-3-(5-methyl pyrazin-2-yl)phenyl)amino)nicotinamide |
| 214 | 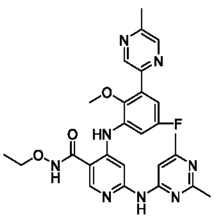 | 6-(((2, 6-dimethyl pyrimidin-4-yl)amino)-N-ethoxy-4-((5-fluoro-2-methoxy-3-(5-methyl pyrazin-2-yl)phenyl)amino)nicotinamide |
| 215 | 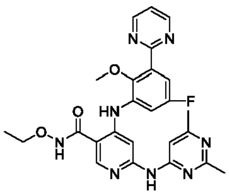 | 6-((2, 6-dimethyl pyrimidin-4-yl)amino)-N-ethoxy-4-((5-fluoro-2-methoxy-3-(pyrimidin-2-yl)phenyl)amino)nicotinamide |

(continued)

| Compound # | Structure | nomenclature |
|---|---|---|
| 216* | 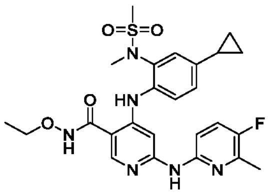 | 4-((4-cyclopropyl-2-(N-methyl methanesulfonamido)phenyl)amino)-N-ethoxy-6-((5-fluoro-6-methyl pyridin-2-yl)amino)nicotinamide |
| 217 | 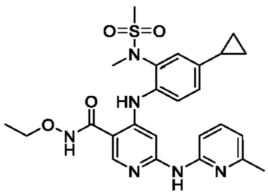 | 4-((4-cyclopropyl-2-(N-methyl methanesulfonamido)phenyl)amino)-N-ethoxy-6-((6-methyl pyridin-2-yl)amino)nicotinamide |
| 218 | 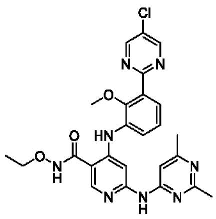 | 4-((3-(5-chloropyrimidin-2-yl)-2-methoxy phenyl)amino)-6-((2, 6-dimethyl pyrimidin-4-yl)amino)-N-ethoxy nicotinamide |
| 219 | 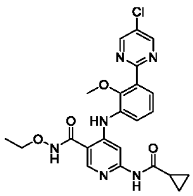 | 4-((3-(5-chloropyrimidin-2-yl)-2-methoxy phenyl)amino)-6-(cyclopropylcarboxamido)-N-ethoxy nicotinamide |
| 220 | 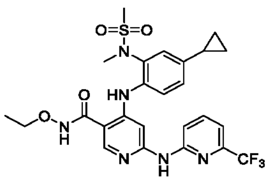 | 4-((4-cyclopropyl-2-(N-methyl methanesulfonamido)phenyl)amino)-N-ethoxy-6-((6-(trifluoromethyl)pyridin-2-yl)amino)nicotinamide |
| 221* | 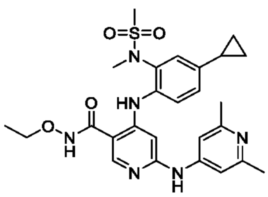 | 4-((4-cyclopropyl-2-(N-methyl methanesulfonamido)phenyl)amino)-6-(((2, 6-dimethyl pyridin-4-yl)amino)-N-ethoxy nicotinamide |
| 222 | 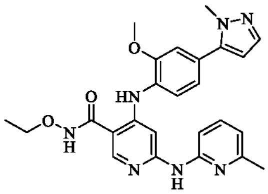 | N-ethoxy-4-((2-methoxy-4-(1-methyl-1H-pyrazol-5-yl)phenyl)amino)-6-((6-methyl pyridin-2-yl)amino)nicotinamide |

(continued)

| Compound # | Structure | nomenclature |
|---|---|---|
| 223 | 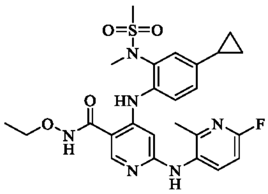 | 4-((4-cyclopropyl-2-(N-methyl methanesulfonamido)phenyl)amino)-N-ethoxy-6-((6-fluoro-2-methyl pyridin-3-yl)amino)nicotinamide |
| 224 | 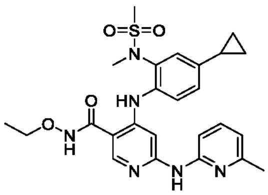 | 4-((4-cyclopropyl-2-(N-methyl methanesulfonamido)phenyl)amino)-N-ethoxy-6-((6-methyl pyridin-2-yl)amino)nicotinamide |
| 225 | 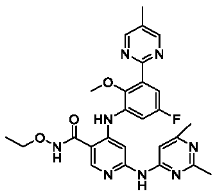 | 6-((2, 6-dimethyl pyrimidin-4-yl)amino)-N-ethoxy-4-((5-fluoro-2-methoxy-3-(5-methyl pyrimidin-2-yl)phenyl)amino)nicotinamide |
| 226 | 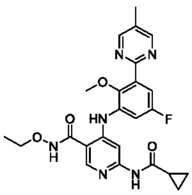 | 6-(cyclopropylcarboxamido)-N-ethoxy-4-((5-fluoro-2-(methylamino)-3-(5-methyl pyrimidin-2-yl)phenyl)amino)nicotinamide |
| 227* | 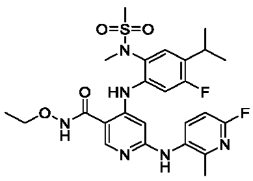 | N-ethoxy-6-((6-fluoro-2-methyl pyridin-3-yl)amino)-4-((5-fluoro-4-isopropyl-2-(N-methyl methanesulfonamido)phenyl)amino)nicotinamide |
| 228* | 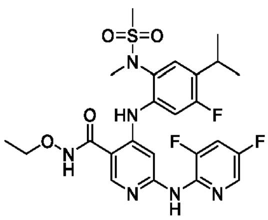 | 6-((3, 5-difluoropyridin-2-yl)amino)-N-ethoxy-4-((5-fluoro-4-isopropyl-2-(N-methyl methanesulfonamido)phenyl)amino) nicotinamide |
| 231* | 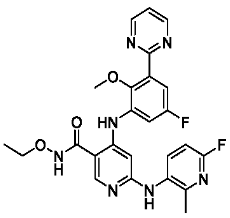 | N-ethyl-4-((5-fluoro-2-methoxy-3-(pyrimidin-2-yl)phenyl)amino)-6-((6-fluoro-2-methyl pyridin-3-yl)amino)nicotinamide |

(continued)

| Compound # | Structure | nomenclature |
|---|---|---|
| 232* | 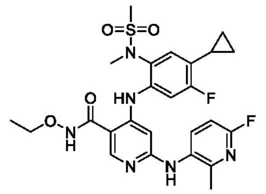 | 4-((4-cyclopropyl-5-fluoro-2-(N-methyl methanesulfonamido)phenyl)amino)-N-ethoxy-6-((6-fluoro-2-methyl pyridin-3-yl)amino)nicotinamide |
| 233* | 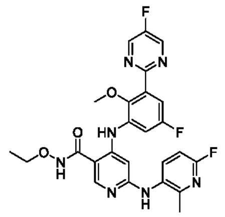 | N-ethoxy-6-((6-fluoro-2-methyl pyridin-3-yl)amino)-4-((5-fluoro-3-(5-fluoropyrimidin-2-yl)-2-methoxy phenyl)amino)nicotinamide |
| 234 | 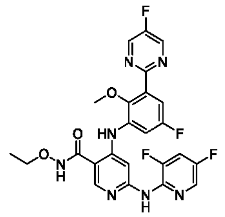 | 6-((3, 5-difluoropyridin-2-yl)amino)-N-ethoxy-4-((5-fluoro-3-(5-fluoropyrimidin-2-yl)-2-methoxy phenyl)amino)nicotinamide |
| 235 | 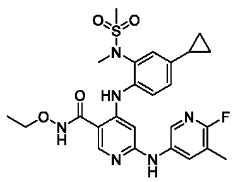 | 4-((4-cyclopropyl-2-(N-methyl methanesulfonamido)phenyl)amino)-N-ethoxy-6-((6-fluoro-5-methyl pyridin-3-yl)amino)nicotinamide |
| 236 | 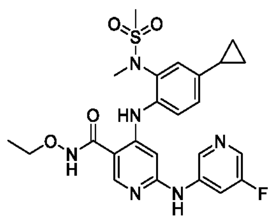 | 4-((4-cyclopropyl-2-(N-methyl methanesulfonamido)phenyl)amino)-N-ethoxy-6-((5-fluoropyridin-3-yl)amino)nicotinamide |
| 237 | 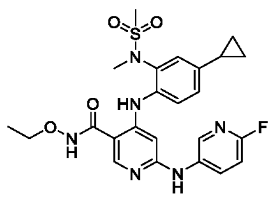 | 4-((4-cyclopropyl-2-(N-methyl methanesulfonamido)phenyl)amino)-N-ethoxy-6-((6-fluoropyridin-3-yl)amino)nicotinamide |
| 238 | 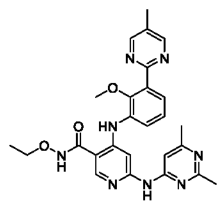 | 6-((2, 6-dimethyl pyrimidin-4-yl)amino)-N-ethoxy-4-((2-methoxy-3-(5-methyl pyrimidin-2-yl)phenyl)amino)nicotinamide |

(continued)

| Compound # | Structure | nomenclature |
|---|---|---|
| 239 | 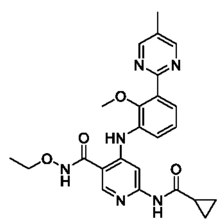 | 6-(cyclopropylcarboxamido)-N-ethoxy-4-((2-methoxy-3-(5-methyl pyrimidin-2-yl)phenyl)amino)nicotinamide |
| 240 | 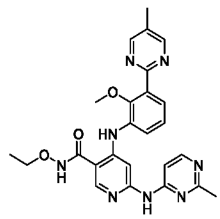 | N-ethoxy-4-((2-methoxy-3-(5-methyl pyrimidin-2-yl)phenyl)amino)-6-((2-methyl pyrimidin-4-yl)amino)nicotinamide |
| 241* | 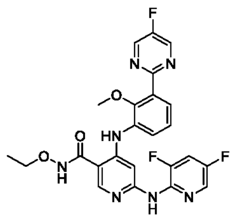 | 6-((3, 5-difluoropyridin-2-yl)amino)-N-ethoxy-4-((3-(5-fluoropyrimidin-2-yl)-2-methoxy phenyl)amino)nicotinamide |
| 242 | 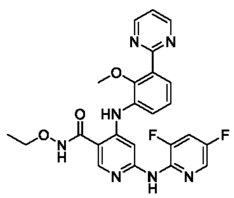 | 6-((3, 5-difluoropyridin-2-yl)amino)-N-ethoxy-4-((2-methoxy-3-(pyrimidin-2-yl)phenyl)amino)nicotinamide |
| 243 | 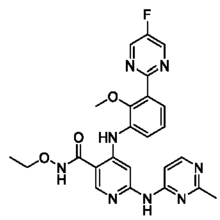 | N-ethoxy-4-((3-(5-fluoropyrimidin-2-yl)-2-methoxy phenyl)amino)-6-((2-methyl pyrimidin-4-yl)amino)nicotinamide |
| 244 | 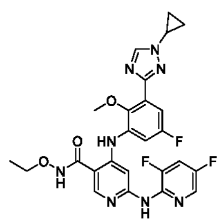 | 4-((3-(1-cyclopropyl-1H-1, 2, 4-triazol-3-yl)-5-fluoro-2-methoxy phenyl)amino)-6-((3, 5-difluoropyridin-2-yl)amino)-N-ethoxy nicotinamide |
| 245 | 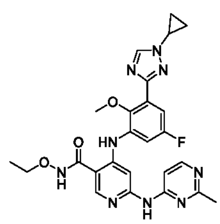 | 4-((3-(1-cyclopropyl-1H-1, 2, 4-triazol-3-yl)-5-fluoro-2-methoxy phenyl)amino)-N-ethoxy-6-((2-methyl pyrimidin-4-yl)amino)nicotinamide |

(continued)

| Compound # | Structure | nomenclature |
|---|---|---|
| 246* | 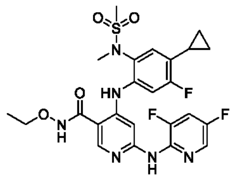 | 4-((4-cyclopropyl-5-fluoro-2-(N-methyl methanesulfonamido)phenyl)amino)-6-((3, 5-difluoropyridin-2-yl)amino)-N-ethoxy nicotinamide |
| 247* | 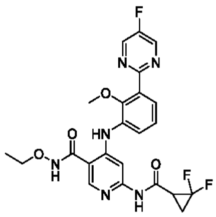 | 6-(2, 2-difluorocyclopropyl-1-carboxamido)-N-ethoxy-4-((3-(5-fluoropyrimidin-2-yl)-2-methoxy phenyl)amino)nicotinamide |
| 248 | 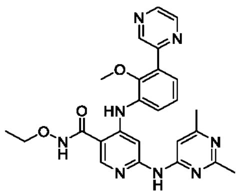 | 6-((2, 6-dimethyl pyrimidin-4-yl)amino)-N-ethoxy-4-((2-methoxy-3-(pyrazin-2-yl)phenyl)amino)nicotinamide |
| 249* | 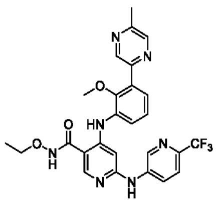 | N-ethoxy-4-((2-methoxy-3-(5-methyl pyrazin-2-yl)phenyl)amino)-6-((6-(trifluoromethyl)pyridin-3-yl)amino)nicotinamide |
| 250* | 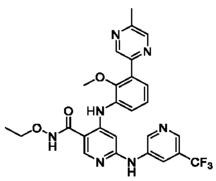 | N-ethoxy-4-((2-methoxy-3-(5-methyl pyrazin-2-yl)phenyl)amino)-6-((5-(trifluoromethyl)pyridin-3-yl)amino)nicotinamide |
| 251* | 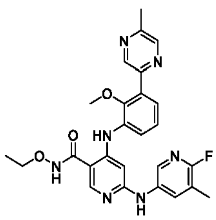 | N-ethoxy-6-((6-fluoro-5-methyl pyridin-3-yl)amino)-4-((2-methoxy-3-(5-methyl pyrazin-2-yl)phenyl)amino)nicotinamide |
| 252* | 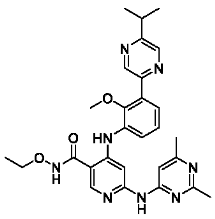 | 6-((2, 6-dimethyl pyrimidin-4-yl)amino)-N-ethoxy-4-((3-(5-isopropyl pyrazin-2-yl)-2-methoxy phenyl)amino)nicotinamide |

(continued)

| Compound # | Structure | nomenclature |
|---|---|---|
| 253* control | 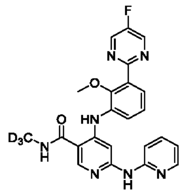 | 4-((3-(5-fluoropyrimidin-2-yl)-2-methoxy phenyl)amino)-N-(methyl-d3)-6-(pyridin-2-ylamino)nicotinamide |
| 254* | 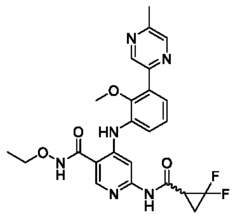 | 6-(2, 2-difluorocyclopropyl-1-carboxamido)-N-ethoxy-4-((2-methoxy-3-(5-methyl pyrazin-2-yl)phenyl)amino)nicotinamide |
| 255* | 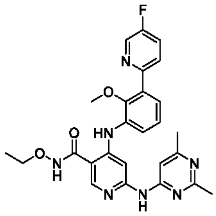 | 6-((2, 6-dimethyl pyrimidin-4-yl)amino)-N-ethoxy-4-((3-(5-fluoropyridin-2-yl)-2-methoxy phenyl)amino)nicotinamide |
| 256* | 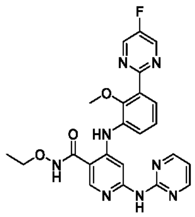 | N-ethoxy-4-((3-(5-fluoropyrimidin-2-yl)-2-methoxy phenyl)amino)-6-(pyrimidin-2-ylamino)nicotinamide |
| 257* | 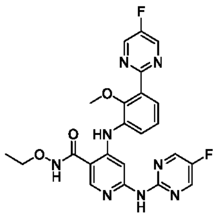 | N-ethoxy-4-((3-(5-fluoropyrimidin-2-yl)-2-methoxy phenyl)amino)-6-((5-fluoropyrimidin-2-yl)amino)nicotinamide |
| 258 | 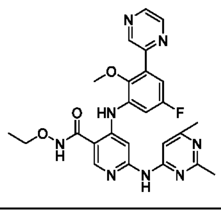 | 6-((2, 6-dimethyl pyrimidin-4-yl)amino)-N-ethoxy-4-((5-fluoro-2-methoxy-3-(pyrazin-2-yl)phenyl)amino)nicotinamide |
| 259* | 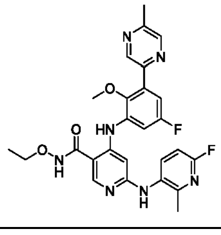 | N-methoxy-4-((5-fluoro-2-methoxy-3-(5-methyl pyrazin-2-yl)phenyl)amino)-6-((6-fluoro-2-methyl pyridin-3-yl)amino)nicotinamide |

(continued)

| Compound # | Structure | nomenclature |
|---|---|---|
| 260* | 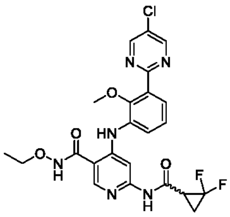 | 4-((3-(5-chloropyrimidin-2-yl)-2-methoxy phenyl)amino)-6-(2, 2-difluorocyclopropyl-1-carboxamido)-N-ethoxy nicotinamide |
| 261* | 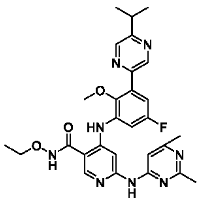 | 6-((2, 6-dimethyl pyrimidin-4-yl)amino)-N-ethoxy-4-((5-fluoro-3-(5-isopropyl pyrazin-2-yl)-2-methoxy phenyl)amino)nicotinamide |
| 262* | 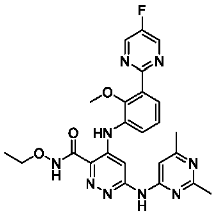 | 6-((2, 6-dimethyl pyrimidin-4-yl)amino)-N-ethoxy-4-((3-(5-fluoropyrimidin-2-yl)-2-methoxy phenyl)amino)pyridazine-3-carboxamide |
| 263* | 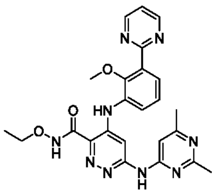 | 6-((2, 6-dimethyl pyrimidin-4-yl)amino)-N-ethoxy-4-((2-methoxy-3-(pyrimidin-2-yl)phenyl)amino)pyridazine-3-carboxamide |
| 264* | 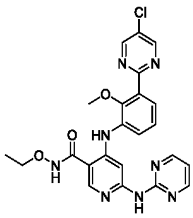 | 4-((3-(5-chloropyrimidin-2-yl)-2-methoxy phenyl)amino)-N-ethoxy-6-(pyrimidin-2-ylamino)nicotinamide |
| 265* | 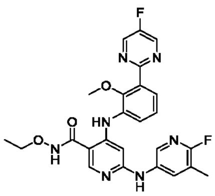 | N-ethoxy-6-((6-fluoro-5-methyl pyridin-3-yl)amino)-4-((3-(5-fluoropyrimidin-2-yl)-2-methoxy phenyl)amino)nicotinamide |
| 266* | 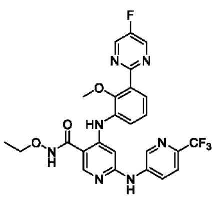 | N-ethoxy-4-((3-(5-fluoropyrimidin-2-yl)-2-methoxy phenyl)amino)-6-((6-(trifluoromethyl)pyridin-3-yl)amino)nicotinamide |

(continued)

| Compound # | Structure | nomenclature |
|---|---|---|
| 267* | 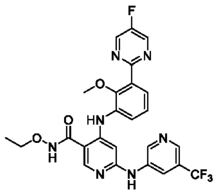 | N-ethoxy-4-((3-(5-fluoropyrimidin-2-yl)-2-methoxy phenyl)amino)-6-((5-(trifluoromethyl)pyridin-3-yl)amino)nicotinamide |
| 268 | 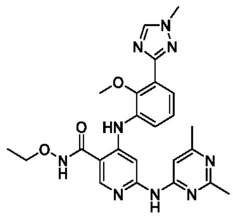 | 6-((2, 6-dimethyl pyrimidin-4-yl)amino)-N-ethoxy-4-((2-methoxy-3-(1-methyl-1H-1, 2, 4-triazol-3-yl)phenyl)amino)nicotinamide |
| 269* | 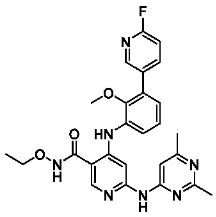 | 6-((2, 6-dimethyl pyrimidin-4-yl)amino)-N-ethoxy-4-((3-(6-fluoropyridin-3-yl)-2-methoxy phenyl)amino)nicotinamide |
| 270* | 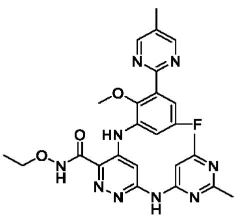 | 6-((2, 6-dimethyl pyrimidin-4-yl)amino)-N-ethoxy-4-((5-fluoro-2-methoxy-3-(5-methyl pyrimidin-2-yl)phenyl)amino)pyridazine-3-carboxamide |
| 271* | 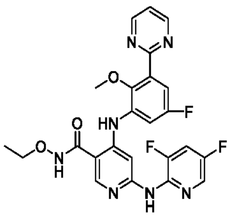 | 6-((3, 5-difluoropyridin-2-yl)amino)-N-ethoxy-4-((5-fluoro-2-methoxy-3-(pyrimidin-2-yl)phenyl)amino)nicotinamide |
| 272* | 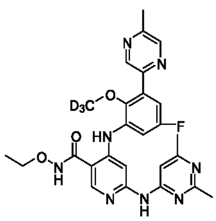 | 6-((2, 6-dimethyl pyrimidin-4-yl)amino)-N-ethoxy-4-((5-fluoro-2-(methoxy-d3)-3-(5-methyl pyrazin-2-yl)phenyl)amino)nicotinamide |
| 273 | 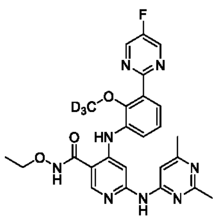 | 6-((2, 6-dimethyl pyrimidin-4-yl)amino)-N-ethoxy-4-((3-(5-fluoropyrimidin-2-yl)-2-(methoxy-d3)phenyl)amino)nicotinamide |

(continued)

| Compound # | Structure | nomenclature |
|---|---|---|
| 274* | 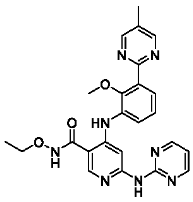 | N-ethoxy-4-((2-methoxy-3-(5-methyl pyrimidin-2-yl)phenyl)amino)-6-(pyrimidin-2-ylamino)nicotinamide |
| 275* | 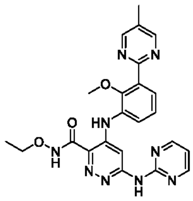 | N-ethoxy-4-((2-methoxy-3-(5-methyl pyrimidin-2-yl)phenyl)amino)-6-(pyrimidin-2-ylamino)pyridazine-3-carboxamide |
| 276* | 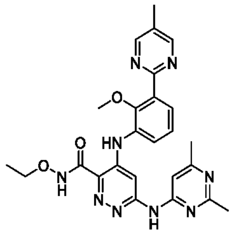 | 6-((2, 6-dimethyl pyrimidin-4-yl)amino)-N-ethoxy-4-((2-methoxy-3-(5-methyl pyrimidin-2-yl)phenyl)amino)pyridazine-3-carboxamide |
| 277 | 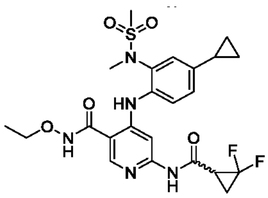 | 4-((4-cyclopropyl-2-(N-methyl methanesulfonamido)phenyl)amino)-6-(2, 2-difluorocyclopropyl-1-carboxamido)-N-ethoxy nicotinamide |
| 278* | 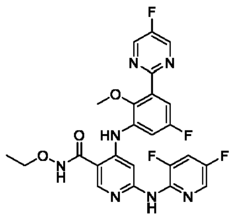 | 6-[(3, 5-difluoropyridin-2-yl)amino]-N-ethoxy-4-((5-fluoro-3-(5-fluoropyrimidin-2-yl)-2-methoxy phenyl)amino)pyridine-3-carboxamide |
| 279 | 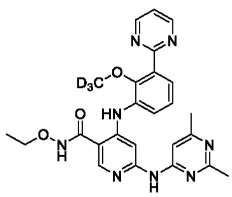 | 6-((2, 6-dimethyl pyrimidin-4-yl)amino)-N-ethoxy-4-((2-(methoxy-d3)-3-(pyrimidin-2-yl)phenyl)amino)nicotinamide |
| 280* | 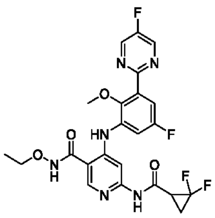 | 6-(2, 2-difluorocyclopropyl-1-carboxamido)-N-ethoxy-4-((5-fluoro-3-(5-fluoropyrimidin-2-yl)-2-methoxy phenyl)amino)nicotinamide |

(continued)

| Compound # | Structure | nomenclature |
|---|---|---|
| 281* | 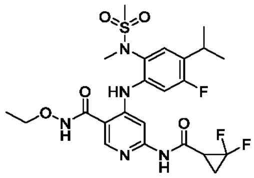 | 6-(2, 2-difluorocyclopropyl-1-carboxamido)-N-ethoxy-4-((5-fluoro-4-isopropyl-2-(N-methyl methanesulfonamido)phenyl)amino)nicotinamide |
| 282* | 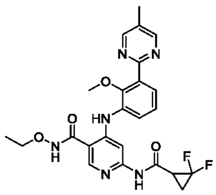 | 6-(2, 2-difluorocyclopropyl-1-carboxamido)-N-ethoxy-4-((2-methoxy-3-(5-methyl pyrimidin-2-yl)phenyl)amino)nicotinamide |
| 283* | 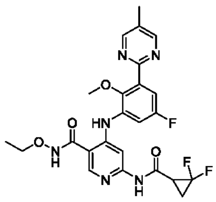 | 6-(2, 2-difluorocyclopropyl-1-carboxamido)-N-ethoxy-4-((5-fluoro-2-methoxy-3-(5-methyl pyrimidin-2-yl)phenyl)amino)nicotinamide |
| 284* | 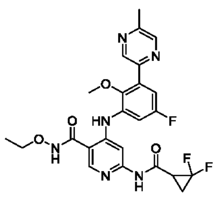 | 6-(2, 2-difluorocyclopropyl-1-carboxamido)-N-ethoxy-4-((5-fluoro-2-methoxy-3-(5-methyl pyrazin-2-yl)phenyl)amino)nicotinamide |
| 285* | 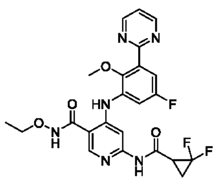 | 6-(2, 2-difluorocyclopropyl-1-carboxamido)-N-ethoxy-4-((5-fluoro-2-methoxy-3-(pyrimidin-2-yl)phenyl)amino)nicotinamide |
| 286* | 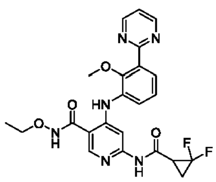 | 6-(2, 2-difluorocyclopropyl-1-carboxamido)-N-ethoxy-4-((2-methoxy-3-(pyrimidin-2-yl)phenyl)amino)nicotinamide |
| NB: An asterisk (*) indicates that the compound is not part of the present invention | | |

The invention is further described by claims 14 and 15.

As used herein, the term "pharmaceutically acceptable salt of compound represented by Formula (I) or Formula (I') or Formula (I") is exemplified by those organic acid addition salt formed from organic acids that form pharmaceutically acceptable anions.

The pharmaceutically acceptable salt can be obtained by using standard procedures well known in the art, for example, by reacting sufficient amounts of basic compound with suitable acid providing pharmaceutically acceptable anion.

**Preparation method:**

Herein also disclosed is a method for preparing the compound of the present invention. The preparation of

compound represented by Formula (I) or Formula (I') or Formula (I") of the present invention can be accomplished by following exemplary methods and Examples, which, however, should not be recognized in any way as a limitation to the scope of the present invention. The compound of the present invention can also be synthesized through those synthesis technologies known to those skilled in the art, or through those synthesis technologies known to those skilled in the art in combination with the preparation method described in the present invention. Conventional separation technologies known in the art can be adapted to obtain the products of each step of reaction, including but not limited to extraction, filtration, distillation, crystallization, chromatographic separation, etc. The starting materials and chemical reagents needed for the reactions can be synthesized according to the conventional synthesis process described in the literature (such as, Scifinder), or are commercially available.

Compound represented by Formula (I) or Formula (I') or Formula (I") of the present invention can be synthesized according to a scheme described in the following preparation method: 1) reacting a starting material A1 with hydroxylamine having different substituents in oxygen atom (i.e.,

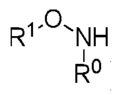

) via condensation reaction, to result in A2; 2) reacting A2 with (hetero)aryl amines containing functional groups (i.e. $R^2$-$NH_2$) via substitution reaction in the presence of a base, to result in A3; 3) reacting A3 with amides or aromatic amines having different functional groups (i.e. $R^3$-$NH_2$ ) via Buchwald coupling reaction, to result in the target compound A4: wherein X Y $R^0$, $R^1$, $R^2$ and $R^3$ are as defined above;

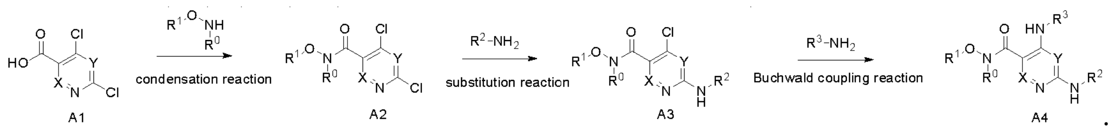


The present invention further provides a pharmaceutical composition, comprising the compound or a pharmaceutically acceptable salt thereof as described above, and optionally comprising, a pharmaceutical acceptable carrier and/or adjuvant and/or diluent.

In some embodiments, the pharmaceutical composition may further comprise other drugs for treating and/or preventing a related disease mediated by TYK2.

Method for preparing a pharmaceutical composition comprising a certain amount of active ingredient is known in the art, or obvious to those skilled in the art in light of the disclosure of the present invention. As described in such as REMINGTON'S PHARMACEUTICAL SCIENCES, Martin, E.W., ed., Mack Publishing Company, 19th ed.(1995), method for preparing the pharmaceutical composition comprises the incorporation of suitable pharmaceutical excipient(s), carrier(s), diluent(s), etc.

The present invention further provides a pharmaceutical formulation comprising the compound or a pharmaceutically acceptable salt thereof as described above, along with a pharmaceutical acceptable carrier and/or adjuvant and/or diluent.

In another aspect, the present invention further provides use of the compound or pharmaceutically acceptable salt thereof or the pharmaceutical composition or the pharmaceutical formulation as described above in the preparation of a medicament for treating and/or preventing a related disease mediated by TYK2.

In another aspect, the present invention further provides a compound or a pharmaceutically acceptable salt thereof or the pharmaceutical composition or the pharmaceutical formulation as described above, for use in treating and/or preventing a related disease mediated by TYK2.

In another aspect, the present invention further provides a method for treating and/or preventing a related disease mediated by TYK2, comprising administering a therapeutically and/or preventively effective amount of the compound or pharmaceutically acceptable salt thereof or the pharmaceutical composition or the pharmaceutical formulation as decribed above to a subject in need thereof.

In some embodiments, the disease includes inflammatory disease, autoimmune disease and cancer.

In the present invention, "treat", "treating", or "treatment" generally refers to obtaining the desired pharmacological and/or physiological effects, which may be preventive in sense of completely or partially preventing the disease or its symptoms, or may be therapeutic in sense of partially or completely stabilizing or curing of the disease and/or the adverse effects caused by the disease. The term "treat", "treating", or "treatment" used herein encompasses any treatment of the disease in the patient, including: (a) preventing the occurrence of disease or its symptoms in patient who is susceptible to the disease but has not been diagnosed; (b) arresting symptoms of the disease, i.e., stopping its progress; or (c) alleviating symptoms of the disease, i.e., degenerating the disease or its symptom.

In the present invention, "subject" refers to vertebrates. In some embodiments, vertebrates refer to mammals. Mammals include, but are not limited to, livestock (such as, cattle), pets (such as, cats, dogs, and horses), primates, mice and rats. In some embodiments, mammals refer to humans.

In the present invention, the expression "effective amount" refers to the amount that can effectively achieve the desired therapeutic or preventive effect in terms of both dose and time. The expression "therapeutically effective amount" of the substance/molecule of the present invention may vary according to factors such as the disease status, age, sex and weight of the individual, and the ability of the substance/molecule to trigger the desired response in the individual. The therapeutically effective amount also encompasses the amount that the therapeutically beneficial effect of the substance/molecule outweighs any toxic or harmful consequences. The expression "preventively effective amount" refers to the amount that can effectively achieve the desired preventive effect in terms of both dose and time. Usually, but not necessarily, since the preventive dose is used for the subjects before the onset of the disease or at the early stage of the disease, preventively effective amount will be lower than the therapeutically effective amount. In the case of cancer, the therapeutically effective amount of the drug can lead to the outcomes such as reducing the number of cancer cells; reducing tumor volume; inhibiting (i.e. slowing down to some extent, preferably arresting) the infiltration of cancer cells into the surrounding organs; inhibiting (i.e. slowing down to some extent, preferably arresting) tumor metastasis; inhibiting tumor growth to some extent; and/or alleviating one or more symptoms related to cancer to some extent.

Definitions of terms:

According to the conventional practice in the field, 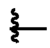 used in the structural formula herein is used to indicate the bond by which the moiety or substituent is connected to the parent or main structure.

The symbol "-" (dash) is used to indicate the connection point of the substitution, except that appears between two letters or symbols. For example, $-CONH_2$ is connected by the carbon atom.

In various parts of the present description, the substituents of the compounds disclosed in the present invention are described in accordance to the type or scope of the group. It should be noted that, the present invention encompasses each and every independent sub-group of individual members within the type and scope of these groups. For example, the term "$C_{1-6}$ alkyl" particularly encompasses independent disclosure of groups such as methyl, ethyl, $C_3$ alkyl, $C_4$ alkyl, $C_5$ alkyl and $C_6$ alkyl, or particularly encompasses independent disclosure of sub-groups such as "$C_{1-4}$ alkyl" and "$C_{1-3}$ alkyl".

As used herein, the term "alkyl" refers to branched and linear saturated aliphatic hydrocarbyl having specified number of carbon atoms. For example, "$C_{1-6}$ alkyl" refers to $C_1$, $C_2$, $C_3$, $C_4$, $C_5$ and $C_6$. In addition, for example, the expression "$C_{1-6}$ alkyl" refers to alkyls having from one to six carbon atoms. Alkyl may be unsubstituted or substituted by replacing one or more of its hydrogen atoms with another chemical group. Example of alkyl includes, but is not limited to, methyl, ethyl, propyl (such as, n-propyl and isopropyl), butyl ( such as, n-butyl, isobutyl, t-butyl), pentyl (such as, n-pentyl, isopentyl, neopentyl), etc.

As used herein, the term "alkoxy" refers to any above described alkyl (such as, $C_{1-6}$ alkyl, $C_1$-$_4$ alkyl, $C_{1-3}$ alkyl, and the like) which is connected to rest of the molecule through the oxygen atom (-O-).

As used herein, the term "halo $C_{1-6}$ alkyl" or "halo $C_{1-6}$ alkoxy" refers to an alkyl or alkoxy wherein one or more (such as, two, or three) hydrogen atom is replaced by halogen atom, such as, fluoro, chloro, bromo, wherein the alkyl and alkoxy are respectively as defined above. In some embodiments, the halogen atom in the term "halo $C_{1-6}$ alkyl" is preferably fluoro, such as the term "halo $C_{1-6}$ alkyl" may be $-CF_3$, - $CHF_2$, $-CH_2F$, $-CH_2CH_2F$, $-CH_2CHF_2$, $-CH_2CF_3$, etc. In some embodiments, the halogen atom in the term "halo $C_{1-6}$ alkoxy" is preferably fluoro, such as the term "halo $C_{1-6}$ alkoxy" may be $-OCF_3$, $-OCHF_2$, $-OCH_2F$, $-OCH_2CH_2F$, $-OCH_2CHF_2$, $-OCH_2CF_3$, etc.

As used herein, the term "$C_{1-6}$ alkyl substituted by hydroxyl group" refers to an alkyl wherein one hydrogen atom is replaced by hydroxyl, wherein the alkyl is as defined above. For example, the term "$C_{1-6}$ alkyl substituted by hydroxyl group" may be hydroxymethyl.

As used herein, the term "alkenyl" means to include straight- or branched hydrocarbon chain having one or more carbon-carbon double bond positioned at any stable point along the chain. For example, "$C_{2-6}$ alkenyl" means to include $C_2$, $C_3$, $C_4$, $C_5$ and $C_6$. Example of alkenyl includes, but is not limited to, ethenyl, 1-propenyl, 2-propenyl, 2-butenyl, 3-butenyl, 2-pentenyl, 3-pentenyl, 4-pentenyl, 2-hexenyl, 3-hexenyl, 4-hexenyl, 5-hexenyl, 2-methyl-2-propenyl, 4-methyl-3-pentenyl, etc.

As used herein, the term "alkynyl" means to include straight- or branched hydrocarbon chain having one or more carbon-carbon triple bond positioned at any stable point along the chain. For example, "$C_{2-6}$ alkynyl" means to include $C_2$, $C_3$, $C_4$, $C_5$ and $C_6$ alkynyl. Example of alkynyl includes, but is not limited to, ethynyl, propynyl, butynyl, pentynyl, hexynyl, etc.

It can be understood by those skilled in the art that, when term "$CO_2$" is used herein, it refers to the group "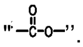".

As used herein, the expression "substituted by" means the replacement of one or more hydrogen on particular

atom or group with specified substituent group, provided that the normal valence of the particular atom or group is not exceeded.

As used herein, the term "cycloalkyl" refers to cyclic alkyl, including monocyclic, bicyclic or multicyclic system. $C_{3-7}$ cycloalkyl means to include $C_3$, $C_4$, $C_5$, $C_6$ and $C_7$ cycloalkyl. Example of cycloalkyl includes, but is not limited to, cyclopropyl, cyclobutyl, cyclopentyl, cyclohexyl, etc. As used herein, the term "carbocyclyl" or "carbocyclyl" residue refers to any stable 3-membered, 4-membered, 5-membered, 6-membered or 7-membered monocyclic or bicyclic ring, or 7-membered, 8-membered, 9-membered, 10-membered, 11-membered or 12-membered bicyclic or tricyclic ring, wherein any ring may be a saturated, partially unsaturated, unsaturated or aromatic ring. Example of such carbocyclyl includes, but is not limited to, cyclopropyl, cyclobutyl, cyclobutenyl, cyclopentyl, cyclopentenyl, cyclohexyl, cyclohexenyl, cycloheptenyl, cycloheptyl, adamantyl, cyclooctyl, phenyl, naphthyl, etc. According to the above description, the definition of carbocycle also encompasses bridged rings, such as [2,2,2] dicyclooctane. Unless stated otherwise, preferred carbocyclyl is cyclopropyl, cyclobutyl, cyclopentyl, or phenyl. Bridged ring may be formed when one or more carbon atoms are connected to two other non-adjacent carbon atoms. The preferred bridge has one or two carbon atoms. It should be noted that the bridge always converts monocyclic ring into bicyclic ring. When a ring is further bridged, the substituent for the ring may also exist on the bridge.

As used herein, the term "aryl" refers to monocyclic or bicyclic aromatic hydrocarbyl having from 6 to 12 carbon atoms in the ring, such as phenyl and naphthyl, each of which can be substituted.

As used herein, the term "heterocyclyl", "heterocycloalkyl" and "heterocyclylic" are interchangeable and refers respectively to substituted and unsubstituted 3-7 membered monocyclic group, 7-11 membered bicyclic group, and 10-15-membered tricyclic group, wherein at least one ring has at least one heteroatom (O, S or N), the ring containing heteroatom preferably has one, two, or three heteroatoms selected from the group consisting of O, S and N. Each ring containing heteroatom of the group may have one or two oxygen or sulfur atoms and/or from one to four nitrogen atoms, provided that each ring contains 4 or less heteroatoms in total, and further provided that the ring has at least one carbon atom. Nitrogen and sulfur atom in the ring may be optionally oxidized, and nitrogen atom may optionally be quaternized. The ring fused to accomplish bicyclic and tricyclic ring may contain merely carbon atom and may be saturated, partially saturated or completely unsaturated. Heterocyclic group may be connected through any available nitrogen or carbon atom. The terms "heterocyclyl", "heterocycloalkyl" and "heterocyclic" used herein all encompass "heteroaryl" as defined as follows.

Exemplary monocyclic heterocycly includes, in addition to the heteroaryl described below, azetidinyl, oxetanyl, pyrrolidinyl, imidazolinyl, oxazolidinyl, isoxazolinyl, thiazolidinyl, tetrahydrofuryl, piperidinyl, piperazinyl, 2-oxopiperazinyl, 2-oxopiperidinyl, 2-oxopyrrolidinyl, 2-oxoazepinyl, 1-pyridinonyl, 4-piperidonyl, tetrahydropyranyl, morpholinyl, 1, 3-dioxalanyl, etc. Exemplary bicyclic heterocycly includes quinuclidinyl. Further monocyclic heterocyclyl includes:

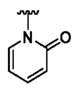
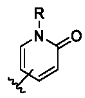
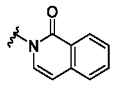
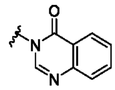

As used herein, the term "saturated heterocyclyl" refers to the monocyclic, bicyclic, or tricyclic group as described above is completely saturated. The term "heterocyclyl" is as defined above. For example, saturated heterocyclyl may be morpholinyl (such as,

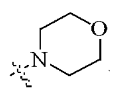

), piperidinyl (such as,

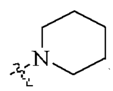

), piperazinyl(

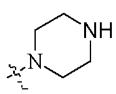

,

etc.

As used herein, the term "heteroaryl" refers to, substituted or unsubstituted, aromatic 5-membered or 6-membered monocyclic group, 9-membered or 10-membered bicyclic group, or 11-14-membered tricyclic group, containing at least one heteroatom (O, N and S) in at least one ring, wherein the heteroatom-containing ring preferably may contain one or two or three heteroatoms selected from the group consisting of O, N and S. Each heteroatom-containing ring of heteroaryl may contain 1 or 2 oxygen or sulfur atoms, and/or from 1 to 4 nitrogen atoms, provided that the total number of heteroatoms in each ring is 4 or less and each ring contains at least one carbon atom. The ring fused to accomplish bicyclic and tricyclic ring may contain merely carbon atom and may be saturated, partially saturated or completely unsaturated. Nitrogen and sulfur atoms may optionally be oxidized, and nitrogen atom may be quaternized. Bicyclic or tricyclic heteroaryl must include at least one complete aromatic ring, but the other one or more fused rings may be aromatic or non-aromatic. Heteroaryl may be connected through any available nitrogen or carbon atom of any ring. The other ring(s), when selected from cycloalkyl or heterocyclyl, may be optionally substituted by =O (oxo), provided that the valence permits.

Exemplary monocyclic heteroaryl comprises pyrrolyl, pyrazolyl, pyrazolinyl, imidazolyl, oxazolyl, isoxazolyl, thiazolyl, thiadiazolyl, isothiazolyl, furyl, thienyl, oxadiazolyl, pyridinyl, pyrazinyl, pyrimidinyl, pyridazinyl, triazinyl, triazolyl, pyridinonyl, 2-pyridinonyl (such as,

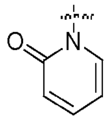

), etc. It is noted that, in the present invention, pyridinone has the structure of

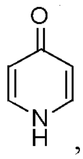
,

and 2-pyridinone has the structure of

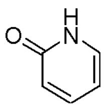
.

Exemplary bicyclic heteroaryl comprises indolyl, benzothiazolyl, benzimidazolyl, benzo-1,3-dioxolyl, benzoxazolyl, benzothienyl, quinolinyl, tetrahydroisoquinolinyl, isoquinolinyl, benzofuryl, indolizinyl, benzopyranyl, chromonyl, coumarinyl, benzopyranyl, quinoxalinyl, indazolyl, pyrrolopyrimidinyl, furanopyridinyl, dihydroisoindolyl, tetrahydroquinolinyl, etc.

Exemplary tricyclic heteroaryl comprises carbazolyl, benzoindolyl, phenanthrolinyl, acridinyl, etc.

In compounds represented by formula (I) or formula (I') or formula (I"), preferred heteroaryl comprises, such as:

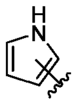 , 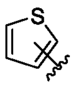 , 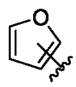 , 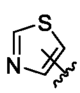 , 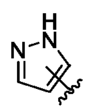 , 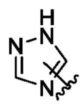 ,

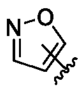 , 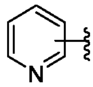 , 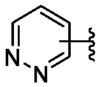 , 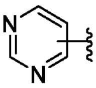 , 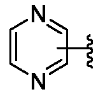 , 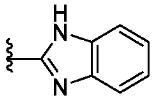 ,

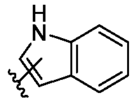 , 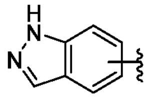 ,

and may optionally be substituted on any available carbon or nitrogen atom.

Unless otherwise specified, when reference is made to the definitely named aryl (such as, phenyl), cycloalkyl (such as, cyclohexyl), heterocyclyl (such as, pyrrolidinyl, piperidyl, morpholinyl) or heteroaryl (such as, imidazolyl, pyrazolyl, triazolyl), such reference refers to the corresponding ring containing 0-3, preferably 0-2, substituents, wherein the substituent may be, as appropriate, selected from the substituents which are described above for aryl, cycloalkyl, heterocyclyl and/or heteroaryl .

As used herein, the term "carbocyclyl" or "carbocyclic" refers to saturated or unsaturated monocyclic or bicyclic ring, wherein all atoms of all rings are carbon atoms. That is, such terms comprise cycloalkyl and aryl ring. Monocyclic carbocyclyl generally contains from three to six carbon atoms, more generally 5 or 6 carbon atoms. Bicyclic carbocyclyl contains from seven to twelve carbon atoms arranged as, e.g., [4, 5], [5, 5], or [6, 6] bicyclic system, or contains 9 or 10 carbon atoms arranged as [5, 6] or [6, 6] bicyclic system. Example of monocyclic and bicyclic carbocyclyl comprises cyclopropyl, cyclobutyl, cyclopentyl, 1-cyclopent-1-enyl, 1-cyclopent-2-enyl, 1-cyclopent-3-enyl, cyclohexyl, 1-cyclohex-1-enyl, 1-cyclohex-2-enyl, 1-cyclohex-3-enyl, phenyl and naphthyl. Carbocyclyl may be substituted, in which circumstance, the substituent may be selected from the substituents which are described above for cycloalkyl and aryl.

As used herein, the term "heteroatom" comprises oxygen atom, sulfur atom, and nitrogen atom.

When a ring or group is preceded by term "unsaturated", as used herein, the ring or group may be completely unsaturated or partially unsaturated.

From all above descriptions, it is obvious to those skilled in the art that, any group named by compounded name, such as "$C_{3-10}$ cycloalkyl-C(O)-", should be interpreted as being constructed by the constituents from which the group is derived, i.e., constructed from carbonyl substituted by $C_{3-10}$ cycloalkyl, wherein cycloalkyl is as above defined. Any other compounded name should be similarly interpreted accordingly.

As used herein, the term "optionally" means the circumstance that follows may be present or not. For example, "$C_{1-6}$ alkyl optionally substituted by from one to three $R^d$ groups", means that, the $C_{1-6}$ alkyl may be substituted by from one to three $R^d$ groups, or not. Any other circumstance should be similarly interpreted accordingly.

Throughout the description, any group and its substituent may be selected by those skilled in the art to provide stable part and compound, as well as compounds useful as pharmaceutically acceptable compound, and/or intermediate useful for preparing pharmaceutically acceptable compounds.

**Effect of the invention:**

The hydroxamate compound represented by formula (I), formula (I') and formula (I") of the present invention exhibits good TYK2 inhibition effect and can be used as drug for the treatment and/or prevention of diseases related to such effect.

**Detailed description of embodiments**

It should be understood that the terms used herein are intended to describe the specific embodiments and are not intended to limit. In addition, although any method, device and material similar to or equivalent to those described herein can be used to implement or test the present invention, the preferred method, device and material are now described.

The structure of the compound is determined by nuclear magnetic resonance (NMR) or mass spectrometry (MS). NMR is measured with Bruker ASCENA-400 nuclear magnetic instrument. The measured solvents used include deuterated dimethyl sulfoxide (DMSO-$d_6$), deuterated chloroform ($CDCl_3$), and deuterated methanol ($CD_3OD$). The internal standard is tetramethylsilane (TMS), and the chemical shift is given in $10^{-6}$ (ppm).

Thermofisher ESQ (ESI) mass spectrometer was used for reaction monitoring and MS determination.

HPLC was determined by using the Thermo U3000 DAD high pressure liquid chromatograph (GL Sciences ODS-HL HP 3μm 3.0*100mm column).

Thin layer chromatography was performed on Qingdao Ocean GF254 silica gel plate, and the silica gel plate used for thin layer chromatography (TLC) are of 0.15-0.2mm, and the high-performance thin layer chromatography preparation plate used for thin layer chromatography separation and purification products are of 0.9-1.0mm. The column chromatography was performed with Qingdao Ocean 200~300 mesh silica gel as the carrier, and the elution system includes A: dichloromethane and methanol system; and B: petroleum ether and ethyl acetate system, the ratio of solvent in volume is adjusted according to the polarity of the compound. The medium pressure preparation liquid phase is purified by using biotage isera one preparation liquid phase.

In the following Examples, unless otherwise specified, all reaction raw materials are available from manufacturers such as Saen Chemical Technology (Shanghai) Co., Ltd., Shanghai Shaoyuan Reagent Co., Ltd., Nanjing Yaoshi Technology Co., Ltd., Jiangsu Aikang Biomedical Research and Development Co., Ltd., and Shanghai Bide Pharmaceutical Technology Co., Ltd.

**Intermediate int-1**

4,6-dichloro-N-methoxy nicotinamide

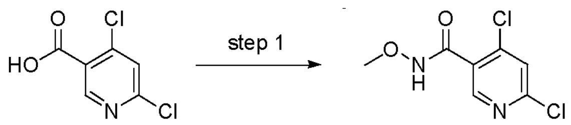


**Int-1a** **Int-1**

1) 4,6-dichloronicotinic acid **(int-1a,** 1.92g, 10mmol) was dissolved in dichloromethane (30ml) in a 100ml two-necked flask. The mixture was cooled to 0°C~5°C with ice-water bath, added with catalytic amount of DMF(0.1ml) dropwise followed by carefully adding oxalyl chloride (1.52g, 12mmol) dropwise, and then stirred at room temperature for 30 min. Upon indication of completed reaction by TLC, the reaction mixture was concentrated at 40°C under reduced pressure, the resulting crude material was added to dichloromethane (20ml), concentrated under reduced pressure, and used directly in next reaction.

2) The methoxy amine in form of hydrochloride salt (1.25g, 15mmol) was added to ethyl acetate/water mixture (40ml) (5:1), added with potassium carbonate (4.14g, 30mmol), and stirred at room temperature for 10 min. The reaction mixture was added with the previously obtained crude material, which was dissolved in dichloromethane (10ml) and carefully added dropwise. After the addition, the mixture was stirred at room temperature overnight. After phase separation, the organic phase was concentrated and then purified by column chromatography (petroleum ether/ethyl acetate=2:1) to provide the desired product 4,6-dichloro-N-methoxy nicotinamide **(int-1,** 2.0g, 9 mmol, 90.5% yield for 2 steps). MS Calcd: 221; MS Found: 222 ($[M+H]^+$).

Intermediate **int-2**

4,6-dichloro-N-ethoxy nicotinamide

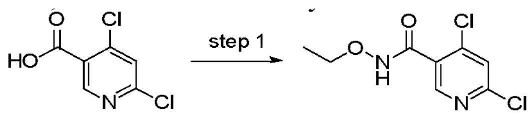


**int-1a** **int-2**

Step 1:1) 4,6-dichloronicotinic acid (int-1a, 1.92g, 10mmol) was dissolved in dichloromethane (30ml) in a 100ml two-necked flask. The mixture was cooled to 0°C~5°C with ice-water bath, added with catalytic amount of DMF(0.1ml) dropwise followed by carefully adding oxalyl chloride (1.52g, 12mmol) dropwise, and stirred at room temperature for 30 min. Upon indication of completed reaction by TLC, the reaction mixture was concentrated at 40°C under reduced pressure. The resulting crude product was added to dichloromethane (20ml) and further concentrated under reduced pressure.

2) The ethoxy amine in form of hydrochloride salt (1.426g, 15mmol) was added to ethyl acetate/water mixture (40ml) (5:1), added with potassium carbonate (4.14g, 30mmol), and stirred at room temperature for 10 min. The reaction mixture was added with the previously obtained crude material, which was dissolved in dichloromethane (10ml) and carefully added dropwise. After the addition, the mixture was stirred at room temperature overnight. After phase separation, the organic phase was concentrated and purified by column chromatography (petroleum ether/ethyl acetate=2:1) to provide 4,6-dichloro-N-ethoxy nicotinamide (**int-2**, 1.6g, 6.8mmol, 68.3% yield). MS Calcd: 234; MS Found: 235 ($[M+H]^+$).

Example **1**

6-(cyclopropylcarboxamido)-N-methoxy-4-((2-oxo-1-(thiazol-2-yl)-1,2-dihydropyridin-3-yl)amino)nicotinamide

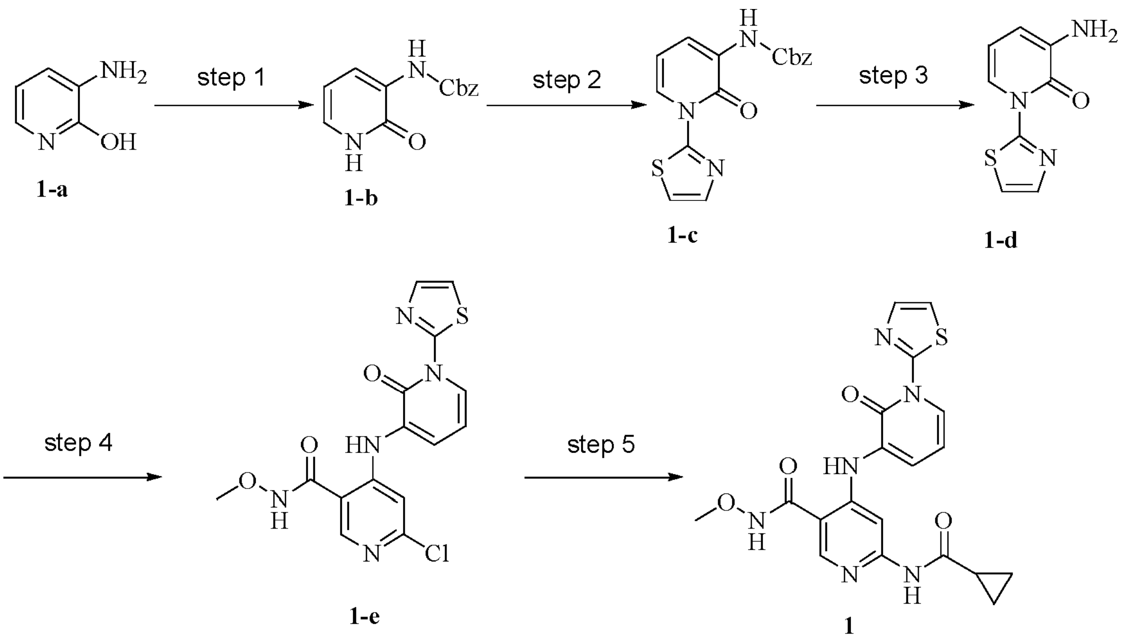


1-a 1-b 1-c 1-d 1-e 1

Step 1: To a 50ml reaction flask was added 3-amino-2-hydroxyl pyridine (**1-a,** 440mg, 4.0mmol) dissolved in dichloromethane (10ml), followed by benzyl chloroformate (750.60mg, 4.4mmol) which was dissolved in dichloromethane (5ml) and carefully added to the reaction flask dropwise at 0°C~5°C. The reaction mixture was stirred at room temperature for 3h. Upon indication of completed reaction by TLC, the reaction mixture was washed respectively with water (10 ml x 1) and brine (10 ml x 1), dried over anhydrous sodium sulfate and concentrated to provide crude material, which was purified by column chromatography to provide the desired product benzyl 2-oxo-1,2-dihydropyridine-3-carbamate (**1-b,** 850mg, 87% yield). MS Calcd:244; MS Found: 245 ($[M+H]^+$).

Step 2: Benzyl 2-oxo-1,2-dihydropyridine-3-carbamate (1-b, 600mg, 2.46mmol), 2-bromothiazole (524.2mg, 3.20mmol), CuI(93.7mg, 0.492mmol), N,N'-dimethyl ethylene diamine (86.74mg, 0.987mmol) and potassium carbonate (679mg, 4.92mmol) were suspended in dioxane (20ml). After atmosphere replacement with nitrogen three times, the mixture was heated to 115°C to react for 5h. Upon indication of completed reaction by TLC, the reaction mixture was concentrated, added with 20 ml of water, and extracted with ethyl acetate (10ml x 3). The combined ethyl acetate layers were dried over anhydrous sodium sulfate, filtered by suction, and concentrated to obtain a crude material, which was purified by column chromatography (PE:EA =4:1) to provide benzyl (2-oxo-1-(thiazol-2-yl)-1,2-dihydropyridin-3-yl)carbamate (**1-c,** 525mg, 1.60mmol, 66% yield). MS Calcd: 327; MS Found: 328 ($[M+H]^+$).

Step 3: Benzyl (2-oxo-1-(thiazol-2-yl)-1,2-dihydropyridin-3-yl)carbamate (**1-c,** 500mg, 1.53mmol) was dissolved in glacial acetic acid (10ml) in a 50 ml reaction flask, added with 40% hydrogen bromide aqueous solution (0.5ml), heated to 90°C to react for 3h. Upon indication of completed reaction by TLC, the reaction mixture was added with water and ethyl acetate, adjusted to pH 7~8 with sodium carbonate, and then extracted with ethyl acetate (10ml x 3). The combined ethyl acetate layers were dried over anhydrous sodium sulfate, filtered by suction, and concentrated to obtain a crude material, which was purified by column chromatography to provide 3-amino-1-(thiazol-2-yl)pyridin-2(1H)-one (**1-d,** 206mg, 1.07mmol, 70% yield). MS Calcd: 193; MS Found: 194 ($[M+H]^+$).

Step 4: 3-amino-1-(thiazol-2-yl)pyridin-2(1H)-one (1-d, 150mg, 0.78mmol) and 4,6-dichloro-N-methoxy nicotinamide **(int-1,** 169.4mg, 0.78mmol) were added to 5ml of anhydrous N,N-dimethylacetamide, added at room temperature with a solution of LiHMDS in tetrahydrofuran(2.34ml, 2.34mmol), and stirred at room temperature for 2h. Upon indication of completed reaction by TLC, the reaction mixture was adjusted to pH 5 by aqueous hydrochloride (1N), and extracted with ethyl acetate (10ml x 3). The combined organic layers were dried, concentrated and then purified by column chromatography to provide 6-chloro-N-methoxy-4-((2-oxo-1-(thiazol-2-yl)-1,2-dihydropyridin-3-yl)amino)nicotinamide (**1-e,** 200mg, 0.53mmol, 68% yield). MS Calcd: 377; MS Found: 378 ($[M+H]^+$).

Step 5: 6-chloro-N-methoxy-4-((2-oxo-1-(thiazol-2-yl)-1,2-dihydropyridin-3-yl)amino)nicotinamide (**1-e,** 100mg, 0.26mmol), cyclopropylcarboxamide (24.8mg, 0.292mmol), cesium carbonate (256.80mg, 0.80mmol), XantPhos(30mg, 0.04mmol) and $Pd_2(dba)_3$(24mg, 0.026mmol) were added to anhydrous dioxane (5ml) and the mixture was evacuated to vacuum. After atmosphere replacement with nitrogen three times, the reaction mixture was heated to 100°C, stirred for 5h, and filtered by suction. The filtrate was concentrated and purified by high performance preparative thin layer chromatography (MeOH:DCM=1:20) to provide the title compound: 6-(cyclopropylcarboxamido)-N-methoxy-4-((2-oxo-1-(thiazol-2-yl)-1,2-dihydropyridin-3-yl)amino)nicotinamide (1, 15mg, 0.035mmol, 13.5% yield). MS Calcd: 426; MS Found: 427 ($[M+H]^+$). $^1$H NMR (400 MHz, DMSO-$d_6$) : δ 11.92 (s, 1H), 10.98 (s, 1H), 10.56 (s, 1H), 8.49-8.47 (dd, $J_1$=1.2 Hz, $J_2$=7.2 Hz,

1H), 8.42 (s, 1H), 8.27 (s, 1H), 7.83-7.82(d, *J* = 3.2 Hz, 1H), 7.73-7.72(d, *J* = 4 Hz, 1H), 7.52-7.50 (t, *J* = 8.0 Hz, 1H), 6.71-6.67-7.50 (t, *J*= 16 Hz, 1H), 3.74 (s, 3H), 1.98-1.95 (m, 1H), 0.85-0.82 (m, 4H).

Example **2**

6-(cyclopropylcarboxamido)-N-methoxy-4-((2-(N-methyl methanesulfonamido)phenyl)amino)nicotinamide

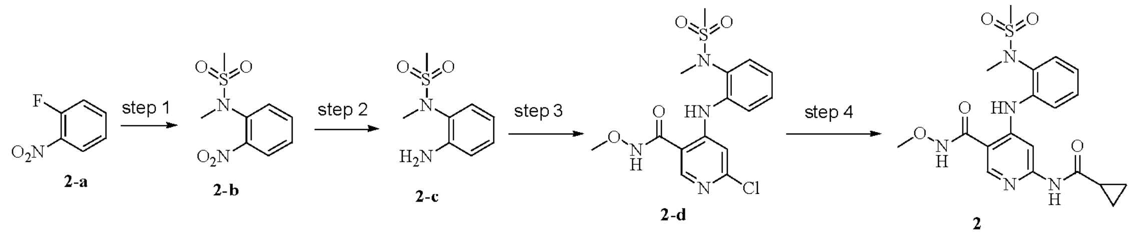


Step 1: 1-fluoro-2-nitrobenzene (**2-a,** 1.0g, 9.16mmol) dissolved in acetonitrile (30ml) was added to a 100ml reaction flask followed by cesium carbonate (5.95g, 18.32mmol) and N-methyl methanesulfonamide (1.227g, 8.702mmol) The reaction mixture was stirred at room temperature overnight. Upon indication of completed reaction by TLC, the reaction mixture was washed respectively with water (30 ml x 1) and brine (30 ml x 1), dried over anhydrous sodium sulfate, and concentrated to obtain a crude material, which was purified by silica gel column chromatography (petroleum ether/ethyl acetate=3:1) to provide the product N-methyl-N-(2-nitrophenyl) methanesulfonamide (**2-b,** 1.2g, 73.6% yield). MS Calcd:230; MS Found: 231([M+H]$^+$).

Step 2: N-methyl-N-(2-nitrophenyl) methanesulfonamide (**2-b,** 460mg, 2mmol) and 10% palladium on carbon (46mg) were added to methanol (15ml), followed by atmosphere replacement by hydrogen three times, and stirred under hydrogen atmosphere at room temperature overnight. The reaction mixture was filtered by suction, and the filtrate was concentrated under reduced pressure to provide N-(2-amino phenyl)-N-methyl methanesulfonamide (**2**-c, 350mg, 1.75mmol, 87.5% yield), which was used directly in the next reaction without further purification. MS Calcd: 200; MS Found: 201 ([M+H]$^+$). Step 3: N-(2-amino phenyl)-N-methyl methanesulfonamide (**2**-c, 200mg, 1mmol) and 4,6-dichloro-N-methoxy nicotinamide **(int-1,** 218mg, 1mmol) were added to 5ml of anhydrous N,N-dimethylacetamide, added at room temperature with a solution of LiHMDS in tetrahydrofuran (3ml, 3mmol), and stirred at room temperature for 2h. Upon indication of completed reaction by TLC, the reaction mixture was adjusted with aqueous hydrochloride (1N) to pH 5, and extracted with ethyl acetate (30 ml x 3). The combined organic layers were dried, concentrated and then purified by column chromatography to provide 6-chloro-N-methoxy-4-((2-(N-methyl methanesulfonamido)phenyl)amino)nicotinamide (**2-d,** 150mg, 0.39mmol, 39% yield). MS Calcd: 384; MS Found: 385 ([M+H]$^+$).

Step 4: 6-chloro-N-methoxy-4-((2-(N-methyl methanesulfonamido)phenyl)amino)nicotinamide (**2-d,** 39mg, 0.1mmol), cyclopropylcarboxamide (9.4mg, 0.11mmol), cesium carbonate (130mg, 0.4mmol), XantPhos(15mg, 0.02mmol) and $Pd_2(dba)_3$ (10mg, 0.01mmol) were added to anhydrous dioxane (5ml) and the mixture was evacuated to vacuum. After atmosphere replacement with nitrogen three times, the reaction mixture was heated to 100°C, and stirred for 5h. After filtration by suction, the filtrate was concentrated and purified by high performance preparative thin layer chromatography (MeOH:DCM=1:20) to provide 6-(cyclopropylcarboxamido)-N-methoxy-4-((2-(N-methyl methanesulfonamido)phenyl)amino)nicotinamide (**2,** 10 mg, 0.023mmol, 23.1% yield). MS Calcd: 433; MS Found: 434 ([M+H]$^+$). $^1H$ NMR (400 MHz, DMSO-$d_6$) : δ 11.82 (s, 1H), 10.81 (s, 1H), 10.05 (s, 1H), 8.33 (s, 1H), 7.92 (s, 1H), 7.58-7.53 (m, 1H), 7.50-7.48 (m, 1H), 7.42-7.24 (m, 1H), 7.23-7.20 (m, 1H), 3.88 (s, 3H), 3.14 (s, 3H), 3.10(s, 3H), 1.97-1.94 (m, 1H), 0.77-0.76 (m, 4H).

Example **3**

6-(cyclopropylcarboxamido)-N-methoxy-4-((2-methoxy-3-(1-methyl-1*H*-benzo[d]imidazol-2-yl)phenyl)amino)nicotinamide

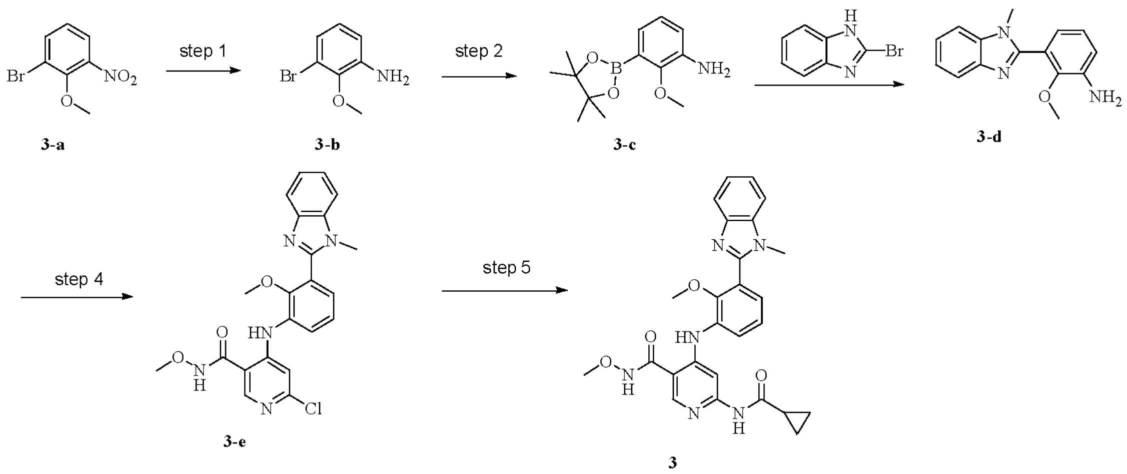


3-a 3-b 3-c 3-d 3-e 3

Step 1: To a 100ml flask were added 1-bromo-2-methoxy-3-nitrobenzene(**3-a,** 1g, 4.32mmol), iron powder (1.21g, 21.6mmol) and glacial acetic acid (20ml). The mixture was heated to 85°C with stirring for 3h. When TLC indicated a completed reaction, the reaction mixture was allowed to cool down to room temperature, added with ethyl acetate and water, and then filtered through diatomaceous earth. The filtrate was subjected to phase separation, and the aqueous phase was extracted with ethyl acetate until no product remained in the aqueous phase. The organic phases were combined and washed with saturated sodium carbonate solution (50 ml x 3), then washed with saturated brine once, dried over anhydrous sodium sulfate, and filtered by suction. The filtrate was concentrated under reduced pressure to provide 3-bromo-2-methoxy aniline **(3-b,** 750mg, 3.73mmol, 86.37% yield), which was used directly in the next reaction without further purification. MS Calcd: 202; MS Found: 203 ($[M+H]^+$).

Step 2: 3-bromo-2-methoxy aniline(**3-b**, 402mg, 2mmol), sodium carbonate (318mg, 3mmol), bis(pinacolato) diboron (609.6g, 2.4mmol) and 1,1'-bis(diphenylphosphino)ferrocene dichloropalladium (II) (146.2mg, 0.2mmol) were added to anhydrous dioxane (10ml). The reaction mixture was evacuated to vacuum. After atmosphere replacement with nitrogen three times, the reaction mixture was heated to 90°C, stirred for 3h, and then filtered by suction. The filtrate was concentrated and purified by silica gel column chromatography (petroleum ether/ethyl acetate=3:1) to provide 2-methoxy-3-(4,4,5,5-tetramethyl-1,3,2-dioxaborolan-2-yl)aniline (**3-c,** 210 mg, 0.84mmol, 42.2% yield). MS Calcd: 249; MS Found: 250 ($[M+H]^+$).

Step 3: 2-bromo-1H-benzo[d]imidazole (**3-c,** 392mg, 2mmol) dissolved in acetonitrile (15ml)was added to a 100ml reaction flask followed by cesium carbonate (975mg, 3mmol), iodomethane (312.4mg, 2.2mmol). The reaction mixture was stirred at room temperature overnight. Upon indication of completed reaction by TLC, the reaction mixture was added with water and ethyl acetate, extracted with ethyl acetate (30 ml x 2). The organic phase was washed with brine (30 ml x 1), dried over anhydrous sodium sulfate, and concentrated to provide a crude material, which was purified by silica gel column chromatography (petroleum ether/ethyl acetate=4:1) to provide 2-bromo-1-methyl-1H-benzo[d]imidazole (**3-d,** 400mg, 1.90mmol, 95.2% yield). MS Calcd:210; MS Found: 211($[M+H]^+$).

Step 4: 2-bromo-1-methyl-1H-benzo[d]imidazole (**3-d,** 176.4mg, 0.84mmol), sodium carbonate (178mg, 1.68mmol), 2-methoxy-3-(4,4,5,5-tetramethyl-1,3,2-dioxaborolan-2-yl)aniline(33067-c, 210 mg, 0.84mmol) and 1,1'-bis(diphenylphosphino)ferrocene dichloropalladium (II) (61.4mg, 0.084mmol) were added to anhydrous dioxane (15ml) and the mixture was evacuated to vacuum. After atmosphere replacement with nitrogen three times, the reaction mixture was heated to 90°C, stirred for 3h, and filtered by suction. The filtrate was concentrated and purified by silica gel column chromatography (petroleum ether/ethyl acetate=2:1) to provide 2-methoxy-3-(1-methyl-1H-benzo[d]imidazol-2-yl)aniline (**3-e,** 105 mg, 0.41mmol, 49.4% yield). MS Calcd: 253; MS Found: 254($[M+H]^+$).

Step 5: 2-methoxy-3-(1-methyl-1H-benzo[d]imidazol-2-yl)aniline (**3-e,** 105 mg, 0.41mmol) and 4,6-dichloro-N-methoxy nicotinamide **(int-1,** 90mg, 0.41mmol) were added to 5ml of anhydrous N,N-dimethylacetamide followed by the addition of a solution of LiHMDS in tetrahydrofuran (1.23ml, 1.23mmol) at room temperature, and stirred at room temperature for 2h. Upon indication of completed reaction by TLC, the reaction mixture was adjusted with aqueous hydrochloride (1N) to pH 5, and extracted with ethyl acetate (30 ml x 3). The combined organic layers were dried, concentrated and then purified by column chromatography to provide 6-chloro-N-methoxy-4-((2-methoxy-3-(1-methyl-1H-benzo[d]imidazol-2-yl)phenyl)amino)nicotinamide (**3-f,** 70mg, 0.16mmol, 39% yield). MS Calcd: 437; MS Found:438 ($[M+H]^+$).

Step 6: 6-chloro-N-methoxy-4-((2-methoxy-3-(1-methyl-1H-benzo[d]imidazol-2-yl)phenyl)amino)nicotinamide (**3-f,** 70mg, 0.16mmol), cyclopropylcarboxamide (21.51mg, 0.19mmol), cesium carbonate (156mg, 0.48mmol), Xant-Phos(18mg, 0.032mmol) and $Pd_2(dba)_3$ (16.5mg, 0.016mmol) were added to anhydrous dioxane (5ml) and the mixture was evacuated to vacuum. After atmosphere replacement with nitrogen three times, the reaction mixture was heated to

100°C, stirred for 5h, and filtered by suction. The filtrate was concentrated and purified by high performance preparative thin layer chromatography (MeOH:DCM=1:20) to provide the title compound: 6-(cyclopropylcarboxamido)-N-methoxy-4-((2-methoxy-3-(1-methyl-1H-benzo[d]imidazol-2-yl)phenyl)amino)nicotinamide (**3,** 10mg, 0.02mmol, 10.8% yield). MS Calcd: 486; MS Found: 487 ([M+H]$^+$). $^1$H NMR (400 MHz, DMSO-$d_6$)δ11.88 (s, 1H), 10.85 (s, 1H), 10.18 (s, 1H), 8.38 (s, 1H), 8.05 (s, 1H), 7.70 (d, *J* = 7.8 Hz, 1H), 7.63 (d, *J* = 7.8 Hz, 2H), 7.37 - 7.24 (m, 4H)., 3.73 (s, 3H), 3.66 (s, 3H), 3.40(s, 3H), 2.33-2.31 (m, 1H), 0.85-0.79 (m, 4H).

Example **4**

6-((5-fluoropyridin-2-yl)amino)-N-methoxy-4-((2-(N-methyl methanesulfonamido)phenyl)amino)nicotinamide

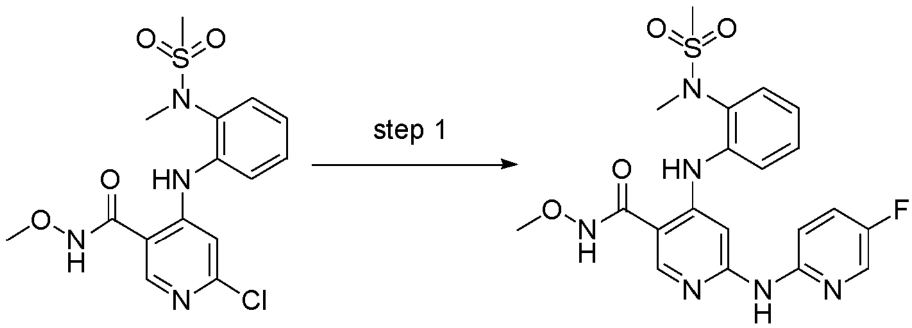


**2-d** 4

Step 1: 6-chloro-N-methoxy-4-((2-(N-methyl methanesulfonamido)phenyl)amino)nicotinamide (**2-d,** 67mg, 0.17mmol), 5-fluoro-2-aminopyridine (21.51mg, 0.19mmol), cesium carbonate (166.7mg, 0.51mmol), XantPhos(18.6mg, 0.034mmol) and $Pd_2(dba)_3$ (16.6mg, 0.017mmol) were added to anhydrous dioxane (5ml) and the mixture was evacuated to vacuum. After atmosphere replacement with nitrogen three times, the reaction mixture was heated to 100°C, stirred for 5h, and filtered by suction. The filtrate was concentrated and purified by high performance preparative thin layer chromatography (MeOH:DCM=1:20) to provide 6-((5-fluoropyridin-2-yl)amino)-N-methoxy-4-((2-(N-methyl methanesulfonamido)phenyl)amino)nicotinamide (**4**, 17 mg, 0.037 mmol, 21.7% yield). MS Calcd: 460; MS Found: 461 ([M+H]$^+$). $^1$H NMR (400 MHz, DMSO-$d_6$) : δ 11.71 (s, 1H), 10.09 (s, 1H), 9.84 (s, 1H), 8.30 (s, 1H), 8.15-8.14 (d, *J* = 2.8 Hz, 1H), 7.67-7.61 (m, 4H), 7.55 (dd, *J= 8.0,* 2.0 Hz, 1H), 7.50-7.46 (m, 1H), 7.20-7.19 (m, 1H), 3.72 (s, 3H), 3.16 (s, 3H), 3.14 (s, 3H).

Example **5**

N-methoxy-6-(((2-methoxy pyridin-3-yl)amino)-4-((2-(N-methyl methanesulfonamido)phenyl)amino)nicotinamide

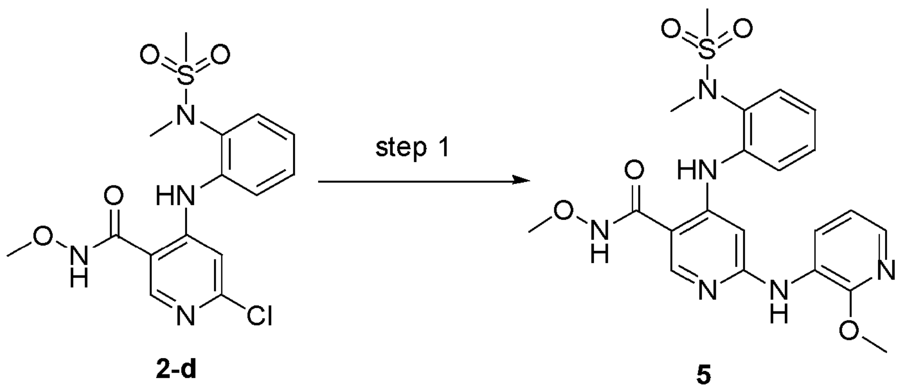


**2-d** **5**

Step 1: 6-chloro-N-methoxy-4-((2-(N-methyl methanesulfonamido)phenyl)amino)nicotinamide (**2-d,** 60mg, 0.16mmol), 2-methoxy-3-aminopyridine (19.4mg, 0.16mmol), cesium carbonate (152mg, 0.47mmol), XantPhos(17.53mg, 0.032mmol) and $Pd_2(dba)_3$(15.6mg, 0.016mmol) were added to anhydrous dioxane (5ml) and the mixture

was evacuated to vacuum. After atmosphere replacement with nitrogen three times, the reaction mixture was heated to 100°C, stirred for 5h, and filtered by suction. The filtrate was concentrated and purified by high performance preparative thin layer chromatography (MeOH:DCM=1:20) to provide the title compound: N-methoxy-6-(((2-methoxy pyridin-3-yl) amino)-4-((2-(N-methyl methanesulfonamido)phenyl)amino)nicotinamide (**5,** 15 mg, 0.032mmol, 20.5% yield). MS Calcd: 472; MS Found: 473 ([M+H]$^+$). $^1$H NMR (400 MHz, DMSO-$d_6$) : δ 11.68 (s, 1H), 9.93 (s, 1H), 8.58 (s, 1H), 8.50-8.48 (m, 1H), 8.33 (s, 1H), 7.73-7.72 (m, 1H), 7.56-7.53 (m, 2H), 7.44-7.40 (m, 1H), 7.21-7.17 (m, 1H), 6.95-6.91 (m, 1H), 6.85-6.84 (m, 1H), 3.91 (s, 3H), 3.71 (s, 3H), 3.16 (s, 3H), 3.14 (s, 3H).

Example **6**

6-(cyclopropylcarboxamido)-N-methoxy-4-((4-methoxy-2-(N-methyl methanesulfonamido)phenyl)amino)nicotinamide

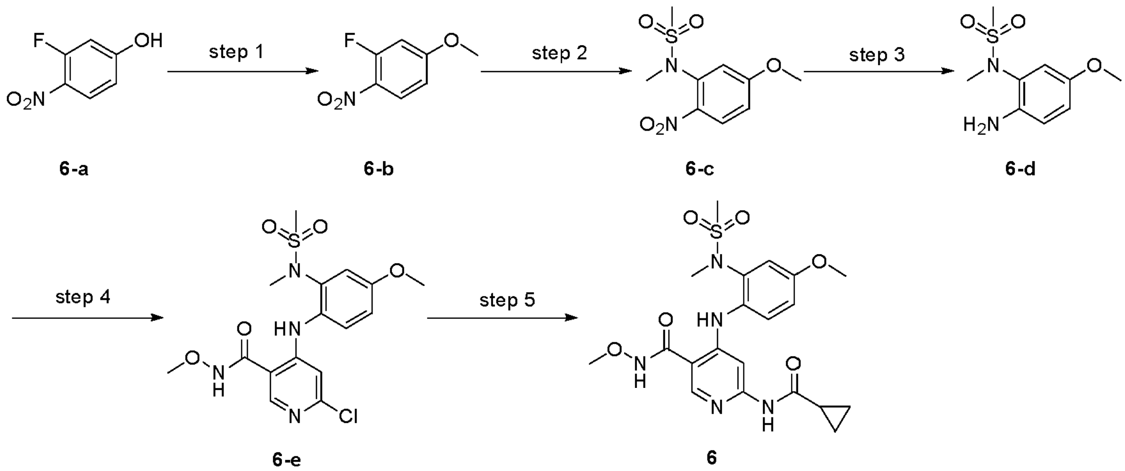


Step 1: 3-fluoro-4-nitrophenol (**6-a,** 628mg, 4mmol) dissolved in acetonitrile (30ml) was added to a 100ml reaction flask followed by cesium carbonate (1.95g, 6mmol) and iodomethane (624.8mg, 4.4mmol). The reaction mixture was stirred at room temperature overnight. Upon indication of completed reaction by TLC, the reaction mixture was added with water and ethyl acetate, extracted with ethyl acetate (30 ml x 2). The organic phase was washed with brine (30 ml x 1), dried over anhydrous sodium sulfate, and concentrated to provide a crude material, which was purified by silica gel column chromatography (petroleum ether/ethyl acetate=3:1) to provide 2-fluoro-4-methoxy-1-nitrobenzene(**6-b,** 650mg, 3.82mmol, 95% yield). MS Calcd:157; MS Found: 158([M+H]$^+$).

Step 2: 2-fluoro-4-methoxy-1-nitrobenzene (**6-b,** 600g, 3.5mmol) dissolved in dichloromethane (30ml)was added to a 100ml reaction flask followed by cesium carbonate (1.70g, 5.25mmol) and N-methyl methanesulfonamide (412mg, 3.85mmol). The reaction mixture was stirred at room temperature overnight. Upon indication of completed reaction by TLC, the reaction mixture was washed respectively with water (30 ml x 1) and brine (30 ml x 1), dried over anhydrous sodium sulfate, and concentrated to provide a crude material, which was purified by silica gel column chromatography (petroleum ether/ethyl acetate=3:1) to provide N-(5-methoxy-2-nitrophenyl)-N-methyl methanesulfonamide (**6-c,** 520mg, 2mmol, 57.1% yield). MS Calcd: 260; MS Found: 261([M+H]$^+$).

Step 3: N-(5-methoxy-2-nitrophenyl)-N-methyl methanesulfonamide (**6-c,** 520mg, 2mmol) and palladium on carbon (46mg) were added to methanol (20ml), followed by atmosphere replacement by hydrogen three times, and stirred under hydrogen atmosphere at room temperature overnight, and filtered by suction. The filtrate was concentrated under reduced pressure to provide N-(2-amino phenyl)-N-methyl methanesulfonamide (**6-d,** 440mg, 1.87mmol, 93.47% yield), which was used directly in the next reaction without further purification. MS Calcd: 230; MS Found: 231 ([M+H]$^+$)

Step 4: N-(2-amino-5-methoxy phenyl)-N-methyl methanesulfonamide (**6-d,** 230mg, 1mmol) and 4,6-dichloro-N-methoxy nicotinamide **(int-1,** 218mg, 1mmol) were added to 5ml of anhydrous N,N-dimethylacetamide. The mixture was added at room temperature with a solution of LiHMDS in tetrahydrofuran (3ml, 3mmol), and stirred at room temperature for 2h. Upon indication of completed reaction by TLC, the mixture was adjusted with aqueous hydrochloride (1N) to pH 5, and extracted with ethyl acetate (30 ml x 3). The combined organic layers were dried, concentrated and then purified by column chromatography to provide 6-chloro-N-methoxy-4-((4-methoxy-2-(N-methyl methanesulfonamido) phenyl)amino)nicotinamide (**6-e,** 200mg, 0.483mmol, 48.3% yield). MS Calcd: 414; MS Found:415 ([M+H]$^+$).

Step 5: 6-chloro-N-methoxy-4-((4-methoxy-2-(N-methyl methanesulfonamido)phenyl)amino)nicotinamide (**6-e,** 100mg, 0.24mmol), cyclopropylcarboxamide (20.4mg, 0.24mmol), cesium carbonate (234mg, 0.72mmol), Xant-

Phos(27.74mg, 0.048mmol) and $Pd_2(dba)_3$(23.4mg, 0.024mmol) were added to anhydrous dioxane (5ml) and the mixture was evacuated to vacuum. After atmosphere replacement with nitrogen three times, the reaction mixture was heated to 100°C, stirred for 5h, and filtered by suction. The filtrate was concentrated and purified by high performance preparative thin layer chromatography (MeOH:DCM=1:20) to provide 6-(cyclopropylcarboxamido)-N-methoxy-4-((4-methoxy-2-(N-methyl methanesulfonamido)phenyl)amino)nicotinamide (**6,** 15 mg, 0.032mmol, 13.3% yield). MS Calcd: 463; MS Found: 464 ($[M+H]^+$). $^1H$ NMR (400 MHz, DMSO-$d_6$) : δ 11.72 (s, 1H), 10.70 (s, 1H), 9.70 (s, 1H), 8.30 (s, 1H), 7.62 (s, 1H), 7.51 (s, 1H), 7.35-7.33 (m, 1H), 7.14 (s, 1H), 3.81 (s, 3H), 3.71 (s, 3H), 3.11 (s, 3H), 3.09 (s, 3H), 1.98-2.01 (m, 1H), 0.75-0.73 (m, 4H).

Example **7**

6-(cyclopropylcarboxamido)-N-methoxy-4-((6-methoxy-2-(N-methyl methanesulfonamido)pyridin-3-yl)amino)nicotinamide

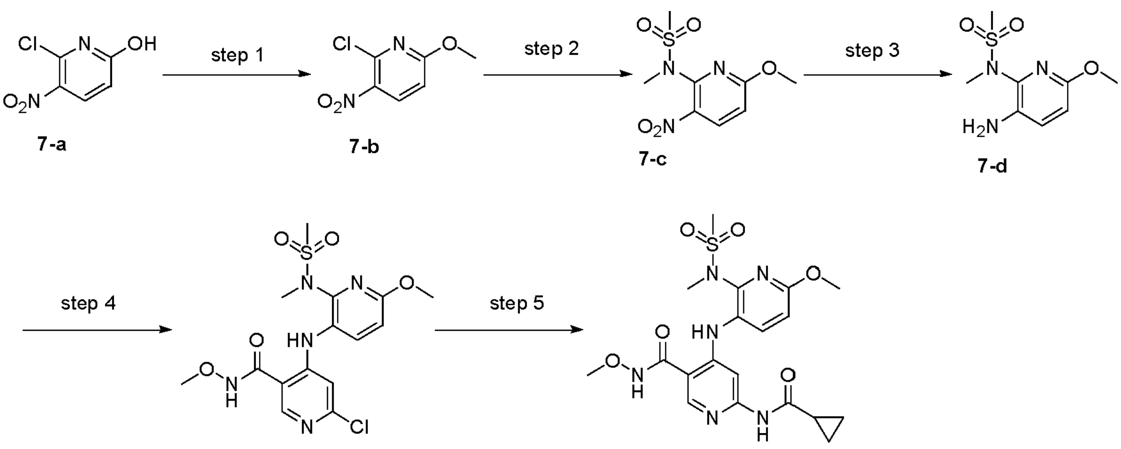


7-a 7-b 7-c 7-d 7-e 7

Step 1: 6-chloro-5-nitropyridin-2-ol (**7-a,** 696mg, 4mmol) dissolved in acetonitrile (30ml) was added to a 100ml reaction flask followed by cesium carbonate (1.95g, 6mmol) and iodomethane (624.8mg, 4.4mmol). The reaction mixture was stirred at room temperature overnight. Upon indication of completed reaction by TLC, the reaction mixture was added with water and ethyl acetate, extracted with ethyl acetate (30 ml x 2). The organic phase was washed with brine (30 ml x 1), dried over anhydrous sodium sulfate, and concentrated to provide a crude material, which was purified by silica gel column chromatography (petroleum ether/ethyl acetate=3:1) to provide 2-chloro-6-methoxy-3-nitropyridine (**7-b,** 700mg, 3.72mmol, 93% yield). MS Calcd:188; MS Found: 189($[M+H]^+$).

Step 2: 2-chloro-6-methoxy-3-nitropyridine (**7-b,** 600g, 3.19mmol) dissolved in dichloromethane (30ml) was added to a 100ml reaction flask followed by cesium carbonate (1.57g, 4.78mmol) and N-methyl methanesulfonamide (379mg, 3.51mmol). The reaction mixture was stirred at room temperature overnight. Upon indication of completed reaction by TLC, the reaction mixture was washed respectively with water (30 ml x 1) and brine (30 ml x 1), dried over anhydrous sodium sulfate, and concentrated to provide a crude material, which was purified by silica gel column chromatography (petroleum ether/ethyl acetate=3:1) to provide N-(6-methoxy-3-nitropyridin-2-yl)-N-methyl methanesulfonamide (**7-c,** 550mg, 2.10mmol, 65.8% yield). MS Calcd: 261; MS Found: 262($[M+H]^+$).

Step 3: N-(6-methoxy-3-nitropyridin-2-yl)-N-methyl methanesulfonamide (**7-c,** 550mg, 2.1mmol) and palladium on carbon (46mg) were added to methanol(20ml), followed by atmosphere replacement by hydrogen three times, and stirred under hydrogen atmosphere at room temperature overnight, and filtered by suction. The filtrate was concentrated under reduced pressure to provide N-(3-amino-6-methoxy pyridin-2-yl)-N-methyl methanesulfonamide (**7-d,** 450mg, 1.95mmol, 92.76% yield), which was used directly in the next reaction without further purification. MS Calcd: 231; MS Found: 232 ($[M+H]^+$).

Step 4: N-(3-amino-6-methoxy pyridin-2-yl)-N-methyl methanesulfonamide (**7-d,** 231mg, 1mmol) and 4,6-dichloro-N-methoxy nicotinamide **(int-1,** 218mg, 1mmol), were added to 5ml of anhydrous N,N-dimethylacetamide. The mixture was added at room temperature with a solution of LiHMDS in tetrahydrofuran (3ml, 3mmol), and stirred at room temperature for 2h. Upon indication of completed reaction by TLC, the mixture was adjusted with aqueous hydrochloride (1N) to pH 5, and extracted with ethyl acetate (30 ml x 3). The combined organic layers were dried, concentrated and then purified by column chromatography to provide 6-chloro-N-methoxy-4-((6-methoxy-2-(N-methyl

methanesulfonamido)pyridin-3-yl)amino)nicotinamide (**7-e,** 210mg, 0.506mmol, 50.6% yield). MS Calcd: 415; MS Found:416 ([M+H]$^+$).

Step 5: 6-chloro-N-methoxy-4-((6-methoxy-2-(N-methyl methanesulfonamido)pyridin-3-yl)amino)nicotinamide (**7-e,** 100mg, 0.24mmol), cyclopropylcarboxamide (20.4mg, 0.24mmol), cesium carbonate (234mg, 0.72mmol), Xant-Phos(27.74mg, 0.048mmol) and $Pd_2(dba)_3$(23.4mg, 0.024mmol) were added to anhydrous dioxane (5ml) and the mixture was evacuated to vacuum. After atmosphere replacement with nitrogen three times, the reaction mixture was heated to 100°C, stirred for 5h, and filtered by suction. The filtrate was concentrated and purified by high performance preparative thin layer chromatography (MeOH:DCM=1:20) to provide the title compound: 6-(cyclopropylcarboxamido)-N-methoxy-4-((6-methoxy-2-(N-methyl methanesulfonamido)pyridin-3-yl)amino)nicotinamide (**7,** 16 mg, 0.034mmol, 14.3% yield). MS Calcd: 464; MS Found: 465 ([M+H]$^+$).

$^1$H NMR (400 MHz, DMSO-$d_6$) : δ 11.79 (s, 1H), 10.76 (s, 1H), 9.78 (s, 1H), 8.34 (s, 1H), 7.85 (d, *J* = 8.4 Hz, 1H), 7.58 (s, 1H), 6.94 (d, *J* = 8.4 Hz, 1H), 3.88 (s, 3H), 3.71 (s, 3H), 3.18 (s, 3H), 3.11 (s, 3H), 1.98-2.01 (m, 1H), 0.76-0.74 (m, 4H).

Example **8**

6-((5-fluoropyridin-2-yl)amino)-N-methoxy-4-((6-methoxy-2-(N-methyl methanesulfonamido)pyridin-3-yl)amino)nicotinamide

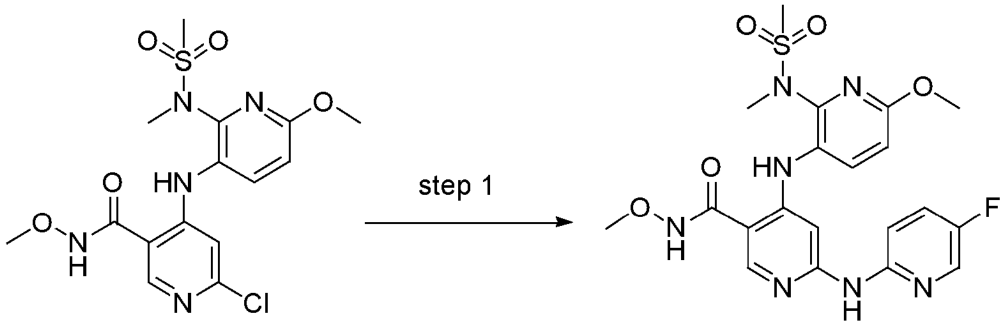


**7-e** **8**

Step 1: 6-chloro-N-methoxy-4-((6-methoxy-2-(N-methyl methanesulfonamido)pyridin-3-yl)amino)nicotinamide (**7-e,** 50mg, 0.12mmol), 5-fluoropyridin-2-ylamine (14.87mg, 0.13mmol), cesium carbonate (117mg, 0.36mmol), Xant-Phos(13.9mg, 0.024mmol) and $Pd_2(dba)_3$(11mg, 0.012mmol) were added to anhydrous dioxane (5ml) and the mixture was evacuated to vacuum. After atmosphere replacement with nitrogen three times, the reaction mixture was heated to 100°C, stirred for 5h, and filtered by suction. The filtrate was concentrated and purified by high performance preparative thin layer chromatography (MeOH:DCM=1:20) to provide the title compound: 6-((5-fluoropyridin-2-yl)amino)-N-methoxy-4-((6-methoxy-2-(N-methyl methanesulfonamido)pyridin-3-yl)amino)nicotinamide (**8,** 8 mg, 0.016mmol, 13.6% yield). MS Calcd: 491; MS Found: 492 ([M+H]$^+$). $^1$H NMR (400 MHz, DMSO-$d_6$) : δ 11.68 (s, 1H), 9.79 (s, 1H), 9.76 (s, 1H), 8.30 (s, 1H), 8.12 (d, *J* = 2.8 Hz, 1H), 7.95 (d, *J* = 8.8 Hz, 1H), 7.68-7.67 (m, 2H), 7.24 (s, 1H), 7.03 (d, *J* = 8.8 Hz, 1H), 3.89 (s, 3H), 3.71 (s, 3H), 3.21 (s, 3H), 3.13 (s, 3H).

Example **9**

N-methoxy-4-((6-methoxy-2-(N-methyl methanesulfonamido)pyridin-3-yl)amino)-6-((2-methoxy pyridin-3-yl)amino)nicotinamide

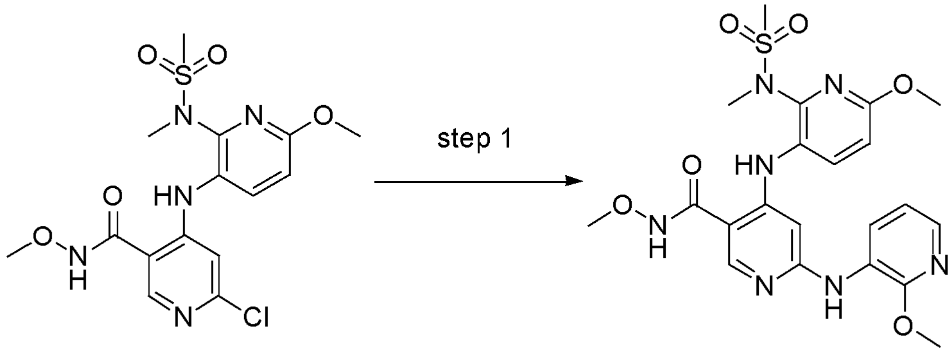


7-e 9

Step 1: 6-chloro-N-methoxy-4-((6-methoxy-2-(N-methyl methanesulfonamido)pyridin-3-yl)amino)nicotinamide (**7-e,** 50mg, 0.12mmol), 2-methoxy pyridin-3-amine (16.12mg, 0.13mmol), cesium carbonate (117mg, 0.36mmol), XantPhos(13.9mg, 0.024mmol) and $Pd_2(dba)_3$(11mg, 0.012mmol) were added to anhydrous dioxane (5ml) and the mixture was evacuated to vacuum. After atmosphere replacement with nitrogen three times, the reaction mixture was heated to 100°C, stirred for 5h, and filtered by suction. The filtrate was concentrated and purified by high performance preparative thin layer chromatography (MeOH:DCM=1:20) to provide the title compound: N-methoxy-4-((6-methoxy-2-(N-methyl methanesulfonamido)pyridin-3-yl)amino)-6-((2-methoxy pyridin-3-yl)amino)nicotinamide (**9**, 8 mg, 0.016mmol, 13.6% yield). MS Calcd: 503; MS Found: 504 ($[M+H]^+$). $^1H$ NMR (400 MHz, DMSO-$d_6$) : δ 11.65 (s, 1H), 9.59(s, 1H)8.52 (dd, $J$ = 8.0, 1.6 Hz, 1H), 8.43 (s, 1H), 8.24(s, 1H)7.88 (d, $J$ = 8.8 Hz, 1H), 7.71 (dd, $J$ = 4.8, 2.0 Hz, 1H), 6.95 (d, $J$ = 8.4 Hz, 1H), 6.92 (dd, $J$ = 8.0, 4.8 Hz, 1H), 6.44 (s, 1H), 3.90 (s, 3H), 3.86 (s, 3H), 3.71 (s, 3H), 3.21(s, 3H), 3.12(s, 3H).

Example **10**

6-(cyclopropylcarboxamido)-N-methoxy-4-((2-(N-methyl methanesulfonamido)-4-(trifluoromethyl)phenyl)amino)nicotinamide

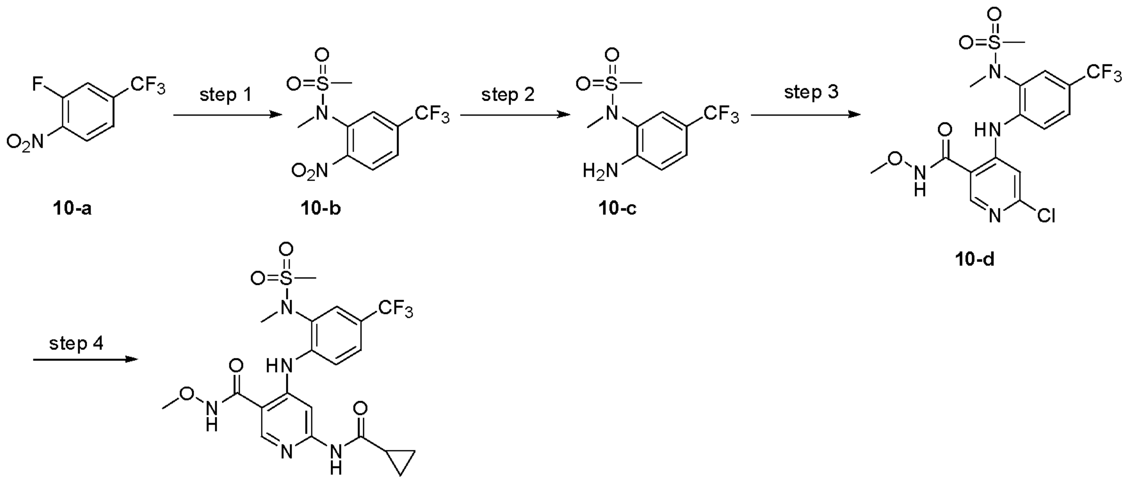

10-a 10-b 10-c 10-d

10

Step 1: 2-fluoro-1-nitro-4-(trifluoromethyl)benzene (**10-a,** 418mg, 2mmol)dissolved in DMF(20ml) was added to a 100ml reaction flask followed by 60% sodium hydride (120°Cg, 3mmol) and N-methyl methanesulfonamide (261.6mg, 2.4mmol). The mixture was stirred at 50°C for 2h. Upon indication of completed reaction by TLC, the reaction mixture was added with water (40ml)/ethyl acetate (30ml), and subjected to phase separation. The organic phase was washed with water (30 ml x 1) and brine (30 ml x 1), dried over anhydrous sodium sulfate, and concentrated to provide a crude material, which was purified by silica gel column chromatography (petroleum ether/ethyl acetate=3:1) to provide the product N-methyl-N-(2-nitro-5-(trifluoromethyl)phenyl) methanesulfonamide(**10-b,** 420mg, 70.4% yield). MS Calcd:298; MS Found: 299($[M+H]^+$).

Step 2: N-methyl-N-(2-nitro-5-(trifluoromethyl)phenyl) methanesulfonamide **(10-b,** 400mg, 1.34mmol) and

palladium on carbon (40mg) were added to methanol(15ml), followed by atmosphere replacement by hydrogen three times, stirred under hydrogen atmosphere at room temperature overnight, and filtered by suction. The filtrate was concentrated under reduced pressure to provide N-(2-amino-5-(trifluoromethyl)phenyl)-N-methyl methanesulfonamide **(10-c,** 350mg, 1.31mmol, 97.4% yield), which was used directly in the next reaction without further purification. MS Calcd: 268; MS Found: 269 ($[M+H]^+$).

Step 3: N-(2-amino-5-(trifluoromethyl)phenyl)-N-methyl methanesulfonamide **(10-c,** 350mg, 1.31mmol) and 4,6-dichloro-N-methoxy nicotinamide **(int-1,** 285.6mg, 1.31mmol) were added to 5ml of anhydrous N,N-dimethylacetamide. The mixture was added at room temperature with a solution of LiHMDS in tetrahydrofuran (4ml, 3.93mmol), and stirred at room temperature for 2h. Upon indication of completed reaction by TLC, the mixture was adjusted with aqueous hydrochloride (1N) to pH 5, and extracted with ethyl acetate (30 ml x 3). The combined organic layers were dried, concentrated and then purified by column chromatography to provide 6-chloro-N-methoxy-4-((2-(N-methyl methanesulfonamido)-4-(trifluoromethyl)phenyl)amino)nicotinamide **(10-d,** 110mg, 0.24mmol, 18.6% yield). MS Calcd: 452; MS Found: 453 ($[M+H]^+$).

Step 4: 6-chloro-N-methoxy-4-((2-(N-methyl methanesulfonamido)-4-(trifluoromethyl)phenyl)amino)nicotinamide **(10-d,** 60mg, 0.13mmol), cyclopropylcarboxamide (12.4mg, 0.15mmol), cesium carbonate (126.7mg, 0.39mmol), XantPhos(11mg, 0.02mmol) and $Pd_2(dba)_3$(10mg, 0.01mmol) were added to anhydrous dioxane (5ml) and the mixture was evacuated to vacuum. After atmosphere replacement with nitrogen three times, the reaction mixture was heated to 120°C, stirred for 5h, and filtered by suction. The filtrate was concentrated and purified by high performance preparative thin layer chromatography (MeOH:DCM=1:20) to provide the title compound: 6-(cyclopropylcarboxamido)-N-methoxy-4-((2-(N-methyl methanesulfonamido)-4-(trifluoromethyl)phenyl)amino)nicotinamide (**10,** 8 mg, 0.016mmol, 12.3% yield).

MS Calcd: 501; MS Found: 502 ($[M+H]^+$). $^1$H NMR (400 MHz, DMSO-$d_6$) : δ 11.91 (s, 1H), 10.93 (s, 1H), 10.37 (s, 1H), 8.40 (s, 1H), 8.17 (s, 1H), 7.92 (d, *J* = 2.0 Hz, 1H), 7.77 - 7.70 (m, 2H), 3.92 (s, 3H), 3.22 (s, 3H), 3.20 (s, 3H), 1.99(m, 1H), 0.85-0.79(m, 4H).

Example **11**

6-((6-fluoropyridin-2-yl)amino)-N-methoxy-4-((2-(N-methyl methanesulfonamido)-4-(trifluoromethyl)phenyl)amino)nicotinamide

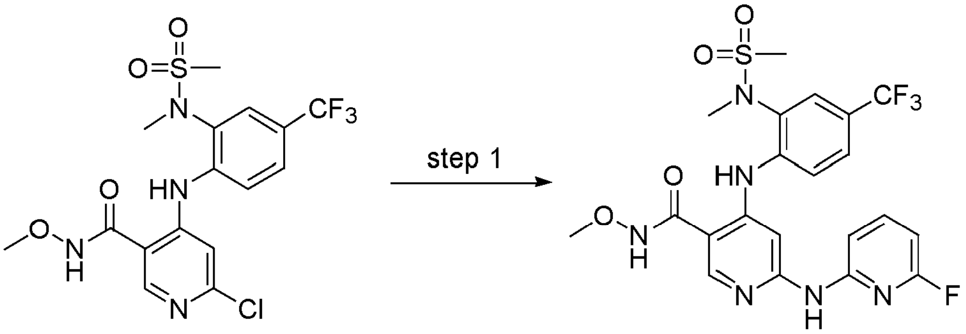


**10-d** **11**

Step 1: 6-chloro-N-methoxy-4-((2-(N-methyl methanesulfonamido)-4-(trifluoromethyl)phenyl)amino)nicotinamide (**10-d,** 50mg, 0.11mmol), 6-fluoro-2-aminopyridine (10.4mg, 0.12mmol), cesium carbonate (108.2mg, 0.33mmol), XantPhos(11mg, 0.02mmol) and $Pd_2(dba)_3$(10mg, 0.01mmol) were added to anhydrous dioxane (5ml) and the mixture was evacuated to vacuum. After atmosphere replacement with nitrogen three times, the reaction mixture was heated to 120°C, stirred for 5h, and filtered by suction. The filtrate was concentrated and purified by high performance preparative thin layer chromatography (MeOH:DCM=1:20) to provide the title compound: 6-((6-fluoropyridin-2-yl)amino)-N-methoxy-4-((2-(N-methyl methanesulfonamido)-4-(trifluoromethyl)phenyl)amino)nicotinamide (**11,** 15 mg, 0.028mmol, 25.8% yield). MS Calcd: 528; MS Found: 529 ($[M+H]^+$). $^1$H NMR (400 MHz, DMSO-$d_6$) : δ 11.84 (s, 1H), 10.46 (s, 1H), 10.16 (s, 1H), 8.38 (s, 1H), 7.92-7.89 (m, 3H), 7.83 (dd, *J* = 16.4, 8.0 Hz, 1H), 7.70 (d, *J* = 8.4 Hz, 1H), 7.45 (d, *J* = 8.0 Hz, 1H), 6.61 (dd, *J* = 8.0, 2.4 Hz, 1H), 3.71(s, 3H), 3.22 (s, 3H), 3.13 (s, 3H).

Example **12**

6-(cyclopropylcarboxamido)-4-((4-cyclopropyl-2-(N-methyl methanesulfonamido)phenyl)amino)-N-methoxy nicotinamide

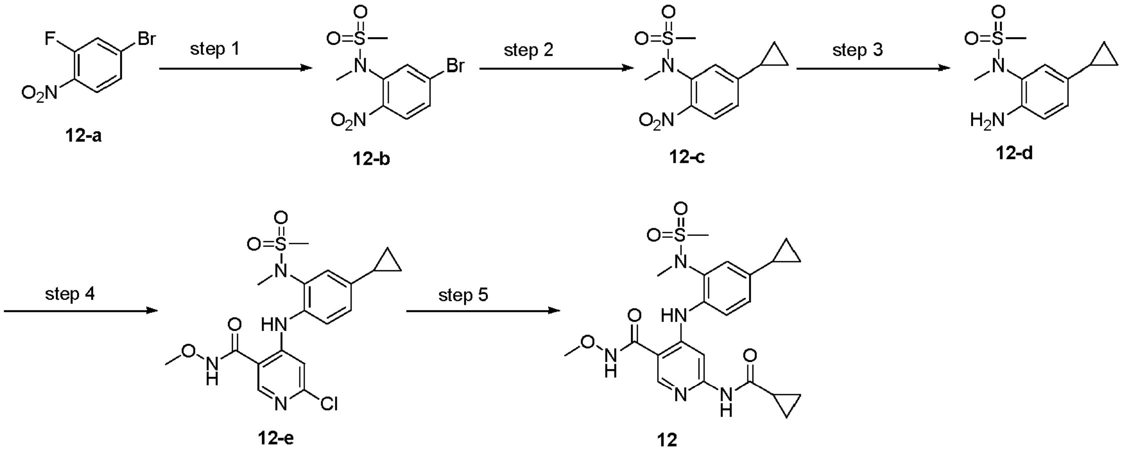

12-a 12-b 12-c 12-d 12-e 12

Step 1: To N-methyl methanesulfonamide (0.6 g, 5.5 mmol) dissolved in 10 mL of N,N-dimethyl formamide was added sodium hydride (0.29g, 7.5 mmol) portionwise. The reaction mixture was heated up to 55°C with continuous stirring for 2 hours, added with 4-bromo-2-fluoro-1-nitrobenzene (**12-a,** 1.1 g, 5.0 mmol), followed by further stirring at the temperature for 2 hours. Upon indication of completed reaction by TLC, the reaction mixture was added with water (40 mL), extracted with ethyl acetate (30 mL x 2). The organic phase was washed with saturated brine (30 mL x 2), dried over anhydrous sodium sulfate, and concentrated under reduced pressure. The residue was separated and purified by silica gel column chromatography (EA:PE=1:5) to provide N-(5-bromo-2-nitrophenyl)-N-methyl methanesulfonamide (**12-b,** 1.6 g, 5.17 mmol, 94% yield), as a pale yellow solid. MS Calcd: 307.95; MS Found: 307.00 ($[M-H]^-$).

Step 2: N-(5-bromo-2-nitrophenyl)-N-methyl methanesulfonamide (**12-b,** 1.6 g, 5.17 mmol), cyclopropyl boronic acid (0.53 g, 6.2 mmol), potassium phosphate (5.27 g, 15.6 mmol) and Pd(dppf)$Cl_2$ (0.38g, 0.52 mmol) were sequentially added to 30 mL of solution of dioxane/water (5/1). The atmosphere of the mixture was evacuated and replaced with nitrogen three times followed by stirring at 110°C for 6 hours. Upon indication of completed reaction by TLC, the reaction mixture was concentrated under reduced pressure. The residue was separated and purified by silica gel column chromatography (EA:PE=1:1) to provide N-(5-cyclopropyl-2-nitrophenyl)-N-methyl methanesulfonamide (**12-c,** 1.1 g, 0.4 mmol, 77.3% yield), as a yellow solid. MS Calcd: 270.07; MS Found: 271.13 ($[M+H]^+$).

Step 3: N-(5-cyclopropyl-2-nitrophenyl)-N-methyl methanesulfonamide (**12-c,** 1 g, 3.7mmol), ammonium chloride (0.98 g, 18 mmol) and iron powder (0.62 g, 11.1 mmol) were sequentially added to 10 mL of mixed solvent of water and ethanol (1:4), and stirred under reflux for 6 hours. Upon TLC indicating a completed reaction, the mixture was filtered by suction, and concentrated. The residue was separated and purified by silica gel column chromatography to provide N-(2-amino-5-cyclopropyl phenyl)-N-methyl methanesulfonamide (**12-d,** 0.72 g, 2.98 mmol, 80.7% yield), as a colorless oil. MS Calcd: 240.09; MS Found: 241.22 ($[M+H]^+$).

Step 4: N-(2-amino-5-cyclopropyl phenyl)-N-methyl methanesulfonamide (**12-d,** 165.8 mg, 0.69 mmol) and 4,6-dichloro-N-methoxy nicotinamide **(int-1,** 150 mg, 0.69 mmol) were added to 10 ml of anhydrous N,N-dimethylacetamide. The mixture was added at room temperature with a solution of LiHMDS in tetrahydrofuran (2.0 ml, 2.0 mmol), and stirred at room temperature for 3 hours. Upon indication of completed reaction by TLC, the mixture was adjusted with aqueous hydrochloride (1N) to pH 5, and extracted with ethyl acetate (20 ml x 3). The combined organic layers were dried, concentrated and then purified by column chromatography (PE:EA =1:1) to provide 6-chloro-4-((4-cyclopropyl-2-(N-methyl methanesulfonamido)phenyl)amino)-N-methoxy nicotinamide (**12-e,** 250 mg, 0.59 mmol, 85.6% yield), as a tan oil. MS Calcd: 424.10; MS Found: 425.29 ($[M+H]^+$).

Step 5: 6-chloro-4-((4-cyclopropyl-2-(N-methyl methanesulfonamido)phenyl)amino)-N-methoxy nicotinamide (**12-e,** 127 mg, 0.3 mmol), cyclopropylcarboxamide (28 mg, 0.33mmol), cesium carbonate (293 mg, 0.9 mmol), XantPhos(34.7mg, 0.06mmol) and $Pd_2(dba)_3$(28.4mg, 0.03 mmol) were added to anhydrous dioxane (5 ml). After the atmosphere of the mixture was evacuated to vacuum and refilled with nitrogen, the reaction mixture was heated to 120°C with stirring for 6 hours, and filtered by suction. The filtrate was concentrated and purified by high performance preparative thin layer chromatography (MeOH:DCM=1:20) to provide the title compound: 6-(cyclopropylcarboxamido)-4-((4-cyclopropyl-2-(N-methyl methanesulfonamido)phenyl)amino)-N-methoxy nicotinamide (**12,** 40mg, 0.085mmol, 28.2% yield). MS Calcd: 473.17; MS Found: 472.36 ($[M-H]^-$). $^1H$ NMR (400 MHz, DMSO-$d_6$) : δ 11.76 (s, 1H), 10.74(s, 1H), 9.789(s, 1H),

8.30 (s, 1H), 7.82(s, 1H), 7.33 (d, $J$ = 8.4 Hz, 1H), 7.26 (d, $J$ = 2.0 Hz, 1H), 7.09 (dd, $J$ = 8.4, 2.0 Hz, 1H), 3.71(s, 3H), 3.12(s, 3H), 3.07(s, 3H), 1.98 - 1.93 (m, 2H), 1.00-0.95(m, 2H)0.77-0.70 (m, 6H).

Example **13**

4-((4-cyclopropyl-2-(N-methyl methanesulfonamido)phenyl)amino)-6-((5-fluoropyridin-2-yl)amino)-N-methoxy nicotinamide

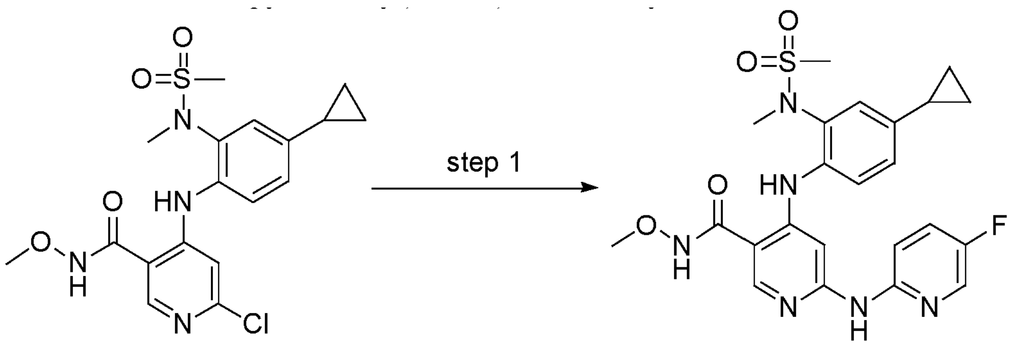


**12-e** **13**

Step 1: 6-chloro-4-((4-cyclopropyl-2-(N-methyl methanesulfonamido)phenyl)amino)-N-methoxy nicotinamide (**12-e,** 127 mg, 0.3 mmol), 5-fluoropyridin-2-ylamine (28 mg, 0.33mmol), cesium carbonate (293 mg, 0.9 mmol), XantPhos (34.7mg, 0.06mmol) and $Pd_2(dba)_3$(28.4mg, 0.03 mmol) were added to anhydrous dioxane (5 ml). After the atmosphere of the mixture was evacuated to vacuum and refilled with nitrogen, the reaction mixture was heated to 120°C with stirring for 6 hours, and filtered by suction. The filtrate was concentrated and purified by high performance preparative thin layer chromatography (MeOH:DCM=1:20) to provide the title compound: 4-((4-cyclopropyl-2-(N-methyl methanesulfonamido) phenyl)amino)-6-((5-fluoropyridin-2-yl)amino)-N-methoxy nicotinamide (**13,** 50mg, 0.085mmol, 33.3% yield). MS Calcd: 500.17; MS Found: 501.36 ([M+H]$^+$). $^1$H NMR (400 MHz, DMSO-$d_6$) : δ 11.67 (s, 1H), 9.92(s, 1H), 9.78(s, 1H), 8.27 (s, 1H), 8.15(d, $J$ = 7.2 Hz, 1H), 7.67-7.64(m, 2H), 7.50 (s, 1H), 7.46 (d, $J$ = 8.4 Hz, 1H), 7.27 (d, $J$ = 2.0 Hz, 1H), 7.16 (dd, $J$ = 8.4, 2.0 Hz, 1H), 3.70 (s, 3H), 3.14 (s, 3H)3.10 (s, 3H), 2.01-1.97 (m, 1H), 1.01-0.97 (m, 2H), 0.74-0.70 (m, 2H).

Example **14**

4-((4-cyclopropyl-2-(N-methyl methanesulfonamido)phenyl)amino)-6-((6-fluoropyridin-2-yl)amino)-N-methoxy nicotinamide

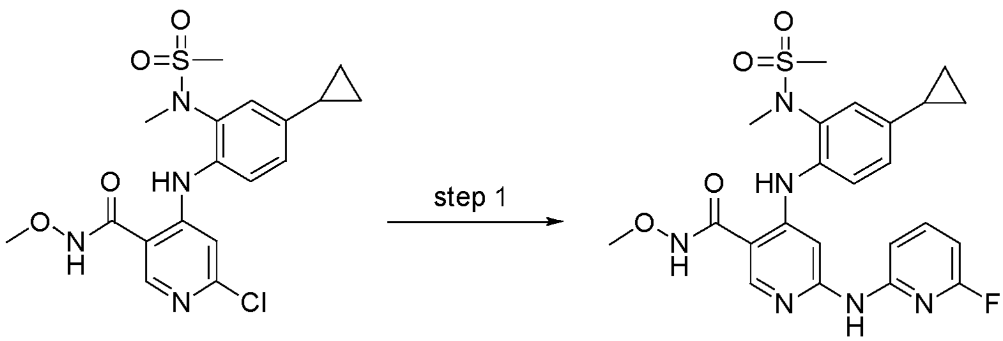


**12-e** **14**

Step 1: 6-chloro-4-((4-cyclopropyl-2-(N-methyl methanesulfonamido)phenyl)amino)-N-methoxy nicotinamide (**12-e,** 127 mg, 0.3 mmol), 6-fluoropyridin-2-ylamine (28 mg, 0.33mmol), cesium carbonate (293 mg, 0.9 mmol), XantPhos(34.7mg, 0.06mmol) and $Pd_2(dba)_3$(28.4mg, 0.03 mmol) were added to anhydrous dioxane (5 ml). After the atmosphere of the mixture was evacuated to vacuum and refilled with nitrogen, the reaction mixture was heated to 120°C

with stirring for 6 hours, and filtered by suction. The filtrate was concentrated and purified by high performance preparative thin layer chromatography (MeOH:DCM=1:20) to provide the title compound: 4-((4-cyclopropyl-2-(N-methyl methanesulfonamido)phenyl)amino)-6-((6-fluoropyridin-2-yl)amino)-N-methoxy nicotinamide (**14,** 45mg, 0.085mmol, 30% yield). MS Calcd: 500.17; MS Found: 501.36 ([M+H]$^+$). $^1$H NMR (400 MHz, DMSO-$d_6$) : δ 11.69 (s, 1H), 9.96(s, 1H), 9.93(s, 1H), 8.30 (s, 1H), 7.79 (dd, *J* = 16.8, 8.4 Hz, 1H), 7.76(s, 1H), 7.54-7.48 (m, 1H), 7.46-7.44 (m, 1H), 7.28 (s, 1H), 7.08 (d, *J* = 8.0 Hz, 1H), 6.56-6.54 (m, 1H), 3.70 (s, 3H), 3.14 (s, 3H)3.07 (s, 3H), 2.01-1.97 (m, 1H), 1.01-0.97 (m, 2H), 0.74-0.70 (m, 2H).

Example **15**

4-((4-cyclopropyl-2-(N-methyl methanesulfonamido)phenyl)amino)-6-((4-fluorophenyl)amino)-N-methoxy nicotinamide

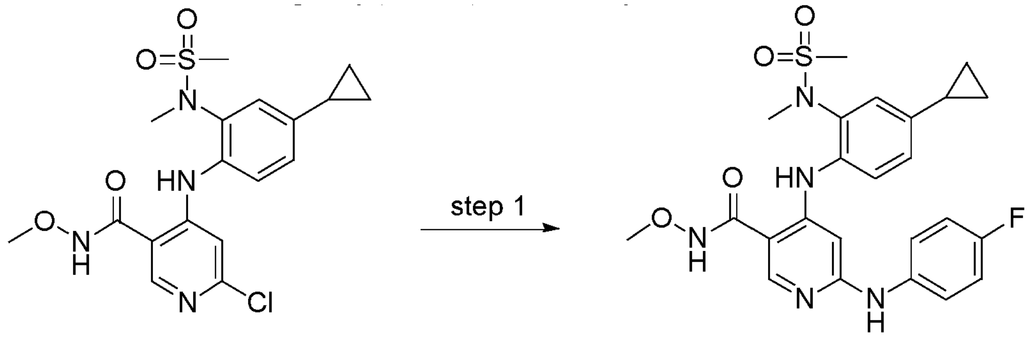


12-e 15

Step 1: 6-chloro-4-((4-cyclopropyl-2-(N-methyl methanesulfonamido)phenyl)amino)-N-methoxy nicotinamide (**15-e,** 100 mg, 0.24 mmol), 4-fluoroaniline (31.5 mg, 0.28mmol), cesium carbonate (230 mg, 0.71 mmol), Xant-Phos(27.3mg, 0.047mmol) and $Pd_2(dba)_3$(23.08mg, 0.023 mmol) were added to anhydrous dioxane (5 ml). After the atmosphere of the mixture was evacuated to vacuum and refilled with nitrogen, the reaction mixture was heated to 120°C with stirring for 6 hours, and filtered by suction. The filtrate was concentrated and purified by high performance preparative thin layer chromatography (MeOH:DCM=1:20) to provide the title compound: 4-((4-cyclopropyl-2-(N-methyl methanesulfonamido)phenyl)amino)-6-((4-fluorophenyl)amino)-N-methoxy nicotinamide (**15,** 40mg, 0.08mmol, 33.3% yield). MS Calcd: 499.17; MS Found: 500.16 ([M+H]$^+$). $^1$H NMR (400 MHz, DMSO-$d_6$) : δ 11.60 (s, 1H), 9.67(s, 1H), 9.04(s, 1H), 8.22 (s, 1H), 7.59-7.57(m, 2H), 7.37 (d, *J* = 8.4 Hz, 1H), 7.27 (d, *J* = 2.0 Hz, 1H), 7.12-7.05 (m, 3H), 6.28 (s, 1H), 3.77 (s, 3H), 3.14 (s, 3H)3.10 (s, 3H), 2.01-1.97 (m, 1H), 1.01-0.97 (m, 2H), 0.74-0.70 (m, 2H).

Example **16**

4-((4-cyclopropyl-2-(N-methyl methanesulfonamido)phenyl)amino)-N-methoxy-6-((5-methoxy pyridin-2-yl)amino)nicotinamide

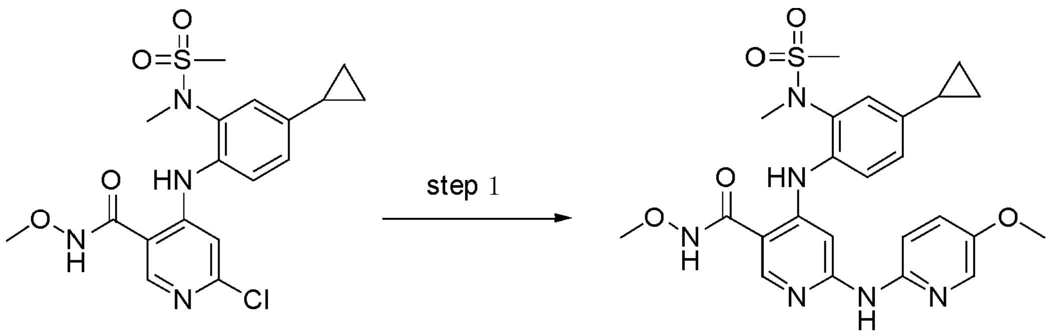


12-e 16

Step 1: 6-chloro-4-((4-cyclopropyl-2-(N-methyl methanesulfonamido)phenyl)amino)-N-methoxy nicotinamide (**12-e,** 80 mg, 0.189 mmol), 5-methoxy pyridin-2-ylamine (25.8 mg, 0.208mmol), cesium carbonate (184.3 mg, 0.567

mmol), XantPhos(21.97mg, 0.038mmol) and $Pd_2(dba)_3$(18.58mg, 0.019 mmol) were added to anhydrous dioxane (5 ml). After the atmosphere of the mixture was evacuated to vacuum and refilled with nitrogen, the reaction mixture was heated to 120°C with stirring for 6 hours, and filtered by suction. The filtrate was concentrated and purified by high performance preparative thin layer chromatography (MeOH:DCM=1:20) to provide the title compound: 4-((4-cyclopropyl-2-(N-methyl methanesulfonamido)phenyl)amino)-N-methoxy-6-((5-methoxy pyridin-2-yl)amino)nicotinamide (**16,** 25mg, 0.049mmol, 25.8% yield). MS Calcd: 512.18; MS Found: 513.22 ($[M+H]^+$). $^1H$ NMR (400 MHz, DMSO-$d_6$) : δ 11.61 (s, 1H), 9.90 (s, 1H), 9.51(s, 1H), 8.25 (s, 1H), 7.87 (d, *J* = 2.8 Hz, 1H), 7.56 (d, *J* = 8.8 Hz, 1H), 7.47-7.44 (m, 2H), 7.35 (dd, *J* = 8.8, 2.8 Hz, 1H), 7.26 (d, *J* = 2.4 Hz, 1H), 7.15-7.13 (m, 1H), 3.77 (s, 3H), 3.70 (s, 3H), 3.14 (s, 3H), 3.10 (s, 3H), 2.01-1.97 (m, 1H), 1.01-0.97(m, 2H), 0.74-0.70 (m, 2H).

Example **17**

6-((4-fluorophenyl)amino)-N-methoxy-4-((2-(N-methyl methanesulfonamido)pyridin-3-yl)amino)nicotinamide

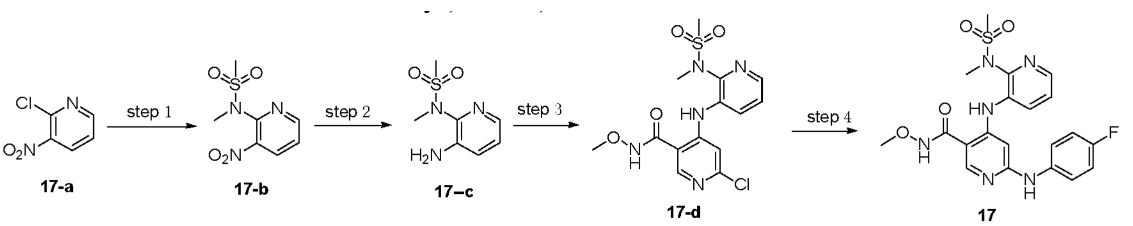


17-a 17-b 17–c 17-d 17

Step 1: N-methyl methanesulfonamide (0.48 g, 4.4 mmol) was dissolved in 10 mL of N,N-dimethyl formamide, added with sodium hydride (0.24g, 6 mmol) portionwise. After that, the reaction mixture was heated to 55°C with continuous stirring for 2 hours, added with 2-chloro-3-nitropyridine (**17-a,** 0.632 g, 4.0 mmol), followed by further stirring at the temperature for 2 hours. Upon indication of completed reaction by TLC, the reaction mixture was added with water (40 mL), and extracted with ethyl acetate (30 mL x 2). The organic phase was washed with saturated brine (30 mL x 2), dried over anhydrous sodium sulfate, and concentrated under reduced pressure. The residue was separated and purified by silica gel column chromatography (EA:PE=1:5) to provide N-methyl-N-(3-nitropyridin-2-yl) methanesulfonamide (**17-b,** 572 mg, 2.48 mmol, 61.9% yield), as a pale yellow solid. MS Calcd: 231.07; MS Found: 230.20 ($[M-H]^-$).

Step 2: N-methyl-N-(3-nitropyridin-2-yl) methanesulfonamide (**17-b,** 572mg, 2.48mmol), ammonium chloride (0.67 g, 12.4 mmol) and iron powder (0.42 g, 7.44mmol) were sequentially added to 10 mL of mixed solvent of water and ethanol (1:4), and stirred under reflux for 6 hours. Upon indication of completed reaction by TLC, the reaction mixture was filtered by suction. The filtrate was concentrated, and the residue was separated and purified by silica gel column chromatography to provide N-(3-aminopyridin-2-yl)-N-methyl methanesulfonamide (**17-c,** 0.41 g, 2.19 mmol, 88.4% yield), as a colorless oil. MS Calcd: 201.06; MS Found: 202.06 ($[M+H]^+$).

Step 3: N-(3-aminopyridin-2-yl)-N-methyl methanesulfonamide (**17-c,** 150 mg, 0.80 mmol) and 4,6-dichloro-N-methoxy nicotinamide **(int-1,** 174.8 mg, 0.80 mmol) were added to 5 ml of anhydrous N,N-dimethylacetamide. The mixture was added at room temperature with a solution of LiHMDS in tetrahydrofuran (2.4 ml, 2.4 mmol), and stirred at room temperature for 3 hours. Upon indication of completed reaction by TLC, the mixture was adjusted with aqueous hydrochloride (1N) to pH 5, and extracted with ethyl acetate (20 ml x 3). The combined organic layers were dried, concentrated and then purified by column chromatography (PE:EA =1:1) to provide 6-chloro-N-methoxy-4-((2-(N-methyl methanesulfonamido)pyridin-3-yl)amino)nicotinamide **(17-d,** 180 mg, 0.467 mmol, 58.4% yield). MS Calcd: 385.06; MS Found: 386.22 ($[M+H]^+$). Step 4: 6-chloro-N-methoxy-4-((2-(N-methyl methanesulfonamido)pyridin-3-yl)amino)nicotinamide (**17-d,** 53 mg, 0.13 mmol), 4-fluoroaniline (18.4 mg, 0.16mmol), cesium carbonate (135mg, 0.41 mmol), XantPhos(16.2mg, 0.028mmol) and $Pd_2(dba)_3$(13.7mg, 0.014 mmol) were added to anhydrous dioxane (5 ml). After the atmosphere of the mixture was evacuated to vacuum and refilled with nitrogen, the reaction mixture was heated to 120°C with stirring for 6 hours, and filtered by suction. The filtrate was concentrated and purified by high performance preparative thin layer chromatography (MeOH:DCM=1:20) to provide the title compound: 6-((4-fluorophenyl)amino)-N-methoxy-4-((2-(N-methyl methanesulfonamido)pyridin-3-yl)amino)nicotinamide (17, 15mg, 0.032mmol, 25% yield). MS Calcd: 460.13; MS Found: 459.2 ($[M-H]^-$). $^1H$ NMR (400 MHz, DMSO-$d_6$) : δ 11.67 (s, 1H), 9.99(s, 1H), 9.13(s, 1H), 8.27 (s, 1H), 8.24 (dd, *J* = 8.4, 1.6 Hz, 1H), 8.02 (dd, *J* = 7.2, 1.6 Hz, 1H), 7.61-7.57 (m, 2H), 7.48 (dd, *J* = 8.4, 4.8 Hz, 1H), 7.12-7.09 (m, 2H), 6.44 (s, 1H), 3.71 (s, 3H), 3.20 (s, 3H), 3.15 (s, 3H).

Example **18**

4-((4-ethynyl-2-(N-methyl methanesulfonamido)phenyl)amino)-N-methoxy-6-((5-methoxy pyridin-2-yl)amino)nicotinamide

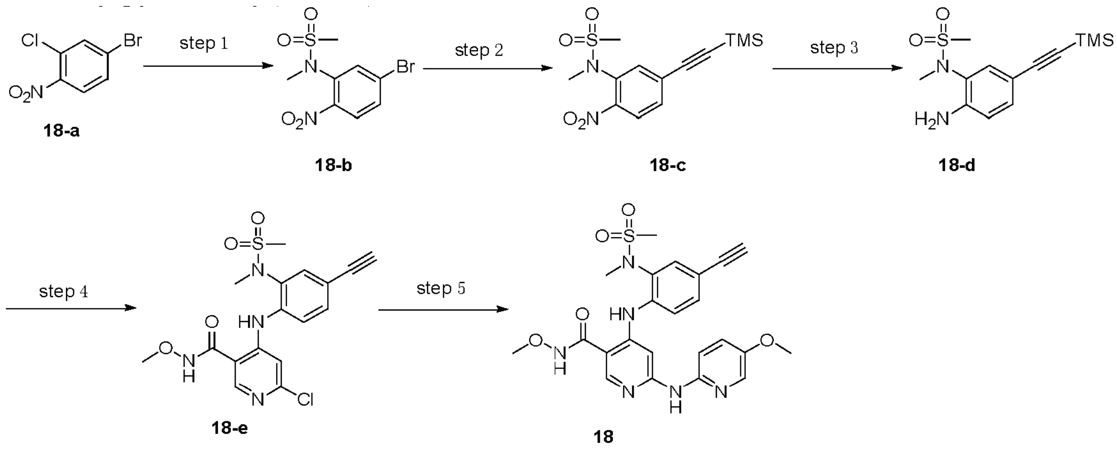


18-a 18-b 18-c 18-d 18-e 18

Step 1: N-methyl methanesulfonamide (0.6 g, 5.5 mmol) dissolved in 10 mL of N,N-dimethyl formamide was added with sodium hydride (0.29g, 7.5 mmol) portionwise. After that, the reaction mixture was heated to 55°C with continuous stirring for 2 hours, added with 4-bromo-2-fluoro-1-nitrobenzene **(18-a,** 1.1 g, 5.0 mmol), followed by further stirring at the temperature for 2 hours. Upon indication of completed reaction by TLC, the reaction mixture was added with water (40 mL), extracted with ethyl acetate (30 mL x 2). The organic phase was washed with saturated brine (30 mL x 2), dried over anhydrous sodium sulfate, and concentrated under reduced pressure. The residue was separated and purified by silica gel column chromatography (EA:PE=1:5) to provide N-(5-bromo-2-nitrophenyl)-N-methyl methanesulfonamide (**18-b,** 1.6 g, 5.17 mmol, 94% yield), as a pale yellow solid. MS Calcd: 307.95; MS Found: 307.00 ($[M-H]^-$).

Step 2: N-(5-bromo-2-nitrophenyl)-N-methyl methanesulfonamide (**18-b,** 0.45 g, 1.46 mmol), ethynyl trimethylsilane (0.43g, 4.38 mmol), cuprous (I) iodide (23.04mg, 0.15 mmol) and $Pd(dppf)Cl_2$ (109.7mg, 0.15 mmol) were sequentially added to 5ml of triethylamine. The atmosphere of the mixture was evacuated and replaced with nitrogen three times, followed by stirring at 60°C for 4 hours. Upon indication of completed reaction by TLC, the mixture was concentrated under reduced pressure. The residue was separated and purified by silica gel column chromatography (EA:PE=1:1) to provide N-methyl-N-(2-nitro-5-((trimethylsilyl)ethynyl)phenyl) methanesulfonamide**(18-c,** 0.375g, 1.07mmol, 73.2% yield), as a yellow solid. MS Calcd: 326.08; MS Found: 327.10 ($[M+H]^-$).

Step 3: N-methyl-N-(2-nitro-5-((trimethylsilyl)ethynyl)phenyl) methanesulfonamide (**18-c,** 0.3375 g, 1.07mmol), ammonium chloride (0.322g, 5.75 mmol) and iron powder (0.62 g, 11.1 mmol) were sequentially added to 10 mL of mixed solvent of water and ethanol (1:4), stirred under reflux for 6 hours. Upon indication of completed reaction by TLC, the reaction mixture was filtered by suction. The filtrate was concentrated, and the residue was separated and purified by silica gel column chromatography to provide N-(2-amino-5-((trimethylsilyl)ethynyl)phenyl)-N-methyl methanesulfonamide (**18-d,** 0.307 g, 1.03 mmol, 96.2% yield), as a colorless oil. MS Calcd: 296.09; MS Found: 297.22 ($[M+H]^+$).

Step 4: N-(2-amino-5-((trimethylsilyl)ethynyl)phenyl)-N-methyl methanesulfonamide (**18-d,** 307 mg, 1.03 mmol) and 4,6-dichloro-N-methoxy nicotinamide **(int-1,** 224.5 mg, 1.03 mmol) were added to 10 ml of anhydrous N,N-dimethylacetamide. The mixture was added at room temperature with a solution of LiHMDS in tetrahydrofuran (3.1 ml, 3.09 mmol), and stirred at room temperature for 3 hours. Upon indication of completed reaction by TLC, the mixture was adjusted with aqueous hydrochloride (1N) to pH 5, and extracted with ethyl acetate (20 ml x 3). The combined organic layers were dried, concentrated and then purified by column chromatography (PE:EA =1:1) to provide 6-chloro-4-((4-ethynyl-2-(N-methyl methanesulfonamido)phenyl)amino)-N-methoxy nicotinamide (**18-e,** 197mg, 0.48 mmol, 46.8% yield), as a tan oil. MS Calcd: 408.10; MS Found: 409.29 ($[M+H]^+$).

Step 5: 6-chloro-4-((4-ethynyl-2-(N-methyl methanesulfonamido)phenyl)amino)-N-methoxy nicotinamide (**18-e,** 100 mg, 0.245 mmol), 5-methoxy pyridin-2-ylamine (33.43 mg, 0.27mmol), cesium carbonate (239 mg, 0.74 mmol), XantPhos(28.9mg, 0.05mmol) and $Pd_2(dba)_3$(24.45mg, 0.025 mmol) were added to anhydrous dioxane (5 ml). After the atmosphere of the mixture was evacuated to vacuum and refilled with nitrogen, the reaction mixture was heated to 120°C with stirring for 6 hours, and filtered by suction. The filtrate was concentrated and purified by high performance preparative thin layer chromatography (MeOH:DCM=1:20) to provide the title compound: 4-((4-ethynyl-2-(N-methyl methanesulfonamido)phenyl)amino)-N-methoxy-6-((5-methoxy pyridin-2-yl)amino)nicotinamide (**18,** 10mg, 0.02mmol, 8.2% yield). MS Calcd: 496.15; MS Found: 497.20 ($[M+H]^+$). $^1H$ NMR (400 MHz, DMSO-$d_6$) : δ 11.71 (s, 1H), 10.32 (s, 1H), 9.67 (s, 1H), 8.31 (s, 1H), 7.95 (d, *J* = 2.8 Hz, 1H), 7.87 (m, 1H), 7.66-7.64 (m, 2H), 7.59-7.54 (m, 2H), 7.37 (dd, *J* = 8.8, 2.8 Hz, 1H), 4.23

(s, 1H), 3.79 (s, 3H), 3.71 (s, 3H), 3.18 (s, 6H).

Example **19**

4-((4-ethynyl-2-(N-methyl methanesulfonamido)phenyl)amino)-6-(((6-fluoropyridin-2-yl)amino)-N-methoxy nicotinamide

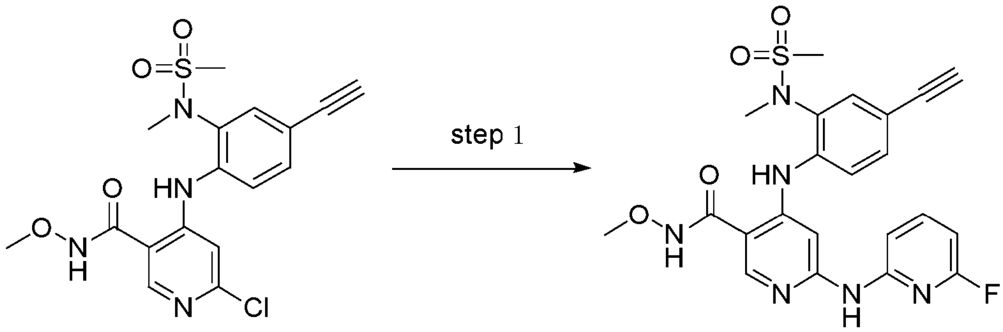


**18-e** **19**

Step 1: 6-chloro-4-((4-ethynyl-2-(N-methyl methanesulfonamido)phenyl)amino)-N-methoxy nicotinamide (**18-e,** 90 mg, 0.22 mmol), 6-fluoropyridin-2-ylamine (27.13 mg, 0.24mmol), cesium carbonate (215 mg, 0.66 mmol), XantPhos(25.4mg, 0.04mmol) and $Pd_2(dba)_3$(21.5mg, 0.022 mmol) were added to anhydrous dioxane (5 ml). After the atmosphere of the mixture was evacuated to vacuum and refilled with nitrogen, the reaction mixture was heated to 120°C with stirring for 6 hours, and filtered by suction. The filtrate was concentrated and purified by high performance preparative thin layer chromatography (MeOH:DCM=1:20) to provide the title compound: 4-((4-ethynyl-2-(N-methyl methanesulfonamido)phenyl)amino)-6-(((6-fluoropyridin-2-yl)amino)-N-methoxy nicotinamide (**19,** 5mg, 0.01mmol, 4.7% yield). MS Calcd: 484.13; MS Found: 485.20 ($[M+H]^+$). $^1H$ NMR (400 MHz, DMSO-$d_6$) : δ 11.78 (s, 1H), 10.30(s, 1H), 10.08 (s, 1H), 8.35 (s, 1H), 7.84-7.78 (m, 1H), 7.70-7.66 (m, 1H), 7.47-7.45 (m, 2H), 7.40-7.29 (m, 2H), 6.58 (d, *J* = 8.0 Hz 1H), 4.21 (s, 1H), 3.71 (s, 3H), 3.17 (s, 3H), 3.16 (s, 3H).

Example **20**

4-((4-cyclopropyl-2-(N-methyl methanesulfonamido)phenyl)amino)-N-ethoxy-6-((5-methoxy pyridin-2-yl)amino)nicotinamide

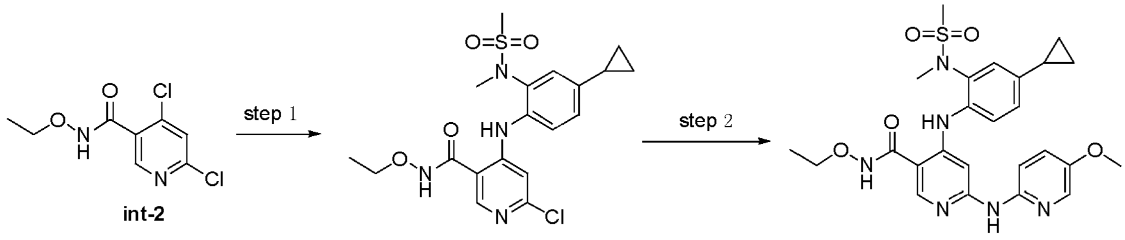


**int-2** **20-a** **20**

Step 1:N-(2-amino-5-cyclopropyl phenyl)-N-methyl methanesulfonamide (**18-b,** 240 mg, 1.0mmol) and 4,6-dichloro-N-ethoxy nicotinamide (**int-2,** 234 mg, 1.0 mmol) were added to 10 ml of anhydrous N,N-dimethylacetamide. The mixture was added at room temperature with a solution of LiHMDS in tetrahydrofuran (3 ml, 3.0 mmol), and stirred at room temperature for 3 hours. Upon indication of completed reaction by TLC, the mixture was adjusted with aqueous hydrochloride (1N) to pH 5, and extracted with ethyl acetate (20 ml x 3). The combined organic layers were dried, concentrated and then purified by column chromatography (PE:EA =1:1) to provide 6-chloro-4-((4-cyclopropyl-2-(N-methyl methanesulfonamido)phenyl)amino)-N-ethoxy nicotinamide (**20-a,** 325mg, 0.74 mmol, 74.2% yield). MS Calcd: 438; MS Found: 439 ($[M+H]^+$).

Step 2: 6-chloro-4-((4-cyclopropyl-2-(N-methyl methanesulfonamido)phenyl)amino)-N-ethoxy nicotinamide

(33169-c, 87 mg, 0.2 mmol), 5-methoxy pyridin-2-ylamine (29.76 mg, 0.24mmol), cesium carbonate (215 mg, 0.66 mmol), XantPhos(25.4mg, 0.04mmol) and $Pd_2(dba)_3$(21.5mg, 0.022 mmol) were added to anhydrous dioxane (5 ml). After the atmosphere of the mixture was evacuated to vacuum and refilled with nitrogen, the reaction mixture was heated to 120°C with stirring for 6 hours, and filtered by suction. The filtrate was concentrated and purified by high performance preparative thin layer chromatography (MeOH:DCM=1:20) to provide the title compound: 4-((4-cyclopropyl-2-(N-methyl methanesulfonamido)phenyl)amino)-N-ethoxy-6-((5-methoxy pyridin-2-yl)amino)nicotinamide (**20,** 20mg, 0.038mmol, 19% yield). MS Calcd: 526.20; MS Found: 527.20 ($[M+H]^-$).

$^1$H NMR (400 MHz, DMSO-$d_6$) : δ 9.86 (s, 1H), 9.50(s, 1H), 8.27 (s, 1H), 7.87 (d, *J* = 3.2 Hz, 1H), 7.58 (d, *J* = 9.2 Hz, 1H), 7.46-7.44 (m, 2H), 7.36 (dd, *J* = 8.8, 3.2 Hz, 1H), 7.26 (d, *J* = 2.0 Hz, 1H), 7.14 (d, *J* =8.4Hz, 1H), 3.93 (q, *J* = 7.2 Hz, 2H), 3.77 (s, 3H), 3.18 - 3.08 (m, 7H), 2.01-1.95 (m, 1H), 1.227 (t, *J* = 7.2 Hz, 3H), 1.01-0.96 (m, 2H), 0.74-0.70 (m, 2H).

Example **21**

4-((4-cyclopropyl-2-(N-methyl methanesulfonamido)phenyl)amino)-6-((2,6-dimethyl pyrimidin-4-yl)amino)-N-ethoxy nicotinamide

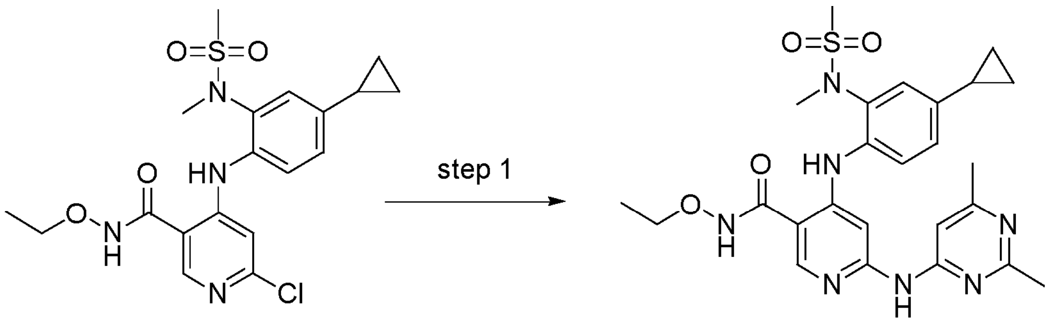


**20-a** **21**

Step 1: 6-chloro-4-((4-cyclopropyl-2-(N-methyl methanesulfonamido)phenyl)amino)-N-ethoxy nicotinamide (**20-a,** 87 mg, 0.2 mmol), 2,6-dimethyl pyrimidin-4-ylamine (29.7 mg, 0.24mmol), cesium carbonate (215 mg, 0.66 mmol), XantPhos(25.4mg, 0.04mmol) and $Pd_2(dba)_3$ (21.5mg, 0.022 mmol) were added to anhydrous dioxane (5 ml). After the atmosphere of the mixture was evacuated to vacuum and refilled with nitrogen, the reaction mixture was heated to 120°C with stirring for 6 hours, and filtered by suction. The filtrate was concentrated and purified by high performance preparative thin layer chromatography (MeOH:DCM=1:20) to provide the title compound: 4-((4-cyclopropyl-2-(N-methyl methanesulfonamido)phenyl)amino)-6-((2,6-dimethyl pyrimidin-4-yl)amino)-N-ethoxy nicotinamide (**21,** 25mg, 0.047mmol, 23.8% yield). MS Calcd: 525.20; MS Found: 526.20 ($[M+H]^+$). $^1$H NMR (400 MHz, DMSO-$d_6$) : δ 11.59 (s, 1H), 9.99(s, 1H), 9.85 (s, 1H), 8.32 (s, 1H), 7.82(s, 1H), 7.45 (d, *J* = 8.4 Hz, 1H), 7.31 (d, *J* = 2.4 Hz, 1H), 7.11 (dd, *J* = 8.4, 2.0 Hz, 1H), 7.04 (s, 1H), 3.94 (q, *J* = 7.2 Hz, 2H), 3.13 (s, 3H), 3.09 (s, 3H), 2.3 1 (s, 3H), 2.26 (s, 3H), 1.98-1.94 (m, 1H), 1.22 (t, *J* = 7.2 Hz, 3H), 0.99-0.95 (m, 2H), 0.72-0.698 (m.2H).

Example **22**

4-((4-cyclopropyl-2-(N-methyl methanesulfonamido)phenyl)amino)-N-ethoxy-6-((6-fluoropyridin-2-yl)amino)nicotinamide

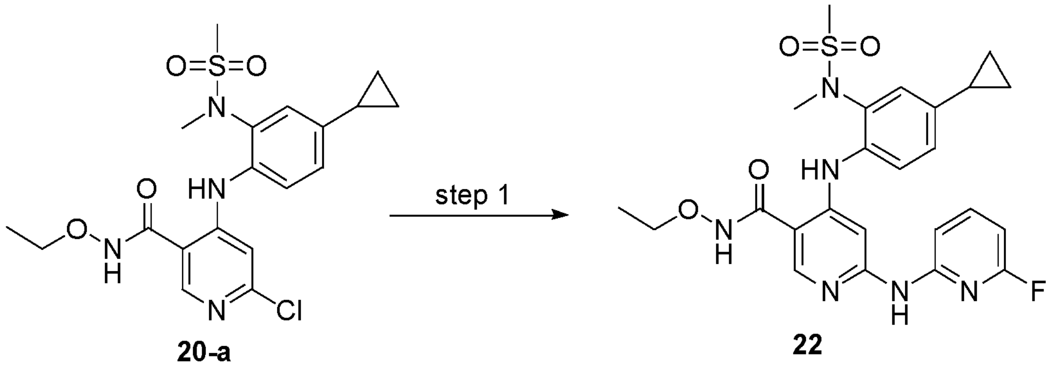


20-a

22

Step 1: 6-chloro-4-((4-cyclopropyl-2-(N-methyl methanesulfonamido)phenyl)amino)-N-ethoxy nicotinamide (**20-a,** 87 mg, 0.2 mmol), 6-fluoropyridin-2-ylamine (26.9 mg, 0.24mmol), cesium carbonate (215 mg, 0.66 mmol), XantPhos(25.4mg, 0.04mmol) and $Pd_2(dba)_3$(21.5mg, 0.022 mmol) were added to anhydrous dioxane (5 ml). After the atmosphere of the mixture was evacuated to vacuum and refilled with nitrogen, the reaction mixture was heated to 120°C with stirring for 6 hours, and filtered by suction. The filtrate was concentrated and purified by high performance preparative thin layer chromatography (MeOH:DCM=1:20) to provide the title compound: 4-((4-cyclopropyl-2-(N-methyl methanesulfonamido)phenyl)amino)-N-ethoxy-6-((6-fluoropyridin-2-yl)amino)nicotinamide (**22,** 20mg, 0.039mmol, 19.4% yield). MS Calcd: 514.18; MS Found: 515.20 ($[M+H]^+$). $^1$H NMR (400 MHz, DMSO-$d_6$) : $\delta$ 11.10 (s, 1H), 9.94 (s, 1H), 9.85 (s, 1H), 8.38 (s, 1H), 7.78 (dd, *J* = 16.4, 8.4 Hz, 1H), 7.51-7.44 (m, 3H), 7.27 (d, *J* = 2.0 Hz, 1H), 7.09-7.06 (m, 1H), 6.64 (dd, *J* = 8.0, 2.8 Hz, 1H), 3.93 (q, *J* = 7.2 Hz, 2H), 3.13 (s, 3H), 3.07 (s, 3H), 1.96-1.94 (m, 1H), 1.21 (t, *J* = 7.2 Hz, 3H), 1.00-0.95 (m, 2H), 0.72-0.698 (m.2H).

Example **23**

4-((4-cyclopropyl-2-(N-methyl methanesulfonamido)phenyl)amino)-6-((6-fluoropyridin-2-yl)amino)-N-isopropoxy nicotinamide

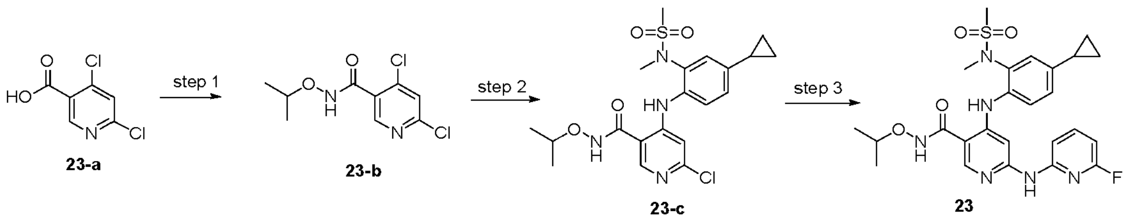


23-a 23-b 23-c 23

Step 1:1) 4,6-dichloronicotinic acid**(23-a,** 288mg, 1.5mmol) dissolved in dichloromethane (10ml) was added to a 50ml two-necked flask, the reaction mixture was cooled with ice-water bath to 0°C~5°C, added with catalytic amount of DMF(0.1ml) dropwise followed by carefully adding oxalyl chloride (229mg, 1.8mmol) dropwise, stirred at room temperature for 30min. Upon indication of completed reaction by TLC, the reaction mixture was concentrated at 40°C under reduced pressure, and the resulting crude product was added to dichloromethane (20ml) and further concentrated under reduced pressure.

2) Hydrochloride salt of isoproxylamine (200.7g, 1.8mmol) was added to a mixture of ethyl acetate/water (40ml) (5:1), added with potassium carbonate (828mg, 6mmol), and stirred at room temperature for 10 min. To which was carefully added the crude material from the previous step dissolved in dichloromethane (5ml) dropwise, followed by stirring at room temperature overnight. After phase separation, the organic phase was concentrated, purified by column chromatography (petroleum ether/ethyl acetate=2:1) to provide the desired product 4,6-dichloro-N-isopropoxy nicotinamide (**23-b,** 350mg, 1.42mmol, 94.8% yield). MS Calcd: 248; MS Found: 249 ($[M+H]^+$).

Step 2: N-(2-amino-5-cyclopropyl phenyl)-N-methyl methanesulfonamide (**23-b,** 120 mg, 0.5mmol) and 4,6-dichloro-N-isopropoxy nicotinamide (123 mg, 0.5 mmol) were added to 5 ml of anhydrous N,N-dimethylacetamide. The mixture was added at room temperature with a solution of LiHMDS in tetrahydrofuran (1.5 ml, 1.5 mmol), and stirred at room temperature for 3 hours. Upon indication of completed reaction by TLC, the mixture was adjusted with aqueous hydrochloride (1N) to pH 5, and extracted with ethyl acetate (20 ml x 3). The combined organic layers were dried, concentrated and then purified by column chromatography (PE:EA =1:1) to provide 6-chloro-4-((4-cyclopropyl-2-(N-methyl methanesulfonamido)phenyl)amino)-N-isopropoxy nicotinamide (**23-c,** 170mg, 0.37 mmol, 75.2% yield), as a tan oil. MS Calcd: 452; MS Found: 453 ([M+H]+).

Step 3: 6-chloro-4-((4-cyclopropyl-2-(N-methyl methanesulfonamido)phenyl)amino)-N-isopropoxy nicotinamide (**23-c,** 71 mg, 0.16 mmol), 6-fluoropyridin-2-ylamine (21.18 mg, 0.19mmol), cesium carbonate (153 mg, 0.47 mmol), XantPhos(18.5mg, 0.03mmol) and $Pd_2(dba)_3$(15.2mg, 0.015 mmol) were added to anhydrous dioxane (5 ml). After the atmosphere of the mixture was evacuated to vacuum and refilled with nitrogen, the reaction mixture was heated to 120°C with stirring for 6 hours, and filtered by suction. The filtrate was concentrated and purified by high performance preparative thin layer chromatography (MeOH:DCM=1:20) to provide the title compound: 4-((4-cyclopropyl-2-(N-methyl methanesulfonamido)phenyl)amino)-6-((6-fluoropyridin-2-yl)amino)-N-isopropoxy nicotinamide **(23,** 15mg, 0.028mmol, 17.8% yield). MS Calcd: 528; MS Found: 529 ([M+H]+). 1H NMR (400 MHz, DMSO-$d_6$) : δ 11.10 (s, 1H), 9.94(s, 1H), 9.85 (s, 1H), 8.33 (s, 1H), 7.78 (dd, *J* = 16.4, 8.4 Hz, 1H), 7.50-7.47 (m, 3H), 7.28 (s, 1H), 7.08 (dd, *J* = 8.4, 2.0 Hz, 1H), 6.55 (dd, *J* = 8.0, 2.8 Hz, 1H), 4.14 (q, *J* = 7.0 Hz, 1H), 3.14 (s, 3H), 3.07 (s, 3H), 1.98-1.96 (m, 1H), 1.22 (d, *J* = 7.0 Hz, 6H), 0.99-0.95 (m, 2H), 0.72-0.69 (m.2H).

Example **24**

4-((4-ethynyl-2-(N-methyl methanesulfonamido)phenyl)amino)-6-(((6-fluoropyridin-2-yl)amino)-N-isopropoxy nicotinamide

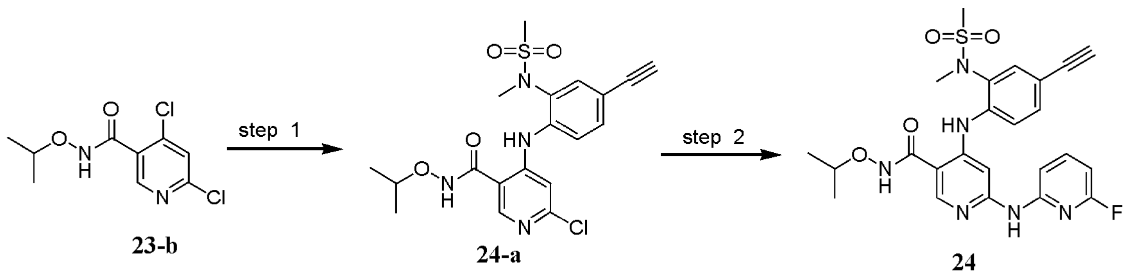


23-b 24-a 24

Step 1: N-(2-amino-5-((trimethylsilyl)ethynyl)phenyl)-N-methyl methanesulfonamide (226 mg, 0.76 mmol) and 4,6-dichloro-N-isopropoxy nicotinamide **(23-b,** 187mg, 0.76 mmol) were added to 5 ml of anhydrous N,N-dimethylacetamide. The mixture was added at room temperature with a solution of LiHMDS in tetrahydrofuran (2.3 ml, 2.28 mmol), and stirred at room temperature for 3 hours. Upon indication of completed reaction by TLC, the mixture was adjusted with aqueous hydrochloride (1N) to pH 5, and extracted with ethyl acetate (20 ml x 3). The combined organic layers were dried, concentrated and then purified by column chromatography (PE:EA =1:1) to provide 6-chloro-4-((4-ethynyl-2-(N-methyl methanesulfonamido)phenyl)amino)-N-isopropoxy nicotinamide **(24-a,** 130mg, 0.298 mmol, 39.2% yield), as a tan oil.

MS Calcd: 436.10; MS Found: 437.29 ([M+H]+).

Step 2: 6-chloro-4-((4-ethynyl-2-(N-methyl methanesulfonamido)phenyl)amino)-N-isopropoxy nicotinamide **(24-a,** 130mg, 0.298 mmol), 6-fluoropyridin-2-ylamine (40.11 mg, 0.36mmol), cesium carbonate (290.6 mg, 0.89 mmol), XantPhos(34.7mg, 0.06mmol) and $Pd_2(dba)_3$(28.41mg, 0.03 mmol) were added to anhydrous dioxane (5 ml). After the atmosphere of the mixture was evacuated to vacuum and refilled with nitrogen, the reaction mixture was heated to 120°C with stirring for 6 hours, and filtered by suction. The filtrate was concentrated and purified by high performance preparative thin layer chromatography (MeOH:DCM=1:20) to provide the title compound: 4-((4-ethynyl-2-(N-methyl methanesulfonamido)phenyl)amino)-6-(((6-fluoropyridin-2-yl)amino)-N-isopropoxy nicotinamide **(24,** 17.2mg, 0.033mmol, 11.2% yield). MS Calcd: 512.16; MS Found: 513.20 ([M+H]+). $^1$H NMR (400 MHz, DMSO-$d_6$) : δ 11.52 (s, 1H), 10.24(s, 1H), 10.05 (s, 1H), 8.35 (s, 1H), 7.82 (dd, *J* = 16.8, 8.0 Hz, 1H), 7.77 (s, 1H), 7.68 (d, *J* = 8.4 Hz, 1H), 7.66 (d, *J* = 2.0 Hz, 1H), 7.48-7.46 (m, 2H), 6.59 (dd, *J* = 8.0, 2.4 Hz, 1H), 4.23 (s, 1H), 4.17-4.11 (m, 1H), 3.19 (s, 3H), 3.17 (s, 3H), 1.22 (d, 3H), 1.21(s, 3H).

Example **25**

6-((2,6-dimethyl pyrimidin-4-yl)amino)-N-ethoxy-4-((2-methoxy-3-(1-methyl-1H-pyrazol-4-yl)phenyl)amino)nicotinamide

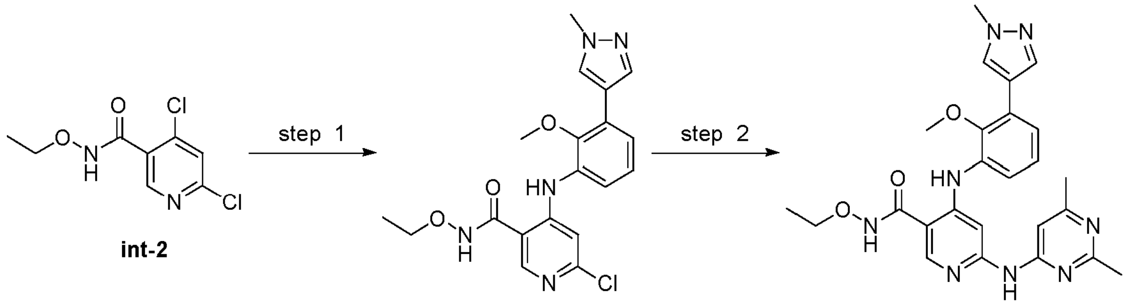


int-2 25-a 25

Step 1: 2-methoxy-3-(1-methyl-1H-pyrazol-4-yl)aniline (203 mg, 1.0mmol) and 4,6-dichloro-N-ethoxy nicotinamide (**int-2,** 234 mg, 1.0 mmol) were added to 10 ml of anhydrous N,N-dimethylacetamide. The mixture was added at room temperature with a solution of LiHMDS in tetrahydrofuran (3 ml, 3.0 mmol), and stirred at room temperature for 3 hours. Upon indication of completed reaction by TLC, the mixture was adjusted with aqueous hydrochloride (1N) to pH 5, and extracted with ethyl acetate (20 ml × 3). The combined organic layers were dried, concentrated and then purified by column chromatography (PE:EA =1:1) to provide 6-chloro-N-ethoxy-4-((2-methoxy-3-(1-methyl-1H-pyrazol-4-yl)phenyl) amino)nicotinamide **(25-a,** 320mg, 0.80 mmol, 80% yield). MS Calcd: 401; MS Found: 402 ($[M+H]^+$).

Step 2: 6-chloro-N-ethoxy-4-((2-methoxy-3-(1-methyl-1H-pyrazol-4-yl)phenyl)amino)nicotinamide **(25-a,** 120 mg, 0.3 mmol), 2,6-dimethyl pyrimidin-4-ylamine (40.59 mg, 0.33mmol), cesium carbonate (292.5 mg, 0.9mmol), XantPhos(34.68mg, 0.06mmol) and $Pd_2(dba)_3$(27.47mg, 0.03 mmol) were added to anhydrous dioxane (5 ml). After the atmosphere of the mixture was evacuated to vacuum and refilled with nitrogen, the reaction mixture was heated to 120°C with stirring for 6 hours, and filtered by suction. The filtrate was concentrated and purified by high performance preparative thin layer chromatography (MeOH:DCM=1:20) to provide the title compound: 6-(((2,6-dimethyl pyrimidin-4-yl) amino)-N-ethoxy-4-((2-methoxy-3-(1-methyl-1H-pyrazol-4-yl)phenyl)amino)nicotinamide **(25,** 6mg, 0.012mmol, 4% yield). MS Calcd: 488.23; MS Found: 489.20 ($[M+H]^+$). $^1H$ NMR (400 MHz, DMSO-$d_6$) : δ 11.70 (s, 1H), 10.23 (s, 1H), 10.08 (s, 1H), 8.38 (s, 1H), 8.17-8.16 (m, 2H), 7.92 (s, 1H), 7.44 (dd, *J* = 8.0, 1.2 Hz, 1H), 7.38 (dd, *J* = 8.0, 1.6 Hz, 1H), 7.19(t, *J* = 8.0 Hz, 1H), 7.07 (s, 1H), 3.96 (q, *J* = 7.2 Hz, 2H), 3.90 (s, 3H), 3.61 (s, 3H), 2.37 (s, 3H), 2.27 (s, 3H), 1.22 (t, *J* = 7.2 Hz, 3H).

Example **26**

6-(cyclopropylcarboxamido)-N-ethoxy-4-((2-methoxy-3-(1-methyl-1H-pyrazol-4-yl)phenyl)amino)nicotinamide

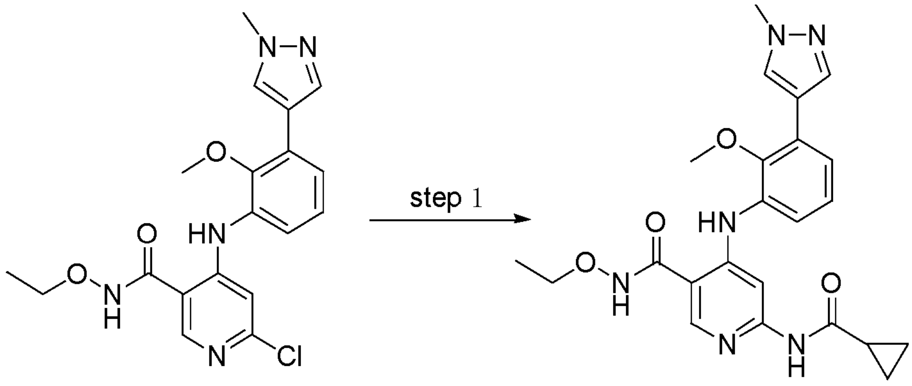


25-a 26

Step 1: 6-chloro-N-ethoxy-4-((2-methoxy-3-(1-methyl-1H-pyrazol-4-yl)phenyl)amino)nicotinamide **(25-a,** 120 mg, 0.3 mmol), cyclopropylcarboxamide (25.5 mg, 0.3mmol), cesium carbonate (292.5 mg, 0.9mmol), XantPhos(34.68mg, 0.06mmol) and $Pd_2(dba)_3$(27.47mg, 0.03 mmol) were added to anhydrous dioxane (5 ml). After the atmosphere of the mixture was evacuated to vacuum and refilled with nitrogen, the reaction mixture was heated to 120°C with stirring for 6 hours, and filtered by suction. The filtrate was concentrated and purified by high performance preparative thin layer chromatography (MeOH:DCM=1:20) to provide the title compound: 6-(cyclopropylcarboxamido)-N-ethoxy-4-((2-methoxy-3-(1-methyl-1H-pyrazol-4-yl)phenyl)amino)nicotinamide **(26,** 40mg, 0.088mmol, 29.5% yield).

MS Calcd: 450.20; MS Found: 451.20([M+H]+). $^1$H NMR (400 MHz, DMSO-$d_6$) : δ 11.76 (s, 1H), 10.79 (s, 1H), 10.14 (s, 1H), 8.38 (s, 1H), 8.16 (s, 1H), 8.06 (s, 1H), 7.91 (s, 1H), 7.36 (dd, *J* = 8.0, 1.6 Hz, 1H), 7.27 (dd, *J* = 8.0, 1.6 Hz, 1H), 7.15 (t, *J* = 8.0 Hz, 1H), 3.96 (q, *J* = 7.2 Hz, 2H), 3.90 (s, 3H), 3.58 (s, 3H), 1.98-1.97 (m, 1H), 1.24-1.21 (t, *J* = 7.2 Hz, 3H), 0.78-0.75 (m, 2H), 0.64-0.58 (m, 2H).

Example **27**

6-((5-cyclopropyl pyridin-2-yl)amino)-N-ethoxy-4-((2-(N-methyl methanesulfonamido)phenyl)amino)nicotinamide

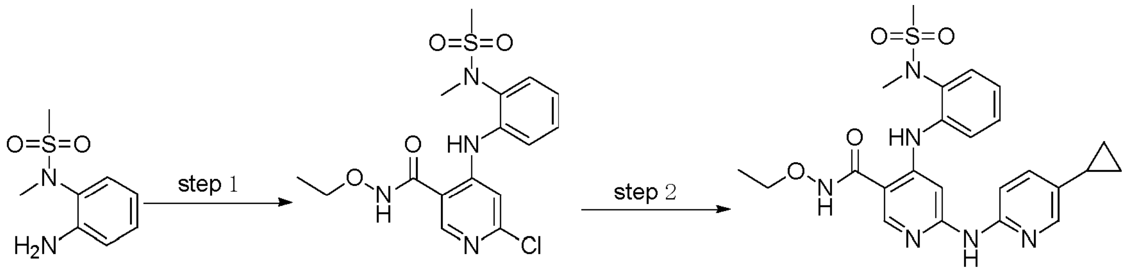


**2-c** **27-a** **27**

Step 1: N-(2-amino phenyl)-N-methyl methanesulfonamide **(2-c,** 201mg, 1mmol) and 4,6-dichloro-N-ethoxy nicotinamide (**int-2,** 236mg, 1mmol) were added to 10ml of anhydrous N,N-dimethylacetamide. The mixture was added at room temperature with a solution of LiHMDS in tetrahydrofuran (3ml, 3mmol), and stirred at room temperature for 2h. Upon indication of completed reaction by TLC, the mixture was adjusted with aqueous hydrochloride (1N) to pH 5, and extracted with ethyl acetate (30ml×3). The combined organic layers were dried, concentrated and then purified by column chromatography to provide 6-chloro-N-ethoxy-4-((2-(N-methyl methanesulfonamido)phenyl)amino)nicotinamide **(27-a,** 380mg, 0.76mmol, 76% yield).

Step 2: 6-chloro-N-ethoxy-4-((2-methoxy-3-(1-methyl-1H-pyrazol-4-yl)phenyl)amino)nicotinamide **(27-a,** 92 mg, 0.185 mmol), 5-cyclopropyl pyridin-2-ylamine (24.8 mg, 0.185mmol, Tetrahedron Letters 2017, 58, 1681), cesium carbonate (175.5 mg, 0.54mmol), XantPhos(20.81mg, 0.036mmol) and $Pd_2(dba)_3$(17.5mg, 0.018 mmol) were added to anhydrous dioxane (5 ml). After the atmosphere of the mixture was evacuated to vacuum and refilled with nitrogen, the reaction mixture was heated to 120°C with stirring for 6 hours, and filtered by suction. The filtrate was concentrated and purified by high performance preparative thin layer chromatography (MeOH:DCM=1:20) to provide the title compound: 6-((5-cyclopropyl pyridin-2-yl)amino)-N-ethoxy-4-((2-(N-methyl methanesulfonamido)phenyl)amino)nicotinamide **(27,** 20mg, 0.04mmol, 21.7% yield). MS Calcd: 496.19.20; MS Found: 497.20([M+H]+). $^1$H NMR (400 MHz, DMSO-$d_6$) : δ 11.57 (s, 1H), 10.06(s, 1H), 9.65 (s, 1H), 8.30 (s, 1H), 8.00-7.99 (m, 1H), 7.73-7.68 (m, 1H), 7.63 (dd, *J* = 8.4, 1.6 Hz, 1H), 7.55 (d, *J* = 8.0 Hz, 1H), 7.48-7.44 (m, 2H), 7.31 (d, *J* = 8.4 Hz, 1H), 7.19 (t, *J* = 8.0 Hz, 1H), 3.96 (q, *J* = 7.2 Hz, 2H), 3.15 (s, 3H), 3.13 (s, 3H), 1.89-1.83 (m, 1H), 1.22 (t, *J* = 7.2 Hz, 3H), 0.92-0.91 (m, 2H), 0.65-0.61 (m, 2H).

Example **28**

6-(cyclopropylcarboxamido)-*N*-methoxy-4-((1-methyl-1*H*-indazol-6-yl)amino)nicotinamide

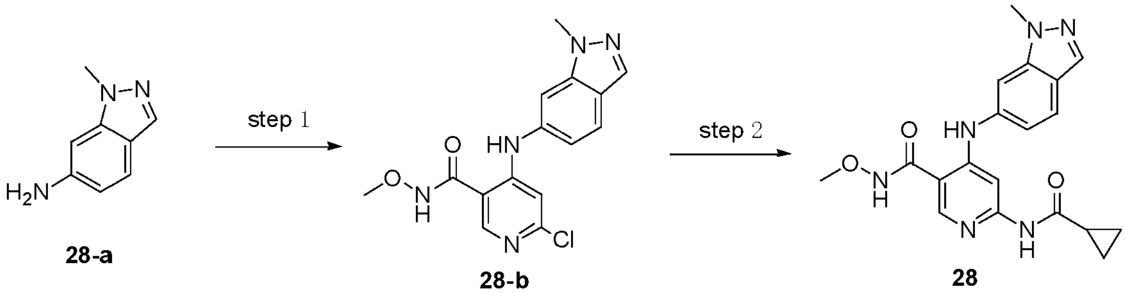


**28-a** **28-b** **28**

Step 1: To a two-necked flask were sequentially added 1-methyl-6-amino-1*H*-indazole (**28-a,** 147mg, 1mmol),

4,6-dichloro-*N*-methoxy nicotinamide **(int-1,** 218mg, 1mmol), and anhydrous tetrahydrofuran (5ml). The mixture was added with a solution of LiHMDS in tetrahydrofuran (3ml, 1mol/L) at room temperature, with continuous stirring at room temperature for 2h. Upon indication of completed reaction by TLC, the mixture was adjusted with aqueous hydrochloride (1N) to pH 5, and extracted with ethyl acetate (30mL x 3). The organic layers were combined, dried and concentrated, followed by purification by column chromatography to provide 6-chloro-*N*-methoxy-4-((1-methyl-1*H*-indazol-6-yl)amino) nicotinamide (**28-b,** 142mg, 0.39mmol, 43% yield). MS Calcd: 331; MS Found: 332($[M+H]^+$).

Step 2: 6-chloro-*N*-methoxy-4-((1-methyl-1*H*-indazol-6-yl)amino)nicotinamide **(28-b,** 33mg, 0.1mmol), cyclopropylcarboxamide (9.4mg, 0.11mmol), cesium carbonate (130mg, 0.4mmol), XantPhos(15mg, 0.02mmol) and $Pd_2(dba)_3$(10mg, 0.01mmol) were added to anhydrous dioxane (5ml). The atmosphere of the mixture was replaced by nitrogen three times. The reaction mixture was heated to 125°C with stirring for 8h, and filtered by suction. The filtrate was concentrated and purified by high performance preparative thin layer chromatography (MeOH:DCM=1:20) to provide the title compound: 6-(cyclopropylcarboxamido)-*N*-methoxy-4-((1-methyl-1*H*-indazol-6-yl)amino)nicotinamide (**28,** 12mg, 0.032mmol, 32% yield). MS Calcd: 380; MS Found: 381($[M+H]^+$). $^1H$ NMR (400 MHz, DMSO-$d_6$)$\delta$11.83 (s, 1H), 10.74 (s, 1H), 10.07 (s, 1H), 8.37 (s, 1H), 8.03 (s, 1H), 8.00 (d, $J$ = 1.2 Hz, 1H), 7.75 (d, $J$ = 8.4 Hz, 1H), 7.52 (s, 1H), 7.03 (dd, $J$ = 8.4, 2.0 Hz, 1H), 3.99 (s, 3H), 3.74 (s, 3H), 1.99-1.93 (m, 1H), 0.77 - 0.60 (m, 4H).

Example **29**

6-(cyclopropylcarboxamido)-*N*-methoxy-4-((2-methoxy-3-(1-methyl-1*H*-pyrazol-3-yl)phenyl)amino)nicotinamide

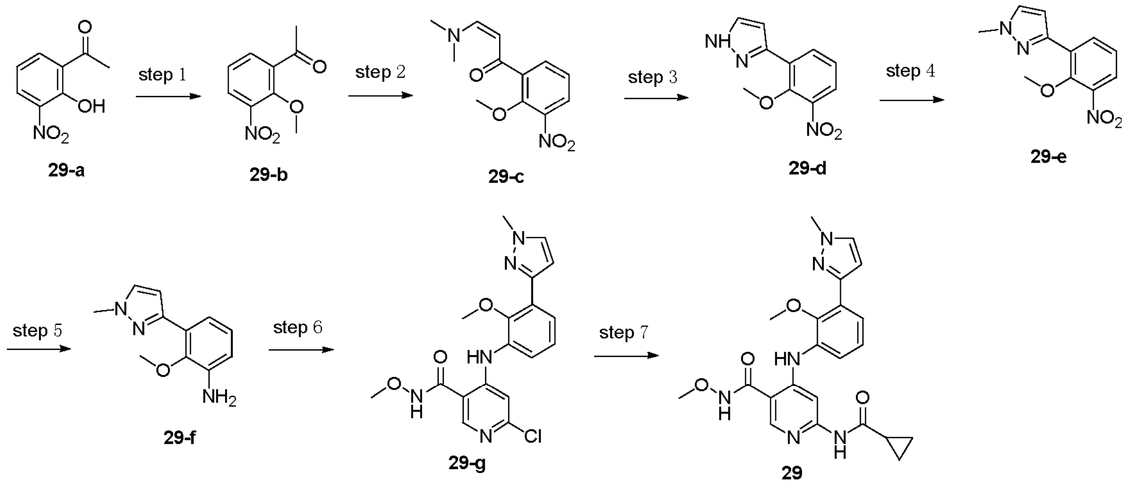


29-a 29-b 29-c 29-d 29-e 29-f 29-g 29

Step 1: To a 100mL flask were sequentially added 1-(2-hydroxyl-3-nitrophenyl)ethan-1-one (**29-a,** 1g, 5.5mmol), iodomethane (1g, 7mmol), anhydrous potassium carbonate (1g, 7.2mmol) and *N,N-dimethyl* formamide (5mL), and stirred at room temperature. Upon indication of completed reaction by TLC, the reaction mixture was added with 20mL of water for dilution of the mother liquid, and extracted three times with ethyl acetate. The organic phases were combined and washed with saturated brine, dried over anhydrous sodium sulfate, followed by solvent removed by rotary evaporation to provide 1-(2-methoxy-3-nitrophenyl)ethan-1-one (**29-b,** 1.03g, 5.28mmol, 96% yield). MS Calcd: 195; MS Found: 196($[M+H]^+$).

Step 2: Into a flask was weighted 1-(2-methoxy-3-nitrophenyl)ethan-1-one (**29-b,** 1.03g, 5.28mmol), added with 2mL of DMF-DMA and stirred at 90 °C. Upon indication of completed reaction by TLC, DMF-DMA was directly removed by rotary evaporation to provide 3-(dimethylamino)-1-(2-methoxy-3-nitrophenyl)prop-2-en-1-one **(29-c),** which was used directly in the next reaction without further purification, adding small amount of ethanol to prepare a ready-use stock solution. MS Calcd: 250; MS Found: 251($[M+H]^+$).

Step 3: Into a flask was added hydrazine monohydrate(0.5mL), added in ice bath with 5mL of ethanol and 1mL of acetic acid and stirred for 5minutes, followed by adding stock solution from the previous Step 2, and stirred at room temperature. Upon indication of completed reaction by TLC, the mixture was subjected to rotary evaporation to remove solvent, washed with small amount of water, and extracted with ethyl acetate. The combined organic phases were washed with saturated sodium chloride aqueous solution, dried over anhydrous sodium sulfate, followed by solvent removed by rotary evaporation to provide 3-(2-methoxy-3-nitrophenyl)-1*H*-pyrazole (**29-d,** 1.07g, 4.88mmol, 92% yield). MS Calcd: 219; MS Found: 220($[M+H]^+$).

Step 4: To a flask was added 3-(2-methoxy-3-nitrophenyl)-1*H*-pyrazole (**29-d,** 1.05g, 4.82mmol), followed by

sequential addition of *N,N-dimethyl* formamide (5mL), anhydrous potassium carbonate (2g, 14.4mmol) and iodomethane (2g, 14.1mmol), with stirring at room temperature. Upon indication of completed reaction by TLC, the reaction mixture was added with small amount of water for dilution of the mother liquid, and extracted with ethyl acetate (30mL x 3). The combined organic phases were washed with saturated sodium chloride aqueous solution (30mL x 3), dried over anhydrous sodium sulfate, followed by solvent removed by rotary evaporation to provide 3-(2-methoxy-3-nitrophenyl)-1-methyl-1*H*-pyrazole (**29-e,** 1.07g, 4.59mmol, 95% yield). MS Calcd: 233; MS Found: 234([M+H]$^+$).

Step 5: To 3-(2-methoxy-3-nitrophenyl)-1-methyl-1*H*-pyrazole (**29-e,** 1.07g, 4.59mmol), were sequentially added iron powder (1.8g, 32mmol), saturated ammonium chloride aqueous solution (6mL) and methanol (6mL). After the addition, the reaction mixture was heated to 100°C and stirred. When the reaction was completed, the mother liquid was filtered through diatomaceous earth, and the filtrate was extracted with ethyl acetate. The combined organic phases were washed with saturated sodium chloride aqueous solution, dried over anhydrous sodium sulfate, followed by solvent removed by rotary evaporation to provide 2-methoxy-3-(1-methyl-1*H*-pyrazol-3-yl)aniline **(29-f,** 580mg, 2.86mmol, 62% yield). MS Calcd: 203; MS Found: 204([M+H]$^+$).

Step 6: To a two-necked flask were sequentially added 2-methoxy-3-(1-methyl-1*H*-pyrazol-3-yl)aniline (**29-f,** 203mg, 1mmol), 4,6-dichloro-*N*-methoxy nicotinamide (218mg, 1mmol), and 5ml of anhydrous tetrahydrofuran as solvent, followed by addition at room temperature of a solution of LiHMDS in tetrahydrofuran (3ml, 1mol/L), with continuous stirring at room temperature for 2h. Upon indication of completed reaction by TLC, the mixture was adjusted with aqueous hydrochloride (1N) to pH 5, and extracted with ethyl acetate (30mLx3). The combined organic layers were dried, concentrated, and purified by column chromatography to provide 6-chloro-*N*-methoxy-4-(2-methoxy-3-(1-methyl-1*H*-pyrazol-3-yl)phenyl)nicotinamide **29-g,** 138mg, 0.36mmol, 36% yield). MS Calcd: 387; MS Found: 388([M+H]$^+$).

Step 7: 6-chloro-*N*-methoxy-4-(2-methoxy-3-(1-methyl-1*H*-pyrazol-3-yl)phenyl)nicotinamide **(29-g,** 39mg, 0.1mmol), cyclopropylcarboxamide (9.4mg, 0.11mmol), cesium carbonate (130mg, 0.4mmol), XantPhos(15mg, 0.02mmol) and $Pd_2(dba)_3$(10mg, 0.01mmol) were added to anhydrous dioxane (5ml). After atmosphere replacement with nitrogen three times, the reaction mixture was heated to 120°C with stirring for 8h, and filtered by suction. The filtrate was concentrated and purified by high performance preparative thin layer chromatography (MeOH:DCM=1:20) to provide the title compound: 6-(cyclopropylcarboxamido)-*N*-methoxy-4-(2-methoxy-3-(1-methyl-1*H*-pyrazol-3-yl)phenyl)nicotinamide (**29,** 14mg, 0.033mmol, 32% yield). MS Calcd: 436; MS Found: 437 ([M+H]$^+$). $^1$H NMR (400 MHz, DMSO-$d_6$)δ11.88 (s, 1H), 10.82 (s, 1H), 10.17 (s, 1H), 8.37 (s, 1H), 8.08 (s, 1H), 7.78 (d, *J* = 2.4 Hz, 1H), 7.61 (dd, *J* = 8.0, 1.6 Hz, 1H), 7.36 (dd, *J* = 8.0, 1.6 Hz, 1H), 7.18 (t, *J* = 8.0 Hz, 1H), 6.72 (d, *J* = 2.4 Hz, 1H), 3.91 (s, 3H), 3.73 (s, 3H), 3.59 (s, 3H), 2.01 - 1.93 (m, 1H), 0.81 - 0.75 (m, 4H).

Example **30**

6-((6-fluoropyridin-2-yl)amino)-*N*-methoxy-4-((2-methoxy-3-(1-methyl-1*H*-pyrazol-3-yl)phenyl)amino)nicotinamide

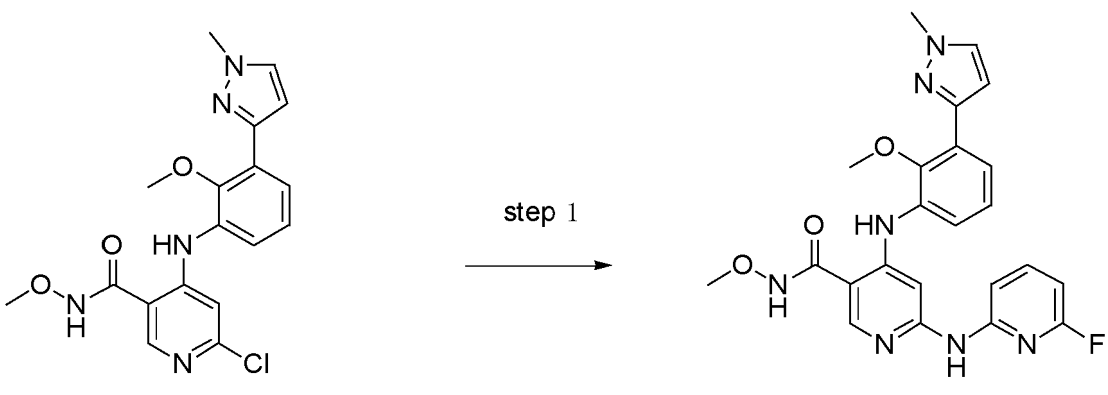


**29-g** **30**

Step 1: 6-chloro-*N*-methoxy-4-(2-methoxy-3-(1-methyl-1*H*-pyrazol-3-yl)phenyl)nicotinamide (**29-g,** 38.7mg, 0.1mmol), 6-fluoropyridin-2-ylamine (12.2mg, 0.11mmol), cesium carbonate (130mg, 0.4mmol), XantPhos(15mg, 0.02mmol) and $Pd_2(dba)_3$(10mg, 0.01mmol) were added to anhydrous dioxane (5ml). After atmosphere replacement with nitrogen three times, the reaction mixture was heated to 120°C with stirring for 8h, and filtered by suction. The filtrate was concentrated and purified by high performance preparative thin layer chromatography (MeOH:DCM=1:20) to provide the title compound: 6-(((6-fluoropyridin-2-yl)amino)-*N*-methoxy-4-(2-methoxy-3-(1-methyl-1*H*-pyrazol-3-yl)phenyl)nicotinamide (**30,** 11.8mg, 0.025mmol, 25% yield). MS Calcd: 463; MS Found: 464([M+H]+). $^1$H NMR (400 MHz, DMSO-$d_6$) δ11.81 (s, 1H), 10.27 (s, 1H), 10.07 (s, 1H), 8.36 (s, 1H), 7.86 - 7.79 (m, 2H), 7.78 (d, *J* = 2.4 Hz, 1H), 7.60 (dd, *J* = 8.0, 1.6 Hz, 1H), 7.56 (dd, *J* = 8.0, 1.6 Hz, 1H), 7.49 (dd, *J* = 8.0, 2.4 Hz, 1H), 7.19 (t, *J* = 8.0 Hz, 1H), 6.74 (d, *J* = 2.4 Hz, 1H), 6.58 (dd, *J* = 7.8, 2.4 Hz, 1H), 3.91 (s, 3H), 3.74 (s, 3H), 3.62 (s, 3H).

Example **31**

4-((3-cyano-2-methoxy phenyl)amino)-6-(cyclopropylcarboxamido)-N-methoxy nicotinamide

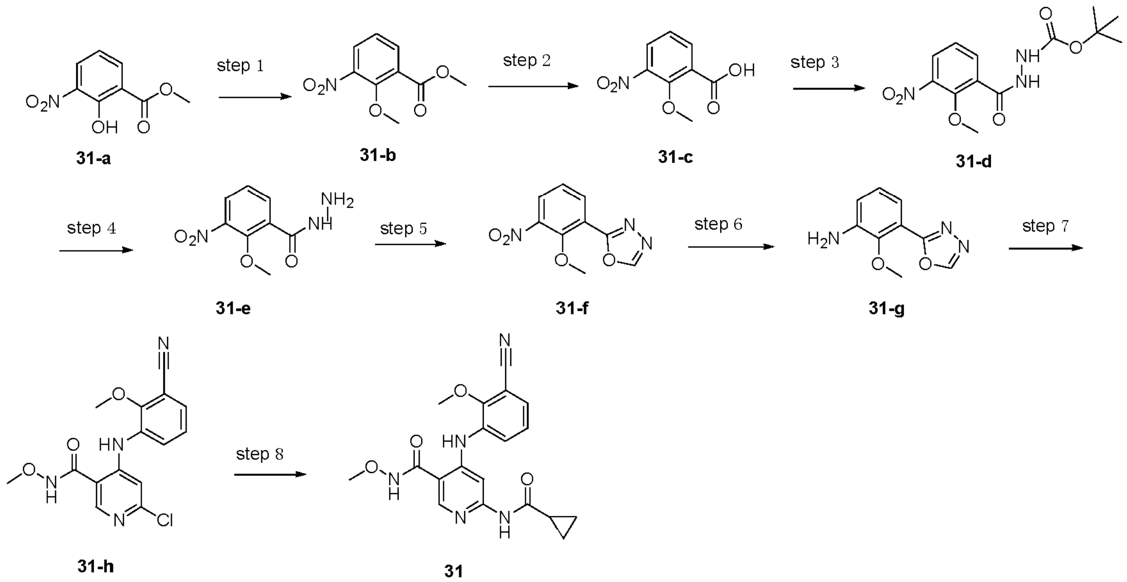


31-a 31-b 31-c 31-d 31-e 31-f 31-g 31-h 31

Step 1: To a 100mL flask were sequentially added methyl 2-hydroxyl-3-nitrobenzoate (**31-a,** 3g, 15mmol), iodomethane (2.8g, 20mmol), anhydrous potassium carbonate (3g, 21.7mmol) and *N,N*-dimethyl formamide (5mL), followed by stirring at room temperature. Upon indication of completed reaction by TLC, the reaction mixture was added with water (20mL) for dilution of the mother liquid, and extracted with ethyl acetate (30mL x 3). The combined organic phases were washed with saturated brine, dried over anhydrous sodium sulfate, followed by solvent removed by rotary evaporation to provide methyl 2-methoxy-3-nitrobenzoate (**31-b,** 2.9g, 13.7mmol, 91.3% yield). MS Calcd: 211; MS Found: 212([M+H]+).

Step 2: To methyl 2-methoxy-3-nitrobenzoate(**31-b**, 2.9g, 13.7mmol) were sequentially added 10mL of methanol, 2ml of water and sodium hydroxide (2g, 40mmol). The reaction mixture was stirred at room temperature. Upon indication of completed reaction by TLC, the reaction mixture was added with small amount of water for dilution of the mother liquid. The mixture was adjusted with aqueous hydrochloride (1N) to pH 5, causing a large amount of solid to precipitate, which was filtered by suction and dried to provide 2-methoxy-3-nitrobenzoic acid(**31-c,** 2.55g, 12.9mmol, 94.5% yield). MS Calcd: 197; MS Found: 198([M+H]+).

Step 3: Into *N,N*-dimethyl formamide (10mL) was added 2-methoxy-3-nitrobenzoic acid(**31-b**, 2.55g, 12.9mmol). After total dissolution, Boc-hydrazide (2.6g, 20mmol), HATU(7.6g, 20mmol) were added and stirred at 45°C overnight. Upon indication of completed reaction by TLC, the reaction mixture was washed with small amount of water, and extracted with ethyl acetate. The combined organic phases were washed with saturated sodium chloride aqueous solution, dried over anhydrous sodium sulfate, and purified by silica gel column chromatography to provide t-butyl 2-(2-methoxy-3-nitrobenzoyl)hydrazine-1-carboxylate (**31-d**, 2.31g, 7.43mmol, 57.6% yield). MS Calcd: 311; MS Found: 312([M+H]+).

Step 4: To dichloromethane (6mL) was added t-butyl 2-(2-methoxy-3-nitrobenzoyl) hydrazine-1-carboxylate (**31-d,** 2.3g, 7.43mmol), followed by adding trifluoroacetic acid (3ml) dropwise in ice bath. The reaction was allowed to warm back to room temperature and stirred. Upon indication of completed reaction by TLC, solvent was directly removed by rotary evaporation to provide 2-methoxy-3-nitrobenzohydrazide (**31-e,** 1.56g, 7.4mmol, 99% yield). MS Calcd: 211; MS Found: 212([M+H]+).

Step 5: To trimethyl orthoformate (3mL) was added 2-methoxy-3-nitrobenzohydrazide (**31-e,** 0.63g, 3mmol), heated to 105°C under nitrogen atmosphere and stirred. Upon indication of completed reaction by TLC, the mixture was subjected to rotary evaporation to remove solvent, washed with small amount of water(30 mL x 3), and extracted with ethyl

acetate (30mL x 3). The combined organic phases were washed with saturated sodium chloride aqueous solution (30mL x 3), dried over anhydrous sodium sulfate to provide 2-(2-methoxy-3-nitrophenyl)-1,3,4-oxadiazole (**31-f,** 521mg, 2.36mmol, 78.7% yield). MS Calcd: 221; MS Found: 222($[M+H]^+$).

Step 6: To 2-(2-methoxy-3-nitrophenyl)-1,3,4-oxadiazole(**31-f**, 300mg, 1.35mmol), were added palladium on carbon (20mg) and 5mL of methanol, allowed to react at room temperature under hydrogen atmosphere. When the reaction was completed, the mother liquid was filtered, and subjected to rotary evaporation to provide 2-methoxy-3-(1,3,4-oxadiazol-2-yl)aniline (**31-g,** 223mg, 1.16mmol, 86% yield). MS Calcd: 191; MS Found: 192($[M+H]^+$).

Step 7: To a two-necked flask were sequentially added 2-methoxy-3-(1,3,4-oxadiazol-2-yl)aniline **(31-g,** 191mg, 1mmol), 4,6-dichloro-*N*-methoxy nicotinamide (**int-1,** 218mg, 1mmol), and 5ml of anhydrous tetrahydrofuran as solvent, followed by adding a solution of LiHMDS in tetrahydrofuran (3ml, 3mmol) at room temperature and with continuous stirring at room temperature for 2h. Upon indication of completed reaction by TLC, the mixture was adjusted with aqueous hydrochloride (1N) to pH 5, and extracted with ethyl acetate (30ml x 3). The combined organic layers were dried, concentrated, and purified by column chromatography to provide 6-chloro-4-((3-cyano-2-methoxy phenyl)amino)-N-methoxy nicotinamide **(31-h,** 156mg, 0.47mmol, 47% yield). MS Calcd: 332; MS Found: 333($[M+H]^+$).

Step 8: 6-chloro-4-((3-cyano-2-methoxy phenyl)amino)-N-methoxy nicotinamide **(31-h,** 33mg, 0.1mmol), cyclopropylcarboxamide (9.4mg, 0.11mmol), cesium carbonate (130mg, 0.4mmol), XantPhos(15mg, 0.02mmol) and $Pd_2(dba)_3$(10mg, 0.01mmol) were added to anhydrous dioxane (5ml). After atmosphere replacement with nitrogen three times, the reaction mixture was heated to 120°C with stirring for 8h, and filtered by suction. The filtrate was concentrated and purified by high performance preparative thin layer chromatography (MeOH:DCM=1:20) to provide the title compound: 4-((3-cyano-2-methoxy phenyl)amino)-6-(cyclopropylcarboxamido)-N-methoxy nicotinamide **(31,** 8mg, 0.021mmol, 21% yield). MS Calcd: 381; MS Found: 382($[M+H]^+$). $^1$H NMR (400 MHz, DMSO-$d_6$)δ11.92 (s, 1H), 10.88 (s, 1H), 10.19 (s, 1H), 8.40 (s, 1H), 7.96 (s, 1H), 7.75 (dd, *J* = 8.0, 1.6 Hz, 1H), 7.54 (dd, *J* = 7.8, 1.6 Hz, 1H), 7.33 (t, *J* = 8.0 Hz, 1H), 3.91 (s, 3H), 3.73 (s, 3H), 2.05 - 1.91 (m, 1H), 0.81 - 0.73 (m, 4H).

Example **32**

6-(cyclopropylcarboxamido)-4-((3-fluoro-2-(*N*-methyl methanesulfonamido)phenyl)amino)-N-methoxy nicotinamide

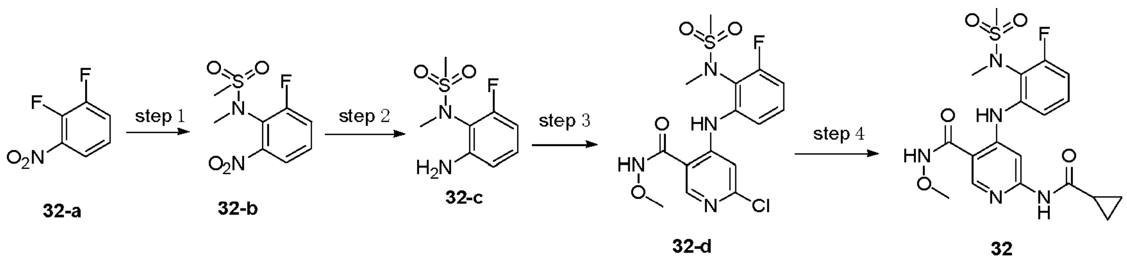


Step 1: 1,2-difluoro-3-nitrobenzene(**32-a**, 0.48g, 3mmol) was dissolved in acetonitrile (10ml), to which were sequentially added cesium carbonate (2.98g, 9mmol), *N*-methyl methanesulfonamide (0.36g, 3.3mmol). The reaction mixture was stirred at room temperature overnight. Upon indication of completed reaction by TLC, the mother liquid was washed respectively with water (30ml x 3) and saturated sodium chloride aqueous solution (30 ml x 3), dried over anhydrous sodium sulfate, and concentrated to provide a crude material, which was purified by silica gel column chromatography (petroleum ether/ethyl acetate=3:1) to provide N-(2-fluoro-6-nitrophenyl)-N-methyl methanesulfonamide *(***32-b,** 0.42g, 1.69mmol, 56.6% yield). MS Calcd:248; MS Found: 249($[M+H]^+$).

Step 2: N-(2-fluoro-6-nitrophenyl)-N-methyl methanesulfonamide *(***32-b,** 420mg, 1.69mmol) and palladium on carbon (15mg) were added to methanol(5ml). After atmosphere replacement by hydrogen three times, the reaction mixture was stirred at room temperature overnight, and filtered by suction. The filtrate was concentrated under reduced pressure to provide *N*-(2-amino-6-fluorophenyl)-*N-methyl methanesulfonamide (***32-c,** 288mg, 1.32mmol, 78% yield). MS Calcd: 218; MS Found: 219($[M+H]^+$).

Step 3: N-(2-amino-6-fluorophenyl)-*N*-methyl methanesulfonamide *(***32-c,** 218mg, 1mmol) and 4,6-dichloro-*N*-methoxy nicotinamide (218mg, 1mmol) were added to anhydrous tetrahydrofuran (5ml), to which was slowly added a solution of LiHMDS in tetrahydrofuran (3ml, 3mmol), and stirred at room temperature for 2h. Upon indication of completed reaction by TLC, the mixture was adjusted with aqueous hydrochloride (1N) to pH 5, and extracted with ethyl acetate (30ml x 3). The combined organic layers were dried, concentrated and then purified by column chromatography to provide 6-chloro-4-((3-fluoro-2-(*N*-methyl methanesulfonamido)phenyl)amino)-*N*-methoxy nicotinamide (**32-d,** 185mg, 0.46mmol, 46% yield). MS Calcd: 402; MS Found: 403($[M+H]^+$).

Step 4: 6-chloro-4-((3-fluoro-2-(*N*-methyl methanesulfonamido)phenyl)amino)-*N*-methoxy nicotinamide (**32-d,** 40mg, 0.1mmol), cyclopropylcarboxamide (9.4mg, 0.11mmol), cesium carbonate (130mg, 0.4mmol), XantPhos(15mg, 0.02mmol) and $Pd_2(dba)_3$(10mg, 0.01mmol) were added to anhydrous dioxane (5ml). After atmosphere replacement with nitrogen three times, the reaction mixture was heated to 125°C with stirring for 5h, and filtered by suction. The filtrate was concentrated and purified by high performance preparative thin layer chromatography to provide the title compound: (cyclopropylcarboxamido)-4-((3-fluoro-2-(*N*-methyl methanesulfonamido)phenyl)amino)-*N*-methoxy nicotinamide (**32,** 16mg, 0.36mmol, 36% yield). MS Calcd: 451; MS Found: 452($[M+H]^+$). $^1$H NMR (400 MHz, DMSO-$d_6$)δ11.87 (s, 1H), 10.87 (s, 1H), 10.17 (s, 1H), 8.40 (s, 1H), 8.38 (s, 1H), 8.24 - 8.23 (m, 1H), 8.05 - 8.03 (m, 1H), 7.36 - 7.31 (m, 1H), 3.72 (s, 3H), 3.16 (s, 3H), 3.14 (s, 3H), 2.06 - 1.90 (m, 1H), 0.82 - 0.73 (m, 4H).

Example **33**

6-(cyclopropylcarboxamido)-*N*-methoxy-4-((3-methyl-2-(*N*-methyl methanesulfonamido)phenyl)amino)nicotinamide

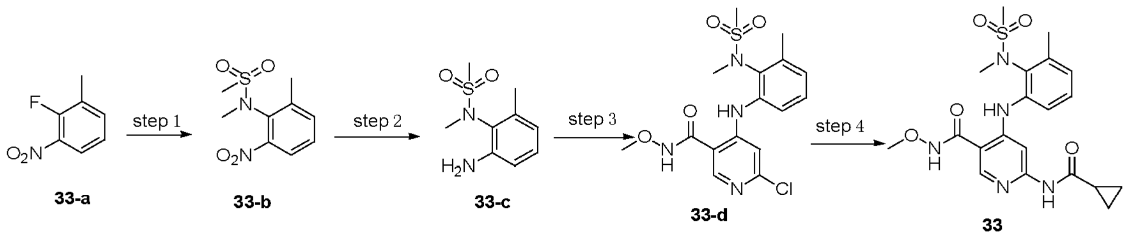

33-a 33-b 33-c 33-d 33

Step 1: 2-fluoro-1-methyl-3-nitrobenzene(**33-a**, 0.46mg, 3mmol) was dissolved in acetonitrile (10ml), to which were sequentially added cesium carbonate (2.97g, 9mmol), *N*-methyl methanesulfonamide (0.36g, 3.3mmol). The reaction mixture was stirred at room temperature overnight. Upon indication of completed reaction by TLC, the mother liquid was washed respectively with water (30ml x 3) and saturated sodium chloride aqueous solution (30ml x 3), dried over anhydrous sodium sulfate, and concentrated to provide a crude material, which was purified by silica gel column chromatography (petroleum ether/ethyl acetate=3: 1) to provide N-methyl-*N*-(2-methyl-6-nitrophenyl) methanesulfonamide(**33-b**, 399mg, 1.63mmol, 54% yield). MS Calcd:244; MS Found: 245($[M+H]^+$).

Step 2: *N*-methyl-*N*-(2-methyl-6-nitrophenyl) methanesulfonamide(**33-b**, 399mg, 1.63mmol) and palladium on carbon (15mg) were added to methanol(5ml). After atmosphere replacement with hydrogen three times, the reaction was stirred at room temperature overnight, and filtered by suction. The filtrate was concentrated under reduced pressure to provide *N*-(2-amino-6-methyl phenyl)-*N*-methyl methanesulfonamide *(***33-c,** 280mg, 1.30mmol, 80% yield). MS Calcd: 214; MS Found: 215($[M+H]^+$).

Step 3: *N*-(2-amino-6-methyl phenyl)-*N*-methyl methanesulfonamide *(***33-c,** 214mg, 1mmol) and 4,6-dichloro-N-methoxy nicotinamide (218mg, 1mmol) were added to anhydrous tetrahydrofuran (5ml), to which was slowly added a solution of LiHMDS in tetrahydrofuran (3ml, 1mol/L), and stirred at room temperature for 2h. Upon indication of completed reaction by TLC, the mixture was adjusted with aqueous hydrochloride (1N) to pH 5, and extracted with ethyl acetate (30ml x 3). The combined organic layers were dried, concentrated and then purified by column chromatography to provide 6-chloro-*N*-methoxy-4-((3-methyl-2-(*N*-methyl methanesulfonamido)phenyl)amino)nicotinamide (**33-d,** 128mg, 0.32mmol, 32% yield). MS Calcd: 398; S Found: 399($[M+H]^+$).

Step 4: 6-chloro-*N*-methoxy-4-((3-methyl-2-(*N*-methyl methanesulfonamido)phenyl)amino)nicotinamide **(33-d,** 40mg, 0.1mmol), cyclopropylcarboxamide (9.4mg, 0.11mmol), cesium carbonate (130mg, 0.4mmol), XantPhos(15mg, 0.02mmol) and $Pd_2(dba)_3$(10mg, 0.01mmol) were added to anhydrous dioxane (5ml). After atmosphere replacement with nitrogen three times, the reaction mixture was heated to 125 °C with stirring for 5h, and filtered by suction. The filtrate was concentrated and purified by high performance preparative thin layer chromatography to provide 6-(cyclopropylcarboxamido)-*N*-methoxy-4-((3-methyl-2-(*N*-methyl methanesulfonamido)phenyl)amino)nicotinamide **(33,** 6mg, 0.013mmol, 13% yield). MS Calcd: 447; MS Found: 448($[M+H]^+$). $^1$H NMR (400 MHz, DMSO-$d_6$)δ11.89 (s, 1H), 10.80 (s, 1H), 9.97 (s, 1H), 8.36 (s, 1H), 7.90 (s, 1H), 7.32 - 7.27 (m, 2H), 7.15 - 7.09 (m, 1H), 3.73 (s, 3H), 3.13 (s, 3H), 3.07 (s, 3H), 2.35 (s, 3H), 2.06 - 1.90 (m, 1H), 0.78 - 0.72 (m, 4H).

Example **34**

6-(cyclopropylcarboxamido)-4-((2-(*N,N*-dimethylaminosulfonyl)phenyl)amino)-*N*-methoxy nicotinamide

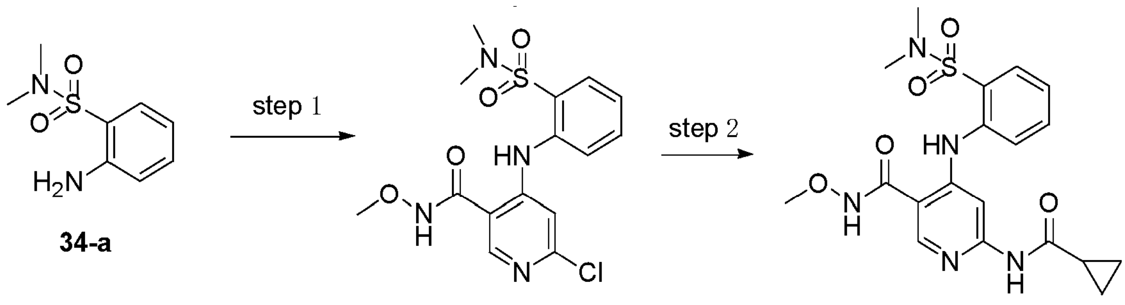


34-a 34-b 34

Step 1: 2-amino-*N,N*-dimethyl benzenesulfonamide(**34-a**, 200mg, 1mmol) and 4,6-dichloro-*N*-methoxy nicotinamide **(int-1,** 218mg, 1mmol) were added to 5ml of anhydrous tetrahydrofuran, to which was slowly added a solution of LiHMDS in tetrahydrofuran (3ml, 1mol/L), and stirred at room temperature for 2h. Upon indication of completed reaction by TLC, the mixture was adjusted with aqueous hydrochloride (1N) to pH 5, and extracted with ethyl acetate (30ml x 3). The combined organic layers were dried, concentrated and then purified by column chromatography to provide 6-chloro-4-((2-(*N,N*-dimethylaminosulfonyl)phenyl)amino)-N-methoxy nicotinamide **(34-b,** 178mg, 0.46mmol, 46% yield). MS Calcd: 384; MS Found: 385($[M+H]^+$).

Step 2: 6-chloro-4-((2-(*N,N*-dimethylaminosulfonyl)phenyl)amino)-*N*-methoxy nicotinamide **(34-b,** 38mg, 0.1mmol), cyclopropylcarboxamide (9.4mg, 0.11mmol), cesium carbonate (130mg, 0.4mmol), XantPhos(15mg, 0.02mmol) and $Pd_2(dba)_3$(10mg, 0.01mmol) were added to anhydrous dioxane (5ml). After atmosphere replacement with nitrogen three times, the reaction mixture was heated to 125°C with stirring for 5h, and filtered by suction. The filtrate was concentrated and purified by high performance preparative thin layer chromatography to provide the title compound: (cyclopropylcarboxamido)-4-((2-(*N,N*-dimethylaminosulfonyl)phenyl)amino)-*N*-methoxy nicotinamide **(34,** 7mg, 0.016mmol, 16% yield). MS Calcd: 433; MS Found:434($[M+H]^+$). $^1$H NMR (400 MHz, DMSO-$d_6$)δ11.83 (s, 1H), 10.87 (s, 1H), 10.27 (s, 1H), 8.38 (s, 1H), 7.97 (s, 1H), 7.84 (dd, *J* = 8.0, 1.6 Hz, 1H), 7.70 - 7.60 (m, 2H), 7.37 - 7.30 (m, 1H), 3.72 (s, 3H), 2.62 (s, 6H), 2.00 - 1.92 (m, 1H), 0.79 - 0.73 (m, 4H).

Example **35**

6-(cyclopropylcarboxamido)-N-methoxy-4-((2-(*N*-methyl methanesulfonamido)pyridin-3-yl)amino)nicotinamide

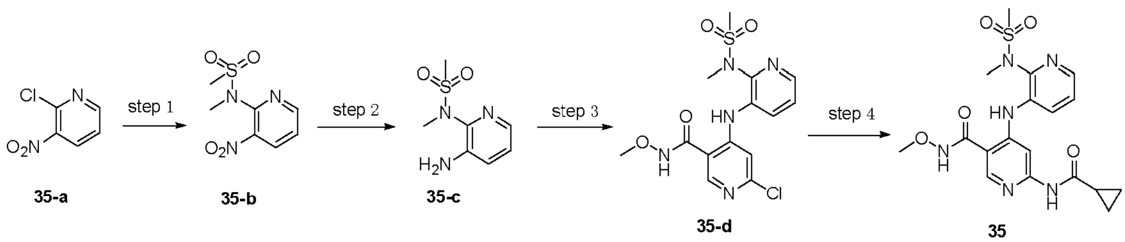


35-a 35-b 35-c 35-d 35

Step 1: 2-chloro-3-nitropyridine **(35-a,** 0.474g, 3mmol) was dissolved in acetonitrile (10ml), to which were sequentially added cesium carbonate (2.97g, 9mmol), N-methyl methanesulfonamide *(*0.36g, 3.3mmol). The reaction mixture was stirred at room temperature overnight. Upon indication of completed reaction by TLC, the mother liquid was washed respectively with water (30ml x 3) and saturated sodium chloride aqueous solution (30ml x 3), dried over anhydrous sodium sulfate, and concentrated to provide a crude material, which was purified by silica gel column chromatography (petroleum ether/ethyl acetate=3:1) to provide *N*-methyl-*N*-(3-nitropyridin-2-yl) methanesulfonamide(**35-b**, 366mg, 1.58mmol, 53% yield). MS Calcd: 231; MS Found: 232($[M+H]^+$).

Step 2: *N*-methyl-*N*-(3-nitropyridin-2-yl) methanesulfonamide(**35-b**, 366mg, 1.58mmol) and palladium on carbon (15mg) were added to methanol(5ml). After atmosphere replacement with hydrogen three times, the reaction mixture was stirred at room temperature overnight, and filtered by suction. The filtrate was concentrated under reduced pressure to provide *N*-(3-aminopyridin-2-yl)-*N*-methyl methanesulfonamide *(***35-c,** 274mg, 1.37mmol, 86% yield). MS Calcd: 201; MS

Found: 202([M+H]+).

Step 3: *N*-(3-aminopyridin-2-yl)-*N*-methyl methanesulfonamide (**35-c,** 201mg, 1mmol) and 4,6-dichloro-*N*-methoxy nicotinamide (218mg, 1mmol) were added to anhydrous tetrahydrofuran (5ml), to which was slowly added a solution of LiHMDS in tetrahydrofuran (3ml, 1mol/L), and stirred at room temperature for 2h. Upon indication of completed reaction by TLC, the mixture was adjusted with aqueous hydrochloride (1N) to pH 5, and extracted with ethyl acetate (30ml x 3). The combined organic layers were dried, concentrated and then purified by column chromatography to provide 6-chloro-*N*-methoxy-4-((2-(*N*-methyl methanesulfonamido)pyridin-3-yl)amino)nicotinamide (**35-d,** 172mg, 0.45mmol, 45% yield). MS Calcd: 385; MS Found: 386 ([M+H]+).

Step 4: 6-chloro-*N*-methoxy-4-((2-(N-methyl methanesulfonamido)pyridin-3-yl)amino)nicotinamide (**35-d,** 39mg, 0.1mmol), cyclopropylcarboxamide (9.4mg, 0.11mmol), cesium carbonate (130mg, 0.4mmol), XantPhos(15mg, 0.02mmol) and $Pd_2(dba)_3$(10mg, 0.01mmol) were added to anhydrous dioxane (5ml). After atmosphere replacement with nitrogen three times, the reaction mixture was heated to 125°C with stirring for 5h, and filtered by suction. The filtrate was concentrated and purified by high performance preparative thin layer chromatography to provide the title compound: 6-(cyclopropylcarboxamido)-N-methoxy-4-((2-(N-methyl methanesulfonamido)pyridin-3-yl)amino)nicotinamide **(35,** 13mg, 0.029mmol, 29% yield). MS Calcd: 434; MS Found: 435([M+H]+). $^1$H NMR (400 MHz, DMSO-$d_6$)δ11.85 (s, 1H), 10.86 (s, 1H), 10.15 (s, 1H), 8.39 (s, 1H), 8.26 (dd, *J* = 4.8, 1.6 Hz, 1H), 7.98 - 7.95 (m, 1H), 7.95 (s, 1H), 7.48 (dd, *J* = 8.0, 1.6 Hz, 1H), 3.73 (s, 3H), 3.18 (s, 3H), 3.14 (s, 3H), 2.01 - 1.92 (m, 1H), 0.82 - 0.74 (m, 4H).

Example **36**

6-((6-fluoropyridin-2-yl)amino)-*N*-methoxy-4-((2-(*N*-methyl methanesulfonamido)pyridin-3-yl)amino)nicotinamide

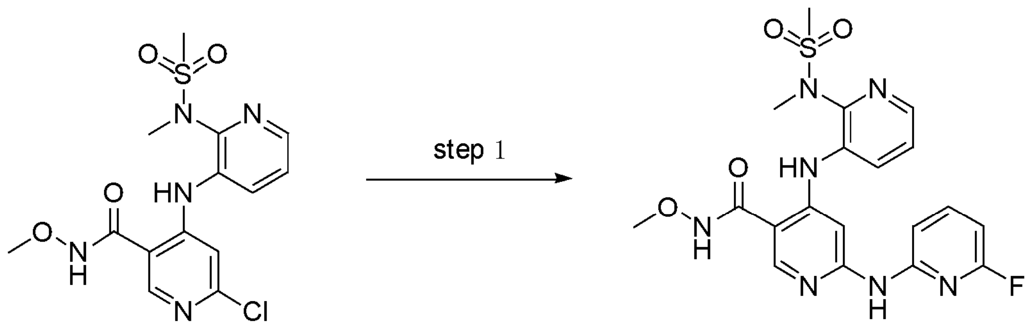


**35-d** **36**

Step 1: 6-chloro-*N*-methoxy-4-((2-(*N*-methyl methanesulfonamido)pyridin-3-yl)amino)nicotinamide **(35-d,** 39mg, 0.1mmol), 6-fluoropyridin-2-ylamine (13.2mg, 0.11mmol), cesium carbonate (130mg, 0.4mmol), XantPhos(15mg, 0.02mmol) and $Pd_2(dba)_3$(10mg, 0.01mmol) were added to anhydrous dioxane (5ml). After atmosphere replacement with nitrogen three times, the reaction mixture was heated to 125°C with stirring for 5h, and filtered by suction. The filtrate was concentrated and purified by high performance preparative thin layer chromatography to provide the title compound: 6-((6-fluoropyridin-2-yl)amino)-*N*-methoxy-4-((2-(*N*-methyl methanesulfonamido)pyridin-3-yl)amino)nicotinamide **(36,** 15mg, 0.032mmol, 32% yield). MS Calcd: 461; MS Found: 462([M+H]+). $^1$H NMR (400 MHz, DMSO-$d_6$)δ11.79 (s, 1H), 10.23 (s, 1H), 10.09 (s, 1H), 8.37 (s, 1H), 8.25 (dd, *J* = 4.6, 1.6 Hz, 1H), 8.13 (d, *J* = 8.4 Hz, 1H), 7.86 - 7.77 (m, 1H), 7.70 (s, 1H), 7.49 - 7.44 (m, 2H), 6.61 - 6.56 (m, 1H), 3.73 (s, 3H), 3.21 (s, 3H), 3.16 (s, 3H).

Example **37**

4-((2-(*N,N*-dimethylaminosulfonyl)phenyl)amino)-6-((6-fluoropyridin-2-yl)amino)-*N*-methoxy nicotinamide

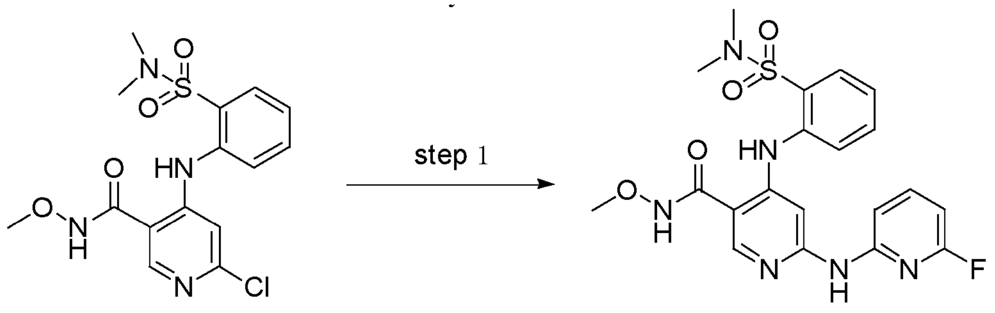


34-b 37

Step 1: 6-chloro-4-((2-(*N,N*-dimethylaminosulfonyl)phenyl)amino)-N-methoxy nicotinamide **(34-b,** 39mg, 0.1mmol), 6-fluoropyridin-2-ylamine (13.2mg, 0.11mmol), cesium carbonate (130mg, 0.4mmol), XantPhos(15mg, 0.02mmol) and $Pd_2(dba)_3$(10mg, 0.01mmol) were added to anhydrous dioxane (5ml). After atmosphere replacement with nitrogen three times, the reaction mixture was heated to 125 °C with stirring for 5h, and filtered by suction. The filtrate was concentrated and purified by high performance preparative thin layer chromatography to provide the title compound: *4-((2-(N,N-dimethylaminosulfonyl)phenyl)amino)-6-((6-fluoropyridin-2-yl)amino)-N*-methoxy nicotinamide **(37,** 13mg, 0.028mmol, 28% yield). MS Calcd: 460; MS Found: 461($[M+H]^+$). $^1H$ NMR (400 MHz, DMSO-$d_6$)$\delta$11.75 (s, 1H), 10.24 (s, 1H), 10.03 (s, 1H), 8.35 (s, 1H), 7.88 - 7.84 (m, 1H), 7.84 - 7.78 (m, 1H), 7.78 - 7.73 (m, 1H), 7.73 - 7.65 (m, 1H), 7.63 (s, 1H), 7.47 - 7.43 (m, 1H), 7.39 - 7.31 (m, 1H), 6.55 (dd, *J* = 7.8, 2.4 Hz, 1H), 3.72 (s, 3H), 2.65 - 2.61 (s, 6H).

Example **38**

4-((3-fluoro-2-(N-methyl methanesulfonamido)phenyl)amino)-6-((6-fluoropyridin-2-yl)amino)-*N*-methoxy nicotinamide

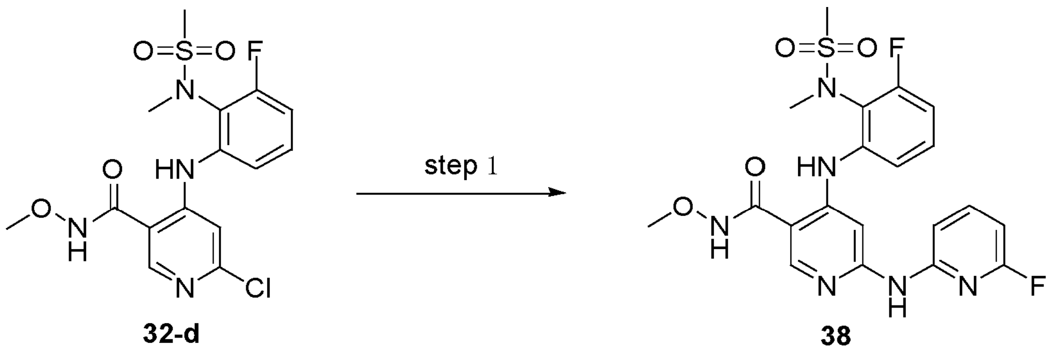


32-d 38

Step 1: 6-chloro-4-((3-fluoro-2-(*N*-methyl methanesulfonamido)phenyl)amino)-*N*-methoxy nicotinamide **(32-d,** 40mg, 0.1mmol), 6-fluoropyridin-2-ylamine (13.2mg, 0.11mmol), cesium carbonate (130mg, 0.4mmol), XantPhos(15mg, 0.02mmol) and $Pd_2(dba)_3$(10mg, 0.01mmol) were added to anhydrous dioxane (5ml). After atmosphere replacement with nitrogen three times, the reaction mixture was heated to 125°C with stirring for 5h, and filtered by suction. The filtrate was concentrated and purified by high performance preparative thin layer chromatography to provide the title compound: 4-((3-fluoro-2-(*N*-methyl methanesulfonamido)phenyl)amino)-6-((6-fluoropyridin-2-yl)amino)-*N*-methoxy nicotinamide **(38,** 18mg, 0.037mmol, 37% yield). MS Calcd: 478; MS Found: 479($[M+H]^+$). $^1H$ NMR (400 MHz, DMSO-$d_6$)$\delta$11.81 (s, 1H), 10.25 (s, 1H), 10.11 (s, 1H), 8.37 (s, 1H), 7.86 - 7.78 (m, 1H), 7.77 (s, 1H), 7.52 (d, *J* = 8.4 Hz, 1H), 7.49 - 7.41 (m, 2H), 7.09 (t, *J* = 9.2 Hz, 1H), 6.59 (dd, *J* = 8.0, 2.4 Hz, 1H), 3.73 (s, 3H), 3.19 (s, 3H), 3.16 (s, 3H).

Example **39**

6-(cyclopropylcarboxamido)-*N*-methoxy-4-((2-(oxetan-3-yloxy)phenyl)amino)nicotinamide

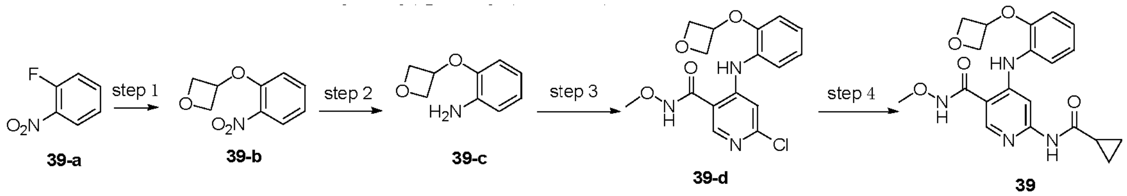


39-a 39-b 39-c 39-d 39

Step 1: 1-fluoro-2-nitrobenzene(**39-a**, 0.42g, 3mmol) was dissolved in acetonitrile (10ml), to which were sequentially added cesium carbonate (2.97g, 9mmol) and oxetan-3-ylmethanol (0.3g, 3.3mmol). The reaction mixture was stirred at room temperature overnight. Upon indication of completed reaction by TLC, the mother liquid was washed respectively with water (30ml x 3) and saturated sodium chloride aqueous solution (30ml x 3), dried over anhydrous sodium sulfate, and concentrated to provide a crude material, which was purified by silica gel column chromatography (petroleum ether/ethyl acetate=3:1) to provide 3-(2-nitrophenoxy)oxetane(**39-b**, 341mg, 1.74mmol, 58% yield). MS Calcd: 195; MS Found: 196($[M+H]^+$).

Step 2: 3-(2-nitrophenoxy) oxetane(**39-b**, 341mg, 1.74mmol), palladium on carbon (15mg) were added to methanol(5ml). After atmosphere replacement with hydrogen three times, the reaction mixture was stirred at room temperature overnight, and filtered by suction. The filtrate was concentrated under reduced pressure to provide 2-(oxetan-3-yloxy) aniline **(39-c,** 247mg, 1.49mmol, 50% yield). MS Calcd: 165; MS Found: 166($[M+H]^+$).

Step 3: 2-(oxetan-3-yloxy) aniline(**39-c**, 165mg, 1mmol) and 4,6-dichloro-*N*-methoxy nicotinamide (218mg, 1mmol) were added to anhydrous tetrahydrofuran (5ml), to which was slowly added a solution of LiHMDS in tetrahydrofuran (3ml, 1mol/L), and stirred at room temperature for 2h. Upon indication of completed reaction by TLC, the mixture was adjusted with aqueous hydrochloride (1N) to pH 5, and extracted with ethyl acetate (30ml x 3). The combined organic layers were dried, concentrated and then purified by column chromatography to provide 6-chloro-*N*-methoxy-4-((2-(oxetan-3-yloxy)phenyl)amino)nicotinamide **(39-d,** 135mg, 0.39mmol, 39% yield). MS Calcd: 349; MS Found: 350($[M+H]^+$).

Step 4: 6-chloro-*N*-methoxy-4-((2-(oxetan-3-yloxy)phenyl)amino)nicotinamide **(39-d,** 35mg, 0.1mmol), cyclopropylcarboxamide (9.4mg, 0.11mmol), cesium carbonate (130mg, 0.4mmol), XantPhos(15mg, 0.02mmol) and $Pd_2(dba)_3$(10mg, 0.01mmol) were added to anhydrous dioxane (5ml). After atmosphere replacement with nitrogen three times, the reaction mixture was heated to 125°C with stirring for 5h, and filtered by suction. The filtrate was concentrated and purified by high performance preparative thin layer chromatography to provide the title compound: 6-(cyclopropylcarboxamido)-*N*-methoxy-4-((2-(oxetan-3-yloxy)phenyl)amino)nicotinamide **(39,** 13mg, 0.032mmol, 32% yield). MS Calcd: 398; MS Found:399($[M+H]^+$). $^1$H NMR (400 MHz, Chloroform-*d*)δ10.00 (s, 1H), 8.84 (s, 1H), 8.31 (s, 1H), 7.98 (s, 1H), 7.48 - 7.42 (m, 1H), 7.10 - 7.02 (m, 2H), 6.52 - 6.44 (m, 1H), 5.26 - 5.17 (m, 1H), 4.98 - 4.89 (m, 2H), 4.84 - 4.75 (m, 2H), 3.92 (s, 3H), 1.46 - 1.38 (m, 1H), 0.92 - 0.72 (m, 4H).

Example **40**

6-(((6-fluoropyridin-2-yl)amino)-*N*-methoxy-4-((2-(oxetan-3-yloxy)phenyl)amino)nicotinamide

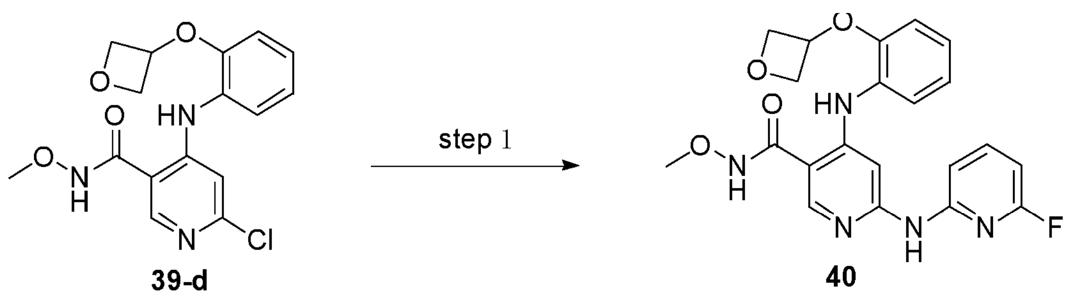


39-d 40

Step 1: 6-chloro-*N*-methoxy-4-((2-(oxetan-3-yloxy)phenyl)amino)nicotinamide **(39-d,** 35mg, 0.1mmol), 6-fluoropyridin-2-ylamine (13.2mg, 0.11mmol), cesium carbonate (130mg, 0.4mmol), XantPhos(15mg, 0.02mmol) and $Pd_2(dba)_3$(10mg, 0.01mmol) were added to anhydrous dioxane (5ml). After atmosphere replacement with nitrogen three times, the reaction mixture was heated to 125°C with stirring for 5h, and filtered by suction. The filtrate was concentrated and purified by high performance preparative thin layer chromatography to provide the title compound: 6-(((6-fluoropyridin-2-yl)amino)-*N*-methoxy-4-((2-(oxetan-3-yloxy)phenyl)amino)nicotinamide **(40,** 12mg, 0.028mmol, 28% yield). MS Calcd: 425; MS Found: 426 ($[M+H]^+$). $^1$H NMR (400 MHz, Chloroform-*d*)δ10.07 (s, 1H), 8.36 (s, 1H), 7.84 - 7.71 (m, 1H), 7.71 - 7.62 (m, 1H), 7.62 - 7.51 (m, 1H), 7.17 - 7.09 (m, 2H), 7.09 - 6.99 (m, 1H), 6.56 - 6.47 (m, 1H), 6.44 (dd, *J* = 8.0, 2.4 Hz,

1H), 5.28 - 5.20 (m, 1H), 5.00 - 4.90 (m, 2H), 4.87 - 4.74 (m, 2H), 3.93 (s, 3H).

Example **41**

6-(cyclopropylcarboxamido)-*N*-methoxy-4-((2-methoxy-3-(1*H*-pyrazol-1-yl)phenyl)amino)nicotinamide

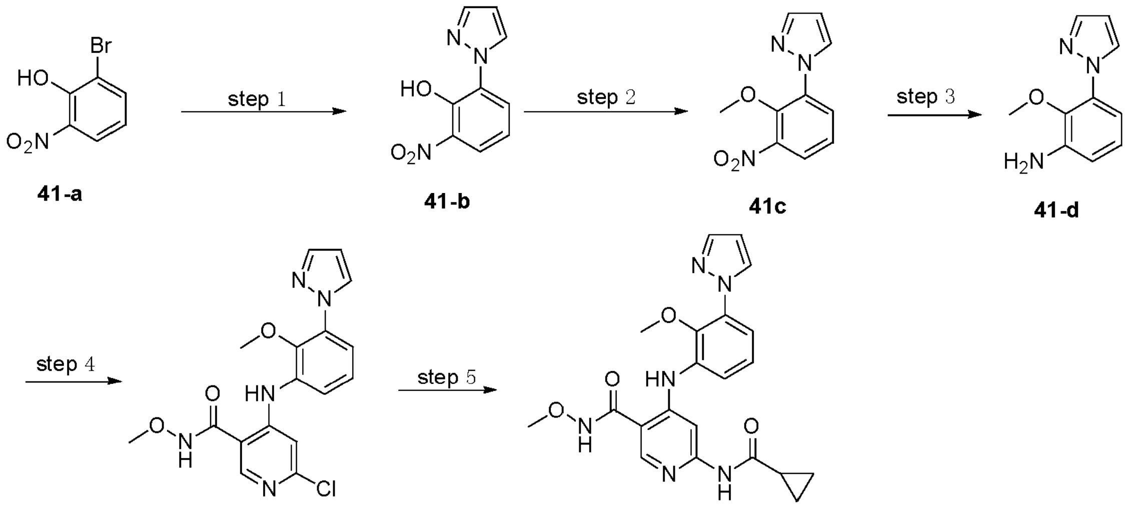


**41-a** **41-b** **41c** **41-d** **41-e** **41**

Step 1: To a 50mL flask were added 2-bromo-6-nitrophenol(**41-a**, 1.09g, 5mmol), pyrazole (0.68g, 10mmol), cuprous (I) iodide (0.095g, 0.5mmol), L-proline (0.115g, 1mmol), anhydrous potassium carbonate (1.38g, 10mmol), and DMSO(10mL), heated to 100°C with stirring for about 20h. Upon TLC indicating a substantially completed reaction, the mother liquid was filtered through diatomaceous earth. The filtrate was adjusted with 1N aqueous hydrochloride to pH 4, causing a large amount of solid to precipitate, which was filtered by suction to provide 2-nitro-6-(1*H*-pyrazol-1-yl)phenol **(41-b,** 460mg, 2.24mmol, 49% yield). MS Calcd: 205; MS Found: 206 ([M+H]$^+$).

Step 2: To a 100mL flask were sequentially added 2-nitro-6-(1H-pyrazol-1-yl)phenol **(41-b,** 460mg, 2.24mmol), iodomethane (0.416g, 3mmol), anhydrous potassium carbonate (0.618g, 4.448mmol) and *N,N*-dimethyl formamide (5mL), and stirred at room temperature. Upon indication of completed reaction by TLC, the reaction mixture was added with water (20mL) for dilution of the mother liquid, and extracted with ethyl acetate (20ml x 3). The combined organic phases were washed with saturated brine (20ml x 3), dried over anhydrous sodium sulfate, followed by solvent removed by rotary evaporation to provide 1-(2-methoxy-3-nitrophenyl)-1*H*-pyrazole **(41-c,** 455mg, 2.07mmol, 93% yield). MS Calcd: 219; MS Found: 220 ([M+H]$^+$).

Step 3: To 1-(2-methoxy-3-nitrophenyl)-1*H*-pyrazole **(41-c,** 455mg, 2.07mmol) were added iron powder (0.56g, 10mmol), saturated ammonium chloride aqueous solution (6mL) and methanol (6mL). After the addition, the reaction mixture was heated to 100°C and stirred. When the reaction was completed, the mother liquid was filtered through diatomaceous earth, and the filtrate was extracted with ethyl acetate (20ml x 3). The combined organic phases were washed with saturated sodium chloride aqueous solution (20ml x 3), dried over anhydrous sodium sulfate, followed by solvent removed by rotary evaporation to provide 2-methoxy-3-(1*H*-pyrazol-1-yl)aniline **(41-d,** 204mg, 1.08mmol, 52% yield). MS Calcd: 189; MS Found: 190 ([M+H]$^+$).

Step 4: To a two-necked flask were sequentially added 2-methoxy-3-(1*H*-pyrazol-1-yl)aniline **(41-d,** 0.189g, 1mmol), 4,6-dichloro-*N*-methoxy nicotinamide (218mg, 1mmol), and 5ml of anhydrous tetrahydrofuran as solvent. To which was added at room temperature with a solution of LiHMDS in tetrahydrofuran (3ml, 1mol/L) and with continuous stirring at room temperature for 2h. Upon indication of completed reaction by TLC, the mixture was adjusted with aqueous hydrochloride (1N) to pH 5, and extracted with ethyl acetate (30ml x 3). The combined organic layers were dried, concentrated, and purified by column chromatography to provide 6-chloro-*N*-methoxy-4-((2-methoxy-3-(1H-pyrazol-1-yl)phenyl)amino)nicotinamide **(41-e,** 198mg, 0.53mmol, 53% yield). MS Calcd; 373; MS Found: 374 ([M+H]$^+$).

Step 5: 6-chloro-N-methoxy-4-((2-methoxy-3-(1*H*-pyrazol-1-yl)phenyl)amino)nicotinamide **(41-e,** 37.3mg, 0.1mmol), cyclopropylcarboxamide (9.4mg, 0.11mmol), cesium carbonate (130mg, 0.4mmol), XantPhos(15mg, 0.02mmol) and $Pd_2(dba)_3$(10mg, 0.01mmol) were added to anhydrous dioxane (5ml). After atmosphere replacement with nitrogen three times, the reaction mixture was heated to 120 °C with stirring for 8h, and filtered by suction. The filtrate was concentrated and purified by high performance preparative thin layer chromatography (MeOH:DCM=1:20) to provide

6-(cyclopropylcarboxamido)-*N*-methoxy-4-((2-methoxy-3-(1*H*-pyrazol-1-yl)phenyl)amino)nicotinamide **(41,** 12mg, 0.028mmol, 28% yield). MS Calcd: 422; MS Found: 423([M+H]+). $^1$H NMR (400 MHz, Chloroform-*d*)δ10.28 (s, 1H), 8.97 (s, 1H), 8.37 (s, 1H), 8.16 (s, 1H), 8.08 (d, *J* = 2.4 Hz, 1H), 7.73 (d, *J* = 1.6 Hz, 1H), 7.53 - 7.43 (m, 2H), 7.35 - 7.26 (m, 1H), 6.48 - 6.46 (m, 1H), 3.92 (s, 3H), 3.51 (s, 3H), 1.46 - 1.37 (m, 1H), 0.95 - 0.83 (m, 4H).

Example **42**

6-((6-fluoropyridin-2-yl)amino)-N-methoxy-4-((2-methoxy-3-(1*H*-pyrazol-1-yl)phenyl)amino)nicotinamide

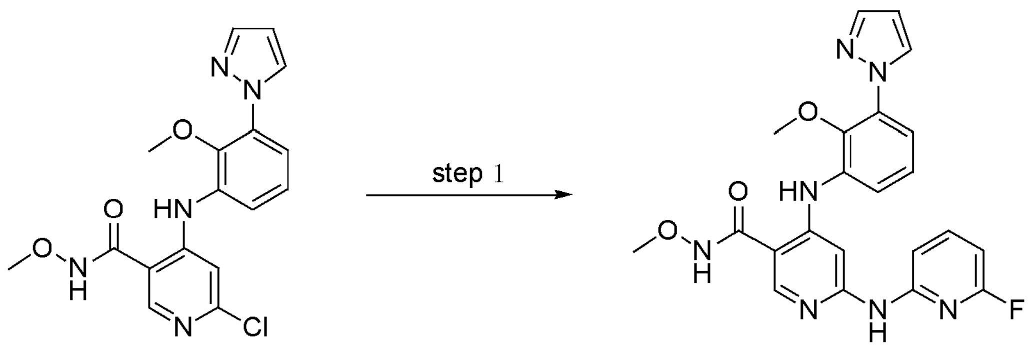


**41-e** **42**

Step 1: 6-chloro-*N*-methoxy-4-((2-methoxy-3-(1*H*-pyrazol-1-yl)phenyl)amino)nicotinamide **(41-e,** 37mg, 0.1mmol), 6-fluoropyridin-2-ylamine (13.2mg, 0.11mmol), cesium carbonate (130mg, 0.4mmol), XantPhos(15mg, 0.02mmol) and $Pd_2(dba)_3$(10mg, 0.01mmol) were added to anhydrous dioxane (5ml). After atmosphere replacement with nitrogen three times, the reaction mixture was heated to 120°C with stirring for 8h, and filtered by suction. The filtrate was concentrated and purified by high performance preparative thin layer chromatography (MeOH:DCM=1:20) to provide the title compound: 6-((6-fluoropyridin-2-yl)amino)-*N*-methoxy-4-((2-methoxy-3-(1*H*-pyrazol-1-yl)phenyl)amino)nicotinamide **(42,** 13mg, 0.029mmol, 29% yield). MS Calcd: 449; MS Found: 450([M+H]+). $^1$H NMR (400 MHz, DMSO-$d_6$) δ11.83 (s, 1H), 10.34 (s, 1H), 10.11 (s, 1H), 8.38 (s, 1H), 8.23 (d, *J* = 2.4 Hz, 1H), 7.87 - 7.79 (m, 2H), 7.78 - 7.77 (m, 1H), 7.68 - 7.62 (m, 1H), 7.54 - 7.47 (m, 1H), 7.38 (dd, *J* = 8.0, 1.6 Hz, 1H), 7.29 (t, *J* = 8.0 Hz, 1H), 6.62 - 6.54 (m, 2H), 3.74 (s, 3H), 3.47 (s, 3H).

Example **43**

6-((5-cyano pyridin-2-yl)amino)-*N*-methoxy-4-((2-(*N*-methyl methanesulfonamido)phenyl)amino)nicotinamide

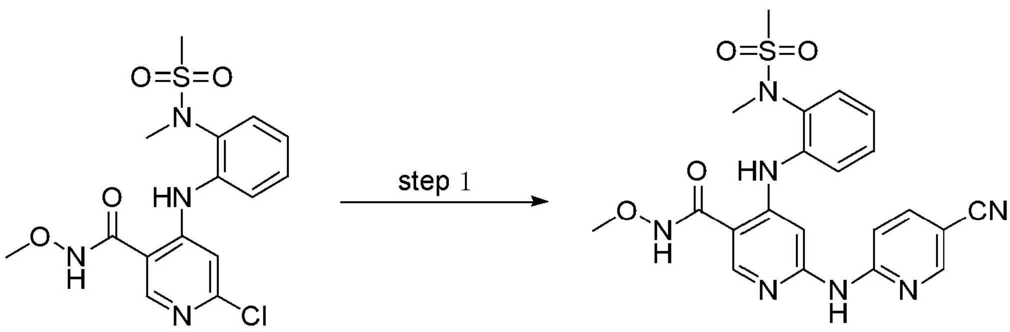


**2-d** **43**

Step 1: 6-chloro-*N*-methoxy-4-((2-(*N*-methyl methanesulfonamido)phenyl)amino)nicotinamide **(2-d,** 117mg, 0.3mmol), 6-aminonicotinonitrile (42mg, 0.35mmol), cesium carbonate (390mg, 3.6mmol), XantPhos(29mg, 0.06mmol) and $Pd_2(dba)_3$(54mg, 0.06mmol) were added to anhydrous dioxane (5ml) and the mixture was evacuated to vacuum. After atmosphere replacement with nitrogen three times, the reaction mixture was heated to 125°C with stirring for 5h, and

filtered by suction. The filtrate was concentrated and purified by high performance preparative thin layer chromatography (MeOH:DCM=1:20) to provide the title compound: 6-((5-cyano pyridin-2-yl)amino)-*N*-methoxy-4-((2-(*N*-methyl methanesulfonamido)phenyl)amino)nicotinamide **(43,** 32mg, 0.068mmol, 20.5% yield). MS Calcd: 467; MS Found: 468 ($[M+H]^+$). $^1H$ NMR (400 MHz, DMSO-$d_6$)δ11.78 (s, 1H), 10.37 (s, 1H), 10.09 (s, 1H), 8.63 - 8.58 (m, 1H), 8.35 (s, 1H), 8.04 (d, *J* = 7.2 Hz, 1H), 7.76 (d, *J* = 8.8 Hz, 1H), 7.73 (s, 1H), 7.62 (d, *J* = 8.0 Hz, 1H), 7.57 (d, *J* = 8.0 Hz, 1H), 7.50 (t, *J* = 7.8 Hz, 1H), 7.22 (t, *J* = 7.8 Hz, 1H), 3.72 (s, 3H), 3.16 (s, 3H), 3.13 (s, 3H).

Example **44**

6-((5-fluoro-4-methyl pyridin-2-yl)amino)-*N*-methoxy-4-((2-(*N*-methyl methanesulfonamido)phenyl)amino)nicotinamide

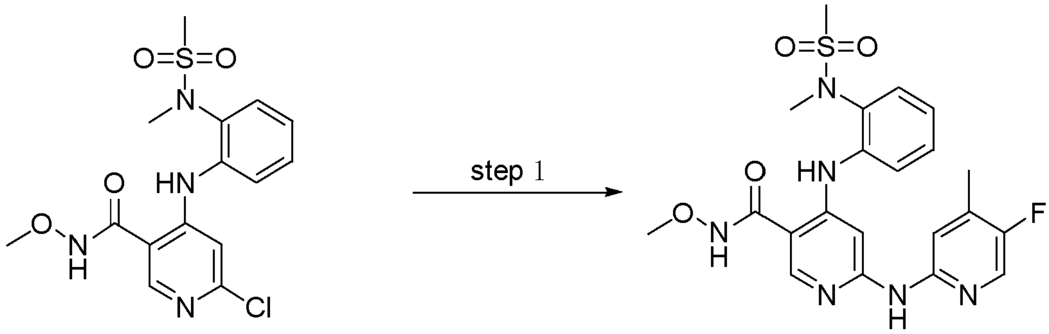


**2-d** **44**

Step 1: 6-chloro-*N*-methoxy-4-((2-(*N*-methyl methanesulfonamido)phenyl)amino)nicotinamide (**2-d,** 117mg, 0.3mmol), 5-fluoro-4-methyl pyridin-2-ylamine (44mg, 0.35mmol), cesium carbonate (390mg, 3.6mmol), XantPhos(29mg, 0.06mmol) and $Pd_2(dba)_3$(54mg, 0.06mmol) were added to anhydrous dioxane (5ml) and the mixture was evacuated to vacuum. After atmosphere replacement with nitrogen three times, the reaction mixture was heated to 125°C with stirring for 5h, and filtered by suction. The filtrate was concentrated and purified by high performance preparative thin layer chromatography (MeOH:DCM=1:20) to provide the title compound: 6-((5-fluoro-4-methyl pyridin-2-yl)amino)-*N*-methoxy-4-((2-(N-methyl methanesulfonamido)phenyl)amino)nicotinamide (**44,** 26mg, 0.055mmol, 16.5% yield). MS Calcd: 474; MS Found: 475 ($[M+H]^+$). $^1H$ NMR (400 MHz, DMSO-$d_6$)δ11.67 (s, 1H), 10.07 (s, 1H), 9.71 (s, 1H), 8.30 (s, 1H), 8.03 (s, 1H), 7.66 - 7.57 (m, 2H), 7.56 - 7.51 (m, 2H), 7.46 (t, *J* = 7.8 Hz, 1H), 7.18 (t, *J* = 7.8 Hz, 1H), 3.71 (s, 3H), 3.15 (s, 3H), 3.13 (s, 3H), 2.23 (s, 3H).

Example **45**

6-(((2,6-dimethyl pyrimidin-4-yl)amino)-*N*-methoxy-4-((2-(N-methyl methanesulfonamido)phenyl)amino)nicotinamide

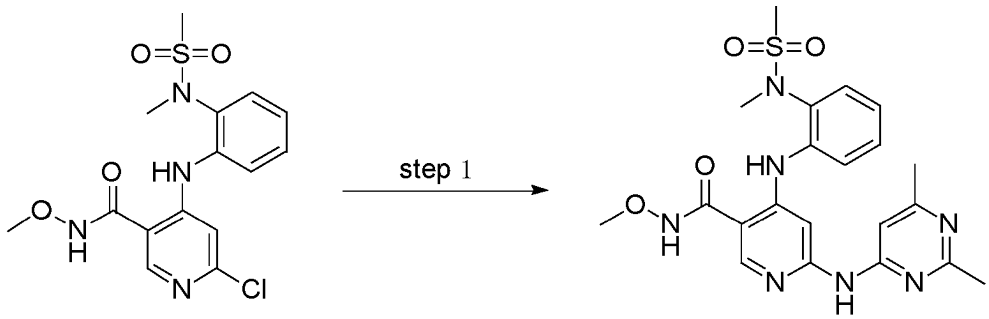


**2-d** **45**

Step 1: 6-chloro-*N*-methoxy-4-((2-(*N*-methyl methanesulfonamido)phenyl)amino)nicotinamide **(2-d,** 117mg, 0.3mmol), 2,6-dimethyl pyrimidin-4-ylamine (43mg, 0.35mmol), cesium carbonate (390mg, 1.2mmol), XantPhos(29mg, 0.06mmol) and $Pd_2(dba)_3$(54mg, 0.06mmol) were added to anhydrous dioxane (5ml) and the mixture was evacuated to

vacuum. After atmosphere replacement with nitrogen three times, the reaction mixture was heated to 125°C with stirring for 5h, and filtered by suction. The filtrate was concentrated and purified by high performance preparative thin layer chromatography (MeOH:DCM=1:20) to provide the title compound: 6-(((2,6-dimethyl pyrimidin-4-yl)amino)-*N*-methoxy-4-((2-(N-methyl methanesulfonamido)phenyl)amino)nicotinamide **(45,** 20mg, 0.042mmol, 12.7% yield). MS Calcd: 471; MS Found: 472 ([M+H]$^+$). $^1$H NMR (400 MHz, DMSO-$d_6$)δ11.75 (s, 1H), 10.11 (s, 1H), 10.05 (s, 1H), 8.34 (s, 1H), 8.00 (s, 1H), 7.65 (d, *J* = 8.0 Hz, 1H), 7.58 (d, *J* = 8.0 Hz, 1H), 7.45 (t, *J* = 7.8 Hz, 1H), 7.23 (t, *J* = 7.8 Hz, 1H), 7.06 (s, 1H), 3.72 (s, 3H), 3.16 (s, 3H), 3.13 (s, 3H), 2.35 (s, 3H), 2.27 (s, 3H).

Example **46**

4-((4-chloro-2-(N-methyl methanesulfonamido)phenyl)amino)-6-((5-fluoropyridin-2-yl)amino)-N-methoxy nicotinamide

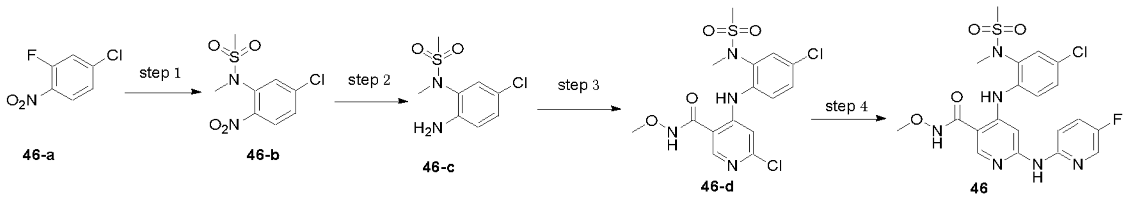


Step 1: To a 100ml reaction flask was added 4-chloro-2-fluoro-1-nitrobenzene(**46-a**, 0.53g, 3.00mmol) dissolved in acetonitrile (30ml), followed by cesium carbonate (2g, 6.1mmol), N-methyl methanesulfonamide (0.56g, 4mmol). The reaction mixture was stirred at room temperature overnight. Upon indication of completed reaction by TLC, the reaction mixture was washed respectively with water (30ml x 3) and saturated sodium chloride aqueous solution (30ml x 3), dried over anhydrous sodium sulfate, and concentrated to provide a crude material, which was purified by silica gel column chromatography (petroleum ether/ethyl acetate=3:1) to provide the product N-(5-chloro-2-nitrophenyl)-N-methyl methanesulfonamide **(46-b,** 0.63g, 81.1% yield). MS Calcd:264; MS Found: 265([M+H]$^+$).

Step 2: N-(5-chloro-2-nitrophenyl)-N-methyl methanesulfonamide **(46-b,** 600mg, 2.3mmol) and iron powder (672mg, 12mmol) were added to methanol (3ml) and saturated ammonium chloride aqueous solution (3ml). The reaction mixture was refluxed at 100°C with stirring for 8h, filtered by suction. The filtrate was extracted with ethyl acetate (30ml x 3). The organic layers were combined, dried, and concentrated under reduced pressure to provide N-(2-amino-5-chloro phenyl)-N-methyl methanesulfonamide **(46-c,** 328mg, 1.4mmol, 60.9% yield), which was used directly in the next reaction without further purification. MS Calcd: 234; MS Found: 235 ([M+H]$^+$).

Step 3: N-(2-amino-5-chloro phenyl)-N-methyl methanesulfonamide **(46-c,** 234mg, 1mmol) and 4,6-dichloro-N-methoxy nicotinamide **(int-1,** 218mg, 1mmol) were added to 5ml of tetrahydrofuran. The mixture was added at room temperature with a solution of LiHMDS in tetrahydrofuran (3ml, 1mol/L), and stirred at room temperature for 2h. Upon indication of completed reaction by TLC, the mixture was adjusted with aqueous hydrochloride (1N) to pH 5, and extracted with ethyl acetate (30 ml x 3). The combined organic layers were dried, concentrated and then purified by column chromatography to provide 6-chloro-4-((4-chloro-2-((N-methyl methanesulfonamido)phenyl)amino)-N-methoxy nicotinamide **(46-d,** 156mg, 0.37mmol, 37% yield). MS Calcd: 418; MS Found: 419 ([M+H]$^+$).

Step 4: 6-chloro-4-((4-chloro-2-((N-methyl methanesulfonamido)phenyl)amino)-N-methoxy nicotinamide **(46-d,** 75mg, 0.18mmol), 5-fluoropyridin-2-ylamine (28mg, 0.25mmol), cesium carbonate (195mg, 0.6mmol), Xant-Phos(20mg, 0.04mmol) and $Pd_2(dba)_3$(37mg, 0.04mmol) were added to anhydrous dioxane (5ml) and the mixture was evacuated to vacuum. After atmosphere replacement with nitrogen three times, the reaction mixture was heated to 125°C with stirring for 5h, and filtered by suction. The filtrate was concentrated and purified by high performance preparative thin layer chromatography (MeOH:DCM=1:20) to provide the title compound: 4-((4-chloro-2-(N-methyl methanesulfonamido)phenyl)amino)-6-((5-fluoropyridin-2-yl)amino)-N-methoxy nicotinamide **(46,** 20mg, 0.04mmol, 22.4% yield). MS Calcd: 494; MS Found: 495 ([M+H]$^+$). $^1$H NMR (400 MHz, DMSO-$d_6$)δ11.75 (s, 1H), 10.15 (s, 1H), 9.85 (s, 1H), 8.32 (s, 1H), 8.24 - 8.19 (m, 1H), 8.00 (s, 1H), 7.88 - 7.85 (m, 1H), 7.83 - 7.79 (m, 1H), 7.21 (s, 1H), 7.12 - 7.08 (m, 1H), 7.02 (s, 1H), 3.71 (s, 3H), 3.17 (s, 3H), 3.13 (s, 3H).

Example **47**

4-((4-chloro-2-(N-methyl methanesulfonamido)phenyl)amino)-6-((6-fluoropyridin-2-yl)amino)-N-methoxy nicotinamide

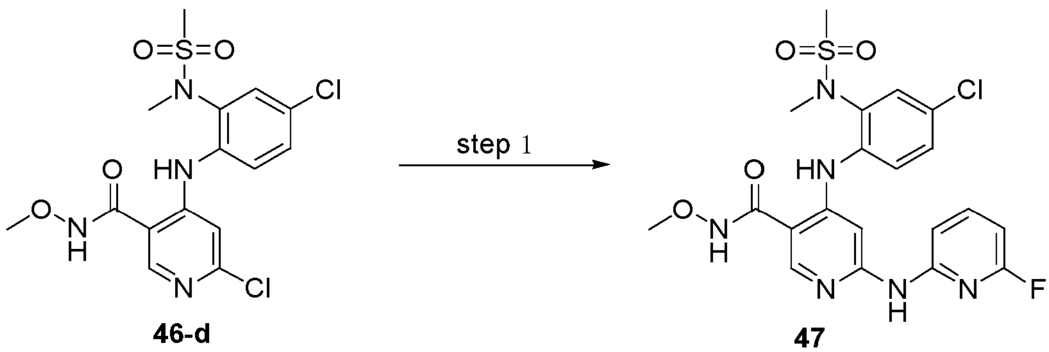


46-d

47

Step 4: 6-chloro-4-((4-chloro-2-((N-methyl methanesulfonamido)phenyl)amino)-N-methoxy nicotinamide **(46-d,** 75mg, 0.18mmol), 6-fluoropyridin-2-ylamine (28mg, 0.25mmol), cesium carbonate (195mg, 0.6mmol), Xant-Phos(20mg, 0.04mmol) and $Pd_2(dba)_3$(37mg, 0.04mmol) were added to anhydrous dioxane (5ml) and the mixture was evacuated to vacuum. After atmosphere replacement with nitrogen three times, the reaction mixture was heated to 125°C with stirring for 5h, and filtered by suction. The filtrate was concentrated and purified by high performance preparative thin layer chromatography (MeOH:DCM=1:20) to provide the title compound: 4-((4-chloro-2-(N-methyl methanesulfonamido)phenyl)amino)-6-((6-fluoropyridin-2-yl)amino)-N-methoxy nicotinamide **(47,** 13mg, 0.026mmol, 14.6% yield). MS Calcd: 494; MS Found: 495 ($[M+H]^+$). $^1H$ NMR (400 MHz, DMSO-$d_6$)δ11.80 (s, 1H), 10.24 (s, 1H), 10.09 (s, 1H), 8.36 (s, 1H), 7.85 - 7.74 (m, 2H), 7.53 - 7.36 (m, 3H), 7.09 (t, J = 9.2 Hz, 1H), 6.58 (dd, J = 9.2, 2.4 Hz, 1H), 3.73 (s, 3H), 3.16 (s, 3H), 3.13 (s, 3H).

Example **48**

4-((2-cyano-3-(1-methyl-1H-pyrazol-4-yl)phenyl)amino)-6-(cyclopropylcarboxamido)-N-methoxy nicotinamide

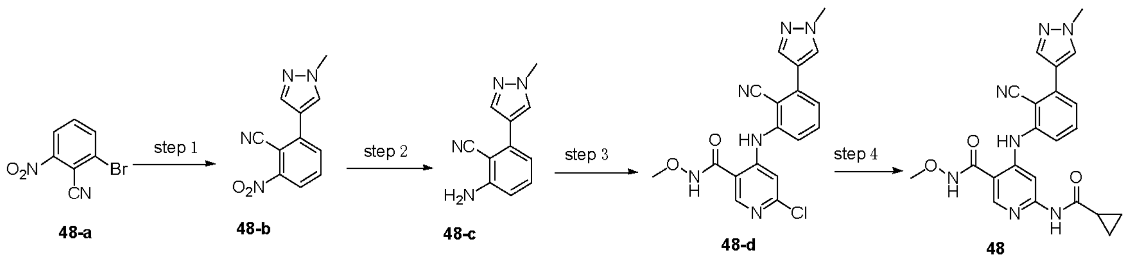


48-a 48-b 48-c 48-d 48

Step 1: 2-bromo-6-nitrobenzonitrile(**48-a**, 500mg, 2.20mmol), 1-methyl-4-(4,4,5,5-tetramethyl-1,3,2-dioxaborolan-2-yl)-1H-pyrazole (458mg, 2.20mmol), Pd(dppf)$Cl_2$(36mg, 0.044mmol) and sodium carbonate (700mg, 6.60mmol) were dissolved in a mixed solvent of water and dioxane (2ml/8mL), heated to 100°C for 3h. The reaction mixture was concentrated, added with 10ml of water, and extracted with ethyl acetate (10ml). The combined ethyl acetate layers were dried over anhydrous sodium sulfate, filtered by suction, and concentrated to obtain a crude material, which was purified by column chromatography (PE:EA =4:1) to provide 2-(1-methyl-1H-pyrazol-4-yl)-6-nitrobenzonitrile (**48-b,** 421mg, 1.85mmol, 84% yield), as ayellow solid. MS Calcd: 228; MS Found: 229 ($[M+H]^+$).

Step 2: To methanol(20ml) were added 2-(1-methyl-1H-pyrazol-4-yl)-6-nitrobenzonitrile **(48-b,** 400mg, 1.75mmol) and palladium on carbon (40mg). After atmosphere replacement by hydrogen three times, the reaction mixture was stirred under hydrogen atmosphere at room temperature overnight, and filtered by suction. The filtrate was concentrated under reduced pressure to provide 2-amino-6-(1-methyl-1H-pyrazol-4-yl) benzonitrile(**48-c**, 330mg, 1.67mmol, 90% yield), as a grey oil, which was used directly in the next reaction without further purification. MS Calcd: 198; MS Found: 199 ($[M+H]^+$).

Step 3: To 5ml of anhydrous tetrahydrofuran were added 2-amino-6-(1-methyl-1H-pyrazol-4-yl) benzonitrile(**48-c**, 200mg, 1.01mmol) and 4,6-dichloro-N-methoxy nicotinamide **(int-1,** 223.2mg, 1.01mmol). The mixture was added at room temperature with a solution of LiHMDS in tetrahydrofuran (2ml, 2.02mmol), and stirred at room temperature for 2 hours. The mixture was adjusted with aqueous hydrochloride (1N) to pH 5, and extracted with ethyl acetate (10ml). The combined organic layers were dried, concentrated and then purified by column chromatography (PE:EA = 1:1) to provide 6-chloro-4-((2-cyano-3-(1-methyl-1H-pyrazol-4-yl)phenyl)amino)-N-methoxy nicotinamide **(48-d,** 120°Cg, 0.31mmol, 30% yield), as a white solid. MS Calcd: 382; MS Found: 383 ($[M+H]^+$).

Step 4: 6-chloro-4-((2-cyano-3-(1-methyl-1H-pyrazol-4-yl)phenyl)amino)-N-methoxy nicotinamide **(48-d,** 50mg, 0.13mmol), cyclopropylcarboxamide (22mg, 0.26mmol), cesium carbonate (127mg, 0.39mmol), XantPhos(30mg, 0.04mmol) and $Pd_2(dba)_3$(24mg, 0.026mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and purified by high performance preparative thin layer chromatography (MeOH:DCM=1:20) to provide 4-((2-cyano-3-(1-methyl-1H-pyrazol-4-yl)phenyl)amino)-6-(cyclopropylcarboxamido)-N-methoxy nicotinamide **(48,** 10mg, 0.023mmol, 18% yield), as a yellow solid. MS Calcd: 431; MS Found: 432 ([M+H]$^+$). 1H NMR (400 MHz, DMSO-$d_6$) : δ 11.87 (s, 1H), 10.89 (s, 1H), 10.46 (s, 1H), 8.44 (s, 1H), 8.26 (s, 1H), 7.95-7.95 (m, 2H), 7.67 (t, $J$ = 8.0 Hz, 1H), 7.45-7.42 (m, 2H), 3.92 (s, 3H), 3.73 (s, 3H), 1.98-1.95 (m, 1H), 0.78-0.75 (m, 2H), 0.65-0.62(m, 2H).

Example **49**

4-((2-cyano-3-(1-methyl-1H-pyrazol-4-yl)phenyl)amino)-6-((5-fluoropyridin-2-yl)amino)-N-methoxy nicotinamide

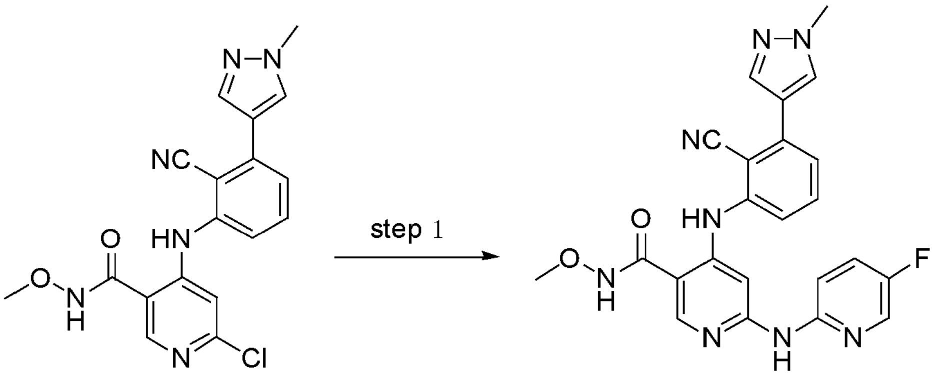


**48-d** **49**

Step 1: 6-chloro-4-((2-cyano-3-(1-methyl-1H-pyrazol-4-yl)phenyl)amino)-N-methoxy nicotinamide **(48-d,** 50mg, 0.13mmol), 5-fluoropyridin-2-ylamine (29mg, 0.26mmol), cesium carbonate (127mg, 0.39mmol), XantPhos(30mg, 0.04mmol) and $Pd_2(dba)_3$(24mg, 0.026mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and purified by high performance preparative thin layer chromatography (MeOH:DCM=1:20) to provide the title compound: 4-((2-cyano-3-(1-methyl-1H-pyrazol-4-yl)phenyl) amino)-6-((5-fluoropyridin-2-yl)amino)-N-methoxy nicotinamide **(49,** 13mg, 0.028mmol, 22% yield). MS Calcd: 458; MS Found: 459 ([M+H]$^+$). 1H NMR (400 MHz, DMSO-$d_6$) : δ 11.85 (s, 1H), 10.51 (s, 1H), 9.94 (s, 1H), 8.40 (s, 1H), 8.28 (s, 1H), 8.11 (s, 1H), 7.96 (s, 1H), 7.75-7.64 (m, 4H), 7.53-7.55 (d, $J$ = 8.0 Hz, 1H), 7.42-7.41 (d, $J$=7.8 Hz, 1H), 3.93 (s, 3H), 3.73 (s, 3H).

Example **50**

6-(cyclopropylcarboxamido)-N-methoxy-4-((3-(1-methyl-1H-pyrazol-3-yl)-2-(2,2,2-trifluoroethoxy)phenyl)amino)nicotinamide

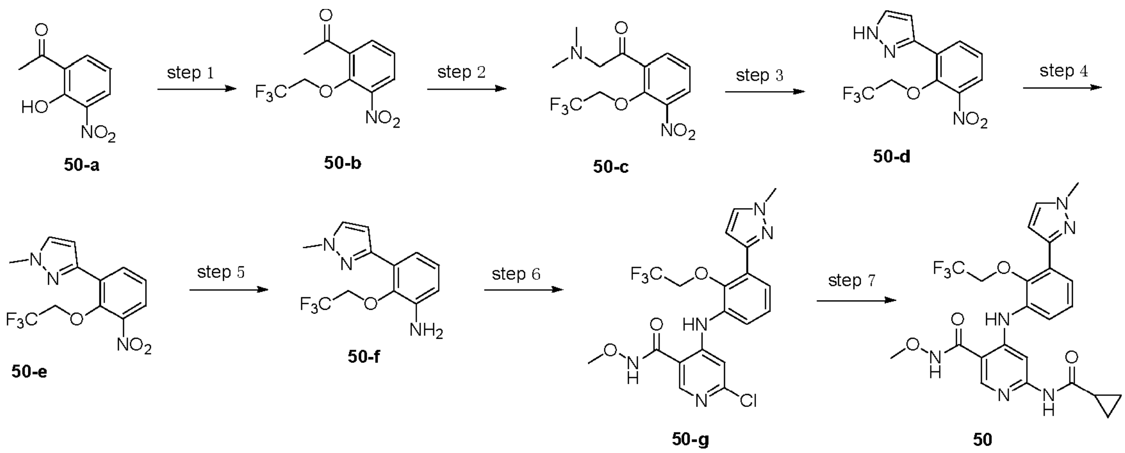


50-a 50-b 50-c 50-d 50-e 50-f 50-g 50

Step 1: 1-(2-hydroxyl-3-nitrophenyl)ethan-1-one **(50-a,** 2g, 11mmol), 2,2,2-trifluoroethyl trifluoromethanesulfonate(2.7g, 11mmol) and potassium carbonate (4.6g, 33mmol) were added to DMF(50ml). The reaction mixture was heated to 80°C with stirring for 2 hours. Upon indication of completed reaction by TLC, the reaction mixture was poured into 250ml of water, extracted with ethyl acetate (100ml). The extracted ethyl acetate organic layer was washed with water, dried over anhydrous sodium sulfate, filtered by suction, concentrated under reduced pressure to provide a brown oil **(50-b,** 2.9g, 10.4mmol, 94% yield).

Step 2: 1-(3-nitro-2-(2,2,2-trifluoroethoxy)phenyl)ethan-1-one **(50-b,** 2.9g, 10.4mmol), was added to DMF-DMA(20ml). The mixture was heated to 80°C with stirring for 30 minutes. Upon indication of completed reaction by TLC, the reaction mixture was directly concentrated, with residual solvent removed with toluene, to provide 2-(dimethylamino)-1-(3-nitro-2-(2,2,2-trifluoroethoxy)phenyl)ethan-1-one **(50-c,** 2.8g, 8.7mmol), as a brown oil, which was used directly in the next reaction.

Step 3: To ethanol was added 2-(dimethylamino)-1-(3-nitro-2-(2,2,2-trifluoroethoxy)phenyl)ethan-1-one **(50-c,** 2.8g, 8.7mmol). The mixture was cooled to 0°C, and added with hydrazine hydrate dropwise. Upon the addition was completed, the reaction mixture was heated to 80°C with stirring for 1 hour. Upon indication of completed reaction by TLC, the reaction mixture was concentrated under reduced pressure, poured into DCM (50ml) to cause a solid precipitation, followed by filtration. The filtrate was concentrated and purified by column chromatography (EA: PE=2: 1) to provide 3-(3-nitro-2-(2,2,2-trifluoroethoxy)phenyl)-1H-pyrazole **(50-d,** 700mg, 2.32mmol, 27% yield), as a yellow oil. MS Calcd: 301; MS Found: 302 ($[M+H]^+$). Step 4: 3-(3-nitro-2-(2,2,2-trifluoroethoxy)phenyl)-1H-pyrazole **(50-a,** 700mg, 2.32mmol) and potassium carbonate (0.97g, 6.96mmol) were added to DMF(20ml), to which was added at room temperature iodomethane (329mg, 2.32mmol) and stirred overnight. Upon indication of completed reaction by TLC, the reaction mixture was poured into 30ml of water, and extracted with ethyl acetate (15ml). The organic layers were dried over anhydrous sodium sulfate, filtered by suction, concentrated and then purified by column chromatography (EA: PE=1:2) to provide 1-methyl-3-(3-nitro-2-(2,2,2-trifluoroethoxy)phenyl)-1H-pyrazole **(50-e,** 620mg, 2.05mmol, 88% yield). MS Calcd: 301; MS Found: 302 ($[M+H]^+$).

Step 5: 1-methyl-3-(3-nitro-2-(2,2,2-trifluoroethoxy)phenyl)-1H-pyrazole **(50-e,** 620mg, 2.05mmol) and palladium on carbon (100mg) were added to methanol(20ml). After atmosphere replacement by hydrogen three times, the reaction mixture was stirred under hydrogen atmosphere at room temperature overnight, and filtered by suction. The filtrate was concentrated under reduced pressure to provide 3-(1-methyl-1H-pyrazol-3-yl)-2-(2,2,2-trifluoroethoxy) aniline(**50-f**, 530mg, 1.96mmol, 95% yield), which was used directly in the next reaction without further purification. MS Calcd: 271; MS Found: 272 ($[M+H]^+$).

Step 6: 3-(1-methyl-1H-pyrazol-3-yl)-2-(2,2,2-trifluoroethoxy) aniline**(50-f**, 200mg, 0.74mmol) and 4,6-dichloro-N-methoxy nicotinamide **(int-1,** 149mg, 0.74mmol) were added to 2ml of anhydrous tetrahydrofuran. The mixture was added at room temperature with a solution of LiHMDS in tetrahydrofuran (1.5ml, 1.48mmol), and stirred at room temperature for 2 hours. The mixture was adjusted with aqueous hydrochloride (1N) to pH 5, and extracted with ethyl acetate (10ml). The combined organic layers were dried, concentrated and then purified by column chromatography (petroleum ether:ethyl acetate =1:2) to provide 6-chloro-N-methoxy-4-((3-(1-methyl-1H-pyrazol-3-yl)-2-(2,2,2-trifluoroethoxy)phenyl)amino)nicotinamide **(50-g,** 120°Cg, 0.26mmol, 35% yield). MS Calcd: 455; MS Found: 456 ($[M+H]^+$).

Step 7: 6-chloro-N-methoxy-4-((3-(1-methyl-1H-pyrazol-3-yl)-2-(2,2,2-trifluoroethoxy)phenyl)amino)nicotinamide **(50-g,** 50mg, 0.11mmol), cyclopropylcarboxamide (19mg, 0.22mmol), cesium carbonate (107mg, 0.33mmol), XantPhos(20mg, 0.03mmol) and $Pd_2(dba)_3$(25mg, 0.027mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and purified by high performance preparative thin layer chromatography (MeOH:DCM=1:20) to provide the title compound: 6-(cyclopropylcarboxamido)-N-methoxy-4-((3-(1-methyl-1H-pyrazol-3-yl)-2-(2,2,2-trifluoroethoxy)phenyl)amino)nicotinamide **(50,** 15mg, 0.03mmol, 27% yield). MS Calcd: 504; MS Found: 505 ($[M+H]^+$). $^1H$ NMR (400 MHz, DMSO-$d_6$) : δ 11.81 (s, 1H), 10.78 (s, 1H), 9.97 (s, 1H), 8.36 (s, 1H), 7.88 (s, 1H), 7.79 (s, 1H), 7.61-7.58 (m, 1H), 7.39-7.24 (m, 2H), 6.64 (s, 1H), 4.29-4.25(m, 2H), 3.91 (s, 3H), 3.71 (s, 3H), 1.97-1.94 (m, 1H), 0.77-0.74 (m, 4H).

Example **51**

6-((5-fluoropyridin-2-yl)amino)-N-methoxy-4-((3-(1-methyl-1H-pyrazol-3-yl)-2-(2,2,2-trifluoroethoxy)phenyl)amino)nicotinamide

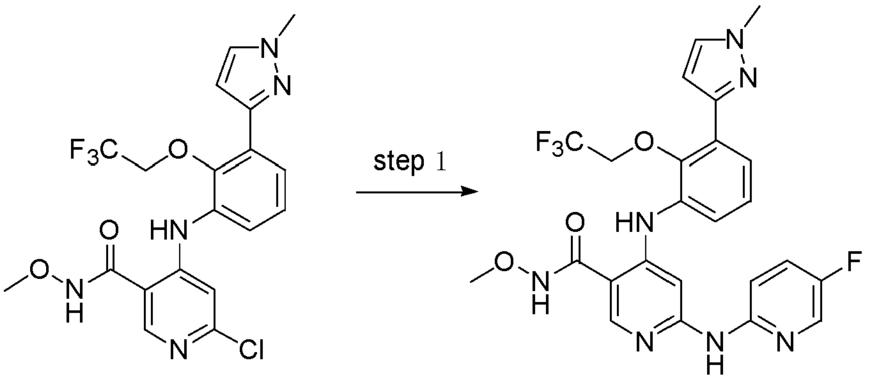


**50-g** **51**

Step 1: 6-chloro-N-methoxy-4-((3-(1-methyl-1H-pyrazol-3-yl)-2-(2,2,2-trifluoroethoxy)phenyl)amino)nicotinamide **(50-g,** 50mg, 0.11mmol), 5-fluoropyridin-2-ylamine (25mg, 0.22mmol), cesium carbonate (107mg, 0.33mmol), XantPhos(20mg, 0.03mmol) and $Pd_2(dba)_3$(25mg, 0.027mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and purified by high performance preparative thin layer chromatography (MeOH:DCM=1:20) to provide the title compound: 6-((5-fluoropyridin-2-yl)amino)-N-methoxy-4-((3-(1-methyl-1H-pyrazol-3-yl)-2-(2,2,2-trifluoroethoxy)phenyl)amino)nicotinamide **(51,** 12mg, 0.02mmol, 21% yield). MS Calcd: 531; MS Found: 532 ($[M+H]^+$). 1H NMR (400 MHz, DMSO-$d_6$) : δ 11.70 (s, 1H), 9.97 (s, 1H), 9.84 (s, 1H), 8.34 (s, 1H), 8.09 (d, *J* = 3.2, 1H), 7.80 (s, 1H), 7.67-7.48 (m, 6H), 6.65 (s, 1H), 4.30-4.28 (m, 2H), 3.91 (s, 3H), 3.71 (s, 3H).

Example **52**

6-(cyclopropylcarboxamido)-N-methoxy-4-((2-methoxy-3-(1-methyl-1H-1,2,4-triazol-3-yl)phenyl)amino)pyridazine-3-carboxamide

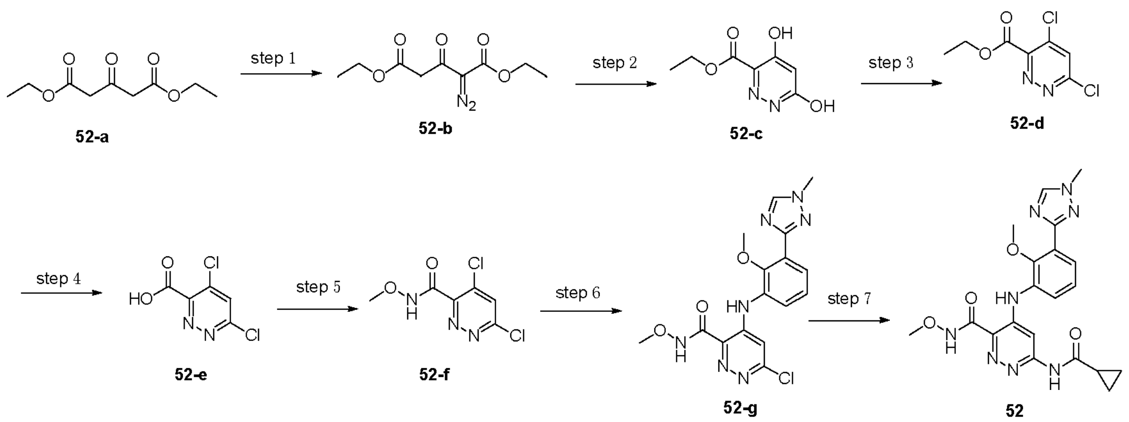


**52-a** **52-b** **52-c** **52-d** **52-e** **52-f** **52-g** **52**

Step 1: Diethyl 3-oxaglutarate **(52-a,** 7.6g, 37.6mmol), 4-acetamidobenzenesulfonyl azide (9g, 37.6mmol), and triethylamine (3.8g, 37.6mmol) were added to acetonitrile (100ml) and stirred at room temperature for 1 hour, followed by filtration. The filter cake was washed with diethyl ether, and the filtrate was concentrated. The residue was slurried with diethyl ether:petroleum ether (1:1), and filtered by suction. The filtrate was concentrated to provide diethyl 2-diazo-3-oxaglutarate **(52-b,** 9.2g), which was used directly in the next reaction without further purification.

Step 2: Diethyl 2-diazo-3-oxaglutarate **(52-b,** 7.2g, 32mmol) and triphenylphosphine (8.4g, 32mmol) was added to diethyl ether (100ml) and stirred at room temperature for 48 hours. The reaction mixture was concentrated, and added with mixed solvent of acetic acid and water (100ml: 10ml), heated to 120°C with stirring for 9 hours, and then concentrated directly. The residue was added to sodium bicarbonate aqueous solution (2N, 200ml), andwashed with ethyl acetate (100ml). The aqueous layer was adjusted to pH 3, and further extracted with ethyl acetate (200ml). The organic layers were dried, filtered by suction, and concentrated to obtain ethyl 4,6-dihydroxyl pyridazine-3-carboxylate, which was used directly in the next reaction without further purification. **(52-c,** 3.6g, 19.5mmol, 61% yield). MS Calcd: 184; MS Found: 183 ($[M-H]^-$).

Step 3: To phosphorus oxychloride (50ml) was added ethyl 4,6-dihydroxyl pyridazine-3-carboxylate **(52-c,** 3.6g, 19.5mmol), heated to 100°C with stirring for 3 hours, and concentrated under reduced pressure. The residue was poured into 50ml of water, and extracted with ethyl acetate (20ml ), The organic layer was dried, filtered by suction, and concentrated. The residue was purified by column chromatography (ethyl acetate:petroleum ether=1:4) to provide ethyl 4,6-dichloropyridazine-3-carboxylate **(52-d,** 2.8g, 12.7mmol, 65% yield), which was used directly in the next reaction without further purification.

Step 4: To a mixed solvent of tetrahydrofuran and water (40ml:10ml) was added ethyl 4,6-dichloropyridazine-3-carboxylate **(52-d,** 2.8g, 12.7mmol), followed by lithium hydroxide (914mg, 38.1mmol), and stirred at room temperature for 2 hours. The mixture was adjusted with aqueous hydrochloride (1N) to pH 5, and concentrated under reduced pressure. The residue was washed with a mixture of methanol and dichloromethane, and filtered by suction. The filtrate was concentrated under reduced pressure to provide 4,6-dichloropyridazine-3-carboxylic acid **(52-e,** 1.8g, 9.3mmol, 73% yield).

Step 5: To dichloromethane(20ml) was added 4,6-dichloropyridazine-3-carboxylic acid **(52-e,** 1.8g, 9.3mmol), followed by catalytic amount of DMF. The reaction mixture was cooled to 0°C, and slowly added with oxalyl chloride dropwise. Upon the addition was completed, the reaction mixture was heated to room temperature slowly, stirred for 2 hours, and concentrated under reduced pressure. The residue was dissolved in 5ml of ethyl acetate, and added slowly to a solution of methoxy amine (900mg, 18mmol) and potassium carbonate (3.7g, 27mmol) in mixed solvent of water and ethyl acetate, and stirred at room temperature overnight, added with ethyl acetate (50ml). After phase separation, the organic layer was dried and concentrated, and the residue was purified by column chromatography (ethyl acetate:petroleum ether=1:2) to provide 4,6-dichloro-N-methoxy pyridazine-3-carboxamide **(52-f,** 1.3g, 5.8mmol, 62% yield). MS Calcd: 221; MS Found: 220 ($[M-H]^-$).

Step 6: To 5ml of anhydrous tetrahydrofuran was added 4,6-dichloro-N-methoxy pyridazine-3-carboxamide **(52-f,** 300mg, 1.35mmol) and 2-methoxy-3-(1-methyl-1H-1,2,4-triazol-3-yl)aniline (275.2mg, 1.35mmol). The mixture was added at room temperature with a solution of LiHMDS in tetrahydrofuran (2.7ml, 2.7mmol), and stirred at room temperature for 2 hours. The mixture was adjusted with aqueous hydrochloride (1N) to pH 5, and extracted with ethyl acetate (10ml). The combined organic layers were dried, concentrated and then purified by column chromatography (PE:EA =1:2) to provide 6-chloro-N-methoxy-4-((2-methoxy-3-(1-methyl-1H-1,2,4-triazol-3-yl)phenyl)amino)pyridazine-3-carboxamide(**52-g**, 150mg, 0.39mmol, 29% yield). MS Calcd: 389; MS Found: 390 ($[M-H]^-$).

Step 7: 6-chloro-N-methoxy-4-((2-methoxy-3-(1-methyl-1H-1,2,4-triazol-3-yl)phenyl)amino)pyridazine-3-carboxamide(**52-g**, 50mg, 0.13mmol), cyclopropylcarboxamide (22mg, 0.26mmol), cesium carbonate (127mg, 0.39mmol), XantPhos(30mg, 0.04mmol) and $Pd_2(dba)_3$(24mg, 0.026mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and purified by high performance preparative thin layer chromatography (MeOH:DCM=1:20) to provide the title compound: 6-chloro-N-methoxy-4-((2-methoxy-3-(1-methyl-1H-1,2,4-triazol-3-yl)phenyl)amino)pyridazine-3-carboxamide **(52,** 13mg, 0.03mmol, 23% yield). MS Calcd: 438; MS Found: 439 ($[M+H]^+$). $^1H$ NMR (400 MHz, DMSO-d6)$\delta$11.64 (s, 1H), 11.34 (s, 1H), 10.53 (s, 1H), 8.57 (s, 1H), 8.56 (s, 1H), 7.63 (ddd, *J* = 12.8, 8.0, 1.6 Hz, 2H), 7.33 (t, *J* = 8.0 Hz, 1H), 3.95 (s, 6H), 3.94 (s, 3H), 2.06 - 1.93 (m, 1H), 0.78-0.74 (m, 4H).

Example **53**

6-(cyclopropylcarboxamido)-N-methoxy-4-((2-methoxy-3-(1-methyl-1H-pyrazol-3-yl)phenyl)amino)pyridazine-3-carboxamide

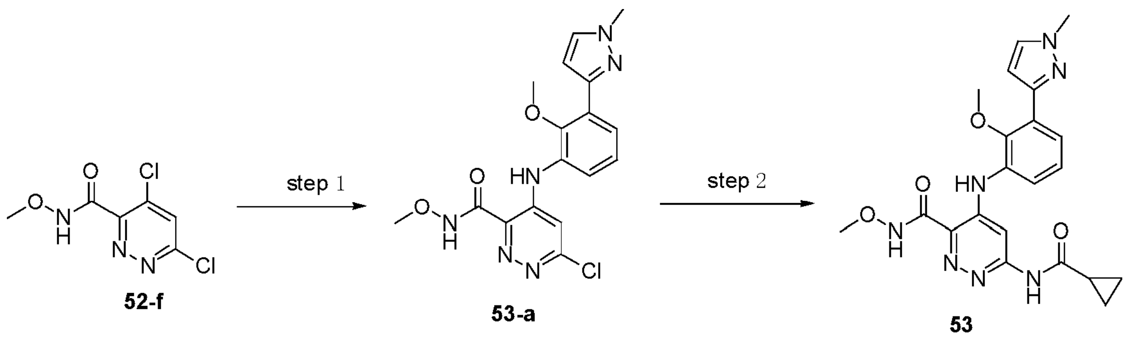


52-f 53-a 53

Step 1: To 5ml of anhydrous tetrahydrofuran were added 4,6-dichloro-N-methoxy pyridazine-3-carboxamide **(52-f,** 260mg, 1.18mmol) and 2-methoxy-3-(1-methyl-1H-pyrazol-3-yl)aniline (252mg, 1.18mmol). The mixture was added at room temperature with a solution of LiHMDS in tetrahydrofuran (2.5ml, 2.4mmol), and stirred at room temperature for 2 hours. The mixture was adjusted with aqueous hydrochloride (1N) to pH 5, and extracted with ethyl acetate (10ml). The combined organic layers were dried, concentrated and then purified by column chromatography (PE:EA =1:1) to provide 6-chloro-N-methoxy-4-((2-methoxy-3-(1-methyl-1H-pyrazol-3-yl)phenyl)amino)pyridazine-3-carboxamide **(53-a,** 124mg, 0.32mmol, 27% yield), as a white solid. MS Calcd: 388; MS Found: 389 ($[M+H]^+$).

Step 2: 6-chloro-N-methoxy-4-((2-methoxy-3-(1-methyl-1H-pyrazol-3-yl)phenyl)amino)pyridazine-3-carboxamide **(53-a,** 50mg, 0.13mmol), cyclopropylcarboxamide (22mg, 0.26mmol), cesium carbonate (127mg, 0.39mmol), XantPhos(30mg, 0.04mmol) and $Pd_2(dba)_3$(24mg, 0.026mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and purified by high performance preparative thin layer chromatography (MeOH:DCM=1:20) to provide the title compound: 6-(cyclopropylcarboxamido)-N-methoxy-4-((2-methoxy-3-(1-methyl-1H-pyrazol-3-yl)phenyl)amino)pyridazine-3-carboxamide (53, 8mg, 0.018mmol, 14% yield). MS Calcd: 437; MS Found: 438 ($[M+H]^+$). $^1$H NMR (400 MHz, DMSO-d6)δ11.33 (s, 1H), 10.65 (s, 1H), 10.52 (s, 1H), 8.19 (s, 1H), 7.93 (d, *J* = 4.5 Hz, 1H), 7.52 (d, *J* = 7.2 Hz, 1H), 7.37 (d, *J* = 7.2 Hz, 1H), 7.30 - 7.16 (m, 2H), 3.90 (s, 3H), 3.76 (s, 3H), 3.60 (s, 3H), 2.06 - 1.93 (m, 1H), 0.78-0.74 (m, 4H)

Example **54**

6-(cyclopropylcarboxamido)-4-((2-(difluoromethoxy)-3-(1-methyl-1H-pyrazol-4-yl)phenyl)amino)-N-methoxy nicotinamide

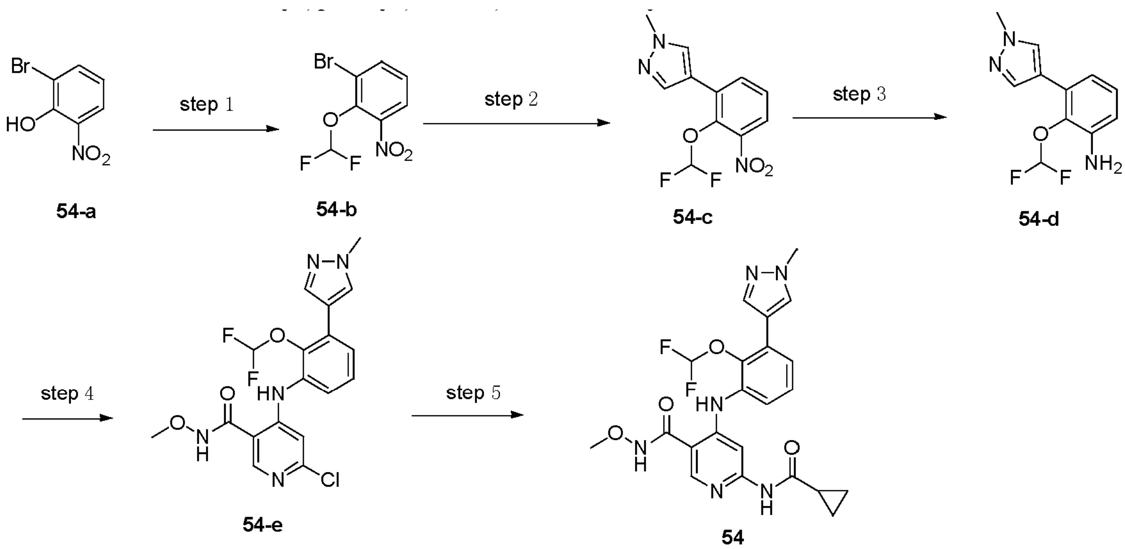


54-a 54-b 54-c 54-d 54-e 54

Step 1: 2-bromo-6-nitrophenol **(54-a,** 900mg, 4.17mmol), 2-chloro-2,2-difluoro acetic acid (812mg, 6.25mmol), and potassium carbonate (2.3g, 16.7mmol) were added to DMF(15ml). The reaction mixture was heated to 100°C, and stirred for 3 hours. Upon indication of completed reaction by TLC, the reaction mixture was cooled to room temperature,

poured into 50ml of water, and extracted with ethyl acetate. After drying over anhydrous sodium sulfate, the organic layer was filtered by suction, concentrated, purified by column chromatography (ethyl acetate:petroleum ether=1:60) to provide the target compound 1-bromo-2-(difluoromethoxy)-3-nitrobenzene. **(54-b,** 530mg, 1.98mmol, 47% yield).

Step 2: 1-bromo-2-(difluoromethoxy)-3-nitrobenzene **(54-b,** 500mg, 1.87mmol), 1-methyl-4-(4,4,5,5-tetramethyl-1,3,2-dioxaborolan-2-yl)-1H-pyrazole (389mg, 1.87mmol), Pd(dppf)$Cl_2$(20mg, 0.02mmol) and sodium carbonate (594mg, 5.61mmol) were dissolved in a mixed solvent of water and dioxane (2ml/8mL), heated to 100°C to react for 3h. The reaction mixture was concentrated, added with water (10ml), and extracted with ethyl acetate (10ml). The combined ethyl acetate layers were dried over anhydrous sodium sulfate, filtered by suction, and concentrated to obtain a crude material, which was purified by column chromatography (PE:EA =3:1) to provide 4-(2-(difluoromethoxy)-3-nitrophenyl)-1-methyl-1H-pyrazole **(54-c,** 420mg, 1.56mmol, 83% yield), as a yellow solid. MS Calcd: 269; MS Found: 270 ($[M+H]^+$).

Step 3: To methanol (20ml) were added 4-(2-(difluoromethoxy)-3-nitrophenyl)-1-methyl-1H-pyrazole **(54-c,** 420mg, 1.56mmol) and palladium on carbon (40mg). After atmosphere replacement by hydrogen three times, the reaction mixture was stirred under hydrogen atmosphere at room temperature overnight, and filtered by suction. The filtrate was concentrated under reduced pressure to provide 2-(difluoromethoxy)-3-(1-methyl-1H-pyrazol-4-yl)aniline **(54-d,** 360mg, 1.50mmol, 95% yield), as a grey oil, which was used directly in the next reaction without further purification. MS Calcd: 239; MS Found: 240 ($[M+H]^+$).

Step 4: To 5ml of anhydrous tetrahydrofuran were added 4,6-dichloro-N-methoxy nicotinamide **(int-1,** 332mg, 1.50mmol) and 2-(difluoromethoxy)-3-(1-methyl-1H-pyrazol-4-yl)aniline **(54-d,** 360mg, 1.50mmol). The mixture was added at room temperature with a solution of LiHMDS in tetrahydrofuran (3ml, 3mmol), and stirred at room temperature for 2 hours. The mixture was adjusted with aqueous hydrochloride (1N) to pH 5, and extracted with ethyl acetate (10ml). The combined organic layers were dried, concentrated and then purified by column chromatography (PE:EA =1:1) to provide 6-chloro-4-((2-(difluoromethoxy)-3-(1-methyl-1H-pyrazol-4-yl)phenyl)amino)-N-methoxy nicotinamide **(54-e,** 210mg, 0.49mmol, 33% yield), as a white solid. MS Calcd: 423; MS Found: 424 ($[M+H]^+$).

Step 5: 6-chloro-4-((2-(difluoromethoxy)-3-(1-methyl-1H-pyrazol-4-yl)phenyl)amino)-N-methoxy nicotinamide **(54-e,** 50mg, 0.12mmol), cyclopropylcarboxamide (22mg, 0.26mmol), cesium carbonate (127mg, 0.36mmol), Xant-Phos(30mg, 0.04mmol) and $Pd_2(dba)_3$(24mg, 0.024mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and purified by high performance preparative thin layer chromatography (MeOH:DCM=1:20) to provide the title compound: 6-(cyclopropylcarboxamido)-4-((2-(difluoromethoxy)-3-(1-methyl-1H-pyrazol-4-yl)phenyl)amino)-N-methoxy nicotinamide **(54,** 9mg, 0.02mmol, 16.7% yield). MS Calcd: 472; MS Found: 473 ($[M+H]^+$). $^1H$ NMR (400 MHz, DMSO-$d_6$) : δ 11.83 (s, 1H), 10.79 (s, 1H), 10.01 (s, 1H), 8.36 (s, 1H), 8.10 (s, 1H), 7.88 (s, 1H), 7.85 (s, 1H), 7.45-7.43 (m, 1H), 7.34-7.32 (m, 2H), 6.95-6.59 (m, 1H), 3.89 (s, 3H), 3.72 (s, 3H), 1.98-1.95 (m, 1H), 0.76-0.74 (m, 4H).

Example **55**

6-(cyclopropylcarboxamido)-N-methoxy-4-((2-methoxy-3-(5-methyl-1, 2,4-oxadiazol-3-yl)phenyl)amino)nicotinamide

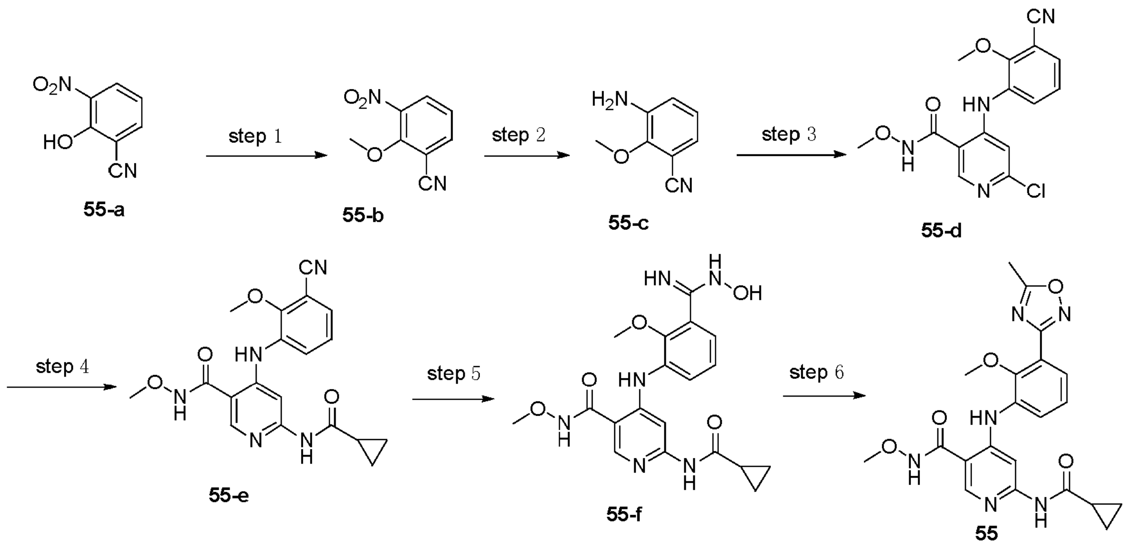


55-a 55-b 55-c 55-d 55-e 55-f 55

Step 1: 2-hydroxyl-3-nitrobenzonitrile **(55-a,** 1.2g, 7.3mmol), iodomethane (3.1g, 22mmol) and potassium carbonate (5.9g, 43mmol) were added to DMF, and stirred at room temperature overnight. The reaction mixture was poured into 150ml of water, filtered by suction, solid was washed with water, and dried to provide 2-methoxy-3-nitrobenzonitrile **(55-b,** 800mg, 4.5mmol, 62% yield).

Step 2: To methanol(20ml) were added 2-methoxy-3-nitrobenzonitrile(**55-b,** 800mg, 4.5mmol) and palladium on carbon (40mg). After atmosphere replacement by hydrogen three times, the reaction mixture was stirred under hydrogen atmosphere at room temperature overnight, and filtered by suction. The filtrate was concentrated under reduced pressure to provide 3-amino-2-methoxy benzonitrile **(55-c,** 630mg, 4.2mmol, 93% yield), which was used directly in the next reaction without further purification. MS Calcd: 148; MS Found: 149 ($[M+H]^+$).

Step 3: To 5ml of anhydrous tetrahydrofuran were added 4,6-dichloro-N-methoxy nicotinamide **(int-1,** 332mg, 1.50mmol) and 3-amino-2-methoxy benzonitrile **(55-c,** 230mg, 1.55mmol). The mixture was added at room temperature with a solution of LiHMDS in tetrahydrofuran (3ml, 3mmol), and stirred at room temperature for 2 hours. The mixture was adjusted with aqueous hydrochloride (1N) to pH 5, and extracted with ethyl acetate (10ml). The combined organic layers were dried, concentrated and then purified by column chromatography (PE:EA =1:1) to provide 6-chloro-4-((3-cyano-2-methoxy phenyl)amino)-N-methoxy nicotinamide **(55-d,** 152mg, 0.46mmol, 29% yield). MS Calcd: 332; MS Found: 333 ($[M+H]^+$).

Step 4: 6-chloro-4-((3-cyano-2-methoxy phenyl)amino)-N-methoxy nicotinamide **(55-d,** 52mg, 0.16mmol), cyclopropylcarboxamide (27mg, 0.32mmol), cesium carbonate (156mg, 0.48mmol), XantPhos(30mg, 0.04mmol) and $Pd_2(dba)_3$(24mg, 0.024mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and purified by high performance preparative thin layer chromatography (MeOH:DCM=1:20) to provide 4-((3-cyano-2-methoxy phenyl)amino)-6-(cyclopropylcarboxamido)-N-methoxy nicotinamide **(55-e,** 15mg, 0.04mmol, 25% yield). MS Calcd: 381; MS Found: 382 ($[M+H]^+$).

Step 5: To a mixed solvent of ethanol and water (1ml: 2ml) were added 4-((3-cyano-2-methoxy phenyl) amino)-6-(cyclopropylcarboxamido)-N-methoxy nicotinamide **(55-e,** 30mg, 0.08mmol) and freed hydroxylamine (11mg, 0.32mmol). The reaction mixture was heated to 90°C with stirring for 6 hours, and concentrated under reduced pressure. The residue was purified by high performance preparative thin layer chromatography ( methanol: dichloromethane=1:10) to provide 6-(cyclopropylcarboxamido)-4-((3-(N-hydroxyaminocarboxamido)-2-methoxy phenyl)amino)-N-methoxy nicotinamide **(55-f,** 9mg, 0.02mmol, 13% yield). MS Calcd: 414; MS Found: 413 ($[M-H]^-$). $^1$H NMR (400 MHz, DMSO-$d_6$) : δ 11.86 (s, 1H), 10.82 (s, 1H), 10.12 (s, 1H), 9.53 (s, 1H), 8.36 (s, 1H), 8.07 (s, 1H), 7.44 (dd, *J*=4, 5.6, 1H), 7.15-7.12 (m, 2H), 5.76 (s, 2H), 3.73 (s, 3H), 3.70 (s, 3H), 1.99-1.96 (m, 1H), 0.78-0.77 (m, 4H).

Step 6: To 1,4-dioxane were added 6-(cyclopropylcarboxamido)-4-((3-(N-hydroxyaminocarboxamido)-2-methoxy phenyl)amino)-N-methoxy nicotinamide **(55-f,** 20mg, 0.05mmol), acetic anhydride (5mg, 0.05mmol), heated to 90°C with stirring for 5 hours, and concentrated under reduced pressure. The residue was purified by high performance preparative thin layer chromatography ( methanol: dichloromethane=1:30) to provide 6-(cyclopropylcarboxamido)-N-methoxy-4-((2-methoxy-3-(5-methyl-1, 2,4-oxadiazol-3-yl)phenyl)amino)nicotinamide **(55,** 9mg, 0.02mmol, 40% yield), as a white solid. MS Calcd: 438; MS Found: 439 ($[M+H]^+$). $^1$H NMR (400 MHz, DMSO-$d_6$) : δ 11.86 (s, 1H), 10.83 (s, 1H), 10.30 (s, 1H), 8.40 (s, 1H), 8.07 (s, 1H), 7.65-7.60 (m, 2H), 7.33 (dd, *J* = 8.0, 4.0 Hz, 1H), 3.74 (s, 3H), 3.73 (s, 3H), 2.68 (s, 3H), 2.00-1.96 (m, 1H), 0.78-0.74 (m, 4H).

Example **56**

6-(cyclopropylcarboxamido)-N-methoxy-4-((4-(N-methyl sulfonamido)pyridin-3-yl)amino)nicotinamide

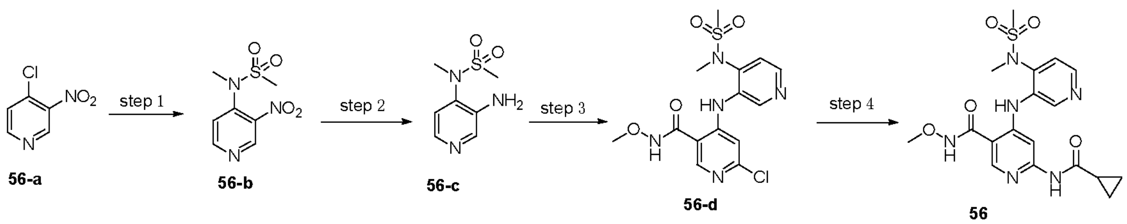


Step 1: To DMF were added 4-chloro-3-nitropyridine **(56-a,** 1g, 6.3mmol), N-methyl methanesulfonamide (680mg, 6.3mmol) and potassium carbonate (2.6g, 19mmol). The reaction mixture was heated to 80°C with stirring for 2 hours. When TLC indicated that all starting material was depleted, the reaction mixture was cooled to room temperature, poured into 100ml of water to cause a solid precipitation, which was filtered by suction to provide N-methyl-N-(3-nitropyridin-4-yl) methanesulfonamide **(56-b,** 920mg, 3.98mmol, 63% yield). MS Calcd: 231; MS Found: 232 ([M-H]$^-$).

Step 2: To methanol(20ml) were added N-methyl-N-(3-nitropyridin-4-yl) methanesulfonamide **(56-b,** 920mg, 3.98mmol) and palladium on carbon (100mg). After atmosphere replacement by hydrogen three times, the reaction mixture was stirred under hydrogen atmosphere at room temperature overnight, and filtered by suction. The filtrate was concentrated under reduced pressure to provide N-(3-aminopyridin-4-yl)-N-methyl methanesulfonamide **(56-c,** 730mg, 3.6mmol, 90% yield), which was used directly in the next reaction without further purification. MS Calcd: 201; MS Found: 202 ([M+H]$^+$).

Step 3: To 2.5ml of anhydrous tetrahydrofuran were added 4,6-dichloro-N-methoxy nicotinamide **(int-1,** 230mg, 1.14mmol) and N-(3-aminopyridin-4-yl)-N-methyl methanesulfonamide **(56-c,** 230mg, 1.14mmol). The mixture was added at room temperature with a solution of LiHMDS in tetrahydrofuran (2.5ml, 2.5mmol), and stirred at room temperature for 2 hours. The mixture was adjusted with aqueous hydrochloride (1N) to pH 5, and extracted with ethyl acetate (10ml). The combined organic layers were dried, concentrated and then purified by column chromatography (PE:EA =1:1) to provide 6-chloro-N-methoxy-4-((4-(N-methyl methanesulfonamido)pyridin-3-yl)amino)nicotinamide **(56-d,** 125mg, 0.32mmol, 28% yield). MS Calcd: 385; MS Found: 386 ([M+H]$^+$).

Step 4: 6-chloro-N-methoxy-4-((4-(N-methyl methanesulfonamido)pyridin-3-yl)amino)nicotinamide **(56-d,** 50mg, 0.13mmol), cyclopropylcarboxamide (22mg, 0.26mmol), cesium carbonate (130mg, 0.4mmol), XantPhos(20mg, 0.03mmol) and $Pd_2(dba)_3$(19mg, 0.02mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and purified by high performance preparative thin layer chromatography (MeOH:DCM=1:20) to provide the title compound: 6-(cyclopropylcarboxamido)-N-methoxy-4-((4-(N-methyl methanesulfonamido)pyridin-3-yl)amino)nicotinamide **(56,** 12mg, 0.03mmol, 23% yield). MS Calcd: 434; MS Found: 435 ([M+H]$^+$). $^1$H NMR (400 MHz, DMSO-*d$_6$*) : δ 11.86 (s, 1H), 10.84 (s, 1H), 9.99 (s, 1H), 8.72 (s, 1H), 8.43 (d, *J* = 5.2, 1H), 8.36 (s, 1H), 7.84 (s, 1H), 7.63 (d, *J* = 5.2, 1H), 3.72 (s, 3H), 3.16 (s, 6H), 1.99-1.94 (m, 1H), 0.77-0.6 (m, 4H).

Example **57**

4-((3-carbamoyl-2-methoxy phenyl)amino)-6-(cyclopropylcarboxamido)-N-methoxy nicotinamide

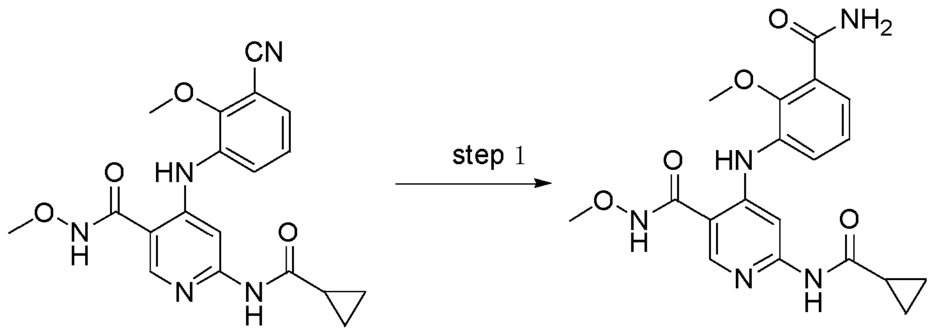


**55-e** **57**

Step 1: 4-((3-cyano-2-methoxy phenyl)amino)-6-(cyclopropylcarboxamido)-N-methoxy nicotinamide **(55-e,** 20mg, 0.05mmol) and potassium carbonate (14mg, 0.1mmol) were added to a mixed solvent of water and ethanol. The reaction mixture was heated to 80°C with stirring for 2 hours, and concentrated under reduced pressure. The residue was purified by high performance preparative thin layer chromatography ( methanol: dichloromethane=1:20) to provide the title compound: 4-((3-carbamoyl-2-methoxy phenyl)amino)-6-(cyclopropylcarboxamido)-N-methoxy nicotinamide **(57,** 8mg, 0.02mmol, 40% yield). MS Calcd: 399; MS Found: 400 ([M+H]$^+$). $^1$H NMR (400 MHz, DMSO-*d$_6$*) : δ 11.84 (s, 1H), 10.82 (s, 1H), 10.13 (s, 1H), 8.37 (s, 1H), 8.03 (s, 1H), 7.74 (s, 1H), 7.52-7.49 (m, 2H), 7.32-7.30 (dd, *J* = 8.0, 1.6 Hz, 1H), 7.20 (t, *J* = 8.0 Hz, 1H), 3.70 (s, 6H), 1.99-1.94 (m, 1H), 0.78-0.76 (m, 4H).

Example **58**

6-((5-fluoropyridin-2-yl)amino)-N-methoxy-4-((4-(N-methyl methanesulfonamido)pyridin-3-yl)amino)nicotinamide

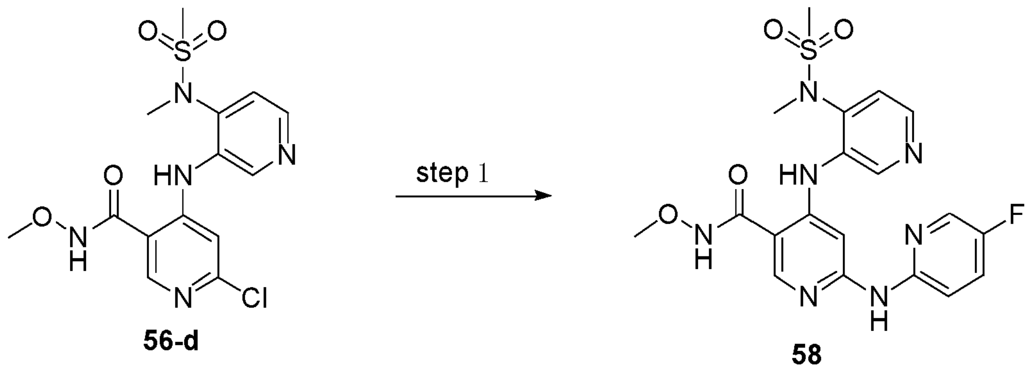


56-d

58

Step 1: 6-chloro-N-methoxy-4-((4-(N-methyl methanesulfonamido)pyridin-3-yl)amino)nicotinamide **(56-d,** 50mg, 0.13mmol), 5-fluoropyridin-2-ylamine (30mg, 0.26mmol), cesium carbonate (130mg, 0.4mmol), XantPhos(20mg, 0.03mmol) and $Pd_2(dba)_3$(19mg, 0.02mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and purified by high performance preparative thin layer chromatography (MeOH:DCM=1:20) to provide the title compound: 6-((5-fluoropyridin-2-yl)amino)-N-methoxy-4-((4-(N-methyl methanesulfonamido)pyridin-3-yl)amino)nicotinamide **(58,** 9mg, 0.02mmol, 15% yield). MS Calcd: 461; MS Found: 462 ($[M+H]^+$). 1H NMR (400 MHz, DMSO-$d_6$) : δ 11.86 (s, 1H), 9.98 (s, 1H), 9.86 (s, 1H), 8.83 (s, 1H), 8.44 (d, $J$ = 5.2 Hz, 1H), 8.33 (s, 1H), 8.08 (d, $J$ = 3.2 Hz, 1H), 7.78-7.77 (m, 1H), 7.64-7.61 (m, 2H), 7.39 (s, 1H), 3.72 (s, 3H), 3.20 (s, 3H), 3.19 (s, 3H).

Example **59**

6-(cyclopropylcarboxamido)-N-methoxy-4-((6-methyl-2-(N-methyl methanesulfonamido)pyridin-3-yl)amino)nicotinamide

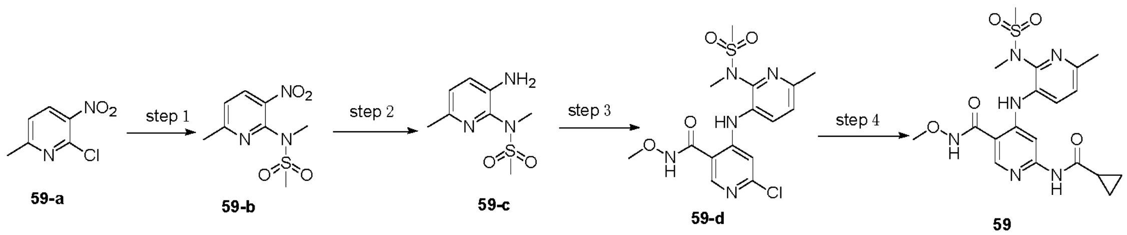


59-a 59-b 59-c 59-d 59

Step 1: To DMF were added 2-chloro-6-methyl-3-nitropyridine **(59-a,** 1g, 5.8mmol), N-methyl methanesulfonamide (630mg, 5.8mmol) and potassium carbonate (2.4g, 17.4mmol). The mixture was heated to 80°C with stirring for 2 hours. When TLC indicated that all starting material was depleted, the reaction mixture was cooled to room temperature, poured into 100ml of water to cause solid precipitation, which was filtered by suction to provide N-methyl-N-(6-methyl-3-nitropyridin-2-yl) methanesulfonamide **(59-b,** 965mg, 3.9mmol, 67% yield). MS Calcd: 245; MS Found: 246 ($[M-H]^-$).

Step 2: To methanol(20ml) were added N-methyl-N-(6-methyl-3-nitropyridin-2-yl) methanesulfonamide **(59-b,** 965mg, 3.9mmol) and palladium on carbon (100mg). After atmosphere replacement by hydrogen three times, the reaction mixture was stirred under hydrogen atmosphere at room temperature overnight, and filtered by suction. The filtrate was concentrated under reduced pressure to provide N-(3-amino-6-methyl pyridin-2-yl)-N-methyl methanesulfonamide **(59-c,** 753mg, 3.5mmol, 89% yield), which was used directly in the next reaction without further purification. MS Calcd: 215; MS Found: 216 ($[M+H]^+$).

Step 4: To 2.5ml of anhydrous tetrahydrofuran were added N-(3-amino-6-methyl pyridin-2-yl)-N-methyl methanesulfonamide **(59-c,** 253mg, 1.17mmol) and 4,6-dichloro-N-methoxy nicotinamide **(int-1,** 230mg, 1.14mmol). The mixture was added at room temperature with a solution of LiHMDS in tetrahydrofuran (2.5ml, 2.5mmol), and stirred at room temperature for 2 hours. The mixture was adjusted with aqueous hydrochloride (1N) to pH 5, and extracted with ethyl acetate (10ml). The combined organic layers were dried, concentrated and then purified by column chromatography (PE:EA =1:1) to provide 6-chloro-N-methoxy-4-((6-methyl-2-(N-methyl methanesulfonamido)pyridin-3-yl)amino)nicotinamide **(59-d,** 138mg, 0.36mmol, 29% yield). MS Calcd: 399; MS Found: 400 ($[M+H]^+$).

Step 5: 6-chloro-N-methoxy-4-((6-methyl-2-(N-methyl methanesulfonamido)pyridin-3-yl)amino)nicotinamide **(59-d,** 50mg, 0.13mmol), cyclopropylcarboxamide (22mg, 0.26mmol), cesium carbonate (130mg, 0.4mmol), Xant-

Phos(20mg, 0.03mmol) and $Pd_2(dba)_3$(19mg, 0.02mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and purified by high performance preparative thin layer chromatography (MeOH:DCM=1:20) to provide the title compound: 6-(cyclopropylcarboxamido)-N-methoxy-4-((6-methyl-2-(N-methyl methanesulfonamido)pyridin-3-yl)amino)nicotinamide **(59,** 18mg, 0.04mmol, 31% yield). MS Calcd: 448; MS Found: 449 ($[M+H]^+$). 1H NMR (400 MHz, DMSO-$d_6$) : δ 11.81 (s, 1H), 10.82 (s, 1H), 10.01 (s, 1H), 8.35 (s, 1H), 7.84-7.82 (m, 2H), 7.34-7.32 (d, *J* = 8.4 Hz, 1H), 3.71 (s, 3H), 3.17 (s, 3H), 3.10 (s, 3H), 2.47 (s, 3H), 1.97-1.95 (m, 1H), 0.78-0.76 (m, 4H).

Example **60**

6-(((5-fluoropyridin-2-yl)amino)-N-methoxy-4-((6-methyl-2-(N-methyl methanesulfonamido)pyridin-3-yl)amino)nicotinamide

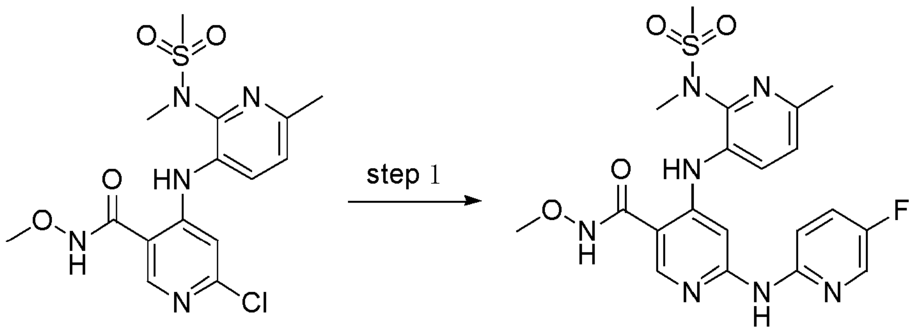


**59-d** **60**

Step 1: 6-chloro-N-methoxy-4-((6-methyl-2-(N-methyl methanesulfonamido)pyridin-3-yl)amino)nicotinamide **(59-d,** 50mg, 0.13mmol), 5-fluoropyridin-2-ylamine (30mg, 0.26mmol), cesium carbonate (130mg, 0.4mmol), Xant-Phos(20mg, 0.03mmol) and $Pd_2(dba)_3$(19mg, 0.02mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and purified by high performance preparative thin layer chromatography (MeOH:DCM=1:20) to provide the title compound: 6-(((5-fluoropyridin-2-yl)amino)-N-methoxy-4-((6-methyl-2-(N-methyl methanesulfonamido)pyridin-3-yl)amino)nicotinamide **(60,** 13mg, 0.03mmol, 23% yield). MS Calcd: 475; MS Found: 476 ($[M+H]^+$). 1H NMR (400 MHz, DMSO-$d_6$) : δ 11.72 (s, 1H), 10.06 (s, 1H), 9.86 (s, 1H), 8.32 (s, 1H), 8.18 (s, 1H), 7.96-7.94 (d, *J* = 8.4 Hz, 1H), 7.66 (d, *J* = 5.2 Hz, 2H), 7.55 (s, 1H), 7.42 (d, *J* = 8.4 Hz, 1H), 3.71 (s, 3H), 3.21 (s, 3H), 3.12 (s, 3H), 2.49 (s, 3H).

Example **61**

4-((4-(cyclopropyl methoxy)-2-(N-methyl methanesulfonamido)phenyl)amino)-6-((5-fluoropyridin-2-yl)amino)-N-methoxy nicotinamide

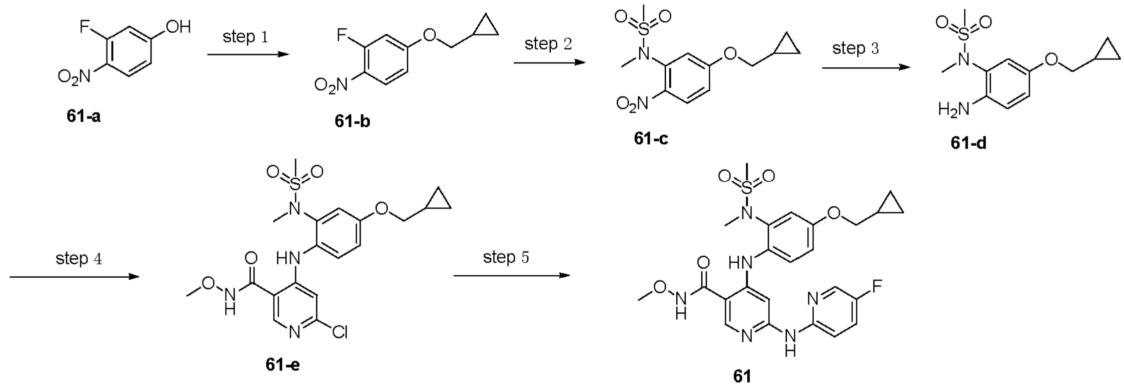


Step 1: To DMF were added 3-fluoro-4-nitrophenol(61-a, 1g, 6.3mmol), (bromo methyl)cyclopropane(850mg, 6.3mmol), and potassium carbonate (2.6g, 18.9mmol). The reaction mixture was heated to 90°C with stirring for 5 hours, followed by cooling to room temperature, added with water, and extracted with ethyl acetate. The organic layer was washed with water (30ml), dried over anhydrous sodium sulfate and filtered by suction, and concentrated under reduced pressure to provide 4-(cyclopropyl methoxy)-2-fluoro-1-nitrobenzene **(61-b,** 1.1g, 5.2mmol, 83% yield). MS Calcd: 211; MS Found: 212 ([M+H]$^+$).

Step 2: To DMF were added 4-(cyclopropyl methoxy)-2-fluoro-1-nitrobenzene (61-b, 1.1g, 5.2mmol), N-methyl methanesulfonamide (630mg, 5.8mmol) and potassium carbonate (2.2g, 15.6mmol). The mixture was heated to 80°C with stirring for 2 hours. When TLC indicated that all starting material was depleted, the reaction mixture was cooled to room temperature, poured into 100ml of water, extracted with ethyl acetate (100ml). The combined organic layers were dried over anhydrous sodium sulfate, filtered by suction, and concentrated under reduced pressure. The residue was purified by column chromatography (ethyl acetate:petroleum ether=1:4) to provide N-(5-(cyclopropyl methoxy)-2-nitrophenyl)-N-methyl methanesulfonamide **(61-c,** 862mg, 2.9mmol, 56% yield). MS Calcd: 300; MS Found: 301 ([M-H]$^-$).

Step 3: To methanol(20ml) were added N-(5-(cyclopropyl methoxy)-2-nitrophenyl)-N-methyl methanesulfonamide **(61-c,** 862mg, 2.9mmol) and palladium on carbon (100mg). After atmosphere replacement by hydrogen three times, the mixture was stirred under hydrogen atmosphere at room temperature overnight, and filtered by suction. The filtrate was concentrated under reduced pressure to provide N-(2-amino-5-(cyclopropyl methoxy)phenyl)-N-methyl methanesulfonamide **(61-d,** 731mg, 2.7mmol, 93% yield), which was used directly in the next reaction without further purification. MS Calcd: 270; MS Found: 271 ([M+H]$^+$).

Step 4: To 2.5ml of anhydrous tetrahydrofuran were added N-(2-amino-5-(cyclopropyl methoxy)phenyl)-N-methyl methanesulfonamide **(61-d,** 261mg, 0.96mmol) and 4,6-dichloro-N-methoxy nicotinamide **(int-1,** 200mg, 1mmol). The mixture was added at room temperature with a solution of LiHMDS in tetrahydrofuran (2ml, 2mmol), and stirred at room temperature for 2 hours. The mixture was adjusted with aqueous hydrochloride (1N) to pH 5, and extracted with ethyl acetate (10ml). The combined organic layers were dried, concentrated and then purified by column chromatography (PE:EA =1:2) to provide 6-chloro-4-((4-(cyclopropyl methoxy)-2-(N-methyl methanesulfonamido)phenyl)amino)-N-methoxy nicotinamide **(61-e,** 185mg, 0.41mmol, 43% yield). MS Calcd: 454; MS Found: 455 ([M+H]$^+$).

Step 5: 6-chloro-4-((4-(cyclopropyl methoxy)-2-(N-methyl methanesulfonamido)phenyl)amino)-N-methoxy nicotinamide **(61-e,** 50mg, 0.11mmol), 5-fluoropyridin-2-ylamine (19mg, 0.22mmol), cesium carbonate (107mg, 0.33mmol), XantPhos(20mg, 0.03mmol) and $Pd_2(dba)_3$(19mg, 0.02mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and purified by high performance preparative thin layer chromatography (MeOH:DCM=1:20) to provide 4-((4-(cyclopropyl methoxy)-2-(N-methyl methanesulfonamido) phenyl)amino)-6-((5-fluoropyridin-2-yl)amino)-N-methoxy nicotinamide **(61,** 16mg, 0.03mmol, 27% yield), as a white solid. MS Calcd: 530; MS Found: 531 ([M+H]$^+$). $^1$H NMR (400 MHz, DMSO-$d_6$) : δ 11.62 (s, 1H), 9.72 (s, 1H), 9.71 (s, 1H), 8.26 (s, 1H), 8.12 (d, *J* = 2.8 Hz, 1H), 7.66-7.60 (m, 2H), 7.43 (d, *J* = 8.8 Hz, 1H), 7.29 (s, 1H), 7.13 (d, *J* = 2.4 Hz, 1H), 7.08 (dd, *J* = 8.8, 2.8 Hz, 1H), 3.88 (d, *J* = 7.2 Hz, 2H), 3.70 (s, 3H), 3.12 (s, 3H), 3.08 (s, 3H), 1.24-1.23 (m, 1H), 0.61-0.57 (m, 2H), 0.37-0.33 (m, 2H).

Example **62**

4-((4-(cyclopropyl methoxy)-2-(N-methyl methanesulfonamido)phenyl)amino)-6-((6-fluoropyridin-2-yl)amino)-N-methoxy nicotinamide

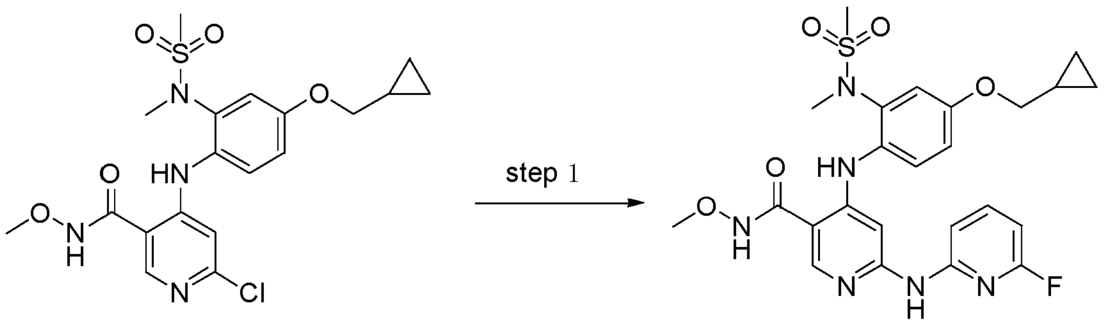


61-e 62

Step 1: 6-chloro-4-((4-(cyclopropyl methoxy)-2-(N-methyl methanesulfonamido)phenyl)amino)-N-methoxy nicotinamide **(61-e,** 50mg, 0.11mmol), 6-fluoropyridin-2-ylamine (19mg, 0.22mmol), cesium carbonate (107mg, 0.33mmol), XantPhos(20mg, 0.03mmol) and $Pd_2(dba)_3$(19mg, 0.02mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and purified by high performance preparative thin layer chromatography (MeOH:DCM=1:20) to provide the title compound: 4-((4-(cyclopropyl methoxy)-2-(N-methyl methanesulfonamido)phenyl)amino)-6-((5-fluoropyridin-2-yl)amino)-N-methoxy nicotinamide **(62,** 13mg, 0.024mmol, 22% yield). MS Calcd: 530; MS Found: 531 ($[M+H]^+$). $^1$H NMR (400 MHz, DMSO-$d_6$) : δ 11.66 (s, 1H), 9.90 (s, 1H), 9.71 (s, 1H), 8.27 (s, 1H), 7.79-7.76 (m, 1H), 7.44 (d, *J* = 8.8 Hz, 2H), 7.29 (s, 1H), 7.15 (d, *J* = 2.8 Hz, 1H), 7.00 (dd, *J* = 8.8, 2.8 Hz, 1H), 6.54 (dd, *J* = 8.0, 2.8 Hz, 1H), 3.865 (d, J=7.2, 2H), 3.72 (s, 3H), 3.14 (s, 3H), 3.07 (s, 3H), 1.26-1.23 (m, 1H), 0.61-0.57 (m, 2H), 0.37-0.33 (m, 2H).

Example **63**

6-(cyclopropylcarboxamido)-4-((4-(cyclopropyl methoxy)-2-(N-methyl methanesulfonamido)phenyl)amino)-N-methoxy nicotinamide

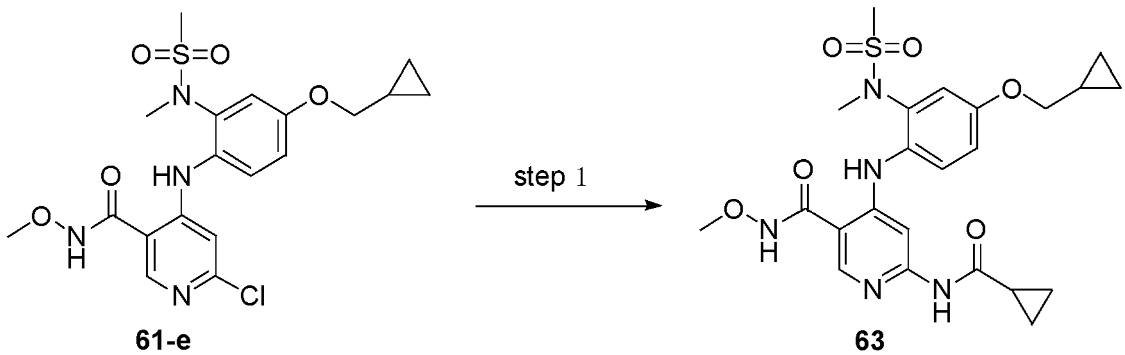


61-e 63

Step 1: 6-chloro-4-((4-(cyclopropyl methoxy)-2-(N-methyl methanesulfonamido)phenyl)amino)-N-methoxy nicotinamide **(61-e,** 50mg, 0.11mmol), cyclopropylcarboxamide (19mg, 0.22mmol), cesium carbonate (107mg, 0.33mmol), XantPhos(20mg, 0.03mmol) and $Pd_2(dba)_3$(19mg, 0.02mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and purified by high performance preparative thin layer chromatography (MeOH:DCM=1:20) to provide 6-(cyclopropylcarboxamido)-4-((4-(cyclopropyl methoxy)-2-(N-methyl methanesulfonamido)phenyl)amino)-N-methoxy nicotinamide **(63,** 15mg, 0.029mmol, 26% yield), as a white solid. MS Calcd: 503; MS Found: 504 ($[M+H]^+$). 1H NMR (400 MHz, DMSO-$d_6$) : δ 11.71 (s, 1H), 10.69 (s, 1H), 9.68 (s, 1H), 8.28 (s, 1H), 7.59 (s, 1H), 7.319 (d, *J* = 8.8 Hz, 1H), 7.15 (d, *J* = 2.8, 1H), 6.98 (dd, *J* = 8.8, 2.8 Hz, 1H), 3.86 (d, *J* = 7.2 Hz, 2H), 3.70 (s, 3H), 3.09 (s, 3H), 3.05 (s, 3H), 1.97-1.96 (m, 1H), 1.23-1.17 (m, 1H), 0.75-0.72 (m, 4H), 0.35-0.34 (m, 2H), 0.28-0.27 (m, 2H).

Example **64**

6-((4-fluorophenyl)amino)-N-methoxy-4-((6-methyl-2-(N-methyl methanesulfonamido)pyridin-3-yl)amino)nicotinamide

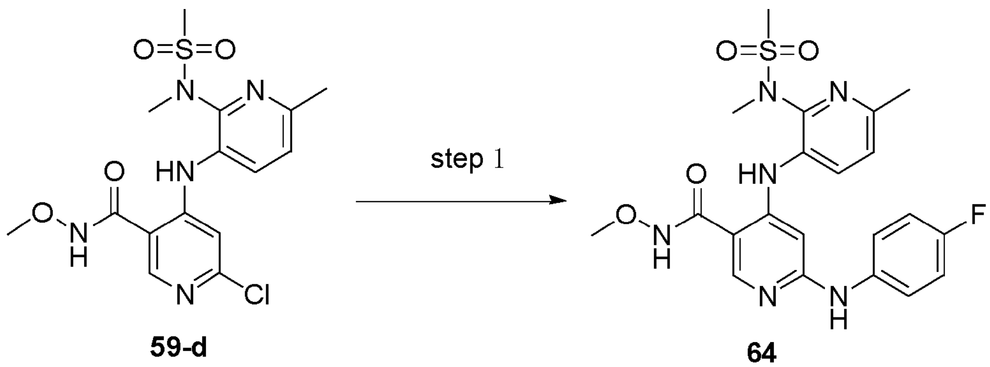


**59-d** **64**

Step 1: 6-chloro-N-methoxy-4-((6-methyl-2-(N-methyl methanesulfonamido)pyridin-3-yl)amino)nicotinamide **(59-d,** 50mg, 0.13mmol), 4-fluoroaniline(30mg, 0.26mmol), cesium carbonate (130mg, 0.4mmol), Xantphos(20mg, 0.03mmol) and $Pd_2(dba)_3$(19mg, 0.02mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and purified by high performance preparative thin layer chromatography (MeOH:DCM=1:20) to provide the title compound: 6-((4-fluorophenyl)amino)-N-methoxy-4-((6-methyl-2-(N-methyl methanesulfonamido)pyridin-3-yl)amino)nicotinamide **(64,** 16mg, 0.03mmol, 23% yield). MS Calcd: 474; MS Found: 475 ($[M+H]^+$).$^1$H NMR (400 MHz, DMSO-$d_6$) : δ 11.65 (s, 1H), 9.82 (s, 1H), 9.08 (s, 1H), 8.26 (s, 1H), 7.90-7.88 (d, *J*=8.0, 1H), 7.59 (d, $J_1$=4.0, $J_2$=8.0, 2H), 7.36-7.34 (d, *J*=8.0, 1H), 7.11-7.07 (t, J=8.0, 2H), 6.29 (m, 1H), 3.71 (s, 3H), 3.20 (s, 3H), 3.12 (s, 3H), 2.48 (s, 3H)

Example **65**

6-((6-fluoropyridin-2-yl)amino)-N-methoxy-N-methyl-4-((2-(N-methyl methanesulfonamido)phenyl)amino)nicotinamide

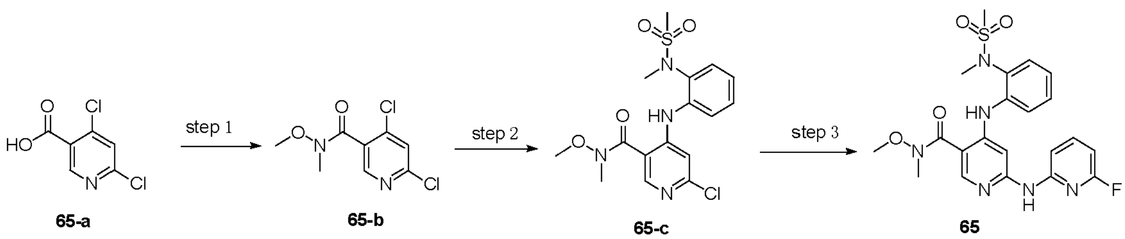


**65-a** **65-b** **65-c** **65**

Step 1: To dichloromethane (20ml) was added 4,6-dichloronicotinic acid**(65-a,** 1g, 5.2mmol), followed by catalytic amount of DMF. The mixture was cooled to 0°C, and slowly added with oxalyl chloride (0.7ml, 7.8mmol) dropwise. Upon the addition was completed, the reaction mixture was heated to room temperature slowly and stirred for 2 hours, then concentrated under reduced pressure. The residue was dissolved in 5ml of ethyl acetate, and added slowly to aqueous solution of dimethyl hydroxylamine hydrochloride (1.5g, 16mmol) and potassium carbonate (3.7g, 27mmol) in mixed solvent of water and ethyl acetate. The mixture was stirred at room temperature overnight, added with ethyl acetate (50ml), and subjected to phase separation. The organic layer was dried and concentrated, and the residue was purified by column chromatography (ethyl acetate:petroleum ether=1:2) to provide 4,6-dichloro-N-methoxy-N-methyl nicotinamide **(65-b,** 800mg, 3.4mmol, 65% yield). MS Calcd: 234; MS Found: 235 ($[M+H]^+$).

Step 2: To 5ml of anhydrous tetrahydrofuran were added 4,6-dichloro-N-methoxy-N-methyl nicotinamide **(65-b,** 200mg, 0.9mmol) and N-(2-amino phenyl)-N-methyl methanesulfonamide (220mg, 1.1mmol). The mixture was added at room temperature with a solution of LiHMDS in tetrahydrofuran (1.8ml, 1.8mmol), and stirred at room temperature for 2 hours. The mixture was adjusted with aqueous hydrochloride (1N) to pH 5, and extracted with ethyl acetate (10ml x 3). The combined organic layers were dried, concentrated and then purified by column chromatography (petroleum ether:ethyl acetate =1:2) to provide 6-chloro-N-methoxy-N-methyl-4-((2-(N-methyl methanesulfonamido)phenyl)amino)nicotinamide **(65-c,** 160mg, 0.4mmol, 44% yield). MS Calcd: 398; MS Found: 399 ($[M+H]^+$).

Step 3: 6-chloro-N-methoxy-N-methyl-4-((2-(N-methyl methanesulfonamido)phenyl)amino)nicotinamide **(65-c,** 100mg, 0.25mmol), 6-fluoropyridin-2-ylamine (56mg, 0.5mmol), cesium carbonate (240mg, 0.75mmol), Xantphos(60mg, 0.08mmol) and $Pd_2(dba)_3$(50mg, 0.05mmol) were added to anhydrous dioxane (3ml). After the atmosphere of the mixture was evacuated to vacuum and refilled with nitrogen, the reaction mixture was heated to 120°C with stirring for 2 hours, and filtered by suction. The filtrate was concentrated and purified by high performance preparative thin layer chromatography (MeOH:DCM=1:20) to provide the title compound: 6-((6-fluoropyridin-2-yl)amino)-N-methoxy-N-methyl-4-((2-(N-methyl methanesulfonamido)phenyl)amino)nicotinamide **(65,** 30mg, 0.06mmol, 24% yield). MS Calcd: 474; MS Found: 475 ($[M+H]^+$).$^1$H NMR (400 MHz, DMSO-$d_6$) : δ 10.01 (brs, 1H), 8.86 (s, 1H), 8.30 (s, 1H), 7.80-7.78 (d, *J*=8.0, 1H), 7.78-7.51 (m, 4H), 7.41-7.37 (m, 1H), 7.20-7.18 (m, 1H), 6.56-6.54 (d, J=8.0, 1H), 3.61 (s, 3H), 3.29 (s, 3H), 3.14 (s, 3H), 3.10 (s, 3H)

Example **66**

6-((4,6-dimethyl pyrimidin-2-yl)amino)-N-ethoxy-4-((4-methyl-2-(N-methyl cyclopropylsulfonamido)phenyl)amino)nicotinamide

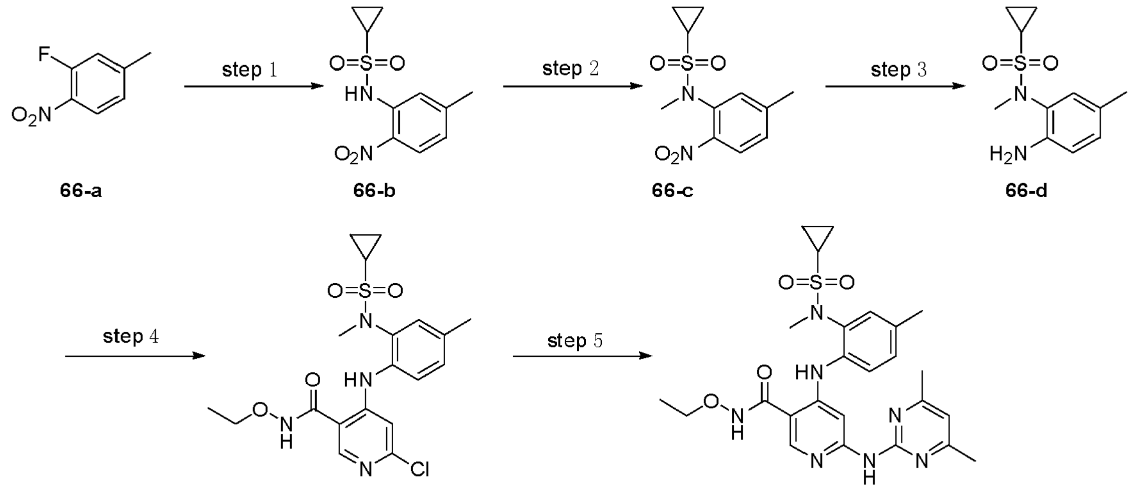


66-a 66-b 66-c 66-d

66-e 66

Step 1: To a 100ml reaction flask was added 2-fluoro-4-methyl-1-nitrobenzene(**66-a,** 465g, 3mmol) dissolved in N,N-dimethyl formamide (30ml), followed by potassium carbonate (1.24g, 9mmol) and cyclopropanesulfonamide (400mg, 3.3mmol). The mixture was heated to 90°C with stirring for 3h. Upon indication of completed reaction by TLC, the reaction mixture was washed respectively with water (30 ml x 1) and brine (30 ml x 1), dried over anhydrous sodium sulfate, and concentrated to provide a crude material, which was purified by silica gel column chromatography (petroleum ether/ethyl acetate=3:1) to provide the product N-(5-methyl-2-nitrophenyl) cyclopropylsulfonamide **(66-b,** 695mg, 90% yield). MS Calcd:256.05; MS Found: 257.10 ($[M+H]^+$)

Step 2: To a 100ml reaction flask was added N-(5-methyl-2-nitrophenyl) cyclopropylsulfonamide **(66-b,** 690mg, 2.72mmol) dissolved in N,N-dimethyl formamide (30ml), followed by sodium hydride (163.2mg, 60%, 4.08mmol) and iodomethane (463mg, 3.26mmol). The reaction mixture was stirred at room temperature for 3h. Upon indication of completed reaction by TLC, the reaction mixture was added with water and ethyl acetate, extracted with ethyl acetate (30 ml x 2). The organic phase was washed with brine (30 ml x 1), dried over anhydrous sodium sulfate, and concentrated to obtain N-(5-methyl-2-nitrophenyl)-N-methyl cyclopropylsulfonamide **(66-c,** 761mg, 2.72mmol, 100% yield). MS Calcd: 270.09; MS Found: 271.22 ($[M+H]^+$).

Step 3: N-(5-methyl-2-nitrophenyl)-N-methyl cyclopropylsulfonamide **(66-c,** 761mg, 2.72mmol), ammonium chloride (1.46g, 27.2 mmol) and iron powder (0.76 g, 13.6 mmol) were sequentially added to 10 mL of mixed solvent of water and ethanol (1:4), stirred under reflux for 3 hours. Upon indication of completed reaction by TLC, the reaction mixture was filtered by suction. The filtrate was concentrated, and the residue was separated and purified by silica gel column chromatography to provide N-(2-amino-5-methyl phenyl)-N-methyl cyclopropylsulfonamide **(66-d,** 0.47 g, 1.96mmol, 72% yield). MS Calcd: 240.09; MS Found: 241.22 ($[M+H]^+$).

Step 4: To 10 ml of anhydrous N,N-dimethylacetamide were added N-(2-amino-5-methyl phenyl)-N-methyl cyclopropylsulfonamide **(66-d,** 125mg, 0.5 mmol) and 4,6-dichloro-N-ethoxy nicotinamide (**int-2,** 117 mg, 0.5 mmol). The mixture was added at room temperature with a solution of LiHMDS in tetrahydrofuran (1.5 ml, 1.5 mmol), and stirred at

room temperature for 3 hours. Upon indication of completed reaction by TLC, the mixture was adjusted with aqueous hydrochloride (1N) to pH 5, and extracted with ethyl acetate (20 ml x 3). The combined organic layers were dried, concentrated and then purified by column chromatography (PE:EA =1:1) to provide 6-chloro-N-ethoxy-4-((4-methyl-2-(N-methyl cyclopropylsulfonamido)phenyl)amino)nicotinamide **(66-e,** 187mg, 0.43 mmol, 85.2% yield). MS Calcd: 438.10; MS Found: 439.29 ($[M+H]^+$).

Step 5: 6-chloro-N-ethoxy-4-((4-methyl-2-(N-methyl cyclopropylsulfonamido)phenyl)amino)nicotinamide **(66-e,** 132mg, 0.3 mmol), 4,6-dimethyl pyrimidin-2-ylamine (40.6mg, 0.33mmol), cesium carbonate (292.5 mg, 0.9 mmol), XantPhos(34.68mg, 0.06mmol) and $Pd_2(dba)_3$(27.5mg, 0.03 mmol) were added to anhydrous dioxane (5 ml). After the atmosphere of the mixture was evacuated to vacuum and refilled with nitrogen, the reaction mixture was heated to 120°C with stirring for 6 hours, and filtered by suction. The filtrate was concentrated and purified by preparative TLC (MeOH:DCM=1:20) to provide the title compound: 6-((4,6-dimethyl pyrimidin-2-yl)amino)-N-ethoxy-4-((4-methyl-2-(N-methyl cyclopropylsulfonamido)phenyl)amino)nicotinamide **(66,** 30mg, 0.057mmol, 19.2% yield). MS Calcd: 525.21; MS Found: 526.20 ($[M+H]^+$). $^1$H NMR (400 MHz, DMSO-$d_6$)δ11.60 (s, 1H), 9.97 (s, 1H), 9.60 (s, 1H), 8.32 (s, 1H), 8.21 (s, 1H), 7.51 (d, *J* = 8.0 Hz, 1H), 7.44 (d, *J* = 2.0 Hz, 1H), 7.26 (dd, *J* = 8.0, 2.0 Hz, 1H), 6.72 (s, 1H), 3.93 (q, *J*= 7.2 Hz, 2H), 3.14 (s, 3H), 2.81-2.77 (m, 1H), 2.34 (s, 3H), 2.25 (s, 6H), 1.22 (t, *J*= 7.2 Hz, 3H), 1.02-0.98 (m, 2H), 0.88 - 0.82 (m, 2H).

Example **67**

4-((4-cyclopropyl-2-(N-methyl methanesulfonamido)phenyl)amino)-6-((6-fluoropyridin-2-yl)amino)-N-methoxy-2-methyl nicotinamide

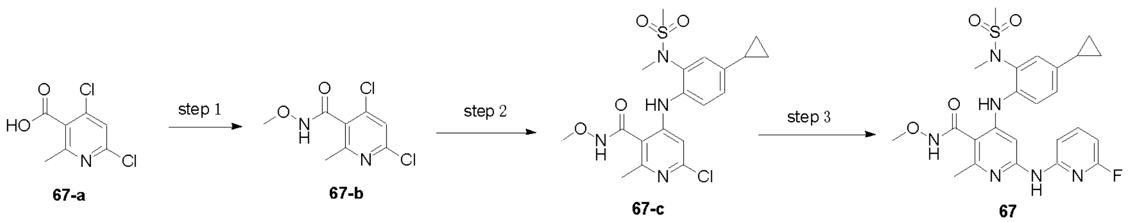

67-a 67-b 67-c 67

Step 1: To dichloromethane (20ml) was added 4,6-dichloro-2-methyl nicotinic acid **(67-**a, 1g, 4.8mmol), followed by catalytic amount of DMF. The mixture was cooled to 0°C, and slowly added with oxalyl chloride (0.7ml, 7.8mmol) dropwise. Upon the addition was completed, the reaction mixture was heated to room temperature slowly and stirred for 2 hours, and concentrated under reduced pressure. The residue was dissolved in 5ml of ethyl acetate, and added slowly to solution of methoxy amine hydrochloride (1.2g, 16mmol) and potassium carbonate (3.7g, 27mmol) in a mixed solvent of water and ethyl acetate. The mixture was stirred at room temperature overnight, added with ethyl acetate (50ml), and subjected to phase separation. The organic layer was dried and concentrated, and the residue was purified by column chromatography (ethyl acetate:petroleum ether=1:2) to provide the title compound: 4,6-dichloro-N-methoxy-2-methyl nicotinamide **(67-b,** 750mg, 3.2mmol, 66% yield).

MS Calcd: 234; MS Found: 235 ($[M+H]^+$).

Step 2: To 5ml of anhydrous tetrahydrofuran were added 4,6-dichloro-N-methoxy-2-methyl nicotinamide **(67-b,** 200mg, 0.9mmol) and N-(2-amino-5-cyclopropyl phenyl)-N-methyl methanesulfonamide (264mg, 1.1mmol). The mixture was added at room temperature with a solution of LiHMDS in tetrahydrofuran (1.8ml, 1.8mmol), and stirred at room temperature for 2 hours. The mixture was adjusted with aqueous hydrochloride (1N) to pH 5, and extracted with ethyl acetate (10ml x 3). The combined organic layers were dried, concentrated and then purified by column chromatography (petroleum ether:ethyl acetate =1:2) to provide 6-chloro-4-((4-cyclopropyl-2-(N-methyl methanesulfonamido)phenyl) amino)-N-methoxy-2-methyl nicotinamide **(67-c,** 187mg, 0.43mmol, 48% yield), MS Calcd: 438; MS Found: 439 ($[M+H]^+$).

Step 3: 6-chloro-4-((4-cyclopropyl-2-(N-methyl methanesulfonamido)phenyl)amino)-N-methoxy-2-methyl nicotinamide **(67-c,** 60mg, 0.14mmol), 6-fluoropyridin-2-ylamine (31mg, 0.28mmol), cesium carbonate (137mg, 0.42mmol), XantPhos(30mg, 0.04mmol) and $Pd_2(dba)_3$(30mg, 0.03mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and purified by high performance preparative thin layer chromatography (MeOH:DCM=1:20) to provide the title compound: 4-((4-cyclopropyl-2-(N-methyl methanesulfonamido)phenyl)amino)-6-((6-fluoropyridin-2-yl)amino)-N-methoxy-2-methyl nicotinamide (67, 25mg, 0.05mmol, 36% yield). MS Calcd: 514; MS Found: 515 ($[M+H]^+$). $^1$H NMR (400 MHz, DMSO-$d_6$) : δ 11.60 (s, 1H), 9.79 (s, 1H), 7.76 (d, J=8.0, 1H), 7.74 (s, 1H), 7.48 (d, J=8.0, 1H), 7.44-7.40 (m, 2H), 7.28 (d, *J*=4.0, 1H), 7.08 (d, *J*=8.0, 1H), 6.50 (d, J=8.0, 1H), 3.73 (s, 3H), 3.13 (s, 3H), 3.10 (s, 3H), 2.35 (s, 3H), 1.96-1.93 (m, 1H), 0.98-0.96 (m, 2H), 0.70-0.68 (m, 2H)

Example **68**

4-((4-cyclopropyl-2-(N-methyl methanesulfonamido)phenyl)amino)-6-((6-fluoropyridin-2-yl)amino)-N-(methoxy-*d*3)nicotinamide

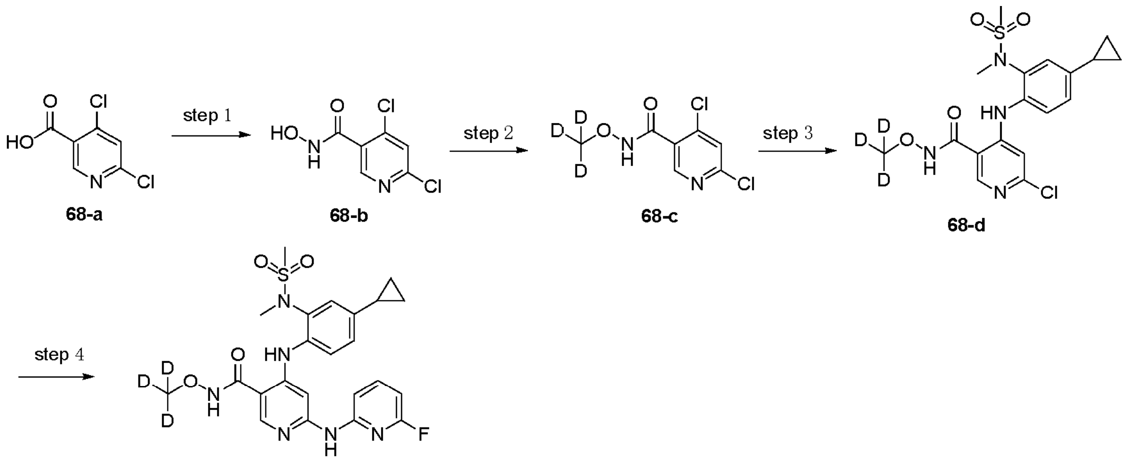


68-a 68-b 68-c 68-d

68

Step 1: To dichloromethane (20ml) was added 4,6-dichloronicotinic acid (68-a, 1g, 5.2mmol), followed by catalytic amount of DMF. The mixture was cooled to 0°C, and slowly added with oxalyl chloride (0.7ml, 7.8mmol) dropwise. Upon the addition was completed, the reaction mixture was heated to room temperature slowly and stirred for 2 hours, and concentrated under reduced pressure. The residue was dissolved in 5ml of ethyl acetate, and added slowly to aqueous solution of hydroxylamine hydrochloride (1g, 16mmol) and potassium carbonate (3.7g, 27mmol) in mixed solvent of water and ethyl acetate. The mixture was stirred at room temperature overnight, added with ethyl acetate (50ml), and subjected to phase separation. After adjusting with aqueous hydrochloride to pH 5, the organic layer was dried and concentrated. The residue was purified by column chromatography (ethyl acetate:petroleum ether=1:2) to provide 4,6-dichloro-N-hydroxyl nicotinamide **(68-b,** 455mg, 2.2mmol, 42% yield). MS Calcd: 206; MS Found: 205 ($[M-H]^-$).

Step 2: To a mixed solvent of water and ethanol (1ml/5ml) were added 4,6-dichloro-N-hydroxyl nicotinamide **(68-b,** 455mg, 2.2mmol) and sodium hydroxide (264mg, 6.6mmol). The mixture was added at room temperature with deuterated iodomethane (319mg, 2.2mmol), and stirred at room temperature for 4 hours. The mixture was adjusted with aqueous hydrochloride (1N) to pH 5, and extracted with ethyl acetate (10ml x 3). The combined organic layers were dried, concentrated and then purified by column chromatography (petroleum ether:ethyl acetate =1:2) to provide 4,6-dichloro-N-(methoxy-d3)nicotinamide **(68-c,** 160mg, 0.72mmol, 33% yield). MS Calcd: 223; MS Found: 224 ($[M+H]^+$).

Step 2: To 5ml of anhydrous tetrahydrofuran were added 4,6-dichloro-N-(methoxy-d3)nicotinamide **(68-c,** 160mg, 0.72mmol) and N-(2-amino-5-cyclopropyl phenyl)-N-methyl methanesulfonamide (180mg, 0.72mmol). The mixture was added at room temperature with a solution of LiHMDS in tetrahydrofuran (1.5ml, 1.5mmol), and stirred at room temperature for 2 hours. The mixture was adjusted with aqueous hydrochloride (1N) to pH 5, and extracted with ethyl acetate (10ml x 3). The combined organic layers were dried, concentrated and then purified by column chromatography (petroleum ether:ethyl acetate =1:2) to provide 6-chloro-4-((4-cyclopropyl-2-(N-methyl methanesulfonamido)phenyl) amino)-N-(methoxy-d3)nicotinamide **(68-d,** 110mg, 0.26mmol, 36% yield)MS Calcd: 427; MS Found: 428 ($[M+H]^+$).

Step 3: 6-chloro-4-((4-cyclopropyl-2-(N-methyl methanesulfonamido)phenyl)amino)-N-(methoxy-d3)nicotinamide **(68-d,** 110mg, 0.26mmol), 6-fluoropyridin-2-ylamine (58mg, 0.52mmol), cesium carbonate (254mg, 0.78mmol), XantPhos(60mg, 0.08mmol) and $Pd_2(dba)_3$(60mg, 0.06mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and purified by high performance preparative thin layer chromatography (MeOH:DCM=1:20) to provide the title compound: 4-((4-cyclopropyl-2-(N-methyl methanesulfonamido)phenyl)amino)-6-((6-fluoropyridin-2-yl)amino)-N-(methoxy-d3)nicotinamide (68, 30mg, 0.06mmol, 23% yield). MS Calcd: 503; MS Found: 504 ($[M+H]^+$). $^1$H NMR (400 MHz, DMSO-$d_6$) : δ 11.69 (s, 1H), 9.97(s, 1H), 9.93 (s, 1H), 8.30 (s, 1H), 7.82-7.76 (m, 1H), 7.54 (s, 1H), 7.49 (d, *J*=8.0, 1H), 7.45 (d, J=4.0, 1H), 6.55 (d, J=4.0, 1H)7.28 (s, 1H), 7.09-7.07 (d, J=8, 1H)3.13 (s, 3H), 3.10 (s, 3H), 2.00-1.96 (m, 1H), 1.01-0.97 (m, 2H), 0.70-0.68 (m, 2H)

Example **69**

6-(cyclopropylcarboxamido)-N-methoxy-4-((2-methoxy-3-(1-methyl-1H-1,2,4-triazol-3-yl)phenyl)amino)-nicotinamide

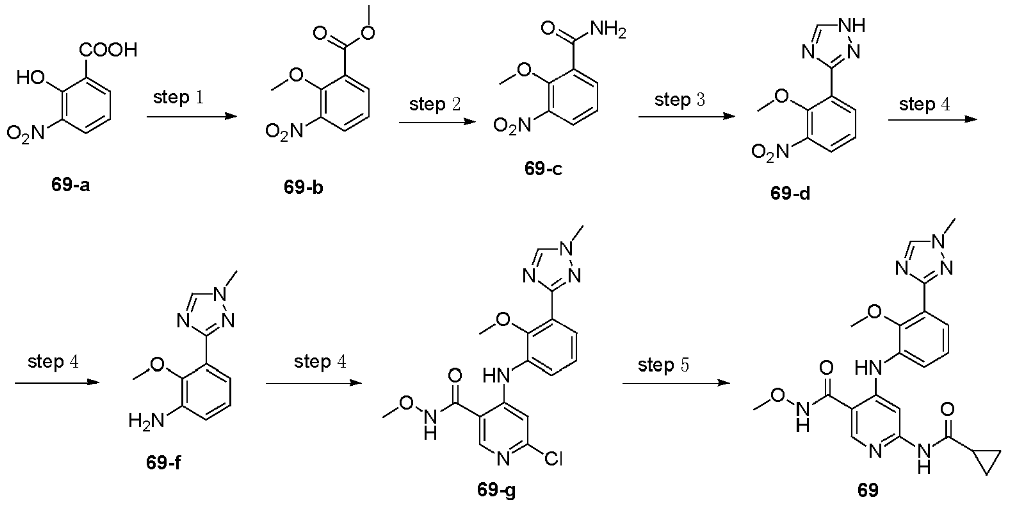


69-a 69-b 69-c 69-d 69-e 69-f 69-g 69

Step 1: To 50 mL of N,N-dimethyl formamide were sequentially added 3-nitrosalicylic acid **(69-a,** 5.0 g, 27 mmol) and potassium carbonate (10.0 g, 36 mmol), followed by iodomethane (5.0 mL, 80 mmol). The mixture was heated to 60°C and stirred overnight. Upon indication of completed reaction by TLC, the reaction mixture was added with 60 mL of water, and extracted with ethyl acetate (60 mL x 3). The combined organic phases were washed with saturated brine (50 mL x 3), dried over anhydrous sodium sulfate, and concentrated under reduced pressure. The residue was separated and purified by silica gel column chromatography (ethyl acetate:petroleum ether=1: 4) to provide methyl 2-methoxy-3-nitrobenzoate **(69-b,** 4.2 g, 19.9 mmol, 74% yield).

Step 2: methyl 2-methoxy-3-nitrobenzoate **(69-b,** 3.7 g, 17.3 mmol) was dissolved in methanol solution of ammonia (7 N, 83 mL), followed by adding high concentration ammonia water (35 mL), and stirred at room temperature overnight. Upon indication of completed reaction by TLC, the reaction mixture was concentrated. The residue was separated and purified by silica gel column chromatography (ethyl acetate:petroleum ether=1: 5) to provide 2-methoxy-3-nitrobenzamide **(69-c,** 3.0 g, 15.3 mmol, 88% yield). MS Calcd: 196.16; MS Found: 196.74 ($[M+H]^+$).

Step 3: 2-methoxy-3-nitrobenzamide **(69-c,** 3.0 g, 15.3 mmol) was suspended in DMF-DMA (20 mL). The mixture was heated to 95°C with stirring for 30 minutes until the reaction mixture was clear. The mixture was concentrated under reduced pressure to remove volatiles, followed by adding 20 mL of ethanol for dissolution. To the flask in ice bath were sequentially added 63 mL of ethanol, 15 mL of acetic acid and hydrazine hydrate (7.4 mL, 152 mmol). The mixture was stirred at room temperature overnight. Upon indication of completed reaction by TLC, organic solvent was removed and water (100 mL) was added to cause solid to precipitate, which was filtered by suction to provide 3-(2-methoxy-3-nitrobenzene)-1-*H*-1,2,4-triazole **(69-d,** 2.31 g, 10.5 mmol, 68% yield).

MS Calcd: 220.19; MS Found: 243.09 ($[M+Na^+]^+$).

Step 4: 3-(2-methoxy-3-nitrobenzene)-1-*H*-1,2,4-triazole **(69-d,** 2.2 g, 10.1 mmol) and potassium carbonate (4.2 g, 30 mmol) were sequentially added to 20 mL of N,N-dimethyl formamide, followed by adding iodomethane (0.86 mL, 13.6 mmol). The mixture was stirred at room temperature overnight. Upon indication of completed reaction by TLC, the reaction mixture was added with 20 ml of water, and extracted with ethyl acetate (20 mL x 3). Combined organic phases were washed with saturated brine (20 mL x 3), dried over anhydrous sodium sulfate, and concentrated under reduced pressure. The residue was separated and purified by silica gel column chromatography (ethyl acetate:petroleum ether=2:1) to provide 3-(2-methoxy-3-nitrobenzene)-1-methyl-1-*H*-1,2,4-triazole **(69-e,** 1.5 g, 6.4 mmol 63% yield).

Step 5: To methanol(20ml) were added 3-(2-methoxy-3-nitrobenzene)-1-methyl-1-*H*-1,2,4-triazole **(69-e,** 0.53 g, 2.2 mmol) and palladium on carbon (100mg). After atmosphere replacement by hydrogen three times, the mixture was stirred under hydrogen atmosphere at room temperature overnight. Upon indication of completed reaction by TLC, the mixture was filtered by suction. The filtrate was concentrated under reduced pressure. The residue was separated and purified by silica gel column chromatography (ethyl acetate) to provide 2-methoxy-3-(1-methyl-1*H*-1,2,4-triazol-3-yl) aniline **(69-f,** 330mg, 1.6mmol, 74% yield).

MS Calcd: 204.23; MS Found: 205.23 ($[M+H]^+$)

Step 6: To 5ml of anhydrous tetrahydrofuran were added 2-methoxy-3-(1-methyl-1*H*-1,2,4-triazol-3-yl)aniline **(69-f,** 150 mg, 0.7 mmol) and 4,6-dichloro-N-methoxy nicotinamide **(int-1,** 140 mg, 0.63 mmol). The mixture was added at room temperature with a solution of LiHMDS in tetrahydrofuran (2.5 ml, 2.5 mmol), and stirred at room temperature for 2

hours. The mixture was adjusted with aqueous hydrochloride (1N) to pH 5, and extracted with ethyl acetate (10ml x 3). The combined organic layers were dried, concentrated and then purified by column chromatography (DCM: MeOH =1: 10) to provide 6-chloro-N-methoxy-4-((2-methoxy-3-(1-methyl-1*H*-1,2,4-triazol-3-yl)phenyl)amino)nicotinamide **(69-g,** 100 mg, 0.25 mmol, 37% yield).

MS Calcd: 388.11; MS Found: 389.23 ([M+H]+).

Step 7: 6-chloro-N-methoxy-4-((2-methoxy-3-(1-methyl-1H-1,2,4-triazol-3-yl)phenyl)amino)nicotinamide **(69-g,** 100 mg, 0.25 mmol), cyclopropylcarboxamide (22mg, 0.26mmol), cesium carbonate (127mg, 0.39mmol), XantPhos(30mg, 0.04mmol) and $Pd_2(dba)_3$(24mg, 0.026mmol) were added to anhydrous dioxane (2ml). After the atmosphere of the mixture was evacuated to vacuum and refilled with nitrogen, the reaction mixture was heated to 120°C with stirring for 3 hours, and filtered by suction. The filtrate was concentrated and purified by high performance preparative thin layer chromatography (MeOH:DCM=1:20) to provide the title compound: 6-(cyclopropylcarboxamido)-N-methoxy-4-((2-methoxy-3-(1-methyl-1H-1,2,4-triazol-3-yl)phenyl)amino)-nicotinamide **(69, 30** mg, 0.068mmol, 27% yield).

MS Calcd: 437.18; MS Found: 438.1 ([M+H]+).

$^1$H NMR (400 MHz, DMSO-$d_6$)$\delta$11.91 (s, 1H), 10.75 (s, 1H), 10.45 (s, 1H), 8.55 (s, 1H), 8.43 (s, 1H), 8.07 (s, 1H), 7.55 (dd, *J* = 8.0, 1.2 Hz, 1H), 7.49 (dd, *J*= 8.0, 1.2 Hz, 1H), 7.22 (t, *J* = 8.0 Hz, 1H), 3.95 (s, 3H), 3.73 (s, 3H), 3.71 (s, 3H), 2.01-1.94 (m, 1H), 0.79-0.75 (m, 4H).

Example **70**

6-(cyclopropylcarboxamido)-4-((4-fluoro-2-(N-methyl methanesulfonamido)phenyl)amino)-N-methoxy nicotinamide

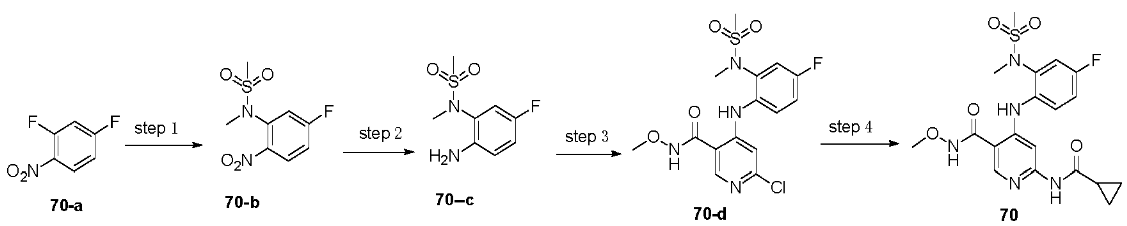


70-a 70-b 70–c 70-d 70

Step 1: To DMF were added 2,4-difluoro-1-nitrobenzene **(70-a,** 1g, 6.3mmol), N-methyl methanesulfonamide (690mg, 6.3mmol) and potassium carbonate (1.7g, 12.6mmol). The mixture was heated to 80°C with stirring for 2 hours. When TLC indicated that all starting material was depleted, the reaction mixture was cooled to room temperature, poured into 100ml of water to cause solid precipitation, which was filtered by suction, and purified by column chromatography (ethyl acetate:petroleum ether=1; 2) to provide N-(5-fluoro-2-nitrophenyl)-N-methyl methanesulfonamide **(70-b,** 823mg, 3.3mmol, 52% yield). MS Calcd: 248; MS Found: 249 ([M-H]-).

Step 2: To methanol(20ml) were added N-(5-fluoro-2-nitrophenyl)-N-methyl methanesulfonamide **(70-b,** 823mg, 3.3mmol), palladium on carbon (100mg). After atmosphere replacement by hydrogen three times, the mixture was stirred under hydrogen atmosphere at room temperature overnight, and filtered by suction. The filtrate was concentrated under reduced pressure to provide N-(2-amino-5-fluorophenyl)-N-methyl methanesulfonamide **(70-c,** 642mg, 2.9mmol, 87% yield), which was used directly in the next reaction without further purification. MS Calcd: 218; MS Found: 219 ([M+H]+).

Step 3: To 2.5ml of anhydrous tetrahydrofuran were added N-(2-amino-5-fluorophenyl)-N-methyl methanesulfonamide **(70-c,** 242mg, 1.1mmol) and 4,6-dichloro-N-methoxy nicotinamide **(int-1,** 230mg, 1.1mmol). The mixture was added at room temperature with a solution of LiHMDS in tetrahydrofuran (2.5ml, 2.5mmol), and stirred at room temperature for 2 hours. The mixture was adjusted with aqueous hydrochloride (1N) to pH 5, and extracted with ethyl acetate (10ml). The combined organic layers were dried, concentrated and then purified by column chromatography (PE:EA =1:1) to provide 6-chloro-4-((4-fluoro-2-(N-methyl methanesulfonamido)phenyl)amino)-N-methoxy nicotinamide **(70-d,** 147mg, 0.37mmol, 34% yield). MS Calcd: 402; MS Found: 403 ([M+H]+).

Step 4: 6-chloro-4-((4-fluoro-2-(N-methyl methanesulfonamido)phenyl)amino)-N-methoxy nicotinamide **(70-d,** 147mg, 0.37mmol), cyclopropylcarboxamide (63mg, 0.74mmol), cesium carbonate (400mg, 1.2mmol), XantPhos(60mg, 0.09mmol) and $Pd_2(dba)_3$(57mg, 0.06mmol) were added to anhydrous dioxane (6ml). After the atmosphere of the mixture was evacuated to vacuum and refilled with nitrogen, the reaction mixture was heated to 120°C with stirring for 2 hours, and filtered by suction. The filtrate was concentrated and purified by high performance preparative thin layer chromatography (MeOH:DCM=1:20) to provide the title compound: 6-(cyclopropylcarboxamido)-4-((4-fluoro-2-(N-methyl methanesulfonamido)phenyl)amino)-N-methoxy nicotinamide **(70,** 32mg, 0.07mmol, 20% yield). MS Calcd: 451; MS Found: 452 ([M+H]+). $^1$H NMR (400 MHz, DMSO-$d_6$) : $\delta$ 11.77 (s, 1H), 10.76 (s, 1H), 9.87 (s, 1H), 8.32 (s, 1H), 7.73 (s, 1H), 7.56 (dd, *J* = 9.6, 2.8 Hz, 1H), 7.50-7.46 (m, 1H), 7.33-7.28 (m, 1H), 3.71 (s, 3H), 3.15 (s, 3H), 3.12 (s, 3H), 1.96-1.93 (m, 1H), 0.77-0.74

(m, 4H).

Example **71**

6-(cyclopropylcarboxamido)-N-ethyl-4-((2-methoxy-3-(1-methyl-1H-pyrazol-4-yl)phenyl)amino)nicotinamide

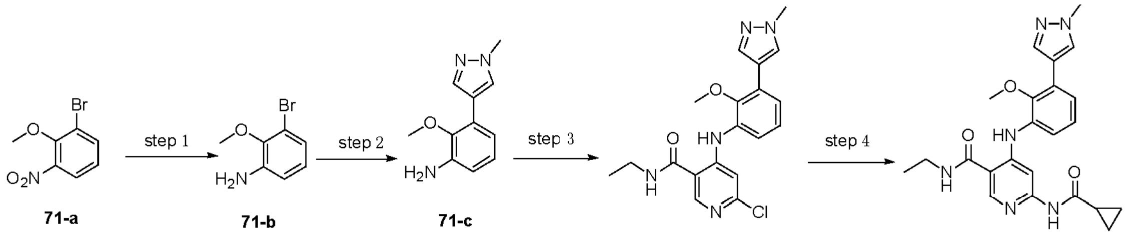


71-a 71-b 71-c 71-d 71

Step 1: 1-bromo-2-methoxy-3-nitrobenzene**(71-a,** 500 mg, 2.16 mmol) and iron powder (900 mg, 16.07 mmol) were sequentially added to a mixed solvent of acetic acid/water (1:1, 15 mL). The reaction mixture was heated to 80°C with stirring for 3 hours. Upon indication of completed reaction by TLC, the reaction mixture was allowed to cool down to room temperature, filtered by suction. The filter cake was washed with ethyl acetate (20 mL) and water (20 mL), followed by isolation of organic phase. The organic phase was sequentially washed with saturated sodium bicarbonate aqueous solution(30 mL) and saturated brine (30 mL), dried over anhydrous sodium sulfate, and concentrated under reduced pressure. The residue was separated and purified by silica gel column chromatography (EA: PE=10:1) to provide 3-bromo-2-methoxy aniline **(71-b,** 380 mg, 1.48 mmol, 69% yield). MS Calcd: 200.98; MS Found: 202.07 ($[M+H]^+$).

Step 3: 3-bromo-2-methoxy aniline **(71-b,** 380 mg, 1.48 mmol), 1-methyl-4-(4,4,5,5-tetramethyl-1,3,2-dioxaborolan-2-yl)-1H-pyrazole (458mg, 2.20mmol), Pd(dppf)$Cl_2$(60 mg, 0.06 mmol) and sodium phosphate (738mg, 4.44 mmol) were dissolved in a mixed solvent of water and dioxane (2ml/8mL). The mixture was heated to 110°C for 3h. The reaction mixture was concentrated, added with water (10ml), and extracted with ethyl acetate (10 ml x 3). The combined ethyl acetate layers were dried over anhydrous sodium sulfate, filtered by suction, and concentrated to obtain a crude material, which was purified by column chromatography (PE:EA =10:1) to provide 2-methoxy-3-(1-methyl-1H-pyrazol-4-yl)aniline **(71-c,** 220 mg, 1.08 mmol, 73% yield). MS Calcd: 203.25; MS Found: 204.1 ($[M+H]^+$).

Step 3: To 5ml of anhydrous tetrahydrofuran were added 2-methoxy-3-(1-methyl-1H-pyrazol-4-yl)aniline **(71-c,** 110 mg, 0.54 mmol) and 4,6-dichloro-N-ethyl nicotinamide (112mg, 0.54 mmol). The mixture was added at room temperature with a solution of LiHMDS in tetrahydrofuran (3 ml, 3.0 mmol), and stirred at room temperature for 2 hours. The mixture was adjusted with aqueous hydrochloride (1N) to pH 5, and extracted with ethyl acetate (10 ml x 3). The combined organic layers were dried, concentrated and then purified by column chromatography (PE:EA =1:1) to provide white solid 6-chloro-N-ethyl-4-((2-methoxy-3-(1-methyl-1H-pyrazol-4-yl)phenyl)amino)nicotinamide **(71-d,** 70 mg, 0.18mmol, 34% yield).

Step 4: 6-chloro-N-ethyl-4-((2-methoxy-3-(1-methyl-1H-pyrazol-4-yl)phenyl)amino)nicotinamide **(71-d,** 70 mg, 0.18mmol), cyclopropylcarboxamide (22mg, 0.26mmol), cesium carbonate (127mg, 0.39mmol), XantPhos(30mg, 0.04mmol) and $Pd_2(dba)_3$(24mg, 0.026mmol) were added to anhydrous dioxane (2ml). After the atmosphere of the mixture was evacuated to vacuum and refilled with nitrogen, the reaction mixture was heated to 125°C with stirring for 3 hours, and filtered by suction. The filtrate was concentrated and purified by high performance preparative thin layer chromatography (MeOH:DCM=1:10) to provide the title compound: 6-(cyclopropylcarboxamido)-N-ethyl-4-((2-methoxy-3-(1-methyl-1H-pyrazol-4-yl)phenyl)amino)nicotinamide **(71,** 10 mg, 0.023mmol, 12.8% yield). MS Calcd: 434.21; MS Found: 433.26 ($[M-H]^-$). $^1H$ NMR (400 MHz, DMSO-$d_6$)$\delta$10.78 (s, 1H), 10.63 (s, 1H), 8.66 (t, *J* = 5.6 Hz, 1H), 8.52 (s, 1H), 8.16 (s, 1H), 8.05 (s, 1H), 7.91 (s, 1H), 7.34 (d, *J* = 7.8 Hz, 1H), 7.26 (d, *J* = 7.8 Hz, 1H), 7.14 (dd, *J* = 7.8 Hz, 1H), 3.89 (s, 3H), 3.57 (s, 3H), 3.29 (t, *J* = 7.2, 2H), 2.00 - 1.96 (m, 1H), 1.15 (t, *J* = 7.2 Hz, 3H), 0.77 (m, 4H).

Example **72**

N-methoxy-4-((2-methoxy-3-(1-methyl-1*H*-pyrazol-4-yl)phenyl)amino)-6-((4-(methanesulfonyl)phenyl)amino)nicotinamide

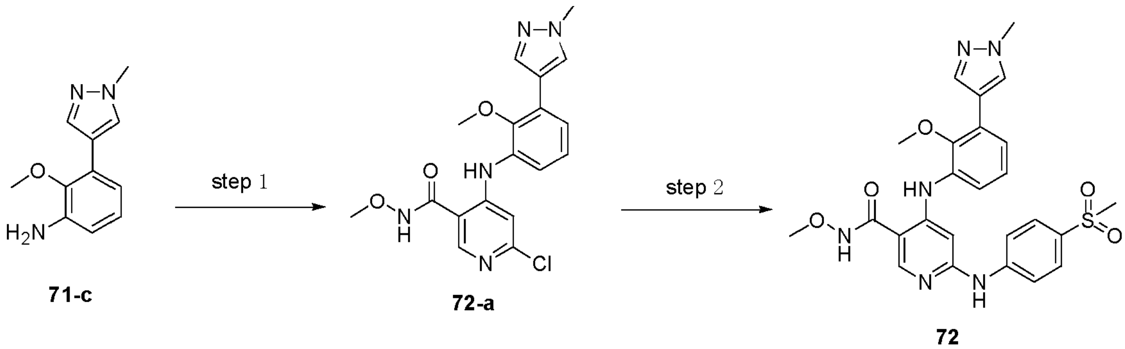


71-c 72-a 72

Step 1: To 5ml of anhydrous tetrahydrofuran were added 2-methoxy-3-(1-methyl-1H-pyrazol-4-yl)aniline **(71-c,** 110 mg, 0.54 mmol), 4,6-dichloro-N-methoxy nicotinamide **(int-1,** 112mg, 0.54 mmol). The mixture was added at room temperature with a solution of LiHMDS in tetrahydrofuran (3 ml, 3.0 mmol), and stirred at room temperature for 2 hours. The mixture was adjusted with aqueous hydrochloride (1N) to pH 5, and extracted with ethyl acetate (10 ml x 3). The combined organic layers were dried, concentrated and then purified by column chromatography (PE:EA =1:1) to provide white solid 6-chloro-N-methoxy-4-((2-methoxy-3-(1-methyl-1H-pyrazol-4-yl)phenyl)amino)nicotinamide **(72-a,** 140 mg, 0.36 mmol, 69% yield). MS Calcd: 387.82; MS Found: 386.19 ($[M-H]^-$).

Step 2: 6-chloro-N-methoxy-4-((2-methoxy-3-(1-methyl-1H-pyrazol-4-yl)phenyl)amino)nicotinamide (32061-d, 140 mg, 0.36mmol), cyclopropylcarboxamide (44mg, 0.52mmol), cesium carbonate (256 mg, 0.8mmol), XantPhos(60mg, 0.08mmol) and $Pd_2(dba)_3$ (48 mg, 0.052 mmol) were added to anhydrous dioxane (2 ml). After the atmosphere of the mixture was evacuated to vacuum and refilled with nitrogen, the reaction mixture was heated to 125°C with stirring for 3 hours, and filtered by suction. The filtrate was concentrated and purified by high performance preparative thin layer chromatography (MeOH:DCM=1:10) to provide the title compound: N-methoxy-4-((2-methoxy-3-(1-methyl-1H-pyrazol-4-yl)phenyl)amino)-6-((4-(methanesulfonyl)phenyl)amino)nicotinamide **(72,** 30 mg, 0.057mmol, 15.9% yield). MS Calcd: 522.17; MS Found: 523.33 ($[M+H]^+$). $^1H$ NMR (400 MHz, DMSO-$d_6$) $\delta$ 11.83 (s, 1H), 10.06 (s, 1H), 9.69 (s, 1H), 8.37 (s, 1H), 8.18 (s, 1H), 7.93 (s, 1H), 7.88 (d, *J* = 8.8 Hz, 2H), 7.76 (d, *J*= 8.8 Hz, 2H), 7.40 (d, *J* = 7.8 Hz, 1H), 7.32 (d, *J* = 7.8 Hz, 1H), 7.19 (t, *J* = 7.8 Hz, 1H), 6.64 (s, 1H), 3.90 (s, 3H), 3.74 (s, 3H), 3.62 (s, 3H), 3.12 (s, 3H).

Example **73**

6-(cyclopropylcarboxamido)-4-((2-(dimethyl phosphoryl)phenyl)amino)-N-methoxy nicotinamide

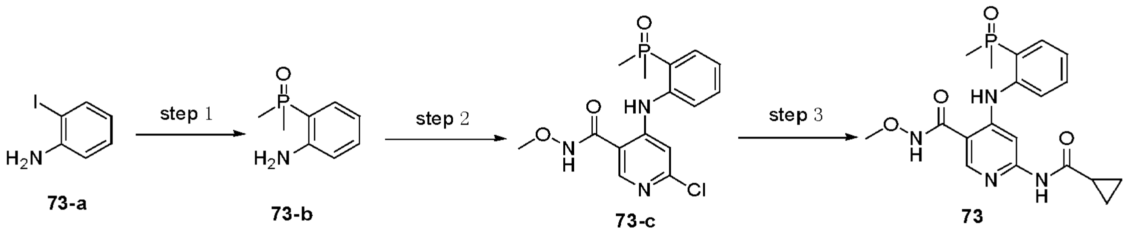


73-a 73-b 73-c 73

Step 1: 2-iodo aniline **(73-a,** 0.3 g, 1.36 mmol), dimethyl phosphorus oxide (128 mg, 1.63 mmol), potassium phosphate (318 mg, 1.50 mmol), palladium acetate (30 mg, 0.14 mmol) and XantPhos (90 mg, 0.16 mmol) were sequentially added to anhydrous dioxane (3 mL). The atmosphere of the mixture was evacuated to vacuum and replaced with nitrogen three times. After stirring at 130°C for 16 hours, TLC indicated a completed reaction. The mixture was concentrated under reduced pressure, and the residue was separated and purified by silica gel column chromatography (DCM: MeOH =10:1) to provide 2-(dimethyl phosphoryl) aniline (32117-b, 200 mg, 1.17 mmol, 87% yield). MS Calcd: 169.16; MS Found: 170.12 ($[M+H]^+$).

Step 2: To 5ml of anhydrous tetrahydrofuran were added 2-(dimethyl phosphoryl) aniline **(73-b,** 200 mg, 1.17 mmol) and 4,6-dichloro-N-methoxy nicotinamide (**int-1,** 257 mg, 1.17 mmol). The mixture was added at room temperature with a solution of LiHMDS in tetrahydrofuran (2.3 ml, 2.3 mmol), and stirred at 50°C for 3 hours. The mixture was adjusted with aqueous hydrochloride (1N) to pH 5, and extracted with ethyl acetate (10ml x 3). The combined organic layers were dried, concentrated and then purified by column chromatography (DCM: MeOH =10:1) to provide 6-chloro-4-((2-(dimethyl phosphoryl)phenyl)amino)-N-methoxy nicotinamide **(73-c,** 200mg, 0.56 mmol, 48% yield). MS Calcd: 353.74; MS Found:

354.23 ([M+H]+).

Step 3: 6-chloro-4-((2-(dimethyl phosphoryl)phenyl)amino)-N-methoxy nicotinamide **(73-c,** 100 mg, 0.28 mmol), cyclopropylcarboxamide (48 mg, 0.52 mmol), cesium carbonate (170 mg, 0.0.52 mmol), XantPhos(23 mg, 0.04mmol) and $Pd_2(dba)_3$(27mg, 0.03 mmol) were added to anhydrous dioxane (2ml). After the atmosphere of the mixture was evacuated to vacuum and refilled with nitrogen, the reaction mixture was heated to 125°C with stirring for 3 hours, and filtered by suction. The filtrate was concentrated and purified by high performance preparative thin layer chromatography (MeOH:DCM=1:20) to provide the title compound: 6-(cyclopropylcarboxamido)-4-((2-(dimethyl phosphoryl)phenyl)amino)-N-methoxy nicotinamide **(73,** 15 mg, 0.037 mmol, 13% yield). MS Calcd: 402.39; MS Found: 403.33 ([M+H]+). $^1$H NMR (400 MHz, DMSO-*d*$_6$)$\delta$11.88 (s, 1H), 10.59 (s, 1H), 8.45 (s, 1H), 7.85 (d, *J* = 7.6 Hz, 1H), 7.69 (d, *J* = 12.8 Hz, 1H), 7.62 - 7.53 (m, 2H), 7.39 (d, *J* = 7.6 Hz, 1H), 7.33 (t, *J* = 7.6 Hz, 1H), 3.63 (s, 3H), 1.95 - 1.87 (m, 1H), 1.68 (s, 3H), 1.65 (s, 3H), 0.75 - 0.65 (m, 4H).

Example **74**

4-((2-(dimethyl phosphoryl)phenyl)amino)-6-((6-fluoropyridin-2-yl)amino)-N-methoxy nicotinamide

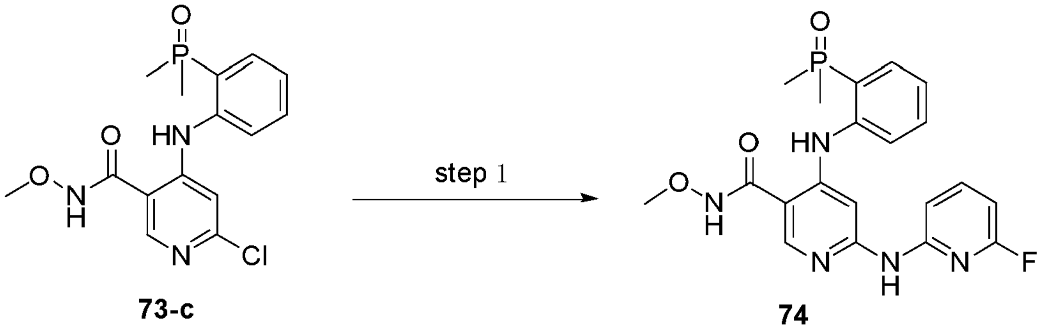


**73-c** **74**

Step 1: 6-chloro-4-((2-(dimethyl phosphoryl)phenyl)amino)-N-methoxy nicotinamide **(73-c,** 100 mg, 0.28 mmol), 2-amino-6-fluoropyridine (62 mg, 0.56 mmol), cesium carbonate (170 mg, 0.52 mmol), XantPhos(23 mg, 0.04mmol) and $Pd_2(dba)_3$(27mg, 0.03 mmol) were added to anhydrous dioxane (2ml). After the atmosphere of the mixture was evacuated to vacuum and refilled with nitrogen, the reaction mixture was heated to 125°C with stirring for 3 hours, and filtered by suction. The filtrate was concentrated and purified by high performance preparative thin layer chromatography (MeOH:DCM=1:20) to provide the title compound: 4-((2-(dimethyl phosphoryl)phenyl)amino)-6-((6-fluoropyridin-2-yl) amino)-N-methoxy nicotinamide

**(74,** 15 mg, 0.035 mmol, 12.5% yield). MS Calcd: 429.14; MS Found: 430.31 ([M+H]+). $^1$H NMR (400 MHz, DMSO-d$_6$)$\delta$11.82 (s, 1H), 10.55 (s, 1H), 9.73 (s, 1H), 8.43 (s, 1H), 7.90 - 7.81 (m, 1H), 7.77-7.71 (m, 1H), 7.60 (t, *J* = 7.6 Hz, 1H), 7.52-7.49 (m, 2H), 7.37-7.32 (m, 1H), 7.16 (s, 1H), 6.47 (dd, *J* = 8.0, 6.4 Hz, 1H), 3.63 (s, 3H), 1.70 (s, 3H), 1.67 (s, 3H).

Example **75**

4-((3-chloro-2-(N-methyl methanesulfonamido)phenyl)amino)-6-(cyclopropylcarboxamido)-N-methoxy nicotinamide

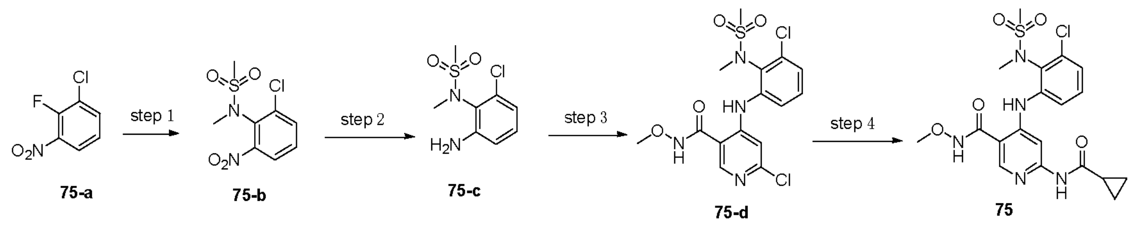


Step 1: To N-methyl methanesulfonamide (0.5 g, 4.58 mmol) dissolved in 20 mL of N,N-dimethyl formamide was added sodium hydride (0.24 g, 10 mmol) portionwise. The mixture was heated to 55 °C with continuous stirring for 2 hours, followed by adding 2-fluoro-3-trifluoromethyl nitrobenzene (32182-a, 0.8 g, 4.57 mmol) with continuous stirring at that

temperature for 6 hours. Upon indication of completed reaction by TLC, the reaction mixture was added with water (40 mL), extracted with ethyl acetate (30 mL x 2) and washed with saturated brine (30 mL x 2), dried over anhydrous sodium sulfate, and concentrated under reduced pressure. The residue was separated and purified by silica gel column chromatography (EA:PE=1:1) to provide N-(2-chloro-6-nitrophenyl)-N-methyl methanesulfonamide **(75-b,** 0.7 g, 2.65 mmol, 58% yield). MS Calcd: 264.68; MS Found: 265.14 ($[M+H]^+$).

Step 2: N-(2-chloro-6-nitrophenyl)-N-methyl methanesulfonamide **(75-b,** 0.6 g, 2.27 mmol), ammonium chloride (0.75 g, 14 mmol) and iron powder (0.6 g, 10.71 mmol) were sequentially added to 20 mL of mixed solvent of water and ethanol(1:4). The mixture was stirred under reflux for 6 hours. Upon indication of completed reaction by TLC, the reaction mixture was filtered by suction. The filtrate was concentrated, and the residue was separated and purified by silica gel column chromatography (EA:PE=1:1) to provide N-(2-amino-6-chloro phenyl)-N-methyl methanesulfonamide **(75-c,** 0.42 g, 1.8 mmol, 79% yield). MS Calcd: 234.70; MS Found: 235.10 ($[M+H]^+$). Step 3: To 10 ml of anhydrous N,N-dimethylacetamide were added N-(2-amino-6-chloro phenyl)-N-methyl methanesulfonamide **(75-c,** 0.42 g, 1.8 mmol) and 4,6-dichloro-N-methoxy nicotinamide **(int-1,** 409 mg, 1.86 mmol). The mixture was added at room temperature with a solution of LiHMDS in tetrahydrofuran (7.2 ml, 7.2 mmol), and stirred at room temperature for 3 hours. Upon indication of completed reaction by TLC, the mixture was adjusted with aqueous hydrochloride (1N) to pH 5, and extracted with ethyl acetate (20 ml x 3). The combined organic layers were dried, concentrated and then purified by column chromatography (PE:EA =1:1) to provide 6-chloro-4-((3-chloro-2-(N-methyl methanesulfonamido)phenyl)amino)-N-methoxy nicotinamide **(75-d,** 0.7 g, 1.6 mmol, 88% yield). MS Calcd: 418.03; MS Found: 417.03 ($[M-H]^-$).

Step 4: 6-chloro-4-((3-chloro-2-(N-methyl methanesulfonamido)phenyl)amino)-N-methoxy nicotinamide **(75-d,** 0.21 g, 0.5 mmol), cyclopropylcarboxamide (85 mg, 1 mmol), cesium carbonate (325 mg, 1 mmol), XantPhos(46mg, 0.05mmol) and $Pd_2(dba)_3$(43mg, 0.075 mmol) were added to anhydrous dioxane (4 ml). After the atmosphere of the mixture was evacuated to vacuum and refilled with nitrogen, the reaction mixture was heated to 125°C with stirring for 3 hours, and filtered by suction. The filtrate was concentrated and purified by high performance preparative thin layer chromatography (MeOH:DCM=1:10) to provide the title compound: 4-((3-chloro-2-(N-methyl methanesulfonamido) phenyl)amino)-6-(cyclopropylcarboxamido)-N-methoxy nicotinamide **(75,** 40mg, 0.085mmol, 16% yield). MS Calcd: 467.10; MS Found: 468.35 ($[M+H]^+$). $^1$HNMR (400 MHz, DMSO-$d_6$)$\delta$11.88 (s, 1H), 10.85 (s, 1H), 10.13 (s, 1H), 8.39 (s, 1H), 7.92 (s, 1H), 7.51 - 7.34 (m, 3H), 3.72 (s, 3H), 3.17 (s, 3H), 3.12 (s, 3H), 2.00 - 1.93 (m, 1H), 0.78 - 0.76 (m, 4H).

Example **76**

4-((3-chloro-2-(N-methyl methanesulfonamido)phenyl)amino)-6-((6-fluoropyridin-2-yl)amino)-N-methoxy nicotinamide

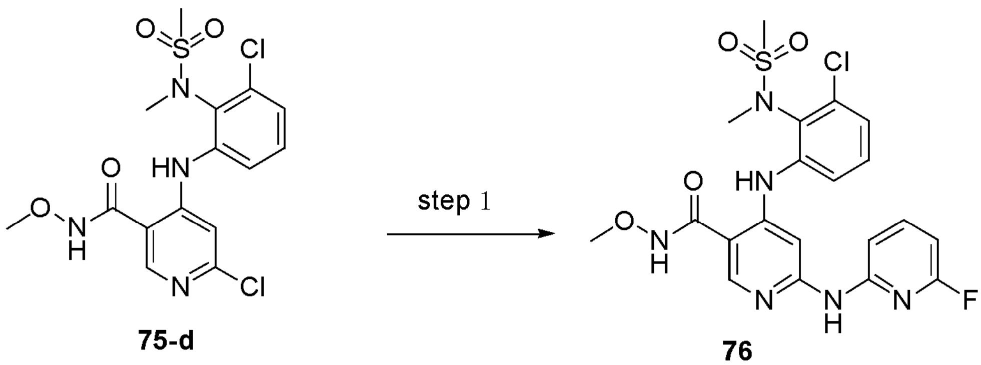


**75-d** **76**

Step 1: 6-chloro-4-((3-chloro-2-(N-methyl methanesulfonamido)phenyl)amino)-N-methoxy nicotinamide **(75-d,** 0.15 g, 0.36 mmol), 2-amino-6-fluoropyridine (80 mg, 0.75 mmol), cesium carbonate (230 mg, 0.72 mmol), XantPhos(31mg, 0.054mmol) and $Pd_2(dba)_3$(32mg, 0.036 mmol) were added to anhydrous dioxane (2 ml). After the atmosphere of the mixture was evacuated to vacuum and refilled with nitrogen, the reaction mixture was heated to 125°C with stirring for 3 hours, and filtered by suction. The filtrate was concentrated and purified by high performance preparative thin layer chromatography (MeOH:DCM=1:10) to provide the title compound: 4-((3-chloro-2-(N-methyl methanesulfonamido) phenyl)amino)-6-((6-fluoropyridin-2-yl)amino)-N-methoxy nicotinamide **(76,** 30mg, 0.06mmol, 40% yield). MS Calcd: 494.09; MS Found: 495.34 ($[M+H]^+$). $^1$HNMR (400 MHz, DMSO-$d_6$)$\delta$11.83 (s, 1H), 10.19 (s, 1H), 10.09 (s, 1H), 8.37 (s, 1H), 7.84 - 7.76 (m, 1H), 7.64 - 7.62 (m, 2H), 7.48 - 7.44 (m, 2H), 7.36 (dd, *J* = 8.0, 1.6 Hz, 1H), 6.58 (dd, *J* = 8.0, 2.4 Hz, 1H), 3.73 (s, 3H), 3.21 (s, 3H), 3.14 (s, 3H).

Example **77**

4-((3-chloro-2-(N-methyl methanesulfonamido)phenyl)amino)-6-((5-fluoropyridin-2-yl)amino)-N-methoxy nicotinamide

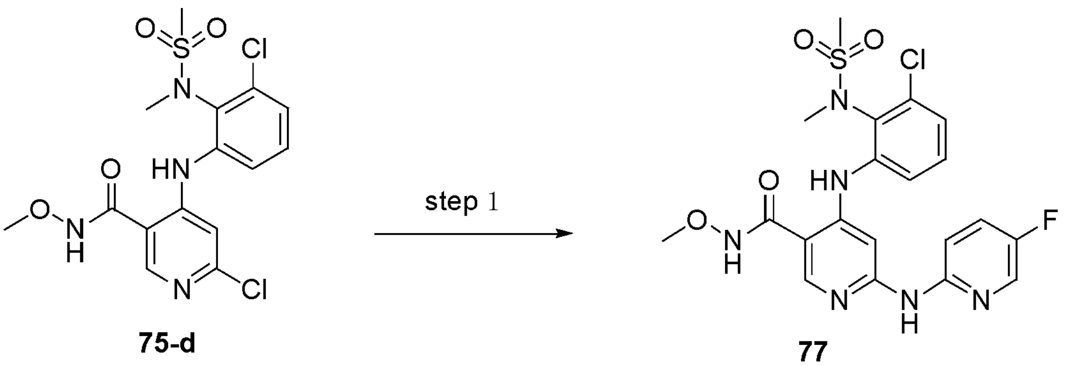


75-d 77

Step 1: 6-chloro-4-((3-chloro-2-(N-methyl methanesulfonamido)phenyl)amino)-N-methoxy nicotinamide **(75-d,** 0.15 g, 0.36 mmol), 2-amino-5-fluoropyridine (80 mg, 0.75 mmol), cesium carbonate (230 mg, 0.72 mmol), Xant-Phos(31mg, 0.054mmol) and $Pd_2(dba)_3$(32mg, 0.036 mmol) were added to anhydrous dioxane (2 ml). After the atmosphere of the mixture was evacuated to vacuum and refilled with nitrogen, the reaction mixture was heated to 125°C with stirring for 3 hours, and filtered by suction. The filtrate was concentrated and purified by high performance preparative thin layer chromatography (MeOH:DCM=1:10) to provide the title compound: 4-((3-chloro-2-(N-methyl methanesulfonamido) phenyl)amino)-6-((5-fluoropyridin-2-yl)amino)-N-methoxy nicotinamide **(77,** 30mg, 0.06mmol, 40% yield). MS Calcd: 494.09; MS Found: 495.31 ([M+H]+). $^1$H NMR (400 MHz, DMSO-$d_6$)$\delta$11.80 (s, 1H), 10.17 (s, 1H), 9.89 (s, 1H), 8.35 (s, 1H), 8.15 (s, 1H), 7.72 - 7.57 (m, 4H), 7.49 (t, *J* = 8.0 Hz, 1H), 7.33 (d, *J* = 8.0 Hz, 1H), 3.72 (s, 3H), 3.21 (s, 3H), 3.14 (s, 3H).

Example **78**

4-((3-chloro-2-(N-methyl methanesulfonamido)phenyl)amino)-N-methoxy-6-((2-methoxy pyridin-3-yl)amino)nicotinamide

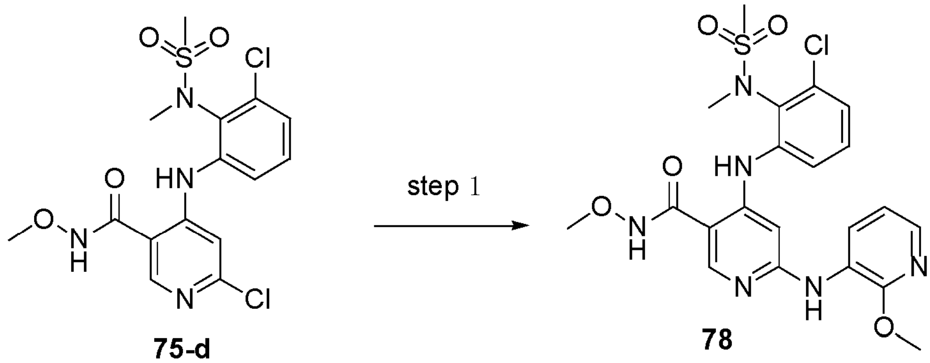


75-d 78

Step 1: 6-chloro-4-((3-chloro-2-(N-methyl methanesulfonamido)phenyl)amino)-N-methoxy nicotinamide **(75-d,** 0.2 g, 0.48 mmol), 2-methoxy-3-aminopyridine (120 mg, 0.96 mmol), cesium carbonate (312 mg, 0.96 mmol), Xant-Phos(42mg, 0.042mmol) and $Pd_2(dba)_3$(48mg, 0.044 mmol) were added to anhydrous dioxane (3 ml). After the atmosphere of the mixture was evacuated to vacuum and refilled with nitrogen, the reaction mixture was heated to 125°C with stirring for 3 hours, and filtered by suction. The filtrate was concentrated and purified by high performance preparative thin layer chromatography (MeOH:DCM=1:10) to provide the title compound: 4-((3-chloro-2-(N-methyl methanesulfonamido) phenyl)amino)-N-methoxy-6-((2-methoxy pyridin-3-yl)amino)nicotinamide **(78,** 30mg, 0.059mmol, 12% yield). MS Calcd: 506.11; MS Found: 507.40 ([M+H]+). $^1$H NMR (400 MHz, DMSO-$d_6$)$\delta$11.76 (s, 1H), 10.01 (s, 1H), 8.62 (s, 1H), 8.50 (dd, *J* = 8.0, 1.6 Hz, 1H), 8.29 (s, 1H), 7.73 (dd, *J* = 5.6, 2.0 Hz, 1H), 7.52 (dd, *J* = 8.0, 1.6 Hz, 1H), 7.46 - 7.41 (m, 1H), 7.33 (dd, *J* = 8.0, 1.6 Hz, 1H), 6.93 (dd, *J* = 8.0, 1.6 Hz, 1H), 6.83 (s, 1H), 3.91 (s, 3H), 3.72 (s, 3H), 3.20 (s, 3H), 3.13 (s, 3H).

Example **79**

4-((4-chloro-2-(N-methyl methanesulfonamido)phenyl)amino)-6-(cyclopropylcarboxamido)-N-methoxy nicotinamide

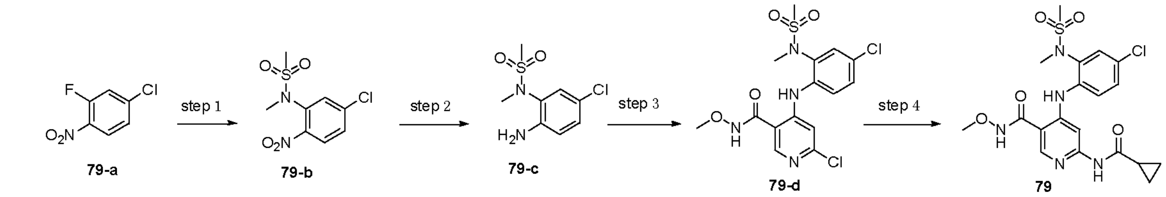


79-a 79-b 79-c 79-d 79

Step 1: To N-methyl methanesulfonamide (0.5 g, 4.5 mmol) dissolved in 20 mL N,N-dimethyl formamide was added with sodium hydride (0.24 g, 10 mmol) portionwise. After that the reaction mixture was heated to 55 °C with continuous stirring for 2 hours, added with 4-chloro-2-fluoro-1-nitrobenzene **(79-a,** 800 mg, 4.5 mmol) with continuous stirring at that temperature for 6 hours. Upon indication of completed reaction by TLC, the reaction mixture was added with water (40 mL), and extracted with ethyl acetate (30 mL x 2) and washed with saturated brine (30 mL x 2), dried over anhydrous sodium sulfate, and concentrated under reduced pressure. The residue was separated and purified by silica gel column chromatography (EA:PE=1:1) to provide N-(5-chloro-2-nitrophenyl)-N-methyl methanesulfonamide **(79-b,** 0.52 g, 1.96 mmol, 43% yield). MS Calcd: 264.00; MS Found: 263.15 ($[M-H]^-$).

Step 2: N-(5-chloro-2-nitrophenyl)-N-methyl methanesulfonamide **(79-b,** 0.19 g, 0.71 mmol), ammonium chloride (0.25 g, 4.7 mmol) and iron powder (0.2 g, 3.57 mmol) were sequentially added to 10 mL of mixed solvent of water and ethanol (1:4), and stirred under reflux for 6 hours. Upon indication of completed reaction by TLC, the reaction mixture was filtered by suction. The filtrate was concentrated, and the residue was separated and purified by silica gel column chromatography to provide N-(2-amino-5-chloro phenyl)-N-methyl methanesulfonamide **(79-c,** 0.15 g, 0.64 mmol, 90% yield). MS Calcd: 234.02; MS Found: 235.13 ($[M+H]^+$).

Step 3: To 10 ml of anhydrous N,N-dimethylacetamide were added N-(2-amino-5-chloro phenyl)-N-methyl methanesulfonamide **(79-c,** 0.15 g, 0.64 mmol) and 4,6-dichloro-N-methoxy nicotinamide **(int-1,** 140 mg, 0.63 mmol). The mixture was added at room temperature with a solution of LiHMDS in tetrahydrofuran (2.5 ml, 2.5 mmol) and stirred at room temperature for 3 hours. Upon indication of completed reaction by TLC, the mixture was adjusted with aqueous hydrochloride (1N) to pH 5 and extracted with ethyl acetate (20 ml x 3). The combined organic layers were dried, concentrated and then purified by column chromatography (PE:EA =1:1) to provide 6-chloro-4-((4-chloro-2-(N-methyl methanesulfonamido)phenyl)amino)-N-methoxy nicotinamide **(79-d,** 240 mg, 0.57 mmol, 89% yield). MS Calcd: 418.03; MS Found: 417.03 ($[M-H]^-$).

Step 4: 6-chloro-4-((4-chloro-2-(N-methyl methanesulfonamido)phenyl)amino)-N-methoxy nicotinamide **(79-d,** 120 mg, 0.29 mmol), cyclopropylcarboxamide (50 mg, 0.58 mmol), cesium carbonate (190 mg, 0.58 mmol), Xant-Phos(25mg, 0.04mmol) and $Pd_2(dba)_3$(27mg, 0.029 mmol) were added to anhydrous dioxane (2 ml). After the atmosphere of the mixture was evacuated to vacuum and refilled with nitrogen, the reaction mixture was heated to 125°C with stirring for 3 hours, and filtered by suction. The filtrate was concentrated and purified by high performance preparative thin layer chromatography (MeOH:DCM=1:20) to provide the title compound: 4-((4-chloro-2-(N-methyl methanesulfonamido) phenyl)amino)-6-(cyclopropylcarboxamido)-N-methoxy nicotinamide (79, 20mg, 0.042mmol, 14% yield). MS Calcd: 467.10; MS Found: 468.31 ($[M+H]^+$). $^1$HNMR (400 MHz, DMSO-$d_6$)$\delta$11.78 (s, 1H), 10.82 (s, 1H), 10.04 (s, 1H), 8.35 (s, 1H), 7.90 (s, 1H), 7.71 (d, $J$ = 2.4 Hz, 1H), 7.49 - 7.48 (m, 2H), 3.72 (s, 3H), 3.15 (s, 3H), 3.13 (s, 3H), 1.97 (m, 1H), 0.79 - 0.76 (m, 4H).

Example **80**

4-((4-chloro-2-(N-methyl methanesulfonamido)phenyl)amino)-6-((5-fluoropyridin-2-yl)amino)-N-methoxy nicotinamide

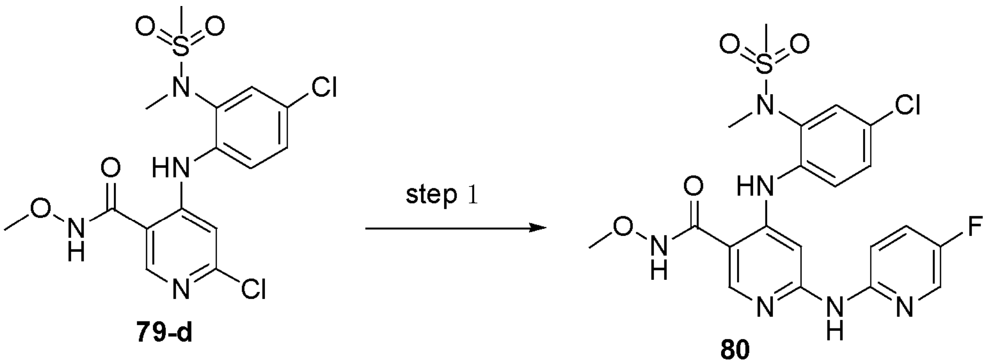


79-d

80

Step 4: 6-chloro-4-((4-chloro-2-(N-methyl methanesulfonamido)phenyl)amino)-N-methoxy nicotinamide **(79-d,** 120 mg, 0.29 mmol), 2-amino-5-fluoropyridine (65 mg, 0.58 mmol), cesium carbonate (190 mg, 0.58 mmol), Xant-Phos(25mg, 0.04mmol) and $Pd_2(dba)_3$(27mg, 0.029 mmol) were added to anhydrous dioxane (2 ml). After the atmosphere of the mixture was evacuated to vacuum and refilled with nitrogen, the reaction mixture was heated to 125°C with stirring for 3 hours, and filtered by suction. The filtrate was concentrated and purified by high performance preparative thin layer chromatography (MeOH:DCM=1:20) to provide the title compound: 4-((4-chloro-2-(N-methyl methanesulfonamido) phenyl)amino)-6-((5-fluoropyridin-2-yl)amino)-N-methoxy nicotinamide **(80,** 20mg, 0.04mmol, 13% yield). MS Calcd: 494.09; MS Found: 493.23 ($[M-H]^-$). $^1H$ NMR (400 MHz, DMSO-$d_6$)δ11.51 (s, 1H), 10.15 (s, 1H), 9.86 (s, 1H), 8.32 (s, 1H), 8.21 (s, 1H), 7.67 - 7.56 (m, 6H), 3.71 (s, 3H), 3.17 (s, 3H), 3.16 (s, 3H).

Example **81**

6-(cyclopropylcarboxamido)-N-methoxy-4-((4-methyl-2-(N-methyl methanesulfonamido)phenyl)amino)nicotinamide

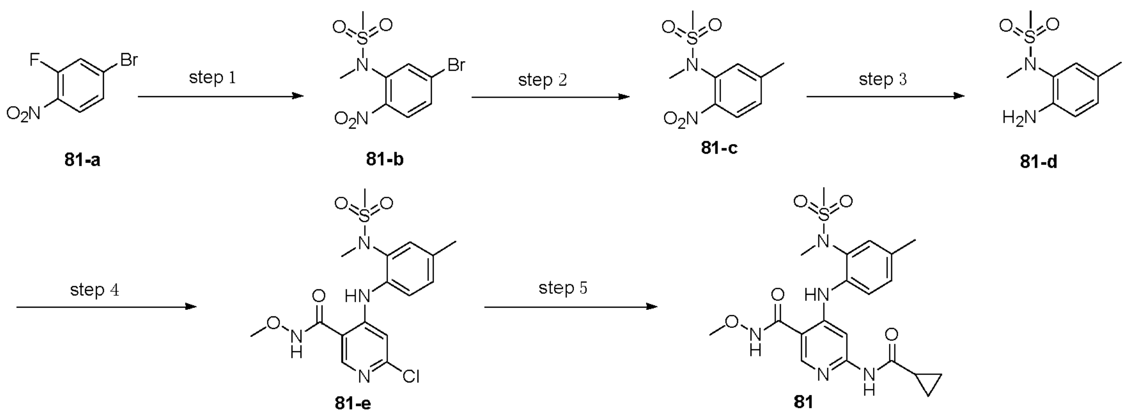


81-a 81-b 81-c 81-d 81-e 81

Step 1: To N-methyl methanesulfonamide (0.5 g, 5.5 mmol) dissolved in 20 mL of N,N-dimethyl formamide was added sodium hydride (0.29g, 12 mmol) portionwise. After that the reaction was heated to 55 °C with continuous stirring for 2 hours, added with 4-bromo-2-fluoro-1-nitrobenzene **(81-a,** 1.2 g, 5.5 mmol) with continuous stirring at that temperature for 6 hours. Upon indication of completed reaction by TLC, the reaction mixture was added with water (40 mL), and extracted with ethyl acetate (30 mL x 2). The organic phase was washed with saturated brine (30 mL x 2), dried over anhydrous sodium sulfate, and concentrated under reduced pressure. The residue was separated and purified by silica gel column chromatography (EA:PE=1:5) to provide N-(5-bromo-2-nitrophenyl)-N-methyl methanesulfonamide **(81-b,** 0.9 g, 2.9 mmol, 52% yield). MS Calcd: 307.95; MS Found: 307.00 ($[M-H]^-$).

Step 2: N-(5-bromo-2-nitrophenyl)-N-methyl methanesulfonamide **(81-b,** 0.308 g, 1 mmol), methyl boronic acid (78 mg, 1.3 mmol), potassium phosphate (0.53 g, 2.5 mmol) and Pd(dppf)$Cl_2$ (0.036 g, 0.05 mmol) were sequentially added to 8 mL of solution of dioxane/water (7/1). The atmosphere of the mixture was evacuated to vacuum and replaced with nitrogen three times, and stirred at 110°C for 6 hours. Upon indication of completed reaction by TLC, the mixture was concentrated under reduced pressure. The residue was separated and purified by silica gel column chromatography (EA:PE=1:1) to provide N-methyl-N-(5-methyl-2-nitrophenyl)methyl methanesulfonamide **(81-c,** 0.1 g, 0.4 mmol, 40% yield).

Step 3: N-methyl-N-(5-methyl-2-nitrophenyl)methyl methanesulfonamide **(81-c,** 0.1 g, 0.4 mmol), ammonium chloride (0.25 g, 4.7 mmol) and iron powder (0.2 g, 3.57 mmol) were sequentially added to 10 mL of mixed solvent of water and ethanol (1:4). The mixture was stirred under reflux for 6 hours. Upon indication of completed reaction by TLC, the reaction mixture was filtered by suction. The filtrate was concentrated, and the residue was separated and purified by silica gel column chromatography to provide colorless oil N-(2-amino-5-methyl phenyl)-N-methyl methanesulfonamide **(81-d,** 0.06 g, 0.28 mmol, 70% yield). MS Calcd: 214.28; MS Found: 215.16 ($[M+H]^+$).

Step 4: To 10 ml of anhydrous N,N-dimethylacetamide were added N-(2-amino-5-methyl phenyl)-N-methyl methanesulfonamide **(81-d,** 0.06 g, 0.28 mmol) and 4,6-dichloro-N-methoxy nicotinamide **(int-1,** 61 mg, 0.28 mmol). The mixture was added at room temperature with a solution of LiHMDS in tetrahydrofuran (1.12 ml, 1.12 mmol), and stirred at room temperature for 3 hours. Upon indication of completed reaction by TLC, the mixture was adjusted with aqueous hydrochloride (1N) to pH 5, and extracted with ethyl acetate (10 ml x 3). The combined organic layers were dried, concentrated and then purified by column chromatography (PE:EA =1:1) to provide 6-chloro-N-methoxy-4-((4-methyl-2-(N-methyl methanesulfonamido)phenyl)amino)nicotinamide **(81-e,** 80 mg, 0.2 mmol, 71% yield), as a tan oil. MS Calcd: 398.08; MS Found: 399.34 ($[M+H]^+$).

Step 5: 6-chloro-N-methoxy-4-((4-methyl-2-(N-methyl methanesulfonamido)phenyl)amino)nicotinamide **(81-e,** 80 mg, 0.2 mmol), cyclopropylcarboxamide (34 mg, 0.4 mmol), cesium carbonate (130 mg, 0.4 mmol), XantPhos(18mg, 0.03mmol) and $Pd_2(dba)_3$(20mg, 0.02 mmol) were added to anhydrous dioxane (2 ml). After the atmosphere of the mixture was evacuated to vacuum and refilled with nitrogen, the reaction mixture was heated to 125°C with stirring for 3 hours, and filtered by suction. The filtrate was concentrated and purified by high performance preparative thin layer chromatography (MeOH:DCM=1:20) to provide the title compound: 6-(cyclopropylcarboxamido)-N-methoxy-4-((4-methyl-2-(N-methyl methanesulfonamido)phenyl)amino)nicotinamide **(81,** 10mg, 0.02mmol, 10% yield). MS Calcd: 447.16; MS Found: 446.25 ($[M-H]^-$). $^1$H NMR (400 MHz, DMSO-$d_6$)$\delta$11.77 (s, 1H), 10.75 (s, 1H), 9.90 (s, 1H), 8.31 (s, 1H), 7.84 (s, 1H), 7.38 (d, $J$ = 2.0 Hz, 1H), 7.35 (d, $J$ = 8.0 Hz, 1H), 7.21 (dd, $J$ = 8.0, 2.0 Hz, 1H), 3.71 (s, 3H), 3.11 (s, 3H), 3.09 (s, 3H), 2.33 (s, 3H), 1.99 - 1.93 (m, 1H), 0.78 - 0.71 (m, 4H).

Example **82**

6-(cyclopropylcarboxamido)-N-methoxy-4-((2-(N-methyl methanesulfonamido)-3-(trifluoromethyl)phenyl)amino)nicotinamide

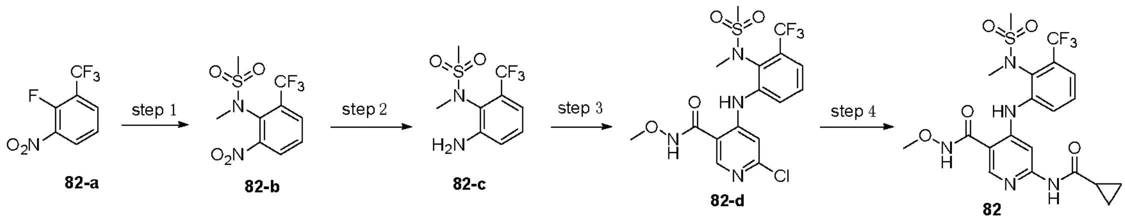


82-a 82-b 82-c 82-d 82

Step 1: To N-methyl methanesulfonamide (0.4 g, 3.6 mmol) dissolved in 20 mL of N,N-dimethyl formamide was added sodium hydride (0.2 g, 8.3 mmol) portionwise. After that, the reaction mixture was heated to 55°C with continuous stirring for 2 hours, added with 2-fluoro-3-trifluoromethyl nitrobenzene **(82-a,** 750 mg, 3.6 mmol), and continuously at that temperature stirred for 6 hours. Upon indication of completed reaction by TLC, the reaction mixture was added with water (40 mL), and extracted with ethyl acetate (30 mL x 2). The organic phase was washed with saturated brine (30 mL x 2), dried over anhydrous sodium sulfate, and concentrated under reduced pressure. The residue was separated and purified by silica gel column chromatography (EA:PE=1:1) to provide N-methyl-N-(2-nitro-6-(trifluoromethyl)phenyl)methanesulfonamide **(82-b,** 1.0 g, 3.3 mmol, 92% yield). MS Calcd: 298.24; MS Found: 321.14 ($[M+Na^+]^+$).

Step 2: N-methyl-N-(2-nitro-6-(trifluoromethyl)phenyl)methanesulfonamide **(82-b,** 0.65 g, 2.18 mmol), ammonium chloride (0.75 g, 14 mmol) and iron powder (0.6 g, 10.71 mmol) were sequentially added to 20 mL of mixed solvent of water and ethanol (1:4). The mixture was stirred under reflux for 6 hours. Upon indication of completed reaction by TLC, the reaction mixture was filtered by suction. The filtrate was concentrated, and the residue was separated and purified by silica gel column chromatography to provide N-(2-amino-6-(trifluoromethyl)phenyl)-N-methyl methanesulfonamide **(82-c,** 0.5 g, 1.86 mmol, 85% yield), as a colorless oil. MS Calcd: 268.25; MS Found: 269.18 ($[M+H]^+$).

Step 3: To 10 ml of anhydrous N,N-dimethylacetamide were added N-(2-amino-6-(trifluoromethyl)phenyl)-N-methyl methanesulfonamide **(82-c,** 0.5 g, 1.86 mmol) and 4,6-dichloro-N-methoxy nicotinamide (**int-1**, 409 mg, 1.86 mmol). The mixture was added at room temperature with a solution of LiHMDS in tetrahydrofuran (7.6 ml, 7.6 mmol), and stirred at room temperature for 3 hours. Upon indication of completed reaction by TLC, the mixture was adjusted with

aqueous hydrochloride (1N) to pH 5, and extracted with ethyl acetate (20 ml x 3). The combined organic layers were dried, concentrated and then purified by column chromatography (PE:EA =1:1) to provide 6-chloro-N-methoxy-4-((2-(N-methyl methanesulfonamido)-3-(trifluoromethyl)phenyl)amino)nicotinamide **(82-d,** 410 mg, 0.9 mmol, 49% yield), as a yellow powdery solid. MS Calcd: 452.83; MS Found: 453.27 ($[M+H]^+$).

Step 4: 6-chloro-N-methoxy-4-((2-(N-methyl methanesulfonamido)-3-(trifluoromethyl)phenyl)amino)nicotinamide **(82-d,** 130 mg, 0.29 mmol), cyclopropylcarboxamide (50 mg, 0.58 mmol), cesium carbonate (190 mg, 0.58 mmol), XantPhos(25mg, 0.04mmol) and $Pd_2(dba)_3$(27mg, 0.029 mmol) were added to anhydrous dioxane (2 ml). After the atmosphere of the mixture was evacuated to vacuum and refilled with nitrogen, the reaction mixture was heated to 125°C with stirring for 3 hours, and filtered by suction. The filtrate was concentrated and purified by high performance preparative thin layer chromatography (MeOH:DCM=1:20) to provide the title compound: 6-(cyclopropylcarboxamido)-N-methoxy-4-((2-(N-methyl methanesulfonamido)-3-(trifluoromethyl)phenyl)amino)nicotinamide **(82,** 20mg, 0.039mmol, 13% yield). MS Calcd: 501.48; MS Found: 502.40 ($[M+H]^+$). $^1H$ NMR (400 MHz, DMSO-$d_6$)$\delta$11.92 (s, 1H), 10.87 (s, 1H), 10.09 (s, 1H), 8.41 (s, 1H), 7.82 (s, 1H), 7.80 (d, $J$= 2.8 Hz, 1H), 7.66 - 7.60 (m, 2H), 3.73 (s, 3H), 3.20 (s, 3H), 3.05 (s, 3H), 1.99 - 1.93 (m, 1H), 0.64-0.60 (m, 4H).

Example **83**

6-((6-fluoropyridin-2-yl)amino)-N-methoxy-4-((2-(N-methyl methanesulfonamido)-3-(trifluoromethyl)phenyl)amino)nicotinamide

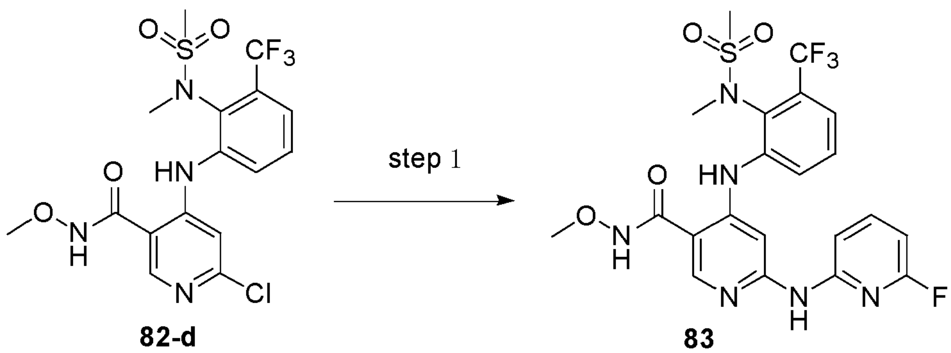


82-d

83

Step 1: 6-chloro-N-methoxy-4-((2-(N-methyl methanesulfonamido)-3-(trifluoromethyl)phenyl)amino)nicotinamide **(82-d,** 130 mg, 0.29 mmol), 2-amino-6-fluoropyridine (65 mg, 0.58 mmol), cesium carbonate (190 mg, 0.58 mmol), XantPhos(25mg, 0.04mmol) and $Pd_2(dba)_3$(27mg, 0.029 mmol) were added to anhydrous dioxane (2 ml). After the atmosphere of the mixture was evacuated to vacuum and refilled with nitrogen, the reaction mixture was heated to 125°C with stirring for 3 hours, and filtered by suction. The filtrate was concentrated and purified by high performance preparative thin layer chromatography (MeOH:DCM=1:20) to provide the title compound: 6-((6-fluoropyridin-2-yl)amino)-N-methoxy-4-((2-(N-methyl methanesulfonamido)-3-(trifluoromethyl)phenyl)amino)nicotinamide **(83,** 30 mg, 0.056mmol, 19% yield). MS Calcd: 528.48; MS Found: 529.40 ($[M+H]^+$). $^1H$NMR (400 MHz, DMSO-$d_6$)$\delta$11.86 (s, 1H), 10.09 (s, 1H), 10.07 (s, 1H), 8.40 (s, 1H), 7.94 (dd, $J$ = 7.2, 2.4 Hz, 1H), 7.84 - 7.78 (m, 1H), 7.67 - 7.61 (m, 2H), 7.49 - 7.46 (m, 2H), 6.57 (dd, $J$ = 8.0, 2.4 Hz, 1H), 3.73 (s, 3H), 3.23 (s, 3H), 3.07 (s, 3H).

Example **84**

6-((5-fluoropyridin-2-yl)amino)-N-methoxy-4-((2-(N-methyl methanesulfonamido)-3-(trifluoromethyl)phenyl)amino)nicotinamide

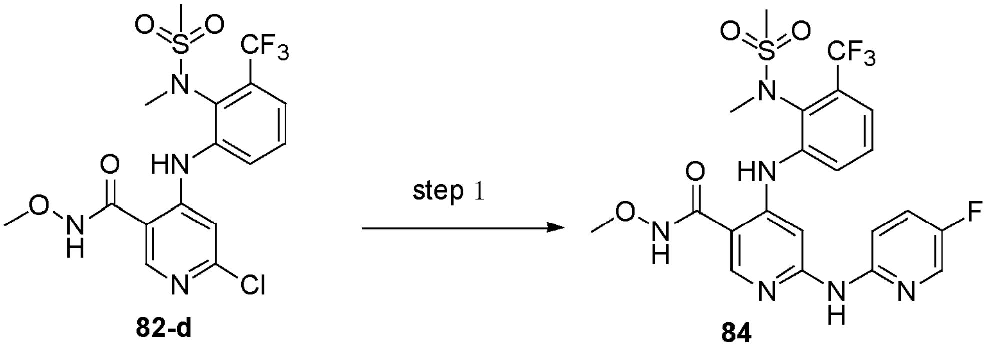


82-d 84

Step 1: 6-chloro-N-methoxy-4-((2-(N-methyl methanesulfonamido)-3-(trifluoromethyl)phenyl)amino)nicotinamide **(82-d,** 130 mg, 0.29 mmol), 2-amino-5-fluoropyridine (65 mg, 0.58 mmol), cesium carbonate (190 mg, 0.58 mmol), XantPhos(25mg, 0.04mmol) and $Pd_2(dba)_3$(27mg, 0.029 mmol) were added to anhydrous dioxane (2 ml). After the atmosphere of the mixture was evacuated to vacuum and refilled with nitrogen, the reaction mixture was heated to 125°C with stirring for 3 hours, and filtered by suction. The filtrate was concentrated and purified by high performance preparative thin layer chromatography (MeOH:DCM=1:20) to provide the title compound: 6-((5-fluoropyridin-2-yl)amino)-N-methoxy-4-((2-(N-methyl methanesulfonamido)-3-(trifluoromethyl)phenyl)amino)nicotinamide **(84,** 30 mg, 0.056mmol, 19% yield). MS Calcd: 528.48; MS Found: 529.40 ($[M+H]^+$). $^1$HNMR (400 MHz, DMSO-$d_6$)$\delta$11.83 (s, 1H), 10.09 (s, 1H), 9.89 (s, 1H), 8.38 (s, 1H), 8.14 (d, *J= 3.2 Hz,* 1H), 7.92 (dd *J* = 8.4, 1.6 Hz, 1H), 7.73 - 7.69 (m, 2H), 7.66 - 7.59 (m, 2H), 7.46 (s, 1H), 3.73 (s, 3H), 3.24 (s, 3H), 3.06 (s, 3H).

Example **85**

6-(cyclopropylcarboxamido)-4-((3-cyclopropyl-2-(N-methyl methanesulfonamido)phenyl)amino)-N-methoxy nicotinamide

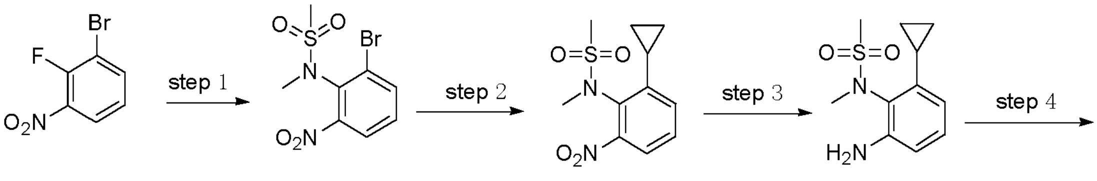


85-a 85-b 85-c 85-d

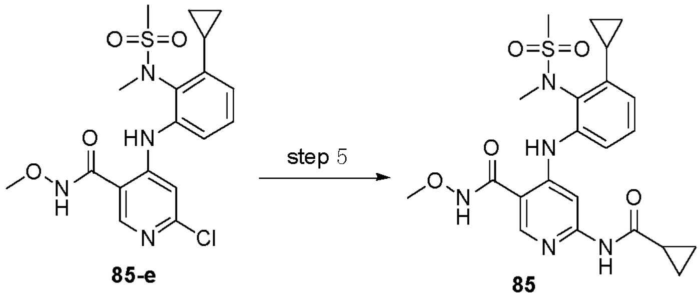


85-e 85

Step 1: To N-methyl methanesulfonamide (0.5 g, 5.5 mmol) dissolved in 20 mL of N,N-dimethyl formamide was added sodium hydride (0.29g, 12 mmol) portionwise. After that, the reaction mixture was heated to 55°C with continuous stirring for 2 hours, added with 4-bromo-2-fluoro-1-nitrobenzene **(85-a,** 0.8 g, 5.5 mmol), with continuous stirring at that temperature for 6 hours. Upon indication of completed reaction by TLC, the reaction mixture was added with water (40 mL), and extracted with ethyl acetate (30 mL x 2). The organic phase was washed with saturated brine (30 mL x 2), dried over anhydrous sodium sulfate, and concentrated under reduced pressure. The residue was separated and purified by silica gel column chromatography (EA:PE=1:5) to provide N-(2-bromo-6-nitrophenyl)-N-methyl methanesulfonamide **(85-b,** 0.9 g, 2.9 mmol, 52% yield), as a pale yellow solid. MS Calcd: 307.95; MS Found: 306.95 ($[M-H]^-$).

Step 2: N-(2-bromo-6-nitrophenyl)-N-methyl methanesulfonamide **(85-b,** 0.5 g, 1.6 mmol), cyclopropyl boronic acid (180 mg, 2.2 mmol), potassium phosphate (850 g, 4 mmol) and Pd(dppf)$Cl_2$ (0.06 g, 0.08 mmol) were sequentially

added to 8 mL of solution of dioxane/water (7/1). The atmosphere of the mixture was evacuated and replaced with nitrogen three times, and stirred at 110°C for 6 hours. Upon indication of completed reaction by TLC, the mixture was concentrated under reduced pressure. The residue was separated and purified by silica gel column chromatography (EA:PE=1:1) to provide N-(2-cyclopropyl-6-nitrophenyl)-N-methyl methanesulfonamide **(85-c,** 0.23 g, 0.85 mmol, 53% yield), as a colorless oil. MS Calcd: 270.07; MS Found: 293.19 ([M+Na]$^+$).

Step 3: N-(2-cyclopropyl-6-nitrophenyl)-N-methyl methanesulfonamide **(85-c,** 0.23 g, 0.85 mmol), ammonium chloride (0.25 g, 4.7 mmol) and iron powder (0.2 g, 3.57 mmol) were sequentially added to 10 mL of mixed solvent of water and ethanol (1:4)。The mixture was stirred under reflux for 6 hours. Upon indication of completed reaction by TLC, the reaction mixture was filtered by suction. The filtrate was concentrated, and the residue was separated and purified by silica gel column chromatography (PE:EA =1:1) to provide N-(2-amino-6-cyclopropyl phenyl)-N-methyl methanesulfonamide **(85-d,** 0.17 g, 0.7 mmol, 83% yield), as a colorless oil. MS Calcd: 240.09; MS Found: 241.17 ([M+Na]$^+$).

Step 4: To 10 ml of anhydrous N,N-dimethylacetamide were added N-(2-amino-6-cyclopropyl phenyl)-N-methyl methanesulfonamide **(85-d,** 0.17 g, 0.7 mmol) and 4,6-dichloro-N-methoxy nicotinamide **(int-1,** 160 mg, 0.72 mmol). The mixture was added at room temperature with a solution of LiHMDS in tetrahydrofuran (2.8 ml, 2.8 mmol), and stirred at room temperature for 3 hours. Upon indication of completed reaction by TLC, the mixture was adjusted with aqueous hydrochloride (1N) to pH 5, and extracted with ethyl acetate (10 ml x 3). The combined organic layers were dried, concentrated and then purified by column chromatography (PE:EA =1:1) to provide 6-chloro-4-((3-cyclopropyl-2-(N-methyl methanesulfonamido)phenyl)amino)-N-methoxy nicotinamide **(85-e,** 240 mg, 0.56 mmol, 80% yield), as a tan oil. MS Calcd: 424.10; MS Found: 425.35 ([M+H]$^+$).

Step 5: 6-chloro-4-((3-cyclopropyl-2-(N-methyl methanesulfonamido)phenyl)amino)-N-methoxy nicotinamide **(85-e,** 120 mg, 0.3mmol), cyclopropylcarboxamide (50g, 0.6mmol), cesium carbonate (190g, 0.6mmol), XantPhos(26mg, 0.045 mmol) and $Pd_2(dba)_3$(27mg, 0.03 mmol) were added to anhydrous dioxane (2 ml). After the atmosphere of the mixture was evacuated to vacuum and refilled with nitrogen, the reaction mixture was heated to 125°C with stirring for 3 hours, and filtered by suction. The filtrate was concentrated and purified by high performance preparative thin layer chromatography (MeOH:DCM=1:20) to provide the title compound: 6-(cyclopropylcarboxamido)-4-((3-cyclopropyl-2-(N-methyl methanesulfonamido)phenyl)amino)-N-methoxy nicotinamide **(85,** 20mg, 0.04mmol, 14% yield). MS Calcd: 473.17; MS Found: 474.38 ([M+H]$^+$). $^1$H NMR (400 MHz, DMSO-$d_6$)$\delta$11.82 (s, 1H), 10.78 (s, 1H), 9.98 (s, 1H), 8.36 (s, 1H), 7.87 (s, 1H), 7.32 - 7.20 (m, 2H), 6.70 (dd, J = 8.0, 1.6 Hz, 1H), 3.73 (s, 3H), 3.19 (s, 3H), 3.10 (s, 3H), 2.18 - 2.12 (m, 1H), 1.99 - 1.92 m, 1H), 1.07 - 0.97 (m, 2H), 0.83 - 0.65 (m, 6H).

Example **86**

4-((3-cyclopropyl-2-(N-methyl methanesulfonamido)phenyl)amino)-6-(6-fluoropyridin-2-yl)-N-methoxy nicotinamide

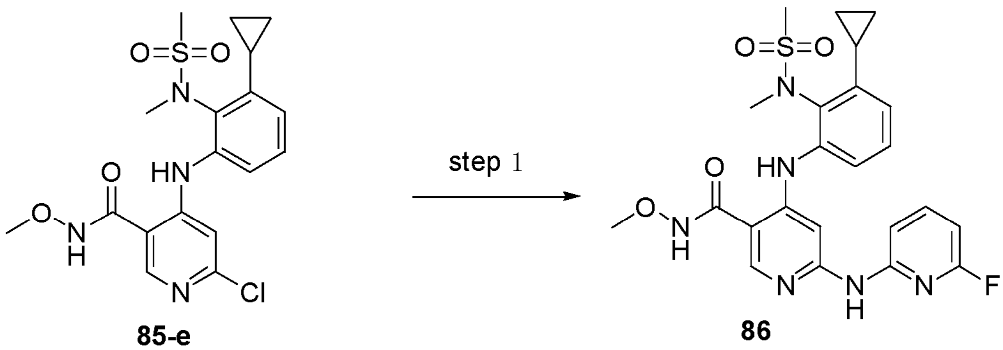


85-e

86

Step 1: 6-chloro-4-((3-cyclopropyl-2-(N-methyl methanesulfonamido)phenyl)amino)-N-methoxy nicotinamide **(85-e,** 120 mg, 0.3mmol), 2-amino-6-fluoropyridine (67g, 0.6mmol), cesium carbonate (190g, 0.6mmol), XantPhos(26mg, 0.045 mmol) and $Pd_2(dba)_3$(27mg, 0.03 mmol) were added to anhydrous dioxane (2 ml). After the atmosphere of the mixture was evacuated to vacuum and refilled with nitrogen, the reaction mixture was heated to 125°C with stirring for 3 hours, and filtered by suction. The filtrate was concentrated and purified by high performance preparative thin layer chromatography (MeOH:DCM=1:20) to provide the title compound: 4-((3-cyclopropyl-2-(N-methyl methanesulfonamido) phenyl)amino)-6-(6-fluoropyridin-2-yl)-N-methoxy nicotinamide **(86,** 20mg, 0.04mmol, 14% yield). MS Calcd: 500.16; MS Found: 499.28 ([M-H]$^-$). $^1$H NMR (400 MHz, DMSO-$d_6$)$\delta$11.82 (s, 1H), 10.02 (d, J = 5.4 Hz, 2H), 8.35 (s, 1H), 7.84 - 7.76 (m, 1H), 7.57 (s, 1H), 7.47 (d, *J* = 8.0 Hz, 1H), 7.40 (d, *J* = 7.5 Hz, 1H), 7.30 (t, *J* = 7.9 Hz, 1H), 6.70 (d, *J* = 7.4 Hz, 1H), 6.55 (d, *J* = 10.1 Hz, 1H), 3.73 (s, 3H), 3.23 (s, 3H), 3.12 (s, 3H), 2.16 (dq, *J* = 8.4, 5.3, 4.2 Hz, 1H), 1.10 - 0.95 (m, 2H), 0.89 - 0.80 (m,

1H), 0.65 (dt, *J* = 9.0, 4.5 Hz, 1H).

Example **87**

6-(cyclopropylcarboxamido)-N-methoxy-4-((3-(1-methyl-1H-pyrazol-4-yl)-2-(N-methyl methanesulfonamido)phenyl) amino)nicotinamide

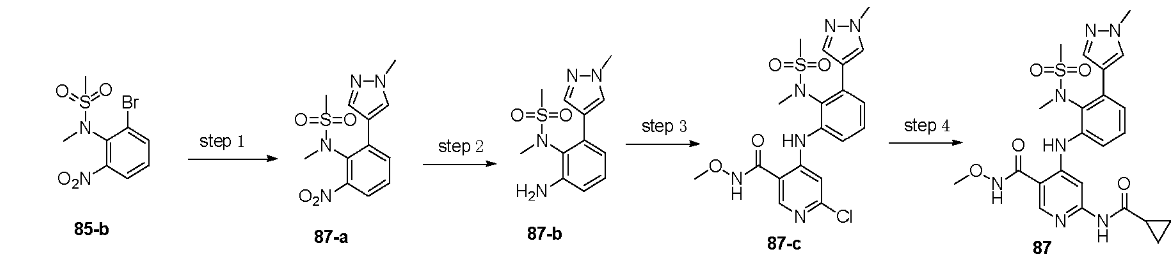


85-b 87-a 87-b 87-c 87

Step 1: N-(2-bromo-6-nitrophenyl)-N-methyl methanesulfonamide **(85-b,** 0.25 g, 0.8 mmol), 1-methyl-4-(4,4,5,5-tetramethyl-1,3,2-dioxaborolan-2-yl)-1H-pyrazole (220 mg, 1.04 mmol), potassium phosphate (424 mg, 2 mmol) and Pd(dppf)$Cl_2$ (0.03 g, 0.04 mmol) were sequentially added to 8 mL of solution of dioxane/water (7/1). The atmosphere of the mixture was evacuated to vacuum and replaced with nitrogen three times, followed by stirring at 110°C for 6 hours. Upon indication of completed reaction by TLC, the mixture was concentrated under reduced pressure. The residue was separated and purified by silica gel column chromatography (EA:PE=2:1) to provide N-methyl-N-(2-(1-methyl-1H-pyrazol-4-yl)-6-nitrophenyl)methanesulfonamide **(87-a,** 0.16 g, 0.51 mmol, 64% yield). MS Calcd: 310.07; MS Found: 311.21 ($[M+H]^+$).

Step 2: N-methyl-N-(2-(1-methyl-1H-pyrazol-4-yl)-6-nitrophenyl)methanesulfonamide **(87-a,** 0.16 g, 0.51 mmol), ammonium chloride (0.25 g, 4.7 mmol) and iron powder (0.2 g, 3.57 mmol) were sequentially added to 10 mL of mixed solvent of water and ethanol (1:4), followed by stirring under reflux for 6 hours. Upon indication of completed reaction by TLC, the reaction mixture was filtered by suction. The filtrate was concentrated, and the residue was separated and purified by silica gel column chromatography (PE:EA =1:2) to provide N-(2-amino-6-(1-methyl-1H-pyrazol-4-yl) phenyl)-N-methyl methanesulfonamide **(87-b,** 0.12 g, 0.42 mmol, 84% yield). MS Calcd: 280.10; MS Found: 281.24 ($[M+H]^+$).

Step 3: To 10 ml of anhydrous N,N-dimethylacetamide were added N-(2-amino-6-(1-methyl-1H-pyrazol-4-yl) phenyl)-N-methyl methanesulfonamide **(87-b,** 0.12 g, 0.42 mmol) and 4,6-dichloro-N-methoxy nicotinamide **(int-1,** 100 mg, 0.45 mmol). The mixture was added at room temperature with a solution of LiHMDS in tetrahydrofuran (1.72 ml, 1.72 mmol), and stirred at room temperature for 3 hours. Upon indication of completed reaction by TLC, the mixture was adjusted with aqueous hydrochloride (1N) to pH 5, and extracted with ethyl acetate (10 ml x 3). The combined organic layers were dried, concentrated and then purified by column chromatography (EA) to provide 6-chloro-N-methoxy-4-((3-(1-methyl-1H-pyrazol-4-yl)-2-(N-methyl methanesulfonamido)phenyl)amino)nicotinamide **(87-c,** 140 mg, 0.56 mmol, 80% yield). MS Calcd: 464.10; MS Found: 465.38 ($[M+H]^+$).

Step 4: 6-chloro-N-methoxy-4-((3-(1-methyl-1H-pyrazol-4-yl)-2-(N-methyl methanesulfonamido)phenyl)amino)nicotinamide **(87-c,** 70 mg, 0.15 mmol), cyclopropylcarboxamide (25 mg, 0.3 mmol), cesium carbonate (100 mg, 0.3mmol), XantPhos(26mg, 0.045 mmol) and $Pd_2(dba)_3$(27mg, 0.03 mmol) were added to anhydrous dioxane (2 ml). After the atmosphere of the mixture was evacuated to vacuum and refilled with nitrogen, the reaction mixture was heated to 125°C with stirring for 3 hours, and filtered by suction. The filtrate was concentrated and purified by high performance preparative thin layer chromatography (MeOH:DCM=1:20) to provide the title compound: 6-(cyclopropylcarboxamido)-N-methoxy-4-((3-(1-methyl-1H-pyrazol-4-yl)-2-(N-methyl methanesulfonamido)phenyl)amino)nicotinamide **(87,** 10mg, 0.019mmol, 12% yield). MS Calcd: 513.18; MS Found: 514.35 ($[M+H]^+$). $^1H$ NMR (400 MHz, DMSO-$d_6$)$\delta$11.83 (s, 1H), 10.79 (s, 1H), 10.01 (s, 1H), 8.39 (s, 1H), 8.07 (s, 1H), 7.43 - 7.37 (m, 2H), 7.33 (dd, *J* = 6.4, 3.2 Hz, 1H), 3.89 (s, 3H), 3.72 (s, 3H), 3.14 (s, 3H), 2.82 (s, 3H), 1.95 - 1.92 (m, 1H), 0.76 - 0.74 (m, 4H).

Example **88**

6-((6-fluoropyridin-2-yl)amino)-N-methoxy-4-((3-(1-methyl-1H-pyrazol-4-yl)-2-(N-methyl methanesulfonamido)phenyl) amino)nicotinamide

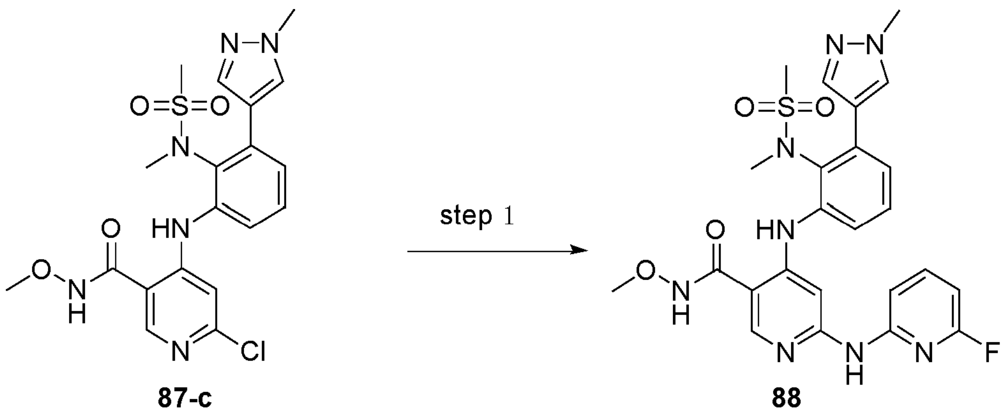


87-c 88

Step 1 6-chloro-N-methoxy-4-((3-(1-methyl-1H-pyrazol-4-yl)-2-(N-methyl methanesulfonamido)phenyl)amino) nicotinamide **(87-c,** 70 mg, 0.15 mmol), 2-amino-6-fluoropyridine (35 mg, 0.3 mmol), cesium carbonate (100 mg, 0.3mmol), XantPhos(26mg, 0.045 mmol) and $Pd_2(dba)_3$(27mg, 0.03 mmol) were added to anhydrous dioxane (2 ml). After the atmosphere of the mixture was evacuated to vacuum and refilled with nitrogen, the reaction mixture was heated to 125°C with stirring for 3 hours, and filtered by suction. The filtrate was concentrated and purified by high performance preparative thin layer chromatography (MeOH:DCM=1:20) to provide the title compound: 6-((6-fluoropyridin-2-yl)amino)-N-methoxy-4-((3-(1-methyl-1H-pyrazol-4-yl)-2-(N-methyl methanesulfonamido)phenyl)amino)nicotinamide **(88,** 10mg, 0.019mmol, 12% yield). MS Calcd: 540.17; MS Found: 541.37 ($[M+H]^+$). 1H NMR (400 MHz, DMSO-$d_6$)$\delta$11.84 (s, 1H), 10.02(s, 1), 9.99 (s, 1H), 8.38 (s, 1H), 8.06 (s, 1H), 7.83 (s, 1H), 7.79 (t, *J* = 8.0 Hz, 1H), 7.50 - 7.45 (m, 3H), 7.44 - 7.38 (m, 2H), 6.55 (dd, *J* = 8.0, 2.4 Hz, 1H), 3.89 (s, 3H), 3.73 (s, 3H), 3.18 (s, 3H), 2.86 (s, 3H).

Example **89**

6-(cyclopropylcarboxamido)-N-methoxy-4-((2-methanesulfonyl)phenyl)amino)nicotinamide

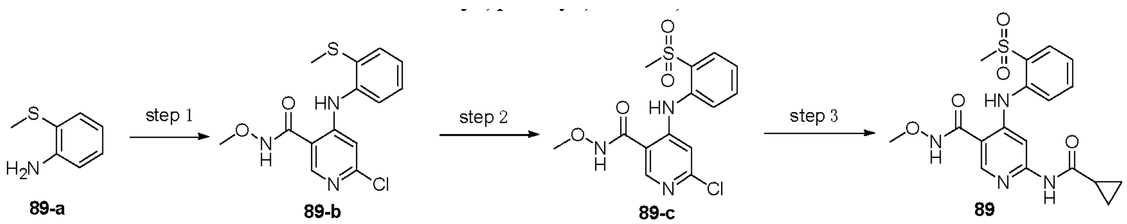


89-a 89-b 89-c 89

Step 1: To 10 ml of anhydrous tetrahydrofuran were added 2-amino thioanisole **(89-a,** 280mg, 2 mmol) and 4,6-dichloro-N-methoxy nicotinamide **(int-1,** 440 mg, 2 mmol). The mixture was added at room temperature with a solution of LiHMDS in tetrahydrofuran (8 ml, 8 mmol), and stirred at room temperature for 3 hours. The mixture was adjusted with aqueous hydrochloride (1N) to pH 5, and extracted with ethyl acetate (20 ml x 3). The combined organic layers were dried, concentrated and then purified by column chromatography (PE:EA =1:1) to provide 6-chloro-N-methoxy-4-((2-methylthio) phenyl)amino)nicotinamide (32226-b, 300 mg, 0.9 mmol, 46% yield). MS Calcd: 323.80; MS Found: 324.22 ($[M+H]^+$).

Step 2: To a mixed solution of hydrogen peroxide (2 mL, 19.5 mmol) and acetic acid (3.2 mL) was added 6-chloro-N-methoxy-4-((2-methylthio)phenyl)amino)nicotinamide **(89-b,** 300 mg, 0.9 mmol), and sodium tungstate (321 mg, 0.97 mmol) was added portionwise, followed by stirring at room temperature for 30 minutes. Upon indication of completed reaction by TLC, the reaction mixture was added with water (20 mL) and ethyl acetate (20 mL). The organic phase was separated, washed with sodium thiosulfate solution (20 mLx3), dried over anhydrous sodium sulfate, and concentrated under reduced pressure. The residue was separated and purified by silica gel column chromatography (EA) to provide 6-chloro-N-methoxy-4-((2-methanesulfonyl)phenyl)amino)nicotinamide **(89-c,** 206 mg, 0.58 mmol, 64% yield). MS Calcd: 355.79; MS Found: 356.17 ($[M+H]^+$).

Step 3: 6-chloro-N-methoxy-4-((2-methanesulfonyl)phenyl)amino)nicotinamide **(89-c,** 106 mg, 0.3 mmol), cyclopropylcarboxamide (50mg, 0.60 mmol), cesium carbonate (195mg, 0.6mmol), Xantphos(26mg, 0.045 mmol) and $Pd_2(dba)_3$(27 mg, 0.03 mmol) were added to anhydrous dioxane (2ml). After the atmosphere of the mixture was evacuated to vacuum and refilled with nitrogen, the reaction mixture was heated to 125°C with stirring for 3 hours, and filtered by suction. The filtrate was concentrated and purified by high performance preparative thin layer chromatography

(MeOH:DCM=1:10) to provide the title compound: 6-(cyclopropylcarboxamido)-N-methoxy-4-((2-methanesulfonyl)phenyl)amino)nicotinamide **(89,** 30 mg, 0.07mmol, 25% yield). MS Calcd: 404.44; MS Found: 405.28 ([M+H]$^+$). $^1$H NMR (400 MHz, DMSO-$d_6$)$\delta$11.86 (s, 1H), 10.88 (s, 1H), 10.34 (s, 1H), 8.43 (s, 1H), 7.98 (s, 1H), 7.93 (dd, *J* = 8.0, 1.6 Hz, 1H), 7.75 - 7.70 (m, 1H), 7.65 (dd, *J* = 8.0, 1.2 Hz, 1H), 7.41 - 7.36 (m, 1H), 3.72 (s, 3H), 3.15 (s, 3H), 2.00 - 1.91 (m, 1H), 0.79 - 0.73 (m, 4H).

Example **90**

6-(cyclopropylcarboxamido)-N-ethoxy-4-((2-(N-methyl methanesulfonamido)phenyl)amino)nicotinamide

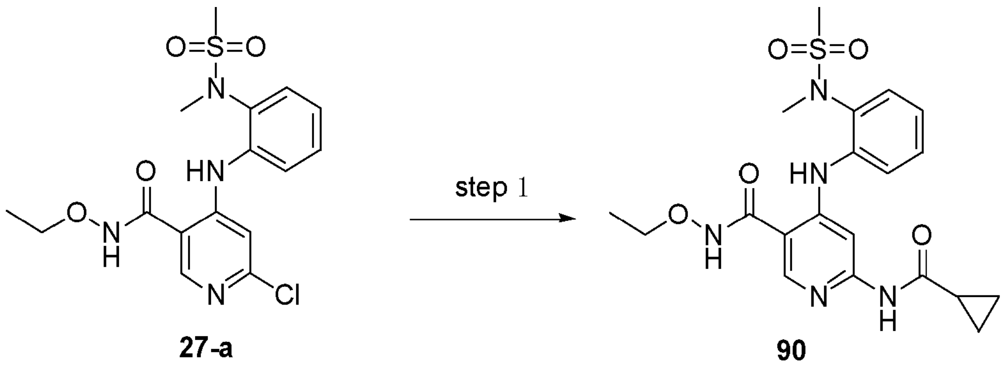


**27-a** **90**

Step 1: 6-chloro-N-ethoxy-4-((2-(N-methyl methanesulfonamido)phenyl)amino)nicotinamide **(27-a,** 80mg, 0.20mmol), cyclopropylcarboxamide (22mg, 0.26mmol), cesium carbonate (127mg, 0.39mmol), XantPhos(30mg, 0.04mmol) and $Pd_2(dba)_3$(24mg, 0.026mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and purified by high performance preparative thin layer chromatography (MeOH:DCM=1:20) to provide the title compound: 6-(cyclopropylcarboxamido)-N-ethoxy-4-((2-(N-methyl methanesulfonamido)phenyl)amino)nicotinamide **(90,** 40mg, 0.089mmol, 44% yield). MS Calcd: 447; MS Found: 448 ([M+H]$^+$). $^1$H NMR (400 MHz, DMSO-$d_6$)$\delta$11.68 (s, 1H), 10.77 (s, 1H), 10.01 (s, 1H), 8.35 (s, 1H), 7.94 (s, 1H), 7.56 (d, *J* = 8.0, 1H), 7.47 (d, *J* = 8.0 Hz, 1H), 7.39 (t, *J* = 8.0 Hz, 1H), 7.21 (t, *J* = 8.0 Hz, 1H), 3.94 (q, *J* = 7.02 Hz, 2H), 3.14 (s, 3H), 3.10 (s, 3H), 1.98-1.95 (m, 1H), 1.23 (t, *J* = 7.2 Hz, 3H), 0.78-0.73 (m, 2H), 0.64-0.60 (m, 2H).

Example **91**

N-(t-butoxy)-6-(cyclopropylcarboxamido)-4-((2-(N-methyl methanesulfonamido)phenyl)amino)nicotinamide

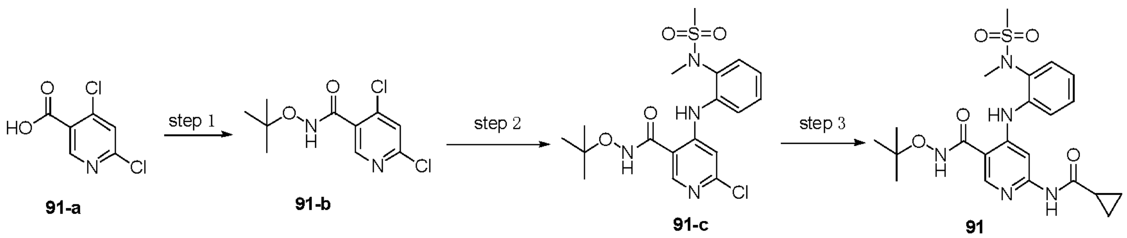


**91-a** **91-b** **91-c** **91**

Step 1:1) To 4,6-dichloronicotinic acid **(91-a,** 500mg, 2.65mmol) dissolved in DCM (5mL) was slowly added oxalyl chloride (401mg, 2.86mmol) dropwise. The reaction was allowed to proceed at room temperature for 3h, and concentrated to provide a crude material, which was used directly in the next reaction. 2) 4,6-dichloronicotinyl chloride (2.65mmol) was added into ethyl acetate (5ml), to which were added t-butoxyamine (255mg, 2.86mmol) and potassium carbonate (719mg, 5.21mmol) dissolved in purified water (1ml). The reaction mixture was stirred at room temperature for 2h, and then concentrated together with silica gel. The residue was purified by column chromatography (petroleum ether:ethyl acetate=1:1) to provide N-(t-butoxy)-4,6-dichloro nicotinamide **(91-b,** 650mg, 2.46mmol, 93% yield). MS Calcd: 262; MS Found: 263 ([M+H]$^+$).

Step 2: To 5ml of anhydrous tetrahydrofuran were added N-(t-butoxy)-4,6-dichloro nicotinamide **(91-b,** 263mg, 1.00mmol), N-(2-amino phenyl)-N-methyl methanesulfonamide (200mg, 1.00mmol). The mixture was added at room temperature with a solution of LiHMDS in tetrahydrofuran (2ml, 2.00mmol), and stirred at room temperature for 2 hours. The mixture was adjusted with aqueous hydrochloride (1N) to pH 5, and extracted with ethyl acetate (10ml). The combined organic layers were dried, concentrated and then purified by column chromatography (petroleum ether:ethyl acetate =1:1) to provide N-(t-butoxy)-6-chloro-4-((2-(N-methyl methanesulfonamido)phenyl)amino)nicotinamide **(91-c,** 50mg, 0.12mmol, 12% yield). MS Calcd: 426; MS Found: 427 ($[M+H]^+$).

Step 3: N-(t-butoxy)-6-chloro-4-((2-(N-methyl methanesulfonamido)phenyl)amino)nicotinamide **(91-c,** 50mg, 0.12mmol), cyclopropylcarboxamide (22mg, 0.26mmol), cesium carbonate (127mg, 0.39mmol), XantPhos(30mg, 0.04mmol) and $Pd_2(dba)_3$(24mg, 0.026mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and purified by high performance preparative thin layer chromatography (MeOH:DCM=1:20) to provide the title compound: N-(t-butoxy)-6-(cyclopropylcarboxamido)-4-((2-(N-methyl methanesulfonamido)phenyl)amino)nicotinamide **(91,** 6mg, 0.013mmol, 11% yield). MS Calcd: 475; MS Found: 476 ($[M+H]^+$). $^1$H NMR (400 MHz, DMSO-$d_6$)δ11.04 (s, 1H), 10.75 (s, 1H), 9.87 (s, 1H), 8.41 (s, 1H), 7.94 (s, 1H), 7.55 (dd, *J* = 8.0, 1.6 Hz, 1H), 7.48 (dd, *J* = 8.0, 1.6 Hz, 1H), 7.41-7.37 (m, 1H), 7.22-7.18 (m, 1H), 3.13 (s, 3H), 3.09 (s, 3H), 2.00-1.96 (m, 1H), 1.25 (s, 9H), 0.77-0.74 (m, 2H), 0.66-0.59 (m, 2H).

Example **92**

N-methoxy-4-((2-(N-methyl methanesulfonamido)phenyl)amino)-6-((5-(trifluoromethyl)pyridin-2-yl)amino)nicotinamide

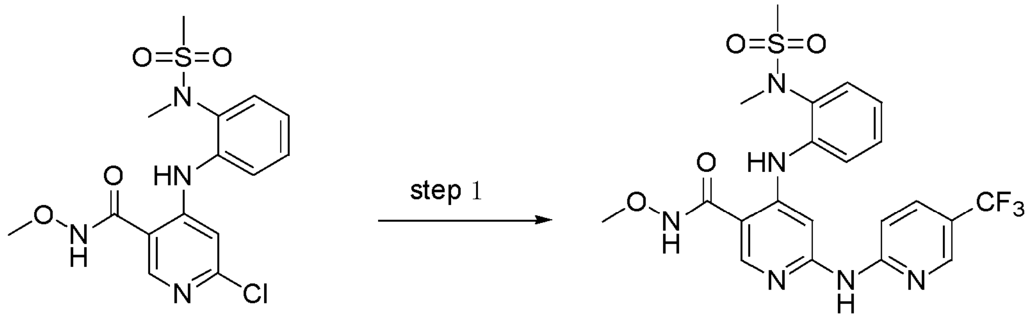


**2-d** **92**

Step 1: 6-chloro-N-methoxy-4-((2-(N-methyl methanesulfonamido)phenyl)amino)nicotinamide **(2-d,** 120°Cg, 0.31mmol), 5-(trifluoromethyl)pyridin-2-ylamine (81mg, 0.50mmol), cesium carbonate (326mg, 1.00mmol), XantPhos(28.56mg, 0.06mmol) and $Pd_2(dba)_3$(27.45mg, 0.03mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and subjected to high performance preparative thin layer chromatography to provide N-methoxy-4-((2-(N-methyl methanesulfonamido)phenyl)amino)-6-((5-(trifluoromethyl)pyridin-2-yl)amino)nicotinamide **(92,** 20mg, 0.039mmol, 13% yield). MS Calcd: 510; MS Found: 511 ($[M+H]^+$). $^1$H NMR (400 MHz, DMSO-$d_6$)δ11.63 (br, 1H), 10.25 (s, 1H), 10.11 (s, 1H), 8.48 (d, *J* = 2.4 Hz, 1H), 8.37 (s, 1H), 7.99 (dd, *J* = 8.8, 2.4 Hz, 1H), 7.82 (d, *J* = 8.8 Hz, 1H), 7.73 (s, 1H), 7.63 (d, *J* = 8.0 Hz, 1H), 7.57 (d, *J* = 8.0 Hz, 1H), 7.49 (t, *J* = 8.0 Hz, 1H), 7.22 (t, *J* = 8.0 Hz, 1H), 3.73 (s, 3H), 3.17 (s, 3H), 3.13 (s, 3H).

Example **93**

6-(cyclopropylcarboxamido)-4-((2-(dimethyl phosphoryl)phenyl)amino)nicotinamide

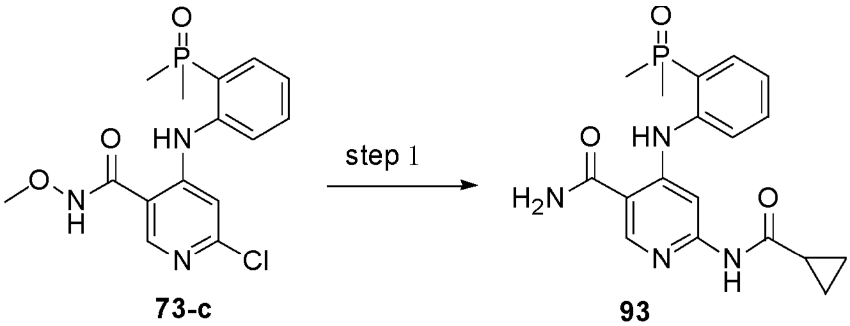


73-c

93

Step 3: 6-chloro-4-((2-(dimethyl phosphoryl)phenyl)amino)-N-methoxy nicotinamide **(73-c,** 40 mg, 0.11 mmol), cyclopropylcarboxamide (22 mg, 0.26mmol), cesium carbonate (150 mg, 0.46mmol), Xantphos(25 mg, 0.04mmol) and $Pd_2(dba)_3$(27mg, 0.03 mmol) were added to anhydrous dioxane (2ml). After the atmosphere of the mixture was evacuated to vacuum and refilled with nitrogen, the reaction mixture was heated to 140°C with stirring for 8 hours, and filtered by suction. The filtrate was concentrated, and purified high performance preparative thin layer chromatography (MeOH:DCM=1:20) to provide the title compound: 6-(cyclopropylcarboxamido)-4-((2-(dimethyl phosphoryl)phenyl)amino)nicotinamide **(93,** 10mg, 0.026mmol, 24% yield). MS Calcd: 372.36; MS Found: 373.32 ($[M+H]^+$).$^1$H NMR (400 MHz, DMSO-$d_6$)$\delta$10.73 (s, 1H), 10.68 (s, 1H), 8.61 (s, 1H), 8.15 (s, 1H), 7.90 - 7.83 (m, 1H), 7.65 (s, 1H), 7.60 (t, *J* = 7.6 Hz, 1H), 7.49 (s, 1H), 7.44 (s, 1H), 7.38 (d, *J* = 7.6 Hz, 1H), 1.97 - 1.92 (m, 1H), 1.65 (s, 3H), 1.62 (s, 3H), 0.78-0.74 (m, 4H).

Example **94**

(*S*)-4-((4-cyclopropyl-2-(N-methyl methanesulfonamido)phenyl)amino)-6-(2, 2-dimethyl cyclopropyl-1-carboxamido)-N-methoxy nicotinamide

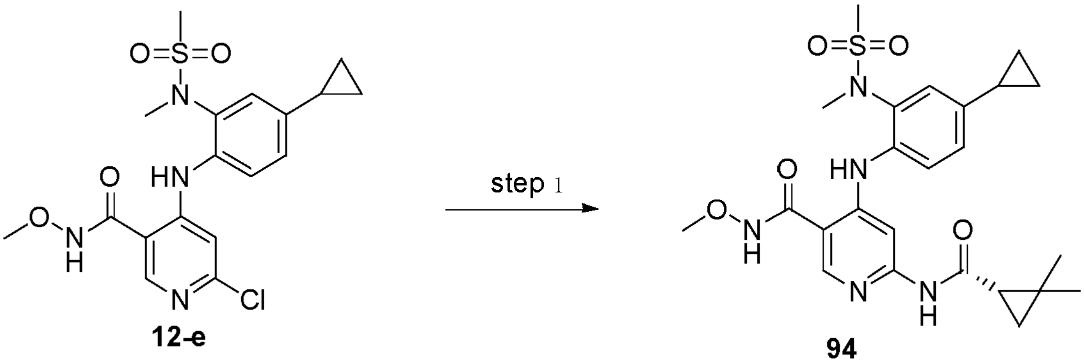


12-e

94

Step 1: 6-chloro-4-((4-cyclopropyl-2-(N-methyl methanesulfonamido)phenyl)amino)-N-methoxy nicotinamide **(12-e,** 157mg, 0.33mmol), (S)-2, 2-dimethyl cyclopropyl-1-carboxamide (35mg, 0.33mmol), cesium carbonate (326mg, 1.00mmol), XantPhos(28.56mg, 0.06mmol) and $Pd_2(dba)_3$(27.45mg, 0.03mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and subjected to high performance thin layer preparative chromatography to provide the title compound: (S)-4-((4-cyclopropyl-2-(N-methyl methanesulfonamido) phenyl)amino)-6-(2, 2-dimethyl cyclopropyl-1-carboxamido)-N-methoxy nicotinamide **(94,** 15mg, 0.030mmol, 10% yield). MS Calcd: 501; MS Found: 502 ($[M+H]^+$). $^1$H NMR (400 MHz, DMSO-*$d_6$*)$\delta$11.75 (s, 1H), 10.57 (s, 1H), 9.92 (s, 1H), 8.30 (s, 1H), 7.84 (s, 1H), 7.35 (d, *J* = 8.4 Hz, 1H), 7.26 (d, *J* = 2.0 Hz, 1H), 7.10 (dd, *J* = 8.4, 2.0 Hz, 1H), 3.71 (s, 3H), 3.12 (s, 3H), 3.08 (s, 3H), 2.02-1.93 (m, 1H), 1.84 (dd, *J* = 8.0, 1.6 Hz, 1H), 1.11 (s, 3H), 1.07 (s, 3H), 0.98 - 0.92 (m, 3H), 0.77 - 0.71 (m, 3H).

Example **95**

4-((4-cyclopropyl-2-(N-methyl methanesulfonamido)phenyl)amino)-N-ethoxy-6-((5-fluoropyridin-2-yl)amino)nicotinamide

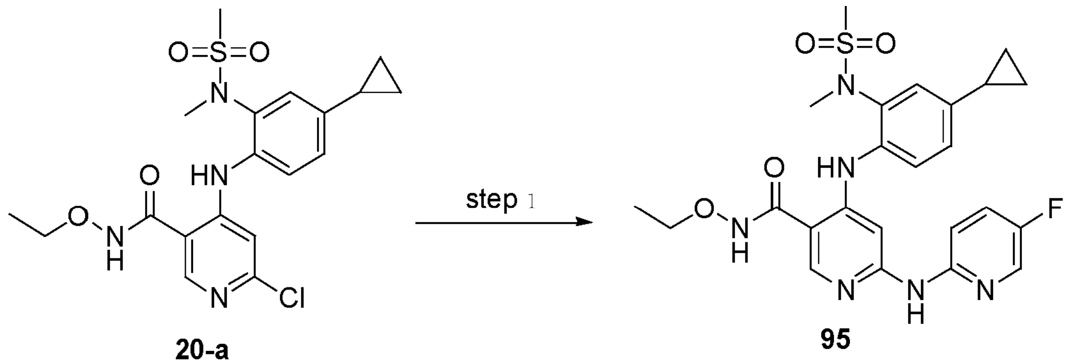


**20-a** **95**

Step 1: 6-chloro-4-((4-cyclopropyl-2-(N-methyl methanesulfonamido)phenyl)amino)-N-ethoxy nicotinamide **(20-a,** 153.65mg, 0.35mmol), 2-amino-5-fluoropyridine (39.2mg, 0.35mmol), cesium carbonate (228.2mg, 0.70mmol), XantPhos(28.56mg, 0.06mmol) and $Pd_2(dba)_3$(27.45mg, 0.03mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and purified by high performance preparative thin layer chromatography to provide the title compound: 4-((4-cyclopropyl-2-(N-methyl methanesulfonamido)phenyl) amino)-N-ethoxy-6-((5-fluoropyridin-2-yl)amino)nicotinamide **(95,** 22mg, 0.037mmol, 13% yield). MS Calcd: 514; MS Found: 515 ($[M+H]^+$). $^1H$ NMR (400 MHz, DMSO-$d_6$)δ11.53 (s, 1H), 9.87 (s, 1H), 9.75 (s, 1H), 8.28 (s, 1H), 8.13 (d, *J* = 2.8 Hz, 1H), 7.71-7.59 (m, 2H), 7.45 (d, *J* = 9.2 Hz, 2H), 7.26 (d, *J* = 2.4 Hz, 1H), 7.16 (dd, *J* = 8.8, 2.0 Hz, 1H), 3.93 (q, *J* = 7.2 Hz, 2H), 3.13 (s, 3H), 3.09 (s, 3H), 2.00-1.95 (m, 1H), 1.21 (t, *J* = 7.2 Hz, 3H), 1.00-0.96 (m, 2H), 0.74-0.72 m, 2H).

Example **96**

4-((4-cyclopropyl-5-fluoro-2-(N-methyl methanesulfonamido)phenyl)amino)-N-ethoxy-6-(pyridin-2-ylamino)nicotinamide

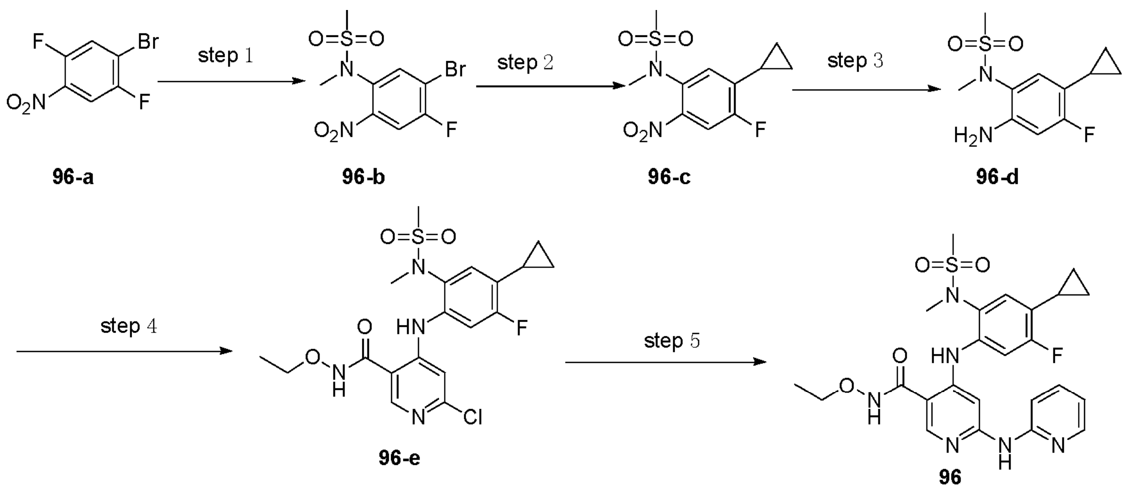


**96-a** **96-b** **96-c** **96-d** **96-e** **96**

Step 1: 1-bromo-2,5-difluoro-4-nitrobenzene**(96-a,** 1185mg, 5.00mmol), N-methyl methanesulfonamide (545mg, 5.00mmol) and potassium carbonate (1380mg, 5.00mmol) were added to 50ml of anhydrous acetonitrile and stirred at 90°C for refluxing for 2 hours. The reaction mixture was subjected to rotary evaporation under reduced pressure to dryness. The residue was added with 50ml of water, and extracted with ethyl acetate (30 ml x 3). The combined organic layers were dried and concentrated to provide N-(5-bromo-4-fluoro-2-nitrophenyl)-N-methyl methanesulfonamide **(96-b,** 1532.30mg, 4.70mmol, 94% yield). MS Calcd: 326; MS Found: 327 ($[M+H]^+$).

Step 2: N-(5-bromo-4-fluoro-2-nitrophenyl)-N-methyl methanesulfonamide **(96-b,** 1532.30mg, 4.70mmol), cyclopropyl boronic acid (435mg, 5mmol), cesium carbonate (3260mg, 10.00mmol), and Pd(dppf)$Cl_2$(457.5mg, 0.5mmol) were added to anhydrous dioxane (20ml) and water (5ml). After the atmosphere of the mixture was evacuated to vacuum and refilled with nitrogen, the reaction mixture was heated to 100 °C with stirring for 2 hours. The reaction mixture was added with 20ml of water, extracted with EA (10ml*3), and separated and purified by silica gel column chromatography (PE:EA=2:1) to provide the product N-(5-cyclopropyl-4-fluoro-2-nitrophenyl)-N-methyl methanesulfonamide **(96-c,** 120°C.63mg, 4.2mmol, 89.3% yield). MS Calcd: 288; MS Found: 289 ($[M+H]^+$).

Step 3: To 20ml of methanol was added N-(5-cyclopropyl-4-fluoro-2-nitrophenyl)-N-methyl methanesulfonamide **(96-c,** 120°C.63mg, 4.2mmol), followed by 100mg Pd/C. The reaction was allowed to proceed at room temperature for 2h. The mixture was filtered to remove Pd/C, and subjected to rotary evaporation to remove methanol to provide the product N-(2-amino-5-cyclopropyl-4-fluorophenyl)-N-methyl methanesulfonamide **(96-d,** 1032.20mg, 4.0mmol, 95.2% yield). MS Calcd: 258; MS Found: 259 ($[M+H]^+$).

Step 4: N-(2-amino-5-cyclopropyl phenyl)-N-methyl methanesulfonamide **(96-d,** 946mg, 4mmol) and N-(2-amino-5-cyclopropyl-4-fluorophenyl)-N-methyl methanesulfonamide (35109-d, 1032.20mg, 4.0mmol) were added to 20ml of anhydrous tetrahydrofuran. The mixture was added at room temperature with a solution of LiHMDS in tetrahydrofuran (10ml, 10.00mmol), and stirred at room temperature for 2 hours. The mixture was adjusted with aqueous hydrochloride (1N) to pH 5, and extracted with ethyl acetate (10ml x 3). The combined organic layers were dried, concentrated and then purified by column chromatography (PE:EA =1:1) to provide 6-chloro-4-((4-cyclopropyl-5-fluoro-2-(N-methyl methanesulfonamido)phenyl)amino)-N-ethoxy nicotinamide **(96-e,** 1007.70mg, 2.21mmol, 55.25% yield). MS Calcd: 456; MS Found: 457 ($[M+H]^+$).

Step 5: 6-chloro-4-((4-cyclopropyl-5-fluoro-2-(N-methyl methanesulfonamido)phenyl)amino)-N-ethoxy nicotinamide **(96-e,** 278mg, 0.5mmol), pyridin-2-ylamine (47mg, 0.5mmol), cesium carbonate (326mg, 1.0mmol), XantPhos(57.6mg, 0.1mmol) and $Pd_2(dba)_3$(45.5mg, 0.05mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and subjected to high performance preparative thin layer chromatography to provide the title compound: 4-((4-cyclopropyl-5-fluoro-2-(N-methyl methanesulfonamido) phenyl)amino)-N-ethoxy-6-(pyridin-2-ylamino)nicotinamide **(96,** 35mg, 0.068mmol, 13.6% yield). MS Calcd: 514; MS Found: 515 ($[M+H]^+$). $^1$H NMR (400 MHz, DMSO-$d_6$)δ11.60 (s, 1H), 10.14 (s, 1H), 9.83 (s, 1H), 8.33 (s, 1H), 8.19 - 8.14 (m, 1H), 7.90 (s, 1H), 7.68-7.63 (m, 1H), 7.55 (d, *J* = 8.0 Hz, 1H), 7.45 (d, *J* = 12.4 Hz, 1H), 7.15 (d, *J* = 8.0 Hz, 1H), 6.92-6.89 (m, 1H), 3.93 (q, *J* = 7.2 Hz, 2H), 3.14 (s, 3H), 3.10 (s, 3H), 2.07 - 1.96 (m, 1H), 1.23 (t, *J*= 7.2 Hz, 3H), 1.00 - 0.97 (m, 2H), 0.80 - 0.77 (m, 2H).

Example **97**

6-(cyclopropylcarboxamido)-4-((4-cyclopropyl-5-fluoro-2-(N-methyl methanesulfonamido)phenyl)amino)-N-ethoxy nicotinamide

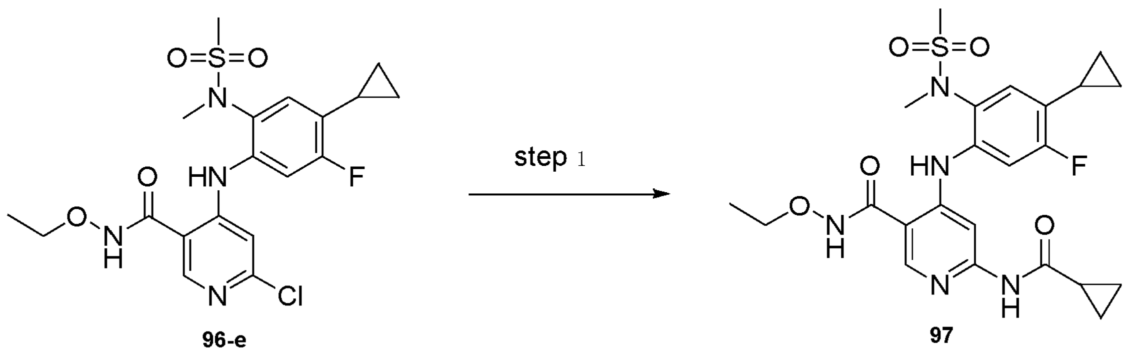


96-e 97

Step 1: 6-chloro-4-((4-cyclopropyl-5-fluoro-2-(N-methyl methanesulfonamido)phenyl)amino)-N-ethoxy nicotinamide **(96-e,** 278mg, 0.5mmol), cyclopropylcarboxamide (43mg, 0.5mmol), cesium carbonate (326mg, 1.0mmol), XantPhos(57.6mg, 0.1mmol) and $Pd_2(dba)_3$(45.5mg, 0.05mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and subjected to high performance thin layer preparative chromatography to provide the title compound: 6-(cyclopropylcarboxamido)-4-((4-cyclopropyl-5-fluoro-2-(N-methyl methanesulfonamido)phenyl)amino)-N-ethoxy nicotinamide **(97,** 25mg, 0.050mmol, 10.0% yield). MS Calcd: 505;

MS Found: 506 ([M+H]+). $^1$H NMR (400 MHz, DMSO-$d_6$)$\delta$11.69 (s, 1H), 10.82 (s, 1H), 10.03 (s, 1H), 8.35 (s, 1H), 7.96 (s, 1H), 7.26 (d, $J$ = 12.0 Hz, 1H), 7.16 (d, $J$ = 8.0 Hz, 1H), 3.93 (q, $J$ = 7.2 Hz, 2H), 3.11 (s, 3H), 3.06 (s, 3H), 2.05- 1.96 (m, 2H), 1.21 (t, $J$ = 7.2 Hz, 3H), 0.99-0.96 (m, 2H), 0.82 - 0.76 (m, 6H).

Example **98**

4-((4-cyclopropyl-5-fluoro-2-(N-methyl methanesulfonamido)phenyl)amino)-6-(((2,6-dimethyl pyrimidin-4-yl)amino)-N-ethoxy nicotinamide

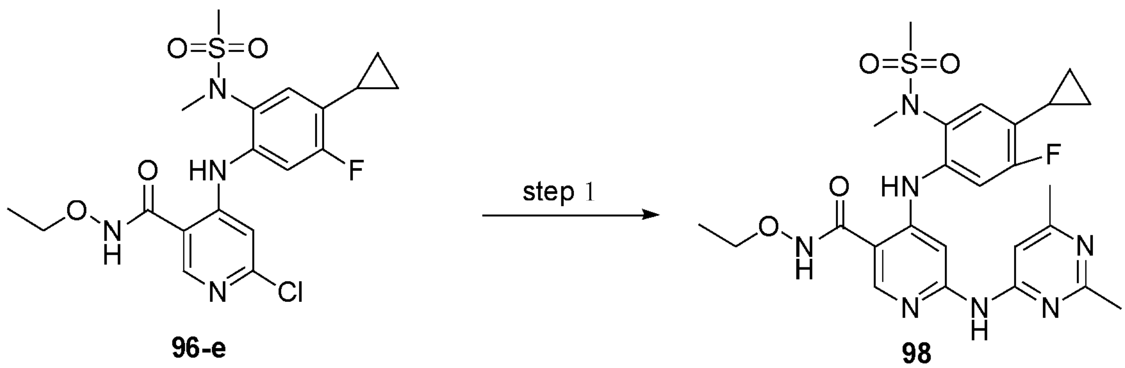


**96-e** **98**

Step 1: 6-chloro-4-((4-cyclopropyl-5-fluoro-2-(N-methyl methanesulfonamido)phenyl)amino)-N-ethoxy nicotinamide **(96-e,** 278mg, 0.5mmol), 2,6-dimethyl pyrimidin-4-ylamine (62mg, 0.5mmol), cesium carbonate (326mg, 1.0mmol), XantPhos(57.6mg, 0.1mmol) and $Pd_2(dba)_3$(45.5mg, 0.05mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and purified by high performance preparative thin layer chromatography to provide the title compound: 4-((4-cyclopropyl-5-fluoro-2-(N-methyl methanesulfonamido)phenyl)amino)-6-(((2,6-dimethyl pyrimidin-4-yl)amino)-N-ethoxy nicotinamide **(98,** 20mg, 0.037mmol, 7.36% yield). MS Calcd: 543; MS Found: 544 ([M+H]+). $^1$H NMR (400 MHz, DMSO-$d_6$)$\delta$11.80 (s, 1H), 11.19 (s, 1H), 10.11 (s, 1H), 8.43 (s, 1H), 7.54 (br, 2H), 7.38 (d, $J$ = 12.0 Hz, 1H), 7.22 (d, $J$ = 8.0 Hz, 1H), 3.95 (q, $J$ = 7.2 Hz, 2H), 3.13 (s, 3H), 3.08 (s, 3H), 2.53 (s, 6H), 2.09 - 1.98 (m, 1H), 1.23 (t, $J$ = 7.2 Hz, 3H), 1.05 - 0.95 (m, 2H), 0.82-0.78 (m, 2H).

Example **99**

4-((4-cyclopropyl-5-fluoro-2-(N-methyl methanesulfonamido)phenyl)amino)-N-ethoxy-6-((5-fluoro-4-methyl pyridin-2-yl)amino)nicotinamide

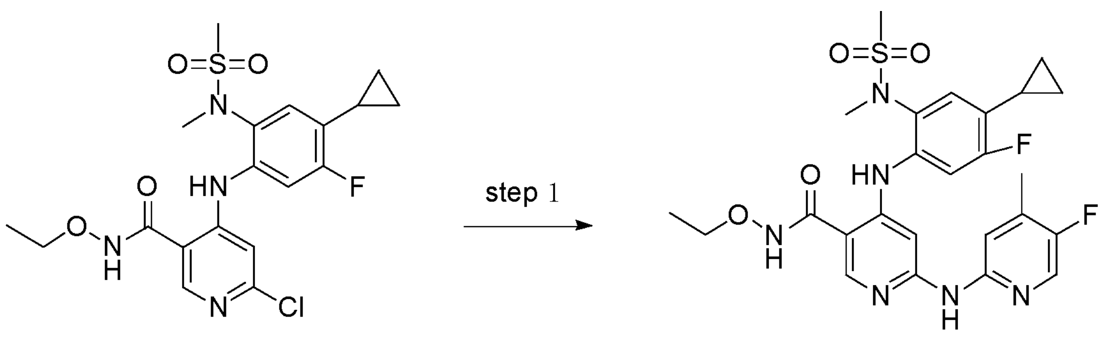


**96-e** **99**

Step 1: 6-chloro-4-((4-cyclopropyl-5-fluoro-2-(N-methyl methanesulfonamido)phenyl)amino)-N-ethoxy nicotinamide **(96-e,** 278mg, 0.5mmol), 5-fluoro-4-methyl-2-aminopyridine (63mg, 0.5mmol), cesium carbonate (326mg, 1.0mmol), XantPhos(57.6mg, 0.1mmol) and $Pd_2(dba)_3$(45.5mg, 0.05mmol) were added to anhydrous dioxane (2ml). After the atmosphere of the mixture was evacuated to vacuum and refilled with nitrogen, the reaction mixture was heated to

120°C with stirring for 2 hours, and filtered by suction. The filtrate was concentrated and purified by high performance preparative thin layer chromatography to provide the title compound: 4-((4-cyclopropyl-5-fluoro-2-(N-methyl methanesulfonamido)phenyl)amino)-N-ethoxy-6-((5-fluoro-4-methyl pyridin-2-yl)amino)nicotinamide **(99,** 45mg, 0.085mmol, 16.5% yield). MS Calcd: 546; MS Found: 547 ($[M+H]^+$). $^1$H NMR (400 MHz, DMSO-$d_6$)$\delta$11.94 (s, 1H), 10.79 (s, 1H), 10.14 (s, 1H), 8.32 (s, 1H), 8.16 (s, 1H), 7.46 (d, *J* = 12.0 Hz, 1H), 7.26 (d, *J*= 8.0 Hz, 1H), 7.17 (s, 1H), 6.89 (s, 1H), 3.95 (q, *J* = 7.2 Hz, 2H), 3.15 (s, 3H), 3.07 (s, 3H), 2.30 (s, 3H), 2.10-2.03 (m, 1H), 1.23 (t, *J* = 7.2 Hz, 3H), 1.05 - 1.01 (m, 2H), 0.85 - 0.83 (m, 2H).

Example **100**

6-((5-fluoropyridin-2-yl)amino)-N-methyl-4-((2-(N-methyl methanesulfonamido)phenyl)amino)nicotinamide

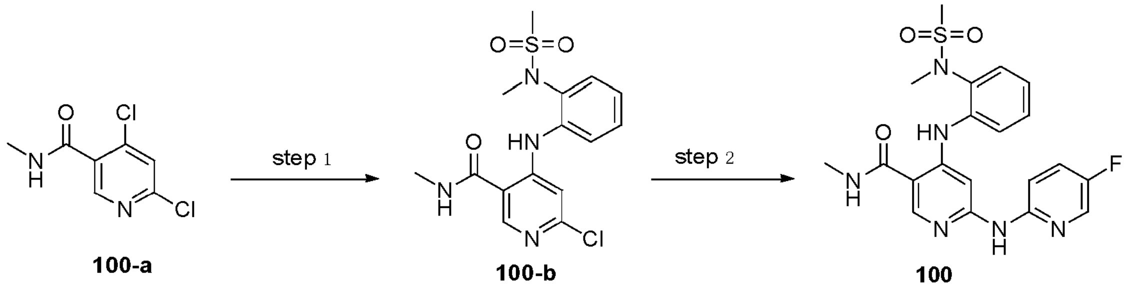


**100-a** **100-b** **100**

Step 1: To 5ml of anhydrous tetrahydrofuran were added N-(2-amino phenyl)-N-methyl methanesulfonamide (101mg, 0.50mmol) and 4,6-dichloro-N-methyl nicotinamide **(100-a,** 102.0mg, 0.5mmol). The mixture was added at room temperature with a solution of LiHMDS in tetrahydrofuran (2ml, 2.00mmol), and stirred at room temperature for 2 hours. The mixture was adjusted with aqueous hydrochloride (1N) to pH 5, and extracted with ethyl acetate (10ml x 3). The combined organic layers were dried, concentrated and then purified by column chromatography (PE:EA = 1:1) to provide 6-chloro-N-methyl-4-((2-(N-methyl methanesulfonamido)phenyl)amino)nicotinamide (**100-b,** 98.12mg, 0.27mmol, 54% yield). MS Calcd: 368; MS Found: 369 ($[M+H]^+$).

Step 2: 6-chloro-N-methyl-4-((2-(N-methyl methanesulfonamido)phenyl)amino)nicotinamide (**100-b,** 98.12mg, 0.27mmol), 5-fluoro-2-aminopyridine (33.60mg, 0.30mmol), cesium carbonate (326mg, 1.00mmol), XantPhos(28.56mg, 0.06mmol) and $Pd_2(dba)_3$(27.45mg, 0.03mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated, and purified by high performance preparative thin layer chromatography to provide the title compound: 6-((5-fluoropyridin-2-yl)amino)-N-methyl-4-((2-(N-methyl methanesulfonamido)phenyl)amino)nicotinamide **(100,** 30mg, 0.065mmol, 21.74% yield). MS Calcd: 460; MS Found: 461 ($[M+H]^+$). $^1$H NMR (400 MHz, DMSO-$d_6$)$\delta$10.57 (s, 1H), 9.79 (s, 1H), 8.51 - 8.40 (m, 2H), 8.35 (s, 1H), 8.14 (d, *J* = 3.2 Hz, 1H), 7.71 - 7.60 (m, 3H), 7.54 - 7.42 (m, 2H), 7.19 - 7.15 (m, 1H), 3.15 (s, 3H), 2.14 (s, 3H), 2.54 (s, 3H).

Example **101**

N-methoxy-6-(((5-methoxy pyridin-2-yl)amino)-4-((2-(N-methyl methanesulfonamido)phenyl)amino)nicotinamide

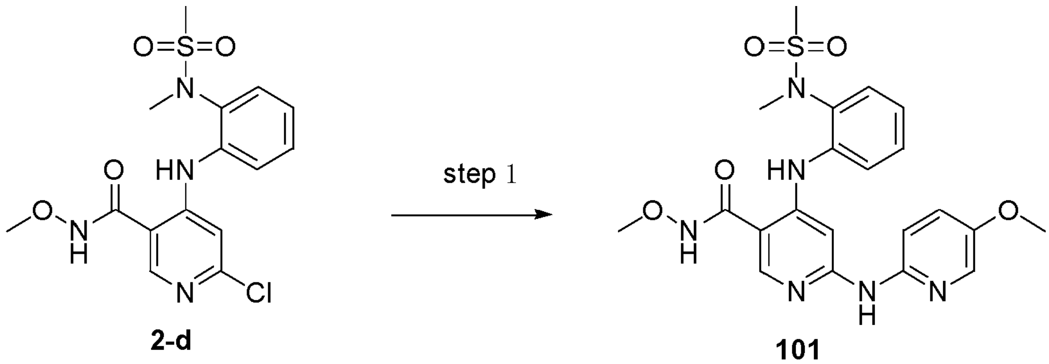


2-d 101

Step 1: To a 50ml flask were added 6-chloro-N-methoxy-4-((2-(N-methyl methanesulfonamido)phenyl)amino) nicotinamide **(2-d,** 150mg, 0.39mmol), 5-methoxy pyridin-2-ylamine (96.9mg, 0.78mmol), XantPhos (113mg, 0.2mmol), 1,4-dioxane (3ml), and $Cs_2CO_3$ (325.8mg, 1.17mmol). The reaction was vacuumed and refilled with $N_2$ twice followed by adding $Pd(dba)_2$ (89.4mg, 0.098mmol). After that, the reaction was vacuumed and refilled with $N_2$ three times and allowed to proceed at 120°C for 4h. Upon indication of completed reaction by TLC (DCM/MeOH=15/1), the system was cooled, and evaporated directly in the presence of silica gel for loading onto and separating by column chromatography, eluted with DCM/MeOH(50/1~30/1) (with 0.1ml of acetic acid per 100 ml of solvent mixture) to provide the title compound: N-methoxy-6-(((5-methoxy pyridin-2-yl)amino)-4-((2-(N-methyl methanesulfonamido)phenyl)amino)nicotinamide **(101,** 30mg, 0.06mmol, 16% yield). MS Calcd: 472; MS Found: 473 ([M+H]$^+$). $^1$H NMR (400 MHz, DMSO-d6)δ11.65 (s, 1H), 10.09 (s, 1H), 9.59 (s, 1H), 8.28 (s, 1H), 7.88 (d, *J* = 3.2 Hz, 1H), 7.67 (s, 1H), 7.63 (d, *J* = 8.4 Hz, 1H), 7.55 (dd, *J* = 8.4, 6.4 Hz, 2H), 7.48-7.44 (m, 1H), 7.36 (dd, *J* = 9.2, 3.2 Hz, 1H), 7.18 (t, *J* = 7.6 Hz, 1H), 3.77 (s, 3H), 3.71 (s, 3H), 3.16 (s, 3H), 3.14 (s, 3H).

Example **102**

N-methoxy-4-((2-(N-methyl methanesulfonamido)phenyl)amino)-6-((6-methyl pyridin-2-yl)amino)nicotinamide

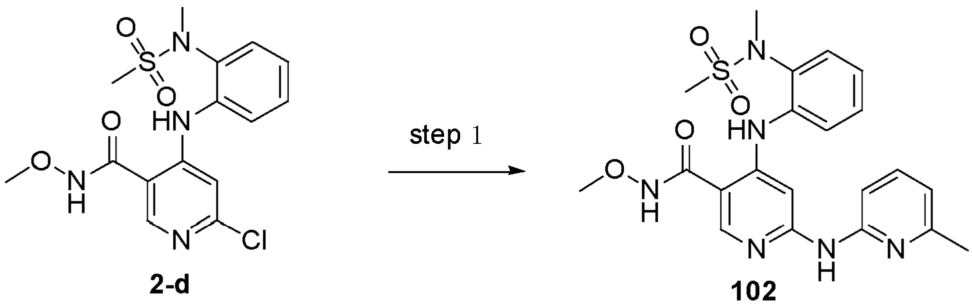


2-d 102

Step 1: To a 50ml flask were added 6-chloro-N-methoxy-4-((2-(N-methyl methanesulfonamido)phenyl)amino) nicotinamide **(2-d,** 150mg, 0.39mmol), 6-methyl pyridin-2-ylamine (84.4mg, 0.78mmol), XantPhos (113mg, 0.2mmol), 1,4-dioxane (3ml), and $Cs_2CO_3$ (325.8mg, 1.17mmol). The reaction was vacuumed and refilled with $N_2$ twice, followed by adding $Pd(dba)_2$ (89.4mg, 0.098mmol). After that, the reaction was vacuumed and refilled with $N_2$ three times and allowed to proceed at 120°C for 4h. Upon indication of completed reaction by TLC (DCM/MeOH=15/1), the system was cooled, and evaporated directly in the presence of silica gel for loading onto and separating by column chromatography to provide the title compound: N-methoxy-4-((2-(N-methyl methanesulfonamido)phenyl)amino)-6-((6-methyl pyridin-2-yl)amino)nicotinamide **(102,** 30mg, 0.06mmol, 16% yield). MS Calcd: 456; MS Found: 457 ([M+H]$^+$). $^1$H NMR (400 MHz, Chloroform-d) δ9.90 (s, 1H), 8.28 (s, 1H), 8.03 (s, 1H), 7.69 (d, *J* = 8.0 Hz, 1H), 7.52 - 7.36 (m, 4H), 7.20 (t, *J* = 7.6 Hz, 2H), 6.82 (d, *J* = 8.0 Hz, 1H), 6.71 (d, *J* = 7.2 Hz, 1H), 3.61 (s, 3H), 3.17 (s, 3H), 3.07 (s, 3H), 2.37 (s, 3H).

Example **103**

6-((4-cyano phenyl)amino)-N-methoxy-4-((2-(N-methyl methanesulfonamido)phenyl)amino)nicotinamide

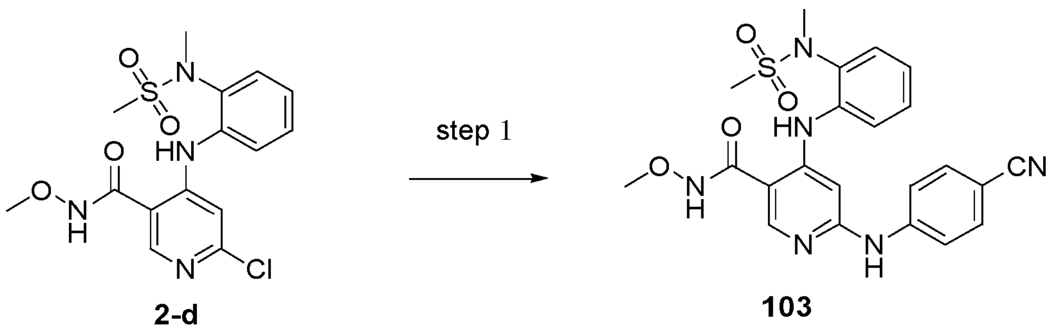


2-d 103

Step 1: To a 50ml flask were added 6-chloro-N-methoxy-4-((2-(N-methyl methanesulfonamido)phenyl)amino) nicotinamide **(2-d,** 150mg, 0.39mmol), 4-aminobenzonitrile(70mg, 0.58mmol), XantPhos(113mg, 0.2mmol), 1,4-dioxane (3ml), and $Cs_2CO_3$ (325.8mg, 1.17mmol). The reaction was vacuumed and refilled with $N_2$ twice, followed by adding $Pd(dba)_2$ (89.4mg, 0.098mmol). After that, the reaction was vacuumed and refilled with $N_2$ three times and allowed to proceed at 120°C for 4h. Upon indication of completed reaction by TLC (DCM/MeOH: v/v=15/1), the system was cooled, and evaporated directly in the presence of silica gel for loading onto and separating by column chromatography (DCM/MeOH: v:v=50/1~30/1) to provide the title compound: 6-((4-cyano phenyl)amino)-N-methoxy-4-((2-(N-methyl methanesulfonamido)phenyl)amino)nicotinamide. **(103,** 30mg, 0.06mmol, 16% yield). MS Calcd: 466; MS Found: 467 ($[M+H]^+$). $^1H$ NMR (400 MHz, DMSO-d6)δ10.89 (br, 1H), 9.38 (s, 1H), 8.46 (s, 1H), 7.81 (d, *J* = 8.4 Hz, 2H), 7.61 (d, *J* = 8.4 Hz, 2H), 7.50 (dd, *J* = 8.0, 4.0 Hz, 2H), 7.40 (t, *J* = 8.0 Hz, 1H), 7.16 (t, *J* = 8.0 Hz, 1H), 6.51 (s, 1H), 3.61 (s, 3H), 3.17 (s, 3H), 3.08 (s, 3H).

Example **104**

6-((4-fluorophenyl)amino)-N-methoxy-4-((2-(N-methyl methanesulfonamido)phenyl)amino)nicotinamide

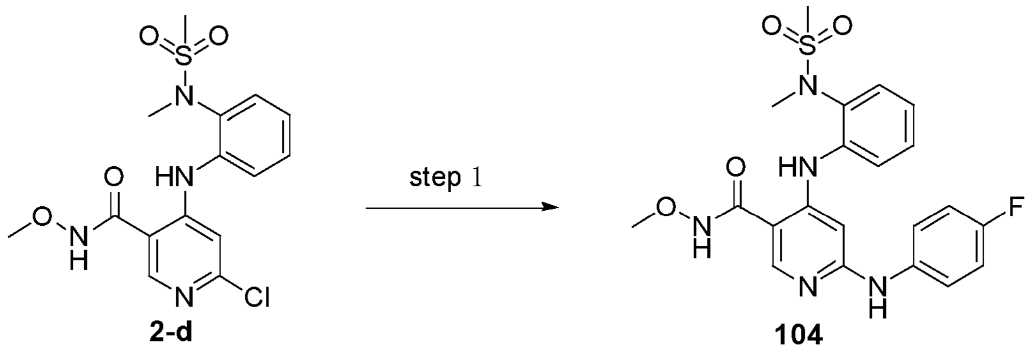


2-d 104

Step 1: To a 50ml flask were added 6-chloro-N-methoxy-4-((2-(N-methyl methanesulfonamido)phenyl)amino) nicotinamide **(2-d,** 150mg, 0.39mmol), 4-fluoroaniline(65mg, 0.58mmol), XantPhos(113mg, 0.2mmol), 1,4-dioxane (3ml), and $Cs_2CO_3$ (325.8mg, 1.17mmol). The reaction was vacuumed and refilled with $N_2$ twice, followed by adding $Pd(dba)_2$ (89.4mg, 0.098mmol). After that, the reaction was vacuumed and refilled with $N_2$ three times and allowed to proceed at 120°C for 4h. Upon indication of completed reaction by TLC (DCM/MeOH=15/1), the system was cooled, and evaporated directly in the presence of silica gel for loading onto and separating by column chromatography (DCM/MeOH: v:v=50/1~30/1) to provide the title compound: 6-((4-fluorophenyl)amino)-N-methoxy-4-((2-(N-methyl methanesulfonamido)phenyl)amino)nicotinamide **(104,** 30mg, 0.06mmol, 16% yield). MS Calcd: 459; MS Found: 460 ($[M+H]^+$). $^1H$ NMR (400 MHz, Methanol-$d_4$)δ8.17 (s, 1H), 7.88 - 7.77 (m, 1H), 7.53 (s, 1H), 7.51 (s, 1H), 7.41 - 7.32 (m, 2H), 7.24 - 7.19 (m, 1H), 7.02 - 6.98 (m, 2H), 6.38 (s, 1H), 3.80 (s, 3H), 3.23 (s, 3H), 3.05 (s, 3H).

Example **105**

(S)-6-(2, 2-dimethyl cyclopropyl-1-carboxamido)-N-methoxy-4-((2-(N-methyl methanesulfonamido)phenyl)amino)nicotinamide

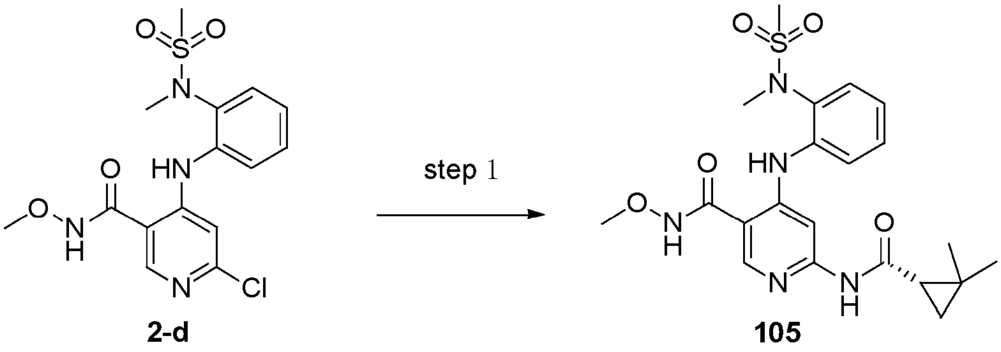


Step 1: To a 100ml flask were sequentially added 6-chloro-N-methoxy-4-((2-(N-methyl methanesulfonamido) phenyl)amino)nicotinamide **(2-d,** 150mg, 0.39mmol), (S)-2, 2-dimethylcyclopropane-1-carboxamide (66.2mg, 0.59mmol), XantPhos(113mg, 0.2mmol), 1,4-dioxane (2ml) and $Cs_2CO_3$ (325.8mg, 1.17mmol). The reaction was vacuumed and refilled with $N_2$ 5 times, followed by adding $Pd(dba)_2$ (72mg, 0.079mmol). After that, the reaction was allowed to proceed at 120°C for 6h under $N_2$ protective atmosphere. Upon indication of completed reaction by TLC (DCM/MeOH=20/1), the system was cooled and washed with water, extracted with DCM three times, dried, concentrated in the presence of silica gel for loading onto and separating by column chromatography (DCM/MeOH: v:v=50/1~30/1) to provide the title compound: (S)-6-(2, 2-dimethylcyclopropane-1-carboxamido)-N-methoxy-4-((2-(N-methyl methanesulfonamido)phenyl)amino)nicotinamide **(105,** 30mg, 0.06mmol, 16% yield). MS Calcd: 461; MS Found: 462 ($[M+H]^+$).

Example **106**

6-((6-fluoropyridin-2-yl)amino)-N-methoxy-4-((4-methyl-2-(N-methyl methanesulfonamido)phenyl)amino)nicotinamide

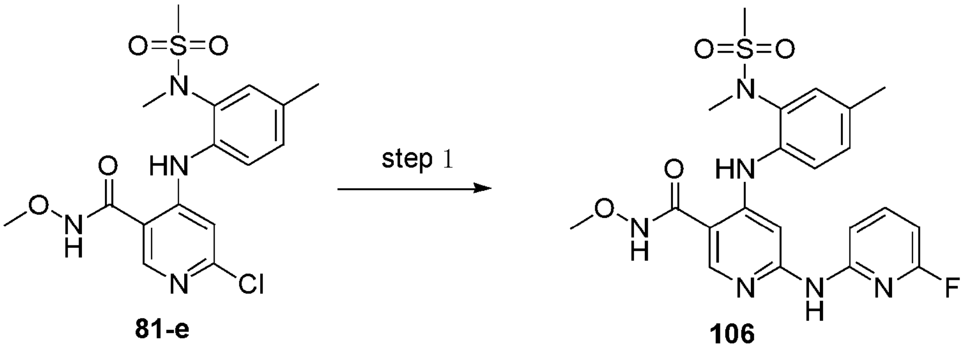


Step 4: To a 50ml flask were sequentially added 6-chloro-N-methoxy-4-((4-methyl-2-(N-methyl methanesulfonamido)phenyl)amino)nicotinamide **(81-e,** 140mg, 0.35mmol), 6-fluoropyridin-2-ylamine (79mg, 0.70mmol), XantPhos(81mg, 0.09mmol), $Cs_2CO_3$ (344mg, 1.05mmol), and 1,4-dioxane (3ml). The reaction was vacuumed and refilled with $N_2$ twice, followed by adding $Pd(dba)_2$ (97mg, 0.1mmol). After that, the reaction was vacuumed and refilled with $N_2$ three times and allowed to proceed at 120°C for 4h. Upon indication of completed reaction by TLC (DCM/MeOH=15/1), the system was cooled, and evaporated directly in the presence of silica gel for loading onto and separating by column chromatography (DCM/MeOH=50/1~30/1) to provide the title compound: 6-((6-fluoropyridin-2-yl)amino)-N-methoxy-4-((4-methyl-2-(N-methyl methanesulfonamido)phenyl)amino)nicotinamide **(106,** 75mg, 0.16mmol, 45.7% yield). MS Calcd: 474; MS Found: 475 ($[M+H]^+$). $^1H$ NMR (400 MHz, DMSO-$d_6$)δ10.12 (s, 1H), 9.93 (s, 1H), 8.35 (s, 1H), 7.79 (q, *J* = 8.4 Hz, 1H), 7.54 - 7.46 (m, 3H), 7.37 (d, *J* = 1.6 Hz, 1H), 7.22 (dd, *J* = 8.4, 2.0 Hz, 1H), 6.55 (dd, *J* = 8.0, 2.4 Hz, 1H), 5.76 (s, 1H), 3.71 (s, 3H), 3.15 (s, 3H), 3.12 (s, 3H), 2.35 (s, 3H).

Example **107**

N-methoxy-4-((4-methyl-2-(N-methyl methanesulfonamido)phenyl)amino)-6-((5-methyl pyridin-2-yl)amino)nicotinamide

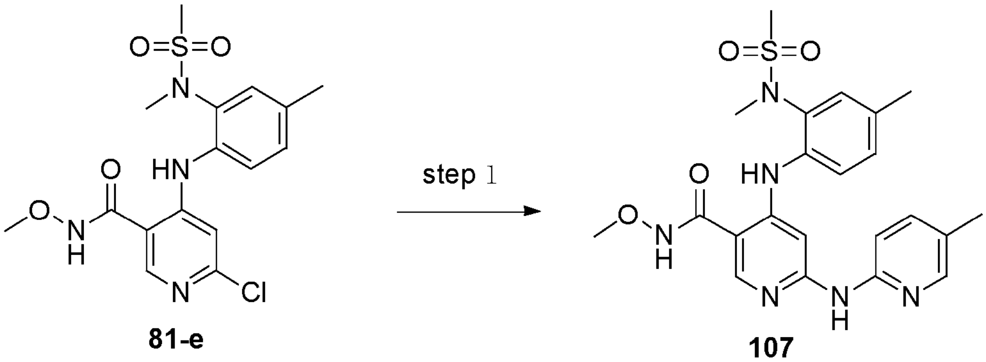


81-e

107

Step 1: To a 50ml flask were added 6-chloro-N-methoxy-4-((4-methyl-2-(N-methyl methanesulfonamido)phenyl) amino)nicotinamide **(81-e,** 140mg, 0.35mmol), 5-methyl pyridin-2-ylamine (87mg, 0.70mmol), XantPhos(81mg, 0.09mmol), $Cs_2CO_3$ (344mg, 1.05mmol), and 1,4-dioxane (3ml). The reaction was vacuumed and refilled with $N_2$ twice, followed by adding $Pd(dba)_2$ (97mg, 0.1mmol). After that, the reaction was vacuumed and refilled with $N_2$ three times and allowed to proceed at 120°C for 4h. Upon indication of completed reaction by TLC (DCM/MeOH=15/1), the system was cooled, and evaporated directly in the presence of silica gel for loading onto and separating by column chromatography (DCM/MeOH=50/1~30/1) to provide the title compound: N-methoxy-4-((4-methyl-2-(N-methyl methanesulfonamido) phenyl)amino)-6-((5-methyl pyridin-2-yl)amino)nicotinamide **(107,** 80mg, 0.17mmol, 48.6% yield). MS Calcd: 470; MS Found: 471 ($[M+H]^+$). $^1H$ NMR (400 MHz, DMSO-$d_6$)δ10.02 (s, 1H), 9.56 (s, 1H), 8.30 (s, 1H), 7.98 (s, 1H), 7.72 - 7.69 (m, 1H), 7.53 - 7.17 (m, 6H), 3.70 (s, 3H), 3.14 (s, 3H), 3.12 (s, 3H), 2.35 (s, 3H), 2.19 (s, 3H).

Example **108**

N-methoxy-6-(((5-methoxy pyridin-2-yl)amino)-4-((4-methyl-2-(N-methyl methanesulfonamido)phenyl)amino)nicotinamide

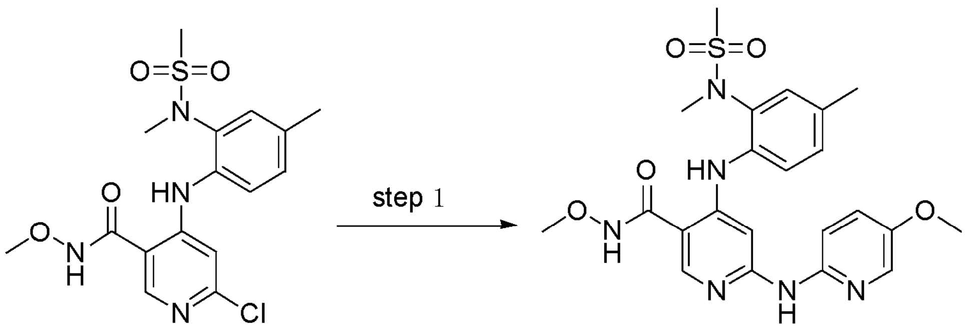


81-e

108

Step 1: To a 50ml flask were added 6-chloro-N-methoxy-4-((4-methyl-2-(N-methyl methanesulfonamido)phenyl) amino)nicotinamide **(81-e,** 120mg, 0.3mmol), 5-methoxy pyridin-2-ylamine (75mg, 0.6mmol), XantPhos(69.8mg, 0.12mmol), $Cs_2CO_3$ (295mg, 0.9mmol), and 1,4-dioxane (3ml). The reaction was vacuumed and refilled with $N_2$ twice, followed by adding $Pd(dba)_2$ (82.8mg, 0.09mmol). After that, the reaction was vacuumed and refilled with $N_2$ three times and allowed to proceed at 120°C for 4h. Upon indication of completed reaction by TLC (DCM/MeOH=15/1), the system was cooled, and evaporated directly in the presence of silica gel for loading onto and separating by column chromatography (DCM/MeOH=50/1~30/1) to provide the title compound: N-methoxy-6-(((5-methoxy pyridin-2-yl)amino)-4-((4-methyl-2-(N-methyl methanesulfonamido)phenyl)amino)nicotinamide **(108,** 80mg, 0.16mmol, 53% yield). MS Calcd: 486; MS Found: 487($[M+H]^+$). $^1H$ NMR (400 MHz, DMSO-$d_6$)δ11.62 (s, 1H), 9.94 (s, 1H), 9.52 (s, 1H), 8.26 (s, 1H), 7.88 (d, *J* = 3.2 Hz, 1H), 7.57 (d, *J* = 9.2 Hz, 1H), 7.52 (s, 1H), 7.48 (d, *J* = 8.4 Hz, 1H), 7.37 - 7.34 (m, 2H), 7.27 (dd, *J* = 8.0, 2.0 Hz, 1H), 3.77 (s, 3H), 3.71 (s, 3H), 3.14 (s, 3H), 3.12 (s, 3H), 2.35 (s, 3H).

Example **109**

6-((6-fluoropyridin-2-yl)amino)-N-methoxy-4-((6-methyl-2-(N-methyl methanesulfonamido)pyridin-3-yl)amino)nicotinamide

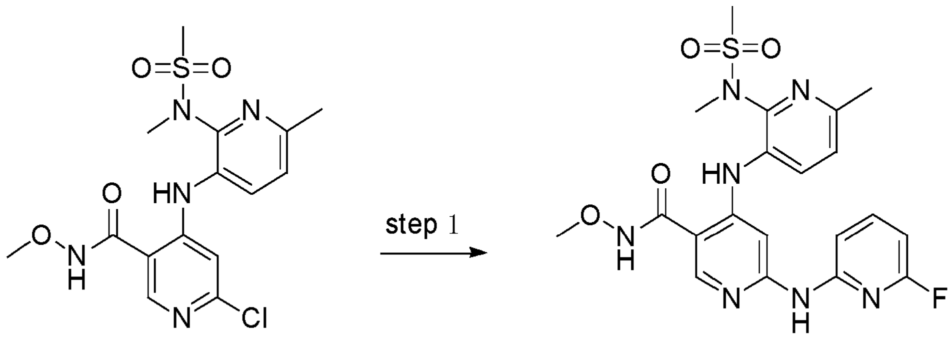


**59-d** **109**

Step 4: To a 50ml flask were added 6-chloro-N-methoxy-4-((6-methyl-2-(N-methyl methanesulfonamido)pyridin-3-yl)amino)nicotinamide **(59-d,** 150mg, 0.38mmol), 6-fluoropyridin-2-ylamine (84mg, 0.76mmol), XantPhos(87mg, 0.15mmol), $Cs_2CO_3$ (367mg, 1.13mmol), and 1,4-dioxane (3ml). The reaction was vacuumed and refilled with $N_2$ twice, followed by adding $Pd(dba)_2$ (103mg, 0.11mmol). After that, the reaction was vacuumed and refilled with $N_2$ three times, and allowed to proceed at 120 °C for 4h. Upon indication of completed reaction by TLC (DCM/MeOH=15/1), the system was cooled, and evaporated directly in the presence of silica gel for loading onto and separating by column chromatography (DCM/MeOH=50/1~30/1) to provide the title compound: 6-((6-fluoropyridin-2-yl)amino)-N-methoxy-4-((6-methyl-2-(N-methyl methanesulfonamido)pyridin-3-yl)amino)nicotinamide **(109,** 76mg, 0.16mmol, 42% yield). MS Calcd: 475; MS Found: 476($[M+H]^+$). $^1H$ NMR (400 MHz, DMSO-$d_6$)δ11.74 (s, 1H), 10.07 (s, 1H), 10.02 (s, 1H), 8.35 (s, 1H), 7.98 (d, *J* = 8.0 Hz, 1H), 7.80 (q, *J* = 8.0 Hz, 1H), 7.55 (s, 1H), 7.46 (dd, *J* = 8.0, 2.0 Hz, 1H), 7.32 (d, *J* = 8.0 Hz, 1H), 6.57 (dd, *J* = 8.0, 2.4 Hz, 1H), 3.73 (s, 3H), 3.32 (s, 3H), 3.21 (s, 3H), 2.49 (s, 3H).

Example **110**

4-((6-cyclopropyl-2-(N-methyl methanesulfonamido)phenyl)amino)-6-((5-fluoro-4-methyl pyridin-2-yl)amino)-N-methoxy nicotinamide

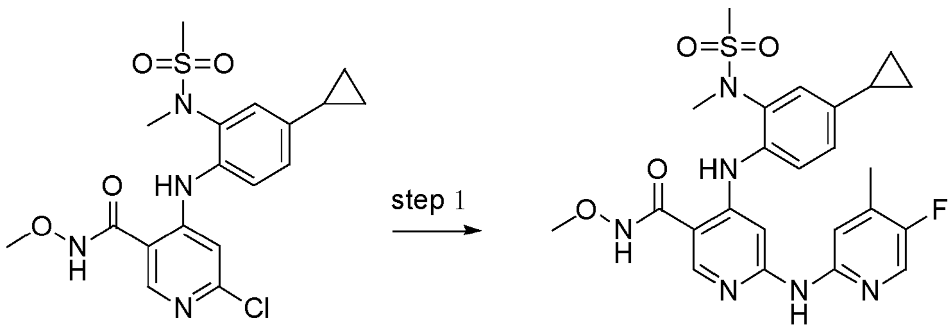


**12-e** **110**

Step 1: To a 50ml flask were added 6-chloro-4-((4-cyclopropyl-2-(N-methyl methanesulfonamido)phenyl)amino)-N-methoxy nicotinamide **(12-e,** 80mg, 0.19mmol), 5-fluoro-4-methyl pyridin-2-ylamine (46m, 0.38mmol), XantPhos(44mg, 0.07mmol), $Cs_2CO_3$ (184mg, 0.57mmol), and 1,4-dioxane (3ml). The reaction was vacuumed and refilled with $N_2$ twice, followed by adding $Pd(dba)_2$ (52mg, 0.057mmol). After that, the reaction was vacuumed and refilled with $N_2$ three times and allowed to proceed at 120°C for 4h. Upon indication of completed reaction by TLC (DCM/MeOH=15/1), the system was cooled, and evaporated directly in the presence of silica gel for loading onto and separating by column chromatography (DCM/MeOH=50/1~30/1) to provide the title compound: 4-((6-cyclopropyl-2-(N-methyl methanesulfonamido)phenyl)amino)-6-((5-fluoro-4-methyl pyridin-2-yl)amino)-N-methoxy nicotinamide **(110,** 30mg, 0.06mmol, 32%

yield). MS Calcd: 515; MS Found: 516([M+H]$^+$). $^1$H NMR (400 MHz, DMSO-$d_6$)δ11.66 (s, 1H), 9.92 (s, 1H), 9.70 (s, 1H), 8.29 (s, 1H), 8.04 (s, 1H), 7.54 (d, *J* = 9.6, 1H), 7.46 (d, *J* = 8.4 Hz, 2H), 7.26 (d, *J* = 2.0 Hz, 1H), 7.15 (dd, *J* = 8.4, 2.0 Hz, 1H), 3.71 (s, 3H), 3.14 (s, 3H), 3.12 (s, 3H), 2.23 (s, 3H), 2.00 - 1.94 (m, 1H), 1.01 - 0.97 (m, 2H), 0.75 - 0.71 (m, 2H).

Example **111**

4-((4-cyclopropyl-2-(N-methyl methanesulfonamido)phenyl)amino)-6-((5-cyano pyridin-2-yl)amino)-N-methoxy nicotinamide

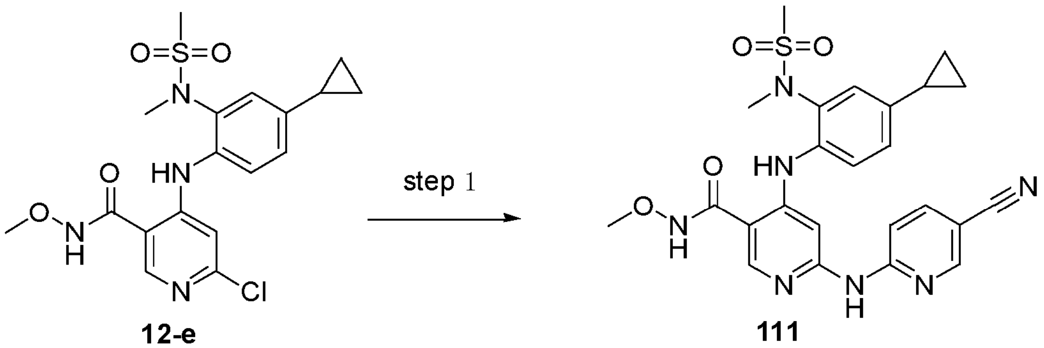


12-e

111

Step 1: To a 50ml flask were sequentially added 6-chloro-4-((4-cyclopropyl-2-(N-methyl methanesulfonamido) phenyl)amino)-N-methoxy nicotinamide **(12-e,** 80mg, 0.19mmol), 5-cyano pyridin-2-ylamine (44mg, 0.38mmol), XantPhos(44mg, 0.07mmol), $Cs_2CO_3$ (184mg, 0.57mmol), and 1,4-dioxane (3ml). The reaction was vacuumed and refilled with $N_2$ twice, followed by adding Pd(dba)$_2$ (52mg, 0.057mmol). After that, the reaction was vacuumed and refilled with $N_2$ three times and allowed to proceed at 120°C for 4h. Upon indication of completed reaction by TLC (DCM/MeOH=15/1), the system was cooled, and evaporated directly in the presence of silica gel for loading onto and separating by column chromatography (DCM/MeOH=50/1~30/1) to provide the title compound: 4-((4-cyclopropyl-2-(N-methyl methanesulfonamido)phenyl)amino)-6-((5-cyano pyridin-2-yl)amino)-N-methoxy nicotinamide **(111,** 30mg, 0.06mmol, 33% yield). MS Calcd: 520; MS Found: 521([M+H]$^+$). $^1$H NMR (400 MHz, DMSO-$d_6$)δ11.74 (s, 1H), 10.31 (s, 1H), 9.94 (s, 1H), 8.60 (d, *J* = 2.4 Hz, 1H), 8.33 (s, 1H), 8.04 (dd, *J* = 8.8, 2.4 Hz, 1H), 7.78 (d, *J* = 8.8 Hz, 1H), 7.57 (s, 1H), 7.46 (d, *J* = 8.4 Hz, 1H), 7.28 (d, *J* = 2.0 Hz, 1H), 7.19 (dd, *J* = 8.4, 2.0 Hz, 1H), 3.72 (s, 3H), 3.14 (s, 3H), 3.10 (s, 3H), 2.02-1.95 (m, 1H), 1.02 - 0.98 (m, 2H), 0.76 - 0.73 (m, 2H).

Example **112**

4-((4-cyclopropyl-2-(N-methyl methanesulfonamido)phenyl)amino)-6-(((2,6-dimethyl pyrimidin-4-yl)amino)-N-methoxy nicotinamide

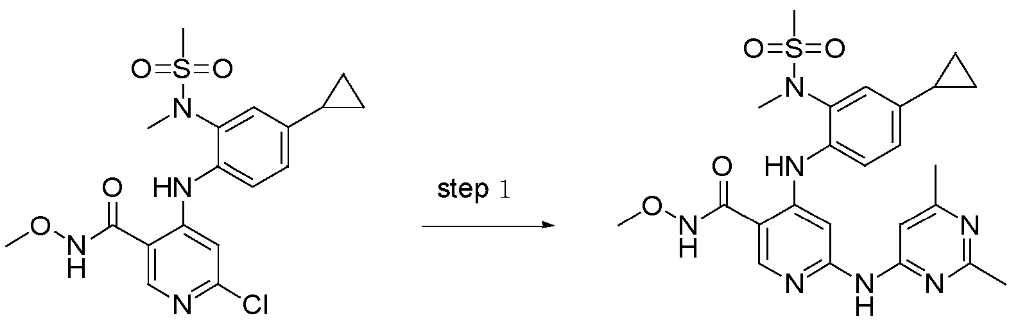


12-e

112

Step 1: To a 50ml flask were added 6-chloro-4-((4-cyclopropyl-2-(N-methyl methanesulfonamido)phenyl)amino)-N-methoxy nicotinamide **(12-e,** 80mg, 0.19mmol), 2,6-dimethyl pyrimidin-4-ylamine (46mg, 0.38mmol), Xant-

Phos(44mg, 0.07mmol), $Cs_2CO_3$ (184mg, 0.57mmol), and 1,4-dioxane (3ml). The reaction was vacuumed and refilled with $N_2$ twice, followed by adding $Pd(dba)_2$ (52mg, 0.057mmol). After that, the reaction was vacuumed and refilled with $N_2$ three times and allowed to proceed at 120°C for 4h. Upon indication of completed reaction by TLC (DCM/MeOH=15/1), the system was cooled, and evaporated directly in the presence of silica gel for loading onto and separating by column chromatography (DCM/MeOH=50/1~30/1) to provide the title compound: 4-((4-cyclopropyl-2-(N-methyl methanesulfonamido)phenyl)amino)-6-(((2,6-dimethyl pyrimidin-4-yl)amino)-N-methoxy nicotinamide **(112,** 30mg, 0.06mmol, 33% yield). MS Calcd: 511; MS Found: 512($[M+H]^+$). $^1H$ NMR (400 MHz, DMSO-$d_6$)δ11.76 - 11.64 (m, 1H), 9.98 (s, 1H), 9.89 (s, 1H), 8.31 (s, 1H), 7.84 (s, 1H), 7.46 (d, $J$ = 8.2 Hz, 1H), 7.31 (d, $J$ = 2.0 Hz, 1H), 7.16 - 7.07 (m, 1H), 7.03 (s, 1H), 3.72 (s, 3H), 3.13 (s, 3H), 3.09 (s, 3H), 2.31 (s, 3H), 2.26 (s, 3H), 2.01 - 1.95 (m, 1H), 1.03 - 0.95 (m, 2H), 0.73 - 0.65 (m, 2H).

Example **113**

4-((4-chloro-2-(N-methyl methanesulfonamido)phenyl)amino)-N-ethoxy-6-((4-methoxy phenyl) amino)nicotinamide

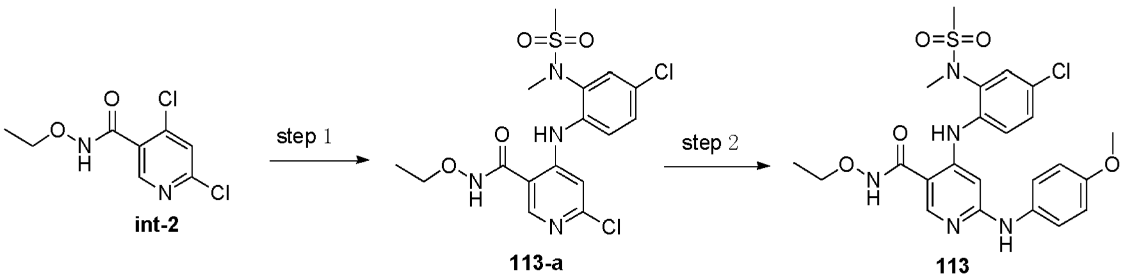

int-2 113-a 113

Step 1: To a 100mL two-necked flask were sequentially added 4,6-dichloro-N-ethoxy nicotinamide (**int-2,** 1.5g, 6.4mmol), N-(2-amino-5-chloro phenyl)-N-methyl methanesulfonamide (1.8g, 7.72mmol), and 5ml of anhydrous DMA as solvent, followed by adding at room temperature a solution of LiHMDS in tetrahydrofuran (19mL, 1mmol/mL) and continuous stirring at room temperature for 2h. Upon indication of completed reaction by TLC, the mixture was adjusted with aqueous hydrochloride (1N) to pH 5, and extracted with ethyl acetate (30mL). The combined organic layers were dried and concentrated, followed by purification by silica gel column chromatography (PE:EA=2; 1) to provide 6-chloro-4-((4-chloro-2-((N-methyl methanesulfonamido)phenyl)amino)-N-ethoxy nicotinamide **(113-a,** 1.65g, 3.77mmol, 59% yield). MS Calcd: 432; MS Found: 433($[M+H]^+$).

Step 2: To a 50ml flask were added 6-chloro-4-((4-chloro-2-((N-methyl methanesulfonamido)phenyl)amino)-N-ethoxy nicotinamide **(113-a,** 140mg, 0.33mmol), 4-methoxy-2-aminopyridine (82mg, 0.66mmol), XantPhos(78mg, 0.14mmol), $Cs_2CO_3$ (327mg, 0.99mmol), and 1,4-dioxane (3ml). The reaction was vacuumed and refilled with $N_2$ twice, followed by adding $Pd(dba)_2$ (92mg, 0.1mmol). After that, the reaction was vacuumed and refilled with $N_2$ three times and allowed to proceed at 120°C for 4h. Upon indication of completed reaction by TLC (DCM/MeOH=15/1), the system was cooled, and evaporated directly in the presence of silica gel for loading onto and separating by column chromatography (DCM/MeOH=50/1~30/1) to provide the title compound: 4-((4-chloro-2-(N-methyl methanesulfonamido)phenyl)amino)-N-ethoxy-6-((4-methoxy phenyl) amino)nicotinamide (113, 120mg, 0.28mmol, 85% yield). MS Calcd: 519; MS Found: 520($[M+H]^+$). $^1H$ NMR (400 MHz, DMSO-$d_6$)δ11.58 (s, 1H), 10.10 (s, 1H), 9.61 (s, 1H), 8.31 (s, 1H), 7.93 (d, $J$ = 2.8 Hz, 1H), 7.76 - 7.53 (m, 6H), 7.36 (dd, $J$ = 8.8, 2.8 Hz, 1H), 3.93 (q, $J$ = 6.8 Hz, 2H), 3.78 (s, 3H), 3.17 (s, 6H), 1.22 (t, $J$ = 6.8 Hz, 3H).

Example **114**

4-((4-chloro-2-(N-methyl methanesulfonamido)phenyl)amino)-N-ethoxy-6-((5-cyano pyridin-2-yl)amino)nicotinamide

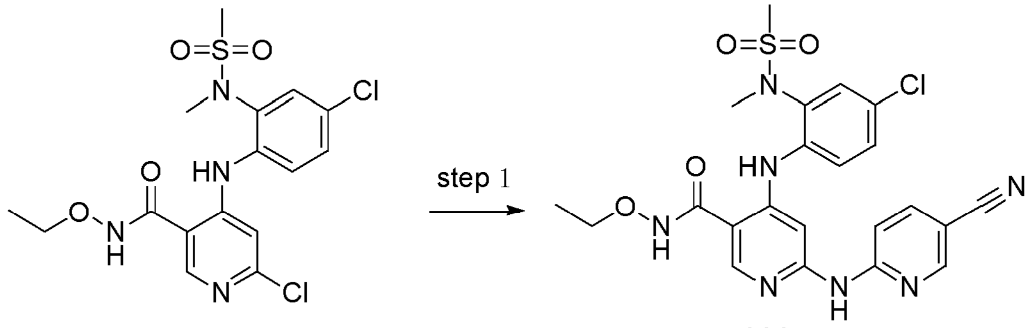


113-a 114

Step 1: To a 50ml flask were added 6-chloro-4-((4-chloro-2-((N-methyl methanesulfonamido)phenyl)amino)-N-ethoxy nicotinamide **(113-a,** 140mg, 0.33mmol), 5-cyano pyridin-2-ylamine (80mg, 0.66mmol), XantPhos(78mg, 0.14mmol), $Cs_2CO_3$ (327mg, 0.99mmol), and 1,4-dioxane (3ml). The reaction was vacuumed and refilled with $N_2$ twice, followed by adding $Pd(dba)_2$ (92mg, 0.1mmol). After that, the reaction was vacuumed and refilled with $N_2$ three times and allowed to proceed at 120°C for 4h. Upon indication of completed reaction by TLC (DCM/MeOH=15/1), the system was cooled, and evaporated directly in the presence of silica gel for loading onto and separating by column chromatography (DCM/MeOH=50/1~30/1) to provide the title compound: 4-((4-chloro-2-(N-methyl methanesulfonamido)phenyl)amino)-N-ethoxy-6-((5-cyano pyridin-2-yl)amino)nicotinamide **(114,** 120mg, 0.28mmol, 85% yield). MS Calcd: 515; MS Found: 516($[M+H]^+$). $^1H$ NMR (400 MHz, DMSO-$d_6$)δ11.70 (s, 1H), 10.39 (s, 1H), 10.11 (s, 1H), 8.67 (d, *J* = 2.4 Hz, 1H), 8.38 (s, 1H), 8.05 (dd, *J* = 8.8, 2.4 Hz, 1H), 7.74 - 7.58 (m, 5H), 3.95 (q, *J* = 7.2 Hz, 2H), 3.17 (s, 3H), 3.16 (s, 3H), 1.22 (t, *J* = 7.2 Hz, 3H).

Example **115**

4-((4-chloro-2-(N-methyl methanesulfonamido)phenyl)amino)-6-((2,6-dimethyl pyrimidin-4-yl)amino)-N-ethoxy nicotinamide

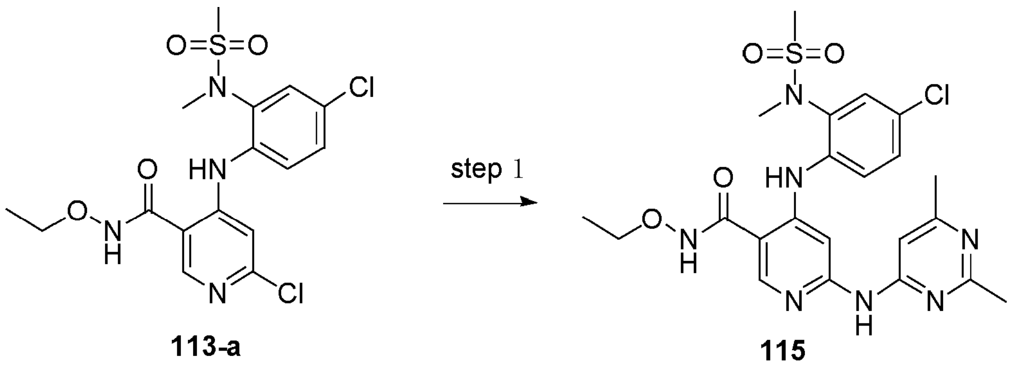


113-a 115

Step 1: To a 50ml flask were added 6-chloro-4-((4-chloro-2-((N-methyl methanesulfonamido)phenyl)amino)-N-ethoxy nicotinamide (**113-a,** 140mg, 0.33mmol), 2,6-dimethyl pyrimidin-4-ylamine (83mg, 0.66mmol), XantPhos(78mg, 0.14mmol), $Cs_2CO_3$ (327mg, 0.99mmol), and 1,4-dioxane (3ml). The reaction was vacuumed and refilled with $N_2$ twice, followed by adding $Pd(dba)_2$ (92mg, 0.1mmol). After that, the reaction was vacuumed and refilled with $N_2$ three times and allowed to proceed at 120°C for 4h. Upon indication of completed reaction by TLC (DCM/MeOH=15/1), the system was cooled, and evaporated directly in the presence of silica gel for loading onto and separating by column chromatography (DCM/MeOH=50/1~30/1) to provide the title compound: 4-((4-chloro-2-(N-methyl methanesulfonamido)phenyl)amino)-6-((2,6-dimethyl pyrimidin-4-yl)amino)-N-ethoxy nicotinamide **(115,** 100mg, 0.22mmol, 80% yield). MS Calcd: 519; MS Found: 520($[M+H]^+$). $^1H$ NMR (400 MHz, DMSO-$d_6$)δ11.66 (s, 1H), 8.36 (s, 1H), 7.91 (s, 1H), 7.72 (d, *J* = 2.4 Hz, 1H), 7.63 (d, *J* = 8.8 Hz, 1H), 7.50 (dd, *J* = 8.8, 2.4 Hz, 1H), 7.27 - 7.22 (m, 1H), 7.18 - 7.13 (m, 1H), 7.09 (s, 1H), 3.94 (q, *J* = 7.2 Hz, 2H), 3.16 (s, 3H), 3.15 (s, 3H), 2.35 (s, 3H), 2.27 (s, 3H), 1.22 (t, *J* = 7.2 Hz, 3H).

Example **116**

4-((2-(cyclopropyl sulfonamido)phenyl)amino)-N-ethoxy-6-((6-fluoropyridin-2-yl)amino)nicotinamide

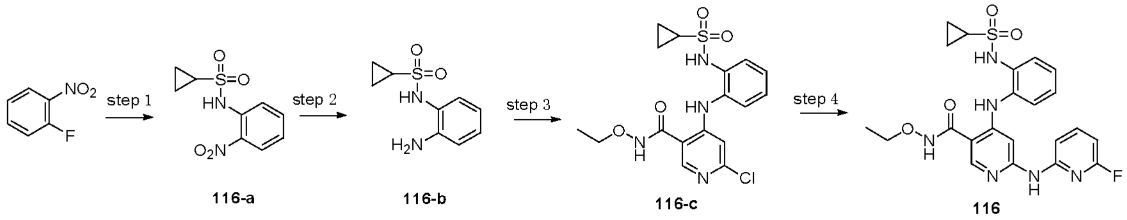


Step 1: To a 100ml flask were added 1-fluoro-2-nitrobenzene(500mg, 3.5mmol), N-methyl methanesulfonamide (546mg, 3.6mmol), and DMF (20ml). The reaction was vacuumed and refilled with $N_2$ three times followed by adding $K_2CO_3$(1.47mg, 10.5mmol). After that, the reaction was allowed to proceed at 90°C for 3h. Upon indication of completed reaction by TLC (petroleum ether/ethyl acetate=1/1), the system was washed with water, and extracted with ethyl acetate three times. The combined organic layers were dried, concentrated, and subjected to column chromatography (petroleum ether/ethyl acetate=2/1~1/2) to provide N-(2-nitrophenyl) cyclopropylsulfonamide **(116-a,** 800mg, 3.3mmol, 94% yield), as a white solid. MS Calcd: 242; MS Found: 343([M+H]$^+$).

Step 2: To a 100ml flask were added N-(2-nitrophenyl) cyclopropylsulfonamide **(116-a,** 800mg, 3.3mmol) and Pd/C(10%, 80mg, 0.33mmol), bubbled with $H_2$ to react for 5h at room temperature. Upon indication of completed reaction by TLC (petroleum ether/ethyl acetate=1/1), the system was filtered, and the filter cake was washed with ethyl acetate three times. The combined organic layers were dried and concentrated to provide N-(2-amino phenyl)cyclopropanesulfonamide **(116-b,** 680mg, 3.2mmol, 97% yield), as a white solid. MS Calcd: 212; MS Found: 213 ([M+H]$^+$).

Step 3: To a 100ml flask were added N-(2-amino phenyl) cyclopropylsulfonamide **(116-b,** 680mg, 3.2mmol), 4,6-dichloro-N-ethoxy nicotinamide **(int-2,** 825mg, 3.3mmol), and anhydrous tetrahydrofuran (30ml).After atmosphere replacement with nitrogen three times, the reaction was slowly added with LiHMDS (10ml, 9.9mmol) and allowed to proceed under nitrogen protection for 6h at room temperature. Upon indication of completed reaction by TLC (petroleum ether/ethyl acetate=1/2), the system was washed with water and adjusted with 0.1mol/L HCl to pH=5, and extracted with ethyl acetate three times. The combined organic layers were dried, concentrated in the presence of silica gel for loading onto and separating by column chromatography (petroleum ether/ethyl acetate=2/1~1/1) to provide 6-chloro-4-((2-(cyclopropyl sulfonamido)phenyl)amino)-N-ethoxy nicotinamide **(116-c,** 500mg, 1.3mmol, 40% yield), as a pale yellow solid. MS Calcd: 410; MS Found: 411 ([M+H]$^+$).

Step 4: To a 50ml flask were added 6-chloro-4-((2-(cyclopropyl sulfonamido)phenyl)amino)-N-ethoxy nicotinamide **(116-c,** 150mg, 0.35mmol), 6-fluoropyridin-2-ylamine (82mg, 0.7mmol), XantPhos (85mg, 0.14mmol), 1,4-dioxane (3ml), and $Cs_2CO_3$ (358mg, 1.0mmol). The reaction was vacuumed and refilled with $N_2$ twice, followed by adding $Pd(dba)_2$ (100mg, 0.1mmol). After that, the reaction was vacuumed and refilled with $N_2$ three times and allowed to proceed at 120°C for 4h. Upon indication of completed reaction by TLC (DCM/MeOH=15/1), the system was cooled, and evaporated directly in the presence of silica gel for loading onto and separating by column chromatography (DCM/MeOH=50/1~30/1) to provide the title compound: 4-((2-(cyclopropyl sulfonamido)phenyl)amino)-N-ethoxy-6-((6-fluoropyridin-2-yl)amino)nicotinamide **(116,** 30mg, 0.06mmol, 16% yield). MS Calcd: 486; MS Found: 485 ([M-H]$^-$). $^1$H NMR (400 MHz, DMSO-$d_6$) δ9.92 (s, 1H), 8.32 (s, 1H), 7.78 (dd, $J$ = 8.4, 16.4 Hz, 1H), 7.58 - 7.52 (m, 2H), 7.48 (dd, $J$ = 8.0, 2.4 Hz, 1H), 7.41 (dd, $J$ = 8.0, 1.6 Hz, 1H), 7.26 (t, $J$ = 8.0 Hz, 1H), 7.13 (dd, $J$ = 8.4, 16.4 Hz, 1H), 6.53 (dd, $J$ = 8.4, 2.8 Hz, 1H), 3.95 (q, $J$ = 7.2 Hz, 2H), 2.66 - 2.60 (m, 1H), 1.22 (t, $J$ = 7.2 Hz, 3H), 0.88 - 2.60 (m, 1H).

Example **117**

N-ethoxy-6-((6-fluoropyridin-2-yl)amino)-4-((2-(methanesulfonamido)phenyl)amino)nicotinamide

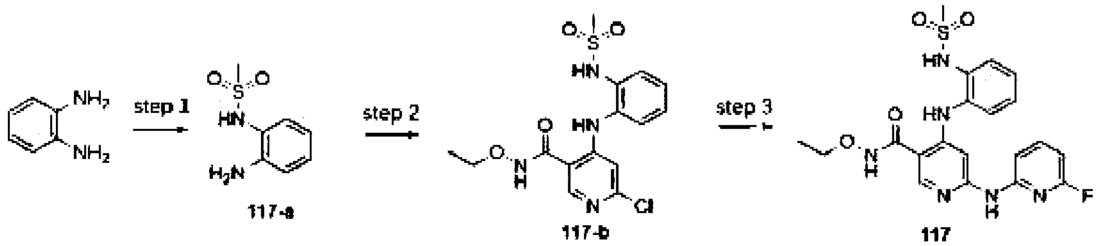


117-a 117-b 117

Step 1: To a 100ml flask were added benzene 1,2-diamine(1g, 7.2mmol), triethylamine (2.19g, 18mmol), and DCM (20ml). To the mixture in ice-water bath was slowly added methanesulfonyl chloride (825mg, 7.2mmol) dropwise, and allowed to warm back to room temperature to react overnight. Upon indication of completed reaction by TLC (petroleum ether/ethyl acetate=1/1), the system was washed with water and adjusted with 0.1mol/L HCl to pH=5, and extracted with ethyl acetate three times. The combined organic layers were dried, concentrated, and subjected to column chromatography (petroleum ether/ethyl acetate=2/1~1/1) to provide N-(2-amino phenyl) methanesulfonamide(117-a, 700mg, 3.7mmol, 51% yield). MS Calcd: 186; MS Found:187($[M+H]^+$).

Step 2: To a 100ml flask were added N-(2-amino phenyl) methanesulfonamide(**117-a**, 700mg, 3.7mmol), 4,6-dichloro-N-ethoxy nicotinamide **(int-2,** 798mg, 3.7mmol), and tetrahydrofuran (30ml). After atmosphere replacement with nitrogen three times, the reaction mixture was slowly added with LiHMDS (11ml, 11.1mmol) and allowed to proceed under $N_2$ protective atmosphere for 6h at room temperature. Upon indication of completed reaction by TLC (petroleum ether/ethyl acetate=1/2), the system was washed with water and adjusted with 0.1mol/L HCl to pH=5, and extracted with ethyl acetate three times. The combined organic layers were dried, concentrated in the presence of silica gel for loading onto and separating by column chromatography (petroleum ether/ethyl acetate=2/1~1/1) to provide 6-chloro-N-ethoxy-4-((2-(methyl sulfonamido)phenyl)amino)nicotinamide **(117-b,** 150mg, 0.35mmol, 10% yield), as a pale yellow solid. MS Calcd: 384; MS Found: 385 ($[M+H]^+$).

Step 3: To a 50ml flask were added 6-chloro-N-ethoxy-4-((2-(methyl sulfonamido)phenyl)amino)nicotinamide **(117-b,** 60mg, 0.16mmol), 6-methyl pyridin-2-ylamine (35mg, 0.32mmol), XantPhos(36mg, 0.04mmol), 1,4-dioxane (3ml), and $Cs_2CO_3$ (153mg, 0.48mmol). The reaction was vacuumed and refilled with $N_2$ twice, followed by adding $Pd(dba)_2$ (43mg, 0.05mmol). After that, the reaction was vacuumed and refilled with $N_2$ three times and allowed to proceed at 120°C for 4h. Upon indication of completed reaction by TLC (DCM/MeOH=15/1), the system was cooled, and evaporated directly in the presence of silica gel for loading onto and separating by column chromatography (DCM/MeOH=50/1~30/1) to provide the title compound: N-ethoxy-6-((6-fluoropyridin-2-yl)amino)-4-((2-(methanesulfonamido)phenyl)amino)nicotinamide (117, 30mg, 0.06mmol, 16% yield). MS Calcd: 459; MS Found: 460 ($[M+H]^+$). $^1H$ NMR (400 MHz, DMSO-$d_6$)δ11.60 (s, 1H), 9.94 (s, 1H), 9.86 (s, 1H), 9.28 (s, 1H), 8.33 (s, 1H), 7.78 (dd, *J* = 8.4, 16.4 Hz, 1H), 7.58 - 7.47 (m, 4H), 7.39 (dd, *J* = 8.0, 1.6 Hz, 1H), 7.33 (t, *J* = 8.0 Hz, 1H), 7.20 - 7.16 (m, 1H), 6.54 (dd, *J* = 8.0, 2.8 Hz, 1H), 3.96 (q, *J* = 7.2 Hz, 2H), 2.99 (s, 3H), 1.23 (t, *J* = 7.2 Hz, 3H).

Example **118**

4-((4-cyclopropyl-2-(N-methyl methanesulfonamido)phenyl)amino)-6-(((2,6-dimethyl pyrimidin-4-yl)amino)-N-(2,2,2-trifluoroethoxy)nicotinamide

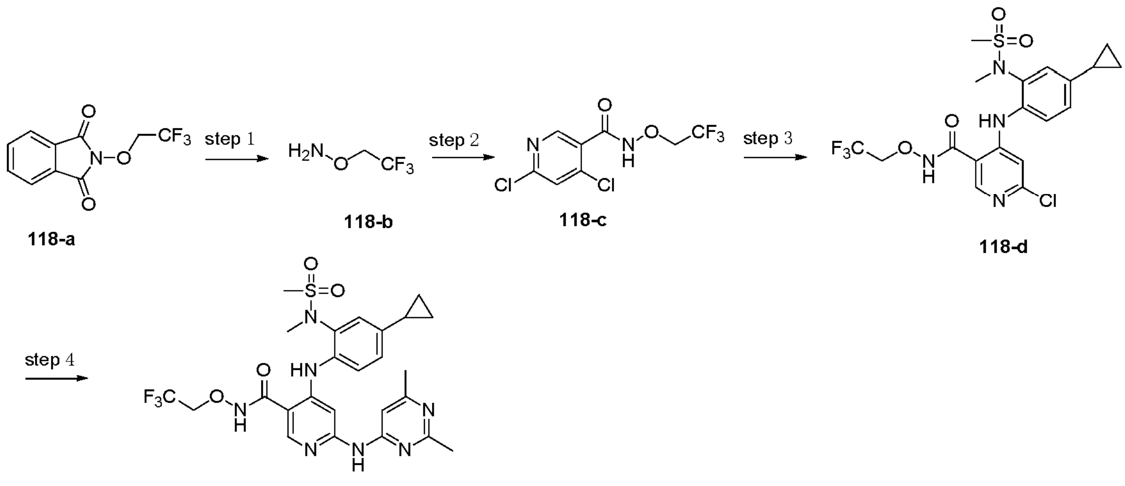


118-a 118-b 118-c 118-d

118

Step 1: To a 100ml flask were added 2-(2,2,2-trifluoroethoxy)isoindoline-1,3-dione **(118-a,** 4g, 16.3mmol) and DCM/MEOH=30ml/3ml. The reaction was vacuumed and refilled with $N_2$ three times, cooled in ice-water bath and slowly added with hydrazine hydrate (816mg, 16.3mmol) dropwise, and allowed to warm back to room temperature to react overnight. The system was washed with water and adjusted with 0.1mol/L HCl to pH=6, and extracted with ethyl acetate three times. The combined organic layers were dried, concentrated, and subjected to column chromatography (petroleum ether/ethyl acetate=10/1~5/1) to provide O-(2,2,2-trifluoroethyl) hydroxylamine **(118-b,** 2.0g, 13.3mmol, 81% yield), as a white solid. MS Calcd: 150; MS Found: 151($[M+H]^+$).

Step 2: To a 100ml flask were added O-(2,2,2-trifluoroethyl) hydroxylamine (1.1g, 7.3mmol), 4,6-dichloronicotinyl chloride **(118-b,** 1.5g, 7.3mmol), $K_2CO_3$(3g, 21.9mmol), and $H_2O$(9ml). After atmosphere replacement with nitrogen three times, the reaction mixture was added with ethyl acetate (15ml) solution of 4,6-dichloronicotinyl chloride (1.5g, 7.3mmol) dropwise, and allowed to proceed for 3h. Upon indication of completed reaction by TLC (petroleum ether/ethyl acetate=2/1), the system was washed with water and extracted with ethyl acetate three times. The combined organic layers were dried, concentrated in the presence of silica gel for loading onto and separating by column chromatography (petroleum ether/ethyl acetate=10/1~2/1) to provide 4,6-dichloro-N-(2,2,2-trifluoroethoxy)nicotinamide **(118-c,** 600mg, 3.1mmol, 42% yield), as a white solid. MS Calcd: 287; MS Found: 288($[M+H]^+$).

Step 3: To a 50ml flask were added 4,6-dichloro-N-(2,2,2-trifluoroethoxy)nicotinamide **(118-c,** 600mg, 3.1mmol) and N-(2-amino-5-cyclopropyl phenyl)-N-methyl methanesulfonamide (750mg, 3.1mmol). After atmosphere replacement with nitrogen three times, the reaction mixture was slowly added with LiHMDS (9.3ml, 9.3mmol) under nitrogen protection and allowed to proceed for 6h. Upon indication of completed reaction by TLC (petroleum ether/ethyl acetate=1/2), the system was washed with water, adjusted with 0.1mol/L HCl to pH=5, and extracted with ethyl acetate three times. The combined organic layers were dried, concentrated in the presence of silica gel for loading onto and separating by column chromatography (petroleum ether/ethyl acetate=2/1~1/1) to provide 6-chloro-4-((4-cyclopropyl-2-(N-methyl methanesulfonamido)phenyl)amino)-N-(2,2,2-trifluoroethoxy)nicotinamide (**118-d**, 120°Cg, 0.24mmol, 8% yield), as a white solid. MS Calcd: 492; MS Found:493 ($[M+H]^+$).

Step 4: To a 50ml flask were added 6-chloro-4-((4-cyclopropyl-2-(N-methyl methanesulfonamido)phenyl)amino)-N-(2,2,2-trifluoroethoxy)nicotinamide **(118-d,** 60mg, 0.12mmol), 2,6-dimethyl pyrimidin-4-ylamine (30mg, 0.24mmol), XantPhos(28mg, 0.048mmol), 1,4-dioxane (3ml), and $Cs_2CO_3$ (119mg, 0.36mmol). The reaction was vacuumed and refilled with $N_2$ twice, followed by adding $Pd(dba)_2$ (33mg, 0.036mmol). After that, the reaction was vacuumed and refilled with $N_2$ three times, and allowed to proceed at 120°C for 4h. Upon indication of completed reaction by TLC (DCM/MeOH=15/1), the system was cooled, and evaporated directly in the presence of silica gel for loading onto and separating by column chromatography (DCM/MeOH=50/1~30/1) to provide the title compound: 4-((4-cyclopropyl-2-(N-methyl methanesulfonamido)phenyl)amino)-6-(((2,6-dimethyl pyrimidin-4-yl)amino)-N-(2,2,2-trifluoroethoxy) nicotinamide **(118,** 30mg, 0.06mmol, 16% yield). MS Calcd: 579; MS Found: 580($[M+H]^+$). $^1H$ NMR (400 MHz, DMSO-$d_6$)δ12.15 (s, 1H), 10.00 (s, 1H), 9.75 (s, 1H), 8.33 (s, 1H), 7.82 (s, 1H), 7.45 (d, $J$ = 8.4 Hz, 1H), 7.32 (s, 1H), 7.13 (dd, $J$ = 8.8, 2.0 Hz, 1H), 7.02 (s, 1H), 4.57 (s, 2H), 3.13 (s, 3H), 3.08 (s, 3H), 2.30 (s, 3H), 2.26 (s, 3H), 2.00 - 1.97 (m, 1H), 1.02-0.97 (m, 2H), 0.732-0.71 (m, 2H).

Example **119**

4-((4-cyclopropyl-2-(N-methyl methanesulfonamido)phenyl)amino)-6-((6-fluoropyridin-2-yl)amino)-N-(2,2,2-trifluoroethoxy)nicotinamide

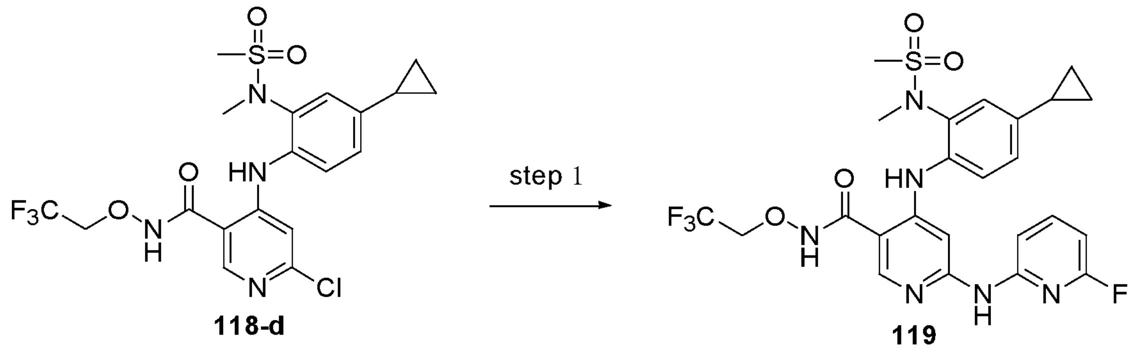


118-d

119

Step 1: To a 50ml flask were added 6-chloro-4-((4-cyclopropyl-2-(N-methyl methanesulfonamido)phenyl)amino)-N-(2,2,2-trifluoroethoxy)nicotinamide **(118-d,** 60mg, 0.12mmol), 6-fluoropyridin-2-ylamine (30mg, 0.24mmol), XantPhos(28mg, 0.048mmol), 1,4-dioxane (3ml), and $Cs_2CO_3$ (119mg, 0.36mmol). The reaction was vacuumed and refilled with $N_2$ twice, followed by adding $Pd(dba)_2$ (33mg, 0.036mmol). After that, the reaction was vacuumed and refilled with $N_2$ three times and allowed to proceed at 120°C for 4h The system was cooled, and evaporated directly in the presence of silica gel for loading onto and separating by column chromatography (DCM/MeOH=50/1~30/1) to provide the title compound: 4-((4-cyclopropyl-2-(N-methyl methanesulfonamido)phenyl)amino)-6-((6-fluoropyridin-2-yl)amino)-N-(2,2,2-trifluoroethoxy)nicotinamide **(119,** 30mg, 0.06mmol, 16% yield). MS Calcd: 568; MS Found: 569([M+H]$^+$). $^1$H NMR (400 MHz, DMSO-$d_6$)δ12.14 (s, 1H), 9.98 (s, 1H), 9.79 (s, 1H), 8.31 (s, 1H), 7.79 (dd, *J* = 8.4, 16.4 Hz, 1H), 7.53 (s, 1H), 7.50 - 7.42 (m, 2H), 7.30 (d, *J* = 2.0 Hz, 1H), 7.09 (dd, *J* = 8.8, 2.0 Hz, 1H), 6.56 (dd, *J* = 8.0, 2.8 Hz, 1H), 4.56 (q, *J* = 9.2 Hz, 2H), 3.14 (s, 3H), 3.09 (s, 3H), 1.99 m, 2H), 7.30 (d, (dd, 3H), 2.30 (s, 3H), 2.26 (s, 3H),.

Example **120**

6-(cyclopropylcarboxamido)-4-((4-cyclopropyl-5-methyl-2-(N-methyl methanesulfonamido)phenyl)amino)-N-ethoxy nicotinamide

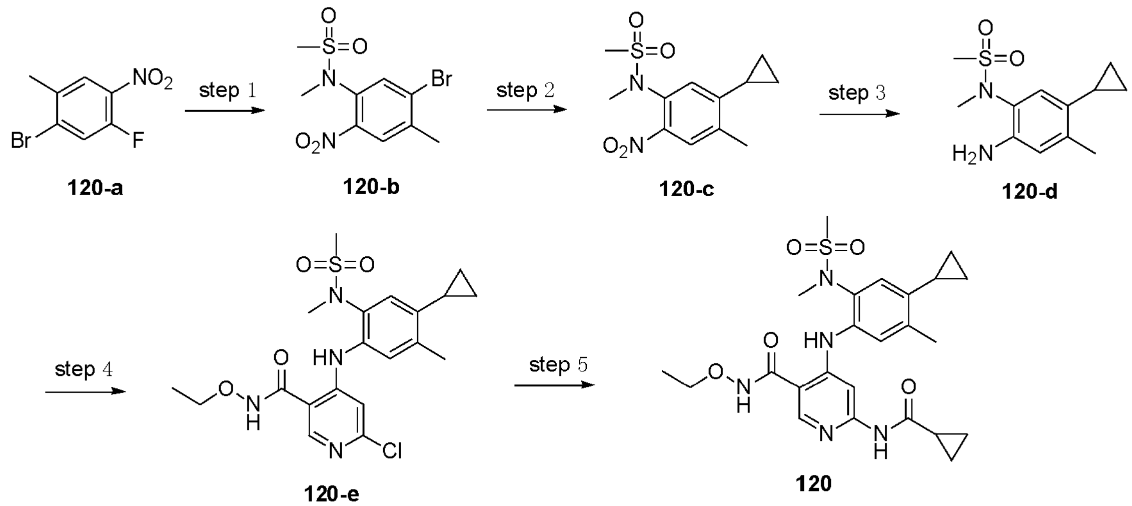


120-a 120-b 120-c 120-d 120-e 120

Step 1: To a 100ml flask were sequentially added 1-bromo-5-fluoro-2-methyl-4-nitrobenzene(**120-a,** 850mg, 3.6mmol), N-methyl methanesulfonamide (437mg, 4.0mmol), anhydrous potassium carbonate (1.5g, 10.8mmol) and *N,N-dimethyl* formamide (20mL). The mixture was stirred at 80°C for 4h. Upon TLC indicated a completed reaction, to the reaction mixture was added 50ml of water for dilution of the mother liquid, and extracted with ethyl acetate three times. The combined organic phases were washed with saturated brine, dried over anhydrous sodium sulfate, removed solvent by

rotary evaporation to provide N-(5-bromo-4-methyl-2-nitrophenyl)-N-methyl methanesulfonamide **(120-b,** 1.2g, 3.7mmol, 98% yield). MS Calcd: 322; MS Found: 323([M+H]+).

Step 2: To a 100ml flask was added N-(5-bromo-4-methyl-2-nitrophenyl)-N-methyl methanesulfonamide **(120-b,** 1.2g, 3.7mmol), followed by cyclopropyl boronic acid(353mg, 4.1mmol), $Cs_2CO_3$ (3.6g, 11.0mmol), Pd(dppf)$Cl_2$ (272mg, 0.37mmol), Diox (20ml), $H_2O$ (4ml). After atmosphere replacement with nitrogen three times, the reaction was allowed to proceed at 100 °C for 5h. Upon indication of completed reaction by TLC, the system was cooled, washed with water, and extracted with ethyl acetate three times. The combined organic layers were dried and concentrated to provide the product N-(5-cyclopropyl-4-methyl-2-nitrophenyl)-N-methyl methanesulfonamide **(120-c,** 1.0g, 3.5mmol, 95% yield). MS Calcd: 284; MS Found: 285([M+H]+.

Step 3: To a 100ml flask were added N-(5-cyclopropyl-4-methyl-2-nitrophenyl)-N-methyl methanesulfonamide **(120-c,** 1.0g, 3.5mmol), palladium on carbon (10%) (100mg, 0.35mmol) and methanol (20ml). The reaction was subjected to atmosphere replacement by $H_2$, and bubbled with hydrogen to react for 4 hours. When TLC indicated a completed reaction, the system was filtered, and concentrated to obtain N-(2-amino-5-cyclopropyl-4-methyl phenyl)-N-methyl methanesulfonamide **(120-d,** 700mg, 2.8mmol, 95% yield).

Step 4: To a 100ml flask were added N-(2-amino-5-cyclopropyl-4-methyl phenyl)-N-methyl methanesulfonamide **(120-d,** 700mg, 2.8mmol), 4,6-dichloro-N-ethoxy nicotinamide **(int-2,** 650mg, 2.8mmol), and THF (10ml). After atmosphere replacement with nitrogen three times, the reaction was slowly added with LiHMDS (8.3ml, 8.4mmol) under nitrogen protection and allowed to proceed for 6h at room temperature. Upon indication of completed reaction by TLC (petroleum ether/ethyl acetate=1/2), the system was washed with water and adjusted with 0. 1mol/L HCl to pH=5, and extracted with ethyl acetate three times. The combined organic layers were dried, concentrated in the presence of silica gel for loading onto and separating by column chromatography (petroleum ether/ethyl acetate=2/1~1/1) to provide 6-chloro-4-((4-cyclopropyl-5-methyl-2-(N-methyl methanesulfonamido)phenyl)amino)-N-ethoxy nicotinamide **(120-e,** 600mg, 1.7mmol, 67% yield). MS Calcd: 452; MS Found: 453 ([M+H]+).

Step 5: To a 50ml flask were added 6-chloro-4-((4-cyclopropyl-5-methyl-2-(N-methyl methanesulfonamido) phenyl)amino)-N-ethoxy nicotinamide **(120-e,** 100mg, 0.2mmol), cyclopropylcarboxamide (39mg, 0.4mmol), XantPhos(53mg, 0.08mmol), anhydrous 1,4-dioxane (3ml), and $Cs_2CO_3$ (223mg, 0.6mmol). The reaction was vacuumed and refilled with $N_2$ twice, followed by adding Pd$(dba)_2$ (63mg, 0.06mmol). After that, the reaction was vacuumed and refilled with $N_2$ three times and allowed to proceed at 120°C for 4h. The system was cooled, and evaporated directly in the presence of silica gel for loading onto and separating by column chromatography (DCM/MeOH=50/1~30/1) to provide the title compound: 6-(cyclopropylcarboxamido)-4-((4-cyclopropyl-5-methyl-2-(N-methyl methanesulfonamido)phenyl)amino)-N-ethoxy nicotinamide **(120,** 60mg, 0.12mmol, 60% yield). MS Calcd: 501; MS Found: 502 ([M+H]+). $^1$H NMR (400 MHz, DMSO-$d_6$)δ11.64 (s, 1H), 10.72 (s, 1H), 9.82 (s, 1H), 8.32 (s, 1H), 7.83 (s, 1H), 7.26 (s, 1H), 7.05 (s, 1H), 3.93 (q, *J* = 7.2 Hz, 2H), 3.09 (s, 3H), 3.02 (s, 3H), 2.35 (s, 3H), 1.99-1.87 (m, 2H), 1.22 (t, *J* = 7.2 Hz, 3H), 0.97 - 0.82 (m, 4H), 0.64 - 0.60 (m, 4H).

Example **121**

4-((4-cyclopropyl-5-methyl-2-(N-methyl methanesulfonamido)phenyl)amino)-6-(((2,6-dimethyl pyrimidin-4-yl)amino)-N-ethoxy nicotinamide

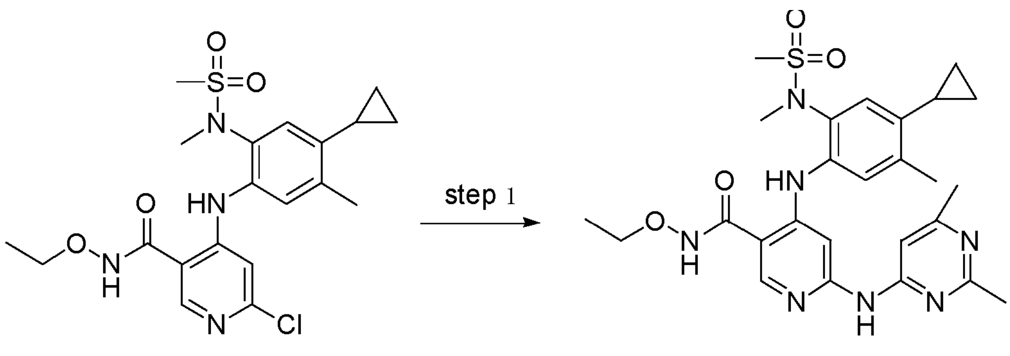


120-e 121

Step 1: To a 50ml flask were added 6-chloro-4-((4-cyclopropyl-5-methyl-2-(N-methyl methanesulfonamido) phenyl)amino)-N-ethoxy nicotinamide **(120-e,** 100mg, 0.2mmol), 2,6-dimethyl pyrimidin-4-ylamine (56mg, 0.4mmol), XantPhos(53mg, 0.08mmol), anhydrous 1,4-dioxane (3ml), and $Cs_2CO_3$ (223mg, 0.6mmol). The reaction was vacuumed

and refilled with $N_2$ twice, followed by adding $Pd(dba)_2$ (63mg, 0.06mmol). After that, the reaction was vacuumed and refilled with $N_2$ three times and allowed to proceed at 120°C for 4h. The system was cooled, and evaporated directly in the presence of silica gel for loading onto and separating by column chromatography (DCM/MeOH=50/1~30/1) to provide the title compound: 4-((4-cyclopropyl-5-methyl-2-(N-methyl methanesulfonamido)phenyl)amino)-6-(((2,6-dimethyl pyrimidin-4-yl)amino)-N-ethoxy nicotinamide **(121,** 60mg, 0.12mmol, 60% yield). MS Calcd: 539; MS Found: 540 ($[M+H]^+$). $^1H$ NMR (400 MHz, DMSO-$d_6$)δ11.58 (s, 1H), 10.00 (s, 1H), 9.76 (s, 1H), 8.32 (s, 1H), 7.62 (s, 1H), 7.31 (s, 1H), 7.23 (s, 1H), 7.09 (s, 1H), 3.93 (q, *J* = 7.2 Hz, 2H), 3.10 (s, 3H), 3.03 (s, 3H), 2.40 (s, 3H), 2.27 (s, 3H), 2.25 (s, 3H), 1.94-1.87 (m, 1H), 1.22 (t, *J* = 7.2 Hz, 3H), 0.97-0.91 (m, 2H), 0.72 - 0.60 (m, 2H).

Example **122**

4-((4-cyclopropyl-2-(N-methyl methanesulfonamido)phenyl)amino)-N-ethoxy-6-((5-(hydroxymethyl)pyridin-2-yl)amino) nicotinamide

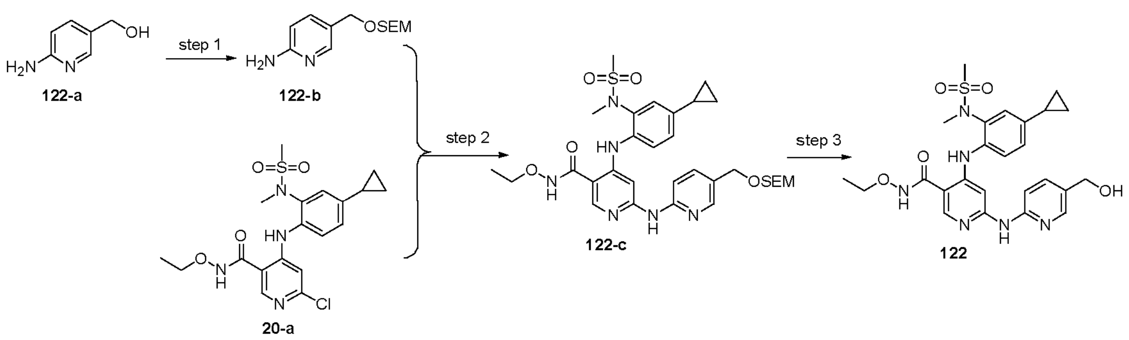


Step 1: 5-hydroxymethyl-2-aminopyridine **(122-a,** 300mg, 2.42mmol) was dissolved in DCM (10mL). The mixture was cooled in ice-water bath and slowly added with sodium hydride(447.7mg, 12mmol). After stirring for 0.5h, the mixture was added with SEMCl (803.22mg, 4.84mmol) and allowed to react at room temperature for 3h. The reaction mixture was added with 30ml of water, and extracted with 20ml of DCM three times. The organic layers are concentrated to provide a crude material, which was separated and purified by normal phase silica gel column chromatography and eluted with petroleum ether:ethyl acetate=1:1. The fractions containing product were concentrated to obtain 5-((((2-(trimethylsilyl)ethoxy)methoxy)methyl)pyridin-2-ylamine **(122-b,** 260 mg, 1.02mmol, 42.15% yield). MS Calcd: 254; MS Found: 255($[M+H]^+$).

Step 2: 5-((((2-(trimethylsilyl)ethoxy)methoxy)methyl)pyridin-2-ylamine **(122-b,** 260 mg, 1.02mmol), 6-chloro-4-((4-cyclopropyl-2-(N-methyl methanesulfonamido)phenyl)amino)-N-ethoxy nicotinamide **(20-a,** 437mg, 1.00mmol), cesium carbonate (652mg, 2.00mmol), XantPhos(115.6mg, 0.1mmol) and $Pd_2(dba)_3$(91.5mg, 0.1mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated, and subjected to high performance preparative thin layer chromatography to provide 4-((4-cyclopropyl-2-(N-methyl methanesulfonamido)phenyl)amino)-N-ethoxy-6-((5-((((2-(trimethylsilyl)ethoxy)methoxy)methyl)pyridin-2-yl)amino)nicotinamide **(122-c,** 100mg, 0.15mmol, 15% yield). MS Calcd: 656; MS Found: 657 ($[M+H]^+$).

Step 3: 4-((4-cyclopropyl-2-(N-methyl methanesulfonamido)phenyl)amino)-N-ethoxy-6-((5-((((2-(trimethylsilyl) ethoxy)methoxy)methyl)pyridin-2-yl)amino)nicotinamide **(122-c,** 100mg, 0.15mmol) was dissolved in 2mL of ethanol. After adding 2ml of concentrated aqueous hydrochloride, the mixture was heated to 80°C refluxing for 4h, and subjected to high performance preparative thin layer chromatography (DCM:MeOH:AcOH=15:1:1) to provide the title compound: 4-((4-cyclopropyl-2-(N-methyl methanesulfonamido)phenyl)amino)-N-ethoxy-6-((5-(hydroxymethyl)pyridin-2-yl)amino) nicotinamide **(122,** 15mg, 0.029mmol, 19.04% yield). MS Calcd: 526; MS Found: 527 ($[M+H]^+$).

Example **123**

4-((4-cyclopropyl-2-(N-methyl methanesulfonamido)phenyl)amino)-6-((4,6-dimethyl pyrimidin-2-yl)amino)-N-ethoxy nicotinamide

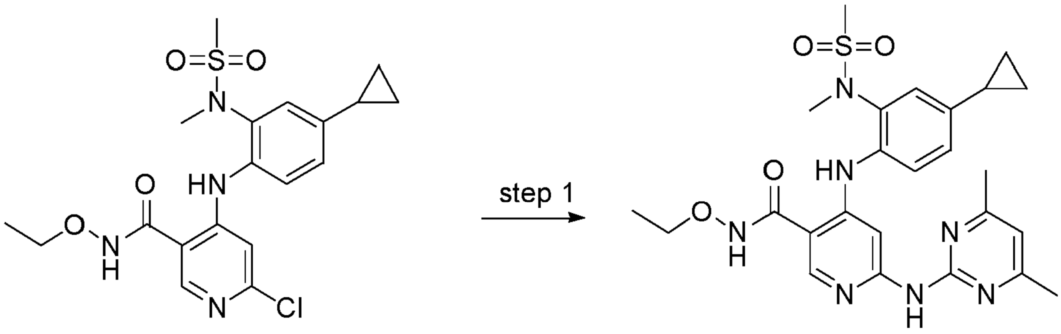


20-a

123

Step 1: To a 50ml flask were added 6-chloro-4-((4-cyclopropyl-2-(N-methyl methanesulfonamido)phenyl)amino)-N-ethoxy nicotinamide **(20-a,** 100mg, 0.2mmol), 4,6-dimethyl pyrimidin-2-ylamine (56mg, 0.4mmol), XantPhos(53mg, 0.08mmol), anhydrous 1,4-dioxane (3ml), and $Cs_2CO_3$ (223mg, 0.6mmol). The reaction was vacuumed and refilled with $N_2$ twice, followed by adding $Pd(dba)_2$ (63mg, 0.06mmol). After that, the reaction was vacuumed and refilled with $N_2$ three times and allowed to proceed at 120°C for 4h. The system was cooled, and evaporated directly in the presence of silica gel for loading onto and separating by column chromatography (DCM/MeOH=50/1~30/1) to provide the title compound: 4-((4-cyclopropyl-2-(N-methyl methanesulfonamido)phenyl)amino)-6-((4,6-dimethyl pyrimidin-2-yl)amino)-N-ethoxy nicotinamide **(123,** 60mg, 0.12mmol, 60% yield). MS Calcd: 525; MS Found: 526 ($[M+H]^+$). $^1H$ NMR (400 MHz, DMSO-$d_6$)δ11.59 (s, 1H), 9.88 (s, 1H), 9.56 (s, 1H), 8.32 (s, 1H), 8.16 (s, 1H), 7.48 (d, *J* = 8.4 Hz, 1H), 7.31 (d, *J* = 2.0 Hz, 1H), 7.12 (dd, *J* = 8.4, 2.0 Hz, 1H), 6.71 (s, 1H), 3.94 (q, *J* = 7.2 Hz, 2H), 3.12 (s, 3H), 3.09 (s, 3H), 2.24 (s, 6H), 2.00-1.95 (m, 1H), 1.23 (q, *J* = 7.2 Hz, 3H), 1.02 - 0.94 (m, 2H), 0.75 - 0.68 (m, 2H).

Example **124**

4-((4-cyclopropyl-2-(N-methyl methanesulfonamido)phenyl)amino)-N-ethoxy-6-(pyrimidin-2-ylamino)nicotinamide

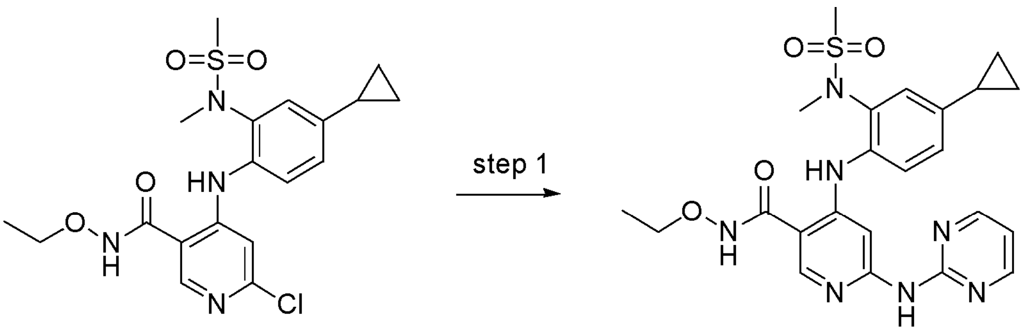


20-a

124

Step 1: To a 50ml flask were added 6-chloro-4-((4-cyclopropyl-2-(N-methyl methanesulfonamido)phenyl)amino)-N-ethoxy nicotinamide **(20-a,** 100mg, 0.2mmol), pyrimidin-2-ylamine (43mg, 0.4mmol), XantPhos(53mg, 0.08mmol), anhydrous 1,4-dioxane (3ml), and $Cs_2CO_3$ (223mg, 0.6mmol). The reaction was vacuumed and refilled with $N_2$ twice, followed by adding $Pd(dba)_2$ (63mg, 0.06mmol). After that, the reaction was vacuumed and refilled with $N_2$ three times and allowed to proceed at 120°C for 4h. The system was cooled, and evaporated directly in the presence of silica gel for loading onto and separating by column chromatography (DCM/MeOH=50/1~30/1) to provide the title compound: 4-((4-cyclopropyl-2-(N-methyl methanesulfonamido)phenyl)amino)-N-ethoxy-6-(pyrimidin-2-ylamino)nicotinamide **(124,** 60mg, 0.12mmol, 60% yield). MS Calcd: 497; MS Found: 498 ($[M+H]^+$). $^1H$ NMR (400 MHz, DMSO-$d_6$)δ11.63 (s, 1H), 10.01 (s, 1H), 9.91 (s, 1H), 8.52 (s, 1H), 8.51 (s, 1H), 8.34 (s, 1H), 8.17 (s, 1H), 7.54 (d, *J* = 8.4 Hz, 1H), 7.27 (d, *J* = 2.0 Hz, 1H), 7.17 (d, *J* = 2.4 Hz, 0H), 6.96 (t, *J* = 4.8 Hz, 1H), 3.95 (q, *J* = 7.2 Hz, 2H), 3.14 (s, 3H), 3.11 (s, 3H), 2.00-1.94 (m, 1H), 1.23 (t, *J* = 7.2 Hz, 3H), 1.00-0.97 (m, 2H), 0.76-0.72 (m, 2H).

Example **125**

4-((4-cyclopropyl-2-(N-methyl methanesulfonamido)phenyl)amino)-N-ethoxy-6-((5-fluoro-4-methyl pyridin-2-yl)amino) nicotinamide

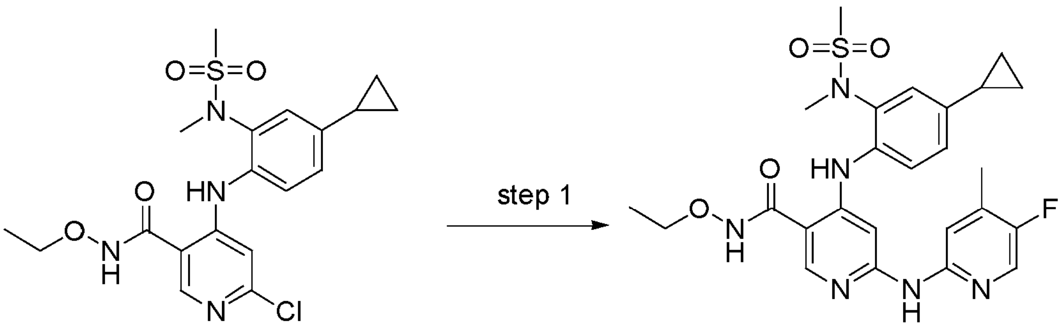


20-a 125

Step 1: 6-chloro-4-((4-cyclopropyl-2-(N-methyl methanesulfonamido)phenyl)amino)-N-ethoxy nicotinamide **(20-a,** 60mg, 0.13mmol), 5-fluoro-4-methyl pyridin-2-ylamine (18.9mg, 0.15mmol), cesium carbonate (132mg, 0.40mmol), XantPhos(23mg, 0.04mmol) and $Pd_2(dba)_3$(25mg, 0.02mmol) were added to anhydrous dioxane (5ml). After atmosphere replacement with nitrogen three times, the reaction mixture was heated to 120°C with stirring for 8h, and filtered by suction. The filtrate was concentrated and purified by high performance preparative thin layer chromatography (MeOH:DCM=1:20) to provide 4-((4-cyclopropyl-2-(N-methyl methanesulfonamido)phenyl)amino)-N-ethoxy-6-((5-fluoro-4-methyl pyridin-2-yl)amino)nicotinamide **(125,** 10mg, 0.018mmol, 14% yield). MS Calcd: 528; MS Found: 529 ($[M+H]^+$). $^1$H NMR (401 MHz, DMSO-$d_6$)δ11.51 (s, 1H), 9.87 (s, 1H), 9.63 (s, 1H), 8.30 (s, 1H), 8.03 (s, 1H), 7.57 (d, *J* = 5.6 Hz, 1H), 7.46-7.39 (m, 2H), 7.26 (d, *J* = 2.0 Hz, 1H), 7.15 (dd, *J* = 8.0, 2.0 Hz, 1H), 3.93 (q, *J* = 7.2 Hz, 2H), 3.14 (s, 3H), 3.10 (s, 3H), 2.23 (s, 3H), 2.00 - 1.95 (m, 1H), 1.22 (t, *J* = 7.2 Hz, 3H), 1.00 - 0.97 (m, 2H), 0.75 - 0.71 (m, 2H).

Example **126**

6-((5-cyano pyridin-2-yl)amino)-4-((4-cyclopropyl-2-(N-methyl methanesulfonamido)phenyl)amino)-N-ethoxy nicotinamide

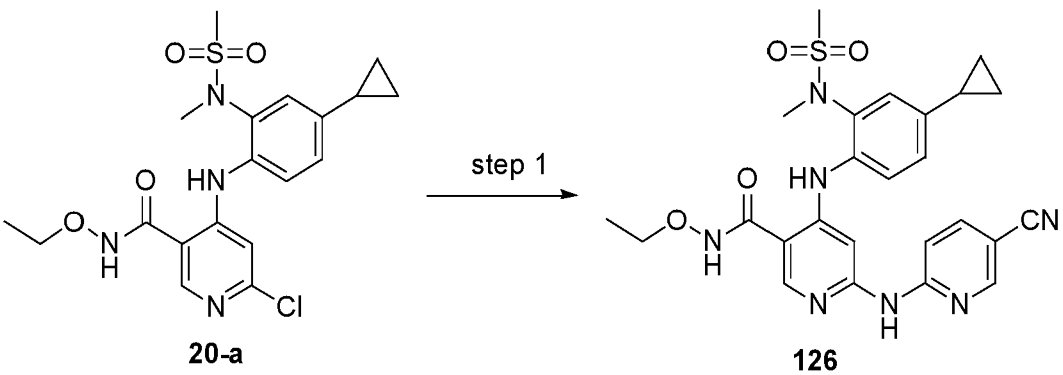


20-a 126

Step 1: 6-chloro-4-((4-cyclopropyl-2-(N-methyl methanesulfonamido)phenyl)amino)-N-ethoxy nicotinamide **(20-a,** 60mg, 0.13mmol), 6-aminonicotinonitrile(17.85mg, 0.15mmol), cesium carbonate (132mg, 0.40mmol), XantPhos(23mg, 0.04mmol) and $Pd_2(dba)_3$(25mg, 0.02mmol) were added to anhydrous dioxane (5ml). After atmosphere replacement with nitrogen three times, the reaction mixture was heated to 120°C with stirring for 8h, and filtered by suction. The filtrate was concentrated and purified by high performance preparative thin layer chromatography (MeOH:DCM=1:15) to provide 6-((5-cyano pyridin-2-yl)amino)-4-((4-cyclopropyl-2-(N-methyl methanesulfonamido)phenyl)amino)-N-ethoxy nicotinamide **(126,** 13mg, 0.023mmol, 18% yield). MS Calcd: 521; MS Found: 522($[M+H]^+$). $^1$H NMR (400 MHz, DMSO-d6)δ11.62 (s, 1H), 10.30 (s, 1H), 9.90 (s, 1H), 8.60 (d, *J* = 2.3 Hz, 1H), 8.34 (s, 1H), 8.04 (dd, *J* = 8.8, 2.4 Hz, 1H), 7.79 (d, *J* = 8.8 Hz, 1H), 7.54 (s, 1H), 7.45 (d, *J* = 8.4 Hz, 1H), 7.28 (d, *J* = 2.0 Hz, 1H), 7.19 (dd, *J* = 8.4, 2.0 Hz, 1H),

3.94 (q, $J$ = 7.2 Hz, 2H), 3.12 (s, 3H), 3.09 (s, 3H), 2.02 - 1.95 (m, 1H), 1.22 (t, $J$ = 7.2 Hz, 4H), 1.01 - 0.97 (m, 2H), 0.76 - 0.72 (m, 2H).

Example **127**

6-((2,6-dimethyl pyrimidin-4-yl)amino)-N-ethoxy-4-((4-ethynyl-2-(N-methyl methanesulfonamido)phenyl)amino)nicotinamide

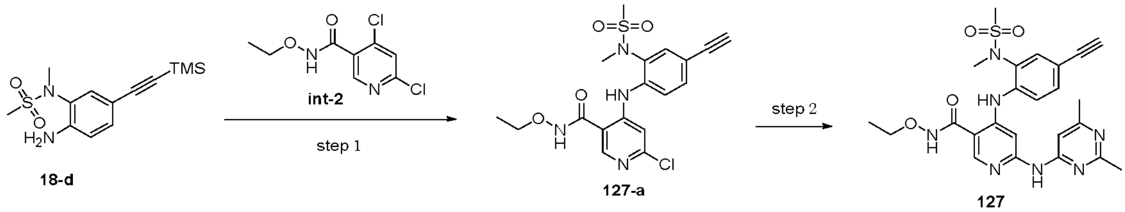


18-d 127-a 127

Step 1: To a 100mL three-necked flask were added 4,6-dichloro-N-ethoxy nicotinamide **(int-2,** 500mg, 2.0mmol) and N-(2-amino-5-((trimethylsilyl)ethynyl)phenyl)-N-methyl methanesulfonamide **(18-d,,** 650mg, 2.19mmol). After adding 25ml of anhydrous DMA, the reaction was vacuumed and refilled with $N_2$ three times, and added with LHMDS (6.5mL, 1mol/L, 0.006mol) dropwise in ice bath. After that the reaction was allowed to proceed at room temperature for 6 hours, and sampled. When TLC indicated a completed reaction, the reaction mixture was added with water and adjusted with diluted aqueous hydrochloride to pH 4-5 in ice bath, and extracted with ethyl acetate. The organic phase was mixed with 200-300 mesh silica gel for loading, and separated and purified by column chromatography, eluted with PE:EA=5:1 gradually changing to PE:EA=2:1, to provide 6-chloro-N-ethoxy-4-((4-ethynyl-2-(N-methyl methanesulfonamido)phenyl)amino) nicotinamide **(127-a,** 375mg, 0.888mmol, 44% yield), as a pale yellow foamy powder. MS Calcd: 422; MS Found: 423($[M+H]^+$)

Step 2: 6-chloro-N-ethoxy-4-((4-ethynyl-2-(N-methyl methanesulfonamido)phenyl)amino)nicotinamide **(127-a,** 103mg, 0.24mmol), 2,6-dimethyl pyrimidin-4-ylamine (44mg, 0.36mmol), cesium carbonate (234mg, 0.72mmol), Xant-Phos(70mg, 0.12mmol) and $Pd_2(dba)_3$(113mg, 0.12mmol) were added to anhydrous dioxane (5ml). After atmosphere replacement with nitrogen three times, the reaction was heated to 120°C with stirring for 8h, and filtered by suction. The filtrate was concentrated and purified by high performance preparative thin layer chromatography (MeOH:DCM=1:13) to provide the title compound: 6-((2,6-dimethyl pyrimidin-4-yl)amino)-N-ethoxy-4-((4-ethynyl-2-(N-methyl methanesulfonamido)phenyl)amino)nicotinamide **(127,** 13mg, 0.024mmol, 10 % yield). MS Calcd: 509; MS Found: 510($[M+H]^+$). $^1$HNMR (400 MHz, DMSO-$d6$)$\delta$11.69 (s, 1H), 10.29 (s, 1H), 10.12 (s, 1H), 8.38 (s, 1H), 8.08 (s, 1H), 7.74 - 7.63 (m, 2H), 7.52 (dd, $J$ = 8.4, 2.0 Hz, 1H), 7.11 (s, 1H), 4.24 (s, 1H), 3.94 (q, $J$ = 7.2 Hz, 2H), 3.17 (s, 6H), 2.39 (s, 3H), 2.29 (s, 3H), 1.22 (t, $J$ = 7.2 Hz, 3H).

Example **128**

4-((4-chloro-2-(N-methyl methanesulfonamido)phenyl)amino)-N-ethoxy-6-((5-fluoropyridin-2-yl)amino)nicotinamide

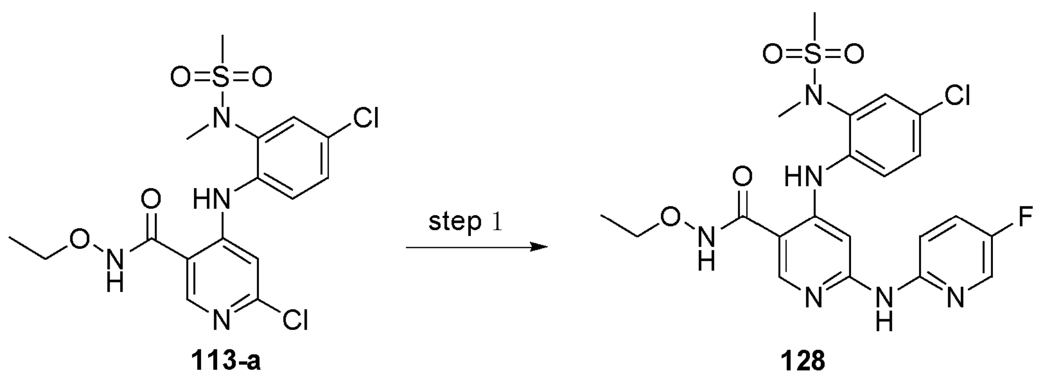


**113-a** **128**

Step 1: 6-chloro-4-((4-chloro-2-((N-methyl methanesulfonamido)phenyl)amino)-N-ethoxy nicotinamide **(113-a,** 150mg, 0.34mmol), 5-fluoropyridin-2-ylamine (58mg, 0.52mmol), cesium carbonate (442mg, 1.36mmol), Xant-Phos(58mg, 0.1mmol) and $Pd_2(dba)_3$(160mg, 0.17mmol) were added to anhydrous dioxane (5ml). After atmosphere replacement with nitrogen three times, the reaction mixture was heated to 120°C with stirring for 8h, and filtered by suction. The filtrate was concentrated and purified by high performance preparative thin layer chromatography (MeOH:DCM=1:15) to provide the title compound: 4-((4-chloro-2-(N-methyl methanesulfonamido)phenyl)amino)-N-ethoxy-6-((5-fluoropyridin-2-yl)amino)nicotinamide **(128,** 30mg, 0.057mmol, 17 % yield). MS Calcd: 508; MS Found: 509 ($[M+H]^+$). $^1H$ NMR (400 MHz, DMSO-$d_6$)δ11.62 (s, 1H), 10.09 (s, 1H), 9.85 (s, 1H), 8.33 (s, 1H), 8.20 (d, *J* = 2.4 Hz, 1H), 7.69 - 7.55 (m, 6H), 3.95 (t, *J*= 7.2 Hz, 2H), 3.17 (s, 3H), 3.16 (s, 3H), 1.23 (t, *J*= 7.2 Hz, 3H).

Example **129**

4-((4-chloro-2-(N-methyl methanesulfonamido)phenyl)amino)-N-ethoxy-6-((6-fluoropyridin-2-yl)amino)nicotinamide

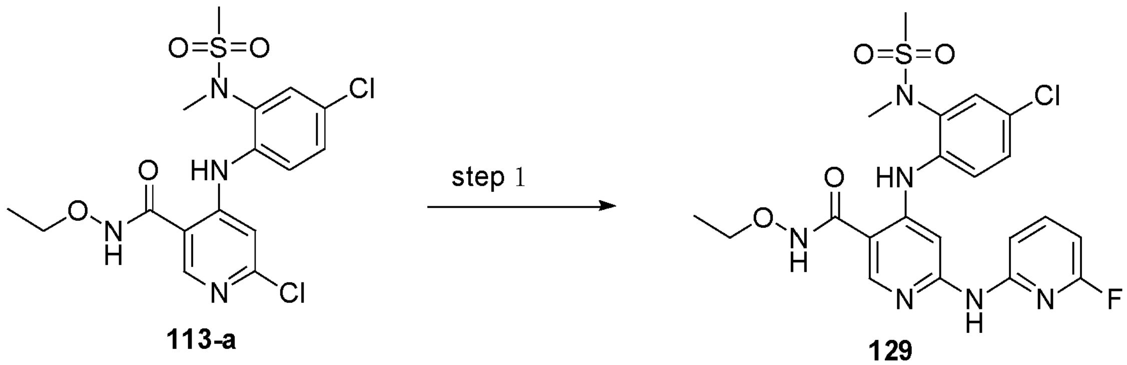


**113-a** **129**

Step 1: 6-chloro-4-((4-chloro-2-((N-methyl methanesulfonamido)phenyl)amino)-N-ethoxy nicotinamide **(113-a,** 150mg, 0.34mmol), 6-fluoropyridin-2-ylamine (58mg, 0.52mmol), cesium carbonate (442mg, 1.36mmol), Xant-Phos(58mg, 0.1mmol) and $Pd_2(dba)_3$(160mg, 0.17mmol) were added to anhydrous dioxane (5ml). After atmosphere replacement with nitrogen three times, the reaction was heated to 120°C with stirring for 8h, and filtered by suction. The filtrate was concentrated and purified by high performance preparative thin layer chromatography (MeOH:DCM=1:15) to provide the title compound: 4-((4-chloro-2-(N-methyl methanesulfonamido)phenyl)amino)-N-ethoxy-6-((6-fluoropyridin-2-yl)amino)nicotinamide **(129,** 38mg, 0.07mmol, 21 % yield). MS Calcd: 508; MS Found: 509($[M+H]^+$). $^1H$ NMR (400 MHz, Chloroform-*d*)δ9.97 (s, 1H), 8.29 (s, 1H), 7.85 (s, 1H), 7.68 (d, *J* = 8.8 Hz, 1H), 7.62 (dd, *J* = 16.4, 8.0 Hz, 1H), 7.44 - 7.39 (m, 3H), 6.85 (d, *J* = 8.0 Hz, 1H), 6.44 (dd, *J* = 8.0, 2.8 Hz, 1H), 4.10 (q, *J* = 7.2 Hz, 2H), 3.27 (s, 3H), 3.10 (s, 3H), 1.34 (t, *J* = 7.2 Hz, 3H).

Example **130**

N-ethoxy-6-(((5-fluoropyridin-2-yl)amino)-4-((2-(N-methyl methanesulfonamido)-4-morpholinophenyl)amino)nicotinamide

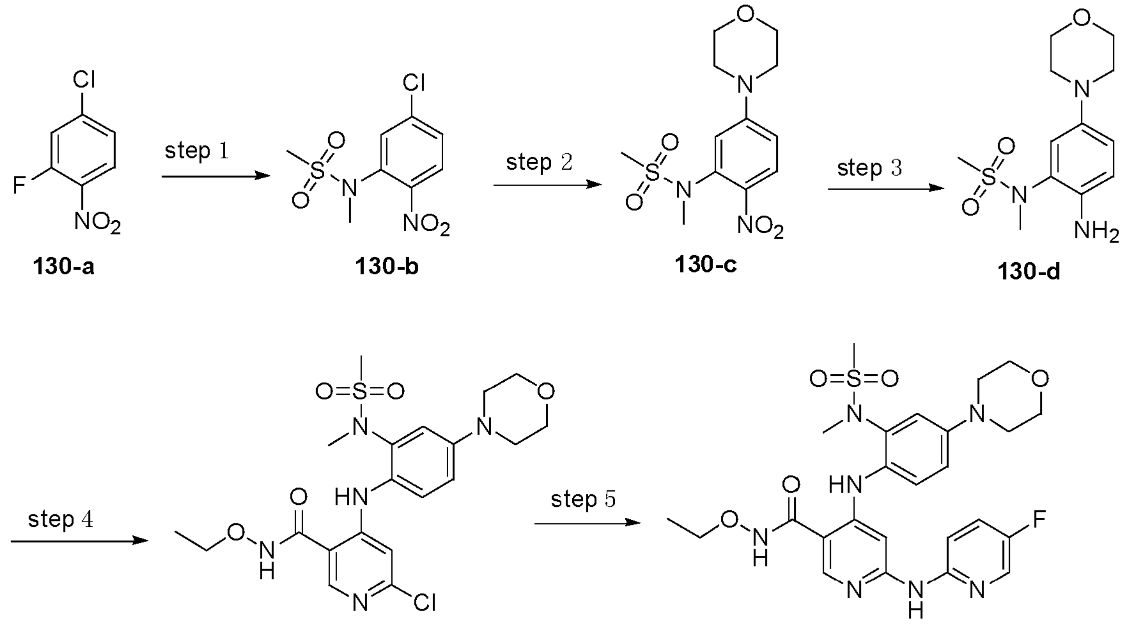


130-a 130-b 130-c 130-d

130-e 130

Step 1: To a 100ml flask were sequentially added 4-chloro-2-fluoro-1-nitrobenzene(**130-a**, 2.5g, 14.3mmol), N-methyl methanesulfonamide (1.8g, 17.16mmol), anhydrous potassium carbonate (3.9g, 28.6mmol) and acetonitrile (30mL), and stirred at 80°C for 6h. Upon indication of completed reaction by TLC, the reaction mixture was added with 20ml of water for dilution of the mother liquid, and extracted with ethyl acetate three times. The combined organic phases were washed with saturated brine, dried over anhydrous sodium sulfate, and subjected to column chromatography to provide N-(5-chloro-2-nitrophenyl)-N-methyl methanesulfonamide **(130-b,** 3.6g, 13.7mmol, 96% yield). MS Calcd: 264; MS Found: 265($[M+H]^+$).

Step 2: Into DMSO (30mL) were dissolved N-(5-chloro-2-nitrophenyl)-N-methyl methanesulfonamide **(130-b,** 600mg, 2.2mmol), morpholine (147mg, 3.34mmol), and anhydrous potassium carbonate (467mg, 6.69mmol) and stirred at 120°C for 4h. Upon indication of completed reaction by TLC, the reaction mixture was added with 20ml of water for dilution of the mother liquid, and extracted with ethyl acetate three times. The combined organic phases were washed with saturated brine, dried over anhydrous sodium sulfate, and subjected to column chromatography to provide N-methyl-N-(5-morpholinyl-2-nitrophenyl) methanesulfonamide(**130-c**, 390mg, 1.2mmol, 54% yield). MS Calcd:315; MS Found: 316($[M+H]^+$).

Step 3: To a 100mL flask were added N-methyl-N-(5-morpholinyl-2-nitrophenyl) methanesulfonamide(**130-c**, 390mg, 1.2mmol), Fe (6.17mmol, 345mg), and ammonium chloride (12.3mmol, 651mg). After adding 20mL of ethanol and 4ml of water, the mixture was stirred at 90°C for 3h. When TLC indicated a completed reaction, the mixture was cooled and added with 50ml of ethyl acetate. The mixture was filtered to remove iron powder, and extracted with ethyl acetate and water. The organic phase was washed with saturated sodium chloride aqueous solution, dried over anhydrous sodium sulfate, followed by solvent removed by rotary evaporation to provide N-(2-amino-5-morpholinophenyl)-N-methyl methanesulfonamide **(130-d,** 340mg, 1.19mmol, 97% yield). MS Calcd: 285; MS Found: 286($[M+H]^+$).

Step 4: To a 100mL two-necked flask were sequentially added 4,6-dichloro-N-ethoxy nicotinamide **(int-2,** 335mg, 1.42mmol), N-(2-amino-5-morpholinophenyl)-N-methyl methanesulfonamide **(130-d,** 340mg, 1.18mmol), and 10ml of anhydrous tetrahydrofuran as solvent. The mixture was added at room temperature with a solution of LiHMDS in tetrahydrofuran (4.7mL, 1mmol/mL), and with continuous stirring at room temperature for 2h. Upon indication of completed reaction by TLC, the mixture was adjusted with aqueous hydrochloride (1N) to pH 5, and extracted with ethyl acetate (30mL). The combined organic layers were dried, concentrated, and purified by column chromatography to provide 6-chloro-N-ethoxy-4-((2-(N-methyl methanesulfonamido)-4-morpholinophenyl)amino)nicotinamide **(130-e,** 200mg, 0.41mmol, 35% yield). MS Calcd: 483; MS Found: 484($[M+H]^+$).

Step 5: 6-chloro-N-ethoxy-4-((2-(N-methyl methanesulfonamido)-4-morpholinophenyl)amino)nicotinamide **(130-e,** 200mg, 0.41mmol), 5-fluoropyridin-2-ylamine (92mg, 0.82mmol), cesium carbonate (400mg, 1.23mmol), XantPhos(47mg, 0.082mmol) and $Pd_2(dba)_3$(77mg, 0.082mmol) were added to anhydrous dioxane (8ml). After atmosphere replacement with nitrogen three times, the reaction mixture was heated to 120°C with stirring for 8h, and filtered by suction. The filtrate was concentrated and purified by high performance preparative thin layer chromatography (MeOH: DCM=1:12) to provide the title compound: N-ethoxy-6-(((5-fluoropyridin-2-yl)amino)-4-((2-(N-methyl methanesulfonamido)-4-morpholinophenyl)amino)nicotinamide **(130,** 80mg, 0.14mmol, 34% yield). MS Calcd: 559; MS Found:

560([M+H]$^+$). $^1$H NMR (400 MHz, DMSO-$d_6$)δ11.50 (s, 1H), 9.70 (s, 1H), 9.65 (s, 1H), 8.27 (s, 1H), 8.11 (d, $J$ = 3.2 Hz, 1H), 7.71 - 7.58 (m, 2H), 7.40 - 7.38 (m, 1H), 7.28 (s, 1H), 7.09 - 7.05 (m, 2H), 3.93 (q, $J$ = 7.2 Hz, 2H), 3.77 (t, $J$ = 4.8 Hz, 4H), 3.19 - 3.14 (m, 4H), 3.13 (s, 3H), 3.07 (s, 3H), 1.22 (t, $J$ = 7.2 Hz, 3H).

Example **131**

6-((2,6-dimethyl pyrimidin-4-yl)amino)-N-ethoxy-4-((4-methoxy-2-(N-methyl methanesulfonamido)phenyl)amino)nicotinamide

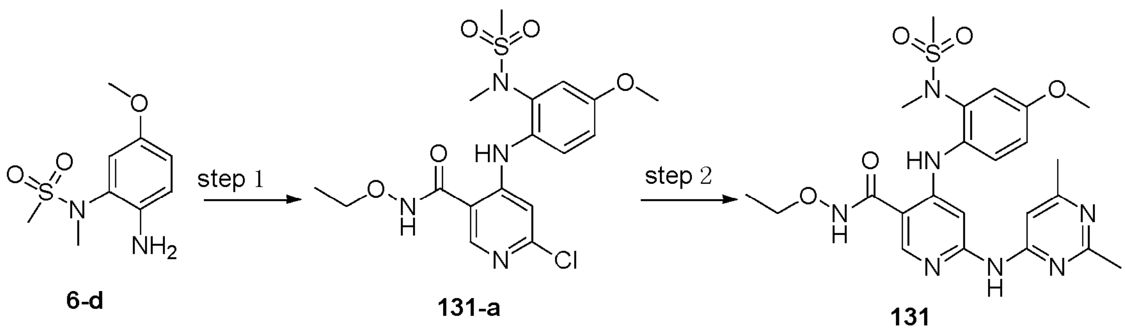


6-d    131-a    131

Step 1: To a 100mL two-necked flask were sequentially added 4,6-dichloro-N-ethoxy nicotinamide **(int-2,** 300mg, 1.2mmol), N-(2-amino-5-methoxy phenyl)-N-methyl methanesulfonamide **(6-d,** 352mg, 1.53mmol), and 10ml of anhydrous tetrahydrofuran as solvent. The mixture was added at room temperature with a solution of LiHMDS in tetrahydrofuran (5mL, 1mmol/mL), with continuous stirring at room temperature for 2h. Upon indication of completed reaction by TLC, the mixture was adjusted with aqueous hydrochloride (1N) to pH 5, and extracted with ethyl acetate (30mL×3). The combined organic layers were dried, concentrated, and purified by column chromatography to provide 6-chloro-N-ethoxy-4-((4-methoxy-2-(N-methyl methanesulfonamido)phenyl)amino)nicotinamide **(131-a,** 260mg, 0.6mmol, 47% yield). MS Calcd: 427; MS Found: 428([M+H]$^+$).

Step 2: 6-chloro-N-ethoxy-4-((4-methoxy-2-(N-methyl methanesulfonamido)phenyl)amino)nicotinamide **(131-a,** 260mg, 0.6mmol), 2,6-dimethyl pyrimidin-4-ylamine (149mg, 1.21mmol), cesium carbonate (585mg, 1.8mmol), XantPhos(70mg, 0.12mmol) and $Pd_2(dba)_3$(113mg, 0.12mmol) were added to anhydrous dioxane (8ml). After atmosphere replacement with nitrogen three times, the reaction mixture was heated to 120°C with stirring for 8h, and filtered by suction. The filtrate was concentrated and purified by high performance preparative thin layer chromatography (MeOH:DCM=1:10) to provide the title compound: 6-((2,6-dimethyl pyrimidin-4-yl)amino)-N-ethoxy-4-((4-methoxy-2-(N-methyl methanesulfonamido)phenyl)amino)nicotinamide (**131,** 85mg, 0.16mmol, 25% yield). MS Calcd: 559; MS Found: 560([M+H]$^+$). $^1$H NMR (400 MHz, DMSO-$d_6$)δ11.56 (s, 1H), 9.94 (s, 1H), 9.64 (s, 1H), 8.31 (s, 1H), 7.62 (s, 1H), 7.44 (d, $J$ = 8.8 Hz, 1H), 7.17 (d, $J$ = 2.8 Hz, 1H), 7.05 (dd, $J$ = 8.8, 2.8 Hz, 1H), 7.01 (s, 1H), 3.94 (q, $J$ = 7.2 Hz, 2H), 3.81 (s, 3H), 3.11 (s, 3H), 3.08 (s, 3H), 2.26 (s, 3H), 2.25 (s, 3H), 1.22 (t, $J$ = 7.2 Hz, 3H).

Example **132**

6-((2,6-dimethyl pyrimidin-4-yl)amino)-N-ethoxy-4-((2-(N-methyl methanesulfonamido)-4-(piperidin-1-yl)phenyl)amino) nicotinamide

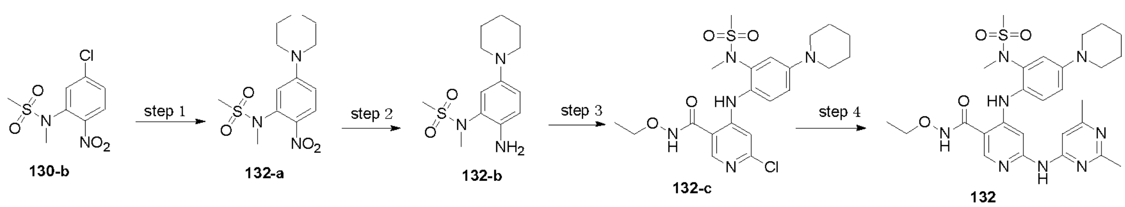


130-b    132-a    132-b    132-c    132

Step 1: Into DMSO(30mL) were dissolved N-(5-chloro-2-nitrophenyl)-N-methyl methanesulfonamide **(130-b,** 900mg, 3.4mmol), piperidine (579mg, 6.8mmol), and anhydrous potassium carbonate (1.4g, 10.2mmol). The mixture was

stirred at 120°C for 4h. Upon indication of completed reaction by TLC, the reaction mixture was added with 20ml of water for dilution of the mother liquid, and extracted with ethyl acetate three times. The combined organic phases were washed with saturated brine, dried over anhydrous sodium sulfate, and subjected to column chromatography to provide N-methyl-N-(2-nitro-5-(piperidin-1-yl)phenyl) methanesulfonamide(**132-a**, 950mg, 3.0mmol, 90% yield). MS Calcd:313; MS Found: 314([M+H]+).

Step 2: To a 100mL flask were added N-methyl-N-(2-nitro-5-(piperidin-1-yl)phenyl) methanesulfonamide **(132-a,** 950mg, 3.0mmol), Fe(15.17mmol, 840mg), and ammonium chloride (30mmol, 1.6g). The mixture was added with 20mL of ethanol and 4ml of water, and stirred at 90°C for 3h. When TLC indicated a completed reaction, the mixture was cooled and added with 50ml of ethyl acetate. The mixture was filtered to remove iron powder, and extracted with ethyl acetate and water. The organic phase was washed with saturated sodium chloride aqueous solution, dried over anhydrous sodium sulfate. followed by solvent removed by rotary evaporation to provide N-(2-amino-5-(piperidin-1-yl)phenyl)-N-methyl methanesulfonamide **(132-b,** 720mg, 2.5mmol, 84% yield). MS Calcd: 283; MS Found: 284([M+H]+).

Step 3: To a 100mL two-necked flask were sequentially added 4,6-dichloro-N-ethoxy nicotinamide **(int-2,** 300mg, 1.2mmol), N-(2-amino-5-(piperidin-1-yl)phenyl)-N-methyl methanesulfonamide **(132-b,** 543mg, 1.9mmol), and 10ml of anhydrous tetrahydrofuran as solvent. The mixture was added at room temperature with a solution of LiHMDS in tetrahydrofuran (3.8ml, 1mmol/L), with continuous stirring at room temperature for 2h. Upon indication of completed reaction by TLC, the mixture was adjusted with aqueous hydrochloride (1N) to pH 5, and extracted with ethyl acetate (30mLx3). The combined organic layers were dried and concentrated, followed by purified by column chromatography to provide 6-chloro-N-ethoxy-4-((2-(N-methyl methanesulfonamido)-4-(piperidin-1-yl)phenyl)amino)nicotinamide **(132-c,** 110mg, 0.22mmol, 18% yield). MS Calcd: 482; MS Found: 483([M+H]+).

Step 4: 6-chloro-N-ethoxy-4-((2-(N-methyl methanesulfonamido)-4-(piperidin-1-yl)phenyl)amino)nicotinamide **(132-c,** 110mg, 0.2mmol), 2,6-dimethyl pyrimidin-4-ylamine (42mg, 0.34mmol), cesium carbonate (195mg, 0.6mmol), XantPhos(23mg, 0.004mmol) and $Pd_2(dba)_3$(40mg, 0.04mmol) were added to anhydrous dioxane (8ml). After atmosphere replacement with nitrogen three times, the reaction mixture was heated to 120 °C with stirring for 8h, and filtered by suction. The filtrate was concentrated and purified by high performance preparative thin layer chromatography (MeOH:DCM=1:15) to provide the title compound: 6-((2,6-dimethyl pyrimidin-4-yl)amino)-N-ethoxy-4-((2-(N-methyl methanesulfonamido)-4-(piperidin-1-yl)phenyl)amino)nicotinamide **(132,** 20mg, 0.035mmol, 18% yield). MS Calcd: 568; MS Found: 569([M+H]+). $^1$HNMR (400 MHz, DMSO-$d_6$)δ11.53 (s, 1H), 9.92 (s, 1H), 9.56 (s, 1H), 8.29 (s, 1H), 7.62 (s, 1H), 7.31 (d, *J* = 8.8 Hz, 1H), 7.08 (d, *J* = 2.8 Hz, 1H), 7.03 - 6.95 (m, 2H), 3.93 (q, *J* = 7.2 Hz, 2H), 3.17 (t, *J* = 5.2 Hz, 4H), 3.11 (s, 3H), 3.05 (s, 3H), 2.27 (s, 3H), 2.25 (s, 3H), 1.70 - 1.50 (m, 6H), 1.22 (t, *J* = 7.2 Hz, 3H).

Example **133**

6-((2,6-dimethyl pyrimidin-4-yl)amino)-N-ethoxy-4-((4-methyl-2-(N-methyl methanesulfonamido)phenyl)amino)nicotinamide

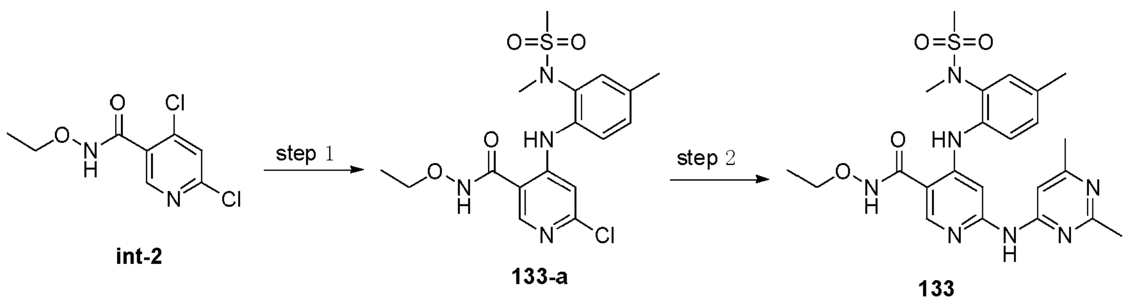


Step 1: To a 100mL two-necked flask were sequentially added 4,6-dichloro-N-ethoxy nicotinamide **(int-2,** 400mg, 1.78mmol), N-(2-amino-5-methyl phenyl)-N-methyl methanesulfonamide (440g, 2.06mmol), and 5ml of anhydrous DMA as solvent. The mixture was added at room temperature with a solution of LiHMDS in tetrahydrofuran (5ml, 1mmol/L), with continuous stirring at room temperature for 2h. Upon indication of completed reaction by TLC, the mixture was adjusted with aqueous hydrochloride (1N) to pH 5, and extracted with ethyl acetate (30mLx3). The combined organic layers were dried, concentrated, and purified by column chromatography to provide 6-chloro-N-ethoxy-4-((4-methyl-2-(N-methyl methanesulfonamido)phenyl)amino)nicotinamide **(133-a,** 500mg, 1.2mmol, 68% yield). MS Calcd: 412; MS Found: 413([M+H]+).

Step 2: 6-chloro-N-ethoxy-4-((4-methyl-2-(N-methyl methanesulfonamido)phenyl)amino)nicotinamide **(133-a,** 200mg, 0.48mmol), 4-amino-2,6-dimethyl pyrimidine (89mg, 0.72mmol), cesium carbonate (468mg, 1.44mmol), Xant-

Phos(50mg, 0.096mmol) and $Pd_2(dba)_3$(90mg, 0.096mmol) were added to anhydrous dioxane (5ml). After atmosphere replacement with nitrogen three times, the reaction mixture was heated to 120°C with stirring for 8h, and filtered by suction. The filtrate was concentrated and purified by high performance preparative thin layer chromatography (MeOH:DCM=1:15) to provide the title compound: 6-((2,6-dimethyl pyrimidin-4-yl)amino)-N-ethoxy-4-((4-methyl-2-(N-methyl methanesulfonamido)phenyl)amino)nicotinamide **(133,** 32mg, 0.06mmol, 12% yield). MS Calcd: 499; MS Found: 500([M+H]$^+$). $^1$H NMR (400 MHz, DMSO-$d_6$)δ11.60 (s, 1H), 9.99 (s, 1H), 9.89 (s, 1H), 8.33 (s, 1H), 7.83 (s, 1H), 7.47 (d, $J$ = 8.4 Hz, 1H), 7.40 (d, $J$ = 2.0 Hz, 1H), 7.25 (dd, $J$ = 8.4, 2.0 Hz, 1H), 7.05 (s, 1H), 3.94 (q, $J$ = 7.2 Hz, 2H), 3.12 (s, 3H), 3.10 (s, 3H), 2.34 (s, 3H), 2.32 (s, 3H), 2.26 (s, 3H), 1.22 (t, $J$ = 7.2 Hz, 3H).

Example **134**

6-((2,6-dimethyl pyrimidin-4-yl)amino)-N-ethoxy-4-((2-(N-methyl methanesulfonamido)-4-morpholinophenyl)amino)nicotinamide

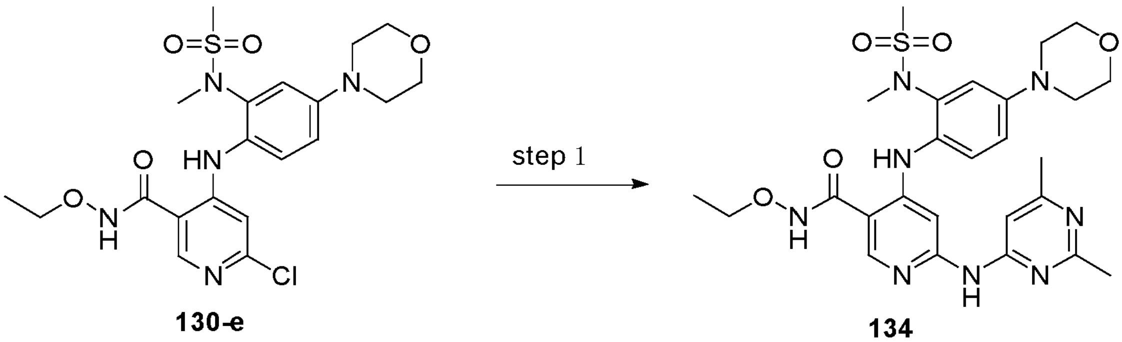


**130-e** **134**

Step 1: 6-chloro-N-ethoxy-4-((2-(N-methyl methanesulfonamido)-4-morpholinophenyl)amino)nicotinamide **(130-e,** 120mg, 0.24mmol), 2,6-dimethyl pyrimidin-4-ylamine (45mg, 0.37mmol), cesium carbonate (234mg, 0.72mmol), XantPhos(27mg, 0.048mmol) and $Pd_2(dba)_3$(45mg, 0.048mmol) were added to anhydrous dioxane (8ml). After atmosphere replacement with nitrogen three times, the reaction mixture was heated to 120°C with stirring for 8h, and filtered by suction. The filtrate was concentrated and purified by high performance preparative thin layer chromatography (MeOH:DCM=1:12) to provide the title compound: 6-((2,6-dimethyl pyrimidin-4-yl)amino)-N-ethoxy-4-((2-(N-methyl methanesulfonamido)-4-morpholinophenyl)amino)nicotinamide **(134,** 20mg, 0.14mmol, 15% yield). MS Calcd: 570; MS Found: 571([M+H]$^+$). $^1$H NMR (400 MHz, DMSO-$d_6$)δ11.54 (s, 1H), 9.92 (s, 1H), 9.60 (s, 1H), 8.30 (s, 1H), 7.63 (s, 1H), 7.36 (d, $J$ = 8.8 Hz, 1H), 7.12 (d, $J$ = 2.8 Hz, 1H), 7.06 - 6.99 (m, 2H), 3.93 (q, $J$ = 7.2 Hz, 2H), 3.76 (t, $J$ = 4.8 Hz, 4H), 3.18 - 3.13 (m, 4H), 3.11 (s, 3H), 3.06 (s, 3H), 2.27 (s, 3H), 2.25 (s, 3H), 3.76 (t, $J$ = 7.2 Hz, 3H).

Example **135**

N-ethoxy-6-(((1-methyl-1H-pyrazol-5-yl)amino)-4-((2-(N-methyl methanesulfonamido)-4-morpholinophenyl)amino)nicotinamide

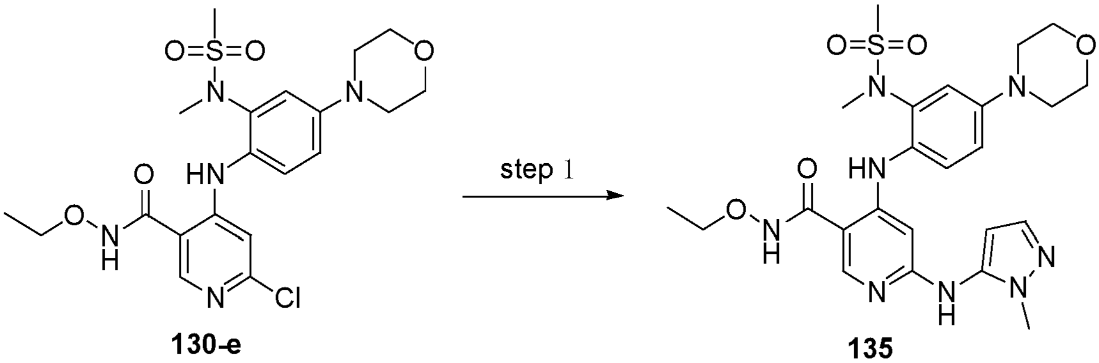


**130-e** **135**

6-chloro-N-ethoxy-4-((2-(N-methyl methanesulfonamido)-4-morpholinophenyl)amino)nicotinamide **(130-e,** 120mg, 0.24mmol), 1-methyl-1H-pyrazol-5-ylamine (35mg, 0.36mmol), cesium carbonate (234mg, 0.72mmol), XantPhos(27mg, 0.048mmol) and $Pd_2(dba)_3$(45mg, 0.048mmol) were added to anhydrous dioxane (8ml). After atmosphere replacement with nitrogen three times, the reaction mixture was heated to 120°C with stirring for 8h, and filtered by suction. The filtrate was concentrated and purified by high performance preparative thin layer chromatography (MeOH:DCM=1:12) to provide the title compound: N-ethoxy-6-(((1-methyl-1H-pyrazol-5-yl)amino)-4-((2-(N-methyl methanesulfonamido)-4-morpholinophenyl)amino)nicotinamide **(135,** 18mg, 0.03mmol, 13% yield). MS Calcd: 544; MS Found: 545($[M+H]^+$). $^1H$ NMR (400 MHz, DMSO-$d_6$)δ11.46 (s, 1H), 9.50 (s, 1H), 8.74 (s, 1H), 8.18 (s, 1H), 7.34 - 7.22 (m, 2H), 7.07 (d, *J* = 2.8 Hz, 1H), 6.99 (dd, *J* = 8.8, 2.8 Hz, 1H), 6.13 (d, *J* = 2.0 Hz, 2H), 3.91 (q, *J* = 7.2 Hz, 2H), 3.75 (t, *J* = 4.8 Hz, 4H), 3.60 (s, 3H), 3.14 (t, *J* = 4.8 Hz, 4H), 3.11 (s, 3H), 3.06 (s, 3H), 1.20 (t, *J* = 7.2 Hz, 3H).

Example **136**

N-ethoxy-4-((2-(N-methyl methanesulfonamido)-4-morpholinophenyl)amino)-6-(pyrazin-2-ylamino)nicotinamide

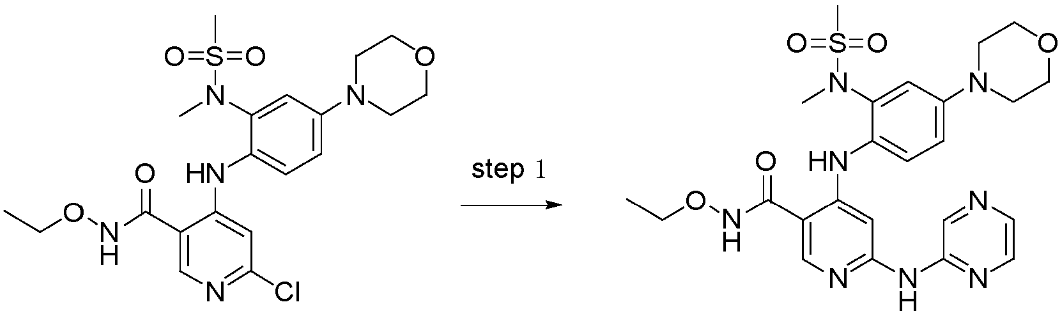


**130-e** **136**

6-chloro-N-ethoxy-4-((2-(N-methyl methanesulfonamido)-4-morpholinophenyl)amino)nicotinamide **(130-e,** 100mg, 0.2mmol), pyrazin-2-ylamine (30mg, 0.3mmol), cesium carbonate (195mg, 0.6mmol), XantPhos(23mg, 0.04mmol) and $Pd_2(dba)_3$(20mg, 0.02mmol) were added to anhydrous dioxane (8ml). After atmosphere replacement with nitrogen three times, the reaction mixture was heated to 120°C with stirring for 8h, and filtered by suction. The filtrate was concentrated and purified by high performance preparative thin layer chromatography (MeOH:DCM=1:12) to provide the title compound: N-ethoxy-4-((2-(N-methyl methanesulfonamido)-4-morpholinophenyl)amino)-6-(pyrazin-2-ylamino) nicotinamide **(136,** 25mg, 0.046mmol, 23% yield). MS Calcd: 542; MS Found: 543($[M+H]^+$). $^1H$NMR (400 MHz, DMSO-$d_6$) δ11.56 (s, 1H), 9.96 (s, 1H), 9.67 (s, 1H), 8.95 (d, *J* = 1.6 Hz, 1H), 8.31 (s, 1H), 8.14 (dd, *J* = 2.8, 1.6 Hz, 1H), 8.05 (d, *J* = 2.8 Hz, 1H), 7.39 (d, *J* = 8.8 Hz, 1H), 7.30 (s, 1H), 7.10 - 7.04 (m, 2H), 3.93 (q, *J* = 7.2 Hz, 2H), 3.77 (t, *J* = 4.8 Hz, 4H), 3.20 - 3.15 (m, 4H), 3.13 (s, 3H), 3.07 (s, 3H), 1.22 (t, *J* = 7.2 Hz, 3H).

Example **137**

N-ethoxy-6-(((1-methyl-1H-pyrazol-3-yl)amino)-4-((2-(N-methyl methanesulfonamido)-4-morpholinophenyl)amino)nicotinamide

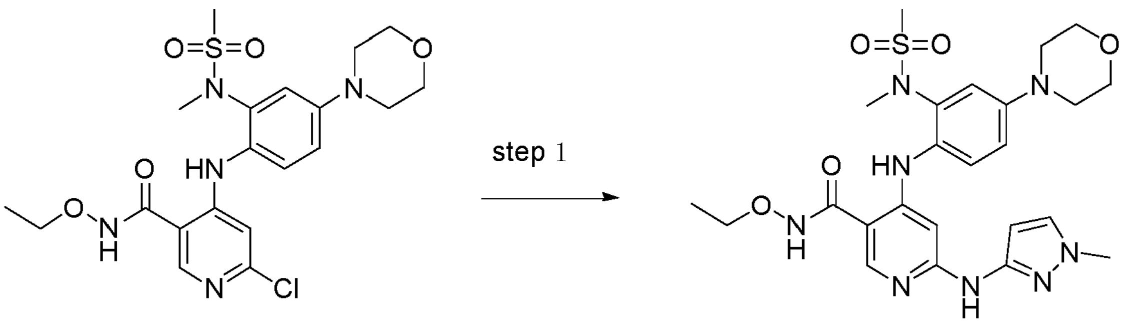


**130-e** **137**

6-chloro-N-ethoxy-4-((2-(N-methyl methanesulfonamido)-4-morpholinophenyl)amino)nicotinamide **(130-e,** 120mg, 0.24mmol), 1-methyl-1H-pyrazol-3-ylamine (35mg, 0.36mmol), cesium carbonate (234mg, 0.72mmol), Xantphos(27mg, 0.048mmol) and $Pd_2(dba)_3$(45mg, 0.048mmol) were added to anhydrous dioxane (8ml). After atmosphere replacement with nitrogen three times, the reaction mixture was heated to 120°C with stirring for 8h, and filtered by suction. The filtrate was concentrated and purified by high performance preparative thin layer chromatography (MeOH:DCM=1:12) to provide the title compound: N-ethoxy-6-(((1-methyl-1H-pyrazol-3-yl)amino)-4-((2-(N-methyl methanesulfonamido)-4-morpholinophenyl)amino)nicotinamide **(137,** 30mg, 0.05mmol, 22% yield). MS Calcd: 544; MS Found: 545($[M+H]^+$). $^1H$ NMR (400 MHz, DMSO-$d_6$)δ11.40 (s, 1H), 9.63 (s, 1H), 9.17 (s, 1H), 8.20 (s, 1H), 7.46 (d, $J$ = 2.4 Hz, 1H), 7.38 (d, $J$ = 8.8 Hz, 1H), 7.09 - 6.96 (m, 3H), 6.04 (d, $J$ = 2.4 Hz, 1H), 3.91 (q, $J$ = 7.2 Hz, 2H), 3.76 (t, $J$ = 4.8 Hz, 4H), 3.66 (s, 3H), 3.16-3.12 (m, 7H), 3.07 (s, 3H), 1.21 (t, $J$ = 7.2Hz, 3H).

Example **138**

4-((4-cyclopropyl-2-(N-methyl methanesulfonamido)phenyl)amino)-N-ethoxy-6-(pyridin-2-ylamino)nicotinamide

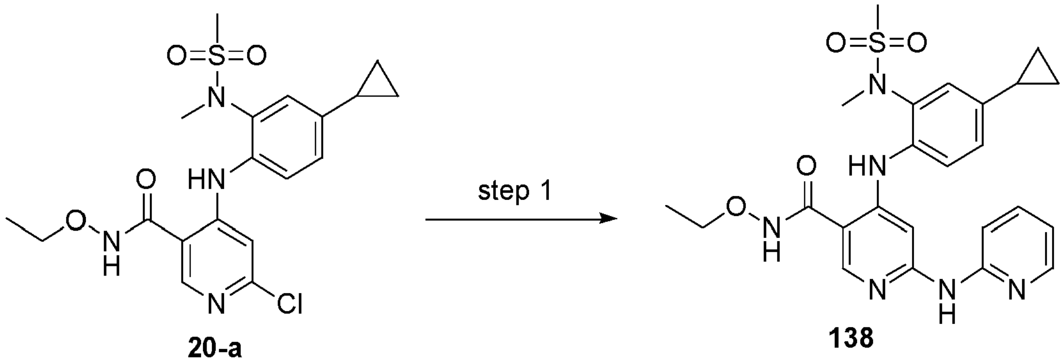


**20-a** **138**

Step 1: 6-chloro-4-((4-cyclopropyl-2-(N-methyl methanesulfonamido)phenyl)amino)-N-ethoxy nicotinamide **(20-a,** 150mg, 0.342mmol), 2-aminopyridine (35.44mg, 0.376mmol), $pd_2(dba)_3$ (62.59mg, 0.068mmol), Xant-Phos(79.15mg, 0.137mmol), $Cs_2CO_3$ (334.27mg, 1.026mmol), and 1,4-dioxane (5mL) are mixed. After atmosphere replacement with nitrogen three times, the mixture was allowed to react at 120°C for 6 hours. When TLC indicated a completed reaction, the mixture was filtered by suction, and the filtrate was concentrated under reduced pressure at 40°C, mixed with 200-300 mesh silica gel for loading, and separated and purified by column chromatography (DCM:MeOH=20:1) to provide the title compound: 4-((4-cyclopropyl-2-(N-methyl methanesulfonamido)phenyl)amino)-N-ethoxy-6-(pyridin-2-ylamino)nicotinamide **(138,** 25mg, 0.05mmol, 17% yield). MS Calcd:496; MS Found: 497($[M+H]^+$). $^1H$ NMR (400 MHz, DMSO-$d_6$)δ11.52 (s, 1H), 9.88 (s, 1H), 9.66 (s, 1H), 8.30 (s, 1H), 8.14 (d, $J$ = 4.8 Hz, 1H), 7.68 (s, 1H), 7.65-7.61 (m, 1H), 7.54 (d, $J$ = 8.4 Hz, 1H), 7.48 (d, $J$ = 8.4 Hz, 1H), 7.26 (s, 1H), 7.15-7.13 (m, 1H), 6.86 (t, $J$ = 7.0 Hz, 1H), 3.94 (q, $J$ = 7.2 Hz, 2H), 3.14 (s, 3H), 3.10 (s, 3H), 2.00-1.95 (m, 1H), 1.24-1.20 ((t, $J$ = 7.2 Hz, 3H), 1.00-0.97 (m, 2H), 0.73-0.72 (m, 2H).

Example **139**

6-(cyclopropylcarboxamido)-4-((4-cyclopropyl-2-(N-methyl methanesulfonamido)phenyl)amino)-N-ethoxy nicotinamide

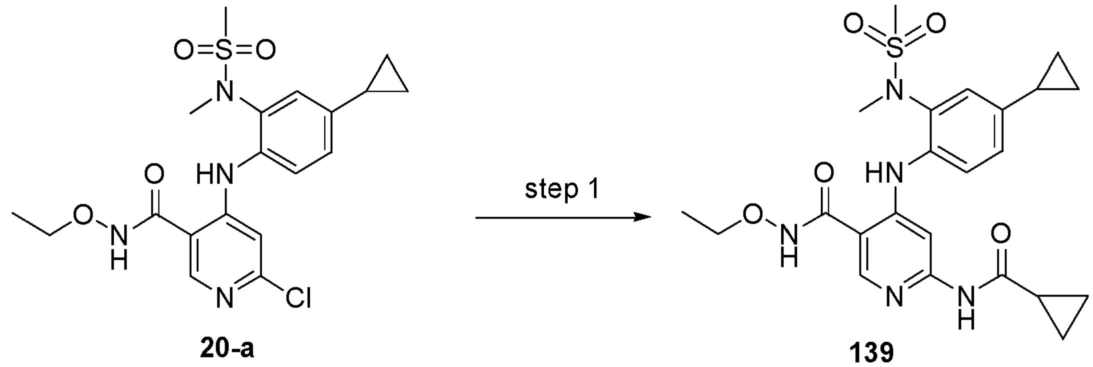


20-a

139

Step 1: 6-chloro-4-((4-cyclopropyl-2-(N-methyl methanesulfonamido)phenyl)amino)-N-ethoxy nicotinamide **(20-a,** 150mg, 0.342mmol), cyclopropylcarboxamide (37.84mg, 0.445mmol), $pd_2(dba)_3$ (93.95mg, 0.102mmol), XantPhos(118.73mg, 0.205mmol), $Cs_2CO_3$ (389.98mg, 1.197mmol), and 1,4-dioxane (7mL) were combined. After atmosphere replacement with nitrogen three times, the mixture was allowed to react at 120°C for 6 hours. When TLC indicated a completed reaction, the mixture was filtered by suction, and concentrated under reduced pressure, mixed with 200-300 mesh silica gel for loading onto and purifying by column chromatography (DCM:MeOH=20:1) to provide the title compound: 6-(cyclopropylcarboxamido)-4-((4-cyclopropyl-2-(N-methyl methanesulfonamido)phenyl)amino)-N-ethoxy nicotinamide **(139,** 29mg, 0.059mmol, 17% yield). MS Calcd:487; MS Found: 488([M+H]$^+$). $^1$H NMR (400 MHz, DMSO-d6)δ11.64 (s, 1H), 10.74 (s, 1H), 9.86 (s, 1H), 8.32 (s, 1H), 7.81 (s, 1H), 7.32 (d, *J* = 8.4 Hz, 1H), 7.26 (d, *J* = 2.0 Hz, 1H), 7.09 (dd, *J* = 8.4, 2.0 Hz, 1H), 3.93 (q, *J* = 7.2 Hz, 2H), 3.11 (s, 3H), 3.07 (s, 3H), 2.02 - 1.91 (m, 1H), 1.52 - 1.46 (m, 1H), 1.22 (t, *J* = 7.2 Hz, 3H), 0.76 - 0.70 (m, 2H), 0.74 - 0.58 (m, 6H).

Example **140**

4-((4-cyclopropyl-2-(N-methyl methanesulfonamido)phenyl)amino)-N-ethoxy-6-((1-methyl-1H-pyrazol-4-ylamino)nicotinamide

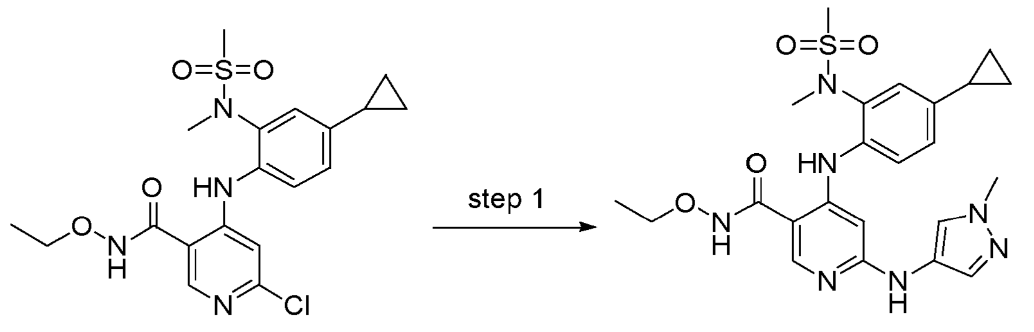


20-a

140

Step 1: 6-chloro-4-((4-cyclopropyl-2-(N-methyl methanesulfonamido)phenyl)amino)-N-ethoxy nicotinamide **(20-a,** 150mg, 0.342mmol), 1-methyl-pyrazol-4-ylamine (43.17mg, 0.445mmol, ), $Pd_2(dba)_3$(93.95mg, 0.1026mmol), XantPhos(118.73mg, 0.205mmol), $Cs_2CO_3$ (389.98mg, 1.197mmol), and 1,4-dioxane (7mL) were combined. After atmosphere replacement with nitrogen three times, the mixture was allowed to react at 120°C for 6 hours. When TLC indicated a completed reaction, the mixture was filtered by suction, and the filtrate was concentrated under reduced pressure at 40°C, mixed with 200-300 mesh silica gel for loading onto and purifying by column chromatography (DCM:MeOH=20:1) to provide the title compound: 4-((4-cyclopropyl-2-(N-methyl methanesulfonamido)phenyl)amino)-N-ethoxy-6-((1-methyl-1H-pyrazol-4-yl)amino)nicotinamide **(140,** 29mg, 0.058mmol, 17% yield).

MS Calcd:499; MS Found: 500([M+H]$^+$) $^1$H NMR (400 MHz, DMSO-$d_6$)δ11.43 (s, 1H), 9.63 (s, 1H), 8.81 (s, 1H),

8.22 (s, 1H), 7.79 (s, 1H), 7.36 (d, *J* = 8.4 Hz, 1H), 7.33 (s, 1H), 7.26 (d, *J* = 2.0 Hz, 1H), 7.08 (dd, *J* = 8.4, 2.0 Hz, 1H), 6.15 (s, 1H), 3.92 (q, *J* = 7.2 Hz, 2H), 3.77 (s, 3H), 3.12 (s, 3H), 3.08 (s, 3H), 2.02-1.92 (m, 1H), 1.21 (t, *J* = 7.2 Hz, 3H), 1.00-0.96 (m, 2H), 0.72-0.68 (m, 2H).

Example **141**

N-ethoxy-4-((4-ethynyl-2-(N-methyl methanesulfonamido)phenyl)amino)-6-(5-methoxy pyridin-2-ylamino)nicotinamide

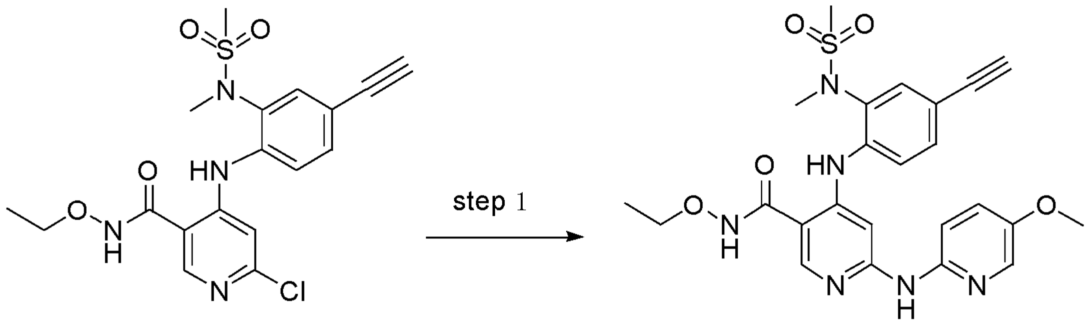


**127-a** **141**

Step 1: 6-chloro-N-ethoxy-4-((4-ethynyl-2-(N-methyl methanesulfonamido)phenyl)amino)nicotinamide **(127-a,** 110mg, 0.266mmol), 2-amino-5-methoxy pyridine (64.6mg, 0.521mmol), $pd_2(dba)_3$ (72.25mg, 0.078mmol), Xant-Phos(60.33mg, 0.104mmol), $Cs_2CO_3$ (254.8mg, 0.781mmol), and 1,4-dioxane (5 mL) were combined. After atmosphere replacement with nitrogen three times, the mixture was allowed to react at 120°C for 6 hours. When TLC indicated a completed reaction, the mixture was filtered by suction, and the filtrate was concentrated under reduced pressure at 40°C, mixed with 200-300 mesh silica gel for loading onto and purifying by column chromatography (DCM: MeOH=20:1) to provide the title compound: N-ethoxy-4-((4-ethynyl-2-(N-methyl methanesulfonamido)phenyl)amino)-6-((5-methoxy pyridin-2-ylamino)amino)nicotinamide **(141,** 24mg, 0.047mmol, 17% yield). MS Calcd:510; MS Found: 511([M+H]$^+$). $^1$H NMR (400 MHz, DMSO-$d_6$)δ11.60 (s, 1H), 10.28 (s, 1H), 9.66 (s, 1H), 8.32 (s, 1H), 7.95 (d, *J* = 2.8 Hz, 1H), 7.76 (s, 1H), 7.67-7.64 (m, 2H), 7.58-7.55 (m, 2H), 7.38 (dd, *J* = 8.8, 2.8 Hz, 1H), 4.23 (s, 1H), 3.93 (q, *J* = 7.2 Hz, 2H), 3.79 (s, 3H), 3.18 (s, 3H), 3.17 (s, 3H), 1.22 (t, *J* = 7.2 Hz, 3H).

Example **142**

6-(5-cyano pyridin-2-ylamino)-N-ethoxy-4-((4-ethynyl-2-(N-methyl methanesulfonamido)phenyl)amino)nicotinamide

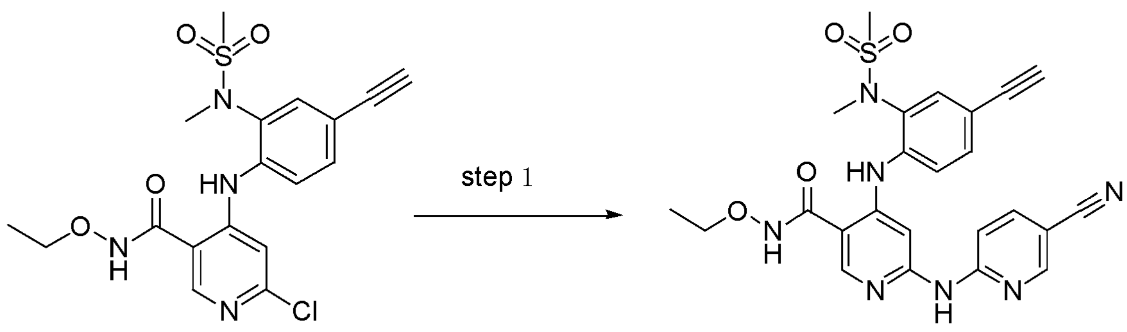


**127-a** **142**

Step 1: 6-chloro-N-ethoxy-4-((4-ethynyl-2-(N-methyl methanesulfonamido)phenyl)amino)nicotinamide **(127-a,** 110mg, 0.266mmol), 2-amino-5-cyano pyridine (60.03mg, 0.521mmol), $pd_2(dba)_3$ (72.25mg, 0.078mmol), Xant-Phos(60.33mg, 0.104mmol), $Cs_2CO_3$ (254.8mg, 0.781mmol), and 1,4-dioxane (5 mL) were combined. After atmosphere replacement with nitrogen three times, the mixture was allowed to react at 120°C for 6 hours. When TLC indicated a completed reaction, the mixture was filtered by suction, and the filtrate was concentrated under reduced pressure at 40°C,

mixed with 200-300 mesh silica gel for loading onto and purifying by column chromatography (DCM: MeOH=20:1) to provide the title compound: 6-((5-cyano pyridin-2-ylamino)amino)-N-ethoxy-4-((4-ethynyl-2-(N-methyl methanesulfonamido)phenyl)amino)nicotinamide **(142,** 25mg, 0.049mmol, 4% yield). MS Calcd:505; MS Found: 506($[M+H]^+$). $^1$H NMR (400 MHz, DMSO-$d_6$)δ11.84 (s, 1H), 10.74 (br, 1H), 10.33 (s, 1H), 8.73 (d, *J* = 2.0 Hz, 1H), 8.39 (s, 1H), 8.11 (dd, *J* = 8.8, 2.4 Hz, 1H), 7.72 (s, 1H), 7.66-7.62 (m, 4H), 4.29 (s, 1H), 3.95 (q, *J* = 7.2 Hz, 2H), 3.19 (s, 3H), 3.16 (s, 3H), 1.22 (t, *J* = 7.2 Hz, 3H).

Example **143**

N-ethoxy-4-((4-ethynyl-2-(N-methyl methanesulfonamido)phenyl)amino)-6-(6-fluoropyridin-2-ylamino)nicotinamide

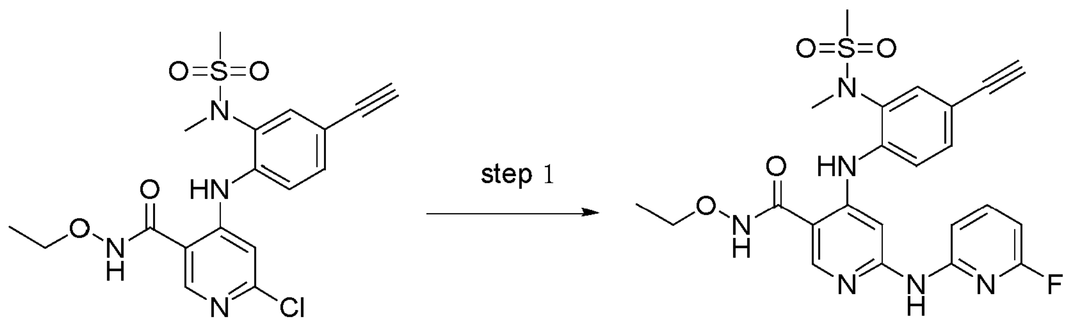


**127-a** **143**

Step 1: 6-chloro-N-ethoxy-4-((4-ethynyl-2-(N-methyl methanesulfonamido)phenyl)amino)nicotinamide **(127-a,** 110mg, 0.266mmol), 2-amino-6-fluoropyridine (49.68mg, 0.521mmol), $pd_2(dba)_3$ (72.25mg, 0.078mmol), Xant-Phos(60.33mg, 0.104mmol), $Cs_2CO_3$ (254.8mg, 0.781mmol), and 1,4-dioxane (5 mL) were combined. After atmosphere replacement with nitrogen three times, the mixture was allowed to react at 120°C for 6 hours. When TLC indicated a completed reaction, the mixture was filtered by suction, and the filtrate was concentrated under reduced pressure at 40°C, mixed with 200-300 mesh silica gel for loading onto and purifying by column chromatography (DCM: MeOH=20:1) to provide the title compound: N-ethoxy-4-((4-ethynyl-2-(N-methyl methanesulfonamido)phenyl)amino)-6-((6-fluoropyridin-2-ylamino)amino)nicotinamide **(143,** 24mg, 0.048mmol, 18% yield). MS Calcd:498; MS Found: 497($[M-H]^-$). $^1$H NMR (400 MHz, Methanol-$d_4$)δ8.29 (s, 1H), 7.93 (s, 1H), 7.78 - 7.68 (m, 2H), 7.63 (d, *J* = 2.0 Hz, 1H), 7.51 (dd, *J* = 8.4, 2.0 Hz, 1H), 7.14 (dd, *J* = 8.0, 2.0 Hz, 1H), 6.49 (dd, *J* = 8.0, 2.4 Hz, 1H), 4.60 (s, 1H), 4.03 (q, *J* = 7.2 Hz, 2H), 3.26 (s, 3H), 3.10 (s, 3H), 1.31 (t, *J* = 7.2 Hz, 3H).

Example **144**

N-ethoxy-4-((4-ethynyl-2-(N-methyl methanesulfonamido)phenyl)amino)-6-(5-fluoropyridin-2-ylamino)nicotinamide

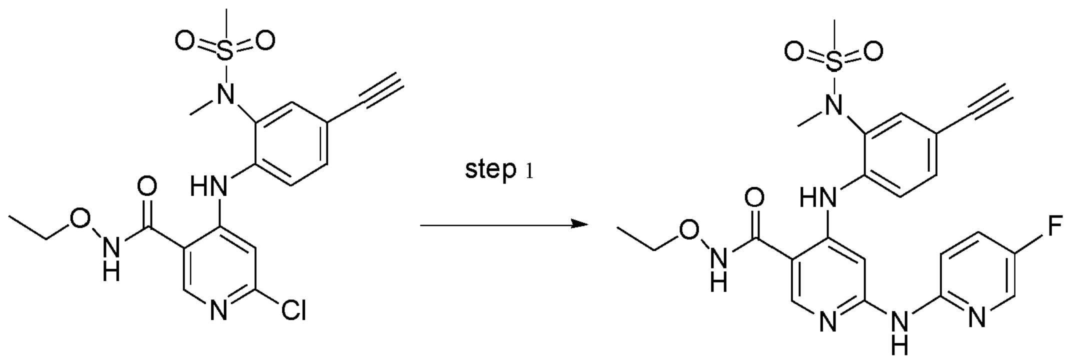


127-a 144

Step 1: 6-chloro-N-ethoxy-4-((4-ethynyl-2-(N-methyl methanesulfonamido)phenyl)amino)nicotinamide **(127-a,** 110 mg, 0.266 mmol), 2-amino-5-fluoropyridine (49.68 mg, 0.521 mmol), $pd_2(dba)_3$ (72.25 mg, 0.078 mmol), Xant-Phos(60.33 mg, 0.104 mmol), $Cs_2CO_3$ (254.8 mg, 0.781 mmol), and 1,4-dioxane (5 mL) were combined. After atmosphere replacement with nitrogen three times, the mixture was allowed to react at 120°C for 6 hours. When TLC indicated a completed reaction, the mixture was filtered by suction, and the filtrate was concentrated under reduced pressure at 40°C, mixed with 200-300 mesh silica gel for loading onto and purifying by column chromatography (DCM: MeOH=20:1) to provide the title compound: N-ethoxy-4-((4-ethynyl-2-(N-methyl methanesulfonamido)phenyl)amino)-6-((5-fluoropyridin-2-ylamino)nicotinamide **(144,** 30 mg, 0.06 mmol, 22% yield). MS Calcd:498; MS Found: 497($[M-H]^+$)

$^1H$ NMR (400 MHz, DMSO-$d_6$)δ11.60 (s, 1H), 10.27 (s, 1H), 9.87 (s, 1H), 8.35 (s, 1H), 8.22 (d, $J$ = 2.8 Hz, 1H), 7.76 (s, 1H), 7.69 - 7.5 7 (m, 5H), 4.22 (s, 1H), 3.94 (q, $J$ = 7.2 Hz, 2H), 3.19 (s, 3H), 3.17 (s, 3H), 1.22 (t, $J$ = 7.2 Hz, 3H).

Example **145**

4-((4-cyclopropyl-2-(N-methyl methanesulfonamido)phenyl)amino)-N-ethoxy-6-(pyrazin-2-ylamino)nicotinamide

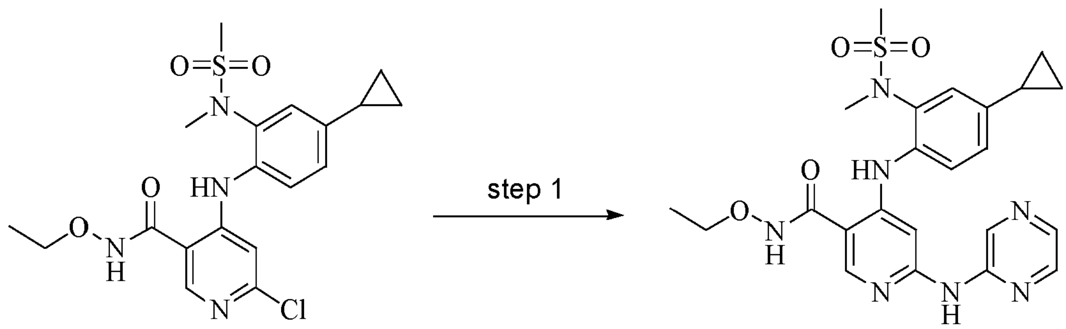


**20-a** **145**

Step 1: 6-chloro-4-((4-cyclopropyl-2-(N-methyl methanesulfonamido)phenyl)amino)-N-ethoxy nicotinamide **(20-a,** 150mg, 0.342mmol), 2-aminopyrazine (35.81mg, 0.376mmol), $pd_2(dba)_3$ (62.59mg, 0.068mmol), Xant-Phos(79.15mg, 0.137mmol), $Cs_2CO_3$ (334.27mg, 1.026mmol), and 1,4-dioxane (5mL) were combined. After atmosphere replacement with nitrogen three times, the mixture was allowed to react at 120°C for 6 hours. When TLC indicated a completed reaction, the mixture was filtered by suction, and the filtrate was concentrated under reduced pressure at 40°C, mixed with 200-300 mesh silica gel for loading onto and purifying by column chromatography (DCM:MeOH=20:1) to provide the title compound: 4-((4-cyclopropyl-2-(N-methyl methanesulfonamido)phenyl)amino)-N-ethoxy-6-(pyrazin-2-ylamino)nicotinamide **(145,** 38mg, 0.076mmol, 23.5% yield).

MS Calcd:497; MS Found: 498($[M+H]^+$).

$^1H$ NMR (401 MHz, DMSO-$d_6$)δ11.58 (s, 1H), 10.00 (s, 1H), 9.89 (s, 1H), 8.96 (d, $J$ = 1.6 Hz, 1H), 8.34 (s, 1H), 8.16 (t, $J$ = 2.0 Hz, 1H), 8.07 (d, $J$ = 2.8 Hz, 1H), 7.66 (d, $J$ = 2.4 Hz, 1H), 7.49 (s, 1H), 7.45 (d, $J$ = 8.4 Hz, 1H), 7.17 - 7.13 (m, 1H), 3.94 (q, $J$ = 7.2 Hz, 2H), 3.14 (s, 3H), 3.10 (s, 3H), 2.01-1.96 (m, 1H), 1.22 (t, $J$ = 7.2 Hz, 3H), 1.02-0.96 (m, 2H), 0.74 - 0.71 (m, 2H).

Example **146**

4-((4-cyclopropyl-2-(N-methyl methanesulfonamido)phenyl)amino)-N-ethoxy-6-((6-methyl pyridin-2-yl)amino)nicotinamide

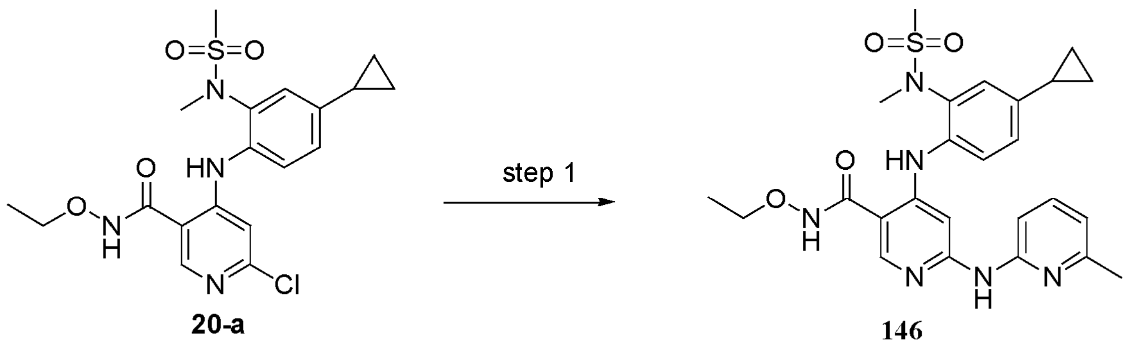

20-a

146

Step 1: 6-chloro-4-((4-cyclopropyl-2-(N-methyl methanesulfonamido)phenyl)amino)-N-ethoxy nicotinamide **(20-a,** 150mg, 0.342mmol), 2-amino-6-methyl pyridine (55.43mg, 0.513mmol), $pd_2(dba)_3$ (93.95mg, 0.106mmol), Xant-Phos(118.73mg, 0.205mmol), $Cs_2CO_3$ (389.98mg, 1.197mmol), and 1,4-dioxane (7mL) were combined. After atmosphere replacement with nitrogen three times, the mixture was allowed to react at 120°C for 6 hours. When TLC indicated a completed reaction, the mixture was filtered by suction, and the filtrate was concentrated under reduced pressure at 40°C, mixed with 200-300 mesh silica gel for loading onto and purifying by column chromatography (DCM:MeOH=20:1) to provide the title compound: 4-((4-cyclopropyl-2-(N-methyl methanesulfonamido)phenyl)amino)-N-ethoxy-6-((6-methyl pyridin-2-yl)amino)nicotinamide **(146,** 22mg, 0.043mmol, 12.76% yield). MS Calcd:510; MS Found: 509($[M-H]^-$). $^1H$ NMR (400 MHz, DMSO-$d_6$)δ11.43 (s, 1H), 9.63 (s, 1H), 8.81 (s, 1H), 8.22 (s, 1H), 7.79 (s, 1H), 7.36 (d, *J* = 8.4 Hz, 1H), 7.33 (s, 1H), 7.26 (d, *J* = 2.0 Hz, 1H), 7.08 (dd, *J* = 8.4, 2.0 Hz, 1H), 6.15 (s, 1H), 3.91 (q, *J* = 7.2 Hz, 2H), 3.77 (s, 3H), 3.64 (s, 1H), 3.12 (s, 3H), 3.08 (s, 3H), 2.00-1.93 (m, 1H), 1.21 (t, *J* = *7.2* Hz, 3H), 1.01 - 0.93 (m, 2H), 0.74 - 0.63 (m, 2H).

Example **147**

4-((4-cyclopropyl-2-(N-methyl methanesulfonamido)phenyl)amino)-N-ethoxy-6-((4-methyl pyridin-2-yl)amino)nicotinamide

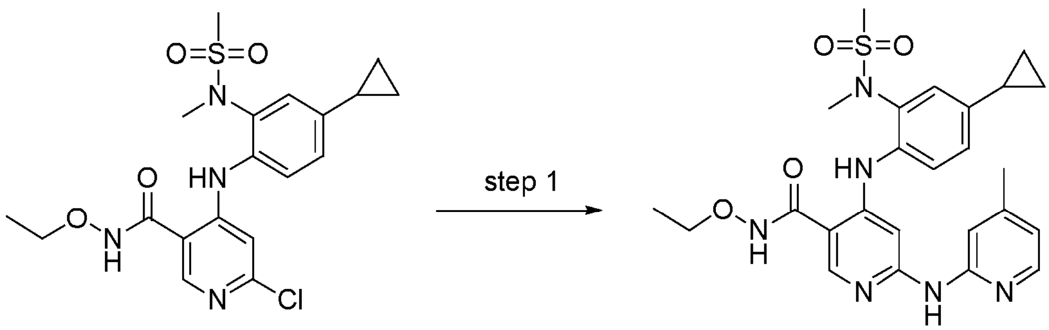

20-a

147

Step 1: 6-chloro-4-((4-cyclopropyl-2-(N-methylmethanesulfonamido)phenyl)amino)-N-ethoxy nicotinamide **(20-a,** 150mg, 0.342mmol), 2-amino-4-methyl pyridine (55.43mg, 0.513mmol), $pd_2(dba)_3$ (93.95mg, 0.106mmol), Xant-Phos(118.73mg, 0.205mmol), $Cs_2CO_3$ (389.98mg, 1.197mmol), and 1,4-dioxane (7mL) were combined. After atmosphere replacement with nitrogen three times, the mixture was allowed to react at 120°C for 6 hours. When TLC indicated a completed reaction, the mixture was filtered by suction, and the filtrate was concentrated under reduced pressure at 40°C, mixed with 200-300 mesh silica gel for loading onto and purifying by column chromatography (DCM:MeOH=20:1) to provide the title compound: 4-((4-cyclopropyl-2-(N-methyl methanesulfonamido)phenyl)amino)-N-ethoxy-6-((4-methyl pyridin-2-yl)amino)nicotinamide **(147,** 28mg, 0.055mmol, 16.05% yield). MS Calcd:510; MS Found: 509([M-H]). $^1H$ NMR (400 MHz, DMSO-$d_6$)δ11.53 (s, 1H), 9.89 (s, 1H), 9.61 (s, 1H), 8.30 (s, 1H), 8.00 (d, *J* = 5.2 Hz, 1H), 7.69 (s, 1H), 7.48 (d, *J* = 8.4 Hz, 1H), 7.36 (s, 1H), 7.26 (d, *J* = 2.0 Hz, 1H), 7.13 (dd, *J* = 8.4, 2.0 Hz, 1H), 6.71 (dd, *J* = 5.2, 1.2 Hz, 1H), 3.92 (t, *J* = 7.2 Hz, 2H), 3.14 (s, 3H), 3.10 (s, 3H), 2.24 (s, 3H), 1.99-1.95 (m, 1H), 1.22 (t, *J* = 7.2 Hz, 3H), 1.02 - 0.95 (m, 2H), 0.75 - 0.67 (m, 2H).

Example **148**

4-((4-cyclopropyl-2-(N-methyl methanesulfonamido)phenyl)amino)-N-ethoxy-6-((6-methyl pyridin-3-yl)amino)nicotinamide

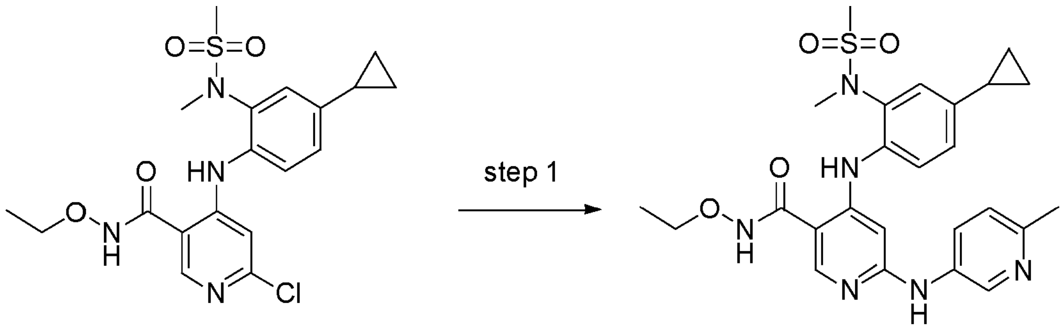


**20-a** **148**

Step 1: 6-chloro-4-((4-cyclopropyl-2-(N-methyl methanesulfonamido)phenyl)amino)-N-ethoxy nicotinamide **(20-a,** 150mg, 0.342mmol), 3-amino-6-methyl pyridine (55.43mg, 0.513mmol), $pd_2(dba)_3$ (93.95mg, 0.106mmol), Xant-Phos(118.73mg, 0.205mmol), $Cs_2CO_3$ (389.98mg, 1.197mmol), and 1,4-dioxane (7mL) were combined. After atmosphere replacement with nitrogen three times, the mixture was allowed to react at 120°C for 6 hours. When TLC indicated a completed reaction, the mixture was filtered by suction, and the filtrate was concentrated under reduced pressure at 40°C, mixed with 200-300 mesh silica gel for loading onto and purifying by column chromatography (DCM:MeOH=20:1) to provide the title compound: 4-((4-cyclopropyl-2-(N-methyl methanesulfonamido)phenyl)amino)-N-ethoxy-6-((6-methyl pyridin-3-yl)amino)nicotinamide **(148,** 32mg, 0.063mmol, 18.56% yield). MS Calcd:510; MS Found: 509($[M-H]^-$). $^1H$ NMR (400 MHz, DMSO-$d_6$)δ11.52 (s, 1H), 9.64 (s, 1H), 9.10 (s, 1H), 8.57 (d, *J* = 2.8 Hz, 1H), 8.25 (s, 1H), 7.97 (dd, *J* = 8.4, 2.8 Hz, 1H), 7.37 (d, *J* = 8.4 Hz, 1H), 7.28 (d, *J* = 2.0 Hz, 1H), 7.15 - 7.06 (m, 2H), 6.29 (s, 1H), 3.92 (q, *J* = 7.2 Hz, 2H), 3.13 (s, 3H), 3.08 (s, 3H), 2.37 (s, 3H), 2.03 - 1.92 (m, 1H), 1.21 (t, *J* = 7.2 Hz, 3H), 1.04 - 0.92 (m, 2H), 0.73 - 0.69 (m, 2H).

Example **149**

4-((5-chloro-4-cyclopropyl-2-(N-methyl methanesulfonamido)phenyl)amino)-6-(((2,6-dimethyl pyrimidin-4-yl)amino)-N-ethoxy nicotinamide

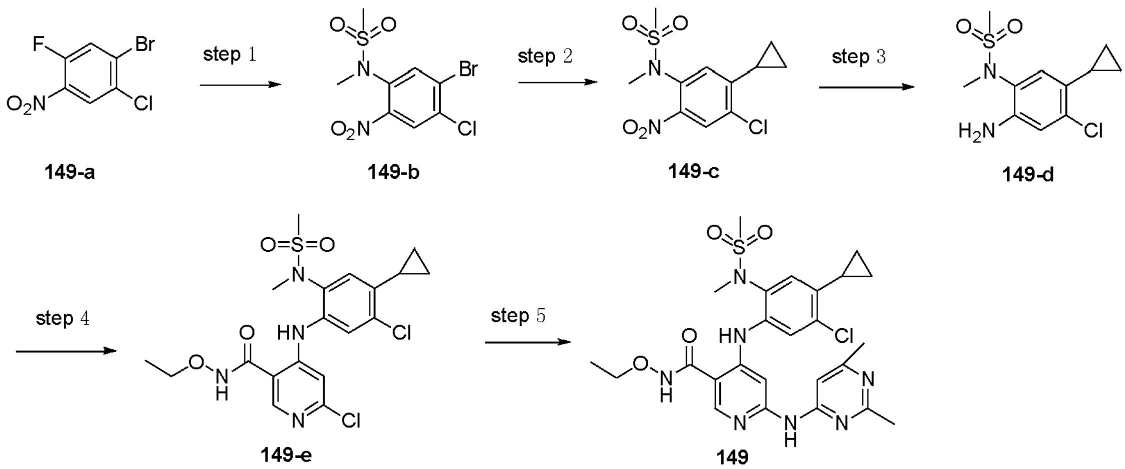


**149-a** **149-b** **149-c** **149-d** **149-e** **149**

Step 1: To a 50mL flask were sequentially added 1-bromo-2-chloro-S-fluoro-4-nitrobenzene(**149-a**, 400mg, 1.57mmol), N-methyl methanesulfonamide (206mg, 1.89mmol), anhydrous potassium carbonate (433mg, 3.14mmol) and acetonitrile (15mL). The mixture was stirred at 90°C for 4 hours. Upon indication of completed reaction by TLC, the reaction mixture was added with 20ml of water for dilution of the mother liquid, and extracted with ethyl acetate three times. The combined organic phases were washed with saturated brine, dried over anhydrous sodium sulfate, followed by

solvent removed by rotary evaporation to provide N-(5-bromo-4-chloro-2-nitrophenyl)-N-methyl methanesulfonamide **(149-b,** 540mg, 1.57mmol, 99% yield). MS Calcd: 342; MS Found: 365, 367([M+Na]$^+$).

Step 2: N-(5-bromo-4-chloro-2-nitrophenyl)-N-methyl methanesulfonamide **(149-b,** 540mg, 1.6mmol), cyclopropyl boronic acid(163mg, 1.9mmol), potassium phosphate (1.02g, 4.8mmol), Pd(dppf)$Cl_2$(117mg, 0.16mmol) were added to dioxane (10mL) and water (2mL). After atmosphere replacement with nitrogen three times, the reaction mixture was heated to 90°C with stirring for 5 hours, and filtered by suction. The filtrate was concentrated and purified by column chromatography (EA:PE=1:2) to provide N-(4-chloro-5-cyclopropyl-2-nitrophenyl)-N-methyl methanesulfonamide **(149-c,** 380mg, 1.25mmol, 78% yield). MS Calcd: 304; MS Found: 327, 329([M+Na]$^+$).

Step 3: To a 100mL flask were sequentially added N-(4-chloro-5-cyclopropyl-2-nitrophenyl)-N-methyl methanesulfonamide **(149-c,** 380mg, 1.25mmol), iron powder (350mg, 6.25mmol), ammonium chloride (675mg, 12.5mmol), and ethanol (10mL) and water (2mL). The mixture was stirred at room temperature for 4 hours. When the reaction was completed, the mother liquid was filtered through diatomaceous earth, and the filtrate was extracted with ethyl acetate. The combined organic phases were washed with saturated sodium chloride aqueous solution, dried over anhydrous sodium sulfate, followed by solvent removed by rotary evaporation to provide N-(2-amino-4-chloro-5-cyclopropyl phenyl)-N-methyl methanesulfonamide **(149-d,** 300mg, 1.09mmol, 87% yield). MS Calcd: 274; MS Found: 275, 277([M+H]$^+$).

Step 4: To a two-necked flask were sequentially added N-(2-amino-4-chloro-5-cyclopropyl phenyl)-N-methyl methanesulfonamide **(149-d,** 300mg, 1.09mmol), 4,6-dichloro-N-ethoxy nicotinamide **(int-2,** 282mg, 1.2mmol), and 6ml of anhydrous tetrahydrofuran as solvent. After atmosphere replacement by nitrogen, the mixture was added in ice bath with a solution of LiHMDS in tetrahydrofuran (3.3ml, 1mol/L) and continuously stirred at room temperature for 8 hours. Upon indication of completed reaction by TLC, the mixture was adjusted with aqueous hydrochloride (1N) to pH 5, and extracted with ethyl acetate (30mLx3). The combined organic layers were dried, concentrated, and purified by column chromatography to provide 6-chloro-4-((5-chloro-4-cyclopropyl-2-(N-methyl methanesulfonamido)phenyl)amino)-N-ethoxy nicotinamide **(149-e,** 140mg, 0.3mmol, 27% yield). MS Calcd: 472; MS Found: 473, 475([M+H]$^+$)

Step 5: 6-chloro-4-((5-chloro-4-cyclopropyl-2-(N-methyl methanesulfonamido)phenyl)amino)-N-ethoxy nicotinamide **(149-e,** 70mg, 0.15mmol), 2,6-dimethyl pyrimidin-4-ylamine (36mg, 0.3mmol), cesium carbonate (145mg, 0.45mmol), X-phos(34mg, 0.06mmol) and $Pd_2(dba)_3$(28mg, 0.03mmol) were added to anhydrous dioxane (2ml). After atmosphere replacement by nitrogen, the mixture was allowed to react in microwave at 120°C for 1 hour, and filtered by suction. The filtrate was concentrated, purified by reverse phase MPLC to provide the title compound: 4-((5-chloro-4-cyclopropyl-2-(N-methyl methanesulfonamido)phenyl)amino)-6-(((2,6-dimethyl pyrimidin-4-yl)amino)-N-ethoxy nicotinamide **(149,** 20mg, 0.04mmol, 26% yield). MS Calcd: 559; MS Found: 560([M+H]$^+$). $^1$H NMR (400 MHz, DMSO-$d_6$) δ11.85 (s, 1H), 10.00 (s, 1H), 8.44 (s, 1H), 7.76 - 7.36 (m, 3H), 7.34 - 6.94 (m, 2H), 3.95 (q, *J* = 7.2 Hz, 2H), 3.13 (s, 3H), 3.07 (s, 3H), 2.52 (s, 3H), 2.47 (s, 3H), 2.17 - 2.09 (m, 1H), 1.22 (t, *J* = 7.2 Hz, 3H), 1.07 - 1.01 (m, 2H), 0.81-0.76 (m, 2H).

Example **150**

4-((5-chloro-4-cyclopropyl-2-(N-methyl methanesulfonamido)phenyl)amino)-6-(cyclopropylcarboxamido)-N-ethoxy nicotinamide

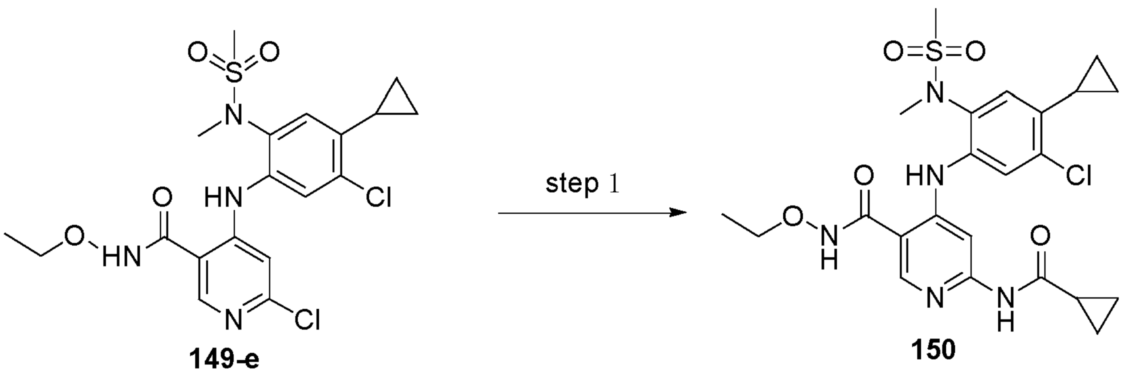


149-e

150

Step 1: 6-chloro-4-((5-chloro-4-cyclopropyl-2-(N-methyl methanesulfonamido)phenyl)amino)-N-ethoxy nicotinamide **(149-e,** 100mg, 0.21mmol), cyclopropylcarboxamido(36mg, 0.42mmol), cesium carbonate (205mg, 0.63mmol), X-phos(46mg, 0.08mmol) and $Pd_2(dba)_3$(37mg, 0.04mmol) were added to anhydrous dioxane (2ml). After atmosphere replacement by nitrogen, the mixture was allowed to react in microwave at 120°C for 1 hour, and filtered by suction. The filtrate was concentrated, purified by reverse phase MPLC to provide the title compound: 4-((5-chloro-4-cyclopropyl-2-(N-methyl methanesulfonamido)phenyl)amino)-6-(cyclopropylcarboxamido)-N-ethoxy nicotinamide **(150,** 40mg, 0.08mmol, 38% yield).MS Calcd: 521; MS Found: 522([M+H]$^+$). $^1$H NMR (400 MHz, DMSO-$d_6$)δ11.83 (s, 1H), 11.04 (s, 1H), 10.06 (s, 1H), 8.35 (s, 1H), 7.69 (s, 1H), 7.54 (s, 1H), 7.17 (s, 1H), 3.93 (q, *J* = 7.2 Hz, 2H), 3.12 (s, 3H), 3.06 (s, 3H), 2.16 - 2.07 (m, 1H), 1.99 - 1.90 (m, 1H), 1.21 (t, *J* = 7.2 Hz, 3H), 1.06 - 0.99 (m, 2H), 0.86 - 0.74 (m, 6H).

**Example 151**

4-((4-chloro-2-(N-methyl methanesulfonamido)phenyl)amino)-N-ethoxy-6-(pyridazin-3-ylamino)nicotinamide

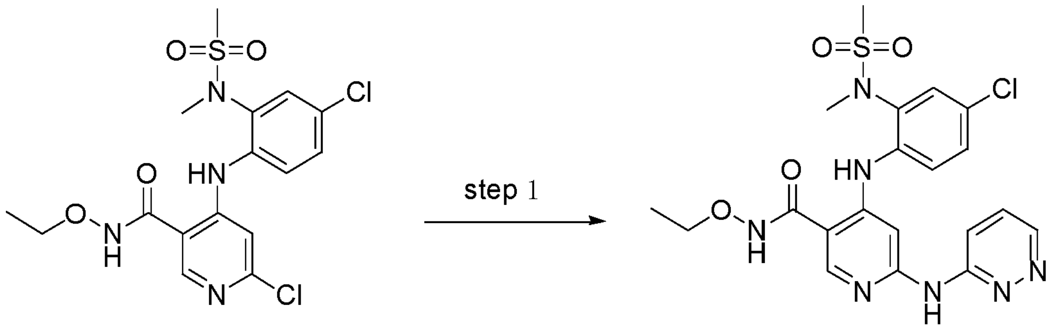


113-a

151

Step 1: 6-chloro-4-((4-chloro-2-(N-methyl methanesulfonamido)phenyl)amino)-N-ethoxy nicotinamide **(113-a,** 130.2 mg, 0.3 mmol), pyridazin-3-ylamine (31.38 mg, 0.33mmol), cesium carbonate (292.5 mg, 0.9mmol), XantPhos(34.68mg, 0.06mmol) and $Pd_2(dba)_3$(27.47mg, 0.03 mmol) were added to anhydrous dioxane (5 ml). After the atmosphere of the mixture was evacuated to vacuum and refilled with nitrogen, the reaction mixture was heated to 120°C with stirring for 6 hours, and filtered by suction. The filtrate was concentrated and purified by preparative TLC (MeOH:DCM=1:15) to provide the title compound 4-((4-chloro-2-(N-methyl methanesulfonamido)phenyl)amino)-N-ethoxy-6-(pyridazin-3-ylamino)nicotinamide **(151,** 25mg, 0.05mmol, 13.9% yield). MS Calcd: 491.11; MS Found: 492.20([M+H]$^+$). $^1$H NMR (400 MHz, DMSO-$d_6$)δ11.67 (s, 1H), 10.14 (s, 1H), 10.04 (s, 1H), 8.76 (dd, *J* = 4.4, 1.6 Hz, 1H), 8.35 (s, 1H), 8.03 (dd, *J* = 9.2, 1.6 Hz, 1H), 7.70 (d, *J* = 2.4 Hz, 1H), 7.62 (d, *J* = 8.8 Hz, 1H), 7.58 (s, 1H), 7.54 (dd, *J* = 9.2, 4.4 Hz, 1H), 7.46 (dd, *J* = 8.8, 2.4 Hz, 1H), 3.95 (q, *J* = 7.2 Hz, 2H), 3.18 (s, 3H), 3.16 (s, 3H), 1.22 (t, *J* = 7.2 Hz, 3H).

**Example 152**

4-((4-chloro-2-(N-methyl methanesulfonamido)phenyl)amino)-6-((5-cyclopropyl pyridin-2-yl)amino)-N-ethoxy nicotinamide

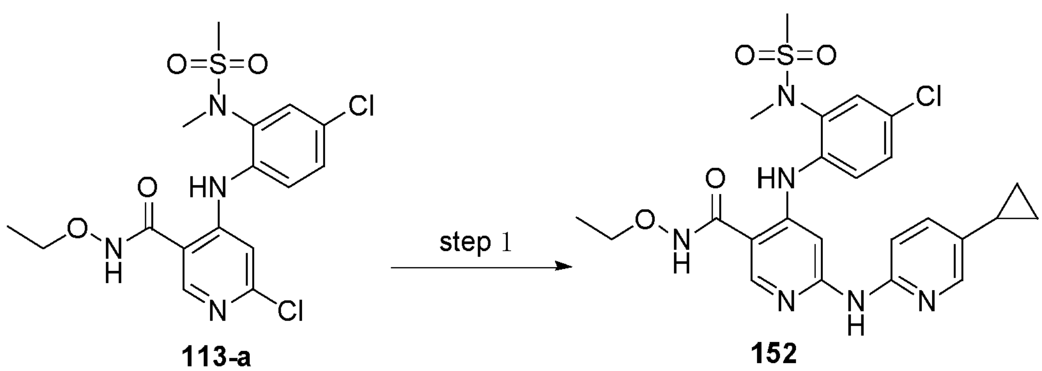


113-a

152

Step 1: 6-chloro-4-((4-chloro-2-((N-methyl methanesulfonamido)phenyl)amino)-N-ethoxy nicotinamide **(113-a,** 86.8 mg, 0.2 mmol), 5-cyclopropyl pyridin-2-ylamine (29.48 mg, 0.22mmol), cesium carbonate (195 mg, 0.6mmol), XantPhos(23.12mg, 0.04mmol) and $Pd_2(dba)_3$(18.31mg, 0.02 mmol) were added to anhydrous dioxane (5 ml). After the atmosphere of the mixture was evacuated to vacuum and refilled with nitrogen, the reaction mixture was heated to 120°C

with stirring for 6 hours, and filtered by suction. The filtrate was concentrated and purified by high performance preparative thin layer chromatography (MeOH:DCM=1:15) to provide the title compound: 4-((4-chloro-2-(N-methyl methanesulfonamido)phenyl)amino)-6-((5-cyclopropyl pyridin-2-yl)amino)-N-ethoxy nicotinamide **(152,** 30mg, 0.05mmol, 28.2% yield). MS Calcd: 530.15; MS Found: 531.11($[M+H]^+$).$^1$H NMR (400 MHz, DMSO-$d_6$)δ11.60 (s, 1H), 10.10 (s, 1H), 9.67 (s, 1H), 8.31 (s, 1H), 8.11 - 7.98 (m, 1H), 7.74 (s, 1H), 7.66-7.63 (m, 2H), 7.55 (dd, *J* = 8.8, 2.4 Hz, 1H), 7.46 (d, *J* = 8.6 Hz, 1H), 7.35 - 7.26 (m, 1H), 3.93 (q, *J* = 7.2 Hz, 2H), 3.17 (s, 6H), 1.91-1.85 (m, 1H), 1.21 (t, *J* = 7.2 Hz, 3H), 0.93-0.89 (m, 2H), 0.66-0.63 (m, 2H).

**Example 153**

4-((4-cyclopropyl-2-(N-methyl methanesulfonamido)phenyl)amino)-N-ethoxy-6-(pyridazin-3-ylamino)nicotinamide

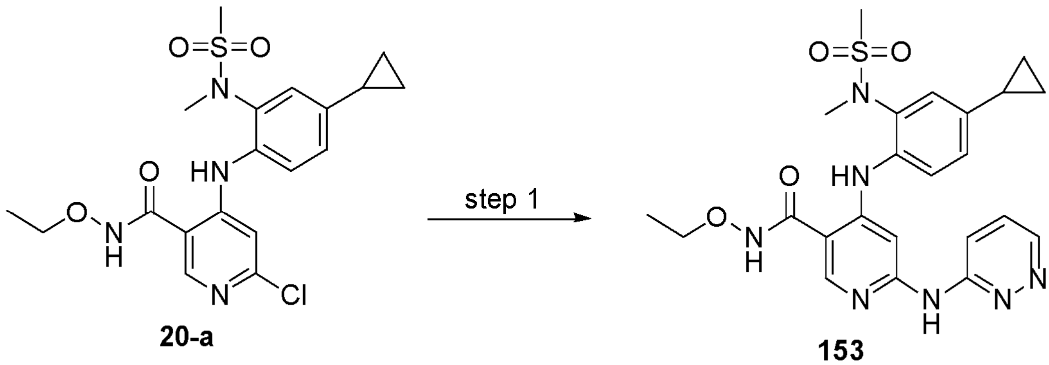


**20-a** **153**

Step 2: 6-chloro-4-((4-cyclopropyl-2-(N-methyl methanesulfonamido)phenyl)amino)-N-ethoxy nicotinamide **(20-a,** 131 mg, 0.3 mmol), pyridazin-3-ylamine (31.38 mg, 0.33mmol), cesium carbonate (292.5 mg, 0.9mmol), Xant-Phos(34.68mg, 0.06mmol) and $Pd_2(dba)_3$(27.47mg, 0.03 mmol) were added to anhydrous dioxane (5 ml). After the atmosphere of the mixture was evacuated to vacuum and refilled with nitrogen, the reaction mixture was heated to 120°C with stirring for 6 hours, and filtered by suction. The filtrate was concentrated and purified by high performance preparative thin layer chromatography (MeOH:DCM=1:15) to provide the title compound: 4-((4-cyclopropyl-2-(N-methyl methanesulfonamido)phenyl)amino)-N-ethoxy-6-(pyridazin-3-ylamino)nicotinamide **(153,** 20mg, 0.04mmol, 13.4% yield). MS Calcd: 497.18.23; MS Found: 498.20 ($[M+H]^+$). $^1$H NMR (400 MHz, DMSO-$d_6$)δ11.61 (s, 1H), 10.08 (s, 1H), 9.87 (s, 1H), 8.74 (dd, *J* = 4.8, 1.6 Hz, 1H), 8.32 (s, 1H), 8.05 (dd, *J* = 9.2, 1.6 Hz, 1H), 7.52 (dd, *J* = 9.2, 4.8 Hz, 1H), 7.46 - 7.40 (m, 2H), 7.27 (d, *J* = 2.0 Hz, 1H), 7.10 (dd, *J* = 8.4, 2.0 Hz, 1H), 3.94 (q, *J* = 7.2 Hz, 2H), 3.15 (s, 3H), 3.10 (s, 3H), 2.01-1.95 (m, 1H), 1.22 (t, *J* = 7.2 Hz, 3H), 1.04 - 0.94 (m, 2H), 0.74-0.70 (m, 2H).

**Example 154**

6-((5-cyclopropyl pyridin-2-yl)amino)-N-ethoxy-4-((4-ethynyl-2-(N-methyl methanesulfonamido)phenyl)amino)nicotinamide

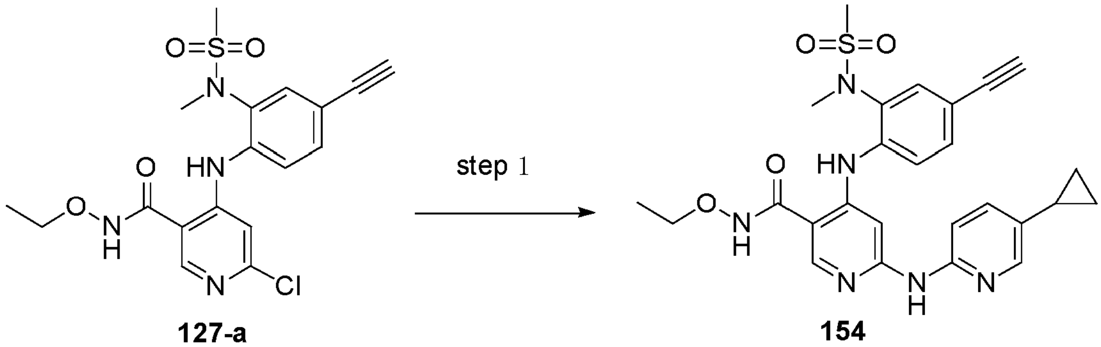


**127-a** **154**

Step 1: 6-chloro-N-ethoxy-4-((4-ethynyl-2-(N-methyl methanesulfonamido)phenyl)amino)nicotinamide **(127-a,** 120 mg, 0.3 mmol), 5-cyclopropyl pyridin-2-ylamine (40.2 mg, 0.33mmol), cesium carbonate (292 mg, 0.9mmol), XantPhos(34.72mg, 0.06mmol) and $Pd_2(dba)_3$(27.47mg, 0.03 mmol) were added to anhydrous dioxane (5 ml). After the atmosphere of the mixture was evacuated to vacuum and refilled with nitrogen, the reaction mixture was heated to 120°C with stirring for 6 hours, and filtered by suction. The filtrate was concentrated and purified by high performance preparative thin layer chromatography (MeOH:DCM=1:15) to provide the title compound: 6-((5-cyclopropyl pyridin-2-yl) amino)-N-ethoxy-4-((4-ethynyl-2-(N-methyl methanesulfonamido)phenyl)amino)nicotinamide **(154,** 25mg, 0.048mmol, 16.02% yield). MS Calcd: 520.19; MS Found: 521.11([M+H]$^+$). $^1$H NMR (400 MHz, DMSO-$d_6$)δ10.29 (s, 1H), 9.72 (s, 1H), 8.33 (s, 1H), 8.06 (d, *J* = 2.4 Hz, 1H), 7.90 (s, 1H), 7.68 - 7.64 (m, 2H), 7.57 (dd, *J* = 8.4, 2.0 Hz, 1H), 7.45 (d, *J* = 8.4 Hz, 1H), 7.31 (dd, *J* = 8.8, 2.4 Hz, 1H), 4.24 (s, 1H), 3.94 (q, *J* = 7.2 Hz, 2H), 3.18 (s, 3H), 3.17 (s, 4H), 1.92-1.88 (m, 1H), 1.21 (t, *J* = 7.2 Hz, 3H), 0.96 - 0.88 (m, 2H), 0.66 - 0.59 (m, 2H).

**Example 155**

6-((5-cyclopropyl pyridin-2-yl)amino)-N-ethoxy-4-((4-methoxy-2-(N-methyl methanesulfonamido)phenyl)amino)nicotinamide

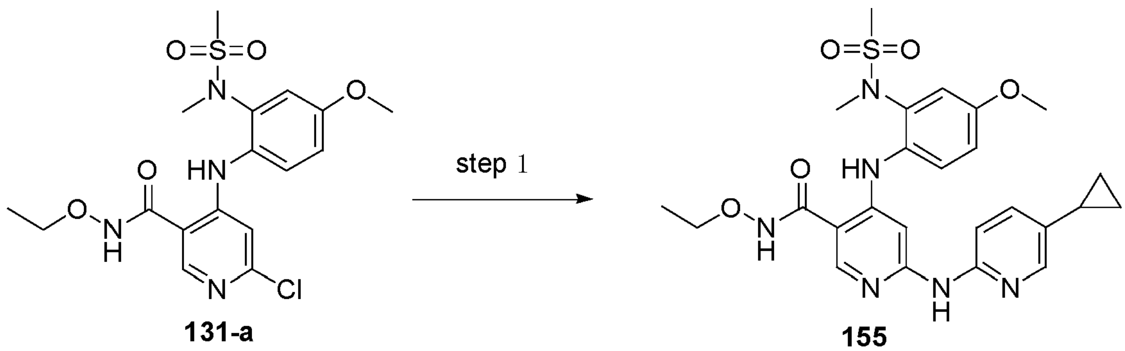


**131-a** **155**

Step 1: 6-chloro-N-ethoxy-4-((4-methoxy-2-(N-methyl methanesulfonamido)phenyl)amino)nicotinamide **(131-a,** 130 mg, 0.3 mmol), 5-cyclopropyl pyridin-2-ylamine (40.2 mg, 0.33mmol), cesium carbonate (292 mg, 0.9mmol), XantPhos(34.72mg, 0.06mmol) and $Pd_2(dba)_3$(27.47mg, 0.03 mmol) were added to anhydrous dioxane (5 ml). After the atmosphere of the mixture was evacuated to vacuum and refilled with nitrogen, the reaction mixture was heated to 120°C with stirring for 6 hours, and filtered by suction. The filtrate was concentrated and purified by high performance preparative thin layer chromatography (MeOH:DCM=1:15) to provide the title compound: 6-((5-cyclopropyl pyridin-2-yl)amino)-N-ethoxy-4-((4-methoxy-2-(N-methyl methanesulfonamido)phenyl)amino)nicotinamide **(155,** 30mg, 0.048mmol, 16.02% yield).

MS Calcd: 526.20; MS Found: 527.11([M+H]$^+$). $^1$H NMR (400 MHz, DMSO-$d_6$)δ11.50 (s, 1H), 9.67 (s, 1H), 9.52 (s, 1H), 8.26 (s, 1H), 7.95 (d, *J* = 2.4 Hz, 1H), 7.46 (dd, *J* = 8.8, 4.4 Hz, 2H), 7.38 (s, 1H), 7.28 (dd, *J* = 8.8, 2.4 Hz, 1H), 7.13 (d, *J* = 2.8 Hz, 1H), 7.08 (dd, *J* = 8.8, 2.8 Hz, 1H), 3.93 (q, *J* = 7.2 Hz, 2H), 3.83 (s, 3H), 3.14 (s, 3H), 3.09 (s, 3H), 1.88-1.82 (m, 1H), 1.21 (t, *J* = 7.2 Hz, 3H), 0.93 - 0.85 (m, 2H), 0.65 - 0.57 (m, 2H).

**Example 156**

4-((4-cyclopropyl-2-(N-methyl methanesulfonamido)phenyl)amino)-N-ethoxy-6-((5-methyl thiazol-2-yl)amino)nicotinamide

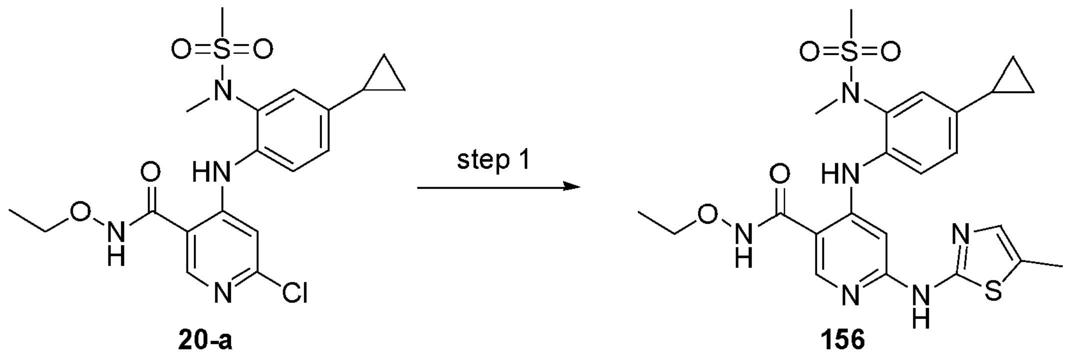


20-a 156

Step 1: 6-chloro-4-((4-cyclopropyl-2-(N-methyl methanesulfonamido)phenyl)amino)-N-ethoxy nicotinamide **(20-a,** 150 mg, 0.34 mmol), 5-methyl thiazol-2-ylamine (46.8 mg, 0.41mmol), cesium carbonate (333 mg, 0.9mmol), XantPhos(19.6mg, 0.034mmol) and $Pd_2(dba)_3$(15.64mg, 0.017 mmol) were added to anhydrous dioxane (5 ml). After the atmosphere of the mixture was evacuated to vacuum and refilled with nitrogen, the reaction mixture was heated to 120°C with stirring for 6 hours, and filtered by suction. The filtrate was concentrated and purified by high performance preparative thin layer chromatography (MeOH:DCM=1:15) to provide the title compound: 4-((4-cyclopropyl-2-(N-methyl methanesulfonamido)phenyl)amino)-N-ethoxy-6-((5-methyl thiazol-2-yl)amino)nicotinamide **(156,** 5mg, 0.01mmol, 2.8% yield). MS Calcd: 516.16; MS Found: 517.11($[M+H]^+$).$^1$H NMR (400 MHz, DMSO-$d_6$)δ11.60 (s, 1H), 10.81 (s, 1H), 9.73 (s, 1H), 8.31 (s, 1H), 7.37 (d, *J* = 8.4 Hz, 1H), 7.28 (d, *J* = 2.0 Hz, 1H), 7.10 (dd, *J* = 8.4, 2.0 Hz, 1H), 6.98 (d, *J* = 1.6 Hz, 1H), 6.62 (s, 1H), 3.93 (q, *J* = 7.2 Hz, 2H), 3.13 (s, 3H), 3.09 (s, 3H), 2.30 (d, *J* = 1.2 Hz, 3H), 2.01-1.94 (m, 1H), 1.22 (t, *J* = 7.2 Hz, 3H), 1.01-0.97 (m, 2H), 0.74 - 0.69 (m, 2H).

**Example 157**

4-((4-cyclopropyl-2-(N-methyl methanesulfonamido)phenyl)amino)-N-ethoxy-6-((4-methyl thiazol-2-yl)amino)nicotinamide

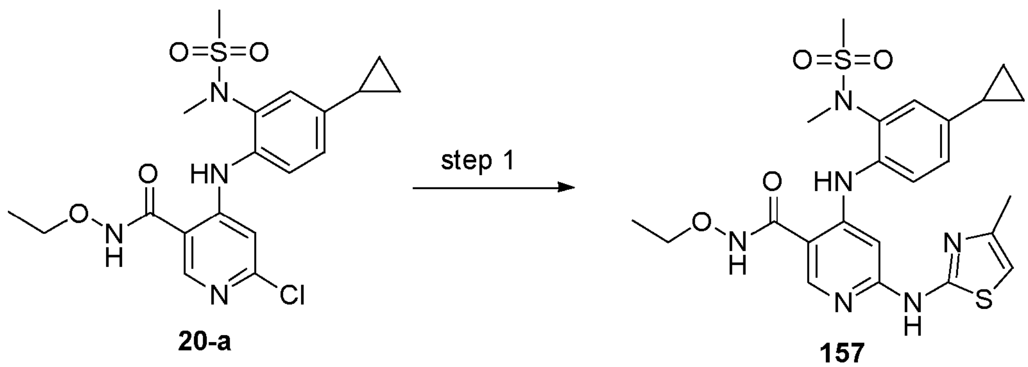


20-a 157

Step 1: 6-chloro-4-((4-cyclopropyl-2-(N-methyl methanesulfonamido)phenyl)amino)-N-ethoxy nicotinamide **(20-a,** 150 mg, 0.34 mmol), 4-methyl thiazol-2-ylamine (46.8 mg, 0.41mmol), cesium carbonate (333 mg, 0.9mmol), XantPhos(19.6mg, 0.034mmol) and $Pd_2(dba)_3$(15.64mg, 0.017 mmol) were added to anhydrous dioxane (5 ml). After the atmosphere of the mixture was evacuated to vacuum and refilled with nitrogen, the reaction mixture was heated to 120°C with stirring for 6 hours, and filtered by suction. The filtrate was concentrated and purified by high performance preparative thin layer chromatography (MeOH:DCM=1:15) to provide the title compound: 4-((4-cyclopropyl-2-(N-methyl methanesulfonamido)phenyl)amino)-N-ethoxy-6-((4-methyl thiazol-2-yl)amino)nicotinamide **(157,** 6mg, 0.011mmol, 3.42% yield). MS Calcd: 516.16; MS Found: 517.11($[M+H]^+$). $^1$H NMR (400 MHz, DMSO-$d_6$)δ11.60 (s, 1H), 10.94 (s, 1H), 9.76 (s, 1H), 8.33 (s, 1H), 7.38 (d, *J* = 8.4 Hz, 1H), 7.28 (d, *J* = 2.0 Hz, 1H), 7.10 (dd, *J* = 8.4, 2.0 Hz, 1H), 6.70 (s, 1H), 6.53 (d, *J* = 1.2 Hz, 1H), 3.93 (q, *J* = 7.2 Hz, 2H), 3.57 (s, 3H), 3.13 (s, 3H), 3.09 (s, 3H), 2.03 - 1.92 (m, 1H), 1.22 (t, *J* = 7.2 Hz, 3H), 1.01-0.97 (m, 2H), 0.73-0.69 (m, 2H).

Example **158**

4-((4-cyclopropyl-2-(N-methyl methanesulfonamido)phenyl)amino)-N-ethoxy-6-(thiazol-2-ylamino)nicotinamide

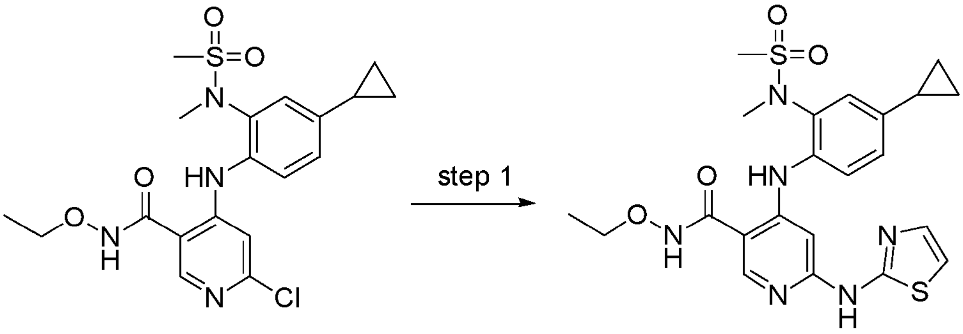


**20-a** **158**

Step 1: To a 50ml flask were added 6-chloro-4-((4-cyclopropyl-2-(N-methyl methanesulfonamido)phenyl)amino)-N-ethoxy nicotinamide **(20-a,** 150mg, 0.34mmol), thiazol-2-ylamine (34.3mg, 0.34mmol), XantPhos(79mg, 0.14mmol), diox (3ml), and $Cs_2CO_3$ (335mg, 1.02mmol). The reaction was vacuumed and refilled with $N_2$ twice, followed by adding $Pd(dba)_2$ (94mg, 0.1mmol). After that, the reaction was vacuumed and refilled with $N_2$ three times and allowed to proceed at 120°C for 4h. Upon indication of completed reaction by TLC (DCM/MeOH=15/1), the system was cooled, and evaporated directly in the presence of silica gel for loading onto and separating by column chromatography, eluted with DCM/MeOH=50/1~30/1(with 0.1ml of acetic acid per 100ml of solvent mixture) to provide the title compound: 4-((4-cyclopropyl-2-(N-methyl methanesulfonamido)phenyl)amino)-N-ethoxy-6-(thiazol-2-ylamino)nicotinamide **(158,** 30mg, 0.06mmol, 16% yield). MS Calcd: 502; MS Found: 503 ($[M+H]^+$). $^1H$ NMR (400 MHz, DMSO-$d_6$)δ10.94 (s, 1H), 10.09 (s, 1H), 8.40 (s, 1H), 7.37 (d, *J* = 8.4 Hz, 1H), 7.31 (d, *J* = 3.6 Hz, 1H), 7.27 (d, *J* = 2.0 Hz, 1H), 7.09 (dd, *J* = 8.4, 2.0 Hz, 1H), 6.95 (d, *J* = 3.6 Hz, 1H), 6.62 (s, 1H), 3.91 (q, *J* = 7.2 Hz, 2H), 3.14 (s, 3H), 3.07 (s, 3H), 2.00-1.94 (m, 1H), 1.22 (t, *J* = 7.2 Hz, 3H), 1.03 - 0.94 (m, 2H), 0.75 - 0.65 (m, 2H).

Example **159**

4-((4-cyclopropyl-2-(N-methyl methanesulfonamido)phenyl)amino)-N-ethoxy-6-((6-methyl pyridazin-3-yl)amino)nicotinamide

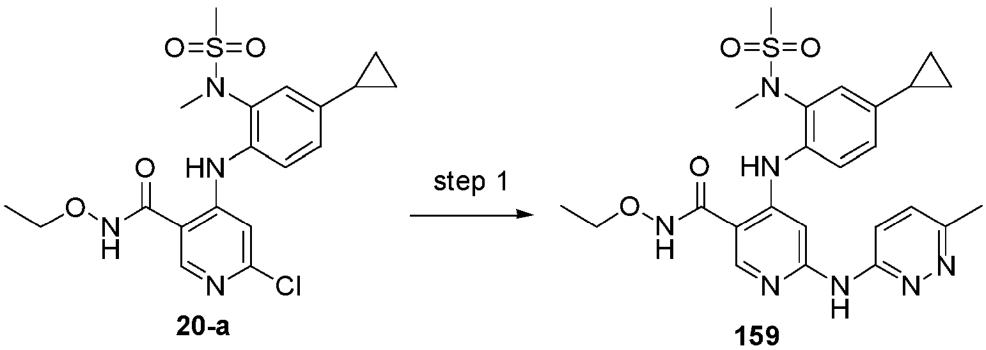


**20-a** **159**

Step 1: To a 50ml flask were added 6-chloro-4-((4-cyclopropyl-2-(N-methyl methanesulfonamido)phenyl)amino)-N-ethoxy nicotinamide **(20-a,** 150mg, 0.34mmol), 6-methyl pyridazin-3-ylamine (75mg, 0.68mmol), XantPhos(79mg, 0.14mmol), diox (3ml), and $Cs_2CO_3$ (335mg, 1.02mmol). The reaction was vacuumed and refilled with $N_2$ twice, followed by adding $Pd(dba)_2$ (94mg, 0.1mmol). After that, the reaction was vacuumed and refilled with $N_2$ three times and allowed to proceed at 120°C for 4h. Upon indication of completed reaction by TLC (DCM/MeOH=15/1), the system was cooled, and evaporated directly in the presence of silica gel for loading onto and separating by column chromatography, eluted with DCM/MeOH=50/1~30/1(with 0.1ml of acetic acid per 100ml of solvent mixture) to provide the title compound: 4-((4-cyclopropyl-2-(N-methyl methanesulfonamido)phenyl)amino)-N-ethoxy-6-((6-methyl pyridazin-3-yl)amino)nicotinamide **(159,** 38mg, 0.07mmol, 18% yield). MS Calcd: 511; MS Found: 512($[M+H]^+$). $^1H$ NMR (400 MHz, DMSO-$d_6$)δ11.59 (s, 1H),

9.98 (s, 1H), 9.85 (s, 1H), 8.29 (s, 1H), 7.96 (d, *J* = 9.1 Hz, 1H), 7.42 (dd, *J* = 8.8, 6.1 Hz, 2H), 7.38 (s, 1H), 7.27 (d, *J* = 2.0 Hz, 1H), 7.10 (dd, *J* = 8.4, 2.1 Hz, 1H), 3.93 (q, *J* = 7.0 Hz, 2H), 3.34 (s, 3H), 3.15 (s, 3H), 3.10 (s, 3H), 1.98 (tt, *J*= 8.4, 5.1 Hz, 1H), 1.22 (t, *J*= 7.0 Hz, 4H), 0.99 (dt, *J* = 8.8, 3.1 Hz, 2H), 0.77 - 0.66 (m, 2H).

Example **160**

4-((4-cyclopropyl-2-(N-methyl methanesulfonamido)phenyl)amino)-N-ethoxy-6-((1-methyl-1H-pyrazol-3-yl)amino)nicotinamide

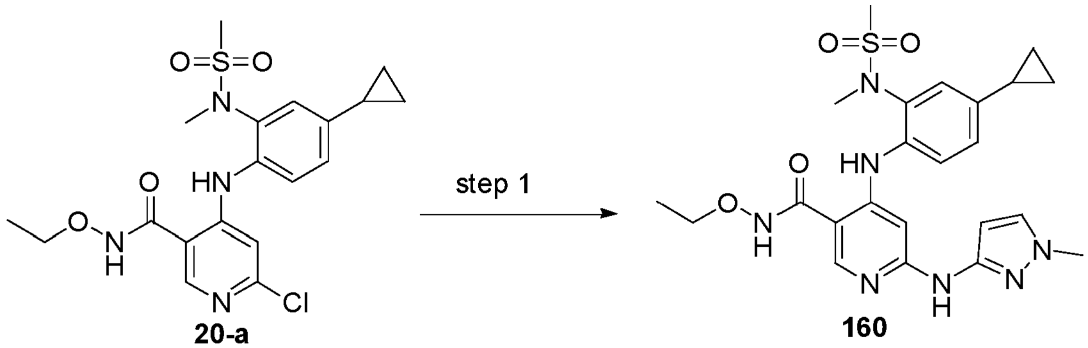


**20-a** **160**

Step 1: 6-chloro-4-((4-cyclopropyl-2-(N-methyl methanesulfonamido)phenyl)amino)-N-ethoxy nicotinamide **(20-a,** 150mg, 0.342mmol), N-methyl-3-aminopyrazole (43.22mg, 0.445mmol), $Pd_2(dba)_3$(93.95mg, 0.103mmol), Xant-Phos(118.73mg, 0.205mmol), $Cs_2CO_3$ (389.98mg, 1.197mmol), and 1,4-dioxane (7mL) were combined. After atmosphere replacement with nitrogen three times, the mixture was allowed to react at 120°Cfor 6 hours. When TLC indicated a completed reaction, the mixture was filtered by suction, and the filtrate was concentrated under reduced pressure at 40°C, mixed with 200-300 mesh silica gel for loading onto and purifying by column chromatography, eluted with DCM:MeOH=30:1, to provide the target compound: 4-((4-cyclopropyl-2-(N-methyl methanesulfonamido)phenyl)amino)-N-ethoxy-6-((1-methyl-1H-pyrazol-3-yl)amino)nicotinamide **(160,** 27mg, 0.054mmol, 15.88% yield). MS Calcd:499; MS Found: 500($[M+H]^+$). $^1H$ NMR (400 MHz, DMSO-$d_6$)δ11.44 (s, 1H), 9.88 (s, 1H), 9.26 (s, 1H), 8.22 (s, 1H), 7.52 - 7.40 (m, 2H), 7.32 - 7.16 (m, 2H), 7.11 (dd, *J* = 8.4, 2.0 Hz, 1H), 6.06 (d, *J* = 2.0 Hz, 1H), 3.92 (q, *J* = 7.2 Hz, 2H), 3.69 (s, 3H), 3.13 (s, 3H), 3.10 (s, 3H), 2.00 - 1.91 (m, 1H), 1.21 (t, *J* = 7.2 Hz, 3H), 1.01 - 0.93 (m, 2H), 0.74 - 0.63 (m, 2H).

Example **161**

4-((4-cyclopropyl-2-(N-methyl methanesulfonamido)phenyl)amino)-N-ethoxy-6-((1-methyl-1H-pyrazol-5-yl)amino)nicotinamide

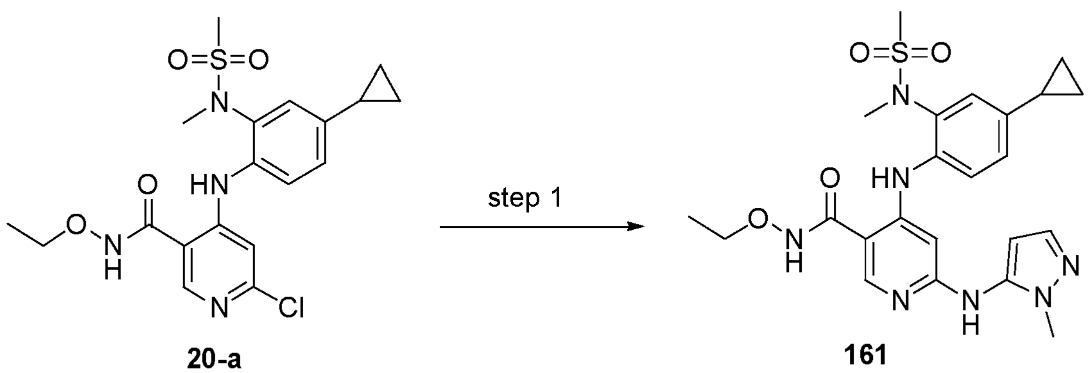


**20-a** **161**

Step 1: 6-chloro-4-((4-cyclopropyl-2-(N-methyl methanesulfonamido)phenyl)amino)-N-ethoxy nicotinamide **(20-a,** 150mg, 0.342mmol), 1-methyl-5-aminopyrazole (43.22mg, 0.445mmol), $Pd_2(dba)_3$(93.95mg, 0.103mmol), Xant-Phos(118.73mg, 0.205mmol), $Cs_2CO_3$ (389.98mg, 1.197mmol), and 1,4-dioxane (7mL) were combined. After atmosphere replacement with nitrogen three times, the mixture was allowed to react at 120°C for 6 hours. When TLC indicated a completed reaction, the mixture was filtered by suction, and the filtrate was concentrated under reduced pressure at 40°C, mixed with 200-300 mesh silica gel for loading onto and purifying by column chromatography, eluted with DCM:MeOH=30:1, to provide the target compound: 4-((4-cyclopropyl-2-(N-methyl methanesulfonamido)phenyl)amino)-N-ethoxy-6-((1-methyl-1H-pyrazol-5-yl)amino)nicotinamide **(161,** 30mg, 0.060mmol, 17.64% yield). MS Calcd:499; MS Found: 500([M+H]$^+$). $^1$H NMR (400 MHz, DMSO-$d_6$)δ11.51 (s, 1H), 9.72 (s, 1H), 8.80 (s, 1H), 8.20 (s, 1H), 7.34 (d, *J* = 8.4 Hz, 1H), 7.28 (d, *J* = 2.0 Hz, 1H), 7.26 (d, *J* = 2.0 Hz, 1H), 7.08 (dd, *J* = 8.4, 2.0 Hz, 1H), 6.30 (s, 1H), 6.16 (d, *J* = 2.0 Hz, 1H), 3.91 (q, *J* = 7.2 Hz, 2H), 3.61 (s, 3H), 3.12 (s, 3H), 3.08 (s, 3H), 1.99 - 1.90 (m, 1H), 1.20 (t, *J* = 7.2 Hz, 3H), 1.00 - 0.94 (m, 2H), 0.72-0.68 (m, 2H).

Example **162**

N-ethoxy-4-((4-ethynyl-2-(N-methyl methanesulfonamido)phenyl)amino)-6-(pyridin-2-ylamino)nicotinamide

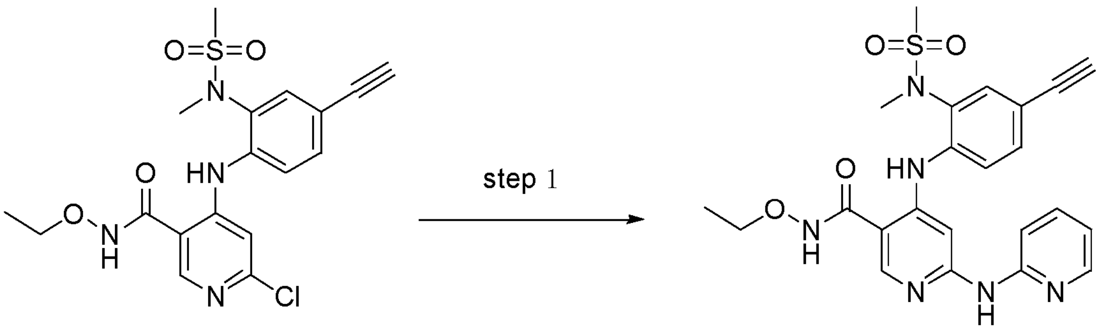


**127-a** **162**

Step 1: 6-chloro-N-ethoxy-4-((4-ethynyl-2-(N-methyl methanesulfonamido)phenyl)amino)nicotinamide **(127-a,** 150mg, 0. 355mmol), 2-aminopyridine (43.48mg, 0.462mmol), $Pd_2(dba)_3$(97.90mg, 0.107mmol), XantPhos(123.12mg, 0.213mmol), $Cs_2CO_3$ (347.42mg, 1.067mmol), and 1,4-dioxane (7mL) were combined. After atmosphere replacement with nitrogen three times, the mixture was allowed to react at 120°C for 6 hours. When TLC indicated a completed reaction, the mixture was filtered by suction, and the filtrate was concentrated under reduced pressure at 40°C, mixed with 200-300 mesh silica gel for loading onto and purifying by column chromatography, eluted with DCM:MeOH=30:1, to provide the target compound: N-ethoxy-4-((4-ethynyl-2-(N-methyl methanesulfonamido)phenyl)amino)-6-(pyridin-2-ylamino)nicotinamide **(162,** 18mg, 0.0375mmol, 10.56% yield).

MS Calcd:480; MS Found:481([M+H]$^+$). $^1$H NMR (400 MHz, DMSO-$d_6$)δ11.64 (s, 1H), 10.28 (s, 1H), 9.83 (s, 1H), 8.35 (s, 1H), 8.21 (dd, *J* = 5.2, 2.0 Hz, 1H), 7.98 (s, 1H), 7.70 - 7.60 (m, 3H), 7.59 - 7.50 (m, 2H), 6.91 - 6.84 (m, 1H), 4.23 (s, 1H), 3.94 (q, *J* = 7.2 Hz, 2H), 3.18 (s, 3H), 3.17 (s, 3H), 1.21 (t, *J* = 7.2 Hz, 3H).

Example **163**

N-ethoxy-4-((4-ethynyl-2-(N-methyl methanesulfonamido)phenyl)amino)-6-(pyrimidin-2-ylamino)nicotinamide

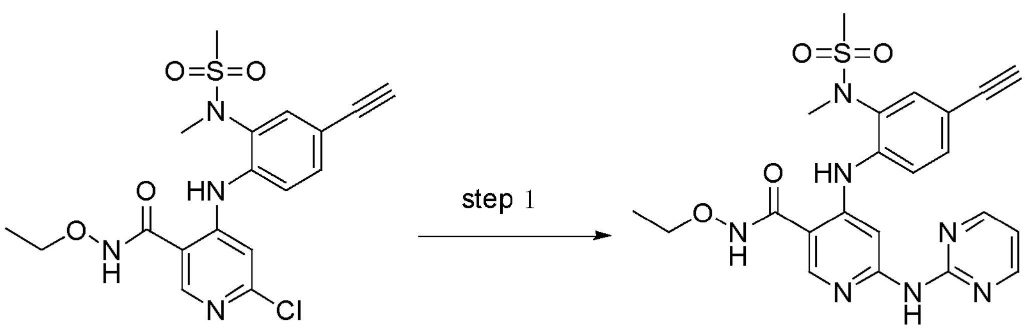


**127-a** **163**

Step 1: 6-chloro-N-ethoxy-4-((4-ethynyl-2-(N-methyl methanesulfonamido)phenyl)amino)nicotinamide **(127-a,** 150mg, 0. 355mmol), 2-aminopyrimidine (43.89mg, 0.461mmol), $Pd_2(dba)_3$(97.90mg, 0.107mmol), Xant-Phos(123.12mg, 0.213mmol), $Cs_2CO_3$ (347.42mg, 1.067mmol), and 1,4-dioxane (7mL) were combined. After atmosphere replacement with nitrogen three times, the mixture was allowed to react at 120°C for 6 hours. When TLC indicated a completed reaction, the mixture was filtered by suction, and the filtrate was concentrated under reduced pressure at 40°C, mixed with 200-300 mesh silica gel for loading onto and purifying by column chromatography, eluted with DCM:MeOH=30:1, to provide the target compound N-ethoxy-4-((4-ethynyl-2-(N-methyl methanesulfonamido)phenyl) amino)-6-(pyrimidin-2-ylamino)nicotinamide **(163,** 31mg, 0.064mmol, 18.24% yield). MS Calcd:481; MS Found:482($[M+H]^+$). $^1H$ NMR (400 MHz, DMSO-$d_6$)δ11.70 (s, 1H), 10.36 (s, 1H), 10.07 (s, 1H), 8.58 (d, *J* = 4.8 Hz, 2H), 8.38 (d, *J* = 4.8 Hz, 2H), 7.73 (d, *J* = 8.4 Hz, 1H), 7.67 (d, *J* = 2.0 Hz, 1H), 7.62 (dd, *J* = 8.4, 2.0 Hz, 1H), 6.99 (t, *J* = 4.8 Hz, 1H), 4.24 (s, 1H), 3.95 (q, *J* = 7.2 Hz, 2H), 3.19 (s, 6H), 1.22 (t, *J* = 7.2 Hz, 3H).

Example **164**

6-((4,6-dimethyl pyrimidin-2-yl)amino)-N-ethoxy-4-((4-ethynyl-2-(N-methyl methanesulfonamido)phenyl)amino)nicotinamide

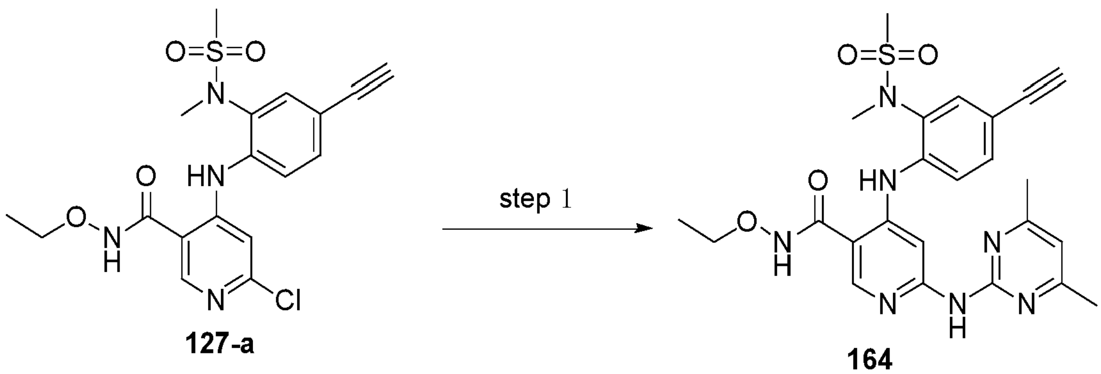


**127-a** **164**

Step 1: 6-chloro-N-ethoxy-4-((4-ethynyl-2-(N-methyl methanesulfonamido)phenyl)amino)nicotinamide **(127-a,** 150mg, 0.355mmol), 4-amino-2,6-dimethyl pyrimidine (56.84mg, 0.462mmol), $Pd_2(dba)_3$(97.90mg, 0.107mmol), Xant-Phos(123.12mg, 0.213mmol), $Cs_2CO_3$ (347.42mg, 1.067mmol), and 1,4-dioxane (7mL) were combined. After atmosphere replacement with nitrogen three times, the mixture was allowed to react at 120°C for 6 hours. When TLC indicated a completed reaction, the mixture was filtered by suction, and the filtrate was concentrated under reduced pressure at 40°C, mixed with 200-300 mesh silica gel for loading onto and purifying by column chromatography, eluted with DCM:MeOH=30:1, to provide the target compound: 6-((4,6-dimethyl pyrimidin-2-yl)amino)-N-ethoxy-4-((4-ethynyl-2-(N-methyl methanesulfonamido)phenyl)amino)nicotinamide **(164,** 18mg, 0.0353mmol, 9.94% yield).

MS Calcd:509; MS Found:510($[M+H]^+$). $^1H$ NMR (400 MHz, DMSO-$d_6$)δ11.65 (brs, 1H), 10.35 (s, 1H), 9.71 (s, 1H), 8.46 (s, 1H), 8.35 (s, 1H), 7.76 - 7.66 (m, 2H), 7.53 (dd, *J* = 8.4, 2.0 Hz, 1H), 6.77 (s, 1H), 4.24 (s, 1H), 3.95 (q, *J* = 7.2 Hz, 2H), 3.18 (s, 3H), 3.17 (s, 3H), 2.31 (s, 6H), 1.23 (t, *J* = 7.2 Hz, 3H).

Example **165**

N-ethoxy-4-((4-methyl-2-(N-methyl methanesulfonamido)phenyl)amino)-6-((4-methyl thiazol-2-yl)amino)nicotinamide

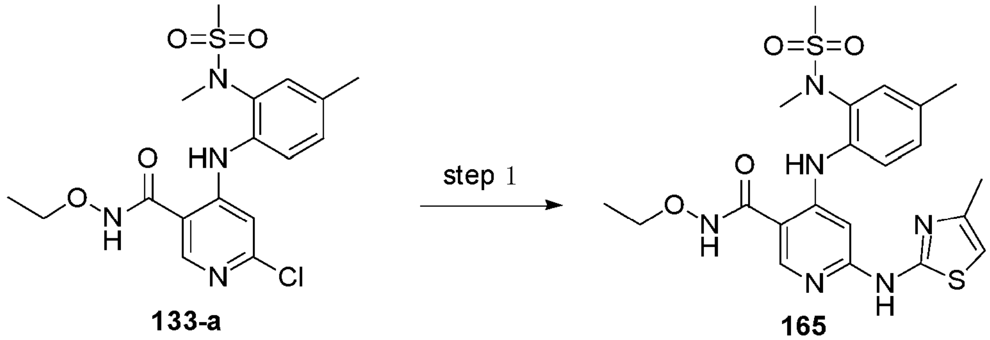

133-a

165

Step 1: To anhydrous dioxane (8ml) were added 6-chloro-N-ethoxy-4-((4-methyl-2-(N-methyl methanesulfonamido)phenyl)amino)nicotinamide **(133-a,** 100mg, 0.24mmol), 4-methyl thiazol-2-ylamine (41mg, 0.36mmol), cesium carbonate (234mg, 0.72mmol), XantPhos(27.4mg, 0.048mmol) and $Pd_2(dba)_3$(22mg, 0.024mmol). After atmosphere replacement with nitrogen three times, the reaction mixture was heated to 120°C with stirring for 8h, and filtered by suction. The filtrate was concentrated and purified by high performance preparative thin layer chromatography (MeOH:DCM=1:20) to provide the title compound: N-ethoxy-4-((4-methyl-2-(N-methyl methanesulfonamido)phenyl) amino)-6-((4-methyl thiazol-2-yl)amino)nicotinamide **(165,** 20mg, 0.04mmol, 16% yield). MS Calcd: 490; MS Found: 491([M+H]$^+$). $^1$H NMR (400 MHz, DMSO-$d_6$)δ11.60 (s, 1H), 10.96 (s, 1H), 9.79 (s, 1H), 8.34 (s, 1H), 7.40 (d, $J$ = 7.2 Hz, 1H), 7.39 (s, 1H), 7.23 (dd, $J$ = 8.2, 2.0 Hz, 1H), 6.72 (s, 1H), 6.53 (d, $J$ = 1.2 Hz, 1H), 3.93 (q, $J$ = 7.2 Hz, 2H), 3.13 (s, 3H), 3.10 (s, 3H), 2.35 (s, 3H), 2.20 (d, $J$ = 1.2 Hz, 3H), 1.21 (t, $J$ = 7.2 Hz, 3H).

Example **166**

6-(cyclopropylcarboxamido)-N-ethoxy-4-((2-methoxy-3-(1-methyl-1H-1,2,4-triazol-3-yl)phenyl)amino)nicotinamide

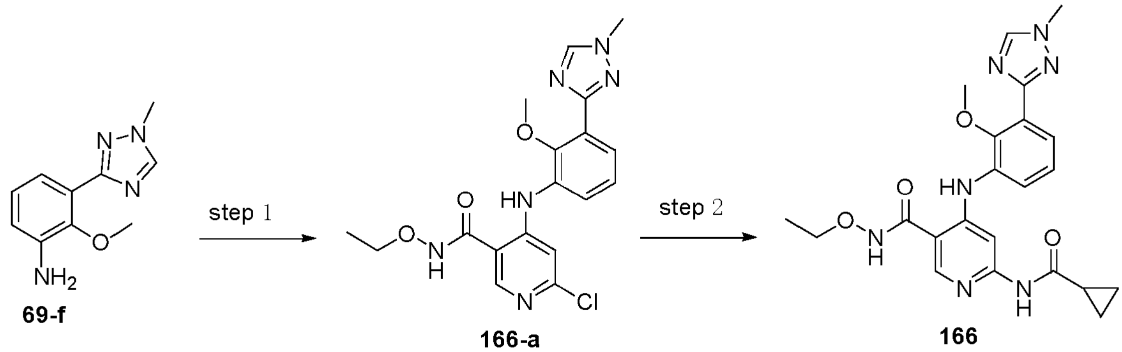

69-f

166-a

166

Step 1: To a 100mL two-necked flask were sequentially added 4,6-dichloro-N-ethoxy nicotinamide **(int-2,** 374mg, 1.59mmol), 2-methoxy-3-(1-methyl-1H-1,2,4-triazol-3-yl)aniline **(69-f,** 250mg, 1.22mmol), and 10ml of anhydrous tetrahydrofuran as solvent. The mixture was cooled in ice bath and added with a solution of LiHMDS in tetrahydrofuran (4.88ml, 1mmol/mL), with continuous stirring at room temperature for 6h. Upon indication of completed reaction by TLC, the mixture was quenched with saturated ammonium chloride solution, and extracted with ethyl acetate (30mLx3). The combined organic layers were dried and concentrated, followed by purified by column chromatography to provide the title compound: 6-chloro-N-ethoxy-4-((2-methoxy-3-(1-methyl-1H-1,2,4-triazol-3-yl)phenyl)amino)nicotinamide **(166-a,** 313mg, 0.77mmol, 63% yield). MS Calcd: 402; MS Found: 403([M+H]$^+$).

Step 2: 6-chloro-N-ethoxy-4-((2-methoxy-3-(1-methyl-1H-1,2,4-triazol-3-yl)phenyl)amino)nicotinamide **(166,** 150mg, 0.37mmol), cyclopropylcarboxamido(47mg, 0.55mmol), cesium carbonate (360mg, 1.11mmol), XantPhos(42mg, 0.007mmol) and $Pd_2(dba)_3$(35mg, 0.04mmol) were added to anhydrous dioxane (10ml). After atmosphere replacement with nitrogen three times, the reaction mixture was heated to 120°C with stirring for 8h, and filtered by suction. The filtrate was concentrated and purified by high performance preparative thin layer chromatography (MeOH:DCM=1:15) to provide the title compound: 6-(cyclopropylcarboxamido)-N-ethoxy-4-((2-methoxy-3-(1-methyl-1H-1,2,4-triazol-3-yl)phenyl)amino)nicotinamide **(166,** 80mg, 0.18mmol, 48% yield). MS Calcd: 451; MS Found: 452([M+H]$^+$). $^1$H NMR (400 MHz, DMSO-$d_6$)δ11.76 (s, 1H), 10.82 (s, 1H), 10.16 (s, 1H), 8.56 (s, 1H), 8.39 (s, 1H), 8.08 (s, 1H), 7.58 (dd, $J$ = 8.0, 1.6 Hz, 1H), 7.49 (dd, $J$ = 8.0, 1.6 Hz, 1H), 7.23 (t, $J$ = 8.0 Hz, 1H), 3.98-3.93 (m, 5H), 3.72 (s, 3H), 2.02 - 1.94 (m, 1H), 1.23 (t, $J$ = 7.2 Hz,

3H), 0.80 - 0.73 (m, 4H).

Example **167**

4-((4-cyclopropyl-5-fluoro-2-(N-methyl methanesulfonamido)phenyl)amino)-N-ethoxy-6-(pyrazin-2-ylamino)nicotinamide

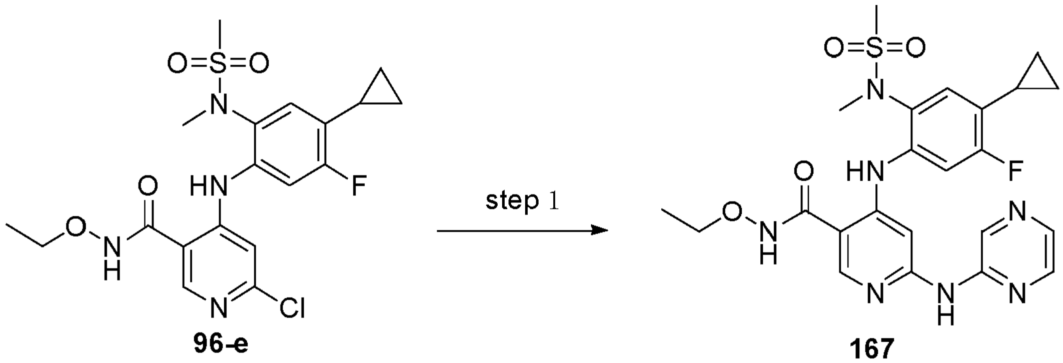


96-e

167

Step 1: 6-chloro-4-((4-cyclopropyl-5-fluoro-2-(N-methyl methanesulfonamido)phenyl)amino)-N-ethoxy nicotinamide **(96-e,** 278mg, 0.5mmol), 2-aminopyrazine (48mg, 0.5mmol), cesium carbonate (326mg, 1.0mmol), XantPhos(57.6mg, 0.1mmol) and $Pd_2(dba)_3$(45.5mg, 0.05mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated, and subjected to high performance thin layer preparative chromatography to provide the title compound: 4-((4-cyclopropyl-5-fluoro-2-(N-methyl methanesulfonamido) phenyl)amino)-N-ethoxy-6-(pyrazin-2-ylamino)nicotinamide

**(167,** 20mg, 0.039mmol, 7.8% yield). MS Calcd: 515; MS Found: 516 ($[M+H]^+$). $^1H$ NMR (400 MHz, DMSO-$d_6$) δ11.66 (s, 1H), 10.14 (s, 1H), 10.13 (s, 1H), 8.99 (d, *J* = 1.6 Hz, 1H), 8.37 (s, 1H), 8.17 (dd, *J* = 2.8, 1.6 Hz, 1H), 8.11 (d, *J* = 2.8 Hz, 1H), 7.67 (d, *J* = 5.8 Hz, 1H), 7.41 (d, *J* = 12.0 Hz, 1H), 7.16 (d, *J* = 8.0 Hz, 1H), 3.94 (q, *J* = 7.2 Hz, 2H), 3.14 (s, 3H), 3.10 (s, 3H), 2.07-2.00 (m, 1H), 1.22 (t, *J* = 7.2 Hz, 3H), 1.01-0.97 (m, 2H), 0.81-0.77 (m, 2H).

Example **168**

4-((4-cyclopropyl-5-fluoro-2-(N-methyl methanesulfonamido)phenyl)amino)-N-ethoxy-6-((1-methyl-1H-pyrazol-5-yl) amino)nicotinamide

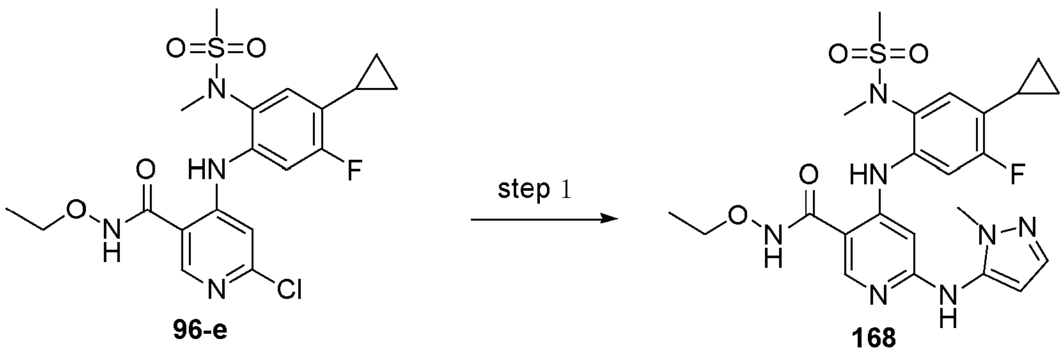


96-e

168

Step 1: 6-chloro-4-((4-cyclopropyl-5-fluoro-2-(N-methyl methanesulfonamido)phenyl)amino)-N-ethoxy nicotinamide **(96-e,** 278mg, 0.5mmol), 5-amino-methyl pyrazole (49mg, 0.5mmol), cesium carbonate (326mg, 1.0mmol), XantPhos(57.6mg, 0.1mmol) and $Pd_2(dba)_3$(45.5mg, 0.05mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and subjected to high performance thin layer

preparative chromatography to provide the title compound: 4-((4-cyclopropyl-5-fluoro-2-(N-methyl methanesulfonamido) phenyl)amino)-N-ethoxy-6-((1-methyl-1*H*-pyrazol-5-yl)amino)nicotinamide **(168,** 28mg, 0.054mmol, 10.8% yield). MS Calcd: 517; MS Found: 518 ([M+H]$^+$). $^1$H NMR (400 MHz, DMSO-$d_6$)δ11.55 (s, 1H), 9.93 (s, 1H), 8.88 (s, 1H), 8.23 (s, 1H), 7.43 - 7.36 (m, 1H), 7.31 (d, *J* = 1.6 Hz, 1H), 7.14 (d, *J* = 8.0 Hz, 1H), 6.47 (s, 1H), 6.21 (d, *J* = 2.0 Hz, 1H), 3.91 (q, *J* = 7.2 Hz, 2H), 3.63 (s, 3H), 3.12 (s, 3H), 3.07 (s, 3H), 2.04 - 1.96 (m, 1H), 1.21 (t, *J* = 7.2 Hz, 3H), 0.99 - 0.95 (m, 2H), 0.79-0.75 (m, 2H).

Example **169**

4-((4-cyclopropyl-5-fluoro-2-(N-methyl methanesulfonamido)phenyl)amino)-N-ethoxy-6-(pyrimidin-2-ylamino)nicotinamide

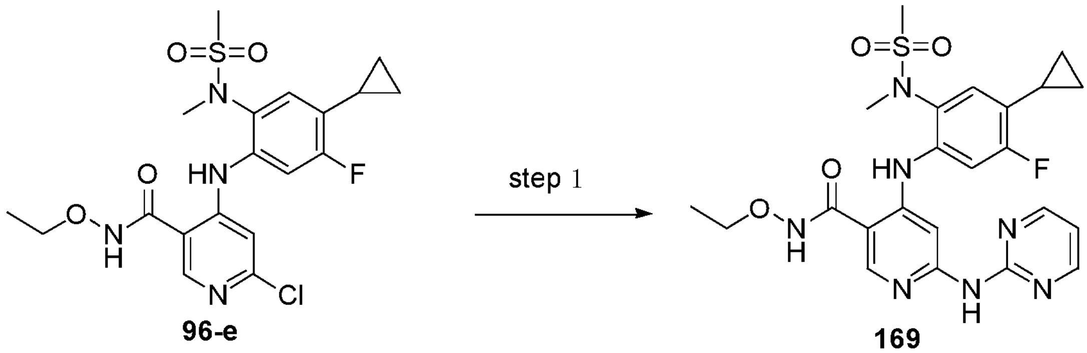


**96-e** **169**

Step 1: 6-chloro-4-((4-cyclopropyl-5-fluoro-2-(N-methyl methanesulfonamido)phenyl)amino)-N-ethoxy nicotinamide **(96-e,** 278mg, 0.5mmol), 2-aminopyrimidine (48mg, 0.5mmol), cesium carbonate (326mg, 1.0mmol), XantPhos(57.6mg, 0.1mmol) and $Pd_2(dba)_3$(45.5mg, 0.05mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and subjected to high performance thin layer preparative chromatography to provide the title compound: 4-((4-cyclopropyl-5-fluoro-2-(N-methyl methanesulfonamido) phenyl)amino)-N-ethoxy-6-(pyrimidin-2-ylamino)nicotinamide (169, 40mg, 0.078mmol, 15.6% yield). MS Calcd: 515; MS Found: 516 ([M+H]$^+$). $^1$H NMR (400 MHz, DMSO-$d_6$)δ11.70 (s, 1H), 10.21 (s, 1H), 10.03 (s, 1H), 8.52 (s, 1H), 8.50 (s, 1H), 8.36 (s, 1H), 8.29 (s, 1H), 7.51 (d, *J* = 12.4 Hz, 1H), 7.16 (d, *J* = 8.0 Hz, 1H), 7.01 (t, *J* = 4.8 Hz, 1H), 3.94 (q, *J* = 7.2 Hz, 2H), 3.14 (s, 3H), 3.11 (s, 3H), 2.07-2.00 (m, 1H), 1.22 (t, *J* = 7.2 Hz, 3H), 1.02 - 0.94 (m, 2H), 0.81-0.78(m, 2H).

Example **170**

4-((4-cyclopropyl-5-fluoro-2-(N-methyl methanesulfonamido)phenyl)amino)-N-ethoxy-6-((4-methyl thiazol-2-yl)amino) nicotinamide

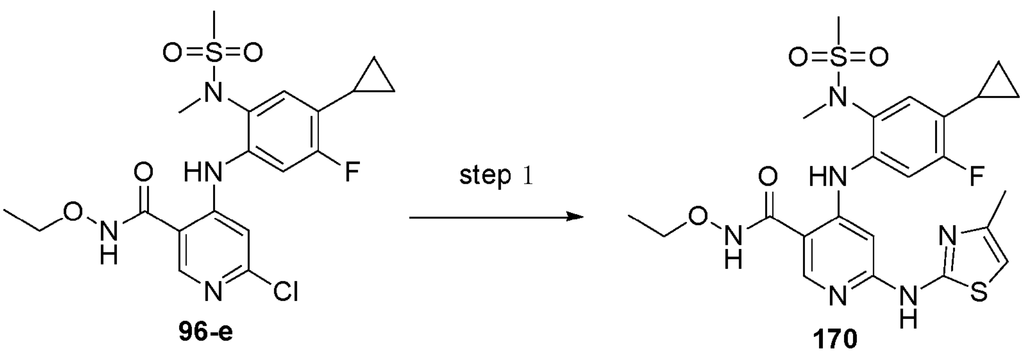


**96-e** **170**

Step 1: 6-chloro-4-((4-cyclopropyl-5-fluoro-2-(N-methyl methanesulfonamido)phenyl)amino)-N-ethoxy nicotinamide **(96-e,** 278mg, 0.5mmol), 4-methyl-2-aminothiazole (57mg, 0.5mmol), cesium carbonate (326mg, 1.0mmol),

XantPhos(57.6mg, 0.1mmol) and $Pd_2(dba)_3$(45.5mg, 0.05mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and subjected to high performance preparative thin layer chromatography to provide the title compound: 4-((4-cyclopropyl-5-fluoro-2-(N-methyl methanesulfonamido)phenyl)amino)-N-ethoxy-6-((4-methyl thiazol-2-yl)amino)nicotinamide **(170,** 80mg, 0.150mmol, 30% yield). MS Calcd: 534; MS Found: 535 ($[M+H]^+$). $^1H$ NMR (400 MHz, DMSO-$d_6$)δ11.64 (s, 1H), 11.06 (s, 1H), 10.02 (s, 1H), 8.36 (s, 1H), 7.33 (d, *J* = 12.4 Hz, 1H), 7.17 (d, *J* = 8.0 Hz, 1H), 6.92 (s, 1H), 6.56 (s, 1H), 3.93 (q, *J* = 7.2 Hz, 2H), 3.13 (s, 3H), 3.09 (s, 3H), 2.21 (s, 3H), 2.06 - 1.97 (m, 1H), 1.22 (t, *J* = 7.2 Hz, 3H), 1.03 - 0.94 (m, 2H), 0.83 - 0.77 (m, 2H).

Example **171**

4-((4-cyclopropyl-5-fluoro-2-(N-methyl methanesulfonamido)phenyl)amino)-N-ethoxy-6-((1-methyl-1H-pyrazol-3-yl) amino)nicotinamide

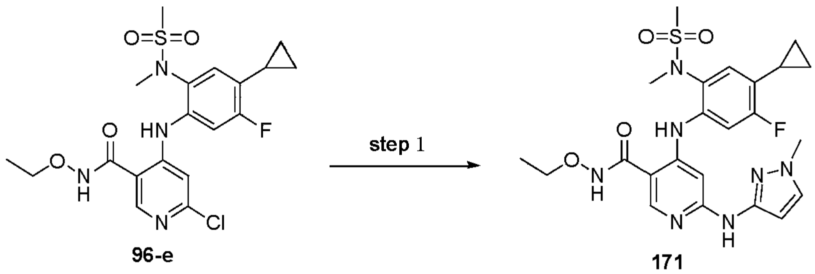


96-e 171

Step 1: 6-chloro-4-((4-cyclopropyl-5-fluoro-2-(N-methyl methanesulfonamido)phenyl)amino)-N-ethoxy nicotinamide **(96-e,** 278mg, 0.5mmol), 3-amino-methyl pyrazole (49mg, 0.5mmol), cesium carbonate (326mg, 1.0mmol), XantPhos(57.6mg, 0.1mmol) and $Pd_2(dba)_3$(45.5mg, 0.05mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and subjected to high performance preparative thin layer chromatography to provide the title compound: 4-((4-cyclopropyl-5-fluoro-2-(N-methyl methanesulfonamido)phenyl)amino)-N-ethoxy-6-((1-methyl-1H-pyrazol-3-yl)amino)nicotinamide **(171,** 40mg, 0.077mmol, 15.4% yield). MS Calcd: 517; MS Found: 518 ($[M+H]^+$). $^1H$ NMR (400 MHz, DMSO-$d_6$)δ11.50 (s, 1H), 10.19 (s, 1H), 9.41 (s, 1H), 7.52 (d, *J* = 2.4 Hz, 2H), 8.26 (s, 1H), 7.41 (d, *J* = 12.4 Hz, 1H), 7.14 (d, *J= 8.0* Hz, 1H), 6.06 (s, 1H), 3.92 (q, *J* = 7.2 Hz, 2H), 3.73 (s, 3H), 3.13 (s, 3H), 3.10 (s, 3H), 2.04-1.98 (m, 1H), 1.21 (t, *J* = 7.2 Hz, 3H), 0.98-0.95 (m, 2H), 0.78-0.74 (m, 2H).

Example **172**

N-ethoxy-4-((4-methoxy-2-(N-methyl methanesulfonamido)phenyl)amino)-6-(5-methyl thiazol-2-ylamino)nicotinamide

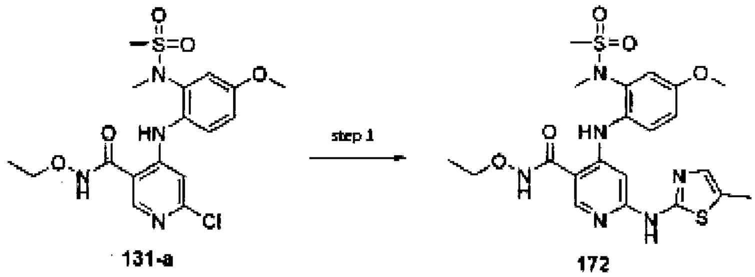


131-a 172

Step 1: To a 50ml flask were added 6-chloro-N-ethoxy-4-((4-methoxy-2-(N-methyl methanesulfonamido)phenyl) amino)nicotinamide **(131-a,** 150mg, 0.35mmol), 5-methyl thiazol-2-ylamine (80mg, 0.35mmol), Xant-phos(81mg, 0.14mmol), $Cs_2CO_3$ (343mg, 1.05mmol), $Pd(dba)_2$ (94mg, 0.1mmol), and 1,4-dioxane (3ml). The reaction was vacuumed and refilled with $N_2$ four times and allowed to proceed at 120°C for 4h. Upon indication of completed reaction by TLC (DCM/MeOH=15/1), the system was washed with water and extracted with ethyl acetate three times. The combined

organic layers were dried, concentrated in the presence of silica gel for loading onto and separating by column chromatography, eluted with DCM/MeOH=50/1~30/1 (with 0.1ml of acetic acid per 100ml of solvent mixture). The organic phase was concentrated, slurried with DCM, and filtered to provide the title compound: N-ethoxy-4-((4-methoxy-2-(N-methyl methanesulfonamido)phenyl)amino)-6-(5-methyl thiazol-2-ylamino)nicotinamide (172, 30mg, 0.06mmol, 16% yield). MS Calcd: 506; MS Found: 507 ([M+H]+). $^1$H NMR (400 MHz, DMSO-$d_6$)δ11.61 (s, 1H), 10.74 (s, 1H), 9.56 (s, 1H), 8.33 (s, 1H), 7.38 (d, *J* = 8.8 Hz, 1H), 7.15 (d, *J* = 2.8 Hz, 1H), 7.03 (dd, *J* = 8.8, 2.8 Hz, 1H), 6.96 (d, *J* = 1.6 Hz, 1H), 6.62 (s, 1H), 6.41 (s, 1H), 3.94 (q, *J* = 7.2 Hz, 2H), 3.81 (s, 3H), 3.13 (s, 3H), 3.07 (s, 3H), 1.21 (t, *J* = 7.2 Hz, 3H).

Example **173**

6-((2,6-dimethyl pyrimidin-4-yl)amino)-N-ethoxy-4-((2-(N-methyl cyclopropyl sulfonamido)phenyl)amino)nicotinamide

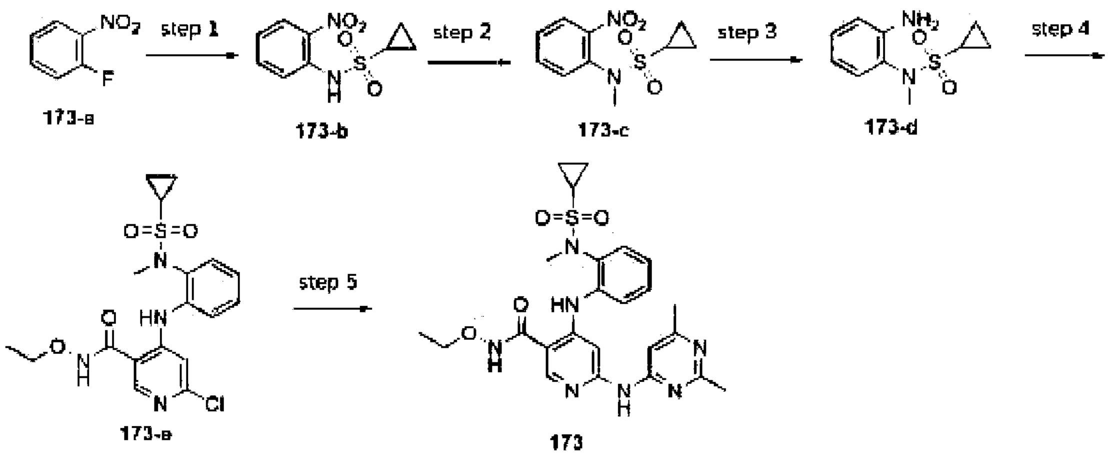

173-a 173-b 173-c 173-d 173-e 173

Step 1: To a 100ml flask were added 1-fluoro-2-nitrobenzene(**173-a**,.2g, 8.5mmol), cyclopropylsulfonamide (1.13g, 8.6mmol), DMF 20ml and $K_2CO_3$(3.5g, 25.5mmol). The reaction was vacuumed and refilled with $N_2$ three times and allowed to proceed at 90°C for 3h. Upon indication of completed reaction by TLC (petroleum ether/ethyl acetate=2/1), the system was washed with water and extracted with ethyl acetate three times. The combined organic layers were dried and concentrated to obtain N-(2-nitrophenyl) cyclopropylsulfonamide 1.0g(**173-b**, 1.0g, 3.5mmol, 41% yield). MS Calcd: 242; MS Found: 243([M+H]$^+$).

Step 2: To a 50ml flask were added N-(2-nitrophenyl) cyclopropylsulfonamide **(173-b,** 1g, 3.5mmol), iodomethane (600mg, 3.6mmol), and DMF (20ml). The mixture was added under nitrogen protection with NaH (126mg, 5.2mmol) portionwise, and allowed to react at room temperature for 5h. Upon indication of completed reaction by TLC (petroleum ether/ethyl acetate=1/1), the system was washed with water and adjusted with 1mol/L HCl to pH≈6, and extracted with ethyl acetate three times. The combined organic layers were dried and concentrated to provide N-methyl-N-(2-nitrophenyl) cyclopropylsulfonamide (**173-c**, 1.0g, 3.5mmol, 99% yield). MS Calcd: 256; MS Found: 257 ([M+H]+).

Step 3: To a 50ml flask were added N-methyl-N-(2-nitrophenyl) cyclopropylsulfonamide **(173-c,** 1g, 3.5mmol), Pd/C(10%, 0.1g, 0.35mmol), and methanol (20ml). The mixture was bubbled with $H_2$ to react for 5h. Upon indication of completed reaction by TLC (petroleum ether/ethyl acetate=1/1), the system was filtered, and the filter cake was washed with methanol three times. The combined organic layers were dried, concentrated, and subjected to column chromatography, eluted with petroleum ether/ethyl acetate=5/1~2/1 to provide N-(2-amino phenyl)-N-methyl cyclopropylsulfonamide **(173-d,** 1.0g, 3.8mmol, 98% yield).

MS Calcd: 230; MS Found: 231 ([M+H]+).

Step 4: To a 100ml flask were added N-(2-amino phenyl)-N-methyl cyclopropylsulfonamide **(173-d,** 1.0g, 3.8mmol), 4,6-dichloro-N-ethoxy nicotinamide **(int-2,** 600mg, 3.7mmol), and anhydrous THF (15ml). After atmosphere replacement with nitrogen three times, the reaction mixture was slowly added with LiHMDS (9.0ml, 9.0mmol) under nitrogen protection and allowed to proceed for 5h. Upon indication of completed reaction by TLC (petroleum ether/ethyl acetate=1/2), the system was washed with water and adjusted with 0.1mol/L HCl to pH=5, and extracted with ethyl acetate three times. The combined organic layers were dried, concentrated, mixed with silica gel for loading and purified by column chromatography eluted with petroleum ether/ethyl acetate=2/1~1/1 to provide 6-chloro-N-ethoxy-4-((2-(N-methyl cyclopropylsulfonamido)phenyl)amino)nicotinamide **(173-e,** 1.0g, 2.3mmol, 62% yield). MS Calcd:424; MS Found: 425 ([M+H]$^+$).

Step 5: To a 50ml flask were added 6-chloro-N-ethoxy-4-((2-(N-methyl cyclopropylsulfonamido)phenyl)amino) nicotinamide **(173-e,** 150mg, 0.35mmol), 2,6-dimethyl pyrimidin-4-ylamine (87mg, 0.7mmol), Xant-phos(81mg, 0.14mmol), $Cs_2CO_3$ (343mg, 1.05mmol), $Pd(dba)_2$ (94mg, 0.1mmol), and 1,4-dioxane (3ml). The reaction was vacuumed and refilled with $N_2$ 4 times and allowed to proceed at 120°C for 4h. Upon indication of completed reaction by TLC (DCM/MeOH=15/1), the system was washed with water and extracted with ethyl acetate three times. The combined organic layers were dried, concentrated in the presence of silica gel for loading onto and separating by column chromatography, eluted with DCM/MeOH=50/1~30/1 (with 0.1ml of acetic acid per 100ml of solvent mixture). The organic phase was concentrated, slurried with DCM, and filtered to provide the title compound: 6-((2,6-dimethyl pyrimidin-4-yl) amino)-N-ethoxy-4-((2-(N-methyl cyclopropyl sulfonamido)phenyl)amino)nicotinamide **(173,** 60mg, 0.12mmol, 34% yield). MS Calcd: 511; MS Found: 512($[M+H]^+$). $^1H$ NMR (400 MHz, DMSO-$d_6$)δ11.63 (s, 1H), 10.10 (s, 1H), 10.05 (s, 1H), 8.35 (s, 1H), 8.01 (s, 1H), 7.64-7.61 (m, 2H), 7.47-7.42 (m, 1H), 7.25-7.20 (m, 1H), 7.06 (s, 1H), 3.94 (q, $J$ = 7.2 Hz, 2H), 3.18 (s, 3H), 2.85-2.79 (m, 1H), 2.34 (s, 3H), 2.27 (s, 3H), 1.22 (t, $J$ = 7.2 Hz, 3H), 1.04-0.99 (m, 2H), 0.89 - 0.84 (m, 2H).

Example **174**

6-((2,6-dimethyl pyrimidin-4-yl)amino)-N-ethoxy-4-((4-methoxy-2-(N-methyl cyclopropylsulfonamido)phenyl)amino)nicotinamide

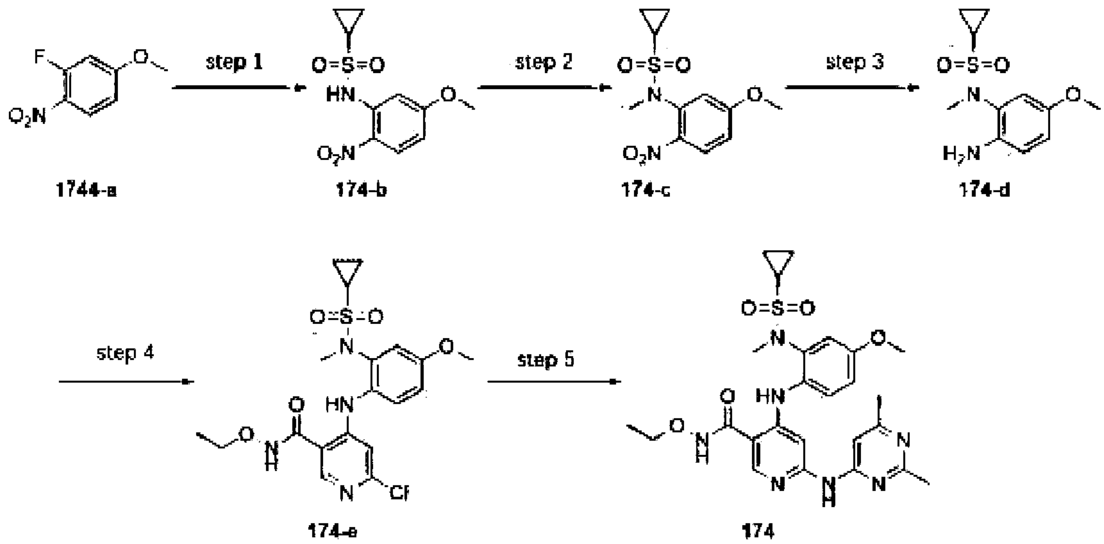

1744-a 174-b 174-c 174-d
174-e 174

Step 1: To a 100ml reaction flask was added 2-fluoro-4-methoxy-1-nitrobenzene(**174-a**, 513g, 3mmol) dissolved in N,N-dimethyl formamide (30ml), followed by potassium carbonate (1.24g, 9mmol) and cyclopropylsulfonamide (400mg, 3.3mmol). The mixture was heated to 90°C with stirring for 3h. Upon indication of completed reaction by TLC, the reaction mixture was washed respectively with water (30ml x 1) and brine (30 ml x 1), dried over anhydrous sodium sulfate, and concentrated to provide a crude material, which was purified by silica gel column chromatography (petroleum ether/ethyl acetate=3:1) to provide the product N-(5-methoxy-2-nitrophenyl) cyclopropylsulfonamide **(174-b,** 658mg, 80.6% yield). MS Calcd:272.05; MS Found: 273($[M+H]^+$)

Step 2: To a 100ml reaction flask was added N-(5-methoxy-2-nitrophenyl) cyclopropylsulfonamide **(174-b,** 658mg, 2.42mmol) dissolved in N,N-dimethyl formamide (30ml), followed by sodium hydride (145.2mg, 60%, 3.63mmol) and iodomethane (515.4mg, 3.63mmol). The reaction mixture was stirred at room temperature for 3h. Upon indication of completed reaction by TLC, the reaction mixture was added with water and ethyl acetate, extracted with ethyl acetate (30 ml x 2). The organic phase was washed with brine (30 ml x 1), dried over anhydrous sodium sulfate, and concentrated to obtain N-(5-methoxy-2-nitrophenyl)-N-methyl cyclopropylsulfonamide **(174-c,** 695mg, 2.42mmol, 100% yield). MS Calcd: 286.09; MS Found: 287.22 ($[M+H]^+$).

Step 3: N-(5-methoxy-2-nitrophenyl)-N-methyl cyclopropylsulfonamide **(174-c,** 695mg, 2.42mmol), ammonium chloride (1.3g, 24.2 mmol) and iron powder (0.67 g, 12.1 mmol) were sequentially added to 10 mL of mixed solvent of water and ethanol (1:4), and stirred under reflux for 6 hours. Upon indication of completed reaction by TLC, the reaction mixture was filtered by suction. The filtrate was concentrated, and the residue was separated and purified by silica gel column chromatography to provide N-(2-amino-5-methoxy phenyl)-N-methyl cyclopropylsulfonamide **(174-d,** 0.59 g, 2.3 mmol, 96.2% yield). MS Calcd: 256.09; MS Found: 257.22 ($[M+H]^+$).

Step 4: Into 10 ml of anhydrous N,N-dimethylacetamide were added N-(2-amino-5-methoxy phenyl)-N-methyl cyclopropylsulfonamide **(174-d,** 256 mg, 1 mmol) and 4,6-dichloro-N-ethoxy nicotinamide **(int-2,** 234 mg, 1 mmol). The mixture was added at room temperature with a solution of LiHMDS in tetrahydrofuran (3 ml, 3 mmol), and stirred at room temperature for 3 hours. Upon indication of completed reaction by TLC, the mixture was adjusted with aqueous hydrochloride (1N) to pH 5, and extracted with ethyl acetate (20 ml x 3). The combined organic layers were dried, concentrated and then purified by column chromatography (PE:EA =1:1) to provide 6-chloro-N-ethoxy-4-((4-methoxy-2-(N-methyl cyclopropylsulfonamido)phenyl)amino)nicotinamide **(174-e,** 350mg, 0.77 mmol, 77.09% yield). MS Calcd: 454.10; MS Found: 455.29 ($[M+H]^+$).

Step 5: 6-chloro-N-ethoxy-4-((4-methoxy-2-(N-methyl cyclopropylsulfonamido)phenyl)amino)nicotinamide **(174-e,** 91mg, 0.2 mmol), 2,6-dimethyl pyrimidin-4-ylamine (27.06 mg, 0.22mmol), cesium carbonate (195 mg, 0.6 mmol), XantPhos(23.12mg, 0.04mmol) and $Pd_2(dba)_3$(27.06mg, 0.02 mmol) were added to anhydrous dioxane (5 ml). After the atmosphere of the mixture was evacuated to vacuum and refilled with nitrogen, the reaction mixture was heated to 120°C with stirring for 6 hours, and filtered by suction. The filtrate was concentrated and purified by high performance preparative thin layer chromatography (MeOH:DCM=1:20) to provide the title compound: 6-((2,6-dimethyl pyrimidin-4-yl) amino)-N-ethoxy-4-((4-methoxy-2-(N-methyl cyclopropylsulfonamido)phenyl)amino)nicotinamide **(174,** 40mg, 0.073mmol, 33.6% yield). MS Calcd: 541.21; MS Found: 542.20 ($[M+H]^+$). $^1H$ NMR (400 MHz, DMSO-$d_6$)δ11.60 (s, 1H), 10.01 (s, 1H), 9.93 (s, 1H), 8.33 (s, 1H), 7.86 (s, 1H), 7.56 - 7.40 (m, 2H), 7.26 (d, *J* = 8.0 Hz, 1H), 7.05 (s, 1H), 3.93 (q, *J* = 7.2 Hz, 2H), 3.15 (s, 3H), 2.84-2.78 (m, 1H), 2.34 (s, 3H), 2.32 (s, 3H), 2.26 (s, 3H), 1.21 (t, *J* = 7.2 Hz, 3H), 1.01-0.99 (m, 2H), 0.87 - 0.85 (m, 2H).

Example **175**

6-((2,6-dimethyl pyrimidin-4-yl)amino)-N-ethoxy-4-((4-methyl-2-(N-methyl cyclopropylsulfonamido)phenyl)amino)nicotinamide

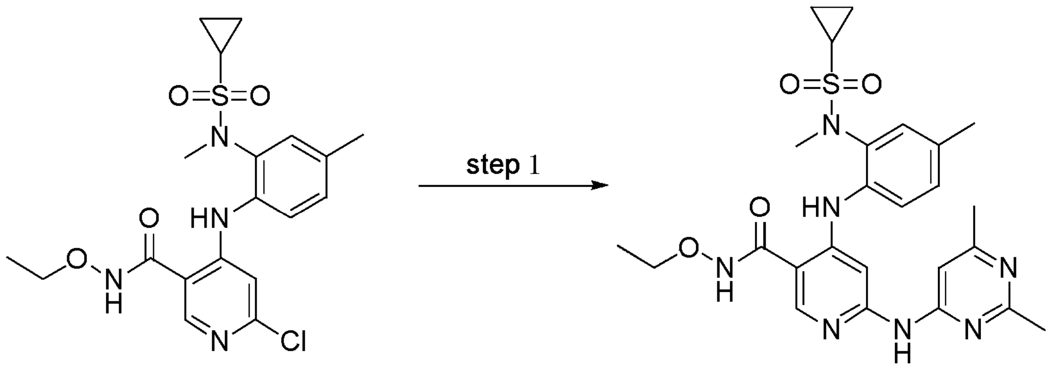


**66-e** **175**

Step 1: 6-chloro-N-ethoxy-4-((4-methyl-2-(N-methyl cyclopropylsulfonamido)phenyl)amino)nicotinamide **(66-e,** 187mg, 0.43 mmol, ), 2,6-dimethyl pyrimidin-4-ylamine (49.8 mg, 0.43mmol), cesium carbonate (415 mg, 1.27 mmol), XantPhos(46.24mg, 0.08mmol) and $Pd_2(dba)_3$(36.6mg, 0.04 mmol) were added to anhydrous dioxane (5 ml). After the atmosphere of the mixture was evacuated to vacuum and refilled with nitrogen, the reaction mixture was heated to 120°C with stirring for 6 hours, and filtered by suction. The filtrate was concentrated and purified by high performance preparative thin layer chromatography (MeOH:DCM=1:20) to provide the title compound: 6-((2,6-dimethyl pyrimidin-4-yl)amino)-N-ethoxy-4-((4-methyl-2-(N-methyl cyclopropylsulfonamido)phenyl)amino)nicotinamide **(175,** 40mg, 0.076mmol, 17.9% yield). MS Calcd: 525.21; MS Found: 526.20 ($[M+H]^+$). $^1H$ NMR (400 MHz, DMSO-$d_6$)δ11.56 (s, 1H), 9.95 (s, 1H), 9.69 (s, 1H), 8.31 (s, 1H), 7.67 (s, 1H), 7.44 (d, *J* = 8.8 Hz, 1H), 7.20 (d, *J* = 2.8 Hz, 1H), 7.06 (dd, *J* = 8.8, 2.8Hz, 1H), 7.01 (s, 1H), 3.93 (q, *J* = 7.2 Hz, 2H), 3.81 (s, 3H), 3.15 (s, 3H), 2.83 - 2.75 (m, 1H), 2.26 (s, 3H), 2.25 (s, 3H), 1.22 (d, *J* = 7.2 Hz, 3H), 1.03-0.98 (m, 2H), 0.87-0.83 (m, 2H).

Example **176**

4-((4-cyclopropyl-5-fluoro-2-(N-methyl methanesulfonamido)phenyl)amino)-N-ethoxy-6-((6-methyl pyridazin-3-yl)amino)nicotinamide

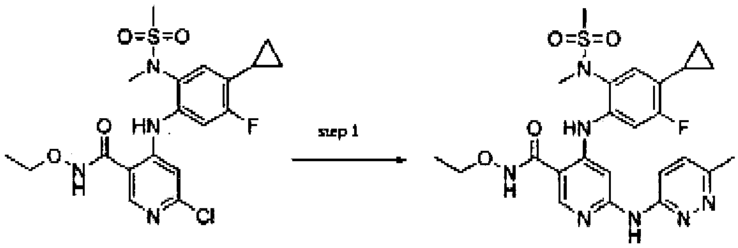


96-e

176

Step 1: 6-chloro-4-((4-cyclopropyl-5-fluoro-2-(N-methyl methanesulfonamido)phenyl)amino)-N-ethoxy nicotinamide **(96-e,** 278mg, 0.5mmol), 3-amino-6-methyl pyridazine (55mg, 0.5mmol), cesium carbonate (326mg, 1.0mmol), XantPhos(57.6mg, 0.1mmol) and $Pd_2(dba)_3$(45.5mg, 0.05mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and subjected to high performance preparative thin layer chromatography to provide the title compound: 4-((4-cyclopropyl-5-fluoro-2-(N-methyl methanesulfonamido)phenyl)amino)-N-ethoxy-6-((6-methyl pyridazin-3-yl)amino)nicotinamide **(176,** 15mg, 0.028mmol, 5.6% yield). MS Calcd: 529; MS Found: 530 ($[M+H]^+$). $^1H$ NMR (401 MHz, DMSO-$d_6$)$\delta$11.62 (s, 1H), 10.09 (s, 1H), 10.07 (s, 1H), 8.32 (s, 1H), 8.03 (d, $J$ = 9.2 Hz, 1H), 7.52 (s, 1H), 7.43 (d, $J$ = 9.2 Hz, 1H), 7.35 (d, $J$ = 11.6 Hz, 1H), 7.15 (d, $J$ = 8.0 Hz, 1H), 3.93 (q, $J$ = 7.2 Hz, 2H), 3.14 (s, 3H), 3.10 (s, 3H), 2.50(s, 3H), 2.04-2.01 (m, 1H), 1.21 (t, $J$ = 7.2 Hz, 3H), 1.01-0.97 (m, 2H), 0.80-0.77 (m, 2H).

Example **177**

4-((4-cyano-2-(N-methyl methanesulfonamido)phenyl)amino)-6-(((2,6-dimethyl pyrimidin-4-yl)amino)-N-ethoxy nicotinamide

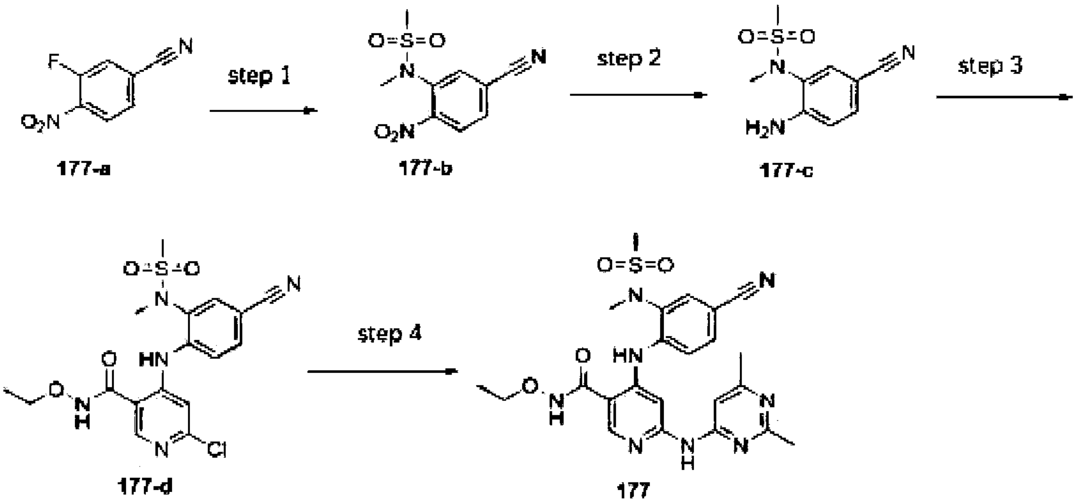


177-a

177-b

177-c

177-d

177

Step 1: Into 5ml of anhydrous acetonitrile were added 3-fluoro-4-nitrobenzonitrile(**177-a,** 166mg, 1.00mmol), N-methyl methanesulfonamide (109mg, 1.00mmol) and potassium carbonate (272mg, 2.00mmol). The mixture was stirred under reflux at 90°C for 2 hours. The reaction was subjected to rotary evaporation under reduced pressure to dryness. The residue was added with 10ml of water, and extracted with ethyl acetate (10ml x 3). The combined organic layers were dried and concentrated to provide N-(5-cyano-2-nitrophenyl)-N-methyl methanesulfonamide **(177-b,** 230 mg, 0.902mmol, 90.2% yield). MS Calcd: 255; MS Found: 256 ($[M+H]^+$).

Step 2: Into 5ml of methanol was added N-(5-cyano-2-nitrophenyl)-N-methyl methanesulfonamido **177-b,** 230

mg, 0.902mmol), followed by 30mg Pd/C. The mixture was allowed to react at room temperature for 2h, filtered to remove Pd/C, and subjected to rotary evaporation to remove methanol to provide N-(5-cyano-2-amino phenyl)-N-methyl methanesulfonamide **(177-c,** 200 mg, 0.89mmol, 98.5% yield). MS Calcd: 225; MS Found: 226 ($[M+H]^+$).

Step 3: 4,6-dichloro-N-ethoxy nicotinamide **(int-2,** 235mg, 1mmol), N-(5-cyano-2-amino phenyl)-N-methyl methanesulfonamide **(177-c,** 200 mg, 0.89mmol) were added to 5ml of anhydrous tetrahydrofuran. The mixture was added at room temperature with a solution of LiHMDS in tetrahydrofuran (3ml, 3.00mmol), and stirred at room temperature for 2 hours. The mixture was adjusted with aqueous hydrochloride (1N) to pH 5, and extracted with ethyl acetate (10ml x 3). The combined organic layers were dried, concentrated and then purified by column chromatography (petroleum ether:ethyl acetate =1:1) to provide 6-chloro-4-((4-cyano-2-((N-methyl methanesulfonamido)phenyl)amino)-N-ethoxy nicotinamide **(177-d,** 100mg, 0.24mmol, 26.50% yield). MS Calcd: 424; MS Found: 425 ($[M+H]^+$).

Step 4: 6-chloro-4-((4-cyano-2-((N-methyl methanesulfonamido)phenyl)amino)-N-ethoxy nicotinamide **(177-d,** 100mg, 0.24mmol), 2,6-dimethyl-4-aminopyrimidine (62mg, 0.5mmol), cesium carbonate (326mg, 1.0mmol), Xant-Phos(57.6mg, 0.1mmol) and $Pd_2(dba)_3$(45.5mg, 0.05mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and subjected to high performance preparative thin layer chromatography to provide the title compound: 4-((4-cyano-2-(N-methyl methanesulfonamido)phenyl)amino)-6-(((2,6-dimethyl pyrimidin-4-yl)amino)-N-ethoxy nicotinamide **(177,** 10mg, 0.019mmol, 8.17% yield). MS Calcd: 510; MS Found: 511 ($[M+H]^+$). $^1$H NMR (401 MHz, DMSO-$d_6$)δ11.75 (s, 1H), 10.60 (s, 1H), 10.23 (s, 1H), 8.42 (s, 1H), 8.22 (s, 1H), 8.10 (s, 1H), 7.89-7.82 (m, 3H), 3.95 (q, *J* = 7.2 Hz, 2H), 3.22 (s, 3H), 3.21 (s, 3H), 2.30 (s, 3H), 2.26 (s, 3H), 1.23 (t, *J* = 7.2 Hz, 3H).

Example **178**

6-((2,6-dimethyl pyrimidin-4-yl)amino)-N-ethoxy-4-((4-isopropoxy-2-(N-methyl methanesulfonamido)phenyl)amino)nicotinamide

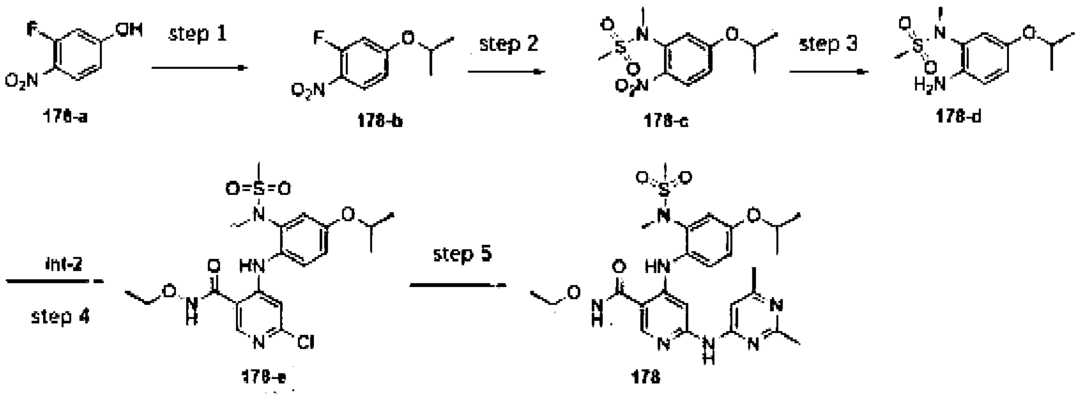


178-a 178-b 178-c 178-d 178-e 178

Step 1: To a 100ml two-necked flask were added 3-fluoro-4-nitrophenol(**178-a**, 500mg, 3.184mmol), isopropanol (287.03mg, 4.776mmol), triphenylphosphine (1.253g, 4.776mmol) and 15ml of THF. After atmosphere replacement with nitrogen three times, the mixture was cooled in ice bath and added with DIAD(965.75mg, 4.776mmol) dropwise. After the addition, the mixture was immediately removed from ice bath, and stirred at room temperature for 16 hours. When TLC indicated a completed reaction, the reaction mixture was mixed with 200-300 mesh silica gel for loading, concentrated under reduced pressure at 40°C, separated and purified by column chromatography, eluted with from PE:EA=100:1 gradually changing to PE:EA=20:1, to provide the target Intermediate 2-fluoro-4-isopropoxy-1-nitrobenzene(**178-b**, 349mg, 1.754mmol, 55.14% yield).

Step 2: To a 100mL flask were added 2-fluoro-4-isopropoxy-1-nitrobenzene(**178-b,** 349mg, 1.754mmol), N-methyl methanesulfonamide (248mg, 2.280mmol), potassium carbonate (484mg, 3.508, mmol), and anhydrous acetonitrile (12ml). The mixture was allowed to react at 90°C for 6 hours. When TLC indicated a completed reaction, the reaction mixture was mixed with silica gel for loading onto and purifying by column chromatography, eluted with PE:EA=8:1 gradually changing to PE:EA=5:1, to provide the target Intermediate N-(5-isopropoxy-2-nitrophenyl)-N-methyl methanesulfonamide (**178-c**, 377mg, 1.310mmol, 74.65% yield). MS Calcd:288

Step 3: To a 50ml flask was added N-(5-isopropoxy-2-nitrophenyl)-N-methyl methanesulfonamide **(178-c,** 377mg, 1.310mmol), reduction iron powder (366.53mg, 6.545mmol), and ammonium chloride (700.33mg, 13.102mmol).

The mixture was added with 10mL of ethanol and 2ml of water, and allowed to react at 80°C for 6 hours. When TLC indicated a completed reaction, the mixture was filtered by suction, with filtrate extracted with EA. The organic phase was concentrated under reduced pressure, to provide the target Intermediate N-(2-amino-5-isopropoxy phenyl)-N-methyl methanesulfonamide **(178-d,** 293mg, 1.136mmol, 86.95% yield). MS Calcd:258; MS Found: 259($[M+H]^+$)

Step 4: To a 50mL three-necked flask were added 4,6-dichloro-N-ethoxy nicotinamide **(int-2,** 136.04g, 0.581mmol) and N-(2-amino-5-isopropoxy phenyl)-N-methyl methanesulfonamide **(178-d,** 150mg, 0.581mmol), followed by 4ml of anhydrous DMA. The reaction was vacuumed and refilled with $N_2$ three times, and cooled in ice bath, added with LHMDS(1.7mL, 1mol/L, 1.744mmol) dropwise. After that the reaction was allowed to proceed at room temperature for 6 hours, and sampled. When TLC indicated a completed reaction, the reaction was quenched by adding saturated aqueous solution of ammonium chloride in ice bath, and extracted with ethyl acetate. The organic phase was mixed with 200-300 mesh silica gel for loading onto and purifying by column chromatography, eluted with PE:EA=5:1 gradually changing to PE:EA=2:1, to provide the target Intermediate 6-chloro-N-ethoxy-4-((4-isopropoxy-2-(N-methyl methanesulfonamido) phenyl)amino)nicotinamide **(178-e,** 161mg, 0.353mmol, 60.77% yield). MS Calcd: 456; MS Found: 457($[M+H]^+$)

Step 5: 6-chloro-N-ethoxy-4-((4-isopropoxy-2-(N-methyl methanesulfonamido)phenyl)amino)nicotinamide **(178-e,** 80mg, 0.175mmol), 4-amino-2,6-dimethyl pyrimidine (25.92mg, 0.210mmol), $pd_2(dba)_3$ (48.19mg, 0.053mmol), XantPhos(60.90mg, 0.105mmol), $Cs_2CO_3$ (200.05mg, 0.614mmol), and 1,4-dioxane (4mL) were combined. After atmosphere replacement with nitrogen three times, the mixture was allowed to react at 120°C for 6 hours. When TLC indicated a completed reaction, the mixture was filtered by suction, and the filtrate was concentrated under reduced pressure at 40°C, mixed with 200-300 mesh silica gel for loading onto and purifying by column chromatography, eluted with DCM:MeOH=30:1, to provide the title compound: 6-((2,6-dimethyl pyrimidin-4-yl)amino)-N-ethoxy-4-((4-isopropoxy-2-(N-methyl methanesulfonamido)phenyl)amino)nicotinamide **(178,** 25mg, 0.046mmol, 26.31% yield). MS Calcd:543; MS Found: 544($[M+H]^+$). $^1H$ NMR (401 MHz, DMSO-d6)δ11.55 (s, 1H), 9.94 (s, 1H), 9.63 (s, 1H), 8.30 (s, 1H), 7.63 (s, 1H), 7.40 (d, *J* = 8.8 Hz, 1H), 7.14 (s, 1H), 7.02 (s, 2H), 4.67 - 4.64 (m, 1H), 3.93 (q, *J* = 7.2 Hz, 2H), 3.11 (s, 3H), 3.06 (s, 3H), 2.26 (s, 3H), 2.25 (s, 3H), 1.31 (s, 3H), 1.29 (s, 3H), 1.22 (t, *J* = 7.2 Hz, 3H).

Example **179**

4-((4-cyclopropyl-2-(N-methyl methanesulfonamido)phenyl)amino)-N-ethoxy-6-((2-methoxy pyrimidin-4-yl)amino)nicotinamide

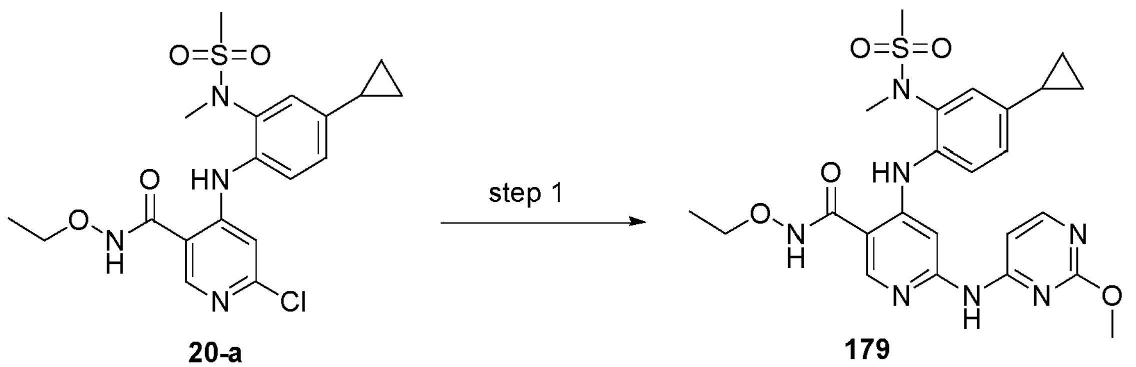


20-a

179

Step 1: 6-chloro-4-((4-cyclopropyl-2-(N-methyl methanesulfonamido)phenyl)amino)-N-ethoxy nicotinamide **(20-a,** 118mg, 0.268mmol), 2-methoxy pyrimidin-4-ylamine (43.81mg, 0.350mmol), $pd_2(dba)_3$ (73.95mg, 0.081mmol), XantPhos(118.73mg, 0.162mmol), $Cs_2CO_3$ (307.21mg, 0.943mmol), and 1,4-dioxane (4mL) were combined. After atmosphere replacement with nitrogen three times, the mixture was allowed to react at 120°C for 6 hours. When TLC indicated a completed reaction, the mixture was filtered by suction, and the filtrate was concentrated under reduced pressure at 40°C, mixed with 200-300 mesh silica gel for loading onto and purifying by column chromatography, eluted with DCM:MeOH=30:1, to provide the title compound 4-((4-cyclopropyl-2-(N-methyl methanesulfonamido)phenyl)amino)-N-ethoxy-6-((2-methoxy pyrimidin-4-yl)amino)nicotinamide **(179,** 27mg, 0.051mmol, 19.02% yield). MS Calcd:527; MS Found: 528($[M+H]^+$). $^1H$ NMR (400 MHz, DMSO-$d_6$)δ11.63 (s, 1H), 10.18 (s, 1H), 9.80 (s, 1H), 8.34 (s, 1H), 8.16 (d, *J* = 5.6 Hz, 1H), 7.70 (s, 1H), 7.41 (d, *J* = 8.4 Hz, 1H), 7.29 (d, *J* = 2.0 Hz, 1H), 7.09 (dd, *J* = 8.4, 2.0 Hz, 1H), 6.99 (d, *J* = 5.6 Hz, 1H), 3.94 (q, *J* = 7.2 Hz, 2H), 3.50 (s, 3H), 3.13 (s, 3H), 3.08 (s, 3H), 2.02-1.97 (m, 1H), 1.22 (t, *J* = 7.2 Hz, 3H), 1.06 - 0.97 (m, 2H), 0.75-0.71 (m, 2H).

Example **180**

6-(((2,6-dimethyl pyrimidin-4-yl)amino)-N-ethoxy-4-((4-ethyl-2-(N-methyl methanesulfonamido)phenyl)amino)nicotinamide

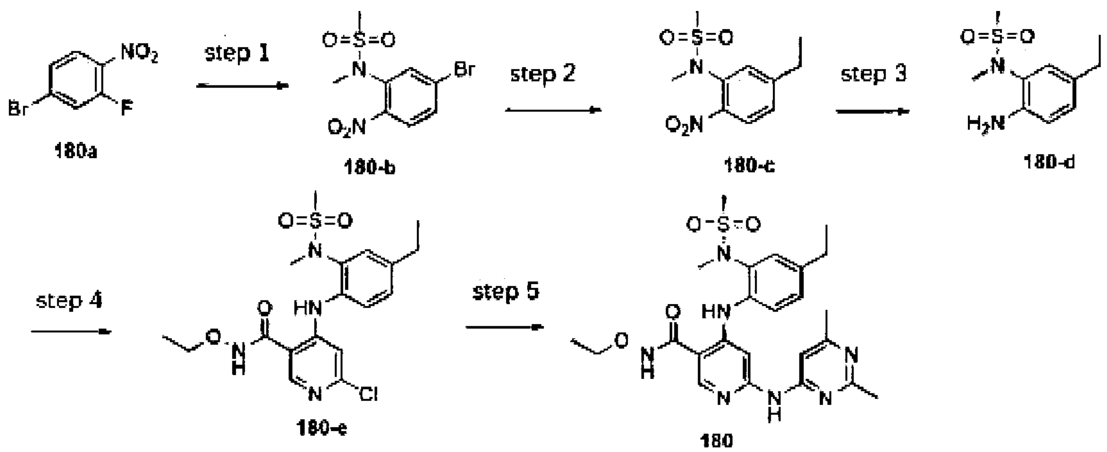


180a 180-b 180-c 180-d 180-e 180

Step 1: To a 50ml flask were added 4-bromo-2-fluoro-1-nitrobenzene(**180-a**, 1g, 4.5mmol), N-methyl methanesulfonamide (0.6g, 4.6mmol), DMF (20ml). $K_2CO_3$(1.8g, 14mmol). The reaction was vacuumed and refilled with $N_2$ three times and allowed to react at 90°C for 3h. Upon indication of completed reaction by TLC (petroleum ether/ethyl acetate=2/1), the system was washed with water and extracted with ethyl acetate three times. The combined organic layers were dried and concentrated to obtain N-(5-bromo-2-nitrophenyl)-N-methyl methanesulfonamido1.0g (**180-b,** 1.3g, 4.4mmol, 99% yield). MS Calcd: 308; MS Found: 309($[M+H]^+$).

Step 2: To a 50ml flask were added N-(5-bromo-2-nitrophenyl)-N-methyl methanesulfonamide **(180-b,** 500mg, 1.6mmol), ethyl boronic acid (133mg, 1.8mmol), cesium carbonate (1.6g, 4.9mmol), Pd(dppf)$Cl_2$(11.6mg, 0.16mmol), diox (10ml), $H_2O$ (2ml). The reaction was vacuumed and refilled with $N_2$ three times and allowed to react at 100°C for 5h. Upon indication of completed reaction by TLC (petroleum ether/ethyl acetate=2/1), the system was washed with water and extracted with EA three times. The combined organic layers were dried, concentrated, and subjected to column chromatography (petroleum ether/ethyl acetate=5/1~3/1) to provide N-(5-ethyl-2-nitrophenyl)-N-methyl methanesulfonamido **(180-c,** 400mg, 1.6mmol, 99% yield).

Step 3: To a 50ml flask were added N-(5-ethyl-2-nitrophenyl)-N-methyl methanesulfonamide **(180-c,** 400mg, 1.6mmol), Pd/C(10%, 0.4g, 0.16mmol), and methanol (10ml). After atmosphere replacement with hydrogen twice, the mixture was bubbled at RT with $H_2$ to react for 5h. Upon indication of completed reaction by TLC (PE/EA=1/1), the system was filtered, and the filter cake was washed with methanol three times. The combined organic layers were dried and concentrated to obtain N-(2-amino-5-ethyl phenyl)-N-methyl methanesulfonamide **(180-d,** 450mg, 1.9mmol, 99.9% yield). MS Calcd: 228; MS Found: 229($[M+H]^+$).

Step 4: To a 50ml flask were added N-(2-amino-5-ethyl phenyl)-N-methyl methanesulfonamide **(180-d,** 450mg, 1.9mmol), 4,6-dichloro-N-ethoxy nicotinamide **(int-2,** 478mg, 2.1mmol), and THF (15ml). After atmosphere replacement with nitrogen three times, the reaction mixture was slowly added with LiHMDS (4.9ml, 4.9mmol) under nitrogen protection and allowed to react at RT for 5h. Upon indication of completed reaction by TLC (petroleum ether/ethyl acetate=1/2), the system was washed with water and adjusted with 0.1N HCl to pH=5 and extracted with ethyl acetate three times. The combined organic layers were dried, concentrated in the presence of silica gel for loading onto and separating by column chromatography, eluted with petroleum ether/ethyl acetate=3/1~1/1, to provide 6-chloro-N-ethoxy-4-((4-ethyl-2-(N-methyl methanesulfonamido)phenyl)amino)nicotinamide **(180-e,** 400mg, 0.94mmol, 50% yield). MS Calcd:426; MS Found: 427 ([M+H]+).

Step 5: To a 50ml flask were added 6-chloro-N-ethoxy-4-((4-ethyl-2-(N-methyl methanesulfonamido)phenyl) amino)nicotinamide **(180-e,** 150mg, 0.35mmol), 2,6-dimethyl pyrimidin-4-ylamine (86.6mg, 0.7mmol), Xant-Phos(81.5mg, 0.14mmol), $Cs_2CO_3$ (344mg, 1.1mmol), Pd$(dba)_2$ (97mg, 0.11mmol), and diox (5ml). The reaction was vacuumed and refilled with $N_2$ 4 times and allowed to proceed at 120°C for 4h. Upon indication of completed reaction by TLC (DCM/MeOH=15/1), the system was directly mixed with silica gel for loading and purified by column chromatography, eluted with DCM/MeOH=50/1~30/1(with 0.1ml of acetic acid per 100ml of solvent mixture) to provide the title compound: 6-(((2,6-dimethyl pyrimidin-4-yl)amino)-N-ethoxy-4-((4-ethyl-2-(N-methyl methanesulfonamido)phenyl)amino)nicotinamide **(180,** 60mg, 0.12mmol, 34% yield). MS Calcd: 513; MS Found: 514($[M+H]^+$). $^1$H NMR (400 MHz, DMSO-$d_6$)δ11.60

(s, 1H), 10.00 (s, 1H), 9.90 (s, 1H), 8.33 (s, 1H), 7.88 (s, 1H), 7.50 (d, *J* = 8.4 Hz, 1H), 7.42 (d, *J* = 2.0 Hz, 1H), 7.35 - 7.22 (m, 1H), 7.03 (s, 1H), 3.94 (q, *J* = 7.2 Hz, 2H), 3.14 (s, 3H), 3.10 (s, 3H), 2.65 (q, *J* = 7.2 Hz, 2H), 2.31 (s, 3H), 2.26 (s, 3H), 1.22 (t, *J* = 7.2 Hz, 6H).

Example **181**

6-(((2,6-dimethyl pyrimidin-4-yl)amino)-N-ethoxy-4-((4-ethoxy-2-(N-methyl methanesulfonamido)phenyl)amino)nicotinamide

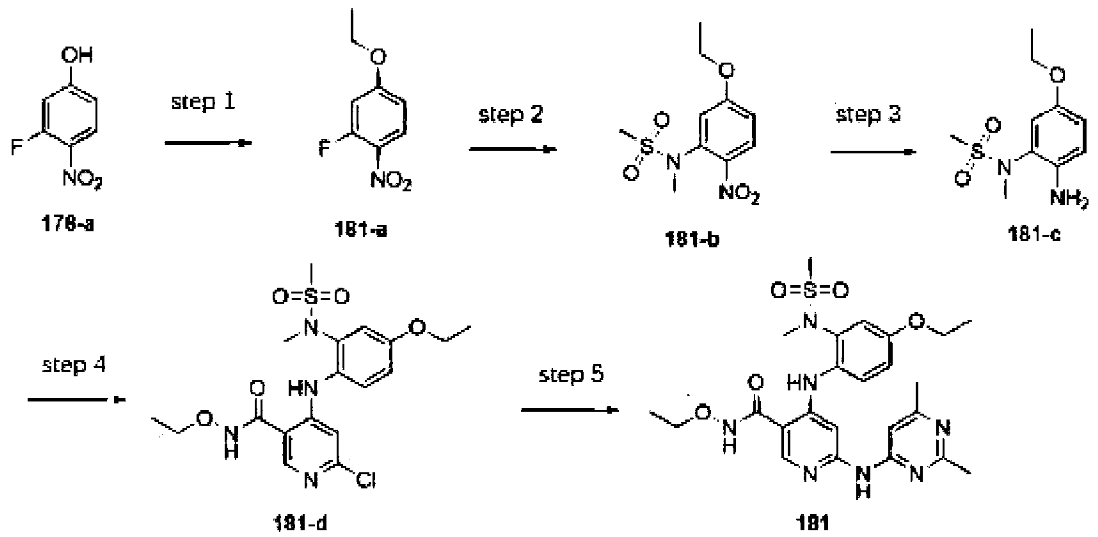


Step 1: To a 100ml flask were sequentially added 2-fluoro-4-hydroxyl-1-nitrobenzene(**178-a**, 1g, 6.3mmol), iodoethane(1.5g, 9.5mmol), anhydrous potassium carbonate (2.6g, 19mmol) and acetonitrile (30mL). The mixture was stirred at 60°C for 6h. Upon indication of completed reaction by TLC, the reaction mixture was added with 20ml of water for dilution of the mother liquid, and extracted with ethyl acetate three times. The combined organic phases were washed with saturated brine, dried over anhydrous sodium sulfate, and subjected to column chromatography to provide 4-ethoxy-2-fluoro-1-nitrobenzene(**181-a**, 1.04g, 5.6mmol, 88% yield). MS Calcd: 185; MS Found: 186([M+H]$^+$).

Step 2: To a 100ml flask were sequentially added 4-ethoxy-2-fluoro-1-nitrobenzene(**181-a**, 900mg, 4.78mmol), N-methyl methanesulfonamide (1.0g, 9.57mmol), anhydrous potassium carbonate (1.9g, 14.3mmol) and acetonitrile (30mL), and stirred at 90°C for 6h. Upon indication of completed reaction by TLC, the reaction mixture was added with 20ml of water for dilution of the mother liquid, and extracted with ethyl acetate three times. The combined organic phases were washed with saturated brine, dried over anhydrous sodium sulfate, and subjected to column chromatography to provide N-(5-ethoxy-2-nitrophenyl)-N-methyl methanesulfonamide **(181-b,** 1.g, 4.67mmol, 98% yield). MS Calcd: 274; MS Found: 275([M+H]$^+$).

Step 3: To a 100mL flask were added N-(5-ethoxy-2-nitrophenyl)-N-methyl methanesulfonamide **(181-b,** 1.3g, 4.7mmol), Fe(23mmol, 1.3g), ammonium chloride (47mmol, 2.5g), and 20mL of ethanol and 4ml of water. The mixture was stirred at 90°C for 3h. When TLC indicated a completed reaction, the mixture was cooled, added with 50ml of ethyl acetate, filtered to remove iron powder, and extracted with ethyl acetate and water. The organic phase was washed with saturated sodium chloride aqueous solution, dried over anhydrous sodium sulfate, followed by solvent removal by rotary evaporation to provide N-(2-amino-5-ethoxy phenyl)-N-methyl methanesulfonamide **(181-c,** 1.1g, 4.4mmol, 95% yield). MS Calcd: 244; MS Found: 245([M+H]$^+$).

Step 3: To a 100mL two-necked flask were sequentially added 4,6-dichloro-N-ethoxy nicotinamide **(int-2,** 300mg, 1.2mmol), N-(2-amino-5-ethoxy phenyl)-N-methyl methanesulfonamide **(181-c,** 375mg, 1.53mmol), and 10ml of anhydrous tetrahydrofuran as solvent. The mixture was added at room temperature with a solution of LiHMDS in tetrahydrofuran (5mL, 1mmol/mL), with continuous stirring at room temperature for 2h. Upon indication of completed reaction by TLC, the mixture was adjusted with aqueous hydrochloride (1N) to pH 5, and extracted with ethyl acetate (30mLx3). The combined organic layers were dried, concentrated, and purified by column chromatography to provide 6-chloro-N-ethoxy-4-((4-ethoxy-2-(N-methyl methanesulfonamido)phenyl)amino)nicotinamide **(181-d,** 450mg, 0.96mmol, 80% yield). MS Calcd: 4442; MS Found: 443([M+H]$^+$).

Step 4: 6-chloro-N-ethoxy-4-((4-ethoxy-2-(N-methyl methanesulfonamido)phenyl)amino)nicotinamide **(181-d,**

150mg, 0.33mmol), 2,6-dimethyl pyrimidin-4-ylamine (54mg, 0.44mmol), cesium carbonate (321mg, 1.0mmol), Xant-Phos(38mg, 0.066mmol) and $Pd_2(dba)_3$(62mg, 0.066mmol) were added to anhydrous dioxane (8ml). After atmosphere replacement with nitrogen three times, the reaction mixture was heated to 120°C with stirring for 8h, and filtered by suction. The filtrate was concentrated and purified by high performance preparative thin layer chromatography (MeOH:DCM=1:15) to provide the title compound: 6-(((2,6-dimethyl pyrimidin-4-yl)amino)-N-ethoxy-4-((4-ethoxy-2-(N-methyl methanesulfonamido)phenyl)amino)nicotinamide **(181,** 70mg, 0.13mmol, 40% yield). MS Calcd: 529; MS Found: 530([M+H]$^+$). $^1$H NMR (400 MHz, DMSO-$d_6$)δ11.55 (s, 1H), 9.93 (s, 1H), 9.63 (s, 1H), 8.30 (s, 1H), 7.62 (s, 1H), 7.42 (d, *J* = 8.8 Hz, 1H), 7.17 (d, *J* = 2.8 Hz, 1H), 7.05-7.02 (m, 2H), 4.08 (q, *J* = 7.2 Hz, 2H), 3.93 (q, *J* = 7.2 Hz, 2H), 3.11 (s, 3H), 3.06 (s, 3H), 2.27 (s, 3H), 2.25 (s, 3H), 1.35 (t, *J* = 7.2 Hz, 3H), 1.22 (t, *J* = 7.2 Hz, 3H).

Example **182**

6-(((2,6-dimethyl pyrimidin-4-yl)amino)-N-ethoxy-4-((4-ethynyl-5-fluoro-2-(N-methyl methanesulfonamido)phenyl)amino)nicotinamide

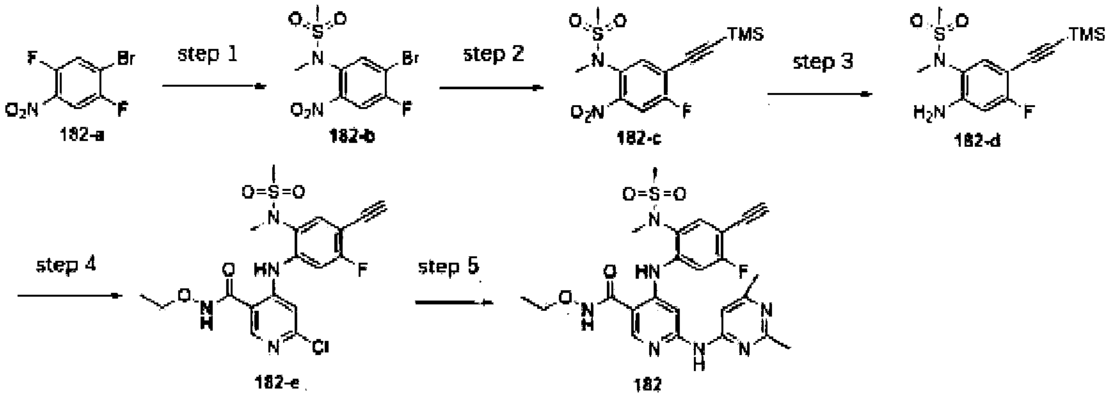


182-a 182-b 182-c 182-d 182-e 182

Step 1: To a 250mL flask were added 4-bromo-2,5-difluoro-nitrobenzene(**182-a**, 5g, 0.021mol), N-methyl methanesulfonamide (3.21g, 0.029mol), anhydrous potassium carbonate (5.79g, 0.42mol), and about 100ml of anhydrous acetonitrile as solvent. The mixture was stirred at 90°C for 6 hours. When TLC indicated a completed reaction, the reaction mixture was mixed with 200-300 mesh silica gel, concentrated under reduced pressure at 40°C, separated and purified by column chromatography, and eluted with petroleum ether:ethyl acetate=10:1 gradually changing to petroleum ether:ethyl acetate=2:1 to provide N-(5-bromo-4-fluoro-2-nitrophenyl)-N-methyl methanesulfonamide **(182-b,** 6.3g, 0.019mol, 92.64% yield).

Step 2: To a 250mL flask were added N-(5-bromo-4-fluoro-2-nitrophenyl)-N-methyl methanesulfonamide **(182-b,** 3g, 0.009mol), trimethylsilyl acetylene (1.17g, 0.012mol), cuprous (I) iodide (0.176g, 0.923mmol), Pd(dppf)$Cl_2$ (0.336g, 0.461mmol), and triethylamine (80mL). After atmosphere replacement with nitrogen three times, the mixture was allowed to react at 60°C for 3 hours. When TLC indicated a completed reaction, the mixture was filtered by suction, and the filtrate was concentrated under reduced pressure at 40°C, separated and purified by column chromatography, eluted with petroleum ether:ethyl acetate=5:1 to provide N-(4-fluoro-2-nitro-5-((trimethylsilyl)ethynyl)phenyl)-N-methyl methanesulfonamide **(182-c,** 2.02g, 5.872mmol, 63.72% yield).

Step 3: To a flask containing **182-c**(2.02g, 5.872mmol) were added reduction iron powder (1.64g, 0.030mol), ammonium chloride (3.14g, 0.059mol), and 60mL of ethanol and 10ml of water. The mixture was allowed to react with stirring at 80°C for 6 hours. When TLC indicated a completed reaction, the mixture was filtered by suction. The filtrate was added with water and extracted with ethyl acetate to provide the target Intermediate N-(2-amino-4-fluoro-5-((trimethylsilyl) ethynyl)phenyl)-N-methyl methanesulfonamide **(182-d,** 1.47g, 4.681mmol, 80% yield). MS Calcd: 314; MS Found:315([M+H]$^+$).

Step 4: To a 50mL two-necked flask were added 4,6-dichloro-N-ethoxy nicotinamide **(int-2,** 193mg, 0.825mmol), N-(2-amino-4-fluoro-S-((trimethylsilyl)ethynyl)phenyl)-N-methyl methanesulfonamide **(182-d,** 200mg, 0.637mmol), and 7ml of anhydrous DMA. The reaction was vacuumed and refilled with $N_2$ three times, cooled in ice bath and followed by adding with LiHMDS(3.2mL, 1mol/L, 3.2mmol) dropwise. After the addition, the mixture was warmed to 50°C to react for 6 hours. The reaction mixture was sampled for TLC several times to determine reaction termination. The reaction mixture was cooled in ice bath, added with water and saturated ammonium chloride aqueous solution, and extracted with ethyl acetate. The organic phase was mixed with 200-300 mesh silica gel for loading onto and purifying by column chromatography, eluted with petroleum ether:ethyl acetate=2:1 to provide 6-chloro-N-ethoxy-4-((4-ethynyl-5-fluoro-2-(N-methyl methanesulfonamido)phenyl)amino)nicotinamide **(182-e,** 160mg, 0.363mmol, 57.55% yield). MS Calcd: 440; MS Found: 441([M+H]+).

Step 5: 6-chloro-N-ethoxy-4-((4-ethynyl-5-fluoro-2-(N-methyl methanesulfonamido)phenyl)amino)nicotinamide **(182-e,** 80mg, 0.182mmol), 4-amino-2,6-dimethyl pyrimidine (29.10mg, 0.236mmol), $pd_2(dba)_3$ (49.95mg, 0.055mmol), XantPhos(63.12mg, 0.109mmol), $Cs_2CO_3$ (177.71mg, 0.545mmol), and 1,4-dioxane (5mL) were combined. After atmosphere replacement with nitrogen three times, the mixture was allowed to react at 120°C for 6 hours. When TLC indicated a completed reaction, the mixture was filtered by suction, and the filtrate was concentrated under reduced pressure at 40°C, mixed with 200-300 mesh silica gel for loading onto and purifying by column chromatography, eluted with DCM:MeOH=30: 1, to provide the target compound 6-(((2,6-dimethyl pyrimidin-4-yl)amino)-N-ethoxy-4-((4-ethynyl-5-fluoro-2-(N-methyl methanesulfonamido)phenyl)amino)nicotinamide **(182,** 14mg, 0.027mmol, 14.53% yield). MS Calcd:527; MS Found:528([M+H]+). $^1$H NMR (400 MHz, DMSO-$d_6$)δ11.74 (s, 1H), 10.54 (s, 1H), 10.20 (s, 1H), 8.41 (s, 1H), 8.10 (s, 1H), 7.77 (d, *J* = 7.6 Hz, 1H), 7.56 (d, *J* = 11.2 Hz, 1H), 7.21 (s, 1H), 4.52 (s, 1H), 3.94 (q, *J* = 7.2 Hz, 2H), 3.19 (s, 3H), 3.17 (s, 3H), 2.41 (s, 3H), 2.30 (s, 3H), 1.22 (t, *J* = 7.2 Hz, 3H).

Example **183**

6-(((2,6-dimethyl pyrimidin-4-yl)amino)-N-ethoxy-4-((4-fluoro-2-(N-methyl cyclopropylsulfonamido)phenyl)amino)nicotinamide

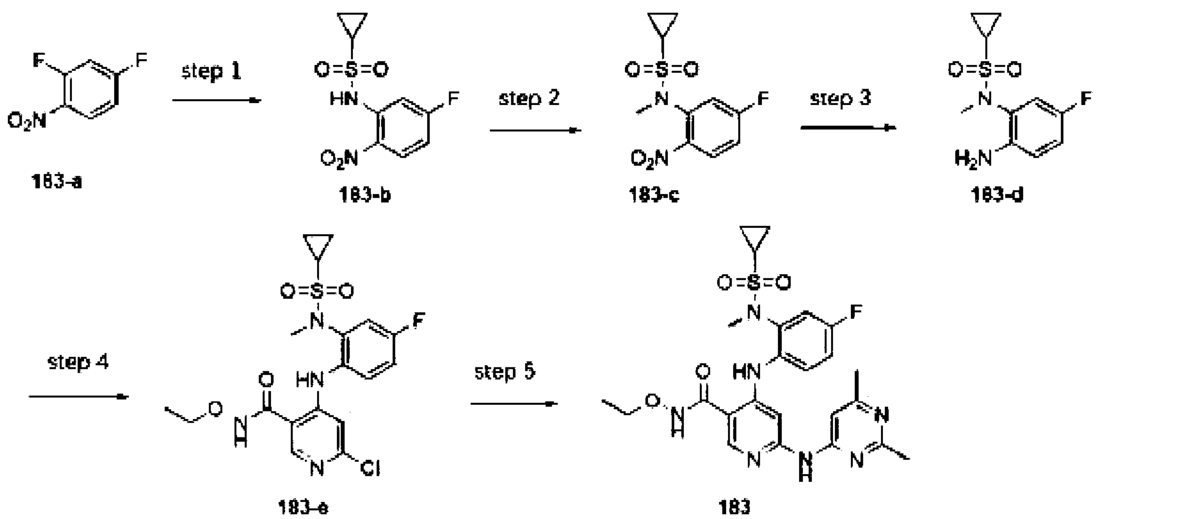


Step 1: To a 50ml reaction flask was added 2,4-difluoro-1-nitrobenzene(**183-a**, 238mg, 1.5mmol) dissolved in N,N-dimethyl formamide (5ml), followed by potassium carbonate (414mg, 3mmol) and cyclopropylsulfonamide (218mg, 1.8mmol). The mixture was heated to 90°C with stirring for 3h. Upon indication of completed reaction by TLC, the reaction mixture was washed respectively with water (30 ml x 1) and brine (30 ml x 1), dried over anhydrous sodium sulfate, and concentrated to provide a crude material, which was purified by silica gel column chromatography (petroleum ether/ethyl acetate=3: 1) to provide N-(5-fluoro-2-nitrophenyl) cyclopropylsulfonamide **(183-b,** 240mg, 0.923mmol, 61.5% yield). MS Calcd:260.03; MS Found: 261.05 ([M+H]+).

Step 2: To a 50ml reaction flask was added N-(5-fluoro-2-nitrophenyl) cyclopropylsulfonamide **(183-b,** 240mg, 0.92mmol) dissolved in N,N-dimethyl formamide (50ml), followed by sodium hydride (55.4mg, 60%, 1.38mmol) and iodomethane (157.2mg, 1.11mmol). The reaction mixture was stirred at room temperature for 3h. Upon indication of completed reaction by TLC, the reaction mixture was added with water and ethyl acetate, extracted with ethyl acetate (30 ml x 2). The organic phase was washed with brine (30 ml x 1), dried over anhydrous sodium sulfate, and concentrated to obtain N-(5-fluoro-2-nitrophenyl)-N-methylcyclopropanesulfonamide (**183-c,** 273mg, 0.92mmol, 100% yield). MS Calcd: 274.09; MS Found: 275.22 ([M+H]+).

Step 3: N-(5-fluoro-2-nitrophenyl)-N-methyl cyclopropylsulfonamide (**183-c,** 273mg, 1mmol), ammonium chloride (530mg, 10 mmol) and iron powder (280 mg, 5 mmol) were sequentially added to 10 mL of mixed solvent of water and ethanol (1:4). The mixture was stirred under reflux for 6 hours. Upon indication of completed reaction by TLC, the reaction mixture was filtered by suction. The filtrate was concentrated, and the residue was separated and purified by silica gel column chromatography to provide N-(2-amino-5-fluorophenyl)-N-methyl cyclopropylsulfonamide (**183-d,** 160 mg, 0.653

mmol, 65.8% yield). MS Calcd: 244.09; MS Found: 245.22 ([M+H]+).

Step 4: N-(2-amino-5-fluorophenyl)-N-methyl cyclopropylsulfonamide (**183-d,** 160 mg, 0.65 mmol), 4,6-dichloro-N-ethoxy nicotinamide (**int-2**, 154 mg, 0.65 mmol) were added to 10 ml of anhydrous N,N-dimethylacetamide. The mixture was added at room temperature with a solution of LiHMDS in tetrahydrofuran (2 ml, 1.97 mmol), and stirred at room temperature for 3 hours. Upon indication of completed reaction by TLC, the mixture was adjusted with aqueous hydrochloride (1N) to pH 5, and extracted with ethyl acetate (20 ml x 3). The combined organic layers were dried, concentrated and then purified by column chromatography (petroleum ether:ethyl acetate =1:1) to provide 6-chloro-N-ethoxy-4-((4-fluoro-2-(N-methyl cyclopropylsulfonamido)phenyl)amino)nicotinamide (**183-e,** 180mg, 0.41 mmol, 62.06% yield). MS Calcd: 442.10; MS Found: 443.29 ([M+H]+).

Step 5: 6-chloro-N-ethoxy-4-((4-fluoro-2-(N-methyl cyclopropylsulfonamido)phenyl)amino)nicotinamide (**183-e,** 100mg, 0.23 mmol, ), 2,6-dimethyl pyrimidin-4-ylamine (27.06 mg, 0.22mmol), cesium carbonate (195 mg, 0.6 mmol), Xant-Phos(23.12mg, 0.04mmol) and $Pd_2(dba)_3$(27.06mg, 0.02 mmol) were added to anhydrous dioxane (5 ml). After the atmosphere of the mixture was evacuated to vacuum and refilled with nitrogen, the reaction mixture was heated to 120°C with stirring for 6 hours, and filtered by suction. The filtrate was concentrated and purified by preparative TLC (MeOH:DCM=1:20) to provide the title compound: 6-(((2,6-dimethyl pyrimidin-4-yl)amino)-N-ethoxy-4-((4-fluoro-2-(N-methyl cyclopropylsulfonamido)phenyl)amino)nicotinamide (**183**, 35mg, 0.066mmol, 29.6% yield). MS Calcd: 529.21; MS Found: 530.20 ([M+H]+). $^1$H NMR (400 MHz, DMSO-$d_6$)δ11.61 (s, 1H), 10.02 (s, 1H), 9.89 (s, 1H), 8.34 (s, 1H), 7.78 (s, 1H), 7.64 - 7.48 (m, 2H), 7.37-7.32 (m, 1H), 7.05 (s, 1H), 3.93 (q, *J* = 7.2 Hz, 2H), 3.17 (s, 3H), 2.86-2.79 (m, 1H), 2.30 (s, 3H), 2.26 (s, 3H), 1.20 (t, *J* = 7.2 Hz, 3H), 1.03-1.01 (m, 2H), 0.87-0.85 (m, 2H).

Example **184**

6-((4,6-dimethyl pyrimidin-2-yl)amino)-N-ethoxy-4-((4-methyl-2-(N-methyl cyclopropylsulfonamido)phenyl)amino)nicotinamide

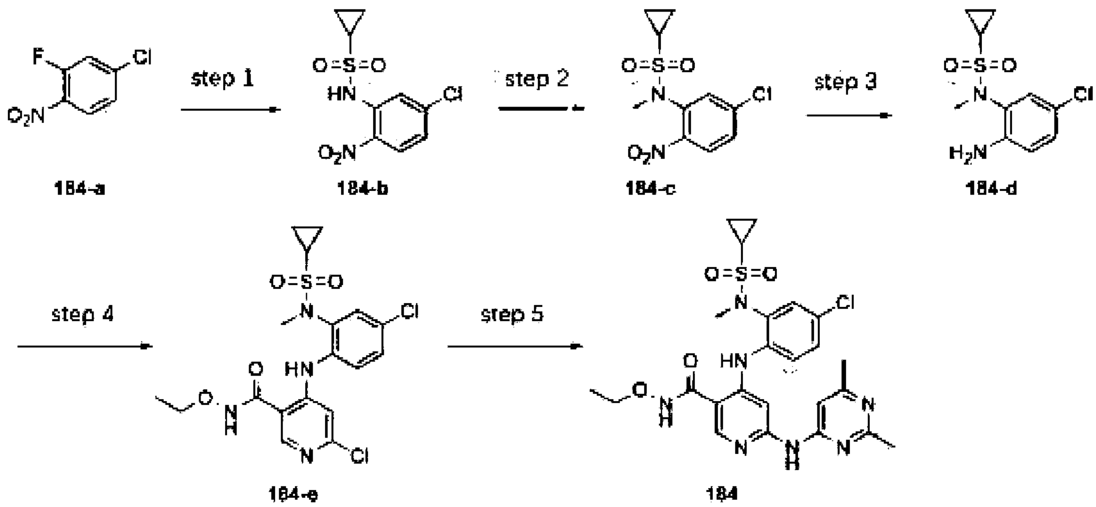


184-a 184-b 184-c 184-d 184-e 184

Step 1: To a 100ml reaction flask was added 4-chloro-2-fluoro-1-nitrobenzene(**184-a**, 350mg, 2mmol) dissolved in N,N-dimethyl formamide (10ml), followed by potassium carbonate (414mg, 3mmol), cyclopropylsulfonamide (242mg, 2mmol). The mixture was heated to 90°C with stirring for 3h. Upon indication of completed reaction by TLC, the reaction mixture was washed respectively with water (30 ml x 1) and brine (30 ml x 1), dried over anhydrous sodium sulfate, and concentrated to provide a crude material, which was purified by silica gel column chromatography (petroleum ether/ethyl acetate=3:1) to provide the product N-(5-chloro-2-nitrophenyl) cyclopropylsulfonamide (**184-b,** 441mg, 80% yield). MS Calcd:276.05; MS Found: 277.10 ([M+H]+)

Step 2: To a 100ml reaction flask was added N-(5-chloro-2-nitrophenyl) cyclopropylsulfonamide (**184-b,** 441mg, 1.60mmol) dissolved in N,N-dimethyl formamide (30ml), followed by sodium hydride (160mg, 60%, 4mmol), and iodomethane (341mg, 2.4mmol). The reaction mixture was stirred at room temperature for 3h. Upon indication of completed reaction by TLC, the reaction mixture was added with water and ethyl acetate, extracted with ethyl acetate (30 ml x 2). The organic phase was washed with brine (30 ml x 1), dried over anhydrous sodium sulfate, and concentrated to obtain N-(5-chloro-2-nitrophenyl)-N-methylcyclopropanesulfonamide (**184-c,** 450mg, 1.55mmol, 96.9% yield). MS Calcd: 290.09; MS Found: 291.22 ([M+H]+).

Step 3: N-(5-chloro-2-nitrophenyl)-N-methyl cyclopropylsulfonamide (**184-c,** 270mg, 0.9mmol), ammonium chloride (486mg, 9 mmol) and iron powder (252 mg, 4.5 mmol) were sequentially added to 10 mL of mixed solvent of water and ethanol (1:4). The mixture was stirred under reflux for 3 hours. Upon indication of completed reaction by TLC, the reaction mixture was filtered by suction. The filtrate was concentrated, and the residue was separated and purified by silica gel column chromatography to provide N-(2-amino-5-chloro phenyl)-N-methyl cyclopropylsulfonamide (**184-d,** 190 mg, 0.73mmol, 81.2% yield). MS Calcd: 260.09; MS Found: 261.22 ($[M+H]^+$).

Step 4: N-(2-amino-5-chloro phenyl)-N-methyl cyclopropylsulfonamide (**184-d,** 190mg, 0.7 mmol), 4,6-dichloro-N-ethoxy nicotinamide (**int-2,** 165 mg, 0.7 mmol) were added into 10 ml of anhydrous N,N-dimethylacetamide. The mixture was added at room temperature with a solution of LiHMDS in tetrahydrofuran (2.1 ml, 2.1 mmol), and stirred at room temperature for 3 hours. Upon indication of completed reaction by TLC, the mixture was adjusted with aqueous hydrochloride (1N) to pH 5, and extracted with ethyl acetate (20 ml x 3). The combined organic layers were dried, concentrated and then purified by column chromatography (petroleum ether:ethyl acetate =1:1) to provide 6-chloro-4-((4-chloro-2-((N-methyl cyclopropylsulfonamido)phenyl)amino)-N-ethoxy nicotinamide (**184-e,** 278mg, 0.60 mmol, 85.2% yield). MS Calcd: 458.10; MS Found: 459.29 ($[M+H]^+$).

Step 5: 6-chloro-4-((4-chloro-2-((N-methyl cyclopropylsulfonamido)phenyl)amino)-N-ethoxy nicotinamide (**184-e,** 125mg, 0.27 mmol, ), 2,6-dimethyl pyrimidin-4-ylamine (36.8 mg, 0.3mmol), cesium carbonate (265.2 mg, 0.816mmol), Xant-Phos(31.2mg, 0.054mmol) and $Pd_2(dba)_3$(24.7mg, 0.027 mmol) were added to anhydrous dioxane (5 ml). After the atmosphere of the mixture was evacuated to vacuum and refilled with nitrogen, the reaction mixture was heated to 120°C with stirring for 6 hours, and filtered by suction. The filtrate was concentrated and purified by preparative TLC (MeOH:DCM=1:20) to provide the title compound: 4-((4-chloro-2-(N-methyl cyclopropylsulfonamido)phenyl)amino)-6-(((2,6-dimethyl pyrimidin-4-yl)amino)-N-ethoxy nicotinamide (**184,** 30mg, 0.055 mmol, 20.27% yield). MS Calcd: 545.21; MS Found: 546.20 ($[M+H]^+$). $^1$H NMR (400 MHz, DMSO-$d_6$)δ11.65 (s, 1H), 10.10 (s, 1H), 10.07 (s, 1H), 8.36 (s, 1H), 7.94 (s, 1H), 7.73 (d, $J$ = 2.4 Hz, 1H), 7.63 (d, $J$ = 8.4 Hz, 1H), 7.51 (dd, $J$ = 8.4, 2.4 Hz, 1H), 7.08 (s, 1H), 3.93 (q, $J$ = 7.2 Hz, 2H), 3.19 (s, 3H), 2.89-2.83 (m, 1H), 2.34 (s, 3H), 2.27 (s, 3H), 1.21 (t, $J$ = 7.2 Hz, 3H), 1.04-1.01 (m, 2H), 0.87-0.85 (m, 2H).

Example **185**

4-((4-cyclopropyl-2-(N-methyl ethyl sulfonamido)phenyl)amino)-6-(((2,6-dimethyl pyrimidin-4-yl)amino)-N-ethoxy nicotinamide

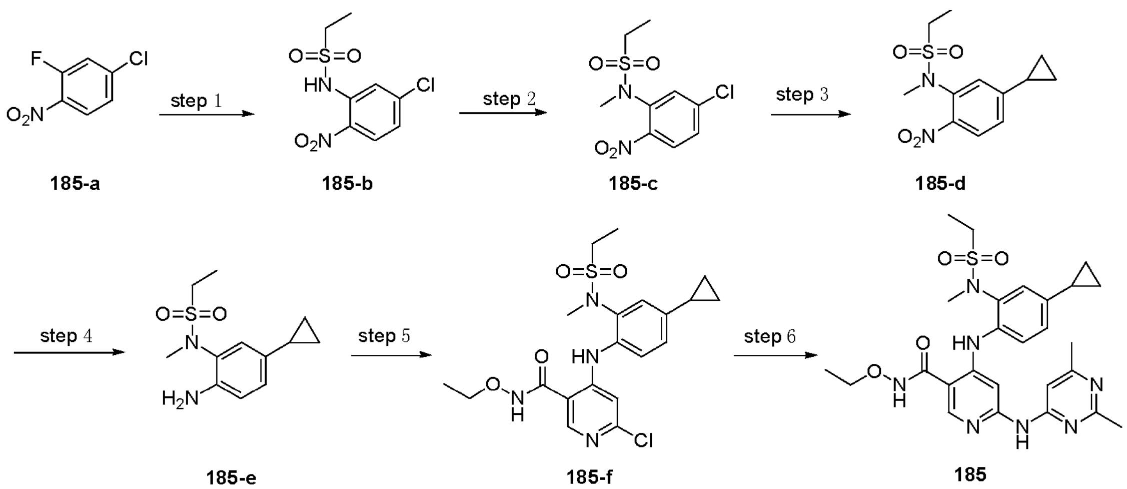


Step 1: 2-fluoro-4-chloronitrobenzene(185-a, 350mg, 2.00mmol), ethanesulfonamide (218mg, 2.00mmol) and potassium carbonate (552mg, 4.00mmol) were added to 5ml of anhydrous DMF. The mixture was stirred under reflux at 90°C for 2 hours, added with 50ml of water, and extracted with ethyl acetate (30 ml x 3). The combined organic layers were dried and concentrated to provide N-(5-chloro-2-nitrophenyl)ethanesulfonamide (**185-b,** 520mg, 1.95mmol, 97.5% yield). MS Calcd: 264; MS Found: 265($[M+H]^+$).

Step 2: N-(5-chloro-2-nitrophenyl) ethanesulfonamide (**185-b,** 520mg, 1.95mmol) was dissolved in 5ml of THF to form a clear and colorless solution. The mixture was cooled in ice water bath to 0°C, added with NaH (160mg, 3.8mmol) and allowed to react at room temperature for 1h, followed by adding with $CH_3I$(266mg, 1.95mmol) to react for 1h. To the

reaction mixture was added slowly 30ml of water to quench the reaction, and extracted with ethyl acetate (15ml*3). The organic phase was purified by column chromatography, eluted with petroleum ether:ethyl acetate=2:1, to provide N-(5-chloro-2-nitrophenyl)-N-methyl ethanesulfonamide (**185-c,** 300.00mg, 1.08mmol, 57.7% yield). MS Calcd: 278; MS Found: 279([M+H]$^+$).

Step 2: N-(5-chloro-2-nitrophenyl)-N-methyl ethanesulfonamide (**185-c,** 278.00mg, 1.0mmol), cyclopropyl boronic acid (86mg, 1.00mmol), cesium carbonate (652mg, 2.00mmol), and Pd(dppf)$Cl_2$(73.1mg, 0.1mmol) were added to anhydrous dioxane (5ml) and water (1ml). After the atmosphere of the mixture was evacuated to vacuum and refilled with nitrogen, the reaction mixture was heated to 100°C with stirring for 2 hours, and added with 20ml of water, and extracted with EA (10ml*3). The organic phase was purified by column chromatography, eluted with petroleum ether:ethyl acetate=2:1 to provide N-(5-cyclopropyl-2-nitrophenyl)-N-methyl ethanesulfonamide (**185-d,** 250mg, 0.88mmol, 88% yield). MS Calcd: 284; MS Found: 285 ([M+H]$^+$).

Step 3: N-(5-cyclopropyl-2-nitrophenyl)-N-methyl ethanesulfonamide (**185-d,** 250mg, 0.88mmol) was added to 20ml of methanol containing 25mg Pd/C. The mixture was allowed to react at room temperature for 2h. The mixture was filtered to remove Pd/C, and subjected to rotary evaporation to remove methanol, to provide N-(5-cyclopropyl-2-nitrophenyl)-N-methyl ethanesulfonamide (**185-e,** 200mg, 0.79mmol, 89.4% yield). MS Calcd: 254; MS Found: 255([M+H]$^+$).

Step 4: 4,6-dichloro-N-ethoxy nicotinamide (**int-2,** 185mg, 0.78mmol) and N-(5-cyclopropyl-2-nitrophenyl)-N-methyl ethanesulfonamide (**185-e,** 200mg, 0.79mmol) were added to 5ml of anhydrous tetrahydrofuran. The mixture was added at room temperature with a solution of LiHMDS in tetrahydrofuran (2ml, 2.00mmol), and stirred at room temperature for 2 hours. The mixture was adjusted with aqueous hydrochloride (1N) to pH 5, and extracted with ethyl acetate (10ml x 3). The combined organic layers were dried, concentrated and then purified by column chromatography (petroleum ether:ethyl acetate=1:1), to provide 6-chloro-4-((4-cyclopropyl-2-(N-methyl ethyl sulfonamido)phenyl)amino)-N-ethoxy nicotinamide (**185-f,** 100.00mg, 0.22mmol, 28.34% yield). MS Calcd: 452; MS Found: 453 ([M+H]$^+$).

Step 5: 6-chloro-4-((4-cyclopropyl-2-(N-methyl ethyl sulfonamido)phenyl)amino)-N-ethoxy nicotinamide (**185-f,** 100.00mg, 0.22mmol), 4-amino-2,6-dimethyl pyrimidine (27.06mg, 0.22mmol), cesium carbonate (163mg, 0.5 mmol), Xant-Phos(57.6mg, 0.1mmol) and $Pd_2(dba)_3$(45.5mg, 0.05mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and subjected to high performance thin layer preparative chromatography to provide the title compound: 4-((4-cyclopropyl-2-(N-methyl ethyl sulfonamido)phenyl) amino)-6-(((2,6-dimethyl pyrimidin-4-yl)amino)-N-ethoxy nicotinamide (**185,** 38mg, 0.070mmol, 32.4% yield). MS Calcd: 539; MS Found: 540 ([M+H]$^+$). $^1$H NMR (400 MHz, DMSO-$d_6$)δ11.81 (s, 1H), 11.35 (s, 1H), 9.91 (s, 1H), 8.43 (s, 1H), 7.40 (d, $J$ = 8.4 Hz, 1H), 7.35 (d, $J$ = 2.0 Hz, 1H), 7.14 (dd, $J$ = 8.4, 2.0 Hz, 1H), 3.95 (q, $J$ = 7.2 Hz, 2H), 3.22 (q, $J$ = 7.2 Hz, 2H), 3.13 (s, 3H), 2.49 (s, 3H), 2.46 (s, 3H), 2.03-1.97 (m, 1H), 1.22 (q, $J$ = 7.2 Hz, 6H), 1.03-0.99 (m, 2H), 0.76 - 0.69 (m, 2H).

Example **186**

6-(((2,6-dimethyl pyrimidin-4-yl)amino)-N-ethoxy-4-((6-methyl-2-(N-methyl methanesulfonamido)pyridin-3-yl)amino)nicotinamide

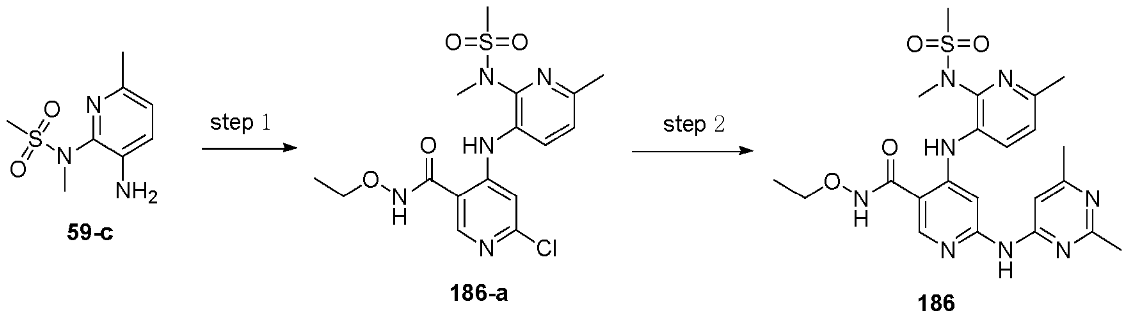


59-c 186-a 186

Step 1: To a 100mL two-necked flask were sequentially added 4,6-dichloro-N-ethoxy nicotinamide (**int-2,** 400mg, 1.7mmol), N-(3-amino-6-methyl pyridin-2-yl)-N-methyl methanesulfonamide (**59-c**, 280mg, 1.3mmol), and 10ml of anhydrous tetrahydrofuran as solvent. The mixture was added at room temperature with a solution of LiHMDS in tetrahydrofuran (5mL, 1mmol/mL), with continuous stirring at room temperature for 2h. Upon indication of completed reaction by TLC, the mixture was adjusted with aqueous hydrochloride (1N) to pH 5, and extracted with ethyl acetate (30mLx3). The combined organic layers were dried, concentrated, and purified by column chromatography to provide 6-chloro-N-ethoxy-4-((6-methyl-2-(N-methyl methanesulfonamido)pyridin-3-yl)amino)nicotinamide (**186-a,** 440mg, 1.06mmol, 81% yield). MS Calcd: 413; MS Found: 414($[M+H]^+$)

Step 2: 6-chloro-N-ethoxy-4-((6-methyl-2-(N-methyl methanesulfonamido)pyridin-3-yl)amino)nicotinamide (39118-d, 130mg, 0.3mmol), 2,6-dimethyl pyrimidin-4-ylamine (58mg, 0.47mmol), cesium carbonate (302mg, 0.93mmol), Xant-phos(53mg, 0.093mmol) and $Pd_2(dba)_3$(58mg, 0.062mmol) were added to anhydrous dioxane (8ml). After atmosphere replacement with nitrogen three times, the reaction mixture was heated to 120 °C with stirring for 8h, and filtered by suction. The filtrate was concentrated, purified by plate chromatography (MeOH:DCM=1:15) to provide 6-(((2,6-dimethyl pyrimidin-4-yl)amino)-N-ethoxy-4-((6-methyl-2-(N-methyl methanesulfonamido)pyridin-3-yl)amino)nicotinamide (**186**, 50mg, 0.1mmol, 32% yield). MS Calcd: 500; MS Found: 501($[M+H]^+$). $^1$H NMR (400 MHz, DMSO-$d_6$)δ11.66 (s, 1H), 10.08 (s, 1H), 10.01 (s, 1H), 8.37 (s, 1H), 7.96 (d, *J* = 8.4 Hz, 1H), 7.82 (s, 1H), 7.37 (d, *J* = 8.4 Hz, 1H), 7.08 (s, 1H), 3.94 (q, *J* = 7.2 Hz, 2H), 3.19 (s, 3H), 3.12 (s, 3H), 3.34 (s, 3H), 2.34 (s, 3H), 2.27 (s, 3H), 1.23 (t, *J* = 7.2 Hz, 3H).

Example **187**

4-((4-chloro-2-(N-methyl ethyl sulfonamido)phenyl)amino)-6-((2,6-dimethyl pyrimidin-4-yl)amino)-N-ethoxy nicotinamide

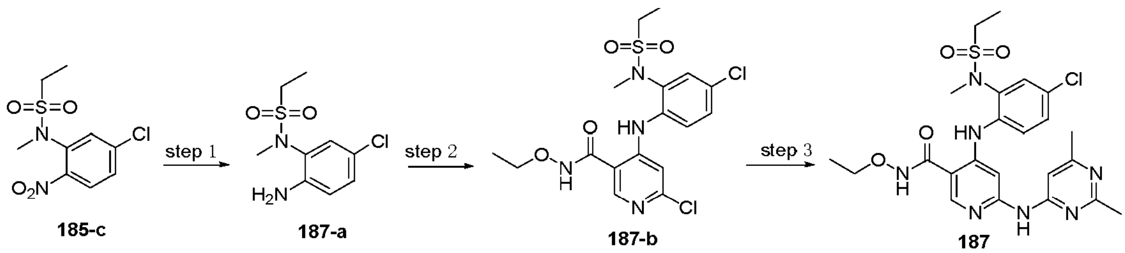


185-c 187-a 187-b 187

Step 1: N-(5-chloro-2-nitrophenyl)-N-methyl ethanesulfonamide (**185-c**, 278.00mg, 1.0mmol), ) was added to 20mL of methanol containing 30mg Pd/C, and allowed to react at room temperature for 2h. The mixture was filtered to remove Pd/C, and subjected to rotary evaporation to remove methanol, to provide N-(5-chloro-2-amino phenyl)-N-methyl ethanesulfonamide (**187-a**, 240.00mg, 0.967mmol, 96.7% yield). MS Calcd: 248; MS Found: 249($[M+H]^+$).

Step 2: 4,6-dichloro-N-ethoxy nicotinamide (**int-2,** 235mg, 1.00mmol), N-(5-chloro-2-amino phenyl)-N-methyl ethanesulfonamide (**187-a**, 240.00mg, 0.967mmol) were added to 5ml of anhydrous tetrahydrofuran. The mixture was added at room temperature with a solution of LiHMDS in tetrahydrofuran (3ml, 3.00mmol), and stirred at room temperature for 2 hours. The mixture was adjusted with aqueous hydrochloride (1N) to pH 5, and extracted with ethyl acetate (10ml x 3). The combined organic layers were dried, concentrated and then purified by column chromatography (petroleum ether:ethyl acetate =1:1), to provide 6-chloro-4-((4-chloro-2-(N-methyl ethyl sulfonamido)phenyl)amino)-N-ethoxy nicotinamide (**187-b**, 95.00mg, 0.21mmol, 21.98% yield). MS Calcd: 446; MS Found: 447 ($[M+H]^+$).

Step 3: 6-chloro-4-((4-chloro-2-(N-methyl ethyl sulfonamido)phenyl)amino)-N-ethoxy nicotinamide (**187-b,** 95.00mg, 0.21mmol), 4-amino-2,6-dimethyl pyrimidine (27.06mg, 0.22mmol), cesium carbonate (163mg, 0.5 mmol), Xant-Phos(57.6mg, 0.1mmol) and $Pd_2(dba)_3$(45.5mg, 0.05mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and purified by high performance preparative thin layer chromatography to provide the title compound: 4-((4-chloro-2-(N-methyl ethyl sulfonamido)phenyl)amino)-6-((2,6-dimethyl pyrimidin-4-yl)amino)-N-ethoxy nicotinamide (**187,** 32mg, 0.060mmol, 28.6% yield). MS Calcd: 533; MS Found: 534 ($[M+H]^+$). $^1$H NMR (400 MHz, DMSO-$d_6$)δ11.66 (s, 1H), 10.07 (s, 1H), 10.02 (s, 1H), 8.37 (s, 1H), 7.87 (s, 1H), 7.71 (d, *J* = 2.4 Hz, 1H), 7.62 (d, *J* = 8.8 Hz, 1H), 7.51 (dd, *J* = 8.8, 2.4 Hz, 1H), 7.08 (s, 1H), 3.94 (q, *J* = 7.2 Hz, 2H), 3.33-3.26 (m, 2H), 3.16 (s, 3H), 2.33 (s, 3H), 2.27 (s, 3H), 1.22 (t, *J* = 7.2 Hz, 6H).

Example **188**

4-((4-cyclopropyl-5-fluoro-2-(N-methyl methanesulfonamido)phenyl)amino)-N-ethoxy-6-((2-methoxy pyrimidin-4-yl) amino)nicotinamide

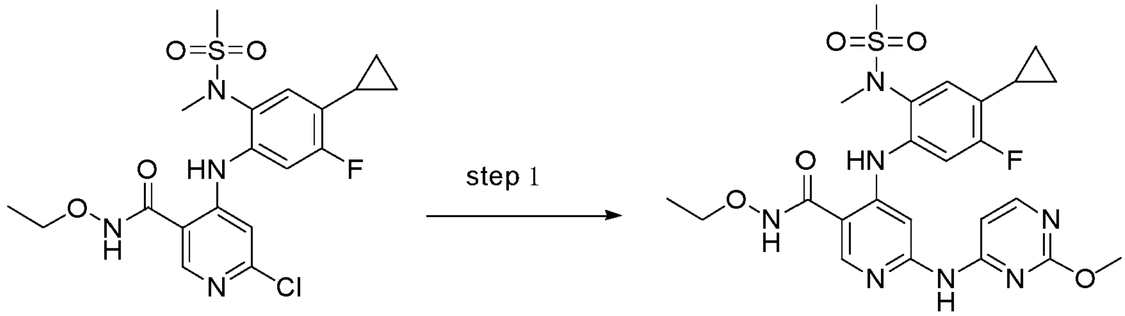


96-e 188

Step 1: 6-chloro-4-((4-cyclopropyl-5-fluoro-2-(N-methyl methanesulfonamido)phenyl)amino)-N-ethoxy nicotinamide (**96-e,** 278mg, 0.5mmol), 4-amino-2-methoxy pyrimidine (63mg, 0.5mmol), cesium carbonate (326mg, 1.0mmol), Xant-Phos(57.6mg, 0.1mmol) and $Pd_2(dba)_3$(45.5mg, 0.05mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and purified by high performance preparative thin layer chromatography to provide the title compound: 4-((4-cyclopropyl-5-fluoro-2-(N-methyl methanesulfonamido) phenyl)amino)-N-ethoxy-6-((2-methoxy pyrimidin-4-yl)amino)nicotinamide (**188,** 60mg, 0.11mmol, 22% yield). MS Calcd: 545; MS Found: 546 ($[M+H]^+$). $^1H$ NMR (400 MHz, DMSO-$d_6$)δ11.69 (s, 1H), 10.25 (s, 1H), 10.00 (s, 1H), 8.36 (s, 1H), 8.20 (s, 1H), 7.78 (s, 1H), 7.35 (d, *J* = 12.0 Hz, 1H), 7.18-7.14 (m, 2H), 3.94 (q, *J* = 7.2 Hz, 2H), 3.64 (s, 3H), 3.12 (s, 3H), 3.07 (s, 3H), 2.07-2.01 (m, 1H), 1.22 (t, *J* = 7.2 Hz, 3H), 1.03-0.98 (m, 2H), 0.86 - 0.77 (m, 2H).

**Example 189**

4-((4-cyclopropyl-2-(N-methyl cyclopropylsulfonamido)phenyl)amino)-6-(((2,6-dimethyl pyrimidin-4-yl)amino)-N-ethoxy nicotinamide

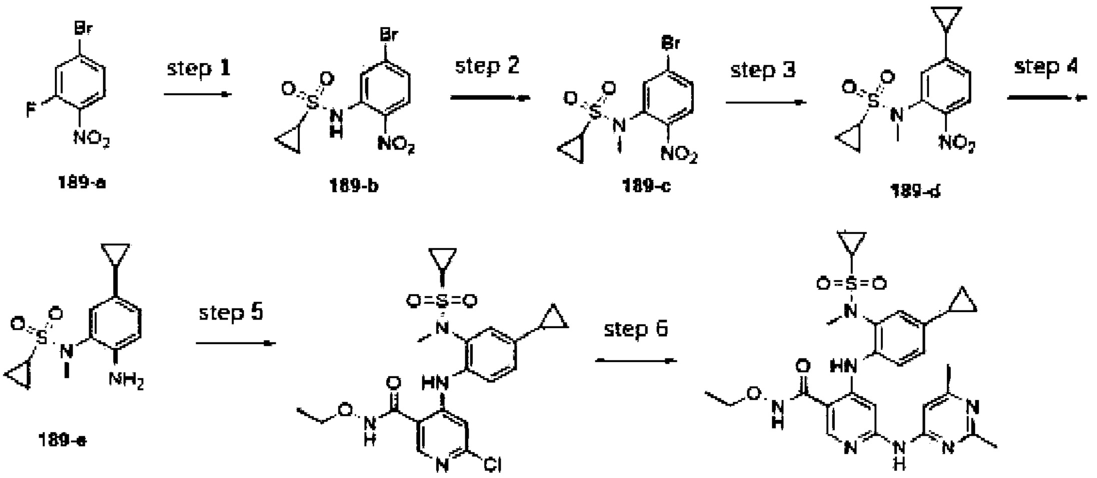


189-a 189-b 189-c 189-d 189-e 189-f 189

Step 1: To a 100ml flask were sequentially added 4-bromo-2-fluoro-1-nitrobenzene(3g, 13.6mmol, **189-a**), cyclopropylsulfonamide (2.2g, 16.3mmol), anhydrous potassium carbonate (2.8g, 20.4mmol) and DMF(40mL). The mixture was stirred at 90°C for 12h. When TLC indicated a completed reaction, the mixture was adjusted with saturated ammonium chloride aqueous solution to pH=7, and extracted with ethyl acetate three times. The combined organic phases were washed with saturated brine, dried over anhydrous sodium sulfate, and subjected to column chromatography to provide N-(5-bromo-2-nitrophenyl) cyclopropylsulfonamide (**189-b**, 3.2g, 10mmol, 71% yield). MS Calcd: 320; MS Found: 321($[M+H]^+$).

Step 2: To a 100ml flask were sequentially added N-(5-bromo-2-nitrophenyl) cyclopropylsulfonamide (**189-b,** 3.1g, 9.7mmol, 39124-b), iodomethane (2.06g, 14.5mmol), anhydrous potassium carbonate (4.0g, 29.1mmol) and DMF(40mL). The mixture was stirred at 50°C for 4h. When TLC indicated a completed reaction, the mixture was extracted with ethyl acetate three times. The combined organic phases were washed with saturated brine, dried over anhydrous

sodium sulfate, and subjected to column chromatography to provide N-(5-bromo-2-nitrophenyl)-N-methyl cyclopropylsulfonamide (**189-c,** 3.2g, 9.5mmol, 90% yield). MS Calcd: 334; MS Found: 335([M+H]$^+$).

Step 3: To a 100mL flask were added N-(5-bromo-2-nitrophenyl)-N-methyl cyclopropylsulfonamide (**189-c,** 1g, 3.0mmol) and cyclopropyl boronic acid(4.5mmol, 382mg), Pd(dppf)$Cl_2$(0.6mmol, 438mg), potassium phosphate (3.0g, 9mmol), followed by 20mL of dioxane and 3ml of water. The mixture was stirred under nitrogen protection and at 110°C for 8h, When TLC indicated a completed reaction, the mixture was extracted with ethyl acetate and water. The organic phase was washed with saturated sodium chloride aqueous solution, and dried over anhydrous sodium sulfate. After solvent removed by rotary evaporation, the residue was purified by chromatography to provide N-(5-cyclopropyl-2-nitrophenyl)-N-methyl cyclopropylsulfonamide (**189-d,** 870mg, 2.9mmol, 97% yield). MS Calcd: 296; MS Found: 297([M+H]$^+$).

Step 4: To a 100mL flask were added N-(5-cyclopropyl-2-nitrophenyl)-N-methyl cyclopropylsulfonamide (**189-d,** 870mg, 2.9mmol), Fe (14.6mmol, 822mg), ammonium chloride (29mmol, 1.5g), and 20mL of ethanol and 4ml of water. The mixture was stirred at 90°C for 3h. When TLC indicated a completed reaction, the mixture was cooled and added with 50ml of ethyl acetate, and then filtered to remove iron powder. The filtrate was extracted with ethyl acetate and water. The organic phase was washed with saturated sodium chloride aqueous solution, dried over anhydrous sodium sulfate, followed by solvent removed by rotary evaporation to provide N-(2-amino-5-cyclopropyl phenyl)-N-methyl cyclopropylsulfonamide (**189-e,** 750mg, 2.81mmol, 96% yield). MS Calcd: 266; MS Found: 267([M+H]$^+$).

Step 5: To a 100mL two-necked flask were sequentially added 4,6-dichloro-N-ethoxy nicotinamide (**int-2**, 500mg, 2.1mmol), N-(2-amino-5-cyclopropyl phenyl)-N-methylcyclopropanesulfonamide (**189-e,** 723mg, 2.55mmol), and 10ml of anhydrous tetrahydrofuran as solvent. The mixture was cooled in ice bath and added under nitrogen protection with a solution of LiHMDS in tetrahydrofuran (6.5mL, 1mmol/mL), with continuous stirring at room temperature for 6h. Upon indication of completed reaction by TLC, the mixture was adjusted with saturated ammonium chloride aqueous solution to pH=7, and extracted with ethyl acetate (30mLx3). The combined organic layers were dried, concentrated, and purified by column chromatography to provide 6-chloro-4-((4-cyclopropyl-2-(N-methyl cyclopropylsulfonamido)phenyl) amino)-N-ethoxy nicotinamide (**189-f,** 0.9g, 1.9mmol, 90% yield). MS Calcd: 464; MS Found: 465([M+H]$^+$).

Step 6: 6-chloro-4-((4-cyclopropyl-2-(N-methyl cyclopropylsulfonamido)phenyl)amino)-N-ethoxy nicotinamide (**189-f,** 150mg, 0.32mmol), 2,6-dimethyl pyrimidin-4-ylamine (47mg, 0.38mmol), cesium carbonate (312mg, 0.96mmol), Xant-Phos(60mg, 0.096mmol) and $Pd_2(dba)_3$(47mg, 0.064mmol) were added to anhydrous dioxane (8ml). After atmosphere replacement with nitrogen three times, the reaction mixture was heated to 120°C with stirring for 8h, and filtered by suction. The filtrate was concentrated and purified by high performance preparative thin layer chromatography (MeOH:DCM=1:15) to provide the title compound: 4-((4-cyclopropyl-2-(N-methyl cyclopropylsulfonamido)phenyl)amino)-6-(((2,6-dimethyl pyrimidin-4-yl)amino)-N-ethoxy nicotinamide (**189,** 100mg, 0.18mmol, 56% yield). MS Calcd: 551; MS Found: 552([M+H]$^+$). $^1$H NMR (400 MHz, DMSO-$d_6$)δ11.59 (s, 1H), 10.00 (s, 1H), 9.89 (s, 1H), 8.32 (s, 1H), 7.85 (s, 1H), 7.45 (d, *J* = 8.4 Hz, 1H), 7.34 (d, *J* = 2.0 Hz, 1H), 7.13 (dd, *J* = 8.4, 2.0 Hz, 1H), 7.03 (s, 1H), 3.93 (q, *J* = 7.2 Hz, 2H), 3.34 (s, 3H), 3.15 (s, 3H), 2.85 - 2.75 (m, 1H), 2.30 (s, 3H), 2.26 (s, 3H), 2.03 - 1.91 (m, 1H), 1.21 (t, *J* = 7.2 Hz, 3H), 1.04 - 0.95 (m, 4H), 0.89 - 0.72 (m, 2H), 0.72 - 0.65 (m, 2H).

Example **190**

4-((4-cyclopropyl-2-(N-methyl cyclopropylsulfonamido)phenyl)amino)-N-ethoxy-6-(pyrimidin-4-ylamino)nicotinamide

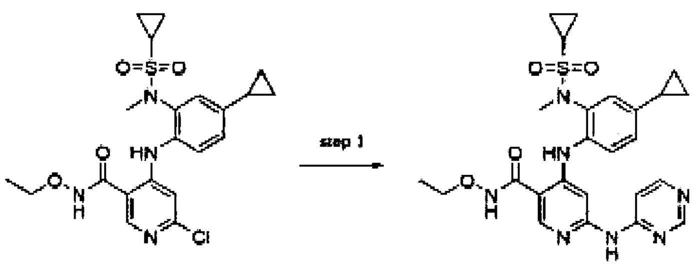


189-f 190

Step 1: 6-chloro-4-((4-cyclopropyl-2-(N-methyl cyclopropylsulfonamido)phenyl)amino)-N-ethoxy nicotinamide (**189-f,** 150mg, 0.32mmol), 4-aminopyrimidine (36mg, 0.38mmol), cesium carbonate (312mg, 0.96mmol), Xant-Phos(60mg, 0.096mmol) and $Pd_2(dba)_3$(47mg, 0.064mmol) were added to anhydrous dioxane (8ml). After atmosphere replacement with nitrogen three times, the reaction mixture was heated to 120°C with stirring for 8h, and filtered by suction. The filtrate was concentrated and purified by high performance preparative thin layer chromatography (MeOH:DCM=1:15) to provide the title compound: 4-((4-cyclopropyl-2-(N-methyl cyclopropylsulfonamido)phenyl)amino)-N-ethoxy-6-(pyrimidin-4-ylamino)nicotinamide (**190,** 140mg, 0.26mmol, 83% yield). MS Calcd: 522; MS Found: 523([M+H]$^+$). $^1$H NMR (400 MHz, DMSO-$d_6$)δ11.63 (s, 1H), 10.05 (s, 1H), 9.95 (s, 1H), 8.52 (d, *J* = 4.8 Hz, 2H), 8.33 (s, 1H), 8.14 (s, 1H), 7.54 (d, *J* = 8.4 Hz, 1H), 7.30 (d, *J* = 2.0 Hz, 1H), 7.20 (dd, *J* = 8.4, 2.0 Hz, 1H), 6.97 (t, *J* = 4.8 Hz, 1H), 3.93 (q, *J* = 7.2 Hz, 2H), 3.34 (s, 4H), 3.17 (s, 3H), 2.85-2.80 (m, 1H), 2.01 - 1.92 (m, 2H), 1.22 (t, *J* = 7.2 Hz, 3H), 1.03-0.96 (m, 4H), 0.88-0.80 (m, 2H), 0.75 - 0.71 (m, 2H).

Example **191**

6-((2,6-dimethyl pyrimidin-4-yl)amino)-N-ethoxy-4-((5-fluoro-4-methyl-2-(N-methyl methanesulfonamido)phenyl)amino)nicotinamide

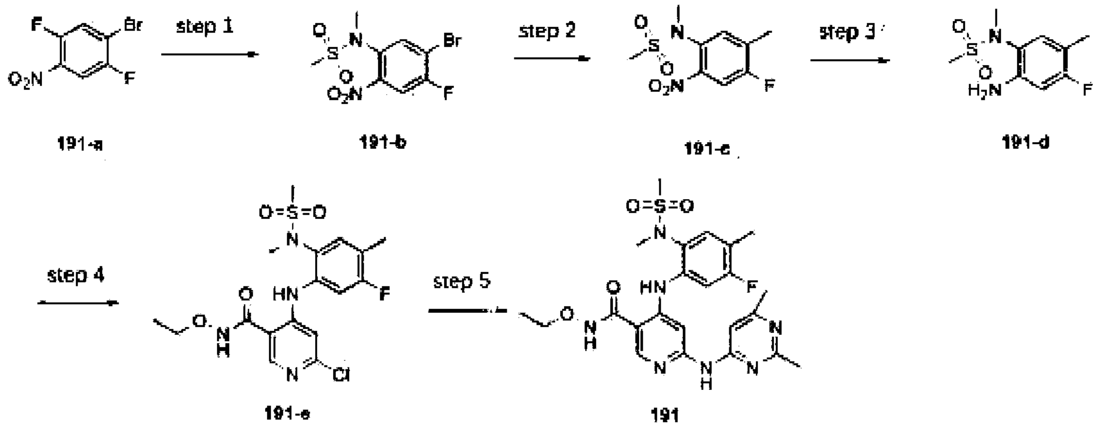


191-a 191-b 191-c 191-d 191-e 191

Step 1: To a 250mL flask were added 4-bromo-2,5-difluoro-nitrobenzene(**191-a**, 5g, 0.021mol), N-methyl methanesulfonamide (3.21g, 0.029mol), anhydrous potassium carbonate (5.79g, 0.42mol), followed by 100ml of anhydrous acetonitrile. The mixture was stirred at 90°C for 6 hours. When TLC indicated a completed reaction, the reaction mixture was mixed with 200-300 mesh silica gel for loading, concentrated under reduced pressure at 40°C, separated and purified by column chromatography, eluted with petroleum ether:ethyl acetate=10:1 gradually changing to petroleum ether:ethyl acetate=2:1, to provide N-(5-bromo-4-fluoro-2-nitrophenyl)-N-methyl methanesulfonamide (**191-b,** 6.3g, 0.019mol, 92.64% yield).

Step 2: To a 100mL flask were added N-(5-bromo-4-fluoro-2-nitrophenyl)-N-methyl methanesulfonamide (**191-b,** 1g, 3.077mmol), methyl boronic acid(221.02mg, 3.692mmol), $K_3PO_4$(1.95g, 9.231mmol), Pd(dppf)$_2$Cl$_2$(0.224g, 0.307mmol), followed by 1,4-dioxane (30ml) and water (5ml). After atmosphere replacement with nitrogen three times, the mixture was allowed to react at 110°C for 4 hours. When TLC indicated a completed reaction, the mixture was filtered by suction, and the filtrate was concentrated under reduced pressure at 40°C, separated and purified by column chromatography, eluted with petroleum ether:ethyl acetate=3:1 to provide N-(4-fluoro-5-methyl-2-nitrophenyl)-N-methyl methanesulfonamide (**191-c,** 618mg, 3.816mmol, 76.67% yield).
MS Calcd: 262

Step 3: To a 50ml flask were added N-(4-fluoro-5-methyl-2-nitrophenyl)-N-methyl methanesulfonamide (**191-c,** 618mg, 2.358mmol), reduction iron powder (553.56mg, 9.885mmol), ammonium chloride (1.05g, 19.77mmol), followed by 20mL of ethanol and 5ml of water. The mixture was stirred at 80°C for 6 hours. When TLC indicated a completed reaction, the mixture was filtered by suction, and the filtrate was added with water and extracted with ethyl acetate to provide N-(2-amino-4-fluoro-5-methyl phenyl)-N-methyl methanesulfonamide (**191-d,** 492mg, 2.120mmol, 90.07% yield). MS Calcd: 232; MS Found:233([M+H]$^+$).

Step 4: To a 50mL two-necked flask were added 4,6-dichloro-N-ethoxy nicotinamide (**int-2,** 166mg, 0.711mmol) and N-(2-amino-4-fluoro-5-methyl phenyl)-N-methyl methanesulfonamide (**191-d,** 150mg, 0.646mmol), followed by 3ml of anhydrous DMA. The reaction was vacuumed and refilled with $N_2$ three times, cooled in ice bath and added with LHMDS(1.9mL, 1mol/L, 1.939mmol) dropwise. After the addition, the mixture was warmed to 60°C to react for 6 hours. The reaction mixture was sampled for TLC several times to determine reaction termination. The reaction was cooled in ice bath, quenched with water and saturated ammonium chloride solution, and extracted with ethyl acetate. The organic phase was dried over anhydrous sodium sulfate, followed by adding 200-300 mesh silica gel mixed with silica gel for loading onto and purifying by column chromatography, eluted with petroleum ether:ethyl acetate=1:1, to provide 6-chloro-N-ethoxy-4-((4-methyl-5-fluoro-2-(N-methyl methanesulfonamido)phenyl)amino)nicotinamide (**191-e,** 109mg, 0.253mmol, 39.20%

yield). MS Calcd: 430; MS Found: 431([M+H]$^+$).

Step 5: 6-chloro-N-ethoxy-4-((4-methyl-5-fluoro-2-(N-methyl methanesulfonamido)phenyl)amino)nicotinamide (**191-e,** 80mg, 0.186mmol), 4-amino-2,6-dimethyl pyrimidine (29.77mg, 0.242mmol), $Pd_2(dba)_3$(31mg, 0.034mmol), xantphos(38.73mg, 0.067mmol), $Cs_2CO_3$ (181.79mg, 0.558mmol), and 1,4-dioxane (2mL) were combined. After atmosphere replacement with nitrogen three times, the mixture was allowed to react at 120°C for 6 hours. When TLC indicated a completed reaction, the mixture was filtered by suction, and the filtrate was concentrated under reduced pressure at 40°C, mixed with 200-300 mesh silica gel for loading onto and purifying by column chromatography, eluted with DCM:MeOH=30:1, to provide the title compound: 6-((2,6-dimethyl pyrimidin-4-yl)amino)-N-ethoxy-4-((5-fluoro-4-methyl-2-(N-methyl methanesulfonamido)phenyl)amino)nicotinamide (**191**, 29mg, 0.056mmol, 30.20% yield). MS Calcd:517; MS Found:518([M+H]$^+$). $^1$H NMR (400 MHz, DMSO-$d_6$)δ11.84 (s, 1H), 11.39 (s, 1H), 10.15 (s, 1H), 8.45 (s, 1H), 7.57 (d, $J$ = 7.8 Hz, 1H), 7.39 (d, $J$ = 10.8 Hz, 1H), 3.96 (q, $J$ = 7.2 Hz, 2H), 3.14 (s, 3H), 3.12 (s, 3H), 2.56 (s, 3H), 2.50 (s, 3H, overlapped with DMSO-$d_6$), 2.27 (d, $J$ = 1.6 Hz, 3H), 1.23 (t, $J$ = 7.2 Hz, 3H).

Example **192**

4-((4-cyclopropyl-2-(N-methyl methanesulfonamido)phenyl)amino)-N-ethoxy-6-((2-methyl pyrimidin-4-yl)amino)nicotinamide

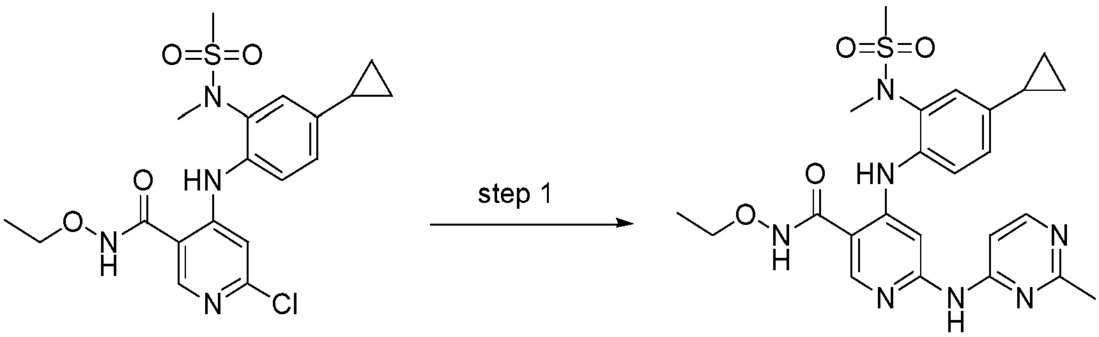


**20-a** **192**

Step 1: 6-chloro-4-((4-cyclopropyl-2-(N-methyl methanesulfonamido)phenyl)amino)-N-ethoxy nicotinamide (**20-a,** 200mg, 0.46mmol), 2-methyl pyrimidin-4-ylamine (99.5mg, 0.91mmol), 4,5-bis-diphenylphosphino-9,9-dimethyl xanthene(105.7mg, 0.18mmol), cesium carbonate (446mg, 1.37mmol), and $Pd_2(dba)_3$(125mg, 0.137mmol) were added to 1,4-dioxane (5mL). After atmosphere replacement with nitrogen three times, the mixture was heated to 120°C for 4h. When TLC indicated a completed reaction, the reaction mixture was cooled to room temperature, evaporated directly in the presence of silica gel for loading onto and purifying by column chromatography (dichloromethane:methanol=50:1~25:1, Rf=0.4) to provide the title compound: 4-((4-cyclopropyl-2-(N-methyl methanesulfonamido)phenyl)amino)-N-ethoxy-6-((2-methyl pyrimidin-4-yl)amino)nicotinamide (**192,** 150mg, 0.29mmol, 63% yield). MS Calcd: 511; MS Found: 512([M+H]$^+$). $^1$H NMR (400 MHz, DMSO-$d_6$)δ11.63 (s, 1H), 10.10 (s, 1H), 9.85 (s, 1H), 8.33 (s, 1H), 8.26 (d, $J$ = 6.0 Hz, 1H), 7.82 (s, 1H), 7.45 (d, $J$ = 8.4 Hz, 1H), 7.32 (d, $J$ = 2.0 Hz, 1H), 7.18 (d, $J$ = 6.0 Hz, 1H), 7.12 (dd, $J$ = 8.4, 2.0 Hz, 1H), 3.94 (q, $J$ = 7.2 Hz, 2H), 3.13 (s, 3H), 3.09 (s, 3H), 2.33 (s, 3H), 2.01-1.95 (m, 1H), 1.22 (t, $J$ = 7.2 Hz, 3H), 1.03 - 0.94 (m, 2H), 0.75 - 0.67 (m, 2H).

Example **193**

6-((2,6-dimethyl pyrimidin-4-yl)amino)-N-ethoxy-4-((4-ethyl-5-fluoro-2-(N-methyl methanesulfonamido)phenyl)amino) nicotinamide

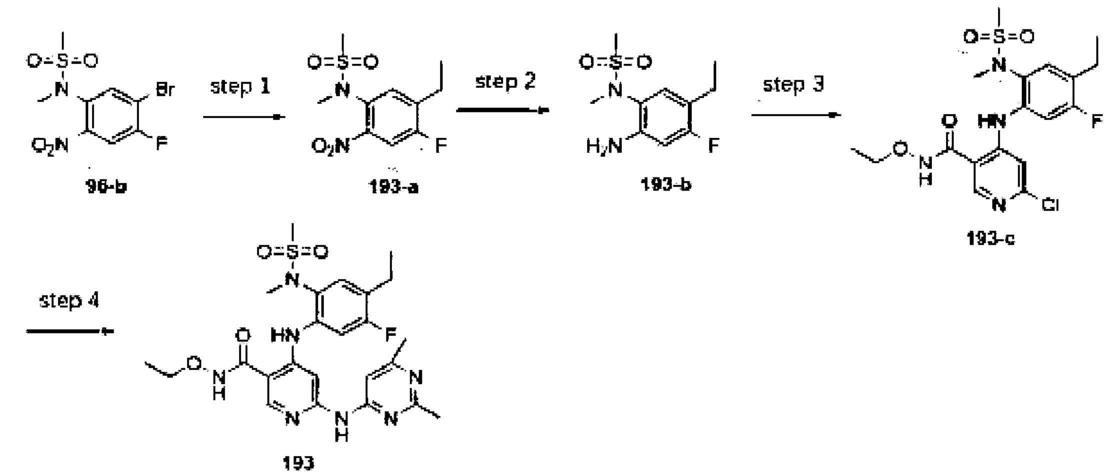

96-b 193-a 193-b 193-c 193

Step 1: To a 50ml flask were added N-(5-bromo-4-fluoro-2-nitrophenyl)-N-methyl methanesulfonamide (**96-b,** 500mg, 1.5mmol), ethyl boronic acid (137mg, 1.8mmol), cesium carbonate (1.5g, 4.6mmol), Pd(dppf)$Cl_2$(169mg, 0.21mmol), 1,4-dioxane (10ml), and water (2ml). After atmosphere replacement with nitrogen three times, the mixture was heated to 100°C under nitrogen protection for 5h. Upon indication of completed reaction by TLC (petroleum ether/ethyl acetate=2/1), the reaction mixture was cooled to room temperature, quenched with water (150ml), and extracted with ethyl acetate (150ml) three times. The organic phases were combined, dried over anhydrous sodium sulfate, concentrated, and purified by column chromatography, eluted with petroleum ether/ethyl acetate=5/1~3/1 to provide N-(5-ethyl-4-fluoro-2-nitrophenyl)-N-methyl methanesulfonamide (**193-a,** 180mg, 0.65mmol, 43% yield).

Step 2: To a 50ml flask were added N-(5-ethyl-4-fluoro-2-nitrophenyl)-N-methyl methanesulfonamide (**193-a**, 180mg, 0.65mmol), iron powder (183mg, 3.26mmol), ammonium chloride (349mg, 6.52mmol), ethanol (10ml), and water (1ml).After atmosphere replacement with nitrogen three times, the mixture was allowed to react under nitrogen protection at 90°C for 3h. Upon indication of completed reaction by TLC (petroleum ether/ethyl acetate=1/1), the system was filtered, and the filter cake was washed with methanol three times. The organic phases were combined, dried over anhydrous sodium sulfate, and concentrated. The residue was dissolved in ethyl acetate (100ml) and washed with water (100ml) twice. The organic phase was dried over anhydrous sodium sulfate, and concentrated to obtain N-(2-amino-5-ethyl-4-fluorophenyl)-N-methyl methanesulfonamide (**193-b,** 160mg, 0.65mmol, 99.9% yield). MS Calcd: 246; MS Found: 247($[M+H]^+$).

Step 3: To a 50ml flask were added N-(2-amino-5-ethyl-4-fluorophenyl)-N-methyl methanesulfonamide (**193-b,** 160mg, 0.65mmol), 4,6-dichloro-N-ethoxy nicotinamide (**int-2,** 167mg, 0.72mmol), and tetrahydrofuran (15ml). After atmosphere replacement with nitrogen three times, the mixture was slowly added with LiHMDS (1.6ml, 1.6mmol), and allowed to react at RT for 5h. Upon indication of completed reaction by TLC (petroleum ether/ethyl acetate=1/2), the system was washed with water (100ml), adjusted with 0.1mol/L HCl to PH=5, and extracted with ethyl acetate (100ml) three times. The organic phases were combined, dried over anhydrous sodium sulfate, concentrated, mixed with silica gel for loading and separated and purified by column chromatography, eluted with petroleum ether/ethyl acetate=3/1~1/1, to provide N-ethoxy-4-((4-ethyl-5-fluoro-2-(N-methyl methanesulfonamido)phenyl)amino)-6-methyl nicotinamide (**193-c,** 400mg, 0.94mmol, 50% yield). MS Calcd:424; MS Found: 425 ($[M+H]^+$).

Step 4: To a 50ml flask were added 6-chloro-N-ethoxy-4-((4-ethyl-2-(N-methyl methanesulfonamido)phenyl) amino)nicotinamide (**193-c,** 150mg, 0.35mmol), 2,6-dimethyl pyrimidin-4-ylamine (86.6mg, 0.7mmol), 4,5-bis-diphenylphosphino-9,9-dimethyl xanthene(81.5mg, 0.14mmol), cesium carbonate (344mg, 1.1mmol), Pd$(dba)_2$ (97mg, 0.11mmol), and 1,4-dioxane (5ml). After atmosphere replacement with nitrogen four times, the mixture was heated under nitrogen protection to 120°C for 4h. Upon indication of completed reaction by TLC (DCM/MeOH=20/1), the system was directly mixed with silica gel for loading onto and purifying by column chromatography DCM/MeOH=50/1~25/1 to provide the title compound: 6-((2,6-dimethyl pyrimidin-4-yl)amino)-N-ethoxy-4-((4-ethyl-5-fluoro-2-(N-methyl methanesulfonamido)phenyl)amino)nicotinamide (**193,** 60mg, 0.12mmol, 34% yield). MS Calcd: 531; MS Found: 532($[M+H]^+$). $^1H$ NMR (400 MHz, DMSO-$d_6$)δ11.67 (s, 1H), 10.13 (s, 1H), 10.10 (s, 1H), 8.36 (s, 1H), 7.98 (s, 1H), 7.52 (d, *J* = 8.0 Hz, 1H), 7.40 (d, *J* = 11.6 Hz, 1H), 7.13 (s, 1H), 3.94 (q, *J* = 7.2 Hz, 2H), 3.14 (s, 3H), 3.13 (s, 3H), 2.64 (q, *J* = 7.6 Hz, 2H), 2.36 (s, 3H), 2.28 (s, 3H), 1.22 (t, *J* = 7.2 Hz, 3H), 0.87 - 0.78 (m, 3H).

Example **194**

4-((4-cyclopropyl-5-fluoro-2-(N-methyl methanesulfonamido)phenyl)amino)-N-ethoxy-6-((2-methyl pyrimidin-4-yl)amino)nicotinamide

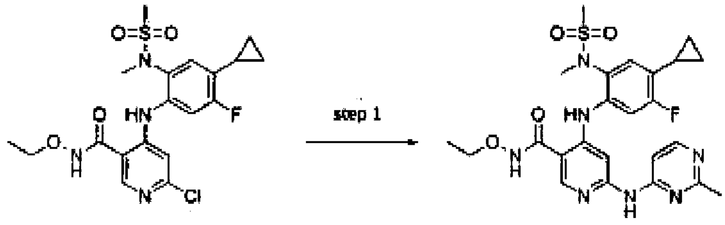


96-e

194

Step 1: 6-chloro-4-((4-cyclopropyl-5-fluoro-2-(N-methyl methanesulfonamido)phenyl)amino)-N-ethoxy nicotinamide (**96-e,** 278mg, 0.5mmol), 4-amino-2-methyl pyrimidine (55mg, 0.5mmol), cesium carbonate (326mg, 1.0mmol), Xant-Phos (57.6mg, 0.1mmol) and $Pd_2(dba)_3$(45.5mg, 0.05mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and subjected to high performance thin layer preparative chromatography to provide the title compound: 4-((4-cyclopropyl-5-fluoro-2-(N-methyl methanesulfonamido) phenyl)amino)-N-ethoxy-6-((2-methyl pyrimidin-4-yl)amino)nicotinamide (**194,** 20mg, 0.038mmol, 7.6% yield). MS Calcd: 529; MS Found: 530 ($[M+H]^+$). $^1H$ NMR (400 MHz, DMSO-$d_6$)δ11.67 (s, 1H), 10.19 (s, 1H), 10.10 (s, 1H), 8.35 (s, 1H), 8.30 (d, *J* = 6.0 Hz, 1H), 7.92 (s, 1H), 7.39 (d, *J* = 11.6 Hz, 1H), 7.29 (d, *J* = 6.0 Hz, 1H), 7.19 (d, *J* = 8.0 Hz, 1H), 3.94 (q, *J* = 7.2 Hz, 2H), 3.13 (s, 3H), 3.09 (s, 3H), 2.40 (s, 3H), 2.06-1.99 (m, 1H), 1.22 (t, *J* = 7.2 Hz, 3H), 1.02-0.97 (m, 2H), 0.81-0.77 (m, 2H).

Example **195**

4-((4-cyclopropyl-5-fluoro-2-(N-methyl methanesulfonamido)phenyl)amino)-6-(3,3-difluoroazetidin-1-ylcarboxamido)-N-ethoxy nicotinamide

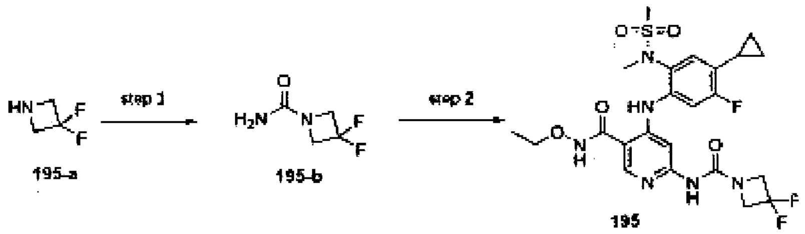


195-a 195-b 195

Step 1: Into 3ml of DMF were added 3,3-difluoroazetidine(**195-a**, 130mg, 1mmol), phenyl carbamate (137mg, 1mmol), potassium carbonate (276mg, 2mmol). The mixture was heated to 60°C with stirring for 4 hours, added with 20ml of water, and extracted with ethyl acetate. The organic phase was dried over anhydrous sodium sulfate, concentrated, purified by column chromatography to provide 3,3-difluoroazetidin-1-ylcarboxamide (**195-b,** 80mg, 0.59mmol, 59% yield).

Step 2: 6-chloro-4-((4-cyclopropyl-5-fluoro-2-(N-methyl methanesulfonamido)phenyl)amino)-N-ethoxy nicotinamide (**96-e,** 278mg, 0.5mmol), 3,3-difluoroazetidine-1-carboxamide (**195-b,** 80mg, 0.59mmol), cesium carbonate (326mg, 1.0mmol), Xant-Phos(57.6mg, 0.1mmol) and $Pd_2(dba)_3$(45.5mg, 0.05mmol) were added to anhydrous dioxane (2ml). After the atmosphere of the mixture was evacuated to vacuum and refilled with nitrogen, the reaction mixture was heated to 120°C with stirring for 2 hours, and filtered by suction. The filtrate was concentrated and subjected to high performance thin layer preparative chromatography to provide the title compound: 4-((4-cyclopropyl-5-fluoro-2-(N-methyl methanesulfonamido)phenyl)amino)-6-(3,3-difluoroazetidin-1-ylcarboxamido)-N-ethoxy nicotinamide (**195,** 10mg, 0.018mmol, 3.6% yield). MS Calcd: 556; MS Found: 557 ($[M+H]^+$).

Example **196**

4-((5-chloro-2-(N-methyl methanesulfonamido)phenyl)amino)-6-((2,6-dimethyl pyrimidin-4-yl)amino)-N-ethoxy nicotinamide

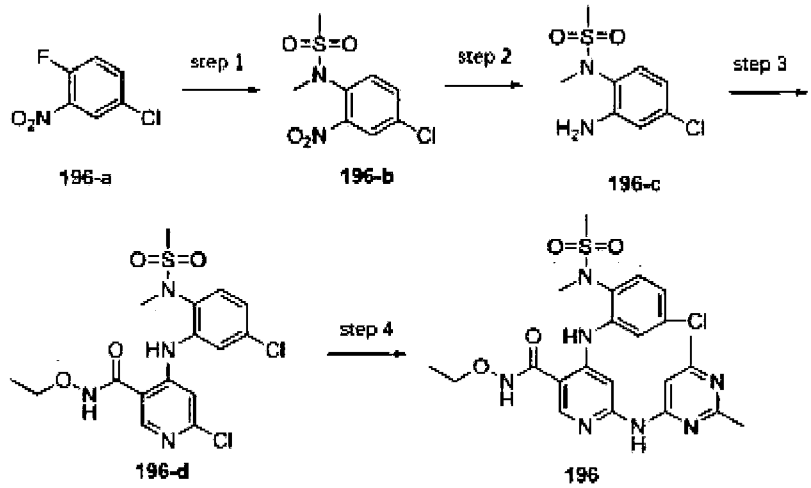


196-a 196-b 196-c 196-d 196

Step 1: To a 50ml reaction flask was added 4-chloro-1-fluoro-2-nitrobenzene(**196-a,** 525mg, 3mmol) dissolved in N,N-dimethyl formamide (10ml), followed by potassium carbonate (621mg, 4.5mmol), and N-methyl methanesulfonamide (360mg, 3.3mmol). The mixture was heated to 90°C with stirring for 3h. Upon indication of completed reaction by TLC, the reaction mixture was washed respectively with water (30 ml x 1) and brine (30 ml x 1), dried over anhydrous sodium sulfate, and concentrated to provide a crude material, which was purified by silica gel column chromatography (petroleum ether/ethyl acetate=3:1) to provide the product N-(4-chloro-2-nitrophenyl)-N-methyl methanesulfonamide (**196-b,** 750mg, 2.84mmol, 94.6% yield)MS Calcd:264.03; MS Found: 265.05 ($[M+H]^+$).

Step 2: N-(4-chloro-2-nitrophenyl)-N-methyl methanesulfonamide (**196-b,** 750mg, 2.84mmol), ammonium chloride (1.52g, 28.4 mmol) and iron powder (795 mg, 14.2mmol) were sequentially added to 10 mL of mixed solvent of water and ethanol (1:4). The mixture was stirred under reflux for 6 hours. Upon indication of completed reaction by TLC, the reaction mixture was filtered by suction. The filtrate was concentrated, and the residue was separated and purified by silica gel column chromatography to provide N-(2-amino-4-chloro phenyl)-N-methyl methanesulfonamide (**196-c,** 600 mg, 2.56 mmol, 90% yield). MS Calcd: 234.09; MS Found: 235.22 ($[M+H]^+$).

Step 3: N-(2-amino-4-chloro phenyl)-N-methyl methanesulfonamide (**196-c,** 117 mg, 0.5mmol), 4,6-dichloro-N-ethoxy nicotinamide (117 mg, 0.5 mmol) were added to 5 ml of anhydrous N,N-dimethylacetamide. The mixture was added at room temperature with a solution of LiHMDS in tetrahydrofuran (1.5ml, 1.5mmol), and stirred at room temperature for 3 hours. Upon indication of completed reaction by TLC, the mixture was adjusted with aqueous hydrochloride (1N) to pH 5, and extracted with ethyl acetate (20 ml x 3). The combined organic layers were dried, concentrated and then purified by column chromatography (petroleum ether:ethyl acetate =1:1) to provide 6-chloro-4-((5-chloro-2-((N-methyl methanesulfonamido)phenyl)amino)-N-ethoxy nicotinamide (**196-d,** 186mg, 0.43 mmol, 86% yield). MS Calcd: 432.10; MS Found: 433.29 ($[M+H]^+$).

Step 4: 6-chloro-4-((5-chloro-2-((N-methyl methanesulfonamido)phenyl)amino)-N-ethoxy nicotinamide (**196-d,** 186mg, 0.43 mmol, ), 2,6-dimethyl pyrimidin-4-ylamine (58.2 mg, 0.47mmol), cesium carbonate (419 mg, 1.29 mmol), Xant-Phos(46.2mg, 0.08mmol) and $Pd_2(dba)_3$(36.5mg, 0.04 mmol) were added to anhydrous dioxane (5 ml). After the atmosphere of the mixture was evacuated to vacuum and refilled with nitrogen, the reaction mixture was heated to 120°C with stirring for 6 hours, and filtered by suction. The filtrate was concentrated and purified by preparative TLC (MeOH:DCM=1:20) to provide the title compound: 4-((5-chloro-2-(N-methyl methanesulfonamido)phenyl)amino)-6-((2,6-dimethyl pyrimidin-4-yl)amino)-N-ethoxy nicotinamide (**196,** 30mg, 0.057mmol, 13.4% yield). MS Calcd: 519.15; MS Found: 520.20 ($[M+H]^+$). $^1H$ NMR (400 MHz, DMSO-$d_6$)$\delta$11.69 (s, 1H), 10.17 (s, 2H), 8.38 (s, 1H), 7.90 (s, 1H), 7.64 - 7.56 (m, 2H), 7.27-7.24 (m, 2H), 3.94 (q, *J*= 7.2 Hz, 2H), 3.15 (s, 3H), 3.15 (s, 3H), 2.37 (s, 3H), 2.29 (s, 3H), 1.22 (t, *J*= 7.2 Hz, 3H).

Example **197**

6-(((2,6-dimethyl pyrimidin-4-yl)amino)-N-ethoxy-4-((3-fluoro-4-methyl-2-(N-methyl methanesulfonamido)phenyl)amino)nicotinamide

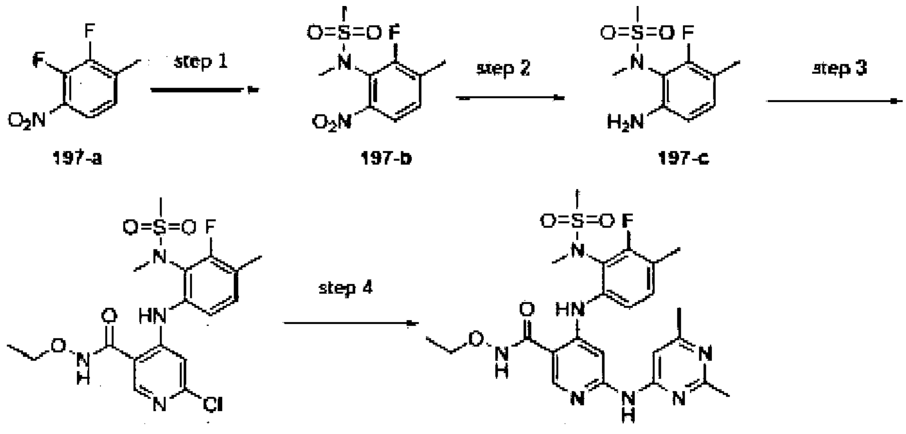


197-a 197-b 197-c

197-d 197

Step 1: To a 50ml reaction flask was added 2,3-difluoro-1-methyl-4-nitrobenzene (**197-a**, 173mg, 1mmol) dissolved in N,N-dimethyl formamide (5ml), followed by potassium carbonate (276mg, 2mmol), and N-methyl methanesulfonamide (120mg, 1.1mmol). The mixture was heated to 90°C with stirring for 3h. Upon indication of completed reaction by TLC, the reaction mixture was washed respectively with water (30 ml x 1) and brine (30 ml x 1), dried over anhydrous sodium sulfate, and concentrated to provide a crude material, which was purified by silica gel column chromatography (petroleum ether/ethyl acetate=3:1) to provide the product N-(2-fluoro-3-methyl-6-nitrophenyl)-N-methyl methanesulfonamide (**197-b,** 220mg, 0.84mmol, 83.9% yield). MS Calcd:262.03; MS Found: 263.05 ($[M+H]^+$).

Step 2: N-(2-fluoro-3-methyl-6-nitrophenyl)-N-methyl methanesulfonamide (**197-b,** 220mg, 0.84mmol), ammonium chloride (445mg, 8.4 mmol) and iron powder (235 mg, 4.2mmol) were sequentially added to 10 mL of mixed solvent of water and ethanol (1:4). The mixture was stirred under reflux for 6 hours. Upon indication of completed reaction by TLC, the reaction mixture was filtered by suction. The filtrate was concentrated, and the residue was separated and purified by silica gel column chromatography to provide N-(6-amino-2-fluoro-3-methyl phenyl)-N-methyl methanesulfonamide (**197-c,** 170 mg, 0.73 mmol, 87.2% yield). MS Calcd: 232.09; MS Found: 233.22 ($[M+H]^+$).

Step 3: N-(6-amino-2-fluoro-3-methyl phenyl)-N-methyl methanesulfonamide (**197-c,** 116 mg, 0.5mmol), and 4,6-dichloro-N-ethoxy nicotinamide (117 mg, 0.5 mmol) were added to 5 ml of anhydrous N,N-dimethylacetamide. The mixture was added at room temperature with a solution of LiHMDS in tetrahydrofuran (1.5ml, 1.5mmol), and stirred at room temperature for 3 hours. Upon indication of completed reaction by TLC, the mixture was adjusted with aqueous hydrochloride (1N) to pH 5, and extracted with ethyl acetate (20 ml x 3). The combined organic layers were dried, concentrated and then purified by column chromatography (petroleum ether:ethyl acetate =1:1) to provide 6-chloro-N-ethoxy-4-((3-fluoro-4-methyl-2-(N-methyl methanesulfonamido)phenyl)amino)nicotinamide (**197-d,** 190mg, 0.43 mmol, 88.3% yield). MS Calcd: 430.10; MS Found: 431.29 ($[M+H]^+$).

Step 4: 6-chloro-N-ethoxy-4-((3-fluoro-4-methyl-2-(N-methyl methanesulfonamido)phenyl)amino)nicotinamide (**197-d,** 190mg, 0.44 mmol, ), 2,6-dimethyl pyrimidin-4-ylamine (59.8 mg, 0.49mmol), cesium carbonate (429 mg, 1.30 mmol), Xant-Phos(46.2mg, 0.08mmol) and $Pd_2(dba)_3$(36.5mg, 0.04 mmol) were added to anhydrous dioxane (5 ml). After the atmosphere of the mixture was evacuated to vacuum and refilled with nitrogen, the reaction mixture was heated to 120°C with stirring for 6 hours, and filtered by suction. The filtrate was concentrated and purified by preparative TLC (MeOH:DCM=1:20) to provide the title compound: 6-(((2,6-dimethyl pyrimidin-4-yl)amino)-N-ethoxy-4-((3-fluoro-4-methyl-2-(N-methyl methanesulfonamido)phenyl)amino)nicotinamide (**197,** 35mg, 0.068mmol, 15.3% yield). MS Calcd: 517.21; MS Found: 518.20 ($[M+H]^+$). $^1$H NMR (400 MHz, DMSO-$d_6$)$\delta$11.68 (s, 1H), 10.08 (s, 1H), 10.04 (s, 1H), 8.37 (s, 1H), 7.92 (s, 1H), 7.39-7.35 (m, 2H), 7.07 (s, 1H), 3.94 (q, $J$ = 7.2 Hz, 2H), 3.15 (s, 3H), 3.13 (s, 3H), 2.34 (s, 3H), 2.27 (d, $J$= 2.4 Hz, 6H), 1.22 (t, $J$ = 7.2 Hz, 3H).

Example **198**

4-((4-chloro-5-fluoro-2-(N-methyl methanesulfonamido)phenyl)amino)-6-((2,6-dimethyl pyrimidin-4-yl)amino)-N-ethoxy nicotinamide

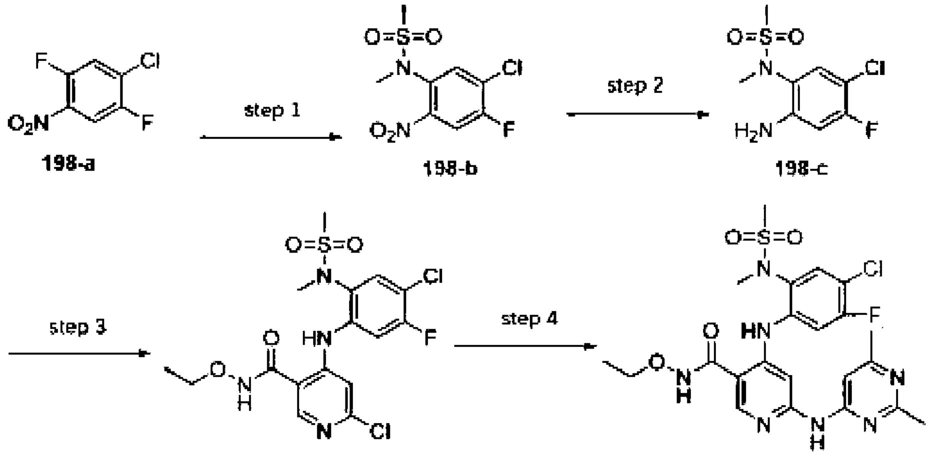


198-a 198-b 198-c 198-d 198

Step 1: To a 50ml reaction flask was added 1-chloro-2,5-difluoro-4-nitrobenzene(**198-a,** 1g, 5.16mmol) dissolved in N,N-dimethyl formamide (15ml), followed by potassium carbonate (1.07g, 7.74mmol), and N-methyl methanesulfonamide (676mg, 6.2mmol). The mixture was heated to 90°C with stirring for 3h. Upon indication of completed reaction by TLC, the reaction mixture was washed respectively with water (30 ml x 1) and brine (30 ml x 1), dried over anhydrous sodium sulfate, and concentrated to provide a crude material, which was purified by silica gel column chromatography (petroleum ether/ethyl acetate=3: 1) to provide the product N-(5-chloro-4-fluoro-2-nitrophenyl)-N-methyl methanesulfonamide (**198-b,** 820mg, 2.91mmol, 56.3% yield). MS Calcd:282.03; MS Found: 283.05 ($[M+H]^+$).

Step 2: N-(5-chloro-4-fluoro-2-nitrophenyl)-N-methyl methanesulfonamide (**198-b** 820mg, 2.9mmol), ammonium chloride (1.55g, 29 mmol) and iron powder (814 mg, 14.5mmol) were sequentially added to 10 mL of mixed solvent of water and ethanol (1:4). The mixture was stirred under reflux for 6 hours. Upon indication of completed reaction by TLC, the reaction mixture was filtered by suction. The filtrate was concentrated, and the residue was separated and purified by silica gel column chromatography to provide N-(2-amino-5-chloro-4-fluorophenyl)-N-methyl methanesulfonamide (**198-c,** 620 mg, 2.46 mmol, 84.8% yield). MS Calcd: 252.09; MS Found: 253.22 ($[M+H]^+$).

Step 3: N-(2-amino-5-chloro-4-fluorophenyl)-N-methyl methanesulfonamide (**198-c,** 504 mg, 2mmol), and 4,6-dichloro-N-ethoxy nicotinamide (**int-2,** 468 mg, 2 mmol) were added to 10 ml of anhydrous N,N-dimethylacetamide. The mixture was added at room temperature with a solution of LiHMDS in tetrahydrofuran (6ml, 6mmol), and stirred at room temperature for 3 hours. Upon indication of completed reaction by TLC, the mixture was adjusted with aqueous hydrochloride (1N) to pH 5, and extracted with ethyl acetate (20 ml x 3). The combined organic layers were dried, concentrated and then purified by column chromatography (petroleum ether:ethyl acetate =1:1) to provide 6-chloro-4-((4-chloro-5-fluoro-2-(N-methyl methanesulfonamido)phenyl)amino)-N-ethoxy nicotinamide (**198-d,** 620mg, 1.37 mmol, 69% yield). MS Calcd: 450.10; MS Found: 451.29 ($[M+H]^+$).

Step 4: 6-chloro-4-((4-chloro-5-fluoro-2-(N-methyl methanesulfonamido)phenyl)amino)-N-ethoxy nicotinamide (**198-d,** 135mg, 0.3 mmol, ), 2,6-dimethyl pyrimidin-4-ylamine (37.5 mg, 0.3mmol), cesium carbonate (292.5 mg, 0.9 mmol), Xant-Phos(34.68mg, 0.06mmol) and $Pd_2(dba)_3$(27.45mg, 0.03 mmol) were added to anhydrous dioxane (5 ml). After the atmosphere of the mixture was evacuated to vacuum and refilled with nitrogen, the reaction mixture was heated to 120°C with stirring for 6 hours, and filtered by suction. The filtrate was concentrated and purified by preparative TLC (MeOH:DCM=1:20) to provide the title compound: 4-((4-chloro-5-fluoro-2-(N-methyl methanesulfonamido)phenyl)amino)-6-((2,6-dimethyl pyrimidin-4-yl)amino)-N-ethoxy nicotinamide (**198,** 30mg, 0.056mmol, 18.6% yield). MS Calcd: 537.21; MS Found: 538.20 ($[M+H]^+$). $^1H$ NMR (400 MHz, DMSO-$d_6$)δ11.73 (s, 1H), 10.36 (s, 1H), 10.14 (s, 1H), 8.40 (s, 1H), 7.97 (s, 1H), 7.88 (d, *J* = 7.6 Hz, 1H), 7.68 (d, *J* = 11.20 Hz, 1H), 7.20 (s, 1H), 3.94 (q, *J* = 7.2 Hz, 2H), 3.18 (s, 3H), 3.17 (s, 3H), 2.38 (s, 3H), 2.29 (s, 3H), 1.22 (t, *J* = 7.2 Hz, 3H).

Example **199**

4-((4-chloro-5-fluoro-2-(N-methyl methanesulfonamido)phenyl)amino)-N-ethoxy-6-((2-methyl pyrimidin-4-yl)amino)nicotinamide

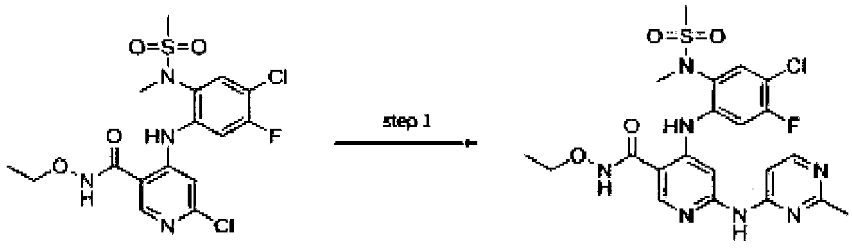


198-d

199

Step 1: 6-chloro-4-((4-chloro-5-fluoro-2-(N-methyl methanesulfonamido)phenyl)amino)-N-ethoxy nicotinamide (**198-d,** 135mg, 0.3 mmol, ), 2-methyl pyrimidin-4-ylamine (32.4mg, 0.3mmol), cesium carbonate (292.5 mg, 0.9 mmol), Xant-Phos(34.68mg, 0.06mmol) and $Pd_2(dba)_3$(27.45mg, 0.03 mmol) were added to anhydrous dioxane (5 ml). After the atmosphere of the mixture was evacuated to vacuum and refilled with nitrogen, the reaction mixture was heated to 120°C with stirring for 6 hours, and filtered by suction. The filtrate was concentrated and purified by preparative TLC (MeOH:DCM=1:20) to provide the title compound: 4-((4-chloro-5-fluoro-2-(N-methyl methanesulfonamido)phenyl)amino)-N-ethoxy-6-((2-methyl pyrimidin-4-yl)amino)nicotinamide (**199,** 30mg, 0.057mmol, 19.1% yield). MS Calcd: 523.21; MS Found: 524.20 ($[M+H]^+$). $^1$H NMR (400 MHz, DMSO-$d_6$)δ11.74 (s, 1H), 10.32 (s, 1H), 10.24 (s, 1H), 8.40 (s, 1H), 8.32 (d, $J$=6.0 Hz, 1H), 7.97 (s, 1H), 7.89 (d, $J$ = 7.6 Hz, 1H), 7.69 (d, $J$= 11.2 Hz, 1H), 7.35 (d, $J$ = 6.0 Hz, 1H), 3.95 (q, $J$ = 7.2 Hz, 2H), 3.18 (s, 3H), 3.17 (s, 3H), 2.41 (s, 3H), 1.22 (t, $J$ = 7.2 Hz, 3H).

Example **200**

4-((4-chloro-5-fluoro-2-(N-methyl methanesulfonamido)phenyl)amino)-N-ethoxy-6-((2-methoxy pyrimidin-4-yl)amino) nicotinamide

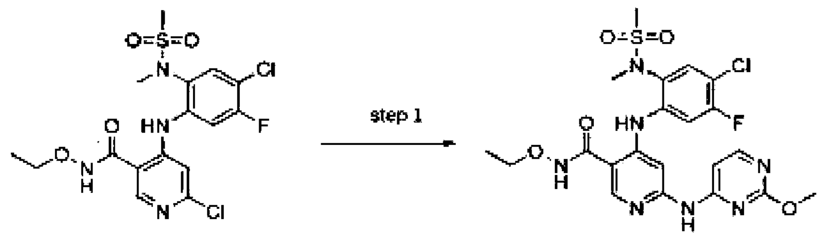


198-d

200

Step 1: 6-chloro-4-((4-chloro-5-fluoro-2-(N-methyl methanesulfonamido)phenyl)amino)-N-ethoxy nicotinamide (**198-d,** 135mg, 0.3 mmol, ), 2-methoxypyrimidin-4-ylamine (37.5mg, 0.3mmol), cesium carbonate (292.5 mg, 0.9 mmol), Xant-Phos(34.68mg, 0.06mmol) and $Pd_2(dba)_3$(27.45mg, 0.03 mmol) were added to anhydrous dioxane (5 ml). After the atmosphere of the mixture was evacuated to vacuum and refilled with nitrogen, the reaction mixture was heated to 120°C with stirring for 6 hours, and filtered by suction. The filtrate was concentrated and purified by preparative TLC (MeOH:DCM=1:20) to provide the title compound: 4-((4-chloro-5-fluoro-2-(N-methyl methanesulfonamido)phenyl)amino)-N-ethoxy-6-((2-methoxy pyrimidin-4-yl)amino)nicotinamide (**200,** 30mg, 0.056mmol, 18.5% yield). MS Calcd: 539.21; MS Found: 540.20 ($[M+H]^+$). $^1$H NMR (400 MHz, DMSO-$d_6$)δ11.75 (s, 1H), 10.31 (s, 1H), 10.23 (s, 1H), 8.39 (s, 1H), 8.22 (d, $J$ = 5.6 Hz, 1H), 7.94 - 7.83 (m, 2H), 7.67 (d, $J$ = 10.8 Hz, 1H), 7.18 (d, $J$ = 5.6 Hz, 1H), 3.94 (q, $J$= 7.2 Hz, 2H), 3.72 (s, 3H), 3.18 (s, 3H), 3.17 (s, 3H), 1.22 (t, $J$ = 7.2 Hz, 3H).

Example **201**

6-((2,6-dimethyl pyrimidin-4-yl)amino)-N-ethoxy-4-((4-isopropyl-2-(N-methyl methanesulfonamido)phenyl)amino)nicotinamide

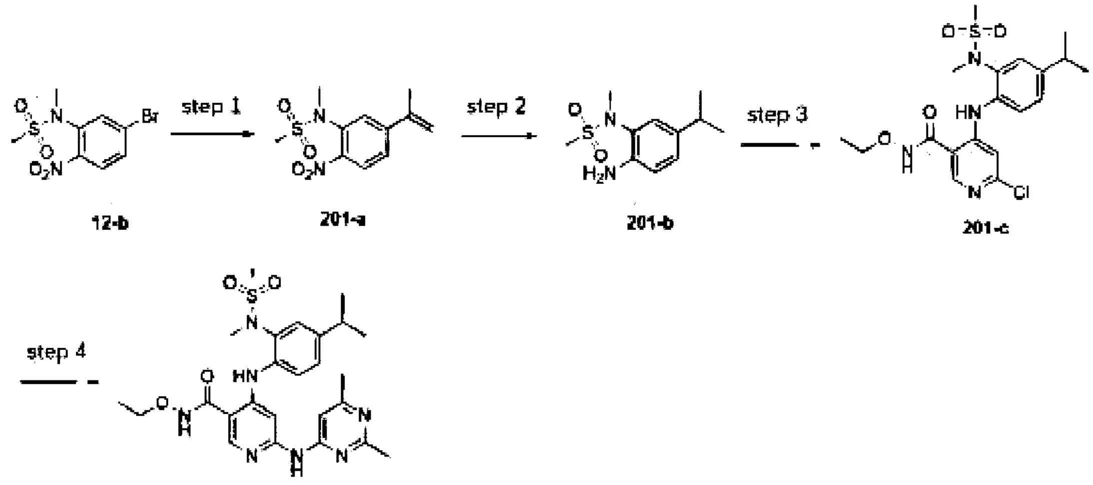

12-b 201-a 201-b 201-c
201

Step 1: To a 250mL flask were added N-(5-bromo-2-nitrophenyl)-N-methyl methanesulfonamide (**12-b,** 1g, 3.247mmol), isopropenyl borate (653.70mg, 3.89mmol), potassium phosphate (2.06g, 9.74mmol), Pd(dppf)$Cl_2$ (241.3mg, 0.33mmol), and 1,4-dioxane (100 mL) and water (20 mL). After atmosphere replacement with nitrogen three times, the mixture was heated to 110°C for 5 hours. When TLC indicated a completed reaction, the mixture was filtered by suction, added with water and extracted with ethyl acetate. The organic phase was concentrated under reduced pressure at 40°C, separated and purified by column chromatography, eluted with petroleum ether:ethyl acetate=1:1 to provide N-methyl-N-(2-nitro-5-(prop-1-en-2-yl)phenyl) methanesulfonamide (**201-a,** 864mg, 3.2112mmol, 98.63% yield).

Step 2: To a 50ml flask was added N-methyl-N-(2-nitro-5-(prop-1-en-2-y)phenyl) methanesulfonamide (**201-a,** 864mg, 3.212mmol), followed by 15mL of methanol and 172mg palladium on carbon. After atmosphere replacement with hydrogen three times, the mixture was allowed to react at room temperature overnight. When TLC indicated a completed reaction, the mixture was filtered by suction, and the filtrate was added with water, and extracted with ethyl acetate. The organic phase was dried over anhydrous sodium sulfate, concentrated under reduced pressure to provide N-(2-amino-5-isopropyl phenyl)-N-methyl methanesulfonamide (**201-b,** 621mg, 2.566mmol, 80.01% yield). MS Calcd:242; MS Found: 243($[M+H]^+$).

Step 3: To a 50mL two-necked flask were added 4,6-dichloro-N-ethoxy nicotinamide (**int-2,** 290mg, 1.239mmol) and N-(2-amino-5-isopropyl phenyl)-N-methyl methanesulfonamide (**201-b,** 250mg, 1.033mmol), followed by 3ml of anhydrous DMA. The reaction was vacuumed and refilled with $N_2$ three times, cooled in ice bath and added with LHMDS(3mL, 1mol/L, 3.099mmol) dropwise. After that the reaction was allowed to proceed at room temperature for 6 hours, and sampled. When TLC indicated a completed reaction, the reaction was cooled in ice bath, quenched with water and saturated ammonium chloride solution, and extracted with ethyl acetate. The organic phases were mixed with 200-300 mesh silica gel for loading onto and purifying by column chromatography, eluted with petroleum ether:ethyl acetate=1:1, to provide 6-chloro-N-ethoxy-4-((4-isopropyl-2-(N-methyl methanesulfonamido)phenyl)amino)nicotinamide (**201-c,** 181mg, 0.411mmol, 40.07% yield). MS Calcd: 440; MS Found: 441($[M+H]^+$).

Step 4: 6-chloro-N-ethoxy-4-((4-isopropyl-2-(N-methyl methanesulfonamido)phenyl)amino)nicotinamide (**201-c,** 100mg, 0.227mmol), 4-amino-2,6-dimethyl pyrimidine (36.37mg, 0.295mmol), $pd_2(dba)_3$ (41.17mg, 0.045mmol), XantPhos(52.60mg, 0.090mmol), $Cs_2CO_3$ (221.86mg, 0.681mmol), and 1,4-dioxane (2mL) were combined. After atmosphere replacement with nitrogen three times, the mixture was allowed to react at 120°C for 6 hours. When TLC indicated a completed reaction, the mixture was filtered by suction, and the filtrate was concentrated under reduced pressure at 40°C, mixed with 200-300 mesh silica gel for loading onto and purifying by column chromatography, eluted with DCM:MeOH=30:1, to provide the title compound: 6-((2,6-dimethyl pyrimidin-4-yl)amino)-N-ethoxy-4-((4-isopropyl-2-(N-methyl methanesulfonamido)phenyl)amino)nicotinamide (**201,** 17mg, 0.032mmol, 14.19% yield). MS Calcd:527; MS Found: 528($[M+H]^+$). $^1H$ NMR (401 MHz, DMSO-$d_6$)$\delta$11.60 (s, 1H), 10.01 (s, 1H), 9.90 (s, 1H), 8.33 (s, 1H), 8.13 (s, 1H), 7.91 (s, 1H), 7.51 (d, $J$ = 8.4 Hz, 1H), 7.44 (d, $J$ = 2.0 Hz, 1H), 7.33 (d, $J$ = 8.4 Hz, 1H), 7.02 (s, 1H), 3.94 (q, $J$ = 7.2 Hz, 2H), 3.15 (s, 3H), 3.09 (s, 3H), 2.97-2.92 (m, 1H), 2.31 (s, 3H), 2.26 (s, 3H), 1.25-1.20 (m, 9H).

Example **202**

6-((2,6-dimethyl pyrimidin-4-yl)amino)-N-ethoxy-4-((2-(N-methyl methanesulfonamido)-4-(trifluoromethyl)phenyl)amino)nicotinamide

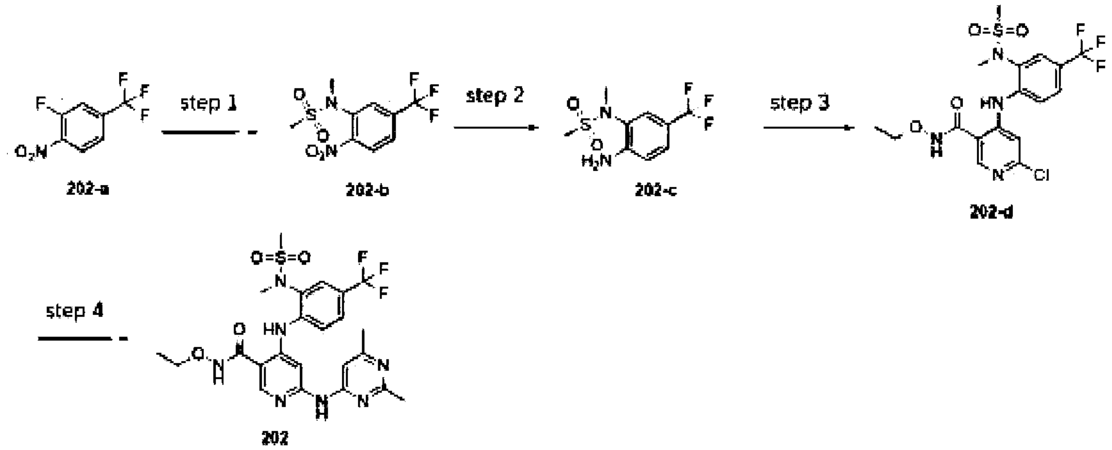


202-a 202-b 202-c 202-d 202

Step 1: To a 100mL flask were added 2-fluoro-1-nitro-4-(trifluoromethyl)benzene (**202-a,** 900mg, 4.30mmol), N-methyl methanesulfonamide (704mg, 6.45mmol), anhydrous potassium carbonate (2.07g, 15.05mmol), followed by about 30ml of anhydrous acetonitrile. The mixture was stirred at 90°C for 6 hours. When TLC indicated a completed reaction, the reaction mixture was mixed with 200-300 mesh silica gel for loading, concentrated under reduced pressure at 40°C, separated and purified by column chromatography, eluted with petroleum ether:ethyl acetate=10:1 gradually changing to petroleum ether:ethyl acetate=2:1, to provide N-methyl-N-(2-nitro-5-(trifluoromethyl)phenyl) methanesulfonamide(**202-b**, 1.25g, 4.19mmol, 87% yield).

Step 2: To a 100mL flask were added N-methyl-N-(2-nitro-5-(trifluoromethyl)phenyl) methanesulfonamide(**202-b**, 1.25g, 4.19mmol), reduction iron powder (1.1g, 20.9mmol), ammonium chloride (2.1g, 41.9mmmol), followed by ethanol (30mL) and water (6mL). The mixture was allowed to react at 80°C for 5 hours. When TLC indicated a completed reaction, the mixture was filtered by suction, added with water and extracted with ethyl acetate. The organic phase was dried over anhydrous sodium sulfate and concentrated under reduced pressure to provide N-(2-amino-5-(trifluoromethyl) phenyl)-N-methyl methanesulfonamide (**202-c**, 1.1g, 4.10mmol, 98% yield). MS Calcd: 268; MS Found: 269($[M+H]^+$).

Step 3: To a 50mL two-necked flask were added 4,6-dichloro-N-ethoxy nicotinamide (**int-2,** 458.40mg, 1.95mmol) and N-(2-amino-5-(trifluoromethyl)phenyl)-N-methyl methanesulfonamide (**202-c,** 350mg, 1.30mmol), followed by 4ml of anhydrous DMA. The reaction was vacuumed and refilled with $N_2$ three times, cooled in ice bath and added with LHMDS(4mL, 1mol/L, 3.917mmol) dropwise. After the addition, the mixture was brought to 60°C to react for 6 hours, and sampled. When TLC indicated a completed reaction, the reaction was cooled in ice bath, quenched with water and saturated ammonium chloride solution, and extracted with ethyl acetate. The organic phases were mixed with 200-300 mesh silica gel for loading onto and purifying by column chromatography, eluted with petroleum ether:ethyl acetate=1:1, to provide 6-chloro-N-ethoxy-4-((2-(N-methyl methanesulfonamido)-4-(trifluoromethyl)phenyl)amino)nicotinamide (**202-d,** 181mg, 0.388mmol, 29.76% yield). MS Calcd: 440; MS Found: 441($[M+H]^+$).

Step 4: 6-chloro-N-ethoxy-4-((2-(N-methyl methanesulfonamido)-4-(trifluoromethyl)phenyl)amino)nicotinamide (**202-d,** 58mg, 0.124mmol), 4-amino-2,6-dimethyl pyrimidine (18.38mg, 0.149mmol), $Pd_2(dba)_3$(22.00mg, 0.024mmol), Xant-Phos(27.81mg, 0.048mmol), $Cs_2CO_3$ (121.19mg, 0.372mmol), and 1,4-dioxane (2mL) were combined. After atmosphere replacement with nitrogen three times, the mixture was allowed to react at 120°C for 6 hours. When TLC indicated a completed reaction, the mixture was filtered by suction, and the filtrate was concentrated under reduced pressure at 40°C, mixed with 200-300 mesh silica gel for loading onto and purifying by column chromatography, eluted with DCM:MeOH=30:1, to provide the title compound: 6-((2,6-dimethyl pyrimidin-4-yl)amino)-N-ethoxy-4-((2-(N-methyl methanesulfonamido)-4-(trifluoromethyl)phenyl)amino)nicotinamide (**202,** 22mg, 0.039mmol, 32.35% yield). MS Calcd: 553; MS Found: 554($[M+H]^+$). $^1H$ NMR (400 MHz, DMSO-$d_6$)$\delta$11.75 (s, 1H), 10.41 (s, 1H), 10.18 (s, 1H), 8.41 (s, 1H), 8.17 (s, 1H), 8.15 (s, 1H), 7.92 (s, 1H), 7.87 (d, $J$ = 8.4 Hz, 1H), 7.78 (s, 1H), 3.95 (q, $J$ = 7.2 Hz, 2H), 3.23 (s, 3H), 3.20 (s, 3H), 2.37 (s, 3H), 2.29 (s, 3H), 1.22 (t, $J$ = 7.2 Hz, 3H).

Example **203**

6-((2,6-dimethyl pyrimidin-4-yl)amino)-N-ethoxy-4-((4-methyl-2-(N-methyl ethyl sulfonamido)phenyl)amino)nicotinamide

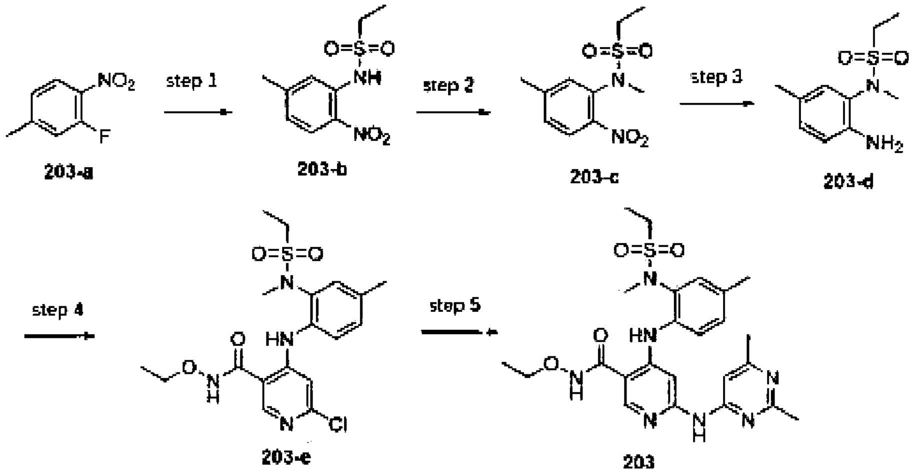


Step 1: To a 50ml flask were added 2-fluoro-4-methyl-1-nitrobenzene(**203-a**, 569mg, 3.7mmol), ethanesulfonamide (400mg, 3.7mmol), N,N-dimethyl formamide (10ml), and potassium carbonate (1.5g, 11.0mmol). After atmosphere replacement with nitrogen three times, the mixture was heated under nitrogen protection to 90°C with stirring for 3h. Upon indication of completed reaction by TLC (petroleum ether/ethyl acetate=2/1), the reaction mixture was cooled to room temperature, quenched with water (200ml), and extracted with ethyl acetate (200ml) three times. The organic phases were combined, dried over anhydrous sodium sulfate, mixed with silica gel for loading onto and purifying by column chromatography, eluted with petroleum ether/ethyl acetate=5:1~3:1, to provide N-(5-methyl-2-nitrophenyl) ethanesulfonamide (**203-b,** 600mg, 2.4mmol, 65% yield).

Step 2: To a 50ml flask were added N-(5-methyl-2-nitrophenyl) ethanesulfonamide (**203-b,** 300mg, 1.2mmol), N,N-dimethyl formamide (5ml). After atmosphere replacement with nitrogen three times, the mixture was cooled in ice-water bath and slowly added with sodium hydride(42mg, 1.76mmol) portionwise. The mixture was allowed to warm back to room temperature to react for half an hour, followed by adding iodomethane (250mg, 1.8mmol), and stirred at room temperature for 2h. Upon indication of completed reaction by TLC (petroleum ether/ethyl acetate=2/1), the system was quenched with water (150ml), extracted with ethyl acetate (150ml) three times. The organic phases were combined, dried over anhydrous sodium sulfate, and concentrated to provide N-methyl-N-(5-methyl-2-nitrophenyl) ethanesulfonamide (**203-c,** 300mg, 1.18mmol, 99% yield).

Step 3: To a 50ml flask was added N-methyl-N-(5-methyl-2-nitrophenyl) ethanesulfonamide (**203-c,** 300mg, 1.2mmol), followed by iron powder (329mg, 5.9mmol), ammonium chloride (624mg, 11.8mmol), ethanol (10ml), and water (1ml). After atmosphere replacement with nitrogen three times, the mixture was heated under nitrogen protection to 90°C with stirring for 3h. Upon indication of completed reaction by TLC (petroleum ether/ethyl acetate=1/1), the system was filtered, and the filter cake was washed with methanol three times. The organic phases were combined, dried over anhydrous sodium sulfate, and concentrated. The residue was dissolved with ethyl acetate (100ml), and washed with water (100ml) twice. The organic phases were combined and dried over anhydrous sodium sulfate, and concentrated to obtain N-(2-amino-5-methyl phenyl)-N-methyl ethanesulfonamide (**203-d,** 280mg, 1.2mmol, 99% yield). MS Calcd: 228; MS Found: 227([M-H]$^+$).

Step 4: To a 50ml flask were added N-(2-amino-5-methyl phenyl)-N-methyl ethanesulfonamide (**203-d,** 280mg, 1.2mmol), 4,6-dichloro-N-ethoxy nicotinamide (**int-2,** 316mg, 1.4mmol), and tetrahydrofuran (10ml). After atmosphere replacement with nitrogen three times, the mixture was slowly added with LiHMDS (3.1ml, 3.1mmol, 1mol/L) dropwise, followed by stirring at room temperature for 5h. Upon indication of completed reaction by TLC (petroleum ether/ethyl acetate=1/2), the system was washed with water (100ml), adjusted with 0.1mol/L diluted aqueous hydrochloride to pH=5, and extracted with ethyl acetate (100ml) three times. The organic phases were combined, dried over anhydrous sodium sulfate, concentrated, mixed with silica gel for loading and purifying by column chromatography, eluted with petroleum ether/ethyl acetate=3/1~1/1 to provide 6-chloro-N-ethoxy-4-((4-methyl-2-(N-methyl ethanesulfonamido)phenyl)amino) nicotinamide (**203-e,** 150mg, 0.35mmol, 29% yield).

Step 5: To a 50ml flask were added 6-chloro-N-ethoxy-4-((4-methyl-2-(N-methyl ethanesulfonamido)phenyl) amino)nicotinamide (**203-e,** 150mg, 0.35mmol), 2,6-dimethyl pyrimidin-4-ylamine (84mg, 0.7mmol), 4,5-bis-diphenylphosphino-9,9-dimethyl xanthene(82mg, 0.14mmol), cesium carbonate (344mg, 1.1mmol), $Pd(dba)_2$ (97mg, 0.11mmol),

and 1,4-dioxane (5ml). After atmosphere replacement with nitrogen four times, the mixture was heated under nitrogen protection to 120°C with stirring for 4h. Upon indication of completed reaction by TLC (DCM/MeOH=20/1), the system was directly mixed with silica gel for loading onto and purifying by column chromatography, eluted by DCM/MeOH=50/1~25/1. The eluents were combined and concentrated to obtain title compound: 6-((2,6-dimethyl pyrimidin-4-yl)amino)-N-ethoxy-4-((4-methyl-2-(N-methyl ethyl sulfonamido)phenyl)amino)nicotinamide (**203,** 120mg, 0.12mmol, 34% yield). MS Calcd: 513; MS Found: 514($[M+H]^+$). $^1H$ NMR (401 MHz, DMSO-$d_6$)δ11.63 (s, 1H), 10.00 (s, 1H), 9.87 (s, 1H), 8.34 (s, 1H), 7.79 (s, 1H), 7.46 (d, $J$ = 8.0 Hz, 1H), 7.40 (s, 1H), 7.25 (d, $J$ = 8.0 Hz, 1H), 7.04 (s, 1H), 3.94 (q, $J$ = 7.2 Hz, 2H), 3.24 (q, $J$ = 7.2 Hz, 2H), 3.12 (s, 3H), 2.33 (s, 3H), 2.30 (s, 3H), 2.26 (s, 3H), 1.22 (t, $J$ = 7.2 Hz, 6H).

Example **204**

4-((3-(1-cyclopropyl-1H-1,2,4-triazol-3-yl)-5-fluoro-2-methoxy phenyl)amino)-6-((2,6-dimethyl pyridin-4-yl)amino)-N-ethyl nicotinamide

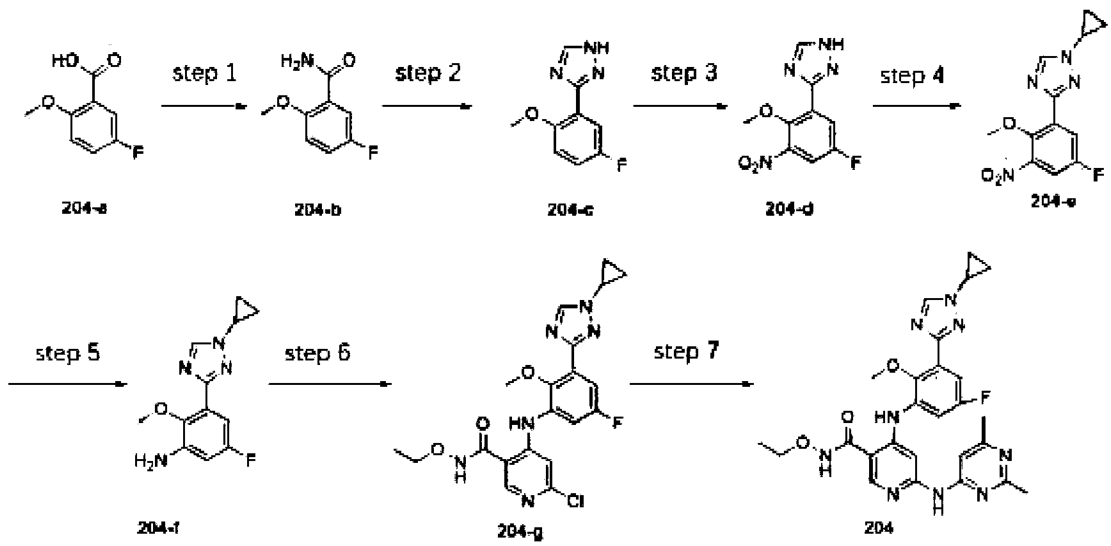


Step 1: To a 100ml two-necked flask was added 5-fluoro-2-methoxy benzoic acid (**204-a,** 3.4g, 20mmol) dissolved in dichloromethane (30ml), the reaction mixture was cooled with ice-water bath to 0°C~5°C, added with catalytic amount of DMF(0.1ml) dropwise, followed by carefully adding oxalyl chloride (3.81g, 30mmol) dropwise. The mixture was stirred at room temperature for 30min. Upon indication of completed reaction by TLC, the reaction mixture was concentrated under reduced pressure at 40°C. The residue was added to dichloromethane (20ml) and concentrated under reduced pressure to provide a crude material for further use. 25%~28% aqueous ammonia (5.6g, 40mmol) was added to tetrahydrofuran (10ml). The resulting reaction mixture was cooled with ice-water bath to 0°C~5°C and added with the previously obtained crude material, which was dissolved in dichloromethane (10ml) and carefully added dropwise. After the addition, the reaction mixture was stirred at room temperature for 2h, added with water, and the reaction mixture was subjected to phase separation. The organic phase was concentrated to provide 5-fluoro-2-methoxy benzamide (**204-b,** 3.3g, 19.5mmol, 97% yield). MS Calcd:169.03; MS Found: 170.05 ($[M+H]^+$).

Step 2: 5-fluoro-2-methoxy benzamide (**204-b,** 3.3g, 19.5mmol) was dissolved in DMF-DMA(25ml). The mixture was heated to 95°C for 1 hour, concentrated under reduced pressure to provide a crude product of DMF-DMA adduct, which was dissolved in ethanol (20ml) for further used. To a flask in ice bath were added ethanol (56ml), acetic acid(17ml). The mixture was stirred for 5minutes, and added with hydrazine hydrate(80wt.%, 8.4ml) dropwise with continuous stirring for 15 minutes. The mixture was added with the ethanol solution of the previous crude DMF-DMA adduct product, and allowed to warm back to room temperature with continuous stirring for 4 hours. The reaction mixture was concentrated under reduced pressure, diluted with ethyl acetate (300ml). The organic phase was washed with saturated sodium bicarbonate aqueous solution, and subjected to phase separation. The organic phase was washed with saturated brine, dried over anhydrous sodium sulfate, and concentrated under reduced pressure to provide 3-(5-fluoro-2-methoxy phenyl)-1H-1,2,4-triazole(**204-c**, 2.4g, 12.43 mmol, 63.7%). MS Calcd:193.03; MS Found: 194.05 ($[M+H]^+$)

Step 3: 3-(5-fluoro-2-methoxy phenyl)-1H-1,2,4-triazole(**204-c**, 1.1g, 5.69 mmol) was dissolved in concentrated sulfuric acid (10ml). The mixture was cooled in ice-water bath and added with nitric acid (68wt.%, 1.05 g, 11.39mmol) dropwise. After the addition, the mixture was continuously stirred in ice-water bath for 2 hours. The reaction mixture was

poured into ice-water, and slowly added with aqueous ammonia dropwise, to adjust pH to about 9, and extracted with ethyl acetate. After phase separation, the organic phase was dried, concentrated under reduced pressure, and subjected to column chromatography to provide 3-(5-fluoro-2-methoxy-3-nitrophenyl)-1H-1,2,4-triazole(**204-d**, 900 mg, 3.78mmol, 66.4%). MS Calcd:238.03; MS Found: 239.05 ($[M+H]^+$)

Step 4: 3-(5-fluoro-2-methoxy-3-nitrophenyl)-1H-1,2,4-triazole (**204-d,** 800mg, 3.36mmol), copper acetate (915 mg, 13.4 mmol), 2,2'-bipyridine (786mg, 5.04mmol), and sodium carbonate (712mg, 6.72mmol) were combined and added into 1,2-dichloroethane(10ml), followed by adding at room temperature cyclopropyl boronic acid (1.56g, 13.4mmol). The mixture was heated to 85°C with stirring for 5 hours, then cooled to room temperature, and filtered. The filtrate was added with ethyl acetate/water, and then subjected to phase separation. The organic phase was concentrated under reduced pressure and subjected to column chromatography to provide 1-cyclopropyl-3-(5-fluoro-2-methoxy-3-nitrophenyl)-1H-1,2,4-triazole (**204-e,** 450mg, 1.62mmol, 48.2%). MS Calcd:279.03; MS Found: 279.05 ($[M+H]^+$).

Step 5: To a solution of 1-cyclopropyl-3-(5-fluoro-2-methoxy-3-nitrophenyl)-1H-1,2,4-triazole(**204-e**, 450mg, 1.62mmol) in methanol (10ml) was added Pd/C (100 mg). The mixture was allowed to react under hydrogen atmosphere at normal temperature and pressure for 8 hours, and filtered through diatomaceous earth to remove the catalyst. The filtrate was concentrated under reduced pressure to provide 3-(1-cyclopropyl-1H-1,2,4-triazol-3-yl)-5-fluoro-2-methoxy aniline(**204-f**, 280mg, 1.13mmol, 70% yield). MS Calcd:248.11.03; MS Found: 249.05 ($[M+H]^+$)

Step 6: 3-(1-cyclopropyl-1H-1,2,4-triazol-3-yl)-5-fluoro-2-methoxy aniline(**204-f**, 280mg, 1.13mmol, ), 4,6-dichloro-N-ethoxy nicotinamide (264.2mg, 1.13mmol) were added to 6ml of anhydrous N,N-dimethylacetamide. The mixture was added at room temperature with a solution of LiHMDS in tetrahydrofuran (3.4ml, 3.39mmol), and stirred at room temperature for 2h. Upon indication of completed reaction by TLC, the mixture was adjusted with aqueous hydrochloride (1N) to pH 5, and extracted with ethyl acetate (30 ml x 3). The combined organic layers were dried, concentrated and then purified by column chromatography to provide 6-chloro-4-((3-(1-cyclopropyl-1H-1,2,4-triazol-3-yl)-5-fluoro-2-methoxy phenyl)amino)-N-ethoxy nicotinamide (**204-g**, 280mg, 0.63mmol, 56% yield). MS Calcd:446.13; MS Found: 447.05 ($[M+H]^+$)

Step 7: 6-chloro-4-((3-(1-cyclopropyl-1H-1,2,4-triazol-3-yl)-5-fluoro-2-methoxy phenyl)amino)-N-ethoxy nicotinamide (**204-g**, 90mg, 0.2mmol), 2,6-dimethyl pyrimidin-4-ylamine (24.6 mg, 0.2mmol), cesium carbonate (195 mg, 0.6 mmol), Xant-Phos(23.1mg, 0.04mmol) and $Pd_2(dba)_3$(18.3mg, 0.02 mmol) were added to anhydrous dioxane (6ml). After the atmosphere of the mixture was evacuated to vacuum and refilled with nitrogen, the reaction mixture was heated to 120°C with stirring for 6 hours, and filtered by suction. The filtrate was concentrated and purified by high performance preparative thin layer chromatography (MeOH:DCM=1:10) to provide the title compound: 4-((3-(1-cyclopropyl-1H-1,2,4-triazol-3-yl)-5-fluoro-2-methoxy phenyl)amino)-6-((2,6-dimethyl pyridin-4-yl)amino)-N-ethyl nicotinamide (**204**, 32mg, 0.06mmol, 30% yield). MS Calcd:533.23; MS Found: 534.05 ($[M+H]^+$). $^1H$ NMR (400 MHz, DMSO-$d_6$)δ11.77 (s, 1H), 10.49 (s, 1H), 10.20 (s, 1H), 8.73 (s, 1H), 8.42 (s, 1H), 8.20 (s, 1H), 7.51 (d, $J$ = 10.0 Hz, 1H), 7.39 - 7.28 (m, 1H), 7.17 (s, 1H), 3.99-3.95 (m, 2H), 3.89 (brs, 1H), 3.75 (s, 3H), 2.41 (s, 3H), 2.30 (s, 3H), 1.25-1.21 (m, 3H), 1.20 - 1.14 (m, 2H), 1.09-1.07 (m, 2H).

Example **205**

6-(cyclopropylcarboxamido)-4-((3-(1-cyclopropyl-1H-1,2,4-triazol-3-yl)-5-fluoro-2-methoxy phenyl)amino)-N-ethyl nicotinamide

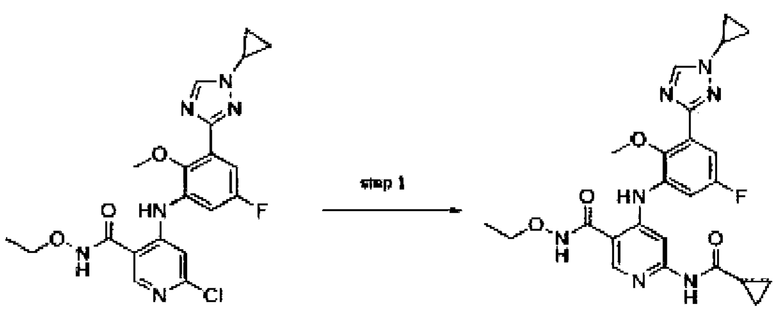


**204-g** **205**

Step 1: 6-chloro-4-((3-(1-cyclopropyl-1H-1,2,4-triazol-3-yl)-5-fluoro-2-methoxy phenyl)amino)-N-ethoxy nicotinamide (**204-g,** 90mg, 0.2mmol), cyclopropylcarboxamide (17 mg, 0.2mmol), cesium carbonate (195 mg, 0.6 mmol),

Xant-Phos(23.1mg, 0.04mmol) and $Pd_2(dba)_3$(18.3mg, 0.02 mmol) were added to anhydrous dioxane (6ml). After the atmosphere of the mixture was evacuated to vacuum and refilled with nitrogen, the reaction mixture was heated to 120°C with stirring for 6 hours, and filtered by suction. The filtrate was concentrated and purified by high performance preparative thin layer chromatography (MeOH:DCM=1:10) to provide the title compound: 6-(cyclopropylcarboxamido)-4-((3-(1-cyclopropyl-1H-1,2,4-triazol-3-yl)-5-fluoro-2-methoxy phenyl)amino)-N-ethyl nicotinamide (**205,** 12mg, 0.02mmol, 8.1% yield). MS Calcd:495.23; MS Found: 496.05 ($[M+H]^+$). $^1H$ NMR (400 MHz, DMSO-$d_6$)δ11.82 (s, 1H), 10.92 (s, 1H), 10.37 (s, 1H), 8.73 (s, 1H), 8.42 (s, 1H), 8.17 (s, 1H), 7.40 - 7.29 (m, 2H), 3.98 - 3.88 (m, 3H), 3.73 (s, 3H), 2.00-1.98 (m, 1H), 1.23 (t, *J*= 7.2 Hz, 3H), 1.19-1.17 (m, 2H), 1.09-1.07 (m, 2H), 0.82-0.79 (m, 4H).

Example **206**

4-((4-cyclopropyl-2-(oxetan-3-yloxy)phenyl)amino)-6-(((2,6-dimethyl pyrimidin-4-yl)amino)-N-ethoxy nicotinamide

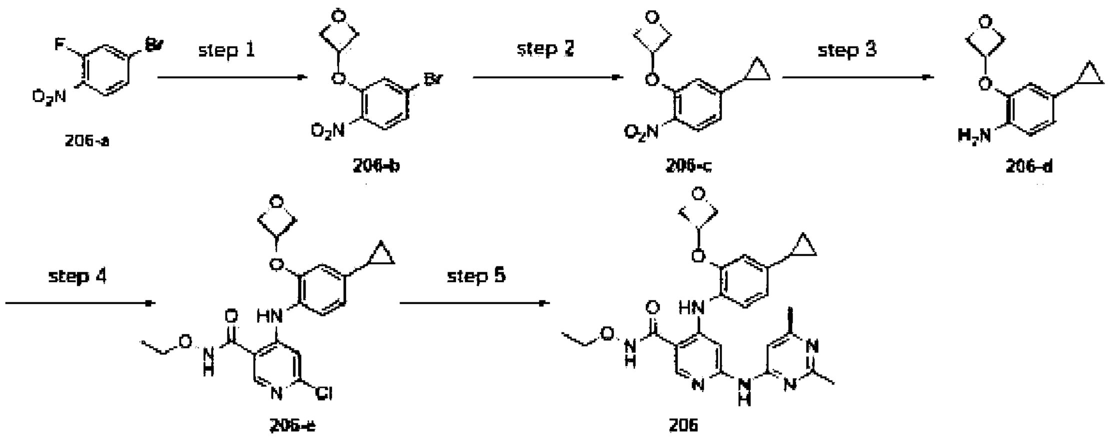


Step 1: Into 10 ml of DMF was added 4-bromo-2-fluoro-1-nitrobenzene (**206-a,** 880 g, 4.0 mmol). The mixture was cooled in ice-water bath, added with sodium hydride (320mg, 8.0 mmol) with continuous stirring for 20minutes, followed by adding with oxetan-3-ol (325.6 mg, 4.4 mmol) and then stirred at room temperature for 1 hour. Upon indication of completed reaction by TLC, the reaction mixture was added with water (20 mL), and extracted with ethyl acetate (30 mL x 2). The organic phase was washed with saturated brine (30 mL x 2), dried over anhydrous sodium sulfate, and concentrated under reduced pressure, to provide 3-(5-bromo-2-nitrophenoxy) oxetane (**206-b,** 700 mg, 2.56 mmol, 63.9% yield). MS Calcd: 272.96; MS Found: 274.10 ($[M-H]^+$).

Step 2: 3-(5-bromo-2-nitrophenoxy) oxetane (**206-b,** 650 mg, 2.37 mmol), cyclopropyl boronic acid (224 mg, 2.6 mmol), potassium carbonate (654 mg, 4.74 mmol) and Pd(dppf)$Cl_2$ (175 mg, 0.24 mmol) were sequentially added to 15 mL of mixed solution of dioxane/water (5/1). The atmosphere of the mixture was evacuated to vacuum and replaced with nitrogen three times, and the mixture was stirred at 90°C for 3 hours. Upon indication of completed reaction by TLC, the mixture was concentrated under reduced pressure. The residue was separated and purified by silica gel column chromatography (EA:PE=2:1) to provide 3-(5-cyclopropyl-2-nitrophenoxy) oxetane(**206-c**, 550 mg, 2.3 mmol, 98% yield). MS Calcd: 235.07; MS Found: 236.13 ($[M+H]^+$).

Step 3: 3-(5-cyclopropyl-2-nitrophenoxy) oxetane(**206-c**, 150 mg, 0.63mmol, ), ammonium chloride (336 mg, 6.35mmol) and iron powder (178 mg, 3.18 mmol) were sequentially added to 10 mL of mixed solvent of water and ethanol (1:4), stirred under reflux for 6 hours. Upon indication of completed reaction by TLC, the reaction mixture was filtered by suction. The filtrate was concentrated, and the residue was separated and purified by silica gel column chromatography to provide 4-cyclopropyl-2-(oxetan-3-yloxy) aniline (**206-d**, 80 mg, 0.39 mmol, 63% yield). MS Calcd: 205.09; MS Found: 206.22 ($[M+H]^+$).

Step 4: 4-cyclopropyl-2-(oxetan-3-yloxy) aniline (**206-d**, 80 mg, 0.39 mmol) and 4,6-dichloro-N-ethoxy nicotinamide (92 mg, 0.4mmol) were added to 5 ml of anhydrous N,N-dimethylacetamide. The mixture was added at room temperature with a solution of LiHMDS in tetrahydrofuran (1.2 ml, 1.2 mmol), and stirred at room temperature for 3 hours. Upon indication of completed reaction by TLC, the mixture was adjusted with aqueous hydrochloride (1N) to pH 5, and extracted with ethyl acetate (20 ml x 3). The combined organic layers were dried, concentrated and then purified by column chromatography (petroleum ether:ethyl acetate =1:1) to provide 6-chloro-4-((4-cyclopropyl-2-(oxetan-3-yloxy)phenyl) amino)-N-ethoxy nicotinamide (**206-e**, 110 mg, 0.57 mmol, 70% yield). MS Calcd: 403.10; MS Found: 404.29 ($[M+H]^+$).

Step 5: 6-chloro-4-((4-cyclopropyl-2-(oxetan-3-yloxy)phenyl)amino)-N-ethoxy nicotinamide (**206-e**, 110 mg,

0.57 mmol), 2,6-dimethyl pyrimidin-4-ylamine (36.3mg, 0.29mmol), cesium carbonate (263 mg, 0.81 mmol), Xant-Phos(24.5mg, 0.027mmol) and $Pd_2(dba)_3$ (28.mg, 0.054 mmol) were added to anhydrous dioxane (5 ml). After the atmosphere of the mixture was evacuated to vacuum and refilled with nitrogen, the reaction mixture was heated to 120°C with stirring for 6 hours, and filtered by suction. The filtrate was concentrated and purified by high performance preparative thin layer chromatography (MeOH:DCM=1:20) to provide 4-((4-cyclopropyl-2-(oxetan-3-yloxy)phenyl)amino)-6-(((2,6-dimethyl pyrimidin-4-yl)amino)-N-ethoxy nicotinamide (**206**, 46mg, 0.093mmol, 34.7% yield). MS Calcd: 490.20; MS Found: 491.29 ($[M+H]^+$). $^1H$ NMR (400 MHz, DMSO-$d_6$)δ11.44 (s, 1H), 10.10 (s, 1H), 8.17 (s, 1H), 7.83 (s, 1H), 7.20 (s, 1H), 6.88 (d, *J* = 8.0 Hz, 1H), 6.58 (d, *J* = 2.0 Hz, 1H), 6.53 (dd, *J* = 8.0, 2.0 Hz, 1H), 4.15 (d, *J* = 8.0 Hz, 1H), 3.70 - 3.66 (m, 2H), 3.65 - 3.60 (m, 2H), 3.53-3.49 (m, 1H), 3.40-3.37 (m, 1H), 2.30 (s, 3H), 2.28 (s, 3H), 1.85-1.78 (m, 1H), 1.13(t, *J* = 7.2 Hz, 3H), 0.91 - 0.81 (m, 2H), 0.60 - 0.51 (m, 2H).

Example **207**

6-(((2,6-dimethyl pyrimidin-4-yl)amino)-N-ethoxy-4-((2-methoxy-4-(1-methyl-1H-pyrazol-5-yl)phenyl)amino)nicotinamide

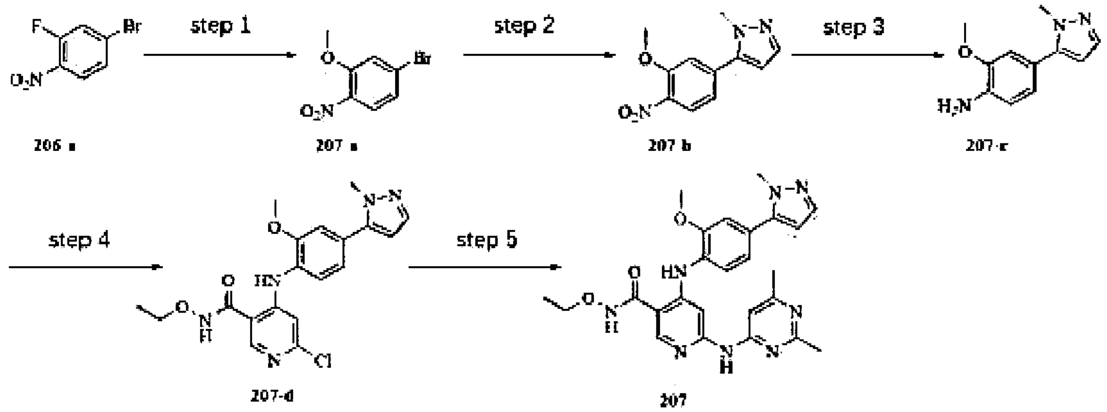


Step 1: To 4-bromo-2-fluoro-1-nitrobenzene(**206-a** 1.32 g, 6.0 mmol) dissolved in 20 mL of methanol was added sodium methoxide (486mg, 9.0 mmol). After that, the reaction mixture was heated to 60°C with continuous stirring for 3 hours. Upon indication of completed reaction by TLC, the reaction mixture was added with water (40 mL), and extracted with ethyl acetate (30 mL x 2). The organic phase was washed with saturated brine (30 mL x 2), dried over anhydrous sodium sulfate, concentrated under reduced pressure, to provide the title compound 4-bromo-2-methoxy-1-nitrobenzene (**207-a,** 1.38 g, 6.0 mmol, 100% yield). MS Calcd: 230.95; MS Found: 232.10 ($[M-H]^+$).

Step 2: 4-bromo-2-methoxy-1-nitrobenzene (**207-a,** 696 mg, 3.0 mmol), 1-methyl-5-(4,4,5,5-tetramethyl-1,3,2-dioxaborolan-2-yl)-1H-pyrazole (749 mg, 3.6 mmol), potassium carbonate (828 mg, 6.0 mmol) and Pd(dppf)$Cl_2$ (219 mg, 0.3 mmol) were sequentially added to 30 mL of mixed solution of dioxane/water (5/1). The atmosphere of the mixture was evacuated to vacuum and replaced with nitrogen three times, and the mixture was stirred at 80°C for 3 hours. Upon indication of completed reaction by TLC, the mixture was concentrated under reduced pressure, and the residue was separated and purified by silica gel column chromatography (EA:PE=2:1) to provide 5-(3-methoxy-4-nitrophenyl)-1-methyl-1H-pyrazole (**207-b,** 605 mg, 2.6 mmol, 86.7% yield). MS Calcd: 233.07; MS Found: 234.13 ($[M+H]^+$).

Step 3: 5-(3-methoxy-4-nitrophenyl)-1-methyl-1H-pyrazole (**207-b,** 490 mg, 2 mmol), ammonium chloride (1.08 g, 20mmol) and iron powder (560 mg, 10 mmol) were sequentially added to 10 mL of mixed solvent of water and ethanol (1:4), followed by stirring under reflux for 6 hours. Upon indication of completed reaction by TLC, the reaction mixture was filtered by suction. The filtrate was concentrated, and the residue was separated and purified by silica gel column chromatography to provide 2-methoxy-4-(1-methyl-1H-pyrazol-5-yl)aniline (**207-c,** 320 mg, 1.57 mmol, 74.4% yield). MS Calcd: 203.09; MS Found: 204.22 ($[M+H]^+$).

Step 4: 2-ethoxy-4-(1-methyl-1H-pyrazol-5-yl)aniline (**207-c,** 203 mg, 1.0 mmol, ), 4,6-dichloro-N-ethoxy nicotinamide (234 mg, 1.0mmol) were added to 5 ml of anhydrous N,N-dimethylacetamide. The mixture was added at room temperature with a solution of LiHMDS in tetrahydrofuran (3.0 ml, 3.0 mmol), and stirred at room temperature for 3 hours. Upon indication of completed reaction by TLC, the mixture was adjusted with aqueous hydrochloride (1N) to pH 5, and extracted with ethyl acetate (20 ml x 3). The combined organic layers were dried, concentrated and then purified by column chromatography (petroleum ether:ethyl acetate =1:1) to provide 6-chloro-N-methoxy-4-((2-ethoxy-4-(1-methyl-1H-pyrazol-5-yl)phenyl)amino)nicotinamide (**207-d,** 220 mg, 0.57 mmol, 56.8% yield). MS Calcd: 401.10; MS Found: 402.29

([M+H]$^+$).

Step 5: 6-chloro-N-ethoxy-4-((2-methoxy-4-(1-methyl-1H-pyrazol-5-yl)phenyl)amino)nicotinamide (**207-d**, 103 mg, 0.25 mmol), 2,6-dimethyl pyrimidin-4-ylamine (32.3mg, 0.26mmol), cesium carbonate (243 mg, 0.75 mmol), XantPhos(24.5mg, 0.025mmol) and $Pd_2(dba)_3$(28.mg, 0.05 mmol) were added to anhydrous dioxane (5 ml). After the atmosphere of the mixture was evacuated to vacuum and refilled with nitrogen, the reaction mixture was heated to 120°C with stirring for 6 hours, and filtered by suction. The filtrate was concentrated and purified by high performance preparative thin layer chromatography (MeOH:DCM=1:20) to provide the title compound: 6-(((2,6-dimethyl pyrimidin-4-yl)amino)-N-ethoxy-4-((2-methoxy-4-(1-methyl-1H-pyrazol-5-yl)phenyl)amino)nicotinamide (**207,** 40mg, 0.082mmol, 32.7% yield). MS Calcd: 488.20; MS Found: 489.29 ([M+H]$^+$). $^1$H NMR (400 MHz, DMSO-$d_6$)δ11.69 (s, 1H), 10.11 (s, 1H), 10.10 (s, 1H), 8.38 (s, 1H), 8.22 (s, 1H), 7.65 (d, *J* = 8.0 Hz, 1H), 7.48 (d, *J* = 4.0 Hz, 1H), 7.23 (d, *J* = 2.0 Hz, 1H), 7.16 (dd, *J* = 8.0, 2.0 Hz, 1H), 7.07 (s, 1H), 6.42 (d, *J* = 2.0 Hz, 1H), 3.96 (q, *J* = 7.2 Hz, 2H), 3.93 (s, 3H), 3.90 (s, 3H), 2.38 (s, 3H), 2.28 (s, 3H), 1.232 (t, *J* = 7.2 Hz, 3H).

Example **208**

6-((2,6-dimethyl pyrimidin-4-yl)amino)-N-ethoxy-4-((3-(5-fluoropyrimidin-2-yl)-2-methoxy phenyl)amino)nicotinamide

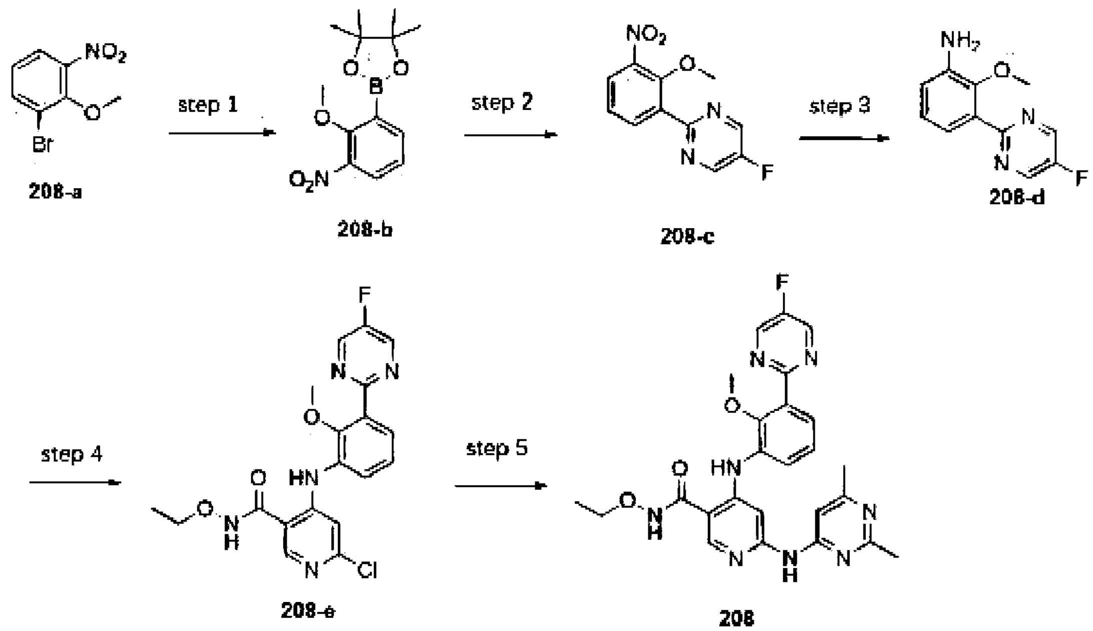


208-a 208-b 208-c 208-d 208-e 208

Step 1: To a 50ml flask were added 1-bromo-2-methoxy-3-nitrobenzene (**208-a,** 1.0g, 4.3mmol), bis(pinacolato) diboron (1.6g, 6.5mmol), potassium acetate (1.27g, 12.9mmol), Pd(dppf)$Cl_2$ (315mg, 0.43mmol), and 1,4-dioxane (20ml). After atmosphere replacement with nitrogen three times, the mixture was heated to 100°C and stirred under nitrogen protection for 3h. Upon indication of completed reaction by TLC (petroleum ether/ethyl acetate=50/1), the reaction mixture was cooled to room temperature, quenched with water(200ml), extracted with ethyl acetate (200ml) three times. The organic phases were combined, dried over anhydrous sodium sulfate, mixed with silica gel for loading onto and purifying by column chromatography, eluted with petroleum ether/ethyl acetate=50:1 to provide 2-(2-methoxy-3-nitrophenyl)-4,4,5,5-tetramethyl-1,3,2-dioxaborolane (**208-b,** 600mg, 2.3mmol, 53% yield).

Step 2: To a 50ml flask were added 2-(2-methoxy-3-nitrophenyl)-4,4,5,5-tetramethyl-1,3,2-dioxaborolane(**208-b**, 600mg, 1.2mmol), 2-chloro-5-fluoropyrimidine (364mg, 2.75mmol), sodium carbonate (728mg, 6.87mmol), pd(dppf) $Cl_2$(167mg, 0.23mmol), and diox (6ml). After atmosphere replacement with nitrogen three times, the mixture was heated to 90°C with stirring for 4h. Upon indication of completed reaction by TLC (petroleum ether/ethyl acetate=5/1), the reaction mixture was quenched with water (150ml), and extracted with ethyl acetate (150ml) three times. The organic phases were combined, dried over anhydrous sodium sulfate, and concentrated to provide 5-fluoro-2-(2-methoxy-3-nitrophenyl) pyrimidine **(208-c,** 400mg, 1.6mmol).

Step 3: To a 50ml flask was added 5-fluoro-2-(2-methoxy-3-nitrophenyl) pyrimidine (**208-c,** 400mg, 1.6mmol), followed by iron powder (450mg, 8.0mmol), ammonium chloride (860mg, 16.0mmol), ethanol (20ml), and water (1ml). After atmosphere replacement with nitrogen three times, the mixture was heated under nitrogen protection to 90°C with

stirring for 3h. Upon indication of completed reaction by TLC (petroleum ether/ethyl acetate=1/1), the mixture was filtered, and the filter cake was washed with methanol three times. The organic phases were combined, dried over anhydrous sodium sulfate, purified by column chromatography and eluted with petroleum ether/ethyl acetate=2:1, and concentrated to provide 3-(5-fluoropyrimidin-2-yl)-2-methoxy aniline(**208-d**, 300mg, 1.37mmol, 86% yield). MS Calcd: 219.22; MS Found: 220.23([M+H]$^+$).

Step 4: To a 50ml flask were added 3-(5-fluoropyrimidin-2-yl)-2-methoxy aniline(**208-d**, 300mg, 1.37mmol), 4,6-dichloro-N-ethoxy nicotinamide (353mg, 1.5mmol), and tetrahydrofuran (10ml). After atmosphere replacement with nitrogen three times, the mixture was slowly added with LiHMDS (3.4ml, 3.4mmol, 1mol/L) dropwise, followed by stirring at RT for 5h. Upon indication of completed reaction by TLC (petroleum ether/ethyl acetate=1/2), the system was washed with water (100ml), adjusted with 0.1mol/L diluted aqueous hydrochloride to pH=5, and extracted with ethyl acetate (100ml) three times. The organic phases were combined, dried over anhydrous sodium sulfate, concentrated, mixed with silica gel for loading and purified by column chromatography, and eluted with petroleum ether/ethyl acetate=3/1~1/1 to provide 6-chloro-N-ethoxy-4-((3-(5-fluoropyrimidin-2-yl)-2-methoxy phenyl)amino)nicotinamide (**208-e,** 300mg, 0.72mmol, 53% yield).

Step 5: To a 50ml flask were added 6-chloro-N-ethoxy-4-((3-(5-fluoropyrimidin-2-yl)-2-methoxy phenyl)amino) nicotinamide (**208-e,** 150mg, 0.36mmol), 2,6-dimethyl pyrimidin-4-ylamine (77mg, 0.7mmol), 4,5-bis-diphenylphosphino-9,9-dimethyl xanthene(82mg, 0.14mmol), cesium carbonate (352mg, 1.1mmol), Pd(dba)$_2$ (99mg, 0.11mmol), and 1,4-dioxane (5ml). After atmosphere replacement with nitrogen four times, the mixture was heated under nitrogen protection to 120°C with stirring for 4h. Upon indication of completed reaction by TLC (DCM/MeOH=20/1), the system was directly mixed with silica gel for loading onto and purifying by column chromatography and eluted with DCM/MeOH=50/1~25/1. After concentration, the title compound 6-((2,6-dimethyl pyrimidin-4-yl)amino)-N-ethoxy-4-((3-(5-fluoropyrimidin-2-yl)-2-methoxy phenyl)amino)nicotinamide (**208**, 30mg, 0.12mmol, 17% yield) was obtained. MS Calcd: 504.20; MS Found: 505.22([M+H]$^+$).

$^1$H NMR (400 MHz, DMSO-$d_6$)δ11.70 (s, 1H), 10.22 (s, 1H), 10.10 (s, 1H), 9.04 (s, 2H), 8.39 (s, 1H), 8.14 (s, 1H), 7.73 (dd, $J$ = 8.0, 1.6 Hz, 1H), 7.46 (dd, $J$ = 7.8, 1.6 Hz, 1H), 7.32 (t, $J$ = 8.0 Hz, 1H), 7.08 (s, 1H), 3.96 (q, $J$ = 7.2 Hz, 2H), 3.69 (s, 3H), 2.38 (s, 3H), 2.28 (s, 3H), 1.23 (t, $J$ = 7.2 Hz, 3H).

Example **209**

6-(cyclopropylcarboxamido)-N-ethoxy-4-((3-(5-fluoropyrimidin-2-yl)-2-methoxy phenyl)amino)nicotinamide

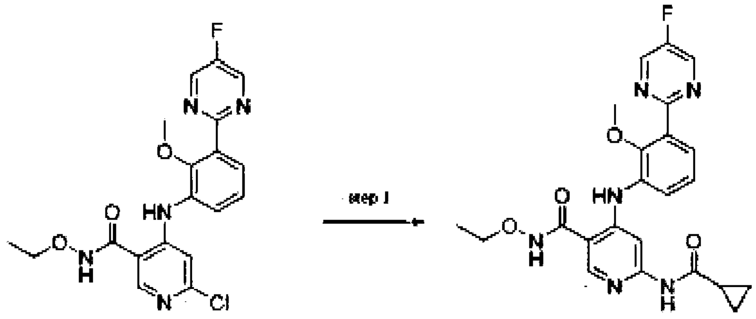


208-e

209

Step 5: To a 50ml flask were added 6-chloro-N-ethoxy-4-((3-(5-fluoropyrimidin-2-yl)-2-methoxy phenyl)amino) nicotinamide (**208-e,** 150mg, 0.36mmol), cyclopropylcarboxamide (61mg, 0.7mmol), 4,5-bis-diphenylphosphino-9,9-dimethyl xanthene(82mg, 0.14mmol), cesium carbonate (352mg, 1.1mmol), Pd$_2$(dba)$_3$(99mg, 0.11mmol), and 1,4-dioxane (5ml). After atmosphere replacement with nitrogen four times, the mixture was heated under nitrogen protection to 120°C with stirring for 4h. Upon indication of completed reaction by TLC (DCM/MeOH=20/1), the system was directly mixed with silica gel for loading onto and purifying by column chromatography and eluted with DCM/MeOH=50/1~25/1. After concentration, 6-(cyclopropylcarboxamido)-N-ethoxy-4-((3-(5-fluoropyrimidin-2-yl)-2-methoxy phenyl)amino)nicotinamide (**209,** 20mg, 0.12mmol, 12% yield) was obtained. MS Calcd: 466.18; MS Found: 467.21([M+H]$^+$). $^1$H NMR (400 MHz, DMSO-$d_6$)δ11.76 (s, 1H), 10.82 (s, 1H), 10.14 (s, 1H), 9.04 (s, 2H), 8.40 (s, 1H), 8.08 (s, 1H), 7.57 (dd, $J$ = 8.0, 1.6 Hz, 1H), 7.44 (dd, $J$ = 8.0, 1.6 Hz, 1H), 7.28 (t, $J$ = 8.0 Hz, 1H), 3.95 (q, $J$ = 7.2 Hz, 2H), 3.67 (s, 3H), 3.33 (s, 3H), 2.19 - 1.89 (m, 1H), 1.22 (t, $J$ = 7.2 Hz, 3H), 0.90 - 0.35 (m, 4H).

Example **210**

6-(cyclopropylcarboxamido)-N-ethoxy-4-((5-fluoro-3-(5-fluoropyrimidin-2-yl)-2-methoxy phenyl)amino)nicotinamide

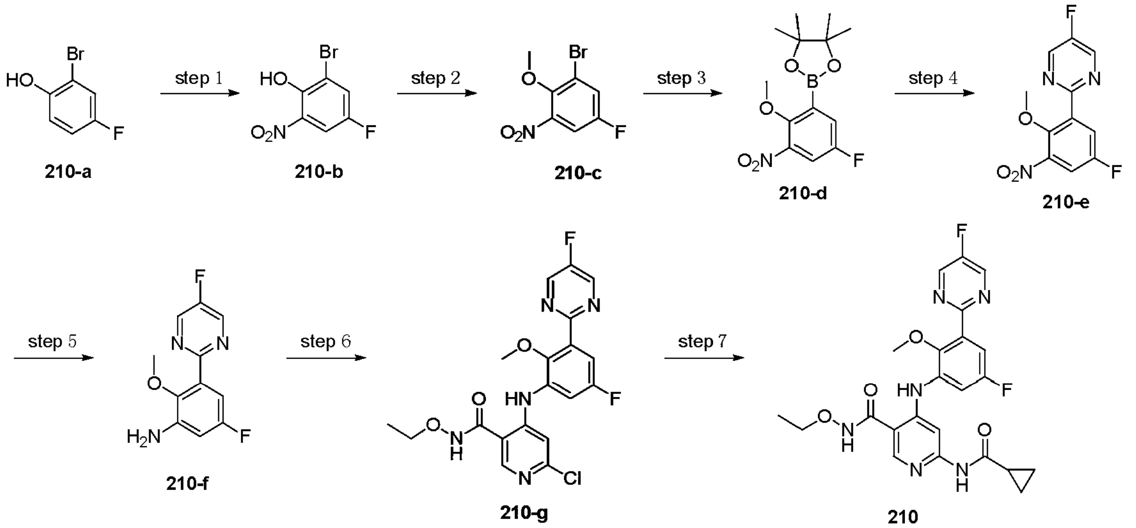


Step 1: To 2-bromo-4-fluorophenol (**210-a,** 2g, 10.47mmol) dissolved in chloroform (30mL) was added a mixture of concentrated sulfuric acid (205mg, 2.09mmol) and nitric acid (68%, 1.07g, 11.52mmol), heated to 45°C with stirring for 2 hours. Upon indication of completed reaction by LC-MS, the reaction mixture was cooled to room temperature, concentrated, diluted by water, and extracted with ethyl acetate. The combined organic phases were sequentially washed with saturated sodium bicarbonate aqueous solution and saturated brine, dried over anhydrous sodium sulfate, followed by solvent removed by rotary evaporation to provide 2-bromo-4-fluoro-6-nitrophenol(**210-b**, 1.0 g, 4.24mmol, 40% yield). MS Calcd: 234.93, 235.93; MS Found: 233.96, 235.96($[M-H]^-$)

Step 2: To 2-bromo-4-fluoro-6-nitrophenol(**210-b**, 1.0 g, 4.24mmol) dissolved in acetonitrile (20mL) were sequentially added potassium carbonate (1.75g, 12.68mmol) and iodomethane (1.2g, 8.45mmol). The mixture was heated to 85°C with stirring for 6 hours. When TLC indicated a completed reaction, the reaction mixture was cooled to room temperature, diluted by water, and extracted with ethyl acetate. The combined organic phases were washed with saturated brine, dried over anhydrous sodium sulfate, concentrated, and purified by column chromatography (petroleum ether/ethyl acetate, 7%) to provide 1-bromo-5-fluoro-2-methoxy-3-nitrobenzene (**210-c**, 800mg, 3.2mmol, 75% yield).

Step 3: 1-bromo-5-fluoro-2-methoxy-3-nitrobenzene(**210-c**, 800mg, 3.2mmol), bis(pinacolato)diboron (1.2g, 4.7mmol), potassium acetate (941mg, 9.6mmol), Pd(dppf)$Cl_2$(220mg, 0.3mmol) were added to dioxane (10mL). After atmosphere replacement with nitrogen three times, the reaction mixture was heated to 95°C with stirring for 4 hours. When TLC indicated a completed reaction, the reaction mixture was cooled to room temperature and filtered through diatomaceous earth. The filtrate was concentrated and purified by column chromatography (ethyl acetate:petroleum ether=1:4) to provide 2-(5-fluoro-2-methoxy-3-nitrophenyl)-4,4,5,5-tetramethyl-1,3,2-dioxaborolane(**210-d**, 720mg, 2.42mmol, 76% yield).

Step 4: To dioxane (10mL) and water (1mL) were added 2-(5-fluoro-2-methoxy-3-nitrophenyl)-4,4,5,5-tetramethyl-1,3,2-dioxaborolane(**210-d**, 720mg, 2.42mmol), 2-chloro-5-fluoropyrimidine (386mg, 2.9mmol), potassium carbonate (1g, 7.26mmol), and Pd(dppf)$Cl_2$(176mg, 0.24mmol). After atmosphere replacement with nitrogen three times, the reaction mixture was heated to 95°C with stirring for 16 hours. When TLC indicated a completed reaction, the reaction mixture was cooled to room temperature, filtered through diatomaceous earth. The filtrate was concentrated and purified by column chromatography (ethyl acetate:petroleum ether=1:4) to provide 5-fluoro-2-(5-fluoro-2-methoxy-3-nitrophenyl) pyrimidine (**210-e,** 400mg, 1.5mmol, 62% yield).

Step 5: To 5-fluoro-2-(5-fluoro-2-methoxy-3-nitrophenyl) pyrimidine (**210-e,** 400mg, 1.5mmol) dissolved in methanol (5mL) and water (1mL) were sequentially and slowly added ammonium chloride (675mg, 12.5mmol) and iron powder (350mg, 6.25mmol), followed by stirring at room temperature for 4 hours. When TLC indicated a completed reaction, the reaction mixture was filtered through diatomaceous earth. The filtrate was diluted with water and extracted with ethyl acetate. The combined organic phases were washed with saturated sodium chloride aqueous solution, dried over anhydrous sodium sulfate, followed by solvent removed by rotary evaporation to provide : 5-fluoro-3-(5-fluoropyrimidin-2-yl)-2-methoxy aniline(**210-f**, 300mg, 1.26mmol, 84% yield). MS Calcd: 237.07; MS Found: 238.($[M+H]^+$)

Step 6: To a two-necked flask were sequentially added 5-fluoro-3-(5-fluoropyrimidin-2-yl)-2-methoxy aniline(**210-f**, 300mg, 1.26mmol), 4,6-dichloro-*N*-ethoxy nicotinamide (385mg, 1.64mmol), and 5ml of anhydrous tetrahydrofuran as solvent. After atmosphere replacement with nitrogen, the mixture was cooled in ice bath and added with a solution of LiHMDS in tetrahydrofuran (3.8ml, 1mol/L) with continuous stirring at room temperature for 2 hours. Upon indication of completed reaction by TLC, the reaction mixture was quenched with saturated ammonium chloride solution, adjusted with aqueous hydrochloride (1*N*) to pH 5, and extracted with ethyl acetate (30mLx3). The combined organic layers were washed with saturated sodium chloride aqueous solution, and dried over anhydrous sodium sulfate.After concentration, the residue was purified by column chromatography to provide 6-chloro-N-ethoxy-4-((5-fluoro-3-(5-fluoropyrimidin-2-yl)-2-methoxy phenyl)amino)nicotinamide (**210-g,** 300mg, 0.69mmol, 42% yield). MS Calcd: 435.09; MS Found: 436.07([M+H]+)

Step 7: 6-chloro-N-ethoxy-4-((5-fluoro-3-(5-fluoropyrimidin-2-yl)-2-methoxy phenyl)amino)nicotinamide (**210-g,** 100mg, 0.23mmol), cyclopropylcarboxamide(25mg, 0.29mmol), cesium carbonate (225mg, 0.69mmol), XantPhos (27mg, 0.047mmol) and $Pd_2(dba)_3$(21mg, 0.023mmol) were added to anhydrous dioxane (2ml). After atmosphere replacement by nitrogen, the mixture was heated with microwave at 120°C for 1 hour. The reaction mixture was a cooled to room temperature, diluted with ethyl acetate, and filtered by suction. The filtrate was concentrated, and purified by plate chromatography (petroleum ether:ethyl acetate=1:4) and reverse phase MPLC to provide the title compound: 6-(cyclopropylcarboxamido)-N-ethoxy-4-((5-fluoro-3-(5-fluoropyrimidin-2-yl)-2-methoxy phenyl)amino)nicotinamide (**210,** 20mg, 0.041mmol, 18% yield). MS Calcd: 484.17; MS Found: 485.18([M+H]+). $^1$H NMR (400 MHz, DMSO-d6)δ11.78 (s, 1H), 10.90 (s, 1H), 10.33 (s, 1H), 9.06 (s, 2H), 8.43 (s, 1H), 8.17 (s, 1H), 7.45 (dd, *J* = 10.0, 3.2 Hz, 1H), 7.23 (dd, *J* = 9.2, 3.2 Hz, 1H), 3.95 (q, *J* = 7.2 Hz, 2H), 3.68 (s, 3H), 2.04 - 1.96 (m, 1H), 1.22 (t, *J* = 7.2 Hz, 3H), 0.85 - 0.77 (m, 4H).

Example **211**

6-(((2,6-dimethyl pyrimidin-4-yl)amino)-N-ethoxy-4-((5-fluoro-3-(5-fluoropyrimidin-2-yl)-2-methoxy phenyl)amino)nicotinamide

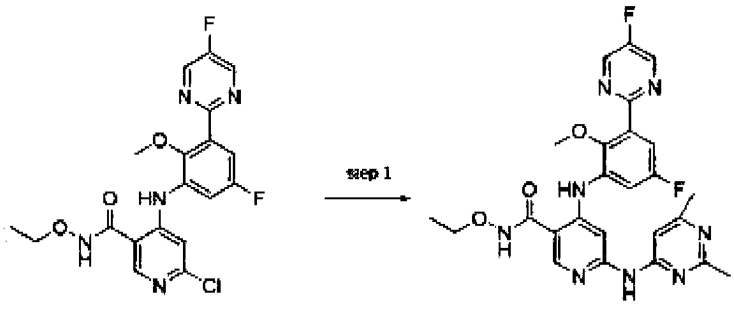


210-g 211

Step 1: 6-chloro-N-ethoxy-4-((5-fluoro-3-(5-fluoropyrimidin-2-yl)-2-methoxy phenyl)amino)nicotinamide (**210-g,** 100mg, 0.23mmol), 4-amino-2,6-dimethyl pyrimidine (36mg, 0.29mmol), cesium carbonate (225mg, 0.69mmol), XantPhos (27mg, 0.047mmol) and $Pd_2(dba)_3$(21mg, 0.023mmol) were added to anhydrous dioxane (2ml). After atmosphere replacement by nitrogen, the mixture was heated to 120°C to react for 6 hours, and cooled to room temperature, then diluted with dichloromethane and methanol, and filtered by suction. The filtrate was concentrated, and purified by plate chromatography (ethyl acetate) and reverse phase MPLC to provide the title compound: 6-(((2,6-dimethyl pyrimidin-4-yl)amino)-N-ethoxy-4-((5-fluoro-3-(5-fluoropyrimidin-2-yl)-2-methoxy phenyl)amino)nicotinamide (**211,** 40mg, 0.076mmol, 33% yield). MS Calcd: 522.19; MS Found: 523.18([M+H]+). $^1$H NMR (400 MHz, DMSO-$d_6$)δ11.76 (s, 1H), 10.44 (s, 1H), 10.16 (s, 1H), 9.05 (s, 2H), 8.42 (s, 1H), 8.17 (s, 1H), 7.58 (dd, *J* = 10.0, 3.2 Hz, 1H), 7.23 (dd, *J* = 9.2, 3.2 Hz, 1H), 7.16 (s, 1H), 3.95 (q, *J* = 7.2 Hz, 2H), 3.70 (s, 3H), 2.41 (s, 3H), 2.29 (s, 3H), 1.22 (t, *J* = 7.2 Hz, 3H).

Example **212**

6-(((2,6-dimethyl pyrimidin-4-yl)amino)-N-ethoxy-4-((2-methoxy-3-(pyrimidin-2-yl)phenyl)amino)nicotinamide

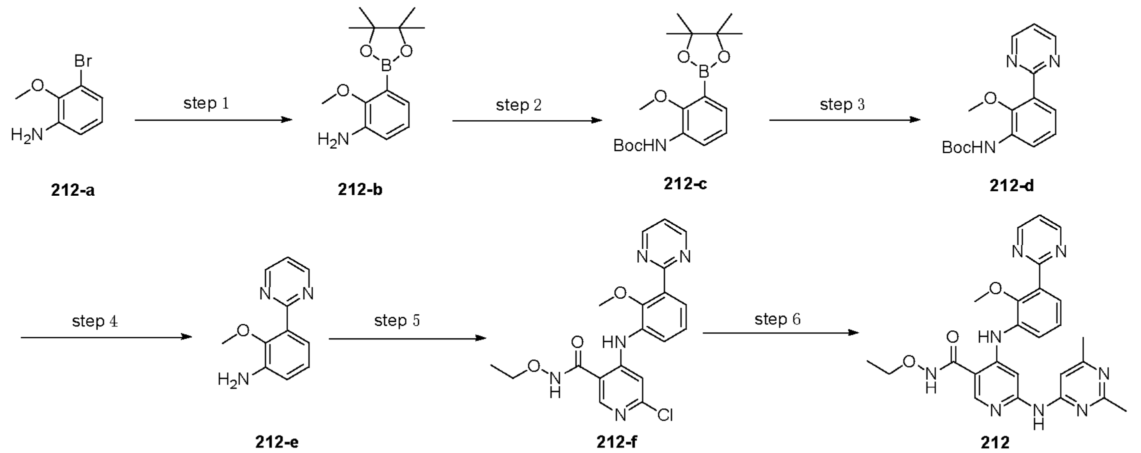


212-a 212-b 212-c 212-d 212-e 212-f 212

Step 1: 3-bromo-2-methoxy aniline (**212-a,** 2.8 g, 13.86 mmol), bis(pinacolato)diboron (5.3 g, 20.79 mmol), Pd(dppf)$Cl_2$ (1.03 g, 1.39 mmol) and potassium acetate (4.08 g, 41.58 mmol) were sequentially added to dioxane. After the atmosphere of the mixture was evacuated to vacuum and replaced with nitrogen three times, the mixture was heated to 110°C with continuous stirring for 20 hours. Upon indication of completed reaction by TLC, the reaction mixture was thoroughly concentrated. The residue was added with water (40 mL), extracted with ethyl acetate (30 mL x 2). The organic phase was washed with saturated brine (30 mL x 2), dried over anhydrous sodium sulfate, and concentrated under reduced pressure. The residue was separated and purified by silica gel column chromatography (ethyl acetate:petroleum ether=1:4) to provide 2-methoxy-3-(4,4,5,5-tetramethyl-1,3,2-dioxaborolan-2-yl)aniline (**212-b,** 2.3 g, 9.23 mmol, 66% yield). MS Calcd: 249.15; MS Found: 250.01 ($[M+H]^+$).

Step 2: Into 10 mL of ethanol solvent were added 2-methoxy-3-(4,4,5,5-tetramethyl-1,3,2-dioxaborolan-2-yl) aniline (**212-b,** 2.3 g, 9.23 mmol) and Boc anhydride (2.41 g, 11.07 mmol). The mixture was stirred overnight at room temperature. Upon indication of completed reaction by TLC, the filtrate was concentrated, and the residue was separated and purified by silica gel column chromatography (ethyl acetate:petroleum ether=1:4) to provide t-butyl (2-methoxy-3-(4,4,5,5-tetramethyl-1,3,2-dioxaborolan-2-yl)phenyl)carbamate (**212-c,** 2.4 g, 8 mmol, 86% yield). MS Calcd: 348.20; MS Found: 250.16 ($[M-100]^+$).

Step 3: t-butyl (2-methoxy-3-(4,4,5,5-tetramethyl-1,3,2-dioxaborolan-2-yl)phenyl)carbamate (**212-c,** 370 mg, 1.06 mmol), 2-bromopyrimidine (200 mg, 1.27 mmol), potassium phosphate (521 mg, 2.46 mmol) and Pd(dppf)$Cl_2$(59 mg, 0.082 mmol) were added to 7 mL of mixed solution of water and dioxane (6:1). After the atmosphere of the mixture was evacuated to vacuum and replaced with nitrogen three times, the mixture was stirred at 105°C for 10 hours. Upon indication of completed reaction by TLC, the reaction mixture was thoroughly concentrated. The residue was separated and purified by silica gel column chromatography (ethyl acetate:petroleum ether=1:1) to provide t-butyl (2-methoxy-3-(pyrimidin-2-yl)phenyl)carbamate (**212-d,** 150 mg, 0.5 mmol, 39% yield). MS Calcd: 300.13; MS Found: 301.12 ($[M+H]^+$).

Step 4: t-butyl (2-methoxy-3-(pyrimidin-2-yl)phenyl)carbamate (**212-d,** 150 mg, 0.5 mmol) was dissolved in dichloromethane, followed by trifluoroacetic acid (421 mg, 3.7 mmol). The mixture was stirred at room temperature for 5h. Upon indication of completed reaction by TLC, the reaction mixture was thoroughly concentrated. The residue was added with saturated sodium bicarbonate aqueous solution, extracted with dichloromethane, washed with saturated brine, dried over anhydrous sodium sulfate, and concentrated under reduced pressure. The residue was separated and purified by silica gel column chromatography to provide the product 2-methoxy-3-(pyrimidin-2-yl)aniline (**212-e,** 70 mg, 0.35 mmol, 70% yield). MS Calcd: 201.23; MS Found: 202.10 ($[M+H]^+$).

Step 5: 2-methoxy-3-(pyrimidin-2-yl)aniline (**212-e,** 70 mg, 0.35 mmol), 4,6-dichloro-N-ethoxy nicotinamide (74 mg, 0.32 mmol) were added to 5 ml of anhydrous N,N-dimethylacetamide. The mixture was added at room temperature with a solution of LiHMDS in tetrahydrofuran (1.28 ml, 1.28 mmol), and stirred at room temperature for 3 hours. Upon indication of completed reaction by TLC, the mixture was adjusted with aqueous hydrochloride (4N) to pH 5, and extracted with ethyl acetate (20 ml x 3). The combined organic layers were dried, concentrated and then purified by column chromatography (PE:EA =1:2) to provide 6-chloro-N-ethoxy-4-((2-methoxy-3-(pyrimidin-2-yl)phenyl)amino)nicotinamide (**212-f,** 110 mg, 0.27 mmol, 72% yield). MS Calcd: 399.11; MS Found: 400.11 ($[M+H]^+$).

Step 6: 6-chloro-N-ethoxy-4-((2-methoxy-3-(pyrimidin-2-yl)phenyl)amino)nicotinamide (**212-f,** 110 mg, 0.27 mmol), 4-amino-2,6-dimethyl pyrimidine (44 mg, 0.36 mmol), cesium carbonate (195 mg, 0.6 mmol), XantPhos(25mg, 0.04mmol) and $Pd_2(dba)_3$(27mg, 0.029 mmol) were added to anhydrous dioxane (2 ml). After the atmosphere of the mixture was evacuated to vacuum and replaced with nitrogen three times, the mixture was heated to 125°C with stirring for 10 hours, and filtered by suction. The filtrate was concentrated and purified by preparative TLC (MeOH:DCM=1:20) to provide the title compound: 6-(((2,6-dimethyl pyrimidin-4-yl)amino)-N-ethoxy-4-((2-methoxy-3-(pyrimidin-2-yl)phenyl)amino)nicotinamide (**212,** 20mg, 0.04mmol, 15% yield). MS Calcd: 486.21; MS Found: 487.21 ([M+H]$^+$). $^1$H NMR (400 MHz, DMSO-$d_6$) $\delta$ 11.70 (s, 1H), 10.22 (s, 1H), 10.10 (s, 1H), 8.95 (d, *J* = 4.8 Hz, 2H), 8.39 (s, 1H), 8.17 (s, 1H), 7.73 (dd, *J* = 8.0, 1.6 Hz, 1H), 7.51 (t, *J* = 4.8 Hz, 1H), 7.46 (dd, *J* = 8.0, 1.6 Hz, 1H), 7.32 (t, *J* = 8.0 Hz, 1H), 7.09 (s, 1H), 3.96 (q, *J* = 7.2 Hz, 2H), 3.70 (s, 3H), 2.39 (s, 3H), 2.28 (s, 3H), 1.23 (t, *J* = 7.2 Hz, 3H).

Example **213**

6-(((2,6-dimethyl pyrimidin-4-yl)amino)-N-ethoxy-4-((2-methoxy-3-(5-methyl pyrazin-2-yl)phenyl)amino)nicotinamide

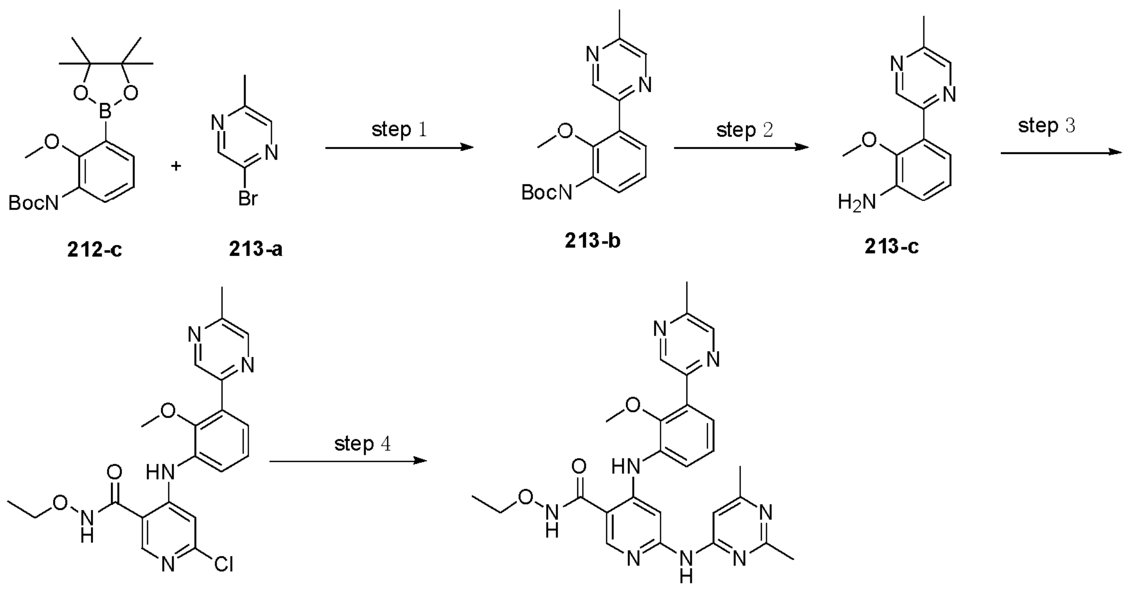


212-c 213-a 213-b 213-c 213-d 213

Step 1: t-butyl (2-methoxy-3-(4,4,5,5-tetramethyl-1,3,2-dioxaborolan-2-yl)phenyl)carbamate (**212-c**, 280 mg, 0.8 mmol), 2-bromo-5-methyl pyrazine (**213-a**, 166 mg, 0.96 mmol), potassium phosphate (508 mg, 2.4 mmol) and Pd(dppf)$Cl_2$(58 mg, 0.08 mmol) were added to 7 mL of mixed solution of water and dioxane (6:1). After the atmosphere of the mixture was evacuated to vacuum and replaced with nitrogen three times, the mixture was stirred at 105°C for 10 hours. Upon indication of completed reaction by TLC, the reaction mixture was thoroughly concentrated. The residue was separated and purified by silica gel column chromatography (ethyl acetate:petroleum ether=1:1) to provide the product t-butyl (2-methoxy-3-(5-methyl pyrazin-2-yl)phenyl)carbamate **(213-b**, 190 mg, 0.6 mmol, 76% yield). MS Calcd: 315.15; MS Found: 316.11 ([M+H]$^+$).

Step 2: t-butyl (2-methoxy-3-(5-methyl pyrazin-2-yl)phenyl)carbamate (**213-b,** 190 mg, 0.6 mmol) was dissolved in dichloromethane, followed by trifluoroacetic acid (1.39 g, 12 mmol). The mixture was stirred at room temperature for 5h. Upon indication of completed reaction by TLC, the reaction mixture was thoroughly concentrated. The residue was added with saturated sodium bicarbonate aqueous solution, extracted with dichloromethane, washed with saturated brine, dried over anhydrous sodium sulfate, and concentrated under reduced pressure. The residue was separated and purified by silica gel column chromatography to provide the product 2-methoxy-3-(5-methyl pyrazin-2-yl)aniline (**213-c,** 110 mg, 0.51 mmol, 85% yield). MS Calcd: 215.11; MS Found: 216.11 ([M+H]$^+$).

Step 3: 2-methoxy-3-(5-methyl pyrazin-2-yl)aniline (**213-c,** 110 mg, 0.51 mmol) and 4,6-dichloro-N-ethoxy nicotinamide (108 mg, 0.46 mmol) were added to 5 ml of anhydrous N,N-dimethylacetamide. The mixture was added at room temperature with a solution of LiHMDS in tetrahydrofuran (1.28 ml, 1.28 mmol), and stirred at room temperature for 3 hours. Upon indication of completed reaction by TLC, the mixture was adjusted with aqueous hydrochloride (4N) to pH 5, and extracted with ethyl acetate (20 ml x 3). The combined organic layers were dried, concentrated and then purified by column chromatography (MeOH: DCM =1:20) to provide 6-chloro-N-ethoxy-4-((2-methoxy-3-(5-methyl pyrazin-2-yl) phenyl)amino)nicotinamide (**213-d,** 100 mg, 0.24 mmol, 47% yield). MS Calcd: 413.13; MS Found: 414.14 ([M+H]$^+$).

Step 4: 6-chloro-N-ethoxy-4-((2-methoxy-3-(5-methyl pyrazin-2-yl)phenyl)amino)nicotinamide (**213-d,** 100 mg, 0.24 mmol), 4-amino-2,6-dimethyl pyrimidine (30 mg, 0.24 mmol), cesium carbonate (234 mg, 0.72 mmol), XantPhos (20mg, 0.036mmol) and $Pd_2(dba)_3$(22mg, 0.024 mmol) were added to anhydrous dioxane (2 ml). After the atmosphere of the mixture was evacuated to vacuum and replaced with nitrogen three times, the mixture was heated to 125°C with stirring

for 10 hours, and filtered by suction. The filtrate was concentrated and purified by preparative TLC (MeOH:DCM=1:20) to provide 6-(((2,6-dimethyl pyrimidin-4-yl)amino)-N-ethoxy-4-((2-methoxy-3-(5-methyl pyrazin-2-yl)phenyl)amino)nicotinamide (**213,** 20mg, 0.04mmol, 16% yield). MS Calcd: 500.23; MS Found: 501.18 ([M+H]$^+$). $^1$H NMR (400 MHz, DMSO-$d_6$) $\delta$ 11.74 (s, 1H), 10.35 (s, 1H), 10.09 (s, 1H), 8.96 (s, 1H), 8.68 (s, 1H), 8.42 (s, 1H), 8.14 (s, 1H), 7.71 (dd, $J$ = 8.0, 1.6 Hz, 1H), 7.49 (dd, $J$ = 8.0, 1.6 Hz, 1H), 7.35 (t, $J$ = 8.0 Hz, 1H), 7.11 (s, 1H), 3.97 (q, $J$ = 7.2 Hz, 2H), 3.54 (s, 3H), 2.57 (s, 3H), 2.38 (s, 3H), 2.29 (s, 3H), 1.25 (d, $J$ = 7.2 Hz, 3H).

Example **214**

6-(((2,6-dimethyl pyrimidin-4-yl)amino)-N-ethoxy-4-((5-fluoro-2-methoxy-3-(5-methyl pyrazin-2-yl)phenyl)amino)nicotinamide

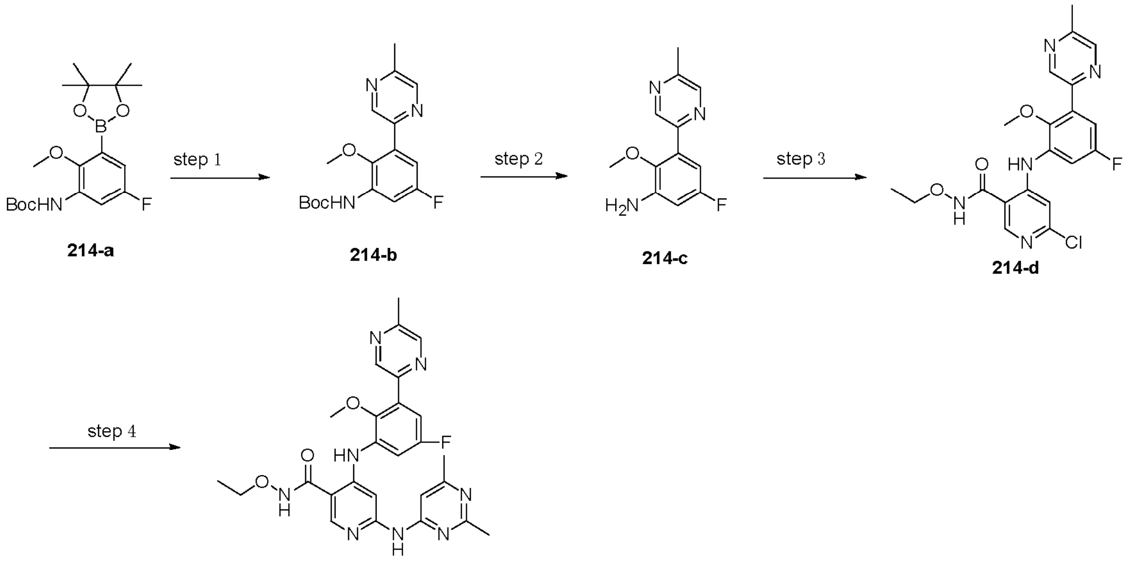


**214-a** **214-b** **214-c** **214-d**

**214**

Step 1: t-butyl (5-fluoro-2-methoxy-3-(4,4,5,5-tetramethyl-1,3,2-dioxaborolan-2-yl)phenyl)carbamate (**214-a,** 280 mg, 0.76 mmol), 2-bromo-5-methyl pyrazine (158 mg, 0.91 mmol), potassium phosphate (483 mg, 2.28 mmol) and Pd(dppf)$Cl_2$(55 mg, 0.076 mmol) were added to 7 mL of mixed solution of water and dioxane (6:1). After the atmosphere of the mixture was evacuated to vacuum and replaced with nitrogen three times, the mixture was stirred at 105°C for 10 hours. Upon indication of completed reaction by TLC, the reaction mixture was thoroughly concentrated, and the residue was separated and purified by silica gel column chromatography (EA:PE=1:4) to provide t-butyl (5-fluoro-2-methoxy-3-(5-methyl pyrazin-2-yl)phenyl)carbamate (**214-b,** 130 mg, 0.40 mmol, 51% yield). MS Calcd: 333.14; MS Found: 334.15 ([M+H]$^+$).

Step 2: t-butyl (5-fluoro-2-methoxy-3-(5-methyl pyrazin-2-yl)phenyl)carbamate (**214-b,** 130 mg, 0.40 mmol) was dissolved in dichloromethane, followed by adding trifluoroacetic acid (950 mg, 8 mmol). The mixture was stirred at room temperature for 5h. Upon indication of completed reaction by TLC, the reaction mixture was thoroughly concentrated. The residue was added with saturated sodium bicarbonate aqueous solution, extracted with dichloromethane, washed with saturated brine, dried over anhydrous sodium sulfate, and concentrated under reduced pressure. The residue was separated and purified by silica gel column chromatography to provide 5-fluoro-2-methoxy-3-(5-methyl pyrazin-2-yl) aniline (**214-c,** 100 mg, 0.43 mmol, 98% yield). MS Calcd: 233.10; MS Found: 234.11 ([M+H]$^-$).

Step 3: 5-fluoro-2-methoxy-3-(5-methyl pyrazin-2-yl)aniline (**214-c,** 100 mg, 0.43 mmol) and 4,6-dichloro-N-ethoxy nicotinamide (92 mg, 0.4 mmol) were added to 5 ml of anhydrous N,N-dimethylacetamide. The mixture was added at room temperature with a solution of LiHMDS in tetrahydrofuran (1.6 ml, 1.6 mmol), and stirred at room temperature for 3 hours. Upon indication of completed reaction by TLC, the mixture was adjusted with aqueous hydrochloride (4N) to pH 5, and extracted with ethyl acetate (20 ml x 3). The combined organic layers were dried, concentrated and then purified by column chromatography (MeOH: DCM =1:20) to provide 6-chloro-N-ethoxy-4-((5-fluoro-2-methoxy-3-(5-methyl pyrazin-2-yl)phenyl)amino)nicotinamide (**214-d,** 110 mg, 0.25 mmol, 59% yield). MS Calcd: 431.12; MS Found: 432.11 ([M+H]$^+$).

Step 4: 6-chloro-N-ethoxy-4-((5-fluoro-2-methoxy-3-(5-methyl pyrazin-2-yl)phenyl)amino)nicotinamide (**214-d,**

110 mg, 0.25 mmol), 4-amino-2,6-dimethyl pyrimidine (32 mg, 0.26 mmol), cesium carbonate (253 mg, 0.78 mmol), XantPhos(23mg, 0.04mmol) and $Pd_2(dba)_3$(24mg, 0.026 mmol) were added to anhydrous dioxane (2 ml). After the atmosphere of the mixture was evacuated to vacuum and replaced with nitrogen three times, the mixture was heated to 125°C with stirring for 10 hours, and filtered by suction. The filtrate was concentrated and purified by preparative TLC (MeOH:DCM=1:20) to provide the title compound: 6-(((2,6-dimethyl pyrimidin-4-yl)amino)-N-ethoxy-4-((5-fluoro-2-methoxy-3-(5-methyl pyrazin-2-yl)phenyl)amino)nicotinamide (**214,** 50mg, 0.09mmol, 38% yield). MS Calcd: 518.22; MS Found: 519.28 ($[M+H]^+$). $^1H$ NMR (400 MHz, DMSO-$d_6$)$\delta$11.78 (s, 1H), 10.52 (s, 1H), 10.18 (s, 1H), 9.03 (s, 1H), 8.70 (s, 1H), 8.44 (s, 1H), 8.19 (s, 1H), 7.56 (dd, *J* = 9.6, 3.2 Hz, 1H), 7.27 (dd, *J* = 9.6, 3.2 Hz, 1H), 7.19 (s, 1H), 3.97 (q, *J* = 7.2 Hz, 2H), 3.55 (s, 3H), 2.58 (s, 3H), 2.42 (s, 3H), 2.31 (s, 3H), 1.24 (t, *J* = 7.2 Hz, 3H).

Example **215**

6-((2,6-dimethyl pyrimidin-4-yl)amino)-N-ethoxy-4-((5-fluoro-2-methoxy-3-(pyrimidin-2-yl)phenyl)amino)nicotinamide

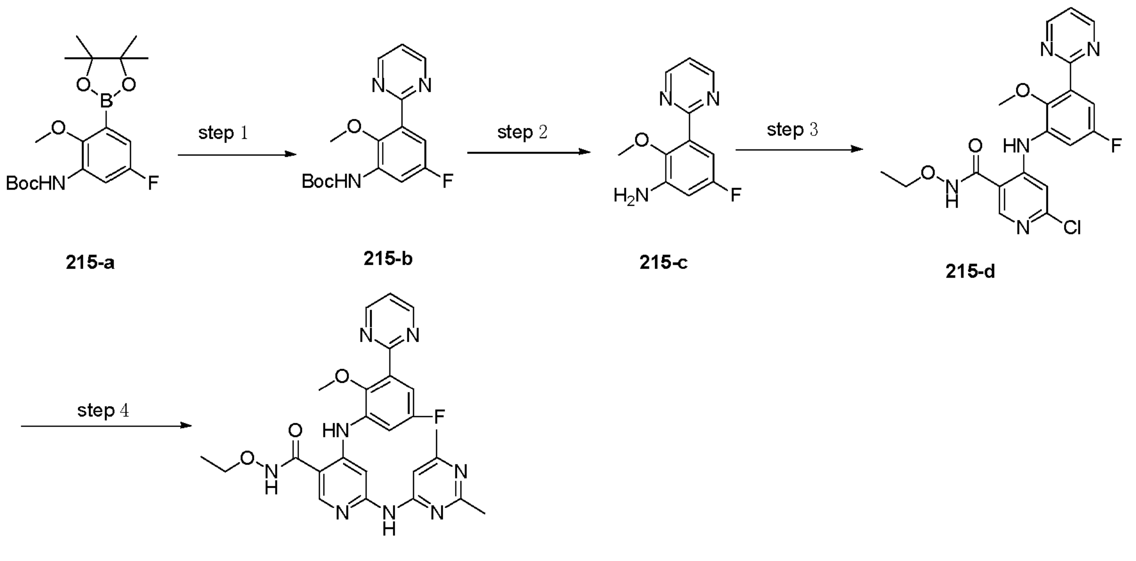


215-a 215-b 215-c 215-d 215

Step 1: t-butyl (5-fluoro-2-methoxy-3-(4,4,5,5-tetramethyl-1,3,2-dioxaborolan-2-yl)phenyl)carbamate (**215-a,** 260 mg, 0.71 mmol), 2-bromopyrimidine (135 mg, 0.85 mmol), potassium phosphate (451 mg, 2.13 mmol) and Pd(dppf)$Cl_2$(62 mg, 0.085 mmol) were added to 7 mL of mixed solution of water and dioxane (6:1). After the atmosphere of the mixture was evacuated to vacuum and replaced with nitrogen three times, the mixture was stirred at 105°C for 10 hours. Upon indication of completed reaction by TLC, the reaction mixture was thoroughly concentrated, and the residue was separated and purified by silica gel column chromatography (EA:PE=1:4) to provide t-butyl (5-fluoro-2-methoxy-3-(pyrimidin-2-yl)phenyl)carbamate (**215-b,** 140 mg, 0.44 mmol, 62% yield). MS Calcd: 319.13; MS Found: 320.13 ($[M+H]^+$).

Step 2: t-butyl (5-fluoro-2-methoxy-3-(pyrimidin-2-yl)phenyl)carbamate (**215-b,** 140 mg, 0.44 mmol) was dissolved in dichloromethane, followed by adding trifluoroacetic acid (1.03 g, 8.8 mmol). The mixture was stirred at room temperature for 5h. Upon indication of completed reaction by TLC, the reaction mixture was thoroughly concentrated. The residue was added with saturated sodium bicarbonate aqueous solution, extracted with dichloromethane, washed with saturated brine, dried over anhydrous sodium sulfate, and concentrated under reduced pressure. The residue was separated and purified by silica gel column chromatography to provide 5-fluoro-2-methoxy-3-(pyrimidin-2-yl)aniline (**215-c,** 100 mg, 0.43 mmol, 98% yield). MS Calcd: 219.22; MS Found: 220.07 ($[M+H]^+$).

Step 3: 5-fluoro-2-methoxy-3-(pyrimidin-2-yl)aniline (**215-c,** 100 mg, 0.43 mmol), 4,6-dichloro-N-ethoxy nicotinamide (97 mg, 0.41 mmol) were added to 5 ml of anhydrous N,N-dimethylacetamide. The mixture was added at room temperature with a solution of LiHMDS in tetrahydrofuran (1.64 ml, 1.64 mmol), and stirred at room temperature for 3 hours. Upon indication of completed reaction by TLC, the mixture was adjusted with aqueous hydrochloride (4N) to pH 5, and extracted with ethyl acetate (20 ml x 3). The combined organic layers were dried, concentrated and then purified by column chromatography (MeOH: DCM =1:20) to provide 6-chloro-N-ethoxy-4-((5-fluoro-2-methoxy-3-(pyrimidin-2-yl)phenyl)amino)nicotinamide (**215-d,** 130 mg, 0.31 mmol, 72% yield). MS Calcd: 417.10; MS Found: 418.06 ($[M+H]^+$).

Step 4: 6-chloro-N-ethoxy-4-((5-fluoro-2-methoxy-3-(pyrimidin-2-yl)phenyl)amino)nicotinamide (**215-d,** 130

mg, 0.31 mmol), 4-amino-2,6-dimethyl pyrimidine (38 mg, 0.31 mmol), cesium carbonate (302 mg, 0.93 mmol), XantPhos(26mg, 0.046mmol) and $Pd_2(dba)_3$(28mg, 0.031 mmol) were added to anhydrous dioxane (2 ml). After the atmosphere of the mixture was evacuated to vacuum and replaced with nitrogen three times, the mixture was heated to 125°C with stirring for 10 hours, and filtered by suction. The filtrate was concentrated and purified by preparative TLC (MeOH:DCM=1:20) to provide the title compound: 6-((2,6-dimethyl pyrimidin-4-yl)amino)-N-ethoxy-4-((5-fluoro-2-methoxy-3-(pyrimidin-2-yl)phenyl)amino)nicotinamide (**215,** 50mg, 0.09mmol, 38% yield). MS Calcd: 504.23; MS Found: 505.18 ([M+H]$^+$). $^1$H NMR (400 MHz, DMSO-$d_6$)$\delta$11.76 (s, 1H), 10.45 (s, 1H), 10.18 (s, 1H), 8.98 (d, *J* = 4.8 Hz, 2H), 8.43 (s, 1H), 8.20 (s, 1H), 7.59 (dd, *J* = 9.6, 3.2 Hz, 1H), 7.55 (t, *J* = 4.8 Hz, 1H), 7.24 (dd, *J* = 9.6, 3.2 Hz, 1H), 7.18 (s, 1H), 3.96 (q, *J* = 7.2 Hz, 2H), 3.71 (s, 3H), 2.42 (s, 3H), 2.31 (s, 3H), 1.23 (t, *J* = 7.2 Hz, 3H).

Example **216**

4-((4-cyclopropyl-2-(N-methyl methanesulfonamido)phenyl)amino)-N-ethoxy-6-((5-fluoro-6-methyl pyridin-2-yl)amino) nicotinamide

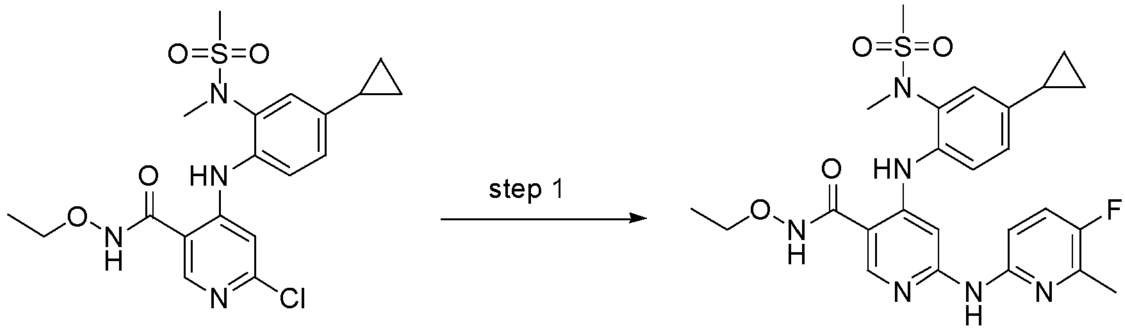


**20-a**

**216**

Step 1: 6-chloro-4-((4-cyclopropyl-2-(N-methyl methanesulfonamido)phenyl)amino)-N-ethoxy nicotinamide (**20-a,** 100mg, 0.228mmol), 5-fluoro-6-methyl pyridin-2-ylamine (57.48mg, 0.456mmol), $Pd_2(dba)_3$(41.75mg, 0.045mmol), XantPhos(53.23mg, 0.092mmol), $Cs_2CO_3$ (222.8mg, 0.684mmol) were mixed and added with 1,4-dioxane (3mL). After atmosphere replacement with nitrogen three times, the mixture was allowed to react at 120°C for 8 hours. When TLC indicated a completed reaction, the mixture was filtered by suction, and the filtrate was concentrated under reduced pressure at 40°C. The residue was separated and purified by reverse phase column chromatography, eluted with water containing 0.05% formic acid: acetonitrile, to provide the title compound: 4-((4-cyclopropyl-2-(N-methyl methanesulfonamido)phenyl)amino)-N-ethoxy-6-((5-fluoro-6-methyl pyridin-2-yl)amino)nicotinamide (**216,** 22mg, 0.041mmol, 18.33% yield). MS Calcd:528.20; MS Found: 529.32([M+H]$^+$). $^1$H NMR (400 MHz, DMSO-$d_6$)$\delta$11.52 (s, 1H), 9.84 (s, 1H), 9.71 (s, 1H), 8.28 (s, 1H), 7.75 (s, 1H), 7.49 (t, *J* = 8.8 Hz, 1H), 7.44 (d, *J* = 8.4 Hz, 1H), 7.30 (d, *J* = 2.4 Hz, 1H), 7.24 (dd, *J* = 8.8, 3.2 Hz, 1H), 7.11 (dd, *J* = 8.4, 2.4 Hz, 1H), 3.93 (q, *J* = 7.2 Hz, 2H), 3.12 (s, 3H), 3.09 (s, 3H), 2.21 (d, *J* = 3.0 Hz, 3H), 2.04 - 1.79 (m, 1H), 1.22 (t, *J* = 7.2 Hz, 3H), 1.04 - 0.94 (m, 2H), 0.79 - 0.46 (m, 2H).

Example **217**

4-((4-cyclopropyl-2-(N-methyl methanesulfonamido)phenyl)amino)-N-ethoxy-6-((6-methyl pyridin-2-yl)amino)nicotinamide

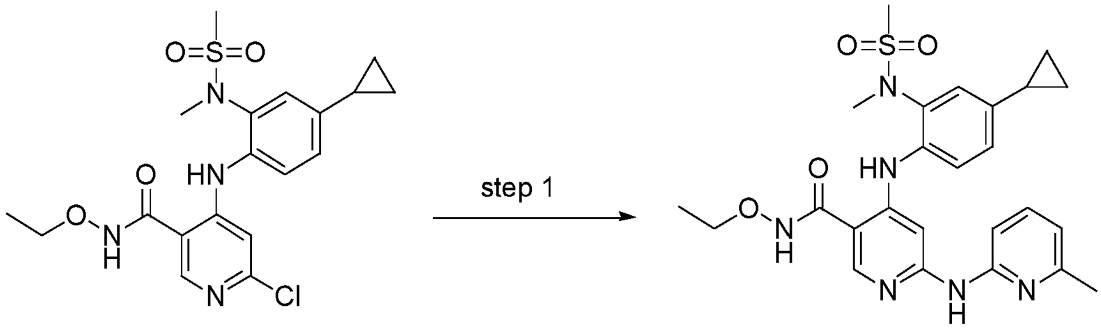


**20-a** **217**

Step 1: 6-chloro-4-((4-cyclopropyl-2-(N-methyl methanesulfonamido)phenyl)amino)-N-ethoxy nicotinamide (**20-a,** 100mg, 0.228mmol), 6-methyl pyridin-2-ylamine (49.27mg, 0.456mmol), $Pd_2(dba)_3$(41.75mg, 0.045mmol), Xant-Phos(53.23mg, 0.092mmol), $Cs_2CO_3$ (222.8mg, 0.684mmol), and 1,4-dioxane (3mL) were mixed. After atmosphere replacement with nitrogen three times, the mixture was allowed to react at 120°C for 8 hours. When TLC indicated a completed reaction, the mixture was filtered by suction, and the filtrate was concentrated under reduced pressure at 40°C, separated and purified by reverse phase column chromatography, eluted with water containing 0.05% formic acid: acetonitrile (the peak appeared at about 22% acetonitrile), to provide the title compound: 4-((4-cyclopropyl-2-(N-methyl methanesulfonamido)phenyl)amino)-N-ethoxy-6-((6-methyl pyridin-2-yl)amino)nicotinamide (**217,** 33mg, 0.064mmol, 30% yield). MS Calcd:510.20; MS Found: 511.23($[M+H]^+$). $^1H$ NMR (400 MHz, DMSO-$d_6$)δ11.52 (s, 1H), 9.85 (s, 1H), 9.63 (s, 1H), 8.29 (s, 1H), 7.92 (s, 1H), 7.53 - 7.39 (m, 2H), 7.30 (d, *J* = 2.0 Hz, 1H), 7.12-7.09 (m, 2H), 6.68 (d, *J* = 7.3 Hz, 1H), 3.94 (q, *J* = 7.2 Hz, 2H), 3.13 (s, 3H), 3.09 (s, 3H), 2.23 (s, 3H), 2.00-1.94 (m, 1H), 1.22 (t, *J* = 7.2 Hz, 3H), 1.07 - 0.90 (m, 2H), 0.70 (dt, *J* = 6.5, 3.2 Hz, 2H).

Example **218**

4-((3-(5-chloropyrimidin-2-yl)-2-methoxy phenyl)amino)-6-((2,6-dimethyl pyrimidin-4-yl)amino)-N-ethoxy nicotinamide

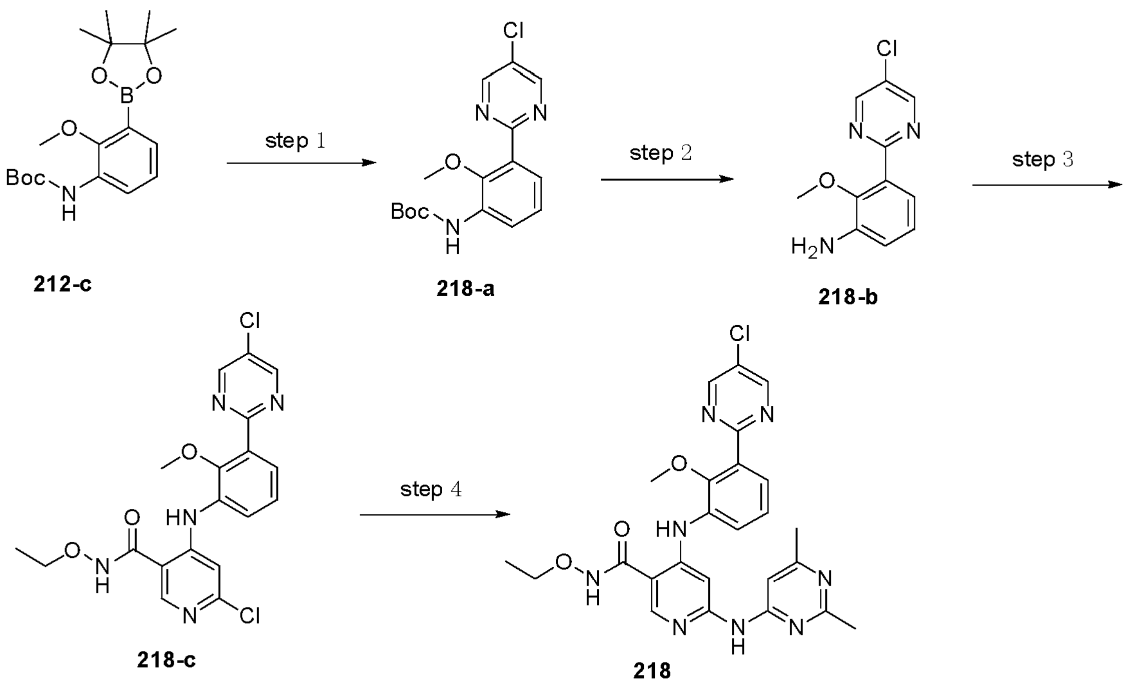


**212-c** **218-a** **218-b** **218-c** **218**

Step 1: To a 50ml flask were added t-butyl (2-methoxy-3-(4,4,5,5-tetramethyl-1,3,2-dioxaborolan-2-yl)phenyl) carbamate (**212-c,** 500mg, 1.886mmol), 2-bromo-5-chloropyrimidine (332.65mg, 1.715mmol), pd(dppf)$Cl_2$(139.53mg, 0.171mmol), potassium phosphate (1.201g, 5.65mmol), 1,4-dioxane (10ml) and water (2ml). After atmosphere replacement with nitrogen three times, the mixture was allowed to react at 80°C for 2 hours. Upon TLC indicated a completed

reaction, the mixture was concentrated under reduced pressure to dryness. The residue was separated and purified by column chromatography (PE:EA=6:1), to provide t-butyl (3-(5-chloropyrimidin-2-yl)-2-methoxy phenyl)carbamate (**218-a,** 453mg, 1.29mmol, 71.67% yield). MS Calcd: 335.10; MS Found: 336.09, 338.23([M+H]$^+$).

Step 2: To a 50ml flask was added t-butyl (3-(5-chloropyrimidin-2-yl)-2-methoxy phenyl)carbamate (**218-a,** 453mg, 1.29mmol), followed by dichloromethane (9ml) and trifluoroacetic acid (3ml). The mixture was allowed to react at room temperature for 2 hours. Upon TLC indicated a completed reaction, the mixture was concentrated under reduced pressure to dryness. The residue was added with water, neutralized with sodium carbonate solution, and extracted with ethyl acetate. The organic phase was dried over anhydrous sodium sulfate, concentrated under reduced pressure to dryness to provide 3-(5-chloropyrimidin-2-yl)-2-methoxy aniline(**218-b**, 380mg, 1.617mmol, 90% yield).

MS Calcd: 235.05; MS Found: 236.09, 238.23, 277.15([M+H]$^+$).

Step 3: To a 50ml two-necked flask were added 3-(5-chloropyrimidin-2-yl)-2-methoxy aniline(**218-b**, 350mg, 1.484mmol) and 4,6-dichloro-N-ethoxy nicotinamide (int-a, 694.5mg, 2.968mmol), followed by anhydrous tetrahydrofuran (8ml). After atmosphere replacement with nitrogen three times, the mixture was cooled in ice bath and added with LHMDS(4.45ml, 4.45mmol) slowly. After the addition, the mixture was allowed to naturally warm back to room temperature to react for 3 hours. When TLC indicated a completed reaction, the mixture was quenched with saturated ammonium chloride aqueous solution, and extracted with ethyl acetate. The organic phase was concentrated under reduced pressure to dryness and separated and purified by column chromatography (PE:EA=3:1), to provide 6-chloro-4-((3-(5-chloropyrimidin-2-yl)-2-methoxy phenyl)amino)-N-ethoxy nicotinamide (**218-c,** 337mg, 0.778mmol, 52.53% yield). MS Calcd: 433.07; MS Found: 435.23, 437.33([M+H]$^+$).

Step 4: To a 25ml flask were added 6-chloro-4-((3-(5-chloropyrimidin-2-yl)-2-methoxy phenyl)amino)-N-ethoxy nicotinamide (**218-c,** 150mg, 0.346mmol), 2,6-dimethyl pyrimidin-4-ylamine (46.93mg, 0.381mmol), $Pd(dba)_3$(63.18mg, 0.069mmol), xantphos(80.07mg, 0.138mmol), cesium carbonate (338.18mg, 1.038mmol), followed by 1,4-dioxane (3ml). After atmosphere replacement with nitrogen three times, the mixture was allowed to react at 120°C for 8 hours. Upon TLC indicated a completed reaction, the mixture was concentrated to dryness. The residue was separated and purified by reverse phase column chromatography (water containing 0.05% formic acid: acetonitrile, the peak appeared at 20% acetonitrile), followed by plate chromatography (DCM:MeOH=20:1), to provide the title compound: 4-((3-(5-chloropyrimidin-2-yl)-2-methoxy phenyl)amino)-6-((2,6-dimethyl pyrimidin-4-yl)amino)-N-ethoxy nicotinamide (**218,** 10mg, 0.019mmol, 5.49% yield). MS Calcd: 520.17; MS Found: 521.29([M+H]$^+$). $^1$H NMR (400 MHz, DMSO-$d_6$)δ11.72 (s, 1H), 10.24 (s, 1H), 10.11 (s, 1H), 9.09 (s, 2H), 8.40 (s, 1H), 8.17 (s, 1H), 7.75 (dd, $J$ = 8.0, 1.6 Hz, 1H), 7.50 (dd, $J$ = 8.0, 1.6 Hz, 1H), 7.33 (t, $J$ = 8.0 Hz, 1H), 7.09 (s, 1H), 3.96 (q, $J$ = 7.2 Hz, 2H), 3.71 (s, 3H), 2.38 (s, 3H), 2.28 (s, 3H), 1.23 (t, $J$ = 7.2 Hz, 3H).

Example **219**

4-((3-(5-chloropyrimidin-2-yl)-2-methoxy phenyl)amino)-6-(cyclopropylcarboxamido)-N-ethoxy nicotinamide

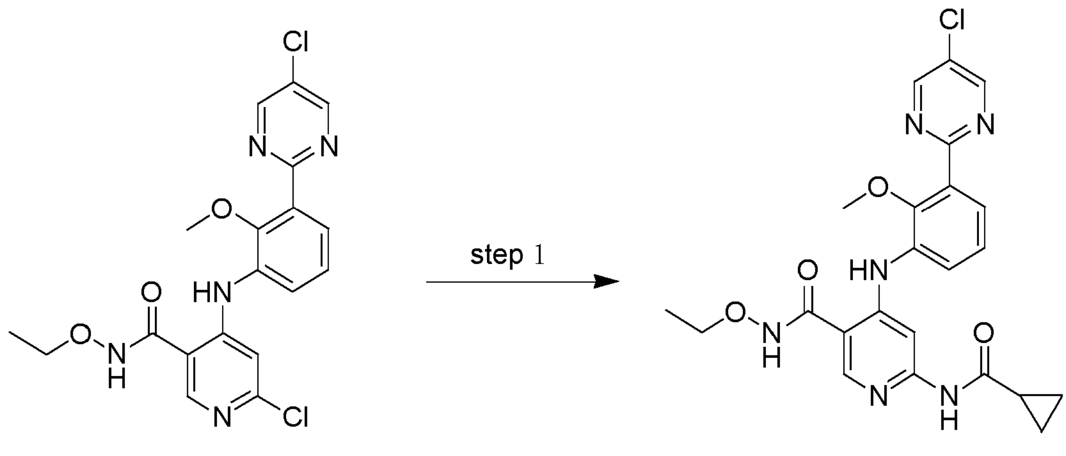


**218-c** **219**

Step 1: To a 25ml flask were added 6-chloro-4-((3-(5-chloropyrimidin-2-yl)-2-methoxy phenyl)amino)-N-ethoxy nicotinamide (**218-c,** 120mg, 0.230mmol), 2,6-dimethyl pyrimidin-4-ylamine (23.58mg, 0.230mmol), $Pd(dba)_3$(42.09mg, 0.046mmol), xantphos(53.23mg, 0.092mmol), cesium carbonate (224.80mg, 0.69mmol), and 1,4-dioxane (3ml). After

atmosphere replacement with nitrogen three times, the mixture was allowed to react at 120°C for 8 hours. Upon TLC indicated a completed reaction, the mixture was concentrated to dryness. The residue was separated and purified by reverse phase column chromatography (water containing 0.05% formic acid: acetonitrile, the peak appeared at 30% acetonitrile), followed by plate chromatography (DCM:MeOH=20:1), to provide the title compound: 4-((3-(5-chloropyrimidin-2-yl)-2-methoxy phenyl)amino)-6-(cyclopropylcarboxamido)-N-ethoxy nicotinamide (**219,** 13mg, 0.026mmol, 11.72% yield). MS Calcd: 482.15; MS Found: 483.19([M+H]$^+$). $^1$H NMR (400 MHz, DMSO-$d_6$)δ11.71 (s, 1H), 10.82 (s, 1H), 10.15 (s, 1H), 9.08 (s, 2H), 8.40 (s, 1H), 8.08 (s, 1H), 7.60 (dd, *J* = 8.0, 1.6 Hz, 1H), 7.48 (dd, *J* = 8.0, 1.6 Hz, 1H), 7.29 (t, *J* = 8.0 Hz, 1H), 3.96 (q, *J* = 7.2 Hz, 2H), 3.68 (s, 3H), 1.99 (t, *J* = 6.0 Hz, 1H), 1.24-1.21 (m, 2H), 0.79-0.76 (m, 2H).

Example **220**

4-((4-cyclopropyl-2-(N-methyl methanesulfonamido)phenyl)amino)-N-ethoxy-6-((6-(trifluoromethyl)pyridin-2-yl)amino) nicotinamide

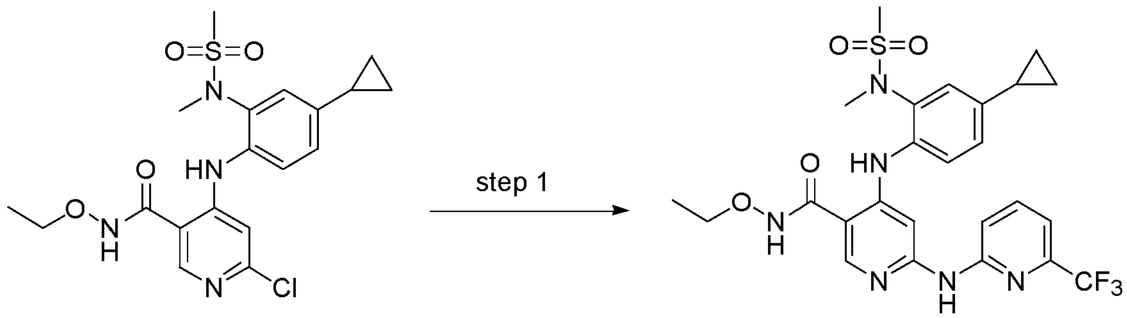


**20-a** **220**

Step 1: 6-chloro-4-((4-cyclopropyl-2-(N-methyl methanesulfonamido)phenyl)amino)-N-ethoxy nicotinamide (**20-a,** 150 mg, 0.34 mmol), 6-(trifluoromethyl)pyridin-2-ylamine (67 mg, 0.41 mmol), cesium carbonate (331 mg, 1.02 mmol), XantPhos(79 mg, 0.132 mmol) and $Pd_2(dba)_3$(62 mg, 0.068 mmol) were added to anhydrous dioxane (5 ml). After the atmosphere of the mixture was evacuated to vacuum and refilled with nitrogen, the reaction mixture was heated to 120°C with stirring for 8 hours, and filtered by suction. The filtrate was concentrated, purified by medium-pressure preparative chromatography (0.05% FA/MeCN) to provide the title compound: 4-((4-cyclopropyl-2-(N-methyl methanesulfonamido)phenyl)amino)-N-ethoxy-6-((6-(trifluoromethyl)pyridin-2-yl)amino)nicotinamide (**220,** 50 mg, 0.088 mmol, 26.2 % yield). MS Calcd:564.18; MS Found: 565.20 ([M+H]$^+$). $^1$H NMR (400 MHz, DMSO-$d_6$)δ11.59 (s, 1H), 10.12 (s, 1H), 9.86 (s, 1H), 8.32 (s, 1H), 7.86 (t, *J* = 8.0 Hz, 1H), 7.76 (d, *J* = 8.4 Hz, 1H), 7.53 (s, 1H), 7.38 (d, *J* = 8.4 Hz, 1H), 7.34 - 7.24 (m, 2H), 7.04 (dd, *J* = 8.4, 2.0 Hz, 1H), 3.94 (q, *J* = 7.2 Hz, 2H), 3.11 (s, 3H), 3.09 (s, 3H), 2.00-1.94 (m, 1H), 1.22 (t, *J* = 7.2 Hz, 3H), 1.04 - 0.95 (m, 2H), 0.75 - 0.66 (m, 2H).

Example **221**

4-((4-cyclopropyl-2-(N-methyl methanesulfonamido)phenyl)amino)-6-(((2,6-dimethyl pyridin-4-yl)amino)-N-ethoxy nicotinamide

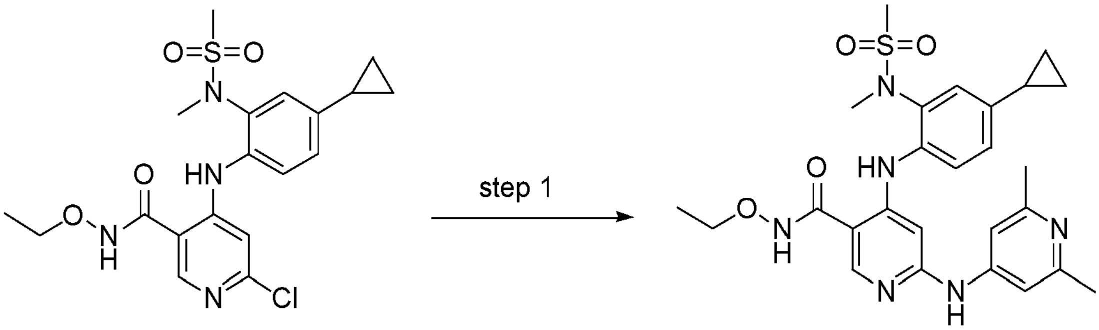


20-a 221

Step 1: 6-chloro-4-((4-cyclopropyl-2-(N-methyl methanesulfonamido)phenyl)amino)-N-ethoxy nicotinamide (**20-a,** 150 mg, 0.34 mmol), 2,6-dimethyl pyridin-4-ylamine (83 mg, 68 mmol), cesium carbonate (331 mg, 1.02 mmol), XantPhos(79 mg, 0.132 mmol) and $Pd_2(dba)_3$(62 mg, 0.068 mmol) were added to anhydrous dioxane (5 ml). After the atmosphere of the mixture was evacuated to vacuum and refilled with nitrogen, the reaction mixture was heated to 120°C with stirring for 8 hours, and filtered by suction. The filtrate was concentrated, purified by medium-pressure preparative chromatography (0.05% FA/MeCN) to provide the title compound: 4-((4-cyclopropyl-2-(N-methyl methanesulfonamido) phenyl)amino)-6-(((2,6-dimethyl pyridin-4-yl)amino)-N-ethoxy nicotinamide (**221,** 100 mg, 0.19 mmol, 56.1 % yield). MS Calcd:524.22; MS Found: 525.19 ($[M+H]^+$). $^1H$ NMR (400 MHz, DMSO-$d_6$)δ11.56 (s, 1H), 9.64 (s, 1H), 9.34 (s, 1H), 8.33 (s, 1H), 7.38 (d, *J* = 8.4 Hz, 1H), 7.30 (d, *J* = 2.0 Hz, 1H), 7.26 (s, 2H), 7.11 (dd, *J* = 8.4, 2.0 Hz, 1H), 6.38 (s, 1H), 3.93 (d, *J* = 7.2 Hz, 2H), 3.13 (s, 3H), 3.08 (s, 3H), 2.31 (s, 6H), 2.01-1.95 (m, 1H), 1.22 (t, *J* = 7.2 Hz, 3H), 1.03 - 0.96 (m, 2H), 0.77 - 0.68 (m, 2H).

Example **222**

N-ethoxy-4-((2-methoxy-4-(1-methyl-1H-pyrazol-5-yl)phenyl)amino)-6-((6-methyl pyridin-2-yl)amino)nicotinamide

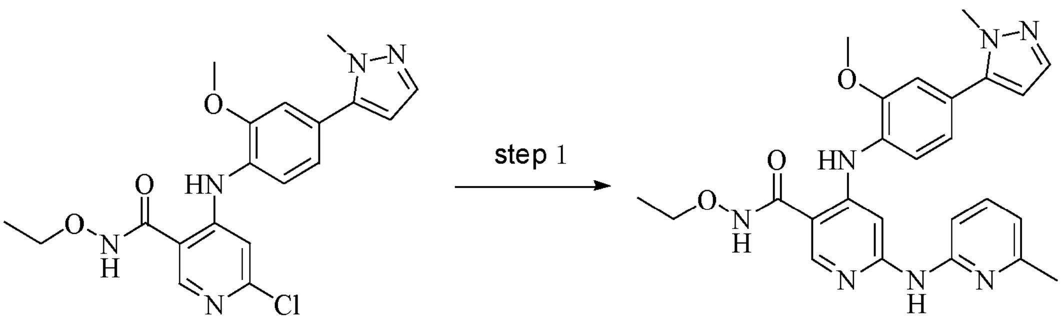


207-d 222

Step 1: 6-chloro-N-ethoxy-4-((2-methoxy-4-(1-methyl-1H-pyrazol-5-yl)phenyl)amino)nicotinamide (**207-d,** 135 mg, 0.33 mmol), 6-methyl pyridin-2-ylamine (73 mg, 0.67mmol), cesium carbonate (322 mg, 0.99 mmol), XantPhos(76 mg, 0.132 mmol) and $Pd_2(dba)_3$(60 mg, 0.067 mmol) were added to anhydrous dioxane (5 ml). After the atmosphere of the mixture was evacuated to vacuum and refilled with nitrogen, the reaction mixture was heated to 120°C with stirring for 8 hours, and filtered by suction. The filtrate was concentrated, purified by medium-pressure preparative chromatography (MeOH/DCM=1/20) to provide the title compound: N-ethoxy-4-((2-methoxy-4-(1-methyl-1H-pyrazol-5-yl)phenyl)amino)-6-((6-methyl pyridin-2-yl)amino)nicotinamide (**222,** 50 mg, 0.105 mmol, 32 % yield). MS Calcd:473.22; MS Found: 474.21 ($[M+H]^+$). $^1H$ NMR (400 MHz, DMSO-$d_6$)δ11.61 (s, 1H), 10.14 (s, 1H), 9.75 (s, 1H), 7.67 (d, *J* = 8.4 Hz, 1H), 7.51 (t, *J* = 7.8 Hz, 1H), 7.47 (d, *J*= 2.0 Hz, 1H), 7.32 - 7.24 (m, 1H), 7.22 (d, *J* = 2.0 Hz, 1H), 7.18 - 7.16 (m, 1H), 7.15 - 7.08 (m, 1H), 6.71 (d, *J* = 7.2 Hz, 1H), 6.41 (d, *J* = 2.0 Hz, 1H), 5.88 (s, 1H), 3.95 (d, *J* = 7.2 Hz, 2H), 3.93 (s, 3H), 3.90 (s, 3H), 2.33 (s, 3H), 1.24 (d, *J* = 7.2 Hz, 3H).

Example **223**

4-((4-cyclopropyl-2-(N-methyl methanesulfonamido)phenyl)amino)-N-ethoxy-6-((6-fluoro-2-methyl pyridin-3-yl)amino) nicotinamide

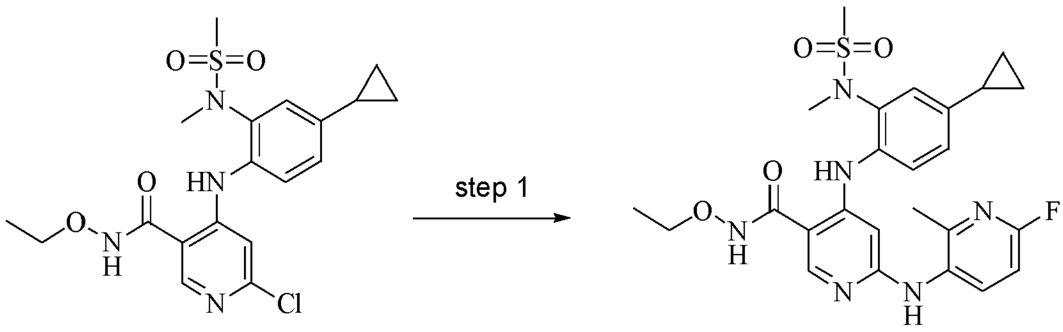

**20-a** **223**

Step 1: 6-chloro-4-((4-cyclopropyl-2-(N-methyl methanesulfonamido)phenyl)amino)-N-ethoxy nicotinamide (**20-a,** 150 mg, 0.34 mmol), 6-fluoro-2-methyl pyridin-3-ylamine (86 mg, 0.68 mmol), cesium carbonate (331 mg, 1.02 mmol), XantPhos(79 mg, 0.132 mmol) and $Pd_2(dba)_3$(62 mg, 0.068 mmol) were added to anhydrous dioxane (5 ml). After the atmosphere of the mixture was evacuated to vacuum and refilled with nitrogen, the reaction mixture was heated to 120°C with stirring for 8 hours, and filtered by suction. The filtrate was concentrated, purified by medium-pressure preparative chromatography (0.05% FA/MeOH) to provide the title compound: 4-((4-cyclopropyl-2-(N-methyl methanesulfonamido)phenyl)amino)-N-ethoxy-6-((6-fluoro-2-methyl pyridin-3-yl)amino)nicotinamide (**223,** 10 mg, 0.018 mmol, 6 % yield). MS Calcd:528.20; MS Found:529.20 ($[M+H]^+$). $^1H$ NMR (400 MHz, DMSO-$d_6$)δ11.48 (s, 1H), 9.71 (s, 1H), 8.47 (s, 1H), 8.15 (s, 1H), 8.04 (t, *J* = 8.4 Hz, 1H), 7.37 (d, *J* = 8.4 Hz, 1H), 7.27 (d, *J* = 2.0 Hz, 1H), 7.09 (dd, *J* = 8.4, 2.0 Hz, 1H), 6.95 (dd, *J* = 8.4, 3.2 Hz, 1H), 6.32 (s, 1H), 3.91 (q, *J* = 7.2 Hz, 2H), 3.13 (s, 3H), 3.09 (s, 3H), 2.31 (s, 3H), 2.09 - 1.86 (m, 1H), 1.20 (t, *J* = 7.2 Hz, 3H), 1.01-0.96 (m, 2H), 0.77 - 0.65 (m, 2H).

Example **224**

4-((4-cyclopropyl-2-(N-methyl methanesulfonamido)phenyl)amino)-N-ethoxy-6-((6-methyl pyridin-2-yl)amino)nicotinamide

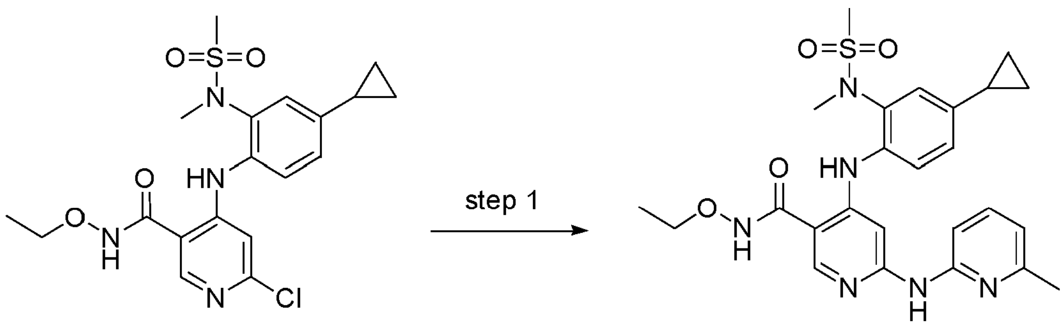

**20-a** **224**

Step 1: 6-chloro-4-((4-cyclopropyl-2-(N-methyl methanesulfonamido)phenyl)amino)-N-ethoxy nicotinamide (**20-a,** 150 mg, 0.34 mmol), 6-methyl pyridin-2-ylamine (74 mg, 0.68 mmol), cesium carbonate (331 mg, 1.02 mmol), XantPhos(79 mg, 0.132 mmol) and $Pd_2(dba)_3$(62 mg, 0.068 mmol) were added to anhydrous dioxane (5 ml). After the atmosphere of the mixture was evacuated to vacuum and refilled with nitrogen, the mixture was heated with microwave at 140 °C for 3 hours, stirred for 8 hours, and filtered by suction. The filtrate was concentrated, purified by medium-pressure preparative chromatography (0.05% FA/MeOH) to provide the title compound: 4-((4-cyclopropyl-2-(N-methyl methanesulfonamido)phenyl)amino)-N-ethoxy-6-((6-methyl pyridin-2-yl)amino)nicotinamide (**224,** 61 mg, 0.119 mmol, 35.2 % yield). MS Calcd:510.20; MS Found:511.19 ($[M+H]^+$). $^1H$ NMR (400 MHz, DMSO-$d_6$)δ11.53 (s, 1H), 9.86 (s, 1H), 9.65 (s,

1H), 8.29 (s, 1H), 7.93 (s, 1H), 7.50 - 7.45 (m, 2H), 7.30 (d, *J* = 2.0 Hz, 1H), 7.11 (d, *J* = 8.0 Hz, 2H), 6.70 (d, *J* = 7.2 Hz, 1H), 3.94 (q, *J* = 7.2 Hz, 2H), 3.13 (s, 3H), 3.10 (s, 3H), 2.23 (s, 3H), 2.03 - 1.92 (m, 1H), 1.22 (t, *J* = 7.2 Hz, 3H), 1.03 - 0.95 (m, 2H), 0.72-0.68 (m, 2H).

Example **225**

6-((2,6-dimethyl pyrimidin-4-yl)amino)-N-ethoxy-4-((5-fluoro-2-methoxy-3-(5-methyl pyrimidin-2-yl)phenyl)amino)nicotinamide

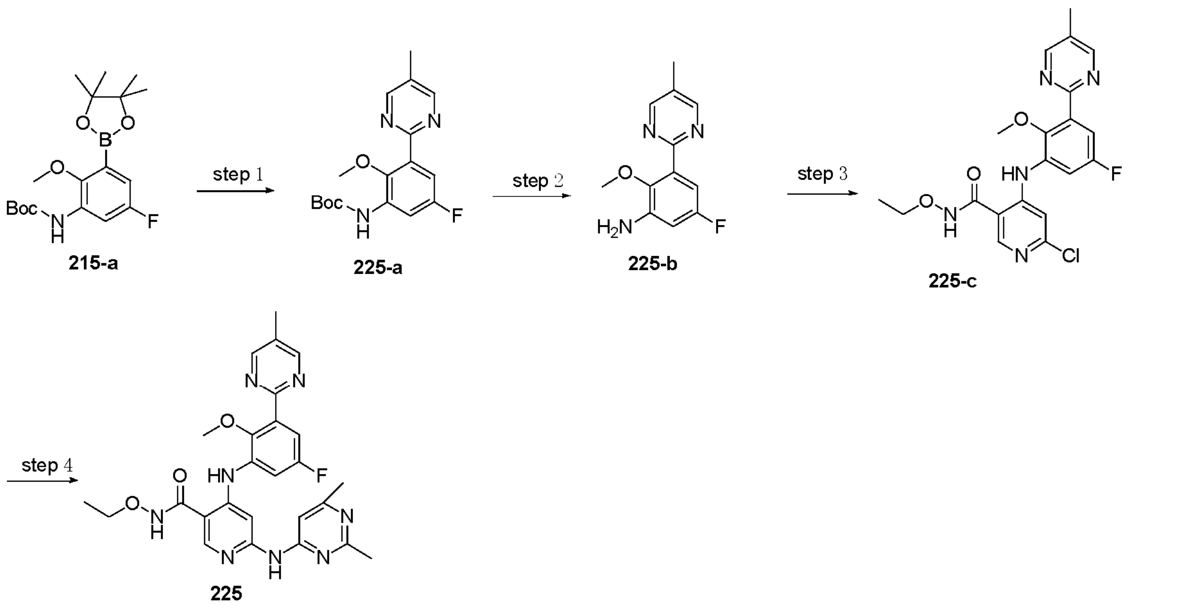

215-a
225-a
225-b
225-c
225

Step 1: t-butyl (5-fluoro-2-methoxy-3-(4,4,5,5-tetramethyl-1,3,2-dioxaborolan-2-yl)phenyl)carbamate (**215-a,** 650mg, 1.77mmol), 2-chloro-5-methyl pyrimidine (226mg, 1.77mmol), potassium phosphate (1.13g, 5.31mmol) and $Pd(dppf)Cl_2$(147mg, 0.18mmol) were added to a mixed solvent of dioxane and water (5ml: 1ml). After the atmosphere of the mixture was evacuated to vacuum and refilled with nitrogen, the reaction mixture was heated to 120°C with stirring for 3 hours, and filtered by suction. The filtrate was concentrated and purified by column chromatography (ethyl acetate:petroleum ether=1:1) to provide t-butyl (5-fluoro-2-methoxy-3-(5-methyl pyrimidin-2-yl)phenyl)carbamate (**225-a,** 450mg, 1.35mmol, 56% yield). MS Calcd: 333.15; MS Found: 334.21($[M+H]^+$).

Step 2: t-butyl (5-fluoro-2-methoxy-3-(5-methyl pyrimidin-2-yl)phenyl)carbamate (**225-a,** 450mg, 1.35mmol) was added to DCM(5ml), followed by solution of HCl in dioxane (4N, 5ml). The mixture was stirred at room temperature for 3 hours, concentrated under reduced pressure. The residue was added with saturated sodium bicarbonate aqueous solution (20ml), and extracted with ethyl acetate (20ml x3). The combined ethyl acetate layers were dried over anhydrous sodium sulfate, concentrated under reduced pressure to provide 5-fluoro-2-methoxy-3-(5-methyl pyrimidin-2-yl)aniline (**225-b,** 300mg, 1.28mmol, 95% yield). MS Calcd: 233.10; MS Found: 234.11($[M+H]^+$).

Step 3: 5-fluoro-2-methoxy-3-(5-methyl pyrimidin-2-yl)aniline (**225-b,** 300mg, 1.28mmol) and 4,6-dichloro-N-ethoxy nicotinamide (304mg, 1.30mmol) were added to 4ml of anhydrous tetrahydrofuran. The mixture was cooled to 0°C and added with a solution of LiHMDS in tetrahydrofuran (4ml, 4mmol), followed by warming slowly back to room temperature and stirring at room temperature for 6 hours. The mixture was adjusted with aqueous hydrochloride (1N) to pH 5, and extracted with ethyl acetate (10ml×3). The combined organic layers were dried, concentrated and then purified by column chromatography (petroleum ether: ethyl acetate =1:1) to provide 6-chloro-N-ethoxy-4-((5-fluoro-2-methoxy-3-(5-methyl pyrimidin-2-yl)phenyl)amino)nicotinamide (**225-c**, 260mg, 0.60mmol, 31% yield). MS Calcd: 431.12; MS Found: 432.21 ($[M+H]^+$).

Step 4: 6-chloro-N-ethoxy-4-((5-fluoro-2-methoxy-3-(5-methyl pyrimidin-2-yl)phenyl)amino)nicotinamide (**225-c,** 100mg, 0.23mmol), 2,6-dimethyl pyrimidin-4-ylamine (42mg, 0.35mmol), cesium carbonate (224mg, 0.69mmol), XantPhos(23mg, 0.04mmol) and $Pd_2(dba)_3$(18mg, 0.02mmol) were added to anhydrous dioxane (2ml). After the atmosphere of the mixture was evacuated to vacuum and refilled with nitrogen, the mixture was heated in microwave reactor to 140°C, followed by stirring for 3 hours, and filtered by suction. The filtrate was concentrated and purified by high performance preparative thin layer chromatography (MeOH:DCM=1:10) to provide the title compound: 6-((2,6-dimethyl

pyrimidin-4-yl)amino)-N-ethoxy-4-((5-fluoro-2-methoxy-3-(5-methyl pyrimidin-2-yl)phenyl)amino)nicotinamide (**225,** 40mg, 0.077mmol, 33% yield). MS Calcd: 518.22; MS Found: 519.24 ([M+H]+). $^1$H NMR (400 MHz, DMSO-$d_6$)δ11.76 (s, 1H), 10.43 (s, 1H), 10.22 (s, 1H), 8.82 (s, 2H), 8.42 (s, 1H), 8.19 (s, 1H), 7.56 (dd, *J* = 10.0, 3.2 Hz, 1H), 7.23-1.97 (m, 2H), 3.95 (q, *J* = 7.2 Hz, 2H), 3.69 (s, 3H), 2.42 (s, 3H), 2.35 (s, 3H), 2.31 (s, 3H), 1.22 (t, *J* = 7.2 Hz, 3H).

Example **226**

6-(cyclopropylcarboxamido)-N-ethoxy-4-((5-fluoro-2-(methoxy)-3-(5-methyl pyrimidin-2-yl)phenyl)amino)nicotinamide

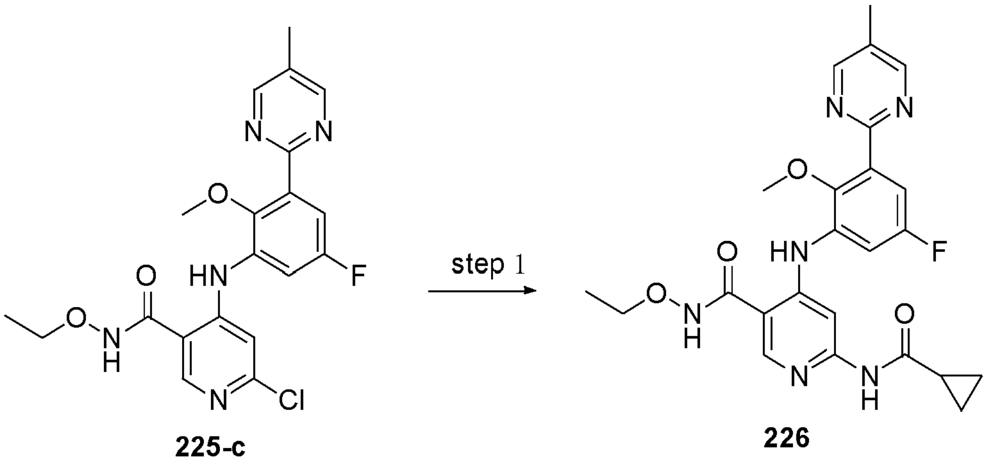


**225-c** **226**

Step 1: 6-chloro-N-ethoxy-4-((5-fluoro-2-methoxy-3-(5-methyl pyrimidin-2-yl)phenyl)amino)nicotinamide (**225-c,** 60mg, 0.14mmol), cyclopropylcarboxamide (24mg, 0.28mmol), cesium carbonate (137mg, 0.42mmol), Xant-Phos(11mg, 0.02mmol) and $Pd_2(dba)_3$(11mg, 0.01mmol) were added to anhydrous dioxane (1ml). After the atmosphere of the mixture was evacuated to vacuum and refilled with nitrogen, the mixture was heated in microwave reactor to 140°C, followed by stirring for 3 hours, and filtered by suction. The filtrate was concentrated and purified by high performance preparative thin layer chromatography (MeOH:DCM=1:10) to provide the title compound: 6-(cyclopropylcarboxamido)-N-ethoxy-4-((5-fluoro-2-(methoxy)-3-(5-methyl pyrimidin-2-yl)phenyl)amino)nicotinamide (**226,** 24mg, 0.05mmol, 36% yield). MS Calcd: 480.19; MS Found: 481.21 ([M+H]+). $^1$H NMR (400 MHz, DMSO-$d_6$)δ11.78 (s, 1H), 10.88 (s, 1H), 10.36 (s, 1H), 8.81 (d, *J* = 0.8 Hz, 2H), 8.42 (s, 1H), 8.17 (s, 1H), 7.41 (dd, *J* = 10.0, 3.2 Hz, 1H), 7.20 (dd, *J* = 9.2, 3.2 Hz, 1H), 3.94 (q, *J* = 7.2 Hz, 2H), 3.67 (s, 3H), 2.35 (s, 3H), 2.08 - 1.94 (m, 1H), 1.21 (t, *J* = 7.2 Hz, 3H), 0.94 - 0.75 (m, 2H), 0.70 - 0.47 (m, 2H).

Example **227**

N-ethoxy-6-((6-fluoro-2-methyl pyridin-3-yl)amino)-4-((5-fluoro-4-isopropyl-2-(N-methyl methanesulfonamido)phenyl) amino)nicotinamide

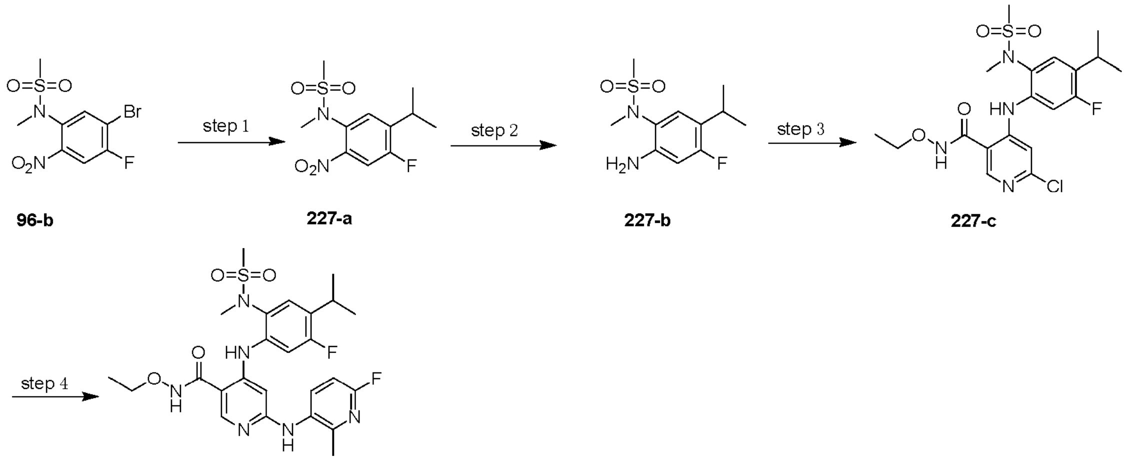


96-b 227-a 227-b 227-c

227

Step 1: To a 250ml flask were added N-(5-bromo-4-fluoro-2-nitrophenyl)-N-methyl methanesulfonamide (**96-b,** 6g, 18.34mmol), 4,4,5,5-tetramethyl-2-(prop-1-en-2-yl)-1,3,2-dioxaborolane (4.6g, 27.51mmol), Pd(dppf)$Cl_2$(1.3g, 1.8mmol), and potassium phosphate (11.7g, 55mmol), followed by 1,4-dioxane (90ml) and water (15ml). After atmosphere replacement with nitrogen three times, the mixture was allowed to react at 110°C for 6 hours. Upon TLC indicated a completed reaction, the mixture was concentrated under reduced pressure to dryness. The residue was mixed with silica gel for loading onto and purifying by column chromatography (petroleum ether:ethyl acetate=5:1), to provide N-(4-fluoro-5-isopropyl-2-nitrophenyl)-N-methyl methanesulfonamide (**227-a,** 4.3g, 13.7mmol, 74.8% yield).

Step 2: To a 250ml flask were added N-(4-fluoro-5-isopropyl-2-nitrophenyl)-N-methyl methanesulfonamide (**227-a,** 4.3g, 13.7mmol) and 10% palladium on carbon (0.5g), followed by methanol (80ml). After atmosphere replacement with hydrogen three times, the mixture was allowed to react at room temperature for 4 hours. Upon TLC indicated a completed reaction, the mixture was filtered by suction. The filtrate was concentrated under reduced pressure to provide N-[2-amino-4-fluoro-5-isopropyl phenyl]-N-methyl methanesulfonamide (**227-b,** 3.3g, 11.4mmol, 76.2% yield). MS Calcd: 260.3; MS Found: 261.71 ($[M+H]^+$).

Step 3: To a 100ml two-necked flask were added N-[2-amino-4-fluoro-5-isopropyl phenyl]-N-methyl methanesulfonamide (**227-b,** 2.6g, 9.99mmol) and 4,6-dichloro-N-ethoxy nicotinamide (**int-2,** 3.5g, 14.98mmol), followed by anhydrous DMA(40ml). After atmosphere replacement with nitrogen three times, the mixture was cooled in ice bath and added slowly with LiHMDS(39.95ml, 40mmol). After the addition, the mixture was removed from ice water bath and allowed to react at room temperature for 2 hours. Upon TLC indicated a completed reaction, the mixture was quenched with saturated ammonium chloride aqueous solution, and extracted with EA. The organic phase was back washed with water twice, and concentrated under reduced pressure to dryness. The residue was mixed with silica gel for loading onto and purifying by column chromatography (petroleum ether:ethyl acetate=1:1), to provide 6-chloro-N-ethoxy-4-((5-fluoro-4-isopropyl-2-(N-methyl methanesulfonamido)phenyl)amino)nicotinamide (**227-c,** 2.98g, 58mmol, 58.3% yield). MS Calcd: 458.9; MS Found: 459.24 ($[M+H]^+$).

Step 4: 6-chloro-N-ethoxy-4-((5-fluoro-4-isopropyl-2-(N-methyl methanesulfonamido)phenyl)amino)nicotinamide (**227-c,** 120mg, 0.26mmol), 6-fluoro-2-methyl pyridin-3-ylamine (49.5mg, 39mmol), $pd_2(dba)_3$ (47.6mg, 0.052mmol), XantPhos(60.2mg, 0.109mmol), $Cs_2CO_3$ (254.1mg, 0.8mmol) were mixed and added with 1,4-dioxane (2ml). After atmosphere replacement with nitrogen three times, the mixture was heated with microwave to 140°C and allowed to react for 3.5 hours. When TLC indicated a completed reaction, the mixture was filtered by suction, and the filtrate was concentrated under reduced pressure at 40°C, separated and purified by high performance preparative thin layer chromatography, eluted with DCM:MeOH=20:1, to provide the title compound: N-ethoxy-6-((6-fluoro-2-methyl pyridin-3-yl)amino)-4-((5-fluoro-4-isopropyl-2-(N-methyl methanesulfonamido)phenyl)amino)nicotinamide (**227,** 63mg, 0.114mmol, 43.9% yield). MS Calcd: 548.6; MS Found: 549.18 ($[M+H]^+$). $^1$H NMR (400 MHz, DMSO-$d_6$)δ11.53 (s, 1H), 9.97 (s, 1H), 8.58 (s, 1H), 8.18 (s, 1H), 8.07 (t, *J*=8.0 Hz, 1H), 7.46 (d, *J* = 8.0 Hz, 1H), 7.31 (d, *J* = 12.0 Hz, 1H), 6.94 (dd, *J* = 8.4, 3.2 Hz, 1H), 6.56 (s, 1H), 3.91 (q, *J* = 7.2 Hz, 2H), 3.16 (s, 3H), 3.13-3.10 (m, 4H), 2.34 (s, 3H), 1.36 - 1.10 (m, 9H).

Example **228**

6-((3,5-difluoropyridin-2-yl)amino)-N-ethoxy-4-((5-fluoro-4-isopropyl-2-(N-methyl methanesulfonamido)phenyl)amino) nicotinamide

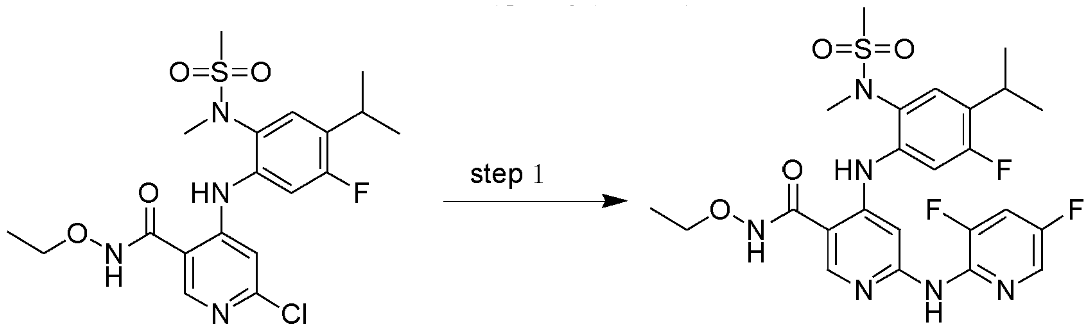


227-c 228

Step 1: 6-chloro-N-ethoxy-4-((5-fluoro-4-isopropyl-2-(N-methyl methanesulfonamido)phenyl)amino)nicotinamide (**227-c,** 120mg, 0.26mmol), 3,5-difluoropyridin-2-ylamine (51mg, 0.39mmol), $Pd_2(dba)_3$(47.6mg, 0.052mmol), XantPhos(60.2mg, 0.109mmol), $Cs_2CO_3$ (254.1mg, 0.8mmol) were mixed and added with 1,4-dioxane (2ml). After atmosphere replacement with nitrogen three times, the mixture was heated with microwave to 140°C and allowed to react for 3.5 hours. When TLC indicated a completed reaction, the mixture was filtered by suction, and the filtrate was concentrated under reduced pressure at 40°C. The residue was separated and purified by high performance preparative thin layer chromatography, eluted with DCM:MeOH=20:1, to provide the title compound: 6-((3,5-difluoropyridin-2-yl)amino)-N-ethoxy-4-((5-fluoro-4-isopropyl-2-(N-methyl methanesulfonamido)phenyl)amino)nicotinamide (**228,** 59 mg, 0.106 mmol, 40.8% yield). MS Calcd: 552.18; MS Found: 553.20 ($[M+H]^+$). $^1H$ NMR (400 MHz, DMSO-$d_6$)δ11.63 (s, 1H), 10.12 (s, 1H), 9.42 (s, 1H), 8.29 (s, 1H), 8.06 (d, $J$ = 2.8 Hz, 1H), 7.96-7.91 (m, 1H), 7.57 (s, 1H), 7.47 (d, $J$ = 8.0 Hz, 1H), 7.40 (d, $J$ = 12.0 Hz, 1H), 3.93 (q, $J$ = 7.2 Hz, 2H), 3.19-3.15 (m, 4H), 3.13 (s, 3H), 1.43 - 1.13 (m, 9H).

Example **229.**

6-((2,6-dimethyl pyrimidin-4-yl)amino)-N-ethoxy-4-((5-fluoro-4-isopropyl-2-(N-methyl methanesulfonamido)phenyl)amino)nicotinamide

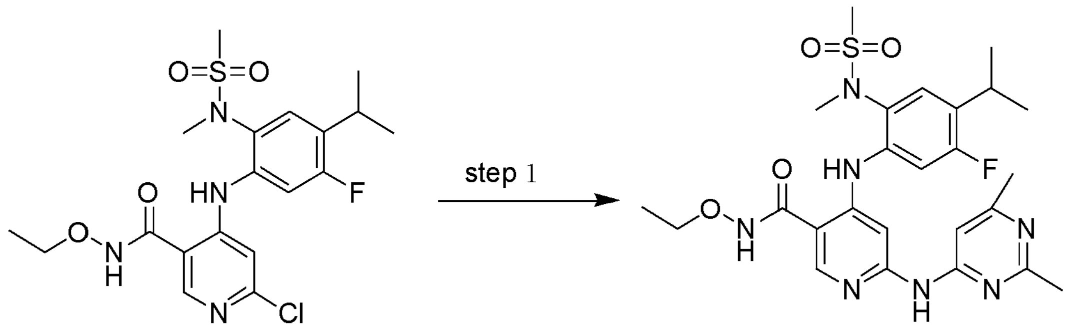


227-c 229

Step 1: 6-chloro-N-ethoxy-4-((5-fluoro-4-isopropyl-2-(N-methyl methanesulfonamido)phenyl)amino)nicotinamide (**227-c,** 120mg, 0.26mmol), 2,6-dimethyl pyrimidin-4-ylamino(48 mg, 0.39mmol), $Pd_2(dba)_3$(47.6mg, 0.052mmol), XantPhos(60.2mg, 0.109mmol), $Cs_2CO_3$ (254.1mg, 0.8mmol) were mixed and added with 1,4-dioxane (2ml). After atmosphere replacement with nitrogen three times, the mixture was heated with microwave to 140°C and allowed to react for 3.5 hours. When TLC indicated a completed reaction, the mixture was filtered by suction, and the filtrate was concentrated under reduced pressure at 40°C. The residue was separated and purified by high performance preparative thin layer chromatography, eluted with DCM:MeOH=20:1, to provide the title compound: 6-((2,6-dimethyl pyrimidin-4-yl)amino)-N-ethoxy-4-((5-fluoro-4-isopropyl-2-(N-methyl methanesulfonamido)phenyl)amino)nicotinamide (**229,** 86 mg, 0.158mmol, 60.6% yield). MS Calcd: 545.22; MS Found: 546.23 ($[M+H]^+$). $^1H$ NMR (400 MHz, DMSO-$d_6$)δ11.66 (s, 1H), 10.13 (s, 1H), 10.10 (s, 1H), 8.36 (s, 1H), 7.99 (s, 1H), 7.49 (d, $J$ = 8.0 Hz, 1H), 7.39 (d, $J$ = 12.0 Hz, 1H), 7.12 (s, 1H), 3.94 (q, $J$ = 7.2 Hz, 2H), 3.18-3.15 (m, 4H), 3.12 (s, 2H), 2.36 (s, 3H), 2.28 (s, 3H), 1.40 - 1.17 (m, 9H).

Example **230**

N-ethoxy-4-((5-fluoro-4-isopropyl-2-(N-methyl methanesulfonamido)phenyl)amino)-6-(pyrimidin-4-ylamino)nicotinamide

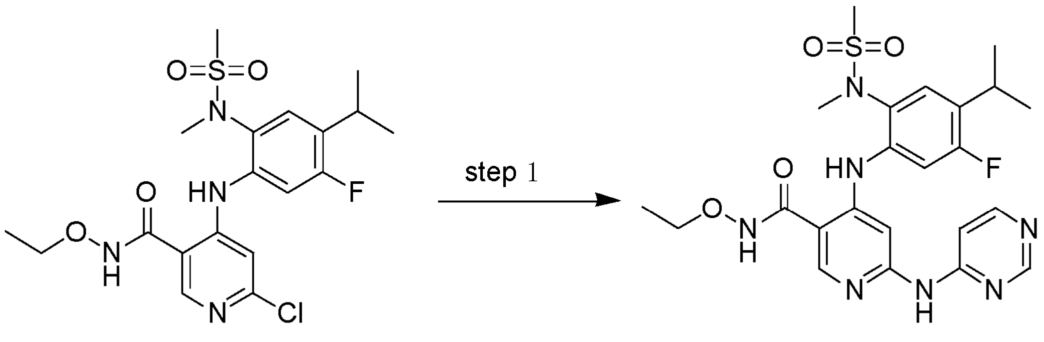


**227-c** **230**

Step 1: 6-chloro-N-ethoxy-4-((5-fluoro-4-isopropyl-2-(N-methyl methanesulfonamido)phenyl)amino)nicotinamide (**227-c,** 120mg, 0.26mmol), 4-aminopyrimidine (37 mg, 0.39mmol), $Pd_2(dba)_3$(47.6mg, 0.052mmol), XantPhos(60.2mg, 0.109mmol), $Cs_2CO_3$ (254.1mg, 0.8mmol) were mixed and added with 1,4-dioxane (2ml). After atmosphere replacement with nitrogen three times, the mixture was heated with microwave to 140°C and allowed to react for 3.5 hours. When TLC indicated a completed reaction, the mixture was filtered by suction, and the filtrate was concentrated under reduced pressure at 40°C. The residue was separated and purified by high performance preparative thin layer chromatography, eluted with DCM:MeOH=20:1, to provide the title compound: N-ethoxy-4-((5-fluoro-4-isopropyl-2-(N-methyl methanesulfonamido)phenyl)amino)-6-(pyrimidin-4-ylamino)nicotinamide (**230,** 72 mg, 0.139mmol, 53.7% yield). MS Calcd: 517.19; MS Found: 518.21 ($[M+H]^+$). $^1$H NMR (401 MHz, DMSO-$d_6$)δ11.69 (s, 1H), 10.22 (s, 1H), 10.10 (s, 1H), 8.51 (d, *J* = 4.8 Hz, 2H), 8.38 (s, 1H), 8.32 (s, 1H), 7.53-7.46 (m, 2H), 7.01 (t, *J* = 4.8 Hz, 1H), 3.95 (q, *J* = 7.2 Hz, 2H), 3.18-3.16 (m, 4H), 3.14 (s, 3H), 1.28 - 1.11 (m, 9H).

Example **231**

N-ethyl-4-((5-fluoro-2-methoxy-3-(pyrimidin-2-yl)phenyl)amino)-6-((6-fluoro-2-methyl pyridin-3-yl)amino)nicotinamide

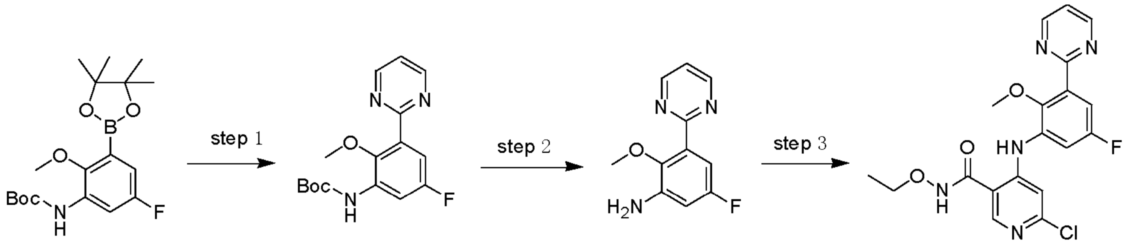


**215-a** **231-a** **231-b** **231-c**

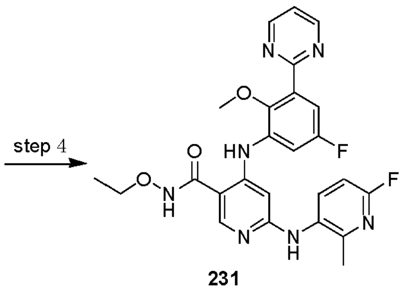


**231**

Step 1: To a 50ml flask were added t-butyl (5-fluoro-2-methoxy-3-(4,4,5,5-tetramethyl-1,3,2-dioxaborolan-2-yl) phenyl)carbamate (**215-a,** 300mg, 0.82mmol), 2-bromopyrimidine (169.5mg, 1.07mmol), pd(dppf)Cl2(59.9mg, 0.08mmol), potassium phosphate (522.3mg, 2.5mmol), followed by 1,4-dioxane (6ml) and water (1.5ml). After atmosphere replacement with nitrogen three times, the mixture was allowed to react at 80°C for 4 hours. When the reaction was found completed by sampling and testing, the mixture was concentrated under reduced pressure to dryness. The residue was separated and purified by column chromatography (petroleum ether:ethyl acetate=10:1~3:1), to provide t-butyl (5-fluoro-2-methoxy-3-(pyrimidin-2-yl)phenyl)carbamate (**231-a,** 216mg, 0.6mmol, 74.3% yield). MS Calcd: 319.13; MS Found:320.14.

Step 2: To a 25ml flask was added t-butyl (5-fluoro-2-methoxy-3-(pyrimidin-2-yl)phenyl)carbamate (**231-a,** 216mg, 0.6mmol), followed by DCM(3ml) and TFA(1ml). The mixture was stirred at room temperature for 2 hours. When the reaction was found completed by sampling and testing, the mixture was concentrated under reduced pressure to dryness. The residue was neutralized with saturated sodium carbonate solution, and extracted with ethyl acetate. The organic phase was dried and concentrated under reduced pressure to dryness to provide 5-fluoro-2-methoxy-3-(pyrimidin-2-yl)aniline (**231-b,** 122mg, 0.6mmol, 82.3% yield). MS Calcd: 219.08; MS Found:220.11, 261.13.

Step 3: To a 25ml two-necked flask were added 5-fluoro-2-methoxy-3-(pyrimidin-2-yl)aniline (**231-b,** 63mg, 0.29mmol) and 4,6-dichloro-N-ethoxy nicotinamide (**int-2,** 102mg, 0.43mmol), followed by anhydrous THF(3ml). After atmosphere replacement with nitrogen three times, the mixture was cooled in ice bath, added with LiHMDS(0.9mmol, 0.87ml), and allowed to react at room temperature for 2 hours. Upon TLC indicated a completed reaction, the mixture was quenched with saturated ammonium chloride solution, and extracted with EA. The organic phase was concentrated under reduced pressure to dryness and mixed with silica gel for loading onto and purifying by column chromatography (petroleum ether:ethyl acetate=1:1), to provide 6-chloro-N-ethoxy-4-((5-fluoro-2-methoxy-3-(pyrimidin-2-yl)phenyl)amino)nicotinamide **(231-c,** 66mg, 0.2mmol, 54.5% yield).

Step 4: 6-chloro-N-ethoxy-4-((5-fluoro-2-methoxy-3-(pyrimidin-2-yl)phenyl)amino)nicotinamide **(231-c,** 66mg, 0.2mmol), 6-fluoro-2-methyl pyridin-3-ylamine (30mg, 0.24mmol), $Pd_2(dba)_3$(18.5mg, 0.032mmol), XantPhos(58.6mg, 0.064mmol), $Cs_2CO_3$ (156.4mg, 0.5mmol) were mixed and added with 1,4-dioxane (1ml). After atmosphere replacement with nitrogen three times, the mixture was heated with microwave to 140°C and allowed to react for 3.5 hours. When TLC indicated a completed reaction, the mixture was filtered by suction, and the filtrate was concentrated under reduced pressure at 40°C, separated and purified by high performance preparative thin layer chromatography, eluted with DCM:MeOH=20:1, to provide the title compound: N-ethyl-4-((5-fluoro-2-methoxy-3-(pyrimidin-2-yl)phenyl)amino)-6-((6-fluoro-2-methyl pyridin-3-yl)amino)nicotinamide **(231,** 58mg, 0.114mmol, 71.4% yield). MS Calcd: 507.18; MS Found: 508.25 ($[M+H]^+$). $^1H$ NMR (400 MHz, DMSO-$d_6$)δ11.60 (s, 1H), 10.25 (s, 1H), 8.97 (d, *J* = 4.8 Hz, 2H), 8.65 (s, 1H), 8.22 (s, 1H), 8.09 (t, *J* = 8.4 Hz, 1H), 7.55 (t, *J* = 4.8 Hz, 1H), 7.47 (dd, *J* = 10.0, 3.2 Hz, 1H), 7.21 (dd, *J* = 9.2, 3.2 Hz, 1H), 6.96 (dd, *J* = 8.8, 3.2 Hz, 1H), 6.72 (s, 1H), 3.92 (q, *J* = 7.2 Hz, 2H), 3.70 (s, 3H), 2.36 (s, 3H), 1.20 (t, *J* = 7.2 Hz, 3H).

Example **232**

4-((4-cyclopropyl-5-fluoro-2-(N-methyl methanesulfonamido)phenyl)amino)-N-ethoxy-6-((6-fluoro-2-methyl pyridin-3-yl)amino)nicotinamide

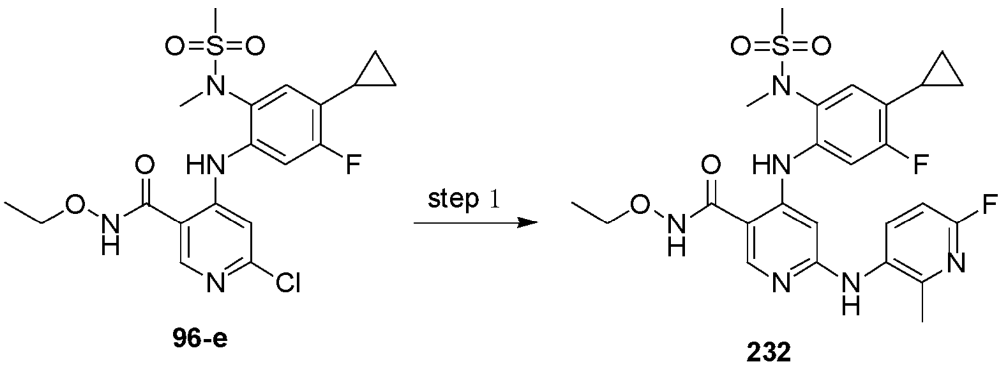


**96-e** **232**

Step 1: 6-chloro-4-((4-cyclopropyl-5-fluoro-2-(N-methyl methanesulfonamido)phenyl)amino)-N-ethoxy nicotinamide **(96-e,** 70mg, 0.15mmol), 3,5-difluoropyridin-2-ylamine (30mg, 0.23mmol), $Pd_2(dba)_3$(28.1mg, 0.032mmol), XantPhos (35.5mg, 0.064mmol), and $Cs_2CO_3$ (149.7mg, 0.5mmol) were mixed and added with 1,4-dioxane (1ml). After atmosphere replacement with nitrogen three times, the mixture was heated with microwave to 140°C and allowed to react

for 3.5 hours. When TLC indicated a completed reaction, the mixture was filtered by suction, and the filtrate was concentrated under reduced pressure at 40°C. The residue was separated and purified by plate chromatography, eluted with DCM:MeOH=20:1, to provide the title compound: 4-((4-cyclopropyl-5-fluoro-2-(N-methyl methanesulfonamido) phenyl)amino)-N-ethoxy-6-((6-fluoro-2-methyl pyridin-3-yl)amino)nicotinamide **(232,** 56mg, 0.11mmol, 66.4% yield). MS Calcd: 546.19; MS Found: 547.20 ($[M+H]^+$). $^1H$ NMR (400 MHz, DMSO-$d_6$)$\delta$11.51 (s, 1H), 9.94 (s, 1H), 8.55 (s, 1H), 8.17 (s, 1H), 8.07 (t, $J$ = 8.0 Hz, 1H), 7.30 (d, $J$ = 12.0 Hz, 1H), 7.15 (d, $J$ = 8.0 Hz, 1H), 6.94 (dd, $J$ = 8.4, 3.6 Hz, 1H), 6.51 (s, 1H), 3.91 (q, $J$ = 7.2 Hz, 2H), 3.12 (s, 3H), 3.09 (s, 3H), 2.34 (s, 3H), 2.04 - 1.97 (m, 1H), 1.20 (t, $J$ = 7.2 Hz, 3H), 1.00-0.95 (m, 2H), 0.83 - 0.64 (m, 2H).

Example **233**

N-ethoxy-6-((6-fluoro-2-methyl pyridin-3-yl)amino)-4-((5-fluoro-3-(5-fluoropyrimidin-2-yl)-2-methoxy phenyl)amino)nicotinamide

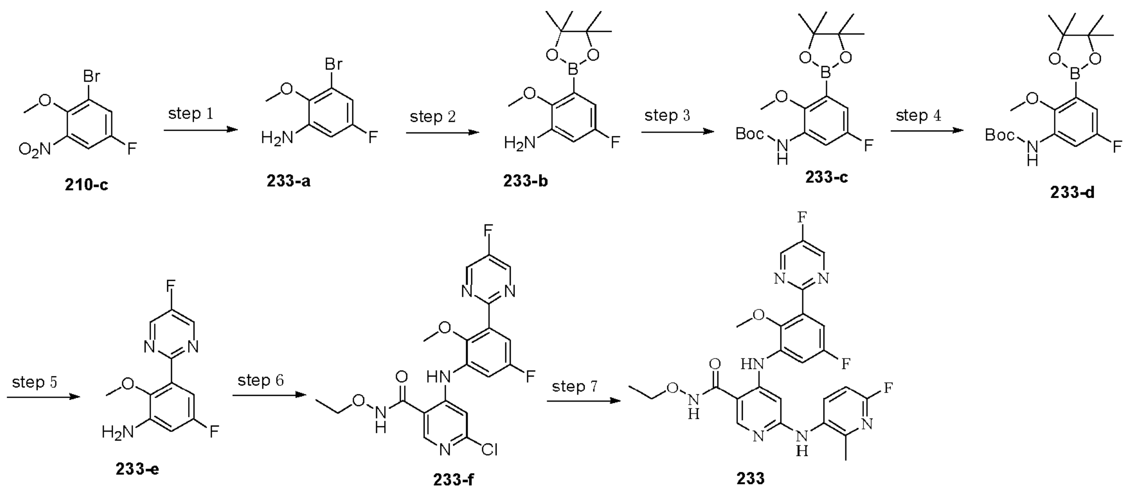

210-c 233-a 233-b 233-c 233-d 233-e 233-f 233

Step 1: To a 250ml flask were added 1-bromo-5-fluoro-2-methoxy-3-nitrobenzene(**210-c**, 4g, 18.26mmol), iron powder (5.11g, 91.32mmol), ammonium chloride (9.77g, 182.6mmol), followed by ethanol (100ml) and water (20ml). The mixture was allowed to react at 85°C for 4 hours. Upon TLC indicated a completed reaction, the mixture was filtered by suction, and the filtrate was added with water and extracted with ethyl acetate. The organic phase was dried over anhydrous sodium sulfate, concentrated under reduced pressure to dryness to provide 3-bromo-5-fluoro-2-methoxy aniline(**233-a**, 3.54g, 16.23mmol, 89% yield).

Step 2: To a 250ml flask were added 3-bromo-5-fluoro-2-methoxy aniline(**233-a**, 3.54g, 16.23mmol), bis(pinacolato)diboron (6.16g, 24.35mmol), potassium acetate (4.77g, 48.69mmol), Pd(dppf)·$CH_2Cl_2$(1.32g, 1.62mmol), followed by 1,4-dioxane (90ml). After atmosphere replacement with nitrogen three times, the mixture was allowed to react at 110°C for 8 hours. When TLC indicated that no further raw material reacted, the reaction mixture was mixed with silica gel for loading onto and separated by column chromatography(PE:EA=5:1), to provide 5-fluoro-2-methoxy-3-(4,4,5,5-tetramethyl-1,3,2-dioxaborolan-2-yl)aniline **(233-b,** 2.3g, 8.61mmol, 53.04%). MS Calcd: 267.14; MS Found:268.23.

Step 3: To a 100ml flask were added 2-(5-fluoro-2-methoxy-3-amino phenyl)-4,4,5,5-tetramethyl-1,3,2-dioxaborolane **(233-b,** 2.3g, 8.61mmol), and $(Boc)_2$(2.44g, 11.20mmol), followed by ethanol (20ml). The mixture was allowed to react at 60°C for 5 hours. Upon TLC indicated a completed reaction, the mixture was added with water and extracted with ethyl acetate. The organic phase was dried and concentrated under reduced pressure to dryness to provide t-butyl (5-fluoro-2-methoxy-3-(4,4,5,5-tetramethyl-1,3,2-dioxaborolan-2-yl)phenyl)carbamate **(233-c,** 2.5g, 6.81mmol, 80.67yield). MS Calcd: 367.20; MS Found:267.23, 312.33.

Step 4: To a 50ml flask were added t-butyl (5-fluoro-2-methoxy-3-(4,4,5,5-tetramethyl-1,3,2-dioxaborolan-2-yl) phenyl)carbamate **(233-c,** 300mg, 0.82mmol), 2-chloro-5-fluoropyrimidine (130mg, 0.98mmol), and potassium phosphate (544.1mg, 2.5mmol), followed by 1,4-dioxane (6ml) and water (1.5ml). After atmosphere replacement with nitrogen three times, the mixture was allowed to react at 110°C for 4 hours. When the reaction was found completed by sampling and testing, the mixture was concentrated under reduced pressure to dryness. The residue was separated and purified by column chromatography (PE:EA=10:1~3:1), to provide t-butyl N-[5-fluoro-3-(5-fluoropyrimidin-2-yl)-2-methoxy phenyl] carbamate **(233-d,** 226mg, 0.6mmol, 73.8% yield). MS Calcd: 337.12; MS Found:282.12

Step 5: To a 25ml flask was added t-butyl N-[5-fluoro-3-(5-fluoropyrimidin-2-yl)-2-methoxy phenyl]carbamate

**(233-d,** 226mg, 0.6mmol), followed by DCM(3ml) and TFA(1ml). The mixture was stirred at room temperature for 2 hours. When the reaction was found completed by sampling and testing, the mixture was concentrated under reduced pressure to dryness. The residue was neutralized with saturated sodium carbonate solution, and extracted with ethyl acetate. The organic phase was dried and concentrated under reduced pressure to dryness to provide 5-fluoro-3-(5-fluoropyrimidin-2-yl)-2-methoxy aniline(**233-e**, 122mg, 0.5mmol, 69.1% yield). MS Calcd: 237.07; MS Found:238.08, 279.08

Step 6: To a 25ml two-necked flask were added 5-fluoro-3-(5-fluoropyrimidin-2-yl)-2-methoxy aniline(**233-e**, 100mg, 0.42mmol) and 4,6-dichloro-N-ethoxy nicotinamide **(int-2,** 148.1mg, 0.63mmol), followed by anhydrous THF(3ml). After atmosphere replacement with nitrogen three times, the mixture was cooled in ice bath, added with LiHMDS(1.3mmol, 1.26ml), and allowed to react at room temperature for 2 hours. Upon TLC indicated a completed reaction, the mixture was quenched with saturated ammonium chloride solution, and extracted with EA. The organic phase was concentrated under reduced pressure to dryness and mixed with silica gel for loading onto and purifying by column chromatography (PE:EA=1:1), to provide 6-chloro-N-ethoxy-4-{[5-fluoro-3-(5-fluoropyrimidin-2-yl)-2-methoxy phenyl] amino}pyridine-3-carboxamide(**233-f**, 152mg, 0.3mmol, 74.8% yield).

Step 7: 6-chloro-N-ethoxy-4-{[5-fluoro-3-(5-fluoropyrimidin-2-yl)-2-methoxy phenyl]amino}pyridine-3-carboxamide(**233-f**, 82mg, 0.19mmol), 6-fluoro-2-methyl pyridin-3-ylamine (35.6mg, 0.28mmol), $Pd_2(dba)_3$(34.5mg, 0.038mmol), XantPhos(43.5mg, 0.076mmol), $Cs_2CO_3$ (183.9mg, 0.6mmol) were mixed and added with 1,4-dioxane (1ml). After atmosphere replacement with nitrogen three times, the mixture was heated with microwave to 140°C and allowed to react for 3.5 hours. When TLC indicated a completed reaction, the mixture was filtered by suction, and the filtrate was concentrated under reduced pressure at 40°C, separated and purified by plate chromatography, eluted with DCM:MeOH=20:1, to provide the target compound N-ethoxy-6-((6-fluoro-2-methyl pyridin-3-yl)amino)-4-((5-fluoro-3-(5-fluoropyrimidin-2-yl)-2-methoxy phenyl)amino)nicotinamide **(233,** 62mg, 0.117mmol, 62.7% yield). MS Calcd: 525.17; MS Found: 526.23 ($[M+H]^+$). $^1$H NMR (400 MHz, DMSO-$d_6$)δ11.61 (s, 1H), 10.26 (s, 1H), 9.07 (d, *J* = 0.8 Hz, 2H), 8.65 (s, 1H), 8.22 (s, 1H), 8.09 (t, *J* = 8.4 Hz, 1H), 7.48 (dd, *J* = 10.0, 3.2 Hz, 1H), 7.20 (dd, *J* = 9.2, 3.2 Hz, 1H), 6.96 (dd, *J* = 8.4, 3.2 Hz, 1H), 6.71 (s, 1H), 3.92 (q, *J* = 7.2 Hz, 2H), 3.69 (s, 3H), 2.36 (s, 3H), 1.20 (t, *J* = 72 Hz, 3H).

Example **234**

6-((3,5-difluoropyridin-2-yl)amino)-N-ethoxy-4-((5-fluoro-3-(5-fluoropyrimidin-2-yl)-2-methoxy phenyl)amino)nicotinamide

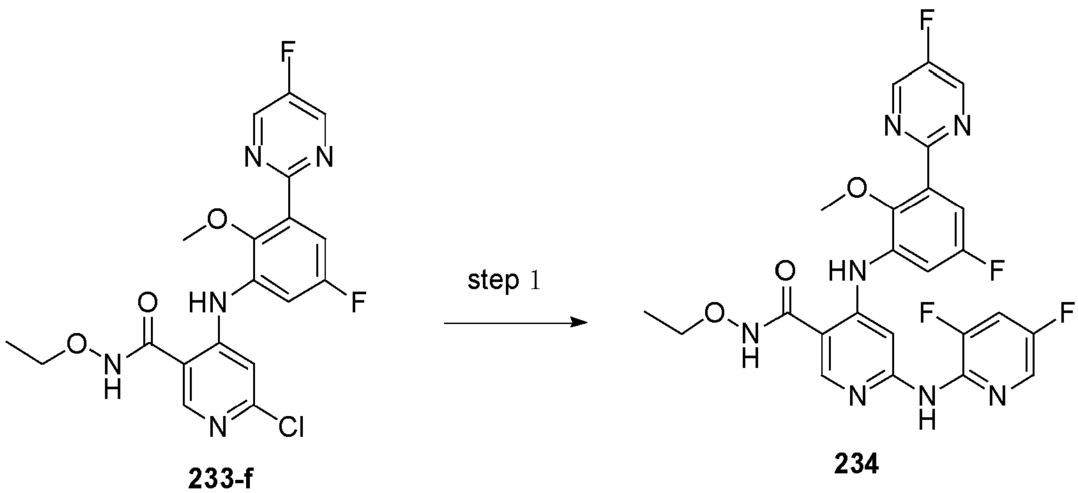


**233-f** **234**

Step 1: 6-chloro-N-ethoxy-4-{[5-fluoro-3-(5-fluoropyrimidin-2-yl)-2-methoxy phenyl]amino}pyridine-3-carboxamide(**233-f**, 70mg, 0.16mmol), 3,5-difluoropyridin-2-ylamine (31.3mg, 0.24mmol), pd2(dba)3 (29.4mg, 0.032mmol), xantphos(37.2mg, 0.064mmol), $Cs_2CO_3$ (157mg, 0.48mmol) were mixed and added with 1,4-dioxane (1ml). After atmosphere replacement with nitrogen three times, the mixture was heated with microwave to 140°C and allowed to react for 3.5 hours. When TLC indicated a completed reaction, the mixture was filtered by suction, and the filtrate was concentrated under reduced pressure at 40°C, separated and purified by plate chromatography, eluted with DCM:MeOH=20:1, to provide the target compound: 6-((3,5-difluoropyridin-2-yl)amino)-N-ethoxy-4-((5-fluoro-3-(5-fluoropyrimidin-2-yl)-2-methoxy phenyl)amino)nicotinamide **(234,** 58mg, 0.109mmol, 68.2% yield). MS Calcd: 529.15; MS Found: 530.18 ($[M+H]^+$). $^1$H NMR (400 MHz, DMSO-$d_6$)δ11.61 (s, 1H), 10.26 (s, 1H), 9.07 (d, *J* = 0.8 Hz, 2H), 8.65 (s, 1H),

8.22 (s, 1H), 8.09 (t, *J* = 8.0 Hz, 1H), 7.48 (dd, *J* = 10.0, 3.2 Hz, 1H), 7.20 (dd, *J* = 8.8, 3.2 Hz, 1H), 6.96 (dd, *J* = 8.8, 3.6 Hz, 1H), 6.71 (s, 1H), 3.92 (q, *J* = 7.2 Hz, 2H), 3.69 (s, 3H), 2.36 (s, 3H), 1.20 (t, *J* = 7.2 Hz, 3H).

Example **235**

4-((4-cyclopropyl-2-(N-methyl methanesulfonamido)phenyl)amino)-N-ethoxy-6-((6-fluoro-5-methyl pyridin-3-yl)amino) nicotinamide

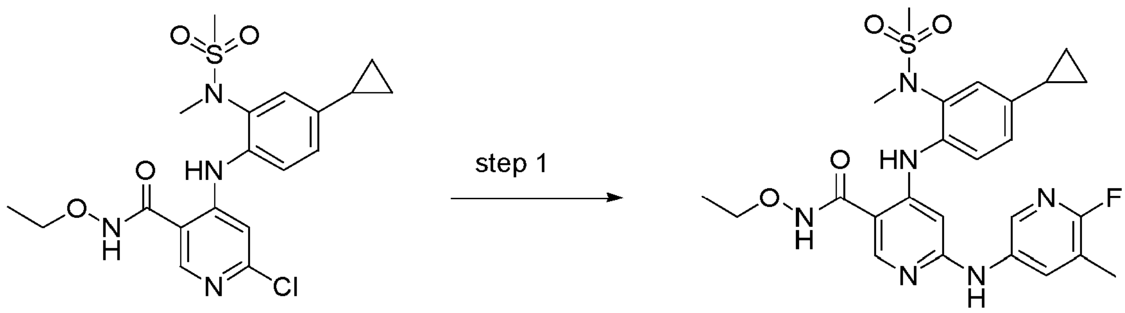


**20-a** **235**

Step 1: To a 10ml microwave tube were sequentially added 6-chloro-4-((4-cyclopropyl-2-(N-methyl methanesulfonamido)phenyl)amino)-N-ethoxy nicotinamide **(20-a,** 100mg, 0.23 mmol), 6-fluoro-5-methyl pyridin-3-ylamine (43.1 mg, 0.34mmol), $Pd_2(dba)_3$(20.9 mg, 0.023mmol), XantPhos(26.4 mg, 0.046mmol), $Cs_2CO_3$ (222.7mg, 0.69mmol) and 1,4-dioxane (4ml). After atmosphere replacement with nitrogen three times, the mixture was heated to 140°C with microwave and stirred for 3 hours under nitrogen protection. When LCMS indicated a completed reaction, the reaction mixture was cooled to room temperature, diluted with 10ml of methanol, filtered through diatomaceous earth. The filter cake was washed with 3*10ml of methanol, and the combined filtrate was subjected to rotary evaporation under reduced pressure to dryness. The crude material was purified by Prep-TLC (DCM: MeOH =15:1) to provide the title compound: 4-((4-cyclopropyl-2-(N-methyl methanesulfonamido)phenyl)amino)-N-ethoxy-6-((6-fluoro-5-methyl pyridin-3-yl)amino) nicotinamide **(235,** 46.0mg, 0.1mmol, 34.2% yield), MS Calcd: 528.20; MS Found: 529.23([M+H]). 1H NMR (400 MHz, DMSO-d6)δ 11.53 (s, 1H), 9.65 (s, 1H), 9.19 (s, 1H), 8.27 (s, 1H), 8.17 (t, J = 2.0 Hz, 1H), 8.10 - 8.02 (m, 1H), 7.38 (d, J = 8.4 Hz, 1H), 7.29 (d, J = 2.0 Hz, 1H), 7.10 (dd, J = 8.4, 2.0 Hz, 1H), 6.28 (s, 1H), 3.92 (q, J = 7.2 Hz, 2H), 3.13 (s, 3H), 3.08 (s, 3H), 2.21 (s, 3H), 2.01-1.94 (m, 1H), 1.21 (t, J = 7.2 Hz, 3H), 1.03 - 0.97 (m, 2H), 0.75 - 0.68 (m, 2H).

Example **236**

4-((4-cyclopropyl-2-(N-methyl methanesulfonamido)phenyl)amino)-N-ethoxy-6-((5-fluoropyridin-3-yl)amino)nicotinamide

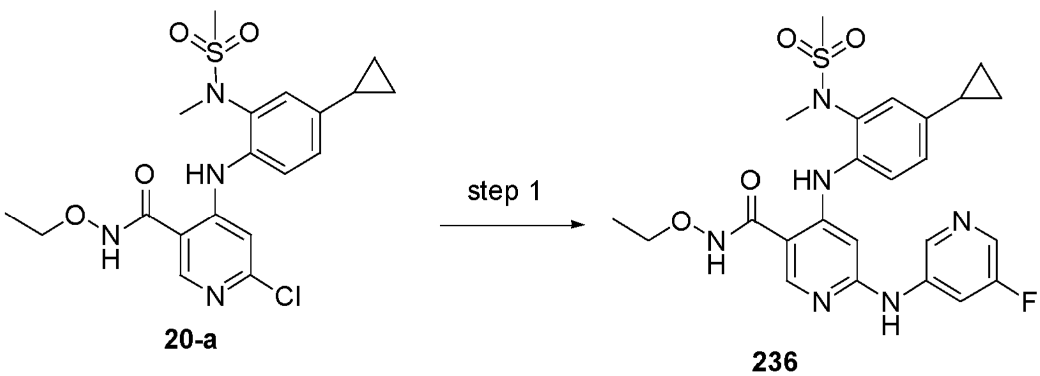


**20-a** **236**

Step 1: To a 10ml microwave tube were sequentially added 6-chloro-4-((4-cyclopropyl-2-(N-methyl methanesulfonamido)phenyl)amino)-N-ethoxy nicotinamide **(20-a,** 100mg, 0.23 mmol), 5-fluoropyridin-3-ylamine (38.4 mg,

0.34mmol), $Pd_2(dba)_3$ (20.9mg, 0.023mmol), XantPhos(26.4 mg, 0.046mmol), $Cs_2CO_3$ (222.7mg, 0.69mmol) and 1,4-dioxane (4ml). After atmosphere replacement with nitrogen three times, the mixture was heated to 140°C with microwave and stirred for 3 hours under nitrogen protection. When LCMS indicated a completed reaction, the reaction mixture was cooled to room temperature, diluted with 10ml of methanol, and filtered through diatomaceous earth. The filter cake was washed with 3*10ml of methanol. The combined filtrate was subjected to rotary evaporation under reduced pressure to dryness, The crude material was purified by high performance preparative thin layer chromatography (DCM: MeOH =15:1) to provide the title compound: 4-((4-cyclopropyl-2-(N-methyl methanesulfonamido)phenyl)amino)-N-ethoxy-6-((5-fluoropyridin-3-yl)amino)nicotinamide **(236,** 85.0mg, 0.16mmol, 65.6% yield), MS Calcd: 514.18; MS Found: 515.20($[M+H]^-$). 1H NMR (400 MHz, DMSO-d6)δ11.60 (s, 1H), 9.64 (s, 1H), 9.53 (s, 1H), 8.43 (t, J = 2.0 Hz, 1H), 8.35 - 8.29 (m, 2H), 8.05 (d, J = 2.4 Hz, 1H), 7.38 (d, J = 8.4 Hz, 1H), 7.31 (d, J = 2.0 Hz, 1H), 7.12 (dd, J = 8.4, 2.0 Hz, 1H), 6.35 (s, 1H), 3.94 (q, J = 7.2 Hz, 2H), 3.14 (s, 3H), 3.09 (s, 3H), 2.01-1.95 (m, 1H), 1.22 (t, J = 7.2 Hz, 3H), 1.04 - 0.96 (m, 2H), 0.76 - 0.69 (m, 2H).

Example **237**

4-((4-cyclopropyl-2-(N-methyl methanesulfonamido)phenyl)amino)-N-ethoxy-6-((6-fluoropyridin-3-yl)amino)nicotinamide

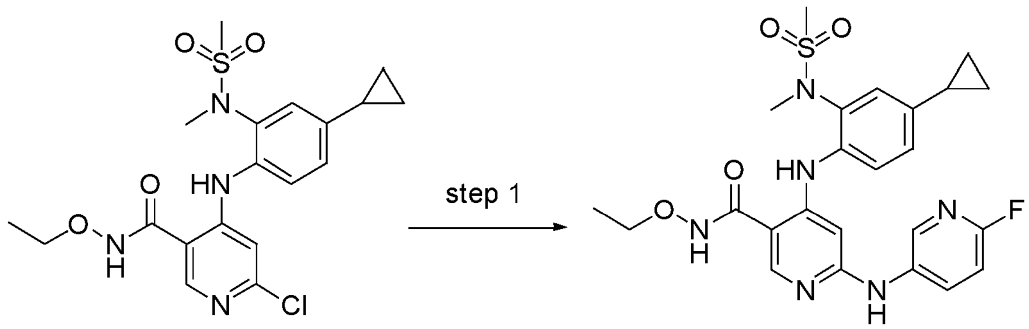


**20-a** **237**

Step 1: To a 10ml microwave tube were sequentially added 6-chloro-4-((4-cyclopropyl-2-(N-methyl methanesulfonamido)phenyl)amino)-N-ethoxy nicotinamide **(20-a,** 100mg, 0.23 mmol), 5-fluoropyridin-3-ylamine (38.4 mg, 0.34mmol), $Pd_2(dba)_3$(20.9mg, 0.023mmol), Xant phos(26.4 mg, 0.046mmol), $Cs_2CO_3$ (222.7mg, 0.69mmol) and 1,4-dioxane (4ml). After atmosphere replacement with nitrogen three times, the mixture was heated to 140°C with microwave and stirred for 3 hours under nitrogen protection. When LCMS indicated a completed reaction, the reaction mixture was cooled to room temperature, diluted with 10ml of methanol, and filtered through diatomaceous earth. The filter cake was washed with 3*10ml of methanol. The combined filtrate was subjected to rotary evaporation under reduced pressure to dryness, and the crude material was purified by Prep-TLC (DCM: MeOH =15:1) to provide the title compound: 4-((4-cyclopropyl-2-(N-methyl methanesulfonamido)phenyl)amino)-N-ethoxy-6-((6-fluoropyridin-3-yl)amino)nicotinamide **(237,** 90.0mg, 0.17mmol, 71.3% yield), MS Calcd: 514.18; MS Found: 515.30([M+H]). 1H NMR (400 MHz, DMSO-d6) δ11.56 (s, 1H), 9.65 (s, 1H), 9.27 (s, 1H), 8.41-8.40 (m, 1H), 8.26 (s, 1H), 8.22-8.17(m, 1H), 7.38 (d, J = 8.4 Hz, 1H), 7.30 (d, J = 2.0 Hz, 1H), 7.15 - 7.05 (m, 2H), 6.29 (s, 1H), 3.93 (q, J = 7.2 Hz, 2H), 3.14 (s, 3H), 3.09 (s, 3H), 2.01-1.95 (m, 1H), 1.22 (t, J = 7.2 Hz, 3H), 1.03 - 0.96 (m, 2H), 0.75 - 0.69 (m, 2H).

Example **238**

6-((2,6-dimethyl pyrimidin-4-yl)amino)-N-ethoxy-4-((2-methoxy-3-(5-methyl pyrimidin-2-yl)phenyl)amino)nicotinamide

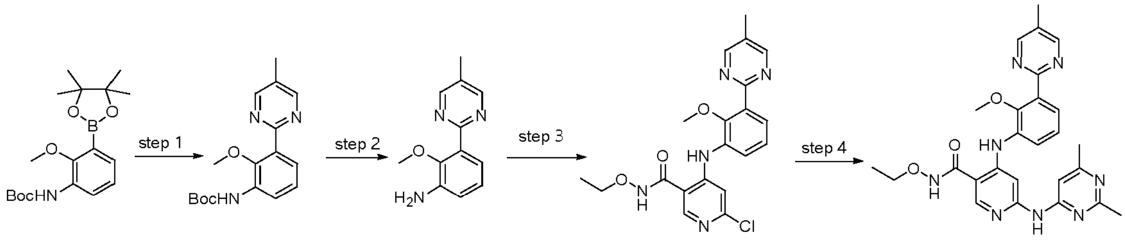


212-c 238-a 238-b 238-c 238

Step 1: To a 100ml flask were sequentially added t-butyl (2-methoxy-3-(4,4,5,5-tetramethyl-1,3,2-dioxaborolan-2-yl)phenyl)carbamate **(212-c,** 700mg, 2.00mmol), 2-chloro-5-methyl pyrimidine (386mg, 3.00mmol), anhydrous potassium carbonate (831mg, 6.00mmol), Pd(dppf)$Cl_2$ (146mg, 0.2mmol), dioxane(10 ml), and $H_2O$(2ml). After atmosphere replacement with nitrogen three times, the mixture was heated to 110°C and stirred under reflux for 3 hours under nitrogen protection. When TLC indicated a completed reaction, the reaction mixture was filtered and the filtrate was added with 30 ml of saturated brine, and extracted with 3*30ml of ethyl acetate three times. The organic phases were combined, dried over anhydrous sodium sulfate, and subjected to rotary evaporation under reduced pressure to dryness. The residue was purified by flash column chromatography (EA: PE = 0 to 30% in 30min) to provide t-butyl (2-methoxy-3-(5-methyl pyrimidin-2-yl)phenyl)carbamate **(238-a,** 460 mg, 1.40mmol, 69.1% yield). MS Calcd: 315.16; MS Found: 316.16([M+H]$^+$).

Step 2: To a 50ml flask was added t-butyl (2-methoxy-3-(5-methyl pyrimidin-2-yl)phenyl)carbamate **(238-a,** 460 mg, 1.40mmol), followed by sequentially addition of dichloromethane (5ml), and hydrochloride solution in dioxane (4M) (5ml). The mixture was stirred at room temperature for 2 hours. When TLC indicated a completed reaction, the reaction mixture was subjected to rotary evaporation under reduced pressure to dryness to provide 2-methoxy-3-(5-methyl pyrimidin-2-yl)aniline **(238-b,** 260mg, 1.20mmol, 89.8% yield). MS Calcd: 215.11; MS Found: 216.12([M+H] $^+$).

Step 3: To a 100ml flask were sequentially added 2-methoxy-3-(5-methyl pyrimidin-2-yl)aniline **(238-b,** 260 mg, 1.20mmol), 4,6-dichloro-N-ethoxy nicotinamide **(int-2,** 283mg, 1.20mmol) and DMA(10ml). The mixture was cooled in ice bath and added with LiHMDS(6ml, 6.00 mol) (1M in THF). The reaction mixture was removed from ice bath and stirred at room temperature for 2 hours. When LCMS monitoring indicated that no raw material unreacted, the mixture was cooled in ice bath and added with 6ml of diluted HCl aqueous solution(1M) to quench the reaction, diluted with 40ml of saturated NaCl, and extracted with 100ml of EA. The organic phase was washed with 3*30ml of saturated NaCl. The organic phases were collected, dried over anhydrous $NaSO_4$, and subjected to rotary evaporation under reduced pressure to dryness. The crude material was purified by flash column chromatography (DCM:MeOH = 0 to 10% in 30min), to provide 6-chloro-N-ethoxy-4-((2-methoxy-3-(5-methyl pyrimidin-2-yl)phenyl)amino)nicotinamide **(238-c,** 220mg, 0.5 mmol, 43.6% yield). MS Calcd: 413.13; MS Found: 314.12([M+H]$^+$).

Step 4: To a 10ml microwave tube were sequentially added 6-chloro-N-ethoxy-4-((2-methoxy-3-(5-methyl pyrimidin-2-yl)phenyl)amino)nicotinamide **(238-c,** 50mg, 0.12 mmol), 2,6-dimethyl pyrimidin-4-ylamine (14.8 mg, 0.12mmol), $Pd_2(dba)_3$(11.1mg, 0.012mmol), Xantphos(14.0 mg, 0.024mmol), $Cs_2CO_3$ (118.1mg, 0.36mmol) and 1,4-dioxane (2ml). After atmosphere replacement with nitrogen three times, the mixture was heated to 140°C with microwave and stirred for 3 hours under nitrogen protection. When LCMS indicated a completed reaction, the reaction mixture was cooled to room temperature, diluted with 5ml of methanol, and filtered through diatomaceous earth. The filter cake was washed with 3*5ml of methanol. The combined filtrate was subjected to rotary evaporation under reduced pressure to dryness. The crude material was purified by high performance preparative thin layer chromatography (DCM: MeOH =15:1) to provide the title compound: 6-((2,6-dimethyl pyrimidin-4-yl)amino)-N-ethoxy-4-((2-methoxy-3-(5-methyl pyrimidin-2-yl) phenyl)amino)nicotinamide **(238,** 35.0mg, 55.2% yield), MS Calcd: 500.23; MS Found: 501.22([M+H]$^+$). 1H NMR (400 MHz, DMSO-d6)δ11.70 (s, 1H), 10.22 (s, 1H), 10.09 (s, 1H), 8.79 (s, 2H), 8.39 (s, 1H), 8.17 (s, 1H), 7.71 (dd, J = 8.0, 2.0 Hz, 1H), 7.43 (dd, J = 8.0, 2.0 Hz, 1H), 7.30 (t, J = 8.0 Hz, 1H), 7.09 (s, 1H), 3.95 (q, J = 7.2 Hz, 2H), 3.69 (s, 3H), 2.39 (s, 3H), 2.34 (s, 3H), 2.28 (s, 3H), 1.23 (t, J = 7.2 Hz, 3H).

Example **239**

6-(cyclopropylcarboxamido)-N-ethoxy-4-((2-methoxy-3-(5-methyl pyrimidin-2-yl)phenyl)amino)nicotinamide

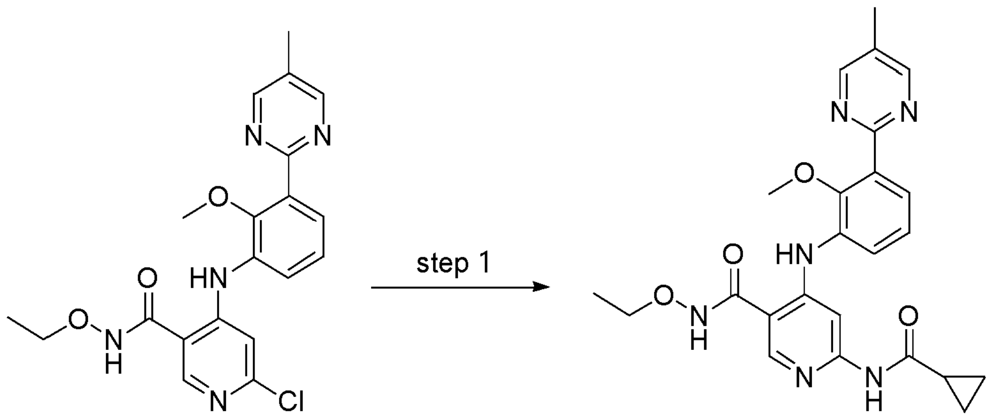


238-c 239

Step 1: To a 10ml microwave tube were sequentially added 6-chloro-N-ethoxy-4-((2-methoxy-3-(5-methyl pyrimidin-2-yl)phenyl)amino)nicotinamide (N210874-035d, 50mg, 0.12 mmol), cyclopropylcarboxamide (10.2 mg, 0.12mmol), $Pd_2(dba)_3$(11.1mg, 0.012mmol), Xant phos(14.0 mg, 0.024mmol), $Cs_2CO_3$ (118.1mg, 0.36mmol) and 1,4-dioxane (2ml). After atmosphere replacement with nitrogen three times, the mixture was heated to 140°C with microwave and stirred for 3 hours under nitrogen protection. When LCMS indicated a completed reaction, the reaction mixture was cooled to room temperature, diluted with 5ml of methanol, and filtered through diatomaceous earth. The filter cake was washed with 3*5ml of methanol. The combined filtrate was subjected to rotary evaporation under reduced pressure to dryness. The crude material was purified by high performance preparative thin layer chromatography (DCM: MeOH =20:1) to provide the title compound: 6-(cyclopropylcarboxamido)-N-ethoxy-4-((2-methoxy-3-(5-methyl pyrimidin-2-yl)phenyl) amino)nicotinamide **(239,** 40.0mg, 70.5% yield). MS Calcd: 462.20; MS Found: 463.18([M+H]). 1H NMR (400 MHz, DMSO-d6)δ11.75 (s, 1H), 10.81 (s, 1H), 10.12 (s, 1H), 8.79 (d, J = 0.8 Hz, 2H), 8.39 (s, 1H), 8.08 (s, 1H), 7.53 (dd, J = 8.0, 1.6 Hz, 1H), 7.42 (dd, J = 8.0, 1.6 Hz, 1H), 7.25 (t, J = 8.0 Hz, 1H), 3.95 (q, J = 7.2 Hz, 2H), 3.66 (s, 3H), 2.34 (s, 3H), 2.03 - 1.94 (m, 1H), 1.22 (t, J = 7.2 Hz, 3H), 0.81 - 0.75 (m, 2H), 0.65 - 0.58 (m, 2H).

Example **240**

N-ethoxy-4-((2-methoxy-3-(5-methyl pyrimidin-2-yl)phenyl)amino)-6-((2-methyl pyrimidin-4-yl)amino)nicotinamide

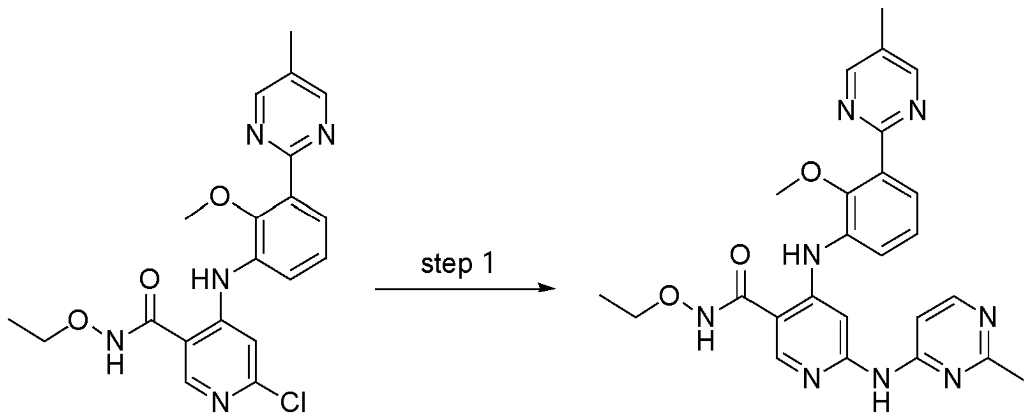


238-c 240

Step 1: To a 10ml microwave tube were sequentially added 6-chloro-N-ethoxy-4-((2-methoxy-3-(5-methyl pyrimidin-2-yl)phenyl)amino)nicotinamide **(238-c,** 50mg, 0.12 mmol), 2-methyl pyrimidin-4-ylamine (13.1 mg, 0.12mmol), $Pd_2(dba)_3$(11.1mg, 0.012mmol), Xantphos(14.0 mg, 0.024mmol), $Cs_2CO_3$ (118.1mg, 0.36mmol) and 1,4-dioxane (2ml). After atmosphere replacement with nitrogen three times, the mixture was heated to 140°C with microwave and stirred for 3 hours under nitrogen protection. When LCMS indicated a completed reaction, the reaction mixture was cooled to room temperature, diluted with 5ml of methanol, and filtered through diatomaceous earth. The filter cake was washed with 3*5ml

of methanol. The combined filtrate was subjected to rotary evaporation under reduced pressure to dryness. The crude material was purified by Prep-TLC (DCM: MeOH =20:1) to provide the title compound N-ethoxy-4-((2-methoxy-3-(5-methyl pyrimidin-2-yl)phenyl)amino)-6-((2-methyl pyrimidin-4-yl)amino)nicotinamide **(240,** 35.0mg, 56.7% yield), MS Calcd: 486.21; MS Found: 487.30([M+H]$^+$). 1H NMR (400 MHz, DMSO-d6)δ11.72 (s, 1H), 10.22 (s, 1H), 10.19 (s, 1H), 8.79 (d, J = 0.8 Hz, 2H), 8.39 (s, 1H), 8.29 (d, J = 6.0 Hz, 1H), 8.17 (s, 1H), 7.71 (dd, J = 8.0, 1.6 Hz, 1H), 7.44 (dd, J = 8.0, 1.6 Hz, 1H), 7.31 (t, J = 8.0 Hz, 1H), 7.23 (d, J = 6.0 Hz, 1H), 3.96 (q, J = 7.2 Hz, 2H), 3.69 (s, 3H), 2.42 (s, 3H), 2.34 (s, 3H), 1.23 (t, J = 7.2 Hz, 3H).

Example **241**

6-((3,5-difluoropyridin-2-yl)amino)-N-ethoxy-4-((3-(5-fluoropyrimidin-2-yl)-2-methoxy phenyl)amino)nicotinamide

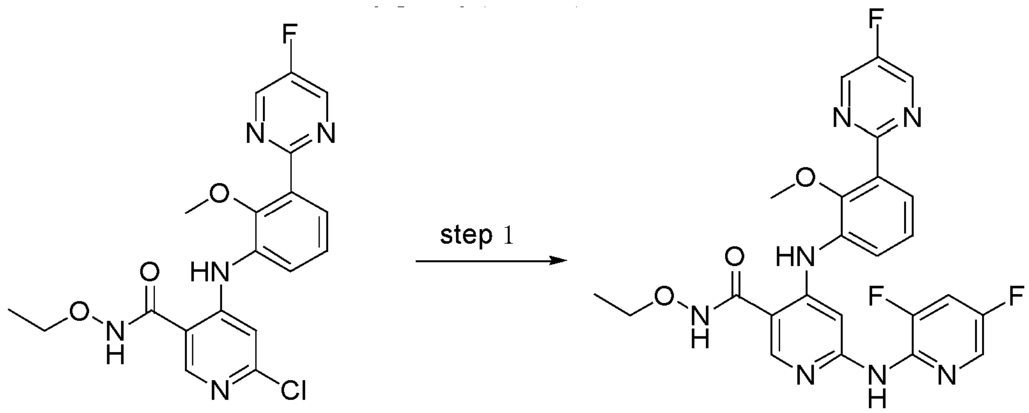


**208-e** **241**

Step 1: To anhydrous dioxane (2 ml) were added 6-chloro-N-ethoxy-4-((3-(5-fluoropyrimidin-2-yl)-2-methoxy phenyl)amino)nicotinamide **(208-e,** 110 mg, 0.27 mmol), 2-amino-3,5-difluoropyridine (44 mg, 0.36 mmol), cesium carbonate (195 mg, 0.6 mmol), XantPhos(25mg, 0.04mmol) and $Pd_2(dba)_3$(27mg, 0.029 mmol). After the atmosphere of the mixture was evacuated to vacuum and replaced with nitrogen three times, the mixture was heated to 140°C, stirred in a microwave reactor for 3 hours, and filtered by suction. The filtrate was concentrated and purified by preparative TLC (MeOH:DCM=1:20) to provide the title compound: 6-((3,5-difluoropyridin-2-yl)amino)-N-ethoxy-4-((3-(5-fluoropyrimidin-2-yl)-2-methoxy phenyl)amino)nicotinamide **(241,** 50mg, 0.09mmol, 37% yield). MS Calcd: 511.16; MS Found: 512.4 ([M+H]$^+$). $^1$H NMR (400 MHz, DMSO-$d_6$)δ11.70 (s, 1H), 10.26 (s, 1H), 9.31 (s, 1H), 9.04 (d, *J* = 0.8 Hz, 2H), 8.34 (s, 1H), 8.16 (d, *J* = 2.4 Hz, 1H), 7.94-7.88 (m, 1H), 7.69 - 7.67 (m, 2H), 7.42 (dd, *J* = 8.0, 2.0 Hz, 1H), 7.35 (t, *J* = 7.2 Hz, 1H), 3.94 (q, *J* = 7.0 Hz, 2H), 3.69 (s, 3H), 1.22 (t, *J* = 7.2 Hz, 3H).

Example **242**

6-((3,5-difluoropyridin-2-yl)amino)-N-ethoxy-4-((2-methoxy-3-(pyrimidin-2-yl)phenyl)amino)nicotinamide

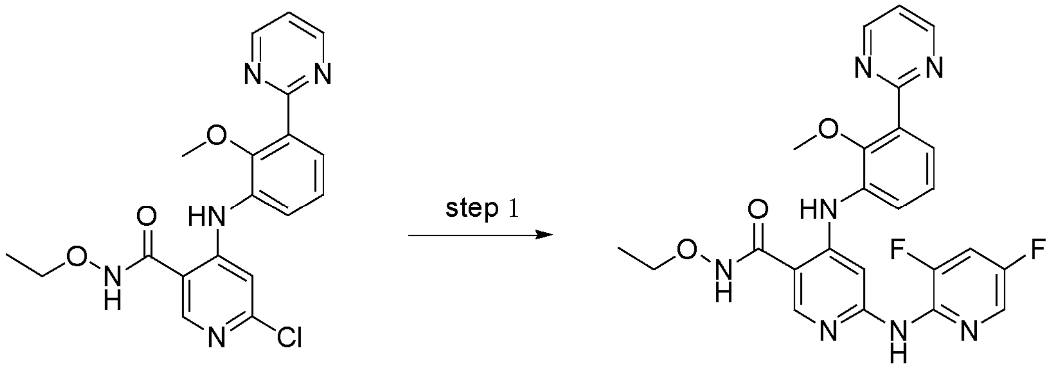


212-f 242

Step 1: 6-chloro-N-ethoxy-4-((2-methoxy-3-(pyrimidin-2-yl)phenyl)amino)nicotinamide **(212-f,** 110 mg, 0.27 mmol), 2-amino-3,5-difluoropyridine (50 mg, 0.38 mmol), cesium carbonate (195 mg, 0.6 mmol), XantPhos(25mg, 0.04mmol) and $Pd_2(dba)_3$(27mg, 0.029 mmol) were added to anhydrous dioxane (2 ml). After the atmosphere of the mixture was evacuated to vacuum and replaced with nitrogen three times, the mixture was heated to 125°C with stirring for 10 hours, and filtered by suction. The filtrate was concentrated and purified by high performance preparative thin layer chromatography (MeOH:DCM=1:20) to provide the title compound: 6-((3,5-difluoropyridin-2-yl)amino)-N-ethoxy-4-((2-methoxy-3-(pyrimidin-2-yl)phenyl)amino)nicotinamide **(242,** 50mg, 0.1mmol, 37% yield). MS Calcd: 493.17; MS Found: 494.19 ($[M+H]^+$). $^1H$ NMR (400 MHz, DMSO-$d_6$)$\delta$11.67 (s, 1H), 10.17 (s, 1H), 9.35 (s, 1H), 8.95 (d, *J* = 4.8 Hz, 2H), 8.32 (s, 1H), 8.17 (d, *J* = 2.6 Hz, 1H), 7.92 (ddd, *J* = 10.8, 8.4, 2.6 Hz, 1H), 7.70 - 7.65 (m, 2H), 7.51 (t, *J* = 4.8 Hz, 1H), 7.43 (dd, *J* = 7.6, 1.6 Hz, 1H), 7.35 (t, *J* = 7.8 Hz, 1H), 3.95 (q,*J* = 7.2 Hz, 2H), 3.70 (s, 3H), 1.22 (t, *J* = 7.2 Hz, 3H).

Example **243**

N-ethoxy-4-((3-(5-fluoropyrimidin-2-yl)-2-methoxy phenyl)amino)-6-((2-methyl pyrimidin-4-yl)amino)nicotinamide

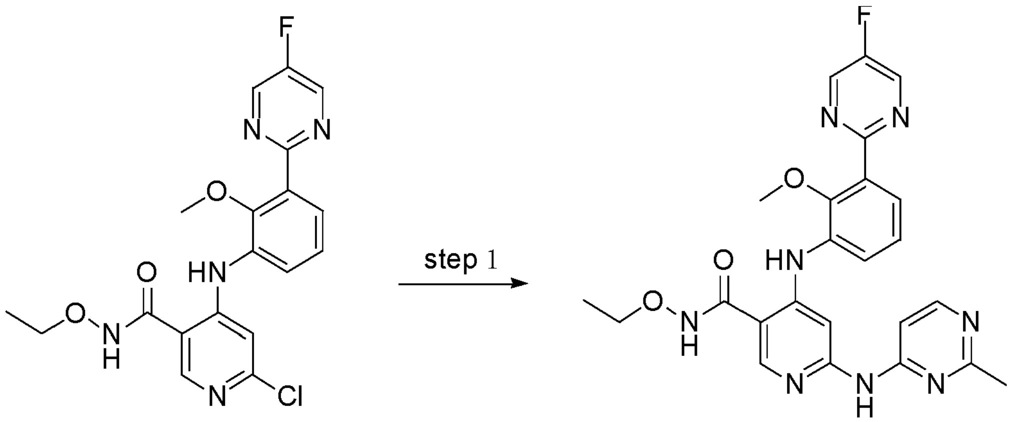


208-e 243

Step 1: 6-chloro-N-ethoxy-4-((3-(5-fluoropyrimidin-2-yl)-2-methoxy phenyl)amino)nicotinamide **(208-e,** 110 mg, 0.27 mmol), 2-methyl-4-aminopyrimidine (39 mg, 0.36 mmol), cesium carbonate (195 mg, 0.6 mmol), XantPhos(25mg, 0.04mmol) and $Pd_2(dba)_3$(27mg, 0.029 mmol) were added to anhydrous dioxane (2 ml). After the atmosphere of the mixture was evacuated to vacuum and replaced with nitrogen three times, the mixture was heated to 140°C, stirred in a microwave reactor for 3 hours, and filtered by suction. The filtrate was concentrated and purified by preparative TLC (MeOH:DCM=1:20) to provide the title compound: N-ethoxy-4-((3-(5-fluoropyrimidin-2-yl)-2-methoxy phenyl)amino)-6-((2-methyl pyrimidin-4-yl)amino)nicotinamide **(243,** 40mg, 0.08mmol, 30% yield). MS Calcd: 490.19; MS Found: 491.2 ($[M+H]^+$). $^1H$ NMR (400 MHz, DMSO-$d_6$)$\delta$11.73 (s, 1H), 10.23 (s, 1H), 10.20 (s, 1H), 9.04 (d, *J* = 0.8 Hz, 2H), 8.40 (s, 1H), 8.29 (d, *J* = 5.6 Hz, 1H), 8.16 (s, 1H), 7.73 (dd, *J* = 8.0, 1.6 Hz, 1H), 7.47 (dd, *J* = 7.6, 1.6 Hz, 1H), 7.33 (t, *J* = 8.0 Hz, 1H), 7.23 (d, *J* = 6.0 Hz, 1H), 3.96 (q, *J* = 7.2 Hz, 2H), 3.69 (s, 3H), 2.41 (s, 3H), 1.23 (t, *J* = 7.2 Hz, 3H).

Example **244**

4-((3-(1-cyclopropyl-1H-1,2,4-triazol-3-yl)-5-fluoro-2-methoxy phenyl)amino)-6-((3,5-difluoropyridin-2-yl)amino)-N-ethoxy nicotinamide

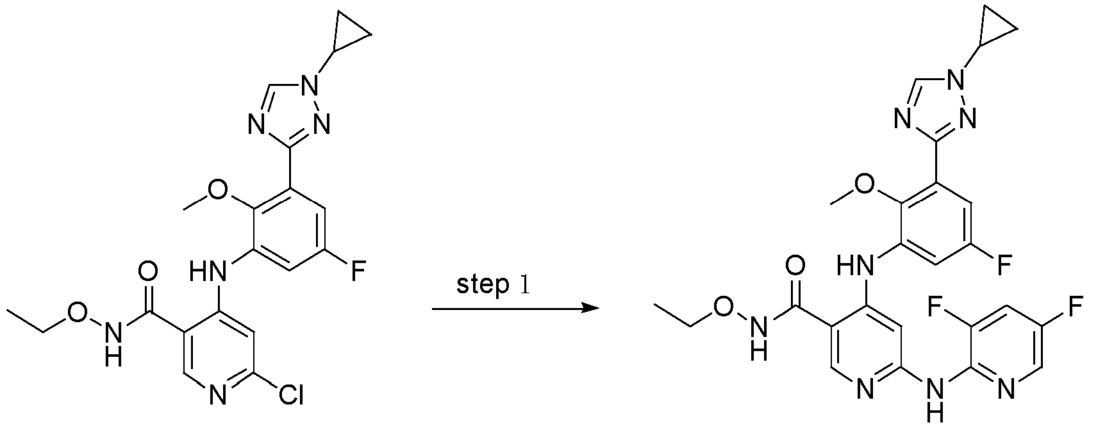


**204-g** **244**

Step 1: 6-chloro-4-((3-(1-cyclopropyl-1H-1,2,4-triazol-3-yl)-5-fluoro-2-methoxy phenyl)amino)-N-ethoxy nicotinamide **(204-g,** 115 mg, 0.26 mmol), 2-amino-3,5-difluoropyridine (33 mg, 0.26 mmol), cesium carbonate (251 mg, 0.78 mmol), XantPhos(22mg, 0.04mmol) and $Pd_2(dba)_3$(24mg, 0.026 mmol) were added to anhydrous dioxane (2 ml). After the atmosphere of the mixture was evacuated to vacuum and refilled with nitrogen, the reaction mixture was heated to 140°C, stirred in a microwave reactor for 3 hours, and filtered by suction. The filtrate was concentrated and purified by high performance preparative thin layer chromatography (MeOH:DCM=1:20) to provide the title compound: 4-((3-(1-cyclopropyl-1H-1,2,4-triazol-3-yl)-5-fluoro-2-methoxy phenyl)amino)-6-((3,5-difluoropyridin-2-yl)amino)-N-ethoxy nicotinamide **(244,** 40mg, 0.1mmol, 29% yield). MS Calcd: 540.18; MS Found: 541.27 ($[M+H]^+$). $^1H$ NMR (400 MHz, DMSO-$d_6$)$\delta$11.73 (s, 1H), 10.42 (s, 1H), 9.50 (s, 1H), 8.74 (s, 1H), 8.35 (s, 1H), 8.10 (d, *J* = 2.4 Hz, 1H), 7.98-7.93 (m, 1H), 7.77 (s, 1H), 7.51 (dd, *J* = 10.0, 3.2 Hz, 1H), 7.31 (dd, *J* = 9.2, 3.2 Hz, 1H), 3.96 (q, *J* = 7.2 Hz, 2H), 3.91-3.87 (m, 1H), 3.76 (s, 3H), 1.23 (t, *J* = 7.2 Hz, 3H), 1.20-1.18 (m, 2H), 1.12 - 1.05 (m, 2H).

Example **245**

4-((3-(1-cyclopropyl-1H-1,2,4-triazol-3-yl)-5-fluoro-2-methoxy phenyl)amino)-N-ethoxy-6-((2-methyl pyrimidin-4-yl) amino)nicotinamide

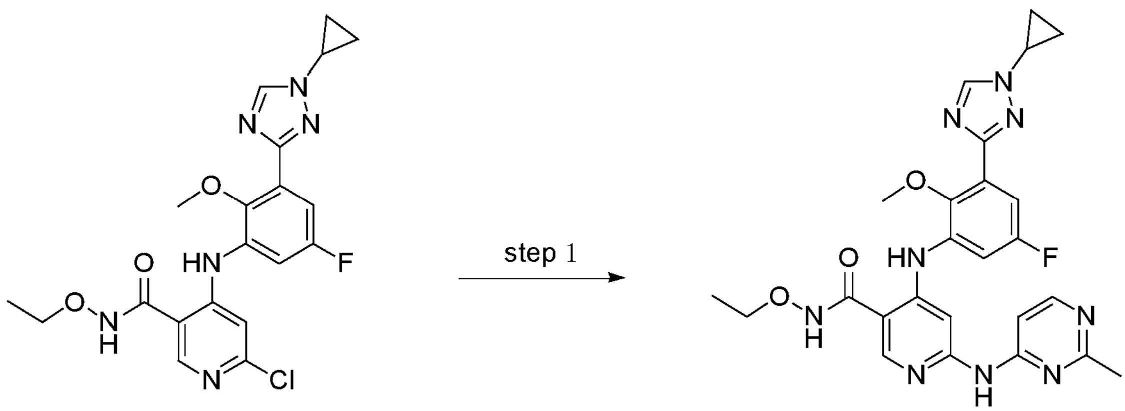


**204-g** **245**

Step 1: 6-chloro-4-((3-(1-cyclopropyl-1H-1,2,4-triazol-3-yl)-5-fluoro-2-methoxy phenyl)amino)-N-ethoxy nicotinamide **(204-g,** 120 mg, 0.27 mmol), 2-methyl-4-aminopyrimidine (29 mg, 0.27 mmol), cesium carbonate (262 mg, 0.8 mmol), XantPhos(22mg, 0.04mmol) and $Pd_2(dba)_3$(24mg, 0.026 mmol) were added to anhydrous dioxane (2 ml). After the atmosphere of the mixture was evacuated to vacuum and refilled with nitrogen, the reaction mixture was heated to 140°C, stirred in a microwave reactor for 3 hours, and filtered by suction. The filtrate was concentrated and purified by high performance preparative thin layer chromatography (MeOH:DCM=1:20) to provide the title compound: 4-((3-(1-cyclopropyl-1H-1,2,4-triazol-3-yl)-5-fluoro-2-methoxy phenyl)amino)-N-ethoxy-6-((2-methyl pyrimidin-4-yl)amino)nicotinamide **(245,** 30mg, 0.1mmol, 22% yield). MS Calcd: 519.21; MS Found: 520.21 ($[M+H]^+$). $^1H$ NMR (400 MHz, DMSO-$d_6$)δ11.80 (s, 1H), 10.51 (s, 1H), 10.29 (s, 1H), 8.74 (s, 1H), 8.43 (s, 1H), 8.33 (d, *J* = 6.0 Hz, 1H), 8.21 (s, 1H), 7.53 (dd, *J* = 10.0, 3.2 Hz, 1H), 7.38 - 7.23 (m, 2H), 3.98 (q, *J* = 7.2 Hz, 2H), 3.93-3.87 (m, 1H), 3.77 (s, 3H), 2.46 (s, 3H), 1.24 (t, *J* = 7.2 Hz, 3H), 1.20-1.17 (m, 2H), 1.11-1.06 (m, 2H).

Example **246**

4-((4-cyclopropyl-5-fluoro-2-(N-methyl methanesulfonamido)phenyl)amino)-6-((3,5-difluoropyridin-2-yl)amino)-N-ethoxy nicotinamide

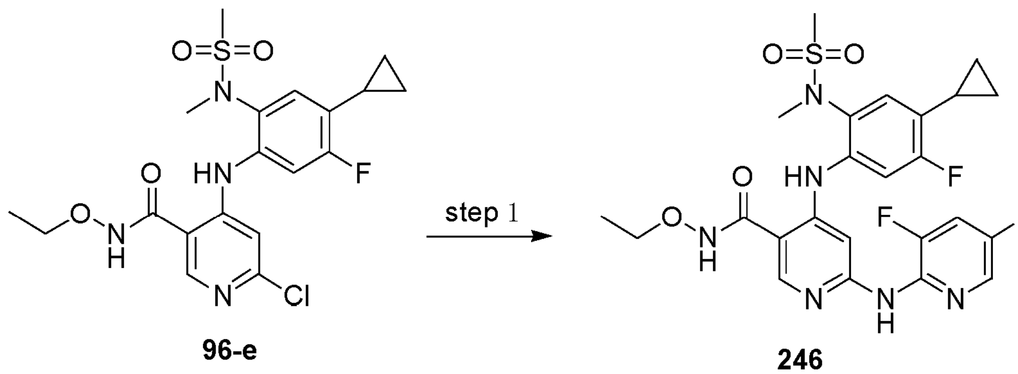


**96-e** **246**

Step 1: 6-chloro-4-((4-cyclopropyl-5-fluoro-2-(N-methyl methanesulfonamido)phenyl)amino)-N-ethoxy nicotinamide **(96-e,** 70mg, 0.15mmol), 3,5-difluoropyridin-2-ylamine (30mg, 0.23mmol), $Pd_2(dba)_3$ (28.1mg, 0.032mmol), XantPhos (35.5mg, 0.064mmol), $Cs_2CO_3$ (149.7mg, 0.5mmol) were mixed and added with 1,4-dioxane (1ml). After atmosphere replacement with nitrogen three times, the mixture was heated with microwave to 140°C and allowed to react for 3.5 hours. When TLC indicated a completed reaction, the mixture was filtered by suction, and the filtrate was concentrated under reduced pressure at 40°C. The residue was separated and purified by high performance preparative thin layer chromatography, eluted with DCM:MeOH=20:1, to provide the title compound 4-((4-cyclopropyl-5-fluoro-2-(N-methyl methanesulfonamido)phenyl)amino)-6-((3,5-difluoropyridin-2-yl)amino)-N-ethoxy nicotinamide **(246,** 28mg, 0.055mmol, 33.2% yield). MS Calcd: 550.16; MS Found: 551.17 ($[M+H]^+$). 1H NMR (400 MHz, DMSO-d6)δ11.63 (s, 1H), 10.11 (s, 1H), 9.41 (s, 1H), 8.29 (s, 1H), 8.08 (d, J = 2.4 Hz, 1H), 7.94 (ddd, J = 10.8, 8.4, 2.4 Hz, 1H), 7.55 (s, 1H), 7.40 (d, J = 12.0 Hz, 1H), 7.15 (d, J = 8.0 Hz, 1H), 3.93 (q, J = 7.2 Hz, 2H), 3.14 (s, 3H), 3.10 (s, 3H), 2.17 - 1.99 (m, 1H), 1.21 (t, J = 7.2 Hz, 3H), 1.01-0.96 (m, 2H), 0.82-0.77 (m, 2H).

Example **247**

6-(2,2-difluorocyclopropyl-1-carboxamido)-N-ethoxy-4-((3-(5-fluoropyrimidin-2-yl)-2-methoxy phenyl)amino)nicotinamide

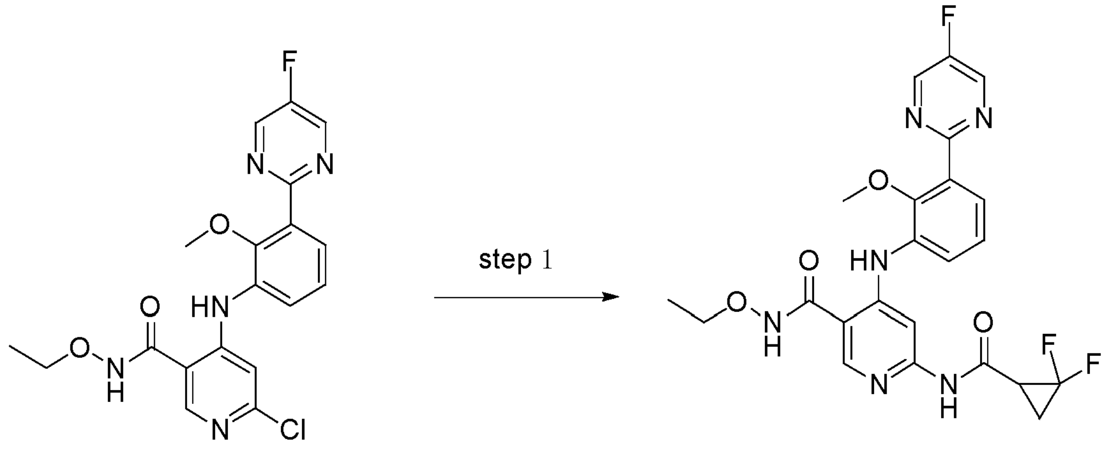


208-e 247

Step 1: To a 10ml microwave tube were sequentially added 6-chloro-N-ethoxy-4-((3-(5-fluoropyrimidin-2-yl)-2-methoxy phenyl)amino)nicotinamide **(208-e,** 50mg, 0.12mmol), 2,2-difluorocyclopropyl-1-carboxamide(29.0mg, 0.24mmol), $Pd_2(dba)_3$(11.0mg, 0.012mmol), Xantphos(13.8mg, 0.024mmol), $Cs_2CO_3$ (117mg, 0.36mmol) and dioxane(2ml). After atmosphere replacement with nitrogen three times, the mixture was heated to 140°C with microwave and stirred for 3 hours under nitrogen protection. When LCMS indicated a completed reaction, the reaction mixture was cooled to room temperature, diluted with 5ml of methanol, and filtered through diatomaceous earth. The filter cake was washed with 3*5ml of methanol. The combined filtrate was subjected to rotary evaporation under reduced pressure to dryness, and the crude material was purified by Prep-TLC (DCM: MeOH =20:1) to provide the title compound: 6-(2,2-difluorocyclopropyl-1-carboxamido)-N-ethoxy-4-((3-(5-fluoropyrimidin-2-yl)-2-methoxy phenyl)amino)nicotinamide **(247,** 18mg, 30.0% yield), MS Calcd: 502.16; MS Found: 503.14($[M+H]^+$). 1H NMR (400 MHz, DMSO-d6)$\delta$11.80 (s, 1H), 11.02 (s, 1H), 10.16 (s, 1H), 9.04 (s, 2H), 8.42 (s, 1H), 8.02 (s, 1H), 7.61 - 7.53 (m, 1H), 7.50 - 7.44 (m, 1H), 7.29 (t, J = 8.0Hz, 1H), 3.96 (q, J = 7.2 Hz, 2H), 3.67 (s, 3H), 2.03-1.96 (m, 1H), 3.00-2.92 (m, 1H), 2.03-1.96 (m, 2H), 1.22(t, J = 7.2 Hz, 3H)

Example **248**

6-((2,6-dimethyl pyrimidin-4-yl)amino)-N-ethoxy-4-((2-methoxy-3-(pyrazin-2-yl)phenyl)amino)nicotinamide

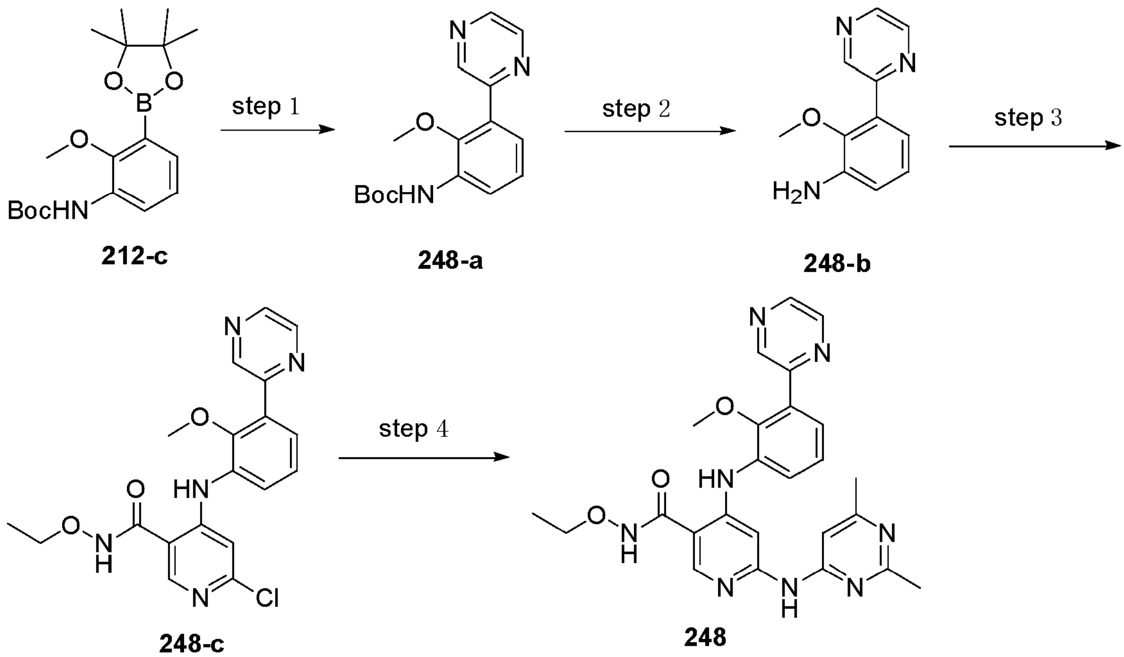


212-c 248-a 248-b

248-c 248

Step 1: To a 100ml flask were sequentially added t-butyl (2-methoxy-3-(4,4,5,5-tetramethyl-1,3,2-dioxaborolan-2-yl)phenyl)carbamate **(212-c,** 300mg, 0.86mmol), 2-bromopyrazine (204mg, 1.3mmol), anhydrous potassium carbonate (356mg, 2.6mmol), Pd(dppf)$Cl_2$ (62mg, 0.086mmol), dioxane(10 ml), and $H_2O$(2ml). After atmosphere replacement with nitrogen three times, the mixture was heated to 110°C and stirred under reflux for 3 hours under nitrogen protection. When TLC indicated a completed reaction, the reaction mixture was filtered and the filtrate was added with 30ml of saturated brine, and extracted with 3*30ml of ethyl acetate three times. The organic phases were combined, dried over anhydrous sodium sulfate, and subjected to rotary evaporation under reduced pressure to dryness. The residue was purified by flash column chromatography (EA: PE = 0 to 20% in 30min) to provide t-butyl (2-methoxy-3-(pyrazin-2-yl)phenyl)carbamate **(248-a,** 230 mg, 0.8mmol, 88% yield). MS Calcd: 301.14; MS Found: 302.1($[M+H]^+$).

Step 2: To a 50ml flask was added t-butyl (2-methoxy-3-(pyrazin-2-yl)phenyl)carbamate **(248-a,** 230 mg, 0.8mmol), followed by sequential addition of dichloromethane (5ml), hydrochloride solution in dioxane (4M) (5ml). The mixture was stirred at room temperature for 2 hours. When TLC indicated a completed reaction, the reaction mixture subjected to rotary evaporation under reduced pressure to dryness to provide 2-methoxy-3-(pyrazin-2-yl)aniline **(248-b,** 170mg, 0.8mmol, 86.4% yield). MS Calcd: 201.09; MS Found: 202.12($[M+H]^+$) ).

Step 3: To a 100ml flask were sequentially added 2-methoxy-3-(pyrazin-2-yl)aniline **(248-b,** 120mg, 0.4mmol), 4,6-dichloro-N-ethoxypyridine-3-carboxamide(112mg, 0.44mmol) and DMA(5ml). The mixture was cooled in ice bath and added with LiHMDS(2.1ml, 2.10 mol) (1M in THF). The reaction mixture was removed from ice bath and stirred at room temperature for 2 hours. When LCMS monitoring indicated that no raw material unreacted, the reaction mixture was cooled in ice bath and added with 3ml of diluted HCl solution (1M) to quench the reaction, diluted with 30ml of water, and extracted with 3*30ml of EA. The organic phases were combined, washed with 3*20ml of saturated NaCl. The organic phase was collected and dried over anhydrous $NaSO_4$, subjected to rotary evaporation under reduced pressure to dryness. The crude material was purified by flash column chromatography (ethyl acetate:petroleum ether = 0 to 60% in 30min), to provide 6-chloro-N-ethoxy-4-((2-methoxy-3-(pyrazin-2-yl)phenyl)amino)nicotinamide **(248-c,** 140mg, 0.3 mmol, 81% yield). MS Calcd: 399.11; MS Found: 400.12 ($[M+H]^+$) ).

**Step** 4: To a 10ml microwave tube were sequentially added 6-chloro-N-ethoxy-4-((2-methoxy-3-(pyrazin-2-yl)phenyl)amino)nicotinamide **(248-c,** 100mg, 0.25mmol), 2,6-dimethyl pyrimidin-4-ylamine (46mg, 0.37mmol), $Pd_2(dba)_3$ (22.8mg, 0.025mmol), Xantphos (28.9mg, 0.050mmol), $Cs_2CO_3$ (243mg, 0.7mmol) and dioxane (3ml). After atmosphere replacement with nitrogen three times, the mixture was heated to 140°C with microwave and stirred for 3 hours under nitrogen protection. When LCMS indicated a completed reaction, the reaction mixture was cooled to room temperature, diluted with 5ml of methanol, and filtered through diatomaceous earth. The filter cake was washed with 3*5ml of methanol. The combined filtrate was subjected to rotary evaporation under reduced pressure to dryness, and the crude material was purified by Prep-TLC (DCM: MeOH =20:1) to provide the title compound 6-((2,6-dimethyl pyrimidin-4-yl)amino)-N-ethoxy-4-((2-methoxy-3-(pyrazin-2-yl)phenyl)amino)nicotinamide **(248,** 67mg, 54.6% yield), MS Calcd: 486.21; MS Found: 487.24($[M+H]^+$). $^1H$ NMR (400 MHz, DMSO-d6)δ11.72 (s, 1H), 10.29 (s, 1H), 10.11 (s, 1H), 9.09 (d, J = 1.6 Hz, 1H), 8.79 (t, J = 2.0 Hz, 1H), 8.65 (d, J = 2.4 Hz, 1H), 8.40 (s, 1H), 8.14 (s, 1H), 7.73 (d, J = 8.0 Hz, 1H), 7.51 (dd, J = 8.0, 1.6 Hz, 1H), 7.37 (t, J = 8.0 Hz, 1H), 7.11 (s, 1H), 3.96 (q, J = 7.2 Hz, 2H), 3.55 (s, 3H), 2.38 (s, 3H), 2.28 (s, 3H), 1.23 (t, J = 7.2 Hz, 3H).

Example **249**

N-ethoxy-4-((2-methoxy-3-(5-methyl pyrazin-2-yl)phenyl)amino)-6-((6-(trifluoromethyl)pyridin-3-yl)amino)nicotinamide

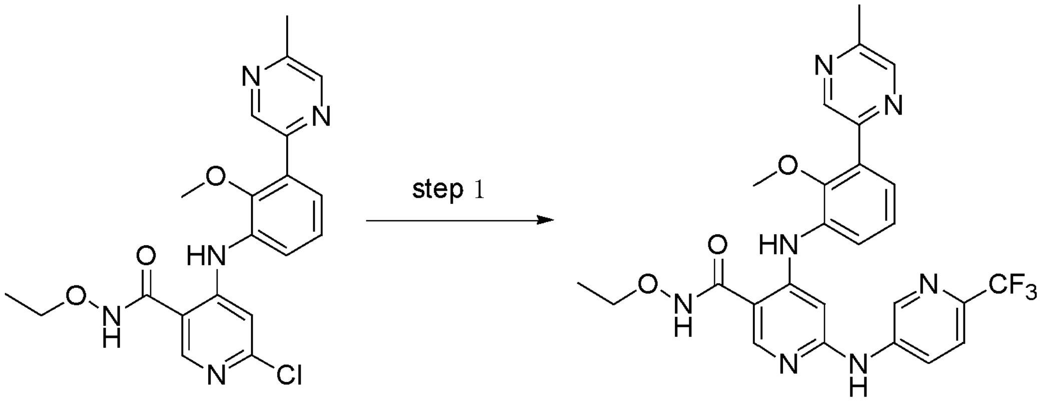


213-d 249

Step 1: To a 10ml microwave tube were sequentially added 6-chloro-N-ethoxy-4-((2-methoxy-3-(5-methyl pyrazin-2-yl)phenyl)amino)nicotinamide **(213-d,** 50mg, 0.12mmol), 6-(trifluoromethyl)pyridin-3-ylamine (29.4 mg, 0.18mmol), $Pd_2(dba)_3$ (11.1mg, 0.012mmol), XantPhos (14.0mg, 0.024mmol), $Cs_2CO_3$ (118mg, 0.36mmol) and dioxane(2ml). After atmosphere replacement with nitrogen three times, the mixture was heated to 140°C with microwave and stirred for 3 hours under nitrogen protection. When LCMS indicated a completed reaction, the reaction mixture was cooled to room temperature, diluted with 5ml of methanol, and filtered through diatomaceous earth. The filter cake was washed with 3*5ml of methanol. The combined filtrate was subjected to rotary evaporation under reduced pressure to dryness, and the crude material was purified by Prep-TLC (DCM: MeOH =20:1) to provide the title compound: N-ethoxy-4-((2-methoxy-3-(5-methyl pyrazin-2-yl)phenyl)amino)-6-((6-(trifluoromethyl)pyridin-3-yl)amino)nicotinamide **(249,** 20mg, 30% yield), MS Calcd: 539.19; MS Found: 540.44($[M+H]^+$). 1H NMR (400 MHz, DMSO-d6)$\delta$11.75 (s, 1H), 10.04 (s, 1H), 9.81 (s, 1H), 8.97 (d, J = 1.6 Hz, 1H), 8.87 (d, J = 2.4 Hz, 1H), 8.69 (dd, J = 1.6, 0.8 Hz, 1H), 8.46 (dd, J = 8.8, 2.4 Hz, 1H), 8.39 (s, 1H), 7.78 (d, J = 8.8 Hz, 1H), 7.60 (dd, J = 8.0, 1.6 Hz, 1H), 7.54 (dd, J = 8.0, 1.6 Hz, 1H), 7.37 (t, J = 8.0 Hz, 1H), 6.66 (s, 1H), 3.97 (q, J = 7.2 Hz, 2H), 3.55 (s, 3H), 2.57 (s, 3H), 1.24 (t, J = 7.2 Hz, 3H).

Example **250**

N-ethoxy-4-((2-methoxy-3-(5-methyl pyrazin-2-yl)phenyl)amino)-6-((5-(trifluoromethyl)pyridin-3-yl)amino)nicotinamide

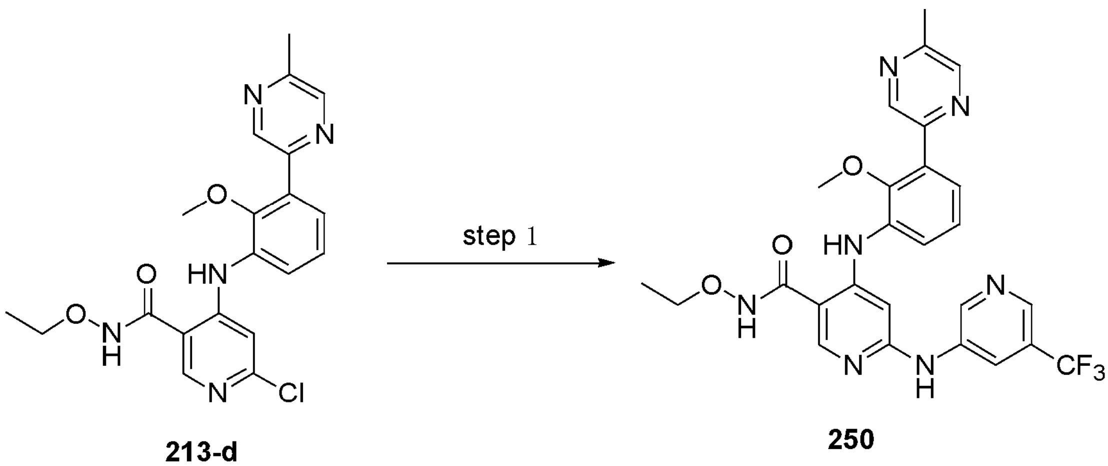


213-d 250

Step 1: To a 10ml microwave tube were sequentially added 6-chloro-N-ethoxy-4-((2-methoxy-3-(5-methyl pyrazin-2-yl)phenyl)amino)nicotinamide **(213-d,** 50 mg, 0.12mmol), 5-(trifluoromethyl)pyridin-3-ylamine (29.4 mg, 0.18mmol), $Pd_2(dba)_3$ (11.1mg, 0.012mmol), Xantphos (14.0mg, 0.024mmol), $Cs_2CO_3$ (118mg, 0.36mmol) and dioxane(2ml). After atmosphere replacement with nitrogen three times, the mixture was heated to 140°C with microwave and stirred for 3 hours under nitrogen protection. When LCMS indicated a completed reaction, the reaction mixture was cooled to room temperature, diluted with 5ml of methanol, and filtered through diatomaceous earth. The filter cake was washed with 3*5ml of methanol. The combined filtrate was subjected to rotary evaporation under reduced pressure to dryness, and

the crude material was purified by Prep-TLC (DCM: MeOH =20:1) to provide the title compound: N-ethoxy-4-((2-methoxy-3-(5-methyl pyrazin-2-yl)phenyl)amino)-6-((5-(trifluoromethyl)pyridin-3-yl)amino) nicotinamide **(250,** 40mg, 61% yield), MS Calcd: 539.19; MS Found: 540.18([M+H]). 1H NMR (400 MHz, DMSO-d6)δ11.70 (s, 1H), 10.05 (s, 1H), 9.78 (s, 1H), 8.97 (d, J = 1.6 Hz, 1H), 8.85 (d, J = 2.4 Hz, 1H), 8.81 - 8.79 (m, 1H), 8.69 (dd, J = 1.6, 0.4 Hz, 1H), 8.45 (dd, J = 2.0, 1.2 Hz, 1H), 8.41 (s, 1H), 7.60 (dd, J = 8.0, 1.6 Hz, 1H), 7.54 (dd, J = 8.0, 1.6 Hz, 1H), 7.36 (t, J = 8.0 Hz, 1H), 6.63 (s, 1H), 3.96 (q, J = 7.2 Hz, 2H), 3.54 (s, 3H), 2.57 (s, 3H), 1.23 (t, J = 7.2 Hz, 3H).

Example **251**

N-ethoxy-6-((6-fluoro-5-methyl pyridin-3-yl)amino)-4-((2-methoxy-3-(5-methyl pyrazin-2-yl)phenyl)amino)nicotinamide

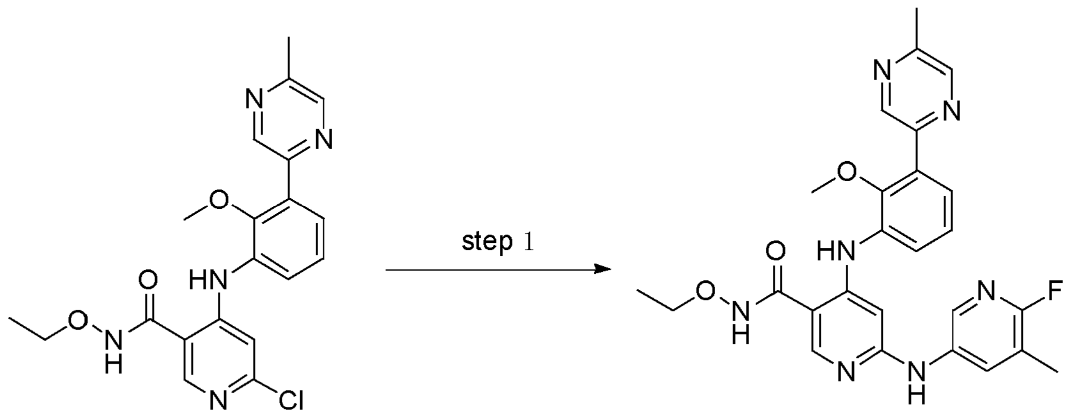


**213-d** **251**

Step 1: To a 10ml microwave tube were sequentially added 6-chloro-N-ethoxy-4-((2-methoxy-3-(5-methyl pyrazin-2-yl)phenyl)amino)nicotinamide **(213-d,** 50mg, 0.12mmol), 6-fluoro-5-methyl pyridin-3-ylamine (20.1mg, 0.15mmol), $Pd_2(dba)_3$ (11.1mg, 0.012mmol), XantPhos(14.0mg, 0.024mmol), $Cs_2CO_3$ (118mg, 0.36mmol) and dioxane(2ml). After atmosphere replacement with nitrogen three times, the mixture was heated to 140°C with microwave and stirred for 3 hours under nitrogen protection. When LCMS indicated a completed reaction, the reaction mixture was cooled to room temperature, diluted with 5ml of methanol, and filtered through diatomaceous earth. The filter cake was washed with 3*5ml of methanol. The combined filtrate was subjected to rotary evaporation under reduced pressure to dryness, and the crude material was purified by Prep-TLC (DCM: MeOH =20:1) to provide the title compound: N-ethoxy-6-((6-fluoro-5-methyl pyridin-3-yl)amino)-4-((2-methoxy-3-(5-methyl pyrazin-2-yl)phenyl)amino)nicotinamide **(251,** 40mg, 66% yield), MS Calcd: 503.21; MS Found: 504.22([M+H]). 1H NMR (400 MHz, DMSO-d6)δ11.65 (s, 1H), 10.03 (s, 1H), 9.32 (s, 1H), 8.96 (d, J = 1.6 Hz, 1H), 8.68 (d, J = 1.6 Hz, 1H), 8.33 (s, 1H), 8.22 (s, 1H), 8.08 (dd, J = 9.2, 2.4 Hz, 1H), 7.59 (dd, J = 8.0, 1.8 Hz, 1H), 7.51 (dd, J = 8.0, 1.6 Hz, 1H), 7.34 (t, J = 8.0 Hz, 1H), 6.55 (s, 1H), 3.95 (q, J = 7.2 Hz, 2H), 3.54 (s, 3H), 2.57 (s, 3H), 2.22 (s, 3H), 1.23 (t, J = 7.2 Hz, 3H).

Example **252**

6-((2,6-dimethyl pyrimidin-4-yl)amino)-N-ethoxy-4-((3-(5-isopropyl pyrazin-2-yl)-2-methoxy phenyl)amino)nicotinamide

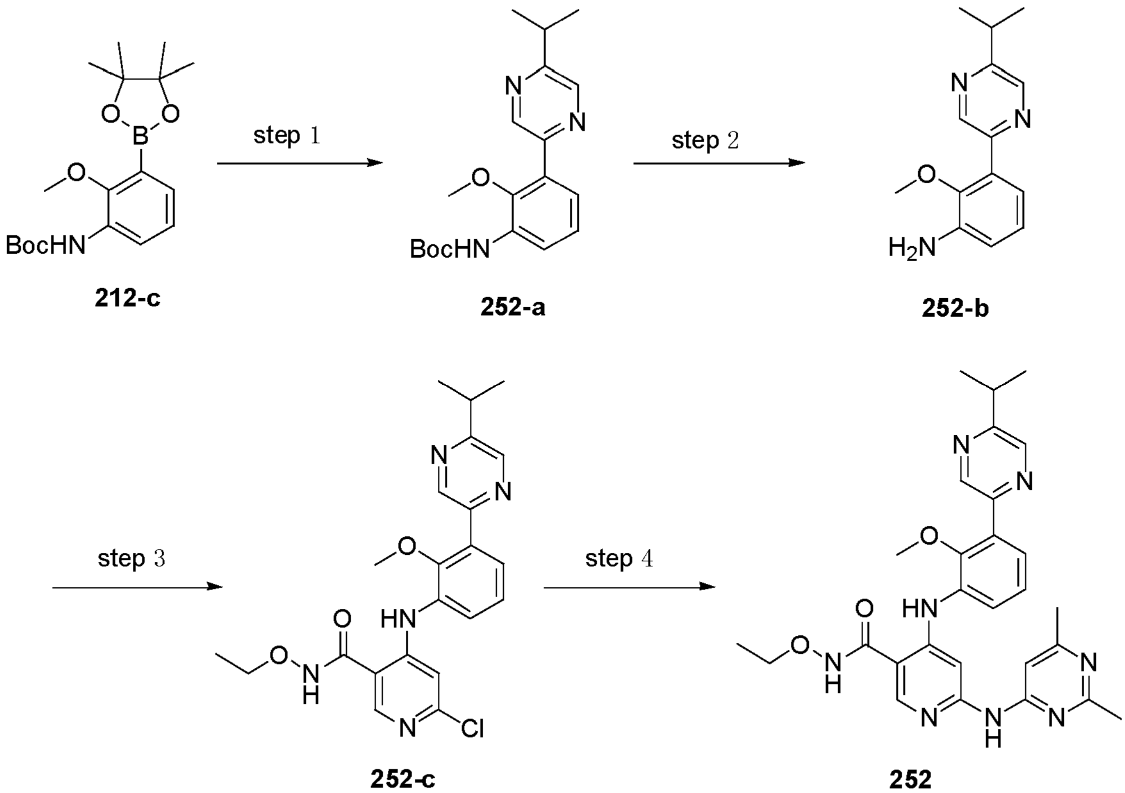


212-c 252-a 252-b

252-c 252

Step 1: To a 100ml flask were sequentially added t-butyl (2-methoxy-3-(4,4,5,5-tetramethyl-1,3,2-dioxaborolan-2-yl)phenyl)carbamate **(212-c,** 930mg, 3.7mmol), 2-bromo-5-isopropyl pyrazine (500mg, 2.49mmol), anhydrous potassium carbonate (1030mg, 7.5mmol), Pd(dppf)$Cl_2$ (181mg, 0.2mmol), dioxane(10 ml), and $H_2O$(2ml). After atmosphere replacement with nitrogen three times, the mixture was heated to 110°C and stirred under reflux for 3 hours under nitrogen protection. When TLC indicated a completed reaction, the reaction mixture was filtered and the filtrate was added with 30ml of saturated brine, extracted with3*30ml of ethyl acetate three times. The organic phases were combined, dried over anhydrous sodium sulfate, and subjected to rotary evaporation under reduced pressure to dryness. The residue was purified by flash column chromatography (EA: PE = 0 to 20% in 30min) to provide t-butyl (3-(5-isopropyl pyrazin-2-yl)-2-methoxy phenyl)carbamate **(252-a,** 500 mg, 1.45mmol, 82.7% yield). MS Calcd: 343.19; MS Found: 344.20($[M+H]^+$).

Step 2: To a 50ml flask was added t-butyl (3-(5-isopropyl pyrazin-2-yl)-2-methoxy phenyl)carbamate **(252-a,** 500 mg, 1.45mmol), followed by sequential addition of dichloromethane (5ml) and hydrochloride solution in dioxane (4M) (5ml). The mixture was stirred at room temperature for 2 hours. When TLC indicated a completed reaction, the reaction mixture was subjected to rotary evaporation under reduced pressure to dryness to provide 3-(5-isopropyl pyrazin-2-yl)-2-methoxy aniline(**252-b**, 350mg, 1.4mmol, 98% yield). MS Calcd:243.14; MS Found: 244.20($[M+H]^+$).

Step 3: To a 100ml flask were sequentially added 3-(5-isopropyl pyrazin-2-yl)-2-methoxy aniline(**252-b**, 200mg, 0.82mmol), 4,6-dichloro-N-ethoxypyridine-3-carboxamide(289mg, 1.2mmol) and DMA(5ml). The mixture wass cooled in ice bath and added with LiHMDS(2.4ml, 2.4 mol) (1M in THF). The reaction mixture was removed from ice bath and stirred at room temperature for 2 hours. When LCMS monitoring indicated that no raw material unreacted, the reaction mixture was cooled in ice bath and added with 3ml of diluted HCl solution (1M) to quench the reaction, diluted with 30ml of water, and extracted with 3*30ml of EA. The organic phases were combined, washed with 3*20ml of saturated NaCl aqueous solution. The organic phases were collected and dried over anhydrous $NaSO_4$, and subjected to rotary evaporation under reduced pressure to dryness. The crude material was purified by flash column chromatography (EA:PE = 0 to 60% in 30min), to provide 6-chloro-N-ethoxy-4-((3-(5-isopropyl pyrazin-2-yl)-2-methoxy phenyl)amino)nicotinamide **(252-c,** 190mg, 52% yield). MS Calcd: 441.16; MS Found: 442.14($[M+H]^+$).

Step 4: To a 10ml microwave tube were sequentially added 6-chloro-N-ethoxy-4-((3-(5-isopropyl pyrazin-2-yl)-2-methoxy phenyl)amino)nicotinamide **(252-c,** 50mg, 0.11mmol), 2,6-dimethyl pyrimidin-4-ylamine (20.9mg, 0.2mmol), $Pd_2(dba)_3$(10.4mg, 0.011mmol), XantPhos(13.0mg, 0.022mmol), $Cs_2CO_3$ (110mg, 0.3mmol) and dioxane(2ml). After atmosphere replacement with nitrogen three times, the mixture was heated to 140°C with microwave and stirred for 3 hours under nitrogen protection. When LCMS indicated a completed reaction, the reaction mixture was cooled to room temperature, diluted with 5ml of methanol, and filtered through diatomaceous earth. The filter cake was washed with 3*5ml of methanol. The combined filtrate was subjected to rotary evaporation under reduced pressure to dryness, and the crude material was purified by Prep-TLC(DCM: MeOH =20:1) to provide the title compound: 6-((2,6-dimethyl pyrimidin-4-yl)amino)-N-ethoxy-4-((3-(5-isopropyl pyrazin-2-yl)-2-methoxy phenyl)amino)nicotinamide **(252,** 15mg, 22% yield), MS Calcd: 528.26; MS Found: 529.26([M+H]$^+$). $^1$H NMR (400 MHz, DMSO-d6)δ11.71 (s, 1H), 10.22 (s, 1H), 10.11 (s, 1H), 8.66 (d, J = 2.4 Hz, 1H), 8.57 (d, J = 2.4 Hz, 1H), 8.39 (s, 1H), 8.21 (s, 1H), 7.73 (d, J = 8.0 Hz, 1H), 7.33 (t, J = 8.0 Hz, 1H), 7.11 - 7.05 (m, 2H), 3.95 (q, J = 7.2 Hz, 2H), 3.38 (s, 3H), 2.97-2.90 (m, 1H), 2.40 (s, 3H), 2.29 (s, 3H), 1.25-1.15 (m, 6H), 1.10-0.97 (m, 3H).

Example **253 (control)**

4-((3-(5-fluoropyrimidin-2-yl)-2-methoxy phenyl)amino)-N-(methyl-d3)-6-(pyridin-2-ylamino)nicotinamide

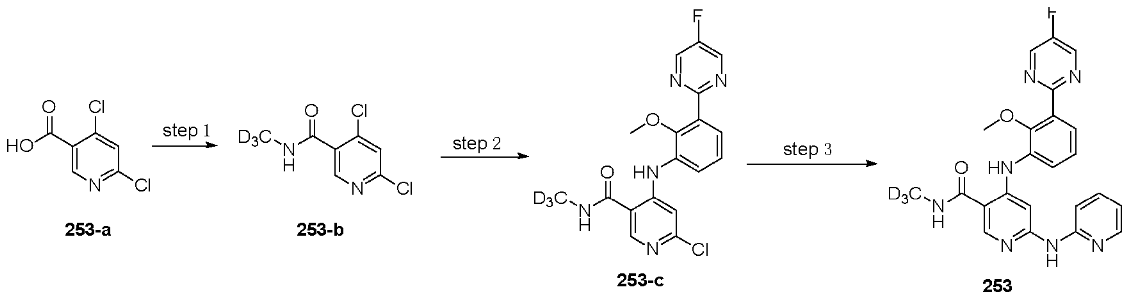


253-a 253-b 253-c 253

Step 1: To a 100ml flask were added 4,6-dichloronicotinic acid **(253-a,** 300mg, 1.56mmol) and 10ml of DCM. The mixture wass cooled in ice bath and slowly added with oxalyl chloride (0.4ml, 4.7mmol) dropwise. Two drops of DMF were added for catalyzing the reaction, with large amount of bubbles produced. The reaction was allowed to natually warm back to room temperature and stirred for 1hr. Sample was taken and mixed with methanol, new spot on TLC indicated the depletion of acyl chloride. The mxiture was subjected to rotary evaporation under reduced pressure to dry for removing excessive oxalyl chloride, and added with 10ml of DCM. The mixture was added with deuterated methylamine hydrochloride (79mg, 2.3mmol), cooled in ice bath, added with DIEA(1.3ml, 7.5mmol) dropwise, and stirred overnight. When LCMS indicated that the majortiy was the target compound, the reaction mixture was diluted with 20ml of DCM, and washed with 3*5ml of saturated brine. The organic phase was dried over anhydrous sodium sulfate, and subjected to rotary evaporation under reduced pressure to dryness. The crude material was purified by flash column chromatography (EA:PE=0 to 30% in 20min) to provide 4,6-dichloro-N-(methyl-d3)nicotinamide **(253-b,** 230mg, 1.1mmol, 70% yield)MS Calcd: 207.00; MS Found: 208.01 ([M+H]$^+$). Step 2: To a 100ml flask were sequentially added 3-(5-fluoropyrimidin-2-yl)-2-methoxy aniline(**208-d**, 110mg, 0.5mmol), 4,6-dichloro-N-(methyl-d3)nicotinamide **(253-b,** 156mg, 0.75mmol) and THF(5ml). The mixture was cooled in ice bath and added with LiHMDS(1.5ml, 1.5 mol) (1M in THF). The reaction mixture was removed from ice bath and stirred at room temperature for 2 hours. When LCMS monitoring indicated that no raw material unreacted, the reaction mixture was cooled in ice bath and added with 3ml of diluted HCl solution (1M) to quench the reaction, diluted with 30ml of water, and extracted with 3*30ml of EA. The organic phases were combined, washed with 3*20ml of saturated NaCl aqueous solution. The organic phases were collected, dried over anhydrous $NaSO_4$, and subjected to rotary evaporation under reduced pressure to dryness. The crude material was purified by flash column chromatography (EA:PE = 0 to 60% in 30min), to provide 6-chloro-4-((3-(5-fluoropyrimidin-2-yl)-2-methoxy phenyl) amino)-N-(methyl-d3)nicotinamide **(253-c,** 88mg, 0.2 mmol, 44% yield)MS Calcd: 390.11; MS Found: 391.10([M+H]$^+$).

Step 3: To a 10ml microwave tube were sequentially added 6-chloro-4-((3-(5-fluoropyrimidin-2-yl)-2-methoxy phenyl)amino)-N-(methyl-d3)nicotinamide **(253-c,** 70mg, 0.18mmol), pyridin-2-ylamine (25.5 mg, 0.3mmol), $Pd_2(dba)_3$ (16.5mg, 0.018mmol), XantPhos(20.9mg, 0.036mmol), $Cs_2CO_3$ (176mg, 0.5mmol) and dioxane(2ml). After atmosphere replacement with nitrogen three times, the mixture was heated to 140°C with microwave and stirred for 3 hours under nitrogen protection. When LCMS indicated a completed reaction, the reaction mixture was cooled to room temperature, diluted with 5ml of methanol, and filtered through diatomaceous earth. The filter cake was washed with 3*5ml of methanol. The combined filtrate was subjected to rotary evaporation under reduced pressure to dryness, and the crude material was purified by Prep-TLC (DCM: MeOH =20:1) to provide the title compound 4-((3-(5-fluoropyrimidin-2-yl)-2-methoxy phenyl) amino)-N-(methyl-d3)-6-(pyridin-2-ylamino)nicotinamide **(253,** 30mg, 36.4% yield), MS Calcd: 448.19; MS Found: 449.21([M+H]$^+$). $^1$H NMR (400 MHz, DMSO-d6)δ10.71 (s, 1H), 9.76 (s, 1H), 9.04 (s, 2H), 8.51 (s, 1H), 8.49 (s, 1H), 8.18 (d, J = 4.4 Hz, 1H), 7.95 (s, 1H), 7.72 (d, J = 8.0 Hz, 1H), 7.65 (t, J = 7.2 Hz, 1H), 7.55 (d, J = 8.4 Hz, 1H), 7.40 (d, J = 7.6 Hz, 1H), 7.32 (t, J = 8.0 Hz, 1H), 6.87 (t, J = 6.0 Hz, 1H), 3.70 (s, 3H).

Example **254**

6-(2,2-difluorocyclopropyl-1-carboxamido)-N-ethoxy-4-((2-methoxy-3-(5-methyl pyrazin-2-yl)phenyl)amino)nicotinamide

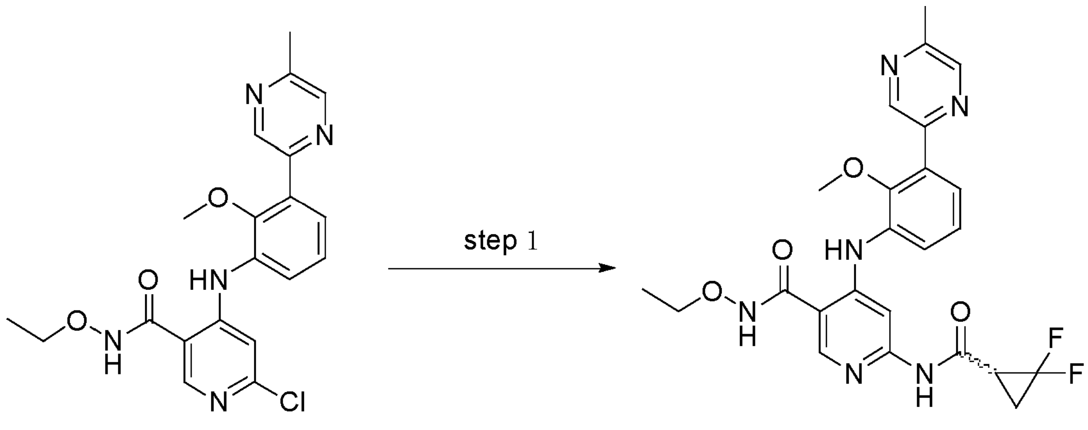


**213-d** **254**

Step 1: To a 10ml microwave tube were sequentially added 6-chloro-N-ethoxy-4-((2-methoxy-3-(5-methyl pyrazin-2-yl)phenyl)amino)nicotinamide **(213-d,** 50 mg, 0.12mmol), 2,2-difluorocyclopropyl-1-carboxamide(17.6 mg, 0.14mmol), $Pd_2(dba)_3$(11.1mg, 0.012mmol), XantPhos(14.0mg, 0.024mmol), $Cs_2CO_3$ (118mg, 0.36mmol) and dioxane(2ml). After atmosphere replacement with nitrogen three times, the mixture was heated to 140°C with microwave and stirred for 3 hours under nitrogen protection. When LCMS indicated a completed reaction, the reaction mixture was cooled to room temperature, diluted with 5ml of methanol, and filtered through diatomaceous earth. The filter cake was washed with 3*5ml of methanol, and the combined filtrate was subjected to rotary evaporation under reduced pressure to dryness. The crude material was purified by Prep-TLC(DCM: MeOH =20:1) to provide the title compound: 6-(2,2-difluorocyclopropyl-1-carboxamido)-N-ethoxy-4-((2-methoxy-3-(5-methyl pyrazin-2-yl)phenyl)amino)nicotinamide **(254,** 15mg, 24% yield), MS Calcd: 498.18; MS Found: 498.90($[M+H]^+$). $^1$H NMR (400 MHz, DMSO-d6)δ11.82 (s, 1H), 11.03 (s, 1H), 10.20 (s, 1H), 8.95 (d, J = 1.6 Hz, 1H), 8.68 (d, J = 1.6 Hz, 1H), 8.42 (s, 1H), 8.02 (s, 1H), 7.57 - 7.46 (m, 2H), 7.33 (t, J = 8.0 Hz, 1H), 3.96 (q, J = 7.2 Hz, 2H), 3.51 (s, 3H), 3.01-2.92 (m, 1H), 2.56 (s, 3H), 2.02-1.92 (m, 2H), 1.23 (t = 7.2 Hz, 3H).

Example **255**

6-((2,6-dimethyl pyrimidin-4-yl)amino)-N-ethoxy-4-((3-(5-fluoropyridin-2-yl)-2-methoxy phenyl)amino)nicotinamide

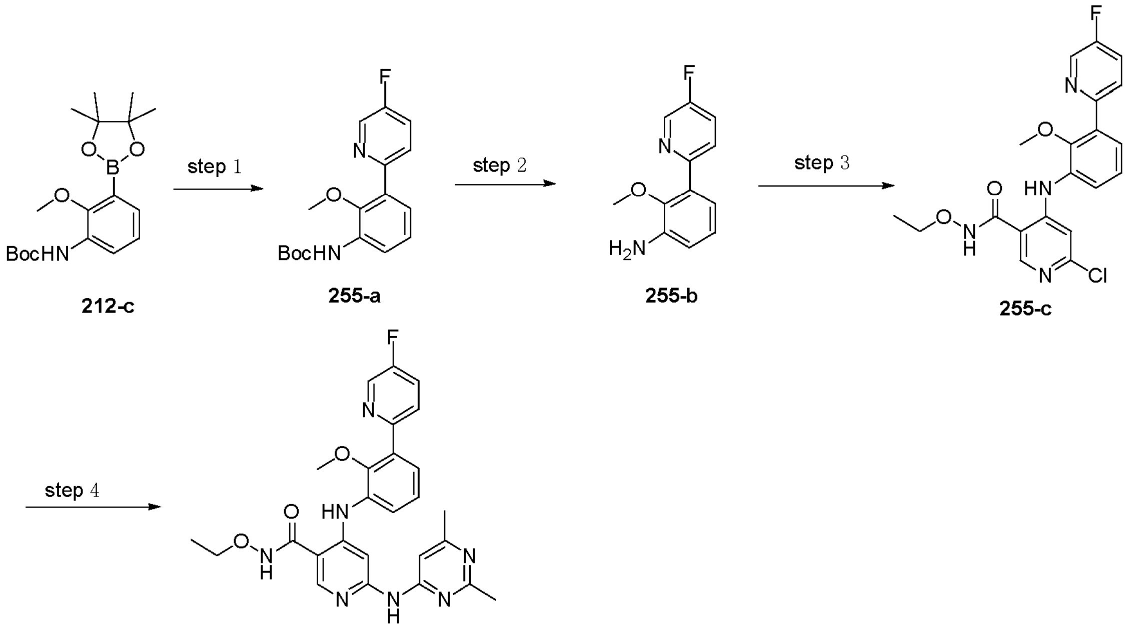


212-c 255-a 255-b 255-c 255

Step 1: To a 100ml flask were sequentially added t-butyl (2-methoxy-3-(4,4,5,5-tetramethyl-1,3,2-dioxaborolan-2-yl)phenyl)carbamate **(212-c,** 400mg, 1.15mmol), 2-bromo-5-fluoropyridine (302mg, 1.72mmol), anhydrous potassium carbonate (1.1g, 3.4mmol), Pd(dppf)$Cl_2$ (125mg, 0.115mmol), dioxane(10 ml), and $H_2O$(2ml). After atmosphere replacement with nitrogen three times, the mixture was heated to 110°C and stirred under reflux for 3 hours under nitrogen protection. When TLC indicated a completed reaction, the reaction mixture was filtered and the filtrate was added with 30ml of saturated brine, and extracted with 3*30ml of ethyl acetate three times. The organic phases were combined, dried over anhydrous sodium sulfate, and subjected to rotary evaporation under reduced pressure to dryness. The residue was purified by flash column chromatography (ethyl acetate:petroleum ether = 0 to 20% in 30min) to provide t-butyl (3-(5-fluoropyridin-2-yl)-2-methoxy phenyl)carbamate **(255-a,** 190mg, 0.6mmol, 52% yield). MS Calcd: 318.14; MS Found: 319.16([M+H]$^+$).

Step 2: To a 50ml flask was added t-butyl (3-(5-fluoropyridin-2-yl)-2-methoxy phenyl)carbamate **(255-a,** 190 mg, 0.6mmol), followed by sequential additon of dichloromethane (5ml) and hydrochloride solution in dioxane (4M) (5ml). The mxiture was stirred at room temperature for 2 hours. When TLC indicated a completed reaction, the reaction mixture subjected to rotary evaporation under reduced pressure to dryness to provide 3-(5-fluoropyridin-2-yl)-2-methoxy aniline(**255-b**, 120mg, 0.6mmol, 92% yield). MS Calcd:218.09; MS Found: 219.12([M+H]$^+$).

Step 3: To a 100ml flask were sequentially added 3-(5-fluoropyridin-2-yl)-2-methoxy aniline(**255-b**, 120mg, 0.6mmol), 4,6-dichloro-N-ethoxypyridine-3-carboxamide(142mg, 0.66mmol) and DMA(5ml). The mixture was cooled in ice bath and added with LiHMDS(1.8ml, 1.80 mol) (1M in THF). The reaction mixture was removed from ice bath and stirred at room temperature for 2 hours. When LCMS monitoring indicated that no raw material unreacted, the reaction mixture was cooled in ice bath and added with 3ml of diluted HCl solution (1M) to quench the reaction, diluted with 30ml of water, and extracted with 3*30ml of EA. The organic phases were combined, washed with 3*20ml of saturated NaCl. The organic phases were collected, dried over anhydrous $NaSO_4$, and subjected to rotary evaporation under reduced pressure to dryness. The crude material was purified by flash column chromatography (EA:PE = 0 to 60% in 30min), to provide 6-chloro-N-ethoxy-4-((3-(5-fluoropyridin-2-yl)-2-methoxy phenyl)amino)nicotinamide **(255-c,** 140mg, 0.3 mmol, 61% yield). MS Calcd: 416.11; MS Found: 417.12([M+H]$^+$).

Step 4: To a 10ml microwave tube were sequentially added 6-chloro-N-ethoxy-4-((3-(5-fluoropyridin-2-yl)-2-methoxy phenyl)amino)nicotinamide **(255-c,** 80mg, 0.19mmol), 2,6-dimethyl pyrimidin-4-ylamine (28mg, 0.23mmol), $Pd_2(dba)_3$ (17.6mg, 0.019mmol), XantPhos(22.2mg, 0.038mmol), $Cs_2CO_3$ (187mg, 0.57mmol) and dioxane(2ml). After atmosphere replacement with nitrogen three times, the mixture was heated to 140°C with microwave and stirred for 3 hours under nitrogen protection. When LCMS indicated a completed reaction, the reaction mixture was cooled to room temperature, diluted with 5ml of methanol, and filtered through diatomaceous earth. The filter cake was washed with 3*5ml of methanol. The combined filtrate was subjected to rotary evaporation under reduced pressure to dryness, and the crude material was purified by Prep-TLC (DCM: MeOH =20:1) to provide the title compound: 6-((2,6-dimethyl pyrimidin-4-yl) amino)-N-ethoxy-4-((3-(5-fluoropyridin-2-yl)-2-methoxy phenyl)amino)nicotinamide **(255,** 60mg, 61% yield), MS Calcd: 503.21; MS Found: 504.46([M+H]$^+$). $^1$H NMR (400 MHz, DMSO-d6)δ11.71 (s, 1H), 10.27 (s, 1H), 10.10 (s, 1H), 8.71 (d, J = 3.2 Hz, 1H), 8.40 (s, 1H), 8.15 (s, 1H), 7.97 - 7.92 (m, 1H), 7.84 (td, J = 8.8, 3.2 Hz, 1H), 7.67 (dd, J = 8.0, 1.6 Hz, 1H), 7.46

(dd, J = 8.0, 1.6 Hz, 1H), 7.32 (t, J = 8.0 Hz, 1H), 7.11 (s, 1H), 3.96 (q, J = 7.2 Hz, 2H), 3.50 (s, 3H), 2.39 (s, 3H), 2.29 (s, 3H), 1.24 (t, J = 7.2 Hz, 3H).

Example **256**

N-ethoxy-4-((3-(5-fluoropyrimidin-2-yl)-2-methoxy phenyl)amino)-6-(pyrimidin-2-ylamino)nicotinamide

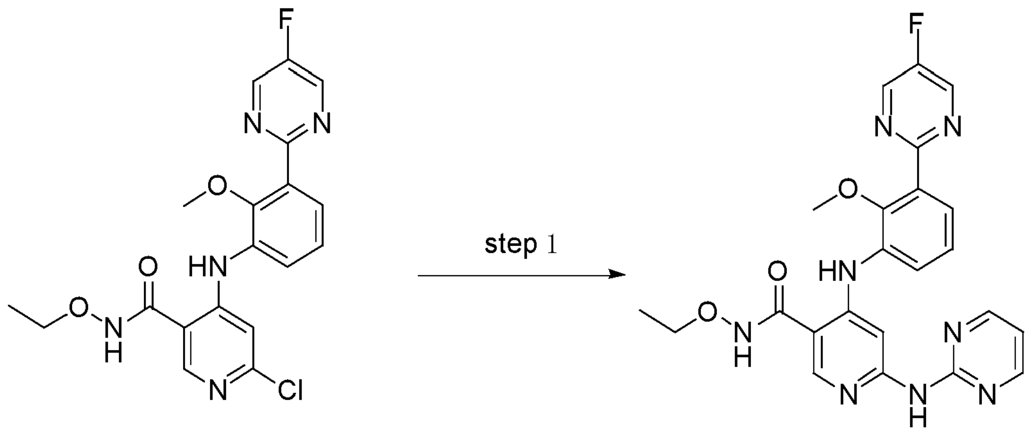


**208-e** **256**

Step 1: To a 10ml microwave tube were sequentially added 6-chloro-N-ethoxy-4-((3-(5-fluoropyrimidin-2-yl)-2-methoxy phenyl)amino)nicotinamide **(208-e,** 100mg, 0.23mmol), pyrimidin-2-ylamine (34mg, 0.35mmol), $Pd_2(dba)_3$ (21mg, 0.023mmol), XantPhos (28mg, 0.046mmol), $Cs_2CO_3$ (224mg, 0.69mmol) and dioxane(2ml). After atmosphere replacement with nitrogen three times, the mixture was heated to 140°C with microwave and stirred for 3 hours under nitrogen protection. When LCMS indicated a completed reaction, the reaction mixture was cooled to room temperature, diluted with 5ml of methanol, and filtered through diatomaceous earth. The filter cake was washed with 3*5ml of methanol. The combined filtrate was subjected to rotary evaporation under reduced pressure to dryness, and the crude material was purified by Prep-TLC (DCM: MeOH =20:1) to provide the title compound: N-ethoxy-4-((3-(5-fluoropyrimidin-2-yl)-2-methoxy phenyl)amino)-6-(pyrimidin-2-ylamino)nicotinamide **(256,** 60mg, 54% yield), MS Calcd: 476.17; MS Found: 477.20($[M+H]^+$). $^1H$ NMR (400 MHz, DMSO-d6)δ11.74 (s, 1H), 10.27 (s, 1H), 10.01 (s, 1H), 9.05 (d, J = 0.8 Hz, 2H), 8.55 (s, 1H), 8.54 (s, 1H), 8.43 (s, 1H), 8.40 (s, 1H), 7.77 (dd, J = 8.0, 1.6 Hz, 1H), 7.43 (dd, J = 8.0, 1.6 Hz, 1H), 7.37 (t, J = 7.6 Hz, 1H), 6.98 (t, J = 4.8 Hz, 1H), 3.96 (q, J = 7.2 Hz, 2H), 3.70 (s, 3H), 1.23 (t, J = 7.2 Hz, 3H).

Example **257**

N-ethoxy-4-((3-(5-fluoropyrimidin-2-yl)-2-methoxy phenyl)amino)-6-((5-fluoropyrimidin-2-yl)amino)nicotinamide

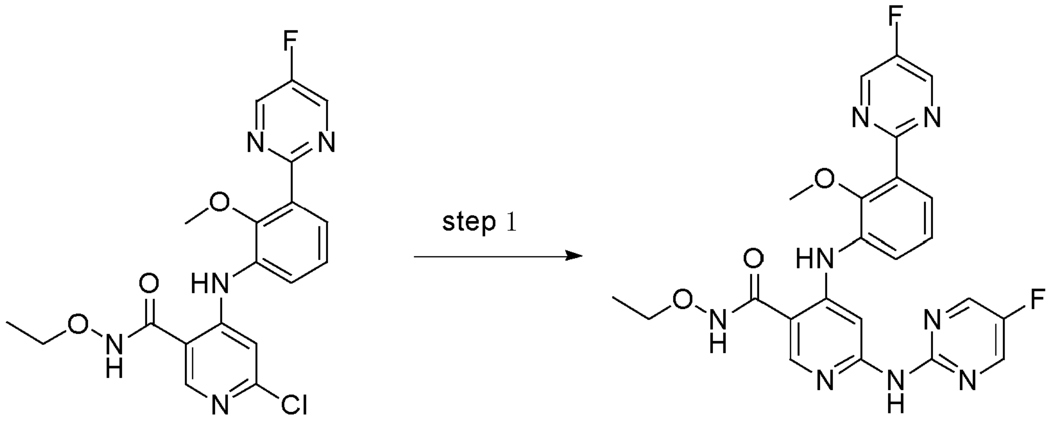


208-e

257

Step 1: To a 10ml microwave tube were sequentially added 6-chloro-N-ethoxy-4-((3-(5-fluoropyrimidin-2-yl)-2-methoxy phenyl)amino)nicotinamide **(208-e,** 100mg, 0.23mmol), 5-fluoropyrimidin-2-ylamine (38mg, 0.34mmol), $Pd_2(dba)_3$(21mg, 0.023mmol), XantPhos (28mg, 0.046mmol), $Cs_2CO_3$ (224mg, 0.69mmol) and dioxane(2ml). After atmosphere replacement with nitrogen three times, the mixture was heated to 140°C with microwave and stirred for 3 hours under nitrogen protection. When LCMS indicated a completed reaction, the reaction mixture was cooled to room temperature, diluted with 5ml of methanol, and filtered through diatomaceous earth. The filter cake was washed with 3*5ml of methanol. The combined filtrate was subjected to rotary evaporation under reduced pressure to dryness, and the crude material was purified by Prep-TLC (DCM: MeOH =20:1) to provide the title compound: N-ethoxy-4-((3-(5-fluoropyrimidin-2-yl)-2-methoxy phenyl)amino)-6-((5-fluoropyrimidin-2-yl)amino)nicotinamide **(257,** 60mg, 52% yield), MS Calcd: 494.16; MS Found: 495.18($[M+H]^+$). $^1$H NMR (400 MHz, DMSO-$d_6$)δ11.71 (s, 1H), 10.20 (s, 1H), 9.05 (d, *J* = 0.8 Hz, 2H), 8.64 (s, 2H), 8.40 (s, 1H), 8.30 (s, 1H), 7.76 (dd, *J* = 7.2, 2.4 Hz, 1H), 7.55 7.29 (m, 2H), 3.96 (q, *J* = 7.2 Hz, 2H), 3.70 (s, 3H), 1.23 (t, *J* = 7.2 Hz, 3H).

Example **258**

6-((2,6-dimethyl pyrimidin-4-yl)amino)-N-ethoxy-4-((5-fluoro-2-methoxy-3-(pyrazin-2-yl)phenyl)amino)nicotinamide

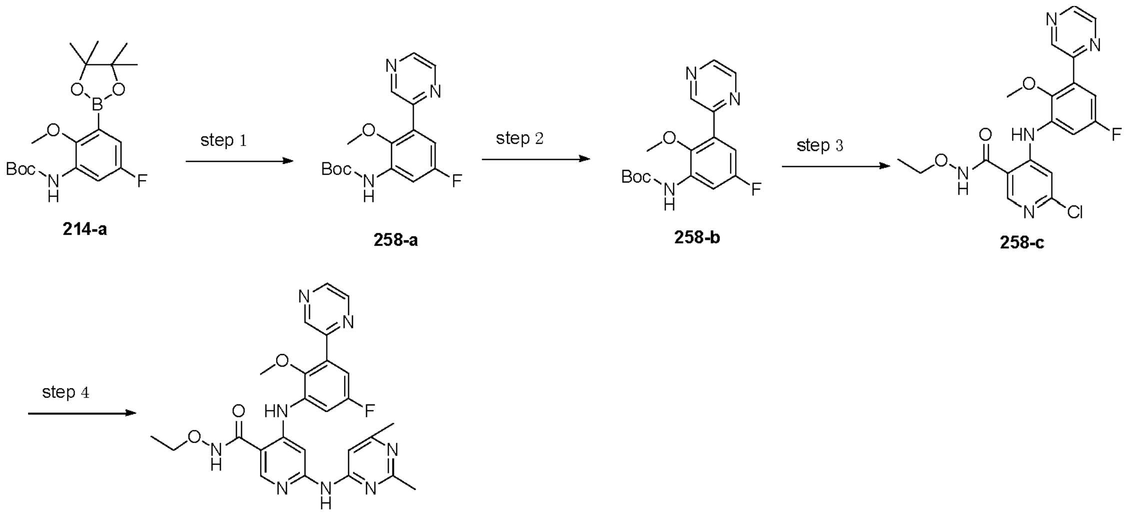


214-a

258-a

258-b

258-c

258

Step 1: To a 50ml flask were added t-butyl (5-fluoro-2-methoxy-3-(4,4,5,5-tetramethyl-1,3,2-dioxaborolan-2-yl) phenyl)carbamate **(214-a,** 200mg, 0.54mmol), 2-bromopyrazine (103mg, 0.65mmol), Pd(dppf)$Cl_2$(44.5mg, 0.1mmol), potassium phosphate (345mg, 1.5mmol), followed by 1,4-dioxane (4ml) and water (1ml). After atmosphere replacement with nitrogen three times, the mixture was allowed to react at 110°C for 4 hours. When the reaction was found completed by

sampling and testing, the mixture was concentrated under reduced pressure to dryness. The residue was added with water and extracted with ethyl acetate, to provide t-butyl (5-fluoro-2-methoxy-3-(pyrazin-2-yl)phenyl)carbamate **(258-a,** 172mg, 0.5mmol, 100% yield). MS Calcd: 319.3; MS Found:320.5 ($[M+H]^+$)

Step 2: To a 25ml flask was added t-butyl (5-fluoro-2-methoxy-3-(pyrazin-2-yl)phenyl)carbamate **(258-a,** 172mg, 0.5mmol), followed by DCM(3ml) and TFA(1ml), and stirred at room temperature for 2 hours. When the reaction was found completed by sampling and testing, the mixture was concentrated under reduced pressure to dryness. The residue was neutralized with saturated sodium carbonate solution, and extracted with ethyl acetate. The organic phase was dried and concentrated under reduced pressure to dryness to provide 5-fluoro-2-methoxy-3-(pyrazin-2-yl)aniline **(258-b,** 99mg, 0.4mmol, 75.3% yield). MS Calcd: 219.2; MS Found:220.11 ($[M+H]^+$). Step 3: To a 25ml two-necked flask were added 5-fluoro-2-methoxy-3-(pyrazin-2-yl)aniline **(258-b,** 99mg, 0.45mmol) and 4,6-dichloro-N-ethoxypyridine-3-carboxamide(158.6mg, 0.67mmol), followed by anhydrous THF(3ml). After atmosphere replacement with nitrogen three times, the mixture was cooled in ice bath, added with LHMDS(2.2mmol, 2.2ml), and allowed to react at room temperature for 2 hours. Upon TLC indicated a completed reaction, the mixture was quenched with saturated ammonium chloride solution, and extracted with ethyl acetate. The organic phase was concentrated under reduced pressure to dryness. The residue was mixed with silica gel for loading onto and purifying by column chromatography (PE:ethyl acetate=1:1), to provide 6-chloro-N-ethoxy-4-((5-fluoro-2-methoxy-3-(pyrazin-2-yl)phenyl)amino)nicotinamide **(258-c,** 112mg, 0.26mmol, 60% yield). MS Calcd: 417.10; MS Found:418.14 ($[M+H]^+$).

Step 4: 6-chloro-N-ethoxy-4-((5-fluoro-2-methoxy-3-(pyrazin-2-yl)phenyl)amino)nicotinamide **(258-c,** 50mg, 0.12mmol), 4-amino-2,6-dimethyl pyrimidine (30mg, 0.24mmol), pd2(dba)3 (21mg, 0.024mmol), xantphos(27mg, 0.048mmol), $Cs_2CO_3$ (117mg, 0.4mmol) were mixed and added with 1,4-dioxane (1ml). After atmosphere replacement with nitrogen three times, the mixture was heated with microwave to 140°C and allowed to react for 3.5 hours. When TLC indicated a completed reaction, the mixture was filtered by suction, and the filtrate was concentrated under reduced pressure at 40°C. The residue was separated and purified by plate chromatography, eluted with DCM:MeOH=20:1, to provide the title compound: 6-((2,6-dimethyl pyrimidin-4-yl)amino)-N-ethoxy-4-((5-fluoro-2-methoxy-3-(pyrazin-2-yl)phenyl)amino)nicotinamide **(258,** 32mg, 0.063mmol, 52.9% yield). MS Calcd: 504.20; MS Found: 505.21 ($[M+H]^+$). $^1H$ NMR (400 MHz, DMSO-$d_6$)δ11.78 (s, 1H), 10.58 (s, 1H), 10.19 (s, 1H), 9.15 (d, *J* = 1.6 Hz, 1H), 8.82 (dd, *J* = 2.4, 1.6 Hz, 1H), 8.70 (d, *J* = 2.4 Hz, 1H), 8.45 (s, 1H), 8.19 (s, 1H), 7.59 (dd, *J* = 10.0, 3.2 Hz, 1H), 7.29 (dd, *J* = 9.2, 3.2 Hz, 1H), 7.19 (s, 1H), 3.97 (q, *J* = 7.2 Hz, 2H), 3.55 (s, 3H), 2.42 (s, 3H), 2.30 (s, 3H), 1.24 (t, *J* = 7.2 Hz, 3H).

Example **259**

N-methoxy-4-((5-fluoro-2-methoxy-3-(5-methyl pyrazin-2-yl)phenyl)amino)-6-((6-fluoro-2-methyl pyridin-3-yl)amino)nicotinamide

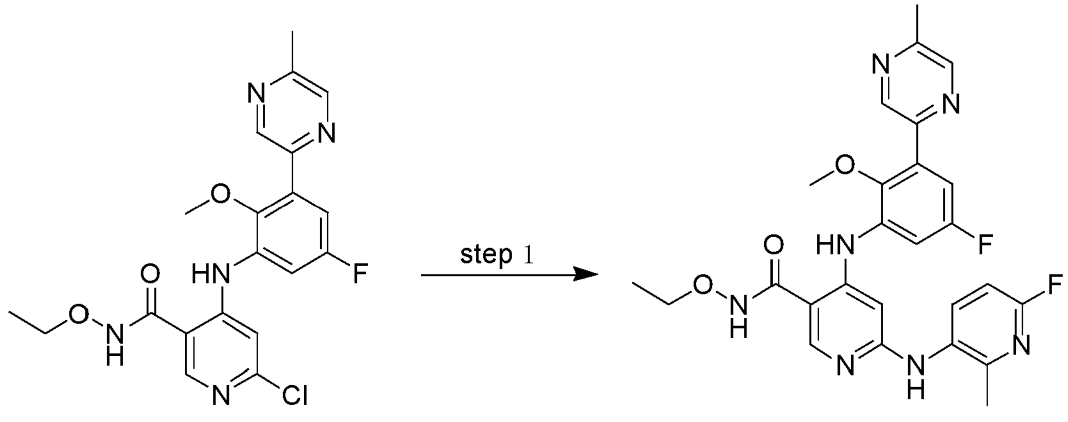


**214-d** **259**

Step 1: 6-chloro-N-ethoxy-4-((5-fluoro-2-methoxy-3-(5-methyl pyrazin-2-yl)phenyl)amino)nicotinamide **(214-d,** 50mg, 0.12mmol), 6-fluoro-2-methyl pyridin-3-ylamine (30mg, 0.24mmol), $Pd_2(dba)_3$(21mg, 0.024mmol), XantPhos(27mg, 0.048mmol), $Cs_2CO_3$ (117mg, 0.4mmol) were mixed and added with 1,4-dioxane (1ml). After atmosphere replacement with nitrogen three times, the mixture was heated with microwave to 140°C and allowed to react for 3.5 hours. When TLC indicated a completed reaction, the mixture was filtered by suction, and the filtrate was concentrated under reduced pressure at 40°C. The residue was separated and purified by plate chromatography, eluted with

DCM:MeOH=20:1, to provide the title compound: N-methoxy-4-((5-fluoro-2-methoxy-3-(5-methyl pyrazin-2-yl)phenyl) amino)-6-((6-fluoro-2-methyl pyridin-3-yl)amino)nicotinamide **(259,** 18mg, 0.053mmol, 28.8% yield). MS Calcd: 521.20; MS Found: 522.20 ([M+H]$^+$). $^1$H NMR (400 MHz, DMSO-$d_6$)δ11.65 (s, 1H), 10.34 (s, 1H), 9.02 (d, $J$ = 1.6 Hz, 1H), 8.70 (d, $J$ = 1.6 Hz, 1H), 8.68 (s, 1H), 8.24 (s, 1H), 8.10 (t, $J$ = 8.4 Hz, 1H), 7.45 (dd, $J$ = 10.0, 3.2 Hz, 1H), 7.25 (dd, $J$ = 10.0, 3.2 Hz, 1H), 6.96 (dd, $J$ = 8.4, 3.2 Hz, 1H), 6.73 (s, 1H), 3.94 (q, $J$ = 7.2 Hz, 2H), 3.53 (s, 3H), 2.58 (s, 3H), 2.37 (s, 3H), 1.21 (t, $J$ = 7.2 Hz, 3H).

Example **260**

4-((3-(5-chloropyrimidin-2-yl)-2-methoxy phenyl)amino)-6-(2,2-difluorocyclopropyl-1-carboxamido)-N-ethoxy nicotinamide

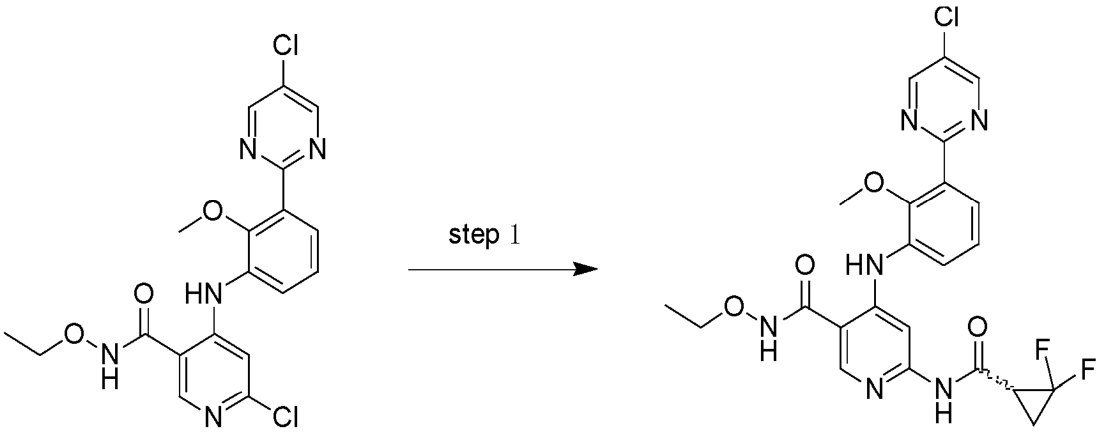


218-c

260

Step 1: To a 25ml flask were added 6-chloro-4-((3-(5-chloropyrimidin-2-yl)-2-methoxy phenyl (amino(-N-ethoxy nicotinamide **(218-c,** 80mg, 0.18mmol), 2,6-dimethyl pyrimidin-4-ylamine (24.5mg, 0.20mmol), $Pd(dba)_3$(33.7mg, 0.036mmol), XantPhos(42.6mg, 0.072mmol), and cesium carbonate (180mg, 0.6mmol), followed by 1,4-dioxane (3ml). After atmosphere replacement with nitrogen three times, the mixture was heated with microwave to 130°C and allowed to react for 2 hours. Upon TLC indicated a completed reaction, the mixture was concentrated to dryness. The residue was separated and purified by reverse phase column chromatography (water containing 0.05% formic acid: acetonitrile, the peak appeared at 30% acetonitrile), followed by high performance preparative thin layer chromatography (DCM:MeOH=20:1), to provide the title compound: 4-((3-(5-chloropyrimidin-2-yl-(2-methoxy phenyl (amino(-6-(2,2-difluorocyclopropyl-1-carboxamido)-N-ethoxy nicotinamide **(260,** 13mg, 0.025mmol, 12.5% yield). MS Calcd: 518.13; MS Found: 519.14([M+H]$^+$). $^1$H NMR (400 MHz, DMSO-$d_6$)δ11.81 (s, 1H), 11.04 (s, 1H), 10.17 (s, 1H), 9.08 (s, 2H), 8.42 (s, 1H), 8.02 (s, 1H), 7.60 (dd, $J$ = 8.0, 1.6 Hz, 1H), 7.51 (dd, $J$ = 8.0, 1.6 Hz, 1H), 7.31 (t, $J$ = 8.0 Hz, 1H), 3.96 (q, $J$ = 7.2 Hz, 2H), 3.69 (s, 3H), 3.00-2.92 (m, 1H), 2.03-1.95 (m, 2H), 1.23 (t, $J$ = 7.2 Hz, 3H).

Example **261**

6-((2,6-dimethyl pyrimidin-4-yl)amino)-N-ethoxy-4-((5-fluoro-3-(5-isopropyl pyrazin-2-yl)-2-methoxy phenyl)amino)nicotinamide

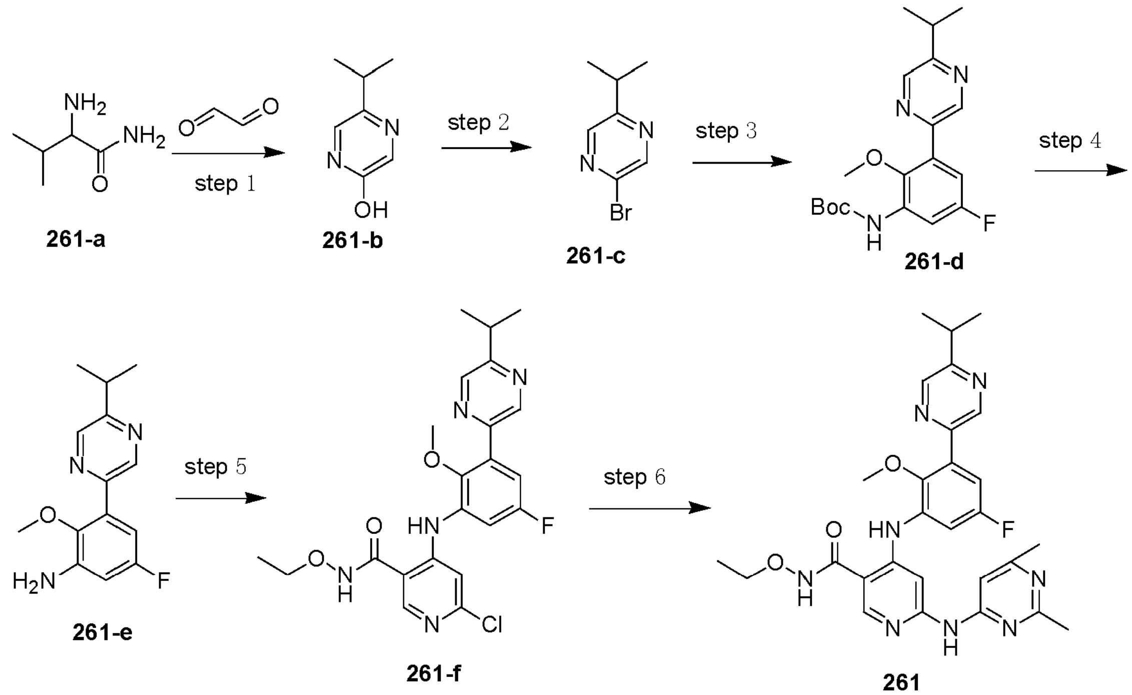


261-a 261-b 261-c 261-d 261-e 261-f 261

Step 1: To a 250ml flask was added DL-valinamide hydrochloride **(261-a,** 3g, 19.66mmol), followed by methanol (30ml) and water (30ml). The mixture was cooled to -40°C, slowly added with glyoxal (1.5g, 25.56mmol), and stirred for 10 minutes. After adding 50% sodium hydroxide aqueous solution (4.8ml), the mixture was allowed to react at room temperature for 6 hours. Upon TLC indicated a completed reaction, the mixture was cooled in ice bath and slowly added with concentrated aqueous hydrochloride (6ml), and stirred for 10minutes. After adding 9.9g of sodium bicarbonate, the mixture was stirred for 10 minutes and extracted with ethyl acetate. The organic phase was concentrated under reduced pressure to dryness to provide 5-(prop-2-yl)pyrazin-2-ol **(261-b,** 2.3g, 15mmol, 76.2% yield). MS Calcd: 138.08; MS Found: 139.13 ($[M+H]^+$).

Step 2: To a 50ml flask was added 5-(prop-2-yl)pyrazin-2-ol **(261-b,** 500mg, 3.62mmol), followed by acetonitrile (10ml). The reaction mixture was placed in a water bath and slowly added with phosphorus oxybromide (3.11g, 10.9mmol). After the addition, the mixutre was heated to 85°C to react for 3 hours. Upon TLC indicated a completed reaction, the mixture was poured into saturated sodium bicarbonate aqueous solution, and extracted with ethyl acetate. The organic phase was concentrated under reduced pressure to dryness to provide 2-bromo-5-isopropyl pyrazine **(261-c,** 423mg, 1.9mmol, 52.3% yield). MS Calcd: 199.99; MS Found: 201.02, 202.99 ($[M+H]^+$). Step 3: To a 50ml flask were added t-butyl (5-fluoro-2-methoxy-3-(4,4,5,5-tetramethyl-1,3,2-dioxaborolan-2-yl)phenyl)carbamate **(214-a,** 300mg, 0.82mmol), 2-bromo-5-isopropyl pyrazine **(261-c,** 150mg, 0.75mmol), pd(dppf)Cl2(61.2mg, 0.07mmol), and potassium phosphate (477mg, 2.3mmol), followed by 1,4-dioxane (5ml) and water (1ml). After atmosphere replacement with nitrogen three times, the mixture was allowed to react at 110°C for 4 hours. When the reaction was found completed by sampling and testing, the mixture was concentrated under reduced pressure to dryness. The residue was added with water and extracted with ethyl acetate, to provide t-butyl (5-fluoro-2-methoxy-3-(5-isopropyl pyrazin-2-yl)phenyl)carbamate **(261-d,** 271mg, 0.8mmol, 100% yield). MS Calcd: 361.18; MS Found: 362.19 ($[M+H]^+$).

Step 4: To a 25ml flask was added t-butyl (5-fluoro-2-methoxy-3-(5-isopropyl pyrazin-2-yl)phenyl)carbamate **(261-d,** 271mg, 0.8mmol), followed by DCM(3ml) and TFA(1ml). The mixture was stirred at room temperature for 2 hours. When the reaction was found completed by sampling and testing, the mixture was concentrated under reduced pressure to dryness. The residue was neutralized with saturated sodium carbonate solution, and extracted with ethyl acetate. The organic phase was dried and separated and purified by column chromatography, to provide Intermediate 5-fluoro-2-methoxy-3-(5-methyl pyrazin-2-yl)aniline **(261-e,** 180mg, 0.69mmol, 92.30% yield). MS Calcd: 261.13; MS Found:262.15 ($[M+H]^+$).

Step 5: To a 25ml two-necked flask were added 5-fluoro-2-methoxy-3-(5-isopropyl pyrazin-2-yl)aniline **(261-e,** 120mg, 0.46 mmol) and 4,6-dichloro-N-ethoxypyridine-3-carboxamide (162mg, 0.69mmol), followed by anhydrous THF(2ml). After atmosphere replacement with nitrogen three times, the mixture was cooled in ice bath, added with LiHMDS(1M, 2.3mmol, 2.3ml) and allowed to react at room temperature for 2 hours. Upon TLC indicated a completed reaction, the mixture was quenched with saturated ammonium chloride solution, and extracted with ethyl acetate. The organic phase was concentrated under reduced pressure to dryness and mixed with silica gel for loading onto and purifying by column chromatography (petroleum ether:ethyl acetate=1:1), to provide Intermediate 6-chloro-N-ethoxy-4-((5-fluoro-2-methoxy-3-(5-isopropyl pyrazin-2-yl)phenyl)amino)nicotinamide **(261-f,** 163mg, 0.3mmol, 69.6% yield). MS Calcd: 459.15; MS Found:460.23 ([M+H]$^+$).

Step 6: 6-chloro-N-ethoxy-4-((5-fluoro-2-methoxy-3-(5-isopropyl pyrazin-2-yl)phenyl)amino)nicotinamide **(261-f,** 50mg, 0.11mmol), 4-amino-2,6-dimethyl pyrimidine (28mg, 0.22mmol), $Pd_2(dba)_3$(22mg, 0.022mmol), Xant-Phos(27mg, 0.044mmol), and $Cs_2CO_3$ (121mg, 0.44mmol) were mixed and added with 1,4-dioxane (1ml). After atmosphere replacement with nitrogen three times, the mixture was heated with microwave to 140°C and allowed to react for 3.5 hours. When TLC indicated a completed reaction, the mixture was filtered by suction, and the filtrate was concentrated under reduced pressure at 40°C. The residue was separated and purified by plate chromatography, eluted with DCM:MeOH=20:1, to provide the title compound: 6-((2,6-dimethyl pyrimidin-4-yl)amino)-N-ethoxy-4-((5-fluoro-3-(5-isopropyl pyrazin-2-yl)-2-methoxy phenyl)amino)nicotinamide **(261,** 18mg, 0.032mmol, 30.50% yield). MS Calcd: 546.25; MS Found: 547.13 ([M+H]$^+$). $^1$H NMR (400 MHz, DMSO-$d_6$)$\delta$11.70 (s, 1H), 10.49 (s, 1H), 10.18 (s, 1H), 8.69 (d, $J$ = 2.4 Hz, 1H), 8.59 (d, $J$ = 2.4 Hz, 1H), 8.43 (s, 1H), 8.22 (s, 1H), 7.57 (dd, $J$ = 10.0, 3.2 Hz, 1H), 7.17 (s, 1H), 6.96 (dd, $J$ = 8.4, 3.2 Hz, 1H), 3.94 (q, $J$ = 7.2 Hz, 2H), 3.37 (s, 3H), 2.94 (p, $J$ = 6.8 Hz, 1H), 2.43 (s, 3H), 2.30 (s, 3H), 1.22 (t, $J$ = 7.2 Hz, 9H).

Example **262**

6-((2,6-dimethyl pyrimidin-4-yl)amino)-N-ethoxy-4-((3-(5-fluoropyrimidin-2-yl)-2-methoxy phenyl)amino)pyridazine-3-carboxamide

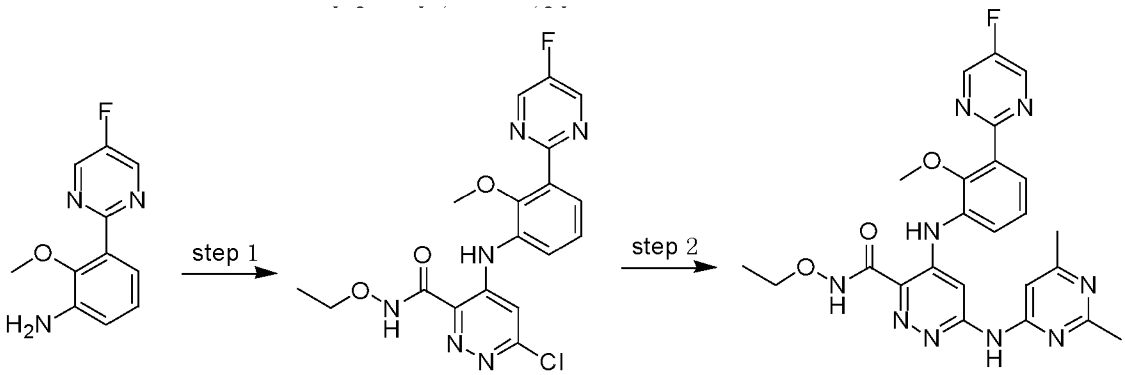


**208-d** **262-a** **262**

Step 1: To a 50ml two-necked flask were added 3-(5-fluoropyrimidin-2-yl)-2-methoxy benzene-1-amine **(208-d,** 160mg, 0.73mmol) and 4,6-dichloro-N-ethoxy-pyridazine-3-carboxamide(206.8mg, 0.88mmol), followed by anhydrous THF(3ml). After atmosphere replacement with nitrogen three times, the reaction mixture was placed in a water bath and slowly added with LHMDS(3.65ml, 3.65mmol). After the addition, the mixture was allowed to react at room temperature for 1 hour. Upon TLC indicated a completed reaction, the mixture was quenched with saturated ammonium chloride solution, and extracted with ethyl acetate. The organic phases were mixed with silica gel for loading onto and purified by column chromatography (PE:EA=1:1) to provide Intermediate 6-chloro-N-ethoxy-4-((3-(5-fluoropyrimidin-2-yl)-2-methoxy phenyl)amino)pyridazine-3-carboxamide(**262-a**, 248mg, 0.5mmol, 69% yield). MS Calcd: 418.10; MS Found: 419.10([M+H]$^+$)

Step 2: 6-chloro-N-ethoxy-4-((3-(5-fluoropyrimidin-2-yl)-2-methoxy phenyl)amino)pyridazine-3-carboxamide(**262-a,** 100mg, 0.24mmol), 4-amino-2,6-dimethyl pyrimidine (44.1mg, 0.36mmol), $Pd_2(dba)_3$(44mg, 0.048mmol), XantPhos(55mg, 0.096mmol), $Cs_2CO_3$ (234mg, 0.7mmol) were mixed and added with 1,4-dioxane (2ml). After atmosphere replacement with nitrogen three times, the mixture was heated with microwave to 140°C and allowed to react for 4 hours. When TLC indicated a completed reaction, the mixture was filtered by suction, and the filtrate was concentrated under reduced pressure at 40°C. The residue was separated and purified by reverse phase column chromatography (acetonitrile:0.5% trifluoroacetic acid aqueous solution, the peak appeared at 30% acetonitrile), to provide the title compound: 6-((2,6-dimethyl pyrimidin-4-yl)amino)-N-ethoxy-4-((3-(5-fluoropyrimidin-2-yl)-2-methoxy phenyl)amino)pyridazine-3-carboxamide(**262**, 8mg, 0.016mmol, 6.6% yield). MS Calcd: 505.20; MS Found: 506.23([M+H]$^+$). $^1$H NMR (400 MHz, DMSO-$d_6$)$\delta$12.26 (s, 1H), 10.58 (s, 1H), 10.52 (s, 1H), 9.05 (s, 2H), 8.36 (s, 1H), 7.74 (dd, $J$ = 8.0, 1.6 Hz, 1H), 7.55 (dd, $J$ = 8.0, 1.6 Hz, 1H), 7.36 (t, $J$ = 8.0 Hz, 1H), 7.14 (s, 1H), 3.99 (q, $J$ = 7.2 Hz, 2H), 3.69 (s, 3H), 2.38 (s, 3H), 2.31 (s, 3H), 1.30 - 1.14 (t, $J$ = 7.2 Hz, 3H).

Example **263**

6-((2,6-dimethyl pyrimidin-4-yl)amino)-N-ethoxy-4-((2-methoxy-3-(pyrimidin-2-yl)phenyl)amino)pyridazine-3-carboxamide

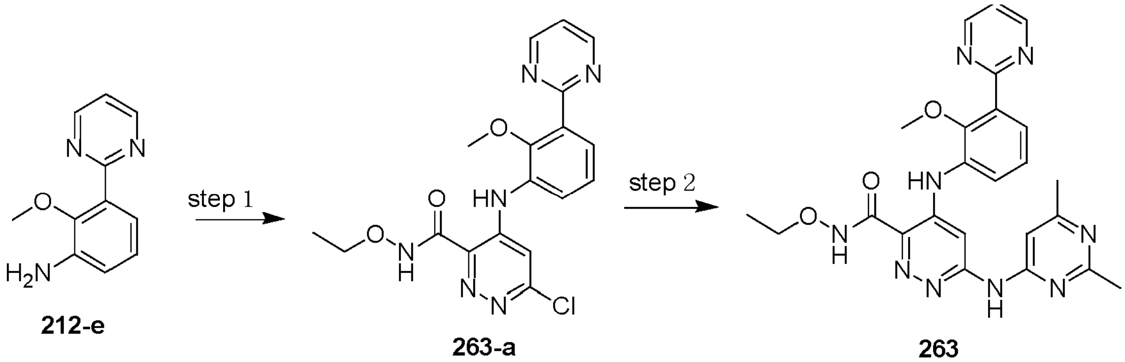


**212-e** **263-a** **263**

Step 1: To a 25ml two-necked flask were added 2-methoxy-3-(pyrimidin-2-yl)aniline **(212-e,** 160mg, 0.67mmol) and 4,6-dichloro-N-ethoxypyridazine-3-carboxamide(189.8mg, 0.80mmol), followed by anhydrous THF(2ml). After atmosphere replacement with nitrogen three times, the mixture was cooled in ice bath, added with LHMDS(3.35mmol, 3.4ml), and allowed to react at room temperature for 1 hour. Upon TLC indicated a completed reaction, the mixture was quenched with saturated ammonium chloride solution, and extracted with EA. The organic phase was concentrated under reduced pressure to dryness, and mixed with silica gel for loading onto and purifying by column chromatography (PE:EA=1:1), to provide Intermediate 6-chloro-N-ethoxy-4-((2-methoxy-3-(pyrimidin-2-yl)phenyl)amino)pyridazine-3-carboxamide **(263-a,** 120mg, 0.3mmol, 40.2% yield). MS Calcd: 400.11; MS Found: 401.16($[M+H]^+$).

Step 2: 6-chloro-N-ethoxy-4-((2-methoxy-3-(pyrimidin-2-yl)phenyl)amino)pyridazine-3-carboxamide(**263-a**, 60mg, 0.15mmol), 4-amino-2, 6-dimethyl pyrimidine (37mg, 0.3mmol), $Pd_2(dba)_3$(14mg, 0.015mmol), XantPhos(17 mg, 0.03mmol), and $Cs_2CO_3$ (146.3mg, 0.45mmol) were mixed and added with 1,4-dioxane (1ml). After atmosphere replacement with nitrogen three times, the reaction mixture was heated with microwave to 130°C and allowed to react for 4 hours. When TLC indicated a completed reaction, the mixture was filtered by suction, and the filtrate was concentrated under reduced pressure at 40°C. The residue was separated and purified by plate chromatography, eluted with DCM:MeOH=15:1, to provide the title compound: 6-((2,6-dimethyl pyrimidin-4-yl)amino)-N-ethoxy-4-((2-methoxy-3-(pyrimidin-2-yl)phenyl)amino)pyridazine-3-carboxamide(**263**, 13mg, 0.026mmol, 17.7% yield). MS Calcd: 487.21; MS Found: 488.21 ($[M+H]^+$). $^1$H NMR (400 MHz, DMSO-$d_6$)δ12.27 (s, 1H), 10.59 (s, 1H), 10.52 (s, 1H), 8.96 (d, *J* = 4.8 Hz, 2H), 8.37 (s, 1H), 7.73 (dd, *J* = 8.0, 1.6 Hz, 1H), 7.61 - 7.47 (m, 2H), 7.36 (t, *J* = 8.0 Hz, 1H), 7.15 (s, 1H), 3.99 (q, *J* = 7.2 Hz, 2H), 3.71 (s, 3H), 2.39 (s, 3H), 2.31 (s, 3H), 1.23 (t, *J* = 7.2 Hz, 3H).

Example **264**

4-((3-(5-chloropyrimidin-2-yl)-2-methoxy phenyl)amino)-N-ethoxy-6-(pyrimidin-2-ylamino)nicotinamide

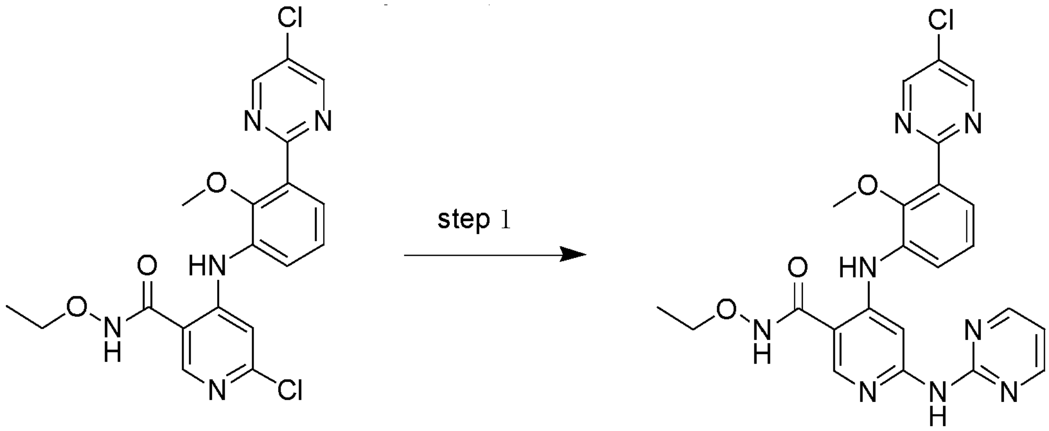


**218-c** **264**

Step 1: To a 10ml microwave tube were added 6-chloro-4-((3-(5-chloropyrimidin-2-yl)-2-methoxy phenyl) amino)-N-ethoxy nicotinamide **(218-c,** 60mg, 0.14mmol), 2-aminopyrimidine (20mg, 0.21mmol), XantPhos(32mg, 0.056mmol), $Pd_2dba_3$(25mg, 0.028mmol), and $Cs_2CO_3$ (113mg, 0.3mmol), followed by 1,4-dioxane (1.5ml). After atmosphere replacement with nitrogen three times, the reaction mixture was heated with microwave to 130°C and allowed to react for 4 hours. The reaction mixture was concentrated under reduced pressure to dryness. The residue was purified by plate chromatography (DCM:MeOH=25:1) to provide the title compound: 4-((3-(5-chloropyrimidin-2-yl)-2-methoxy phenyl)amino)-N-ethoxy-6-(pyrimidin-2-ylamino)nicotinamide **(264,** 10mg, 0.02mmol, 13.2% yield). MS Calcd: 492.14; MS Found: 493.19 ($[M+H]^+$). $^1$H NMR (400 MHz, DMSO-$d_6$)δ11.74 (s, 1H), 10.28 (s, 1H), 9.97 (s, 1H), 9.09 (s, 2H), 8.54 (d, *J* = 4.8 Hz, 2H), 8.41 (d, *J* = 10.4 Hz, 2H), 7.79 (dd, *J* = 8.0, 1.6 Hz, 1H), 7.47 (dd, *J* = 8.0, 1.6 Hz, 1H), 7.38 (t, *J* = 8.0 Hz, 1H), 6.98 (t, *J* = 4.8 Hz, 1H), 3.97 (q, *J* = 7.2 Hz, 2H), 3.71 (s, 3H), 1.25 (t, *J* = 7.2 Hz, 3H).

Example **265**

N-ethoxy-6-((6-fluoro-5-methyl pyridin-3-yl)amino)-4-((3-(5-fluoropyrimidin-2-yl)-2-methoxy phenyl)amino)nicotinamide

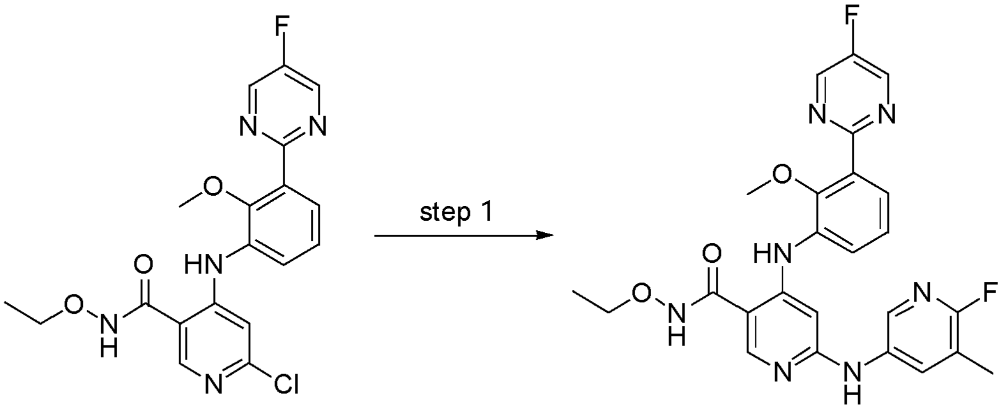


**208-e** **265**

Step 1: To a 10ml microwave tube were sequentially added 6-chloro-4-((3-(5-fluoropyrimidin-2-yl)-2-methoxy phenyl)amino)-N-ethoxy nicotinamide **(208-e,** 50mg, 0.12mmol), 2-fluoro-3-methyl-5-aminopyridine (23.0mg, 0.18mmol), $Pd_2(dba)_3$(11.0mg, 0.012mmol), Xant phos(14.0 mg, 0.024mmol), $Cs_2CO_3$ (117.0mg, 0.36mmol) and dioxane(2ml). After atmosphere replacement with nitrogen three times, the mixture was heated to 140°C with microwave and stirred for 3 hours under nitrogen protection. When LCMS indicated a completed reaction, the reaction mixture was cooled to room temperature, diluted with 5ml of methanol, and filtered through diatomaceous earth. The filter cake was washed with 3*5ml of methanol. The combined filtrate was subjected to rotary evaporation under reduced pressure to dryness. The crude material was purified by Prep-TLC(DCM: MeOH =20:1) to provide the title compound: N-ethoxy-6-((6-fluoro-5-methyl pyridin-3-yl)amino)-4-((3-(5-fluoropyrimidin-2-yl)-2-methoxy phenyl)amino)nicotinamide **(265,** 13.1mg, 21.4% yield), MS Calcd: 507.18; MS Found: 508.21([M+H]). 1H NMR (400 MHz, DMSO-d6)δ11.62 (s, 1H), 9.97 (s, 1H), 9.30 (s, 1H), 9.04 (s, 2H), 8.33 (s, 1H), 8.21 (s, 1H), 8.08 (d, J = 9.2 Hz, 1H), 7.61 (d, J = 7.8 Hz, 1H), 7.46 (d, J = 7.8 Hz, 1H), 7.30 (t, J = 7.8 Hz, 1H), 6.53 (s, 1H), 3.94 (q, J = 7.2 Hz, 2H), 3.69 (s, 3H), 2.21 (s, 3H), 1.22 (t, J = 7.2 Hz, 3H).

Example **266**

N-ethoxy-4-((3-(5-fluoropyrimidin-2-yl)-2-methoxy phenyl)amino)-6-((6-(trifluoromethyl)pyridin-3-yl)amino)nicotinamide

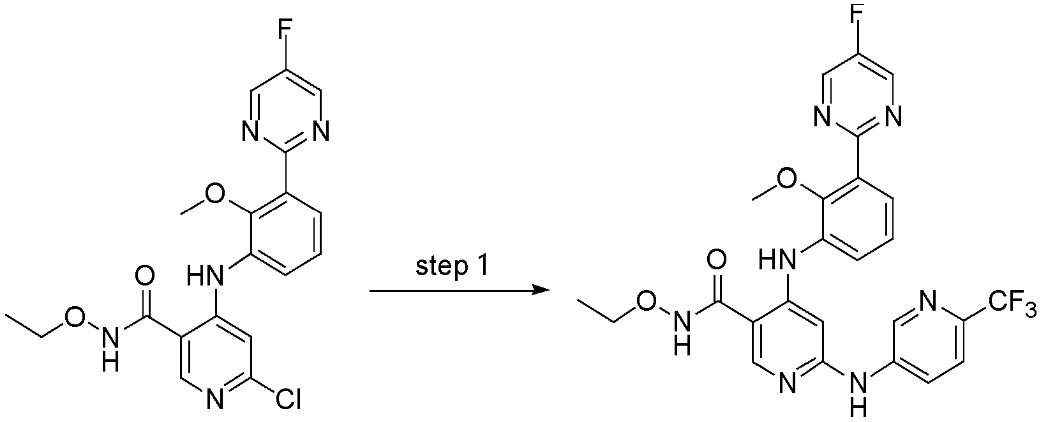


**208-e** **266**

Step 1: To a 10ml microwave tube were sequentially added 6-chloro-4-((3-(5-fluoropyrimidin-2-yl)-2-methoxy phenyl)amino)-N-ethoxy nicotinamide **(208-e,** 50mg, 0.12mmol), 6-(trifluoromethyl)pyridin-3-ylamine (29.16mg, 0.18mmol), $Pd_2(dba)_3$(11.0mg, 0.012mmol), XantPhos(14.0 mg, 0.024mmol), $Cs_2CO_3$ (117.0mg, 0.36mmol)and dioxane(2ml). After atmosphere replacement with nitrogen three times, the mixture was heated to 140°C with microwave and stirred for 3 hours under nitrogen protection. When LCMS indicated a completed reaction, the reaction mixture was cooled to room temperature, diluted with 5ml of methanol, and filtered through diatomaceous earth. The filter cake was washed with 3*5ml of methanol. The combined filtrate was subjected to rotary evaporation under reduced pressure to dryness. The crude material was purified by Prep-TLC(DCM: MeOH =20:1) to provide the title compound: N-ethoxy-4-((3-(5-fluoropyrimidin-2-yl)-2-methoxy phenyl)amino)-6-((6-(trifluoromethyl)pyridin-3-yl)amino)nicotinamide **(266,** 20.1mg, 30.8% yield), MS Calcd: 543.48; MS Found: 544.23($[M+H]^+$). $^1$H NMR (400 MHz, DMSO-$d_6$)δ11.72 (s, 1H), 9.97 (s, 1H), 9.79 (s, 1H), 9.04 (s, 2H), 8.87 (s, 1H), 8.45 (d, *J* = 11.2 Hz, 1H), 8.38 (s, 1H), 7.77 (d, *J* = 8.6 Hz, 1H), 7.63 (d, *J* = 8.0 Hz, 1H), 7.50 (d, *J* = 9.4 Hz, 1H), 7.33 (t, *J* = 7.8 Hz, 1H), 6.63 (s, 1H), 3.96 (q, *J* = 7.2 Hz, 2H), 3.69 (s, 3H), 1.23 (t, *J* = 7.2 Hz, 3H).

Example **267**

N-ethoxy-4-((3-(5-fluoropyrimidin-2-yl)-2-methoxy phenyl)amino)-6-((5-(trifluoromethyl)pyridin-3-yl)amino)nicotinamide

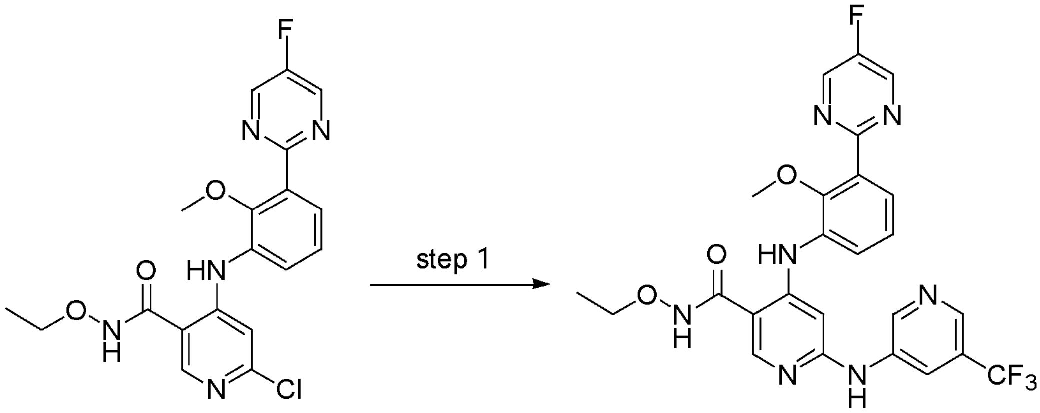


**208-e** **267**

Step 1: To a 10ml microwave tube were sequentially added 6-chloro-4-((3-(5-fluoropyrimidin-2-yl)-2-methoxy phenyl)amino)-N-ethoxy nicotinamide **(208-e,** 50mg, 0.12mmol), 6-(trifluoromethyl)-pyridin-3-ylamine (29.16mg, 0.18mmol), $Pd_2(dba)_3$(11.0mg, 0.012mmol), XantPhos(14.0 mg, 0.024mmol), $Cs_2CO_3$ (117.0mg, 0.36mmol) and dioxane(2ml). After atmosphere replacement with nitrogen three times, the mixture was heated to 140°C with microwave and stirred for 3 hours under nitrogen protection. When LCMS indicated a completed reaction, the reaction mixture was cooled to room temperature, diluted with 5ml of methanol, and filtered through diatomaceous earth. The filter cake was washed with 3*5ml of methanol. The combined filtrate was subjected to rotary evaporation under reduced pressure to dryness. The crude material was purified by Prep-TLC(DCM: MeOH =20:1) to provide the title compound N-ethoxy-4-((3-(5-fluoropyrimidin-2-yl)-2-methoxy phenyl)amino)-6-((5-(trifluoromethyl)pyridin-3-yl)amino)nicotinamide **(267,** 30.1mg, 46.2% yield), MS Calcd: 543.48; MS Found: 544.23($[M+H]^+$). $^1H$ NMR (400 MHz, DMSO-d6)δ11.67 (s, 1H), 9.98 (s, 1H), 9.76 (s, 1H), 9.04 (s, 2H), 8.85 (s, 1H), 8.79 (s, 1H), 8.44 (s, 1H), 8.40 (s, 1H), 7.62 (d, J = 7.6 Hz, 1H), 7.50 (d, J = 7.6 Hz, 1H), 7.33 (t, J = 7.6 Hz, 1H), 6.59 (s, 1H), 3.95 (q, J = 7.2 Hz, 2H), 3.69 (s, 3H), 1.23 (t, J = 7.2 Hz, 3H).

Example **268**

6-((2,6-dimethyl pyrimidin-4-yl)amino)-N-ethoxy-4-((2-methoxy-3-(1-methyl-1H-1,2,4-triazol-3-yl)phenyl)amino)nicotinamide

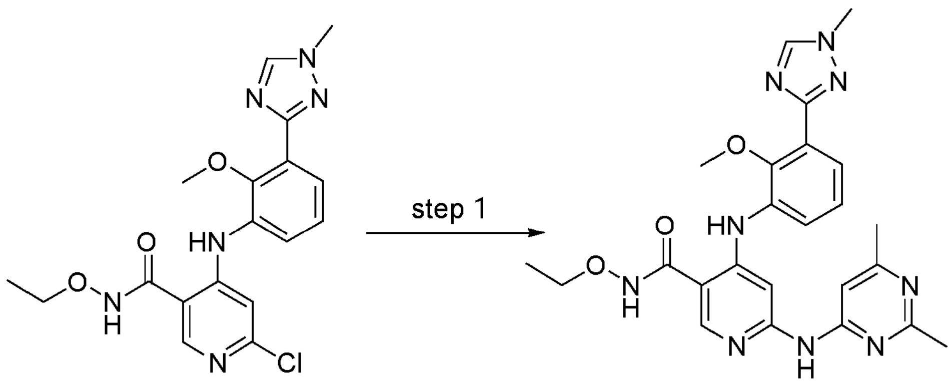


**166-a** **268**

Step 1: To a 10ml microwave tube were sequentially added 6-chloro-N-ethoxy-4-((2-methoxy-3-(1-methyl-1H-1,2,4-triazol-3-yl)phenyl)amino)nicotinamide **(166-a,** 100mg, 0.25mmol), 2,6-dimethyl pyrimidin-4-ylamine (37mg, 0.30mmol), $Pd_2(dba)_3$ (23mg, 0.025mmol), XantPhos (25mg, 0.021mmol), $Cs_2CO_3$ (243mg, 0.75mmol) and dioxane(2ml). After atmosphere replacement with nitrogen three times, the reaction mixture was heated with microwave to 130°C and stirred for 3 hours under nitrogen protection. When LCMS indicated a completed reaction, the reaction mixture was cooled to room temperature, diluted with 5ml of methanol, and filtered through diatomaceous earth. The filter cake was washed with 3*5ml of methanol. The combined filtrate was subjected to rotary evaporation under reduced pressure to

dryness, and the crude material was purified by Prep-TLC (DCM: MeOH =20:1) to provide the title compound 6-((2,6-dimethyl pyrimidin-4-yl)amino)-N-ethoxy-4-((2-methoxy-3-(1-methyl-1H-1,2,4-triazol-3-yl)phenyl)amino)nicotinamide **(268,** 15.1mg, 12.3% yield), MS Calcd: 489.22; MS Found: 490.35($[M+H]^+$). $^1H$ NMR (400 MHz, DMSO-$d_6$)δ11.71 (s, 1H), 10.26 (s, 1H), 10.10 (s, 1H), 8.56 (s, 1H), 8.39 (s, 1H), 8.17 (s, 1H), 7.67 (dd, $J$ = 8.0, 1.6 Hz, 1H), 7.59 (dd, $J$ = 8.0, 1.6 Hz, 1H), 7.28 (t, $J$ = 8.0 Hz, 1H), 7.09 (s, 1H), 3.98 (d, $J$ = 7.2 Hz, 2H), 3.95 (s, 3H), 3.75 (s, 3H), 2.38 (s, 3H), 2.28 (s, 3H), 1.23 (s, 3H).

Example **269**

6-((2,6-dimethyl pyrimidin-4-yl)amino)-N-ethoxy-4-((3-(6-fluoropyridin-3-yl)-2-methoxy phenyl)amino)nicotinamide

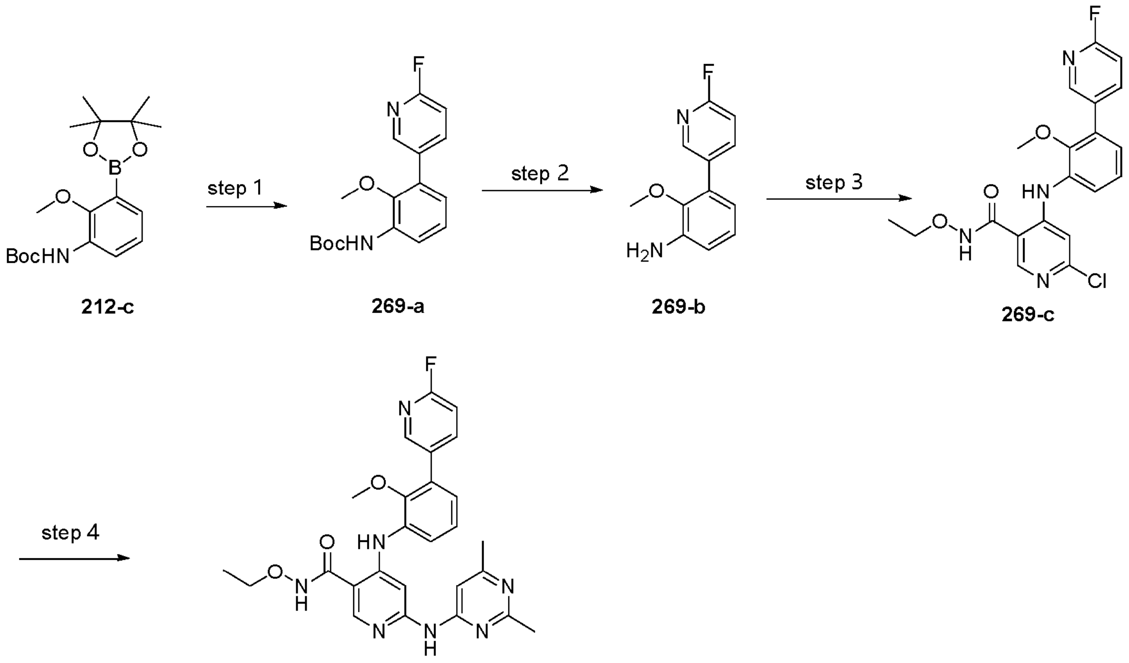


212-c 269-a 269-b 269-c

269

Step 1: To a 100ml flask were sequentially added t-butyl (2-methoxy-3-(4,4,5,5-tetramethyl-1,3,2-dioxaborolan-2-yl)phenyl)carbamate **(212-c,** 400mg, 1.15mmol), 5-bromo-2-fluoropyridine (240mg, 1.37mmol), anhydrous potassium carbonate (480g, 3.45mmol), Pd(dppf)$Cl_2$ (50mg, 0.057mmol), dioxane(8 ml), and $H_2O$(2ml). After atmosphere replacement with nitrogen three times, the reaction mixture was heated to 110°C and stirred with reflux for 5 hours under nitrogen protection. When TLC indicated a completed reaction, the reaction mixture was filtered, and the filtrate was added with 30ml of saturated brine and extracted with 3*30ml of ethyl acetate three times. The organic phases were combined, dried over anhydrous sodium sulfate, and subjected to rotary evaporation under reduced pressure to dryness. The residue was purified by flash column chromatography (ethyl acetate:petroleum ether = 0 to 30% in 30min) to provide t-butyl (3-(6-fluoropyridin-3-yl)-2-methoxy phenyl)carbamate **(269-a,** 300 mg, 0.94mmol, 82.0% yield). MS Calcd: 318.14; MS Found: 263.25($[M-56+H]^+$).

Step 2: To a 50ml flask was added t-butyl (3-(6-fluoropyridin-3-yl)-2-methoxy phenyl)carbamate **(269-a,** 300 mg, 2.19mmol), followed by sequential addition of dichloromethane (8ml) and hydrochloride solution in dioxane (4M) (8ml). The mixture was stirred at room temperature for 2 hours. When TLC indicated a completed reaction, the reaction mixture was subjected to rotary evaporation under reduced pressure to dryness to provide 3-(6-fluoropyridin-3-yl)-2-methoxy **aniline(269-b,** 194mg, 0.89mmol, 94.7% yield). MS Calcd: 218.09; MS Found: 219.31($[M+H]^+$).

Step 3: To a 100ml flask were sequentially added 3-(6-fluoropyridin-3-yl)-2-methoxy aniline(**269-b**, 194mg, 0.89mmol), 4,6-dichloro-N-ethoxy nicotinamide (250mg, 1.08mmol) and DMA(5ml). The mixture was cooled in ice bath and added with LiHMDS(4.45ml, 4.45 mol) (1M in THF). The reaction mixture was removed from ice bath and stirred at room temperature for 2 hours. When LCMS monitoring indicated that no raw material unreacted, the reaction mixture was cooled in ice bath and quenched with 15ml of saturated ammonium chloride solution, diluted with 30ml of water, and extracted with 3*30ml of EA. The organic phases were combined and washed with 50ml of saturated NaCl aqueous solution. The organic phases were collected, dried over anhydrous $NaSO_4$, and subjected to rotary evaporation under

reduced pressure to dryness. The crude material was purified by flash column chromatography (DCM:MeOH = 0 to 10% in 30min), to provide 6-chloro-N-ethoxy-4-((3-(6-fluoropyridin-3-yl)-2-methoxy phenyl)amino)nicotinamide **(269-c,** 200mg, 0.48 mmol, 54.0% yield). MS Calcd: 416.11; MS Found: 417.28([M+H]$^+$).

Step 4: To a 10ml microwave tube were sequentially added 6-chloro-N-ethoxy-4-((3-(6-fluoropyridin-3-yl)-2-methoxy phenyl)amino)nicotinamide **(269-c,** 100mg, 0.24mmol), 2,6-dimethyl pyrimidin-4-ylamine (36mg, 0.29mmol), $Pd_2(dba)_3$ (22mg, 0.024mmol), XantPhos (28 mg, 0.048mmol), $Cs_2CO_3$ (234mg, 0.72mmol) and dioxane(2ml). After atmosphere replacement with nitrogen three times, the mixture was heated to 140°C with microwave and stirred for 3 hours under nitrogen protection. When LCMS indicated a completed reaction, the reaction mixture was cooled to room temperature, diluted with 5ml of methanol, and filtered through diatomaceous earth. The filter cake was washed with 3*5ml of methanol. The combined filtrate was subjected to rotary evaporation under reduced pressure to dryness. The crude material was purified by Prep-TL(DCM: MeOH =20:1) to provide the title compound: 6-((2,6-dimethyl pyrimidin-4-yl) amino)-N-ethoxy-4-((3-(6-fluoropyridin-3-yl)-2-methoxy phenyl)amino)nicotinamide **(269,** 15.1mg, 12.5% yield), MS Calcd: 503.21; MS Found: 504.20([M+H]$^+$). $^1$H NMR (400 MHz, DMSO-$d_6$)δ11.73 (s, 1H), 10.32 (s, 1H), 10.13 (s, 1H), 8.44 (d, *J* = 2.4 Hz, 1H), 8.40 (s, 1H), 8.21 (td, *J* = 8.2, 2.6 Hz, 1H), 8.17 (s, 1H), 7.69 (d, *J* = 8.0 Hz, 1H), 7.33 (d, *J* = 2.8 Hz, 1H), 7.31 (d, *J* = 3.2 Hz, 1H), 7.22 (d, *J* = 9.2 Hz, 1H), 7.12 (s, 1H), 3.97 (t, *J* = 7.2 Hz, 2H), 3.42 (s, 3H), 2.41 (s, 3H), 2.29 (s, 3H), 1.22 (d, *J* = 7.2 Hz, 3H).

Example **270**

6-((2,6-dimethyl pyrimidin-4-yl)amino)-N-ethoxy-4-((5-fluoro-2-methoxy-3-(5-methyl pyrimidin-2-yl)phenyl)amino)pyridazine-3-carboxamide

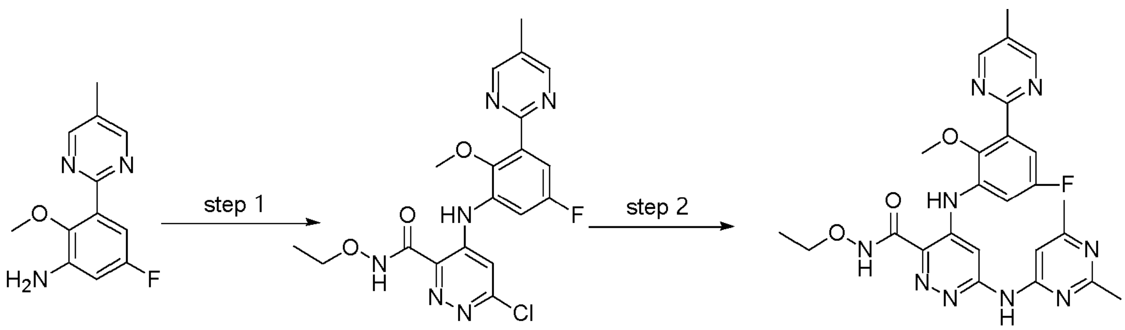


**225-b** **270-a** **270**

Step 1: To a 100ml flask were sequentially added 5-fluoro-2-methoxy-3-(5-methyl pyrimidin-2-yl)aniline **(225-b,** 150mg, 0.64mmol), 4,6-dichloro-N-ethoxypyridazine-3-carboxamide(183mg, 0.77mmol) and DMA(5ml). The mixture was cooled in ice bath and added with LiHMDS(2.24ml, 2.24 mol) (1M in THF). The reaction mixture was removed from ice bath and stirred at room temperature for 2 hours. When LCMS monitoring indicated that no raw material unreacted, the reaction mixture was cooled in ice bath and quenched with 15ml of saturated ammonium chloride solution, diluted with 30ml of water, and extracted with 3*30ml of EA. The organic phases were combined, washed with 50ml of saturated NaCl aqueous solution. The organic phase was collected, dried over anhydrous $NaSO_4$, and subjected to rotary evaporation under reduced pressure to dryness. The crude material was purified by flash column chromatography (DCM:MeOH = 0 to 10% in 30min), to provide 6-chloro-N-ethoxy-4-((5-fluoro-2-methoxy-3-(5-methyl pyrimidin-2-yl)phenyl)amino)pyridazine-3-carboxamide(**270-a**, 30mg, 0.07 mmol, 10.9% yield)MS Calcd: 432.84; MS Found: 433.56([M+H]$^+$).

Step 2: To a 10ml microwave tube were sequentially added 6-chloro-N-ethoxy-4-((5-fluoro-2-methoxy-3-(5-methyl pyrimidin-2-yl)phenyl)amino)nicotinamide **(270-a,** 30mg, 0.07mmol), 2,6-dimethyl pyrimidin-4-ylamine (11mg, 0.09mmol), $Pd_2(dba)_3$(10mg, 0.011mmol), XantPhos(10 mg, 0.017mmol), $Cs_2CO_3$ (68mg, 0.21mmol) and dioxane(1ml). After atmosphere replacement with nitrogen three times, the mixture was heated to 140°C with microwave and stirred for 3 hours under nitrogen protection. When LCMS indicated a completed reaction, the reaction mixture was cooled to room temperature, diluted with 5ml of methanol, and filtered through diatomaceous earth. The filter cake was washed with 3*5ml of methanol. The combined filtrate was subjected to rotary evaporation under reduced pressure to dryness, and the crude material was purified by Prep-TLC (DCM: MeOH =20:1) to provide the title compound: 6-((2,6-dimethyl pyrimidin-4-yl) amino)-N-ethoxy-4-((5-fluoro-2-methoxy-3-(5-methyl pyrimidin-2-yl)phenyl)amino)pyridazine-3-carboxamide(**270**, 1.51mg, 4.2% yield), MS Calcd: 519.54; MS Found: 520.23([M+H]). $^1$H NMR (400 MHz, DMSO-$d_6$)δ11.73 (s, 1H), 10.32 (s, 1H), 10.13 (s, 1H), 8.44 (d, *J* = 2.6 Hz, 1H), 8.40 (s, 1H), 8.21 (td, *J* = 8.2, 2.6 Hz, 1H), 8.17 (s, 1H), 7.69 (d, *J* = 8.2

Hz, 1H), 7.33 (d, $J$ = 2.8 Hz, 1H), 7.31 (d, $J$ = 3.2 Hz, 1H), 7.22 (d, $J$ = 9.2 Hz, 1H), 7.12 (s, 1H), 3.97 (t, $J$ = 7.0 Hz, 2H), 3.42 (s, 3H), 2.41 (s, 3H), 2.29 (s, 3H), 1.22 (d, $J$ = 7.0 Hz, 3H).

Example **271**

6-((3,5-difluoropyridin-2-yl)amino)-N-ethoxy-4-((5-fluoro-2-methoxy-3-(pyrimidin-2-yl)phenyl)amino)nicotinamide

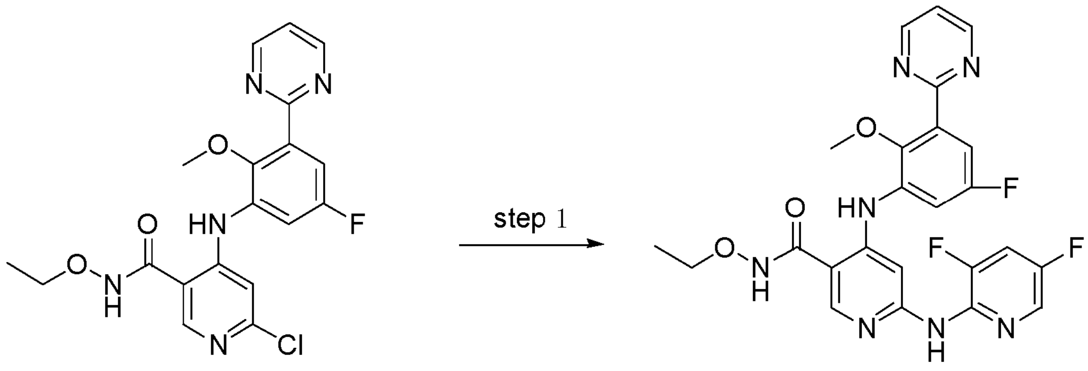


**215-d** **271**

Step 1: 6-chloro-N-ethoxy-4-((5-fluoro-2-methoxy-3-(pyrimidin-2-yl)phenyl)amino)nicotinamide **(215-d,** 104 mg, 0.25 mmol), 2-amino-3,5-difluoropyridine (36 mg, 0.27 mmol), cesium carbonate (243 mg, 0.7 mmol), Xant-Phos(26mg, 0.046mmol) and $Pd_2(dba)_3$(28mg, 0.031 mmol) were added to anhydrous dioxane (2 ml). After the atmosphere of the mixture was evacuated to vacuum and replaced with nitrogen three times, the mixture was heated in a microwave reactor to 140 °C , stirred for 3 hours, and filtered by suction. The filtrate was concentrated and purified by preparative TLC (MeOH:DCM=1:20) to provide the title compound: 6-((3,5-difluoropyridin-2-yl)amino)-N-ethoxy-4-((5-fluoro-2-methoxy-3-(pyrimidin-2-yl)phenyl)amino)nicotinamide **(271,** mg, 0.058mmol, 23.6% yield). MS Calcd: 511.16; MS Found: 512.34 ($[M+H]^+$). $^1H$ NMR (400 MHz, DMSO-d6)δ11.74 (s, 1H), 10.38 (s, 1H), 9.50 (s, 1H), 8.99 (s, 1H), 8.97 (s, 1H), 8.36 (s, 1H), 8.11 (d, J = 2.4 Hz, 1H), 7.99 - 7.92 (m, 1H), 7.77 (s, 1H), 7.60 - 7.54 (m, 2H), 7.22 (dd, J = 9.0, 3.2 Hz, 1H), 3.95 (q, J = 7.2 Hz, 2H), 3.71 (s, 3H), 1.22 (t, J = 7.2 Hz, 3H).

Example **272**

6-((2,6-dimethyl pyrimidin-4-yl)amino)-N-ethoxy-4-((5-fluoro-2-(methoxy-d3)-3-(5-methyl pyrazin-2-yl)phenyl)amino)nicotinamide

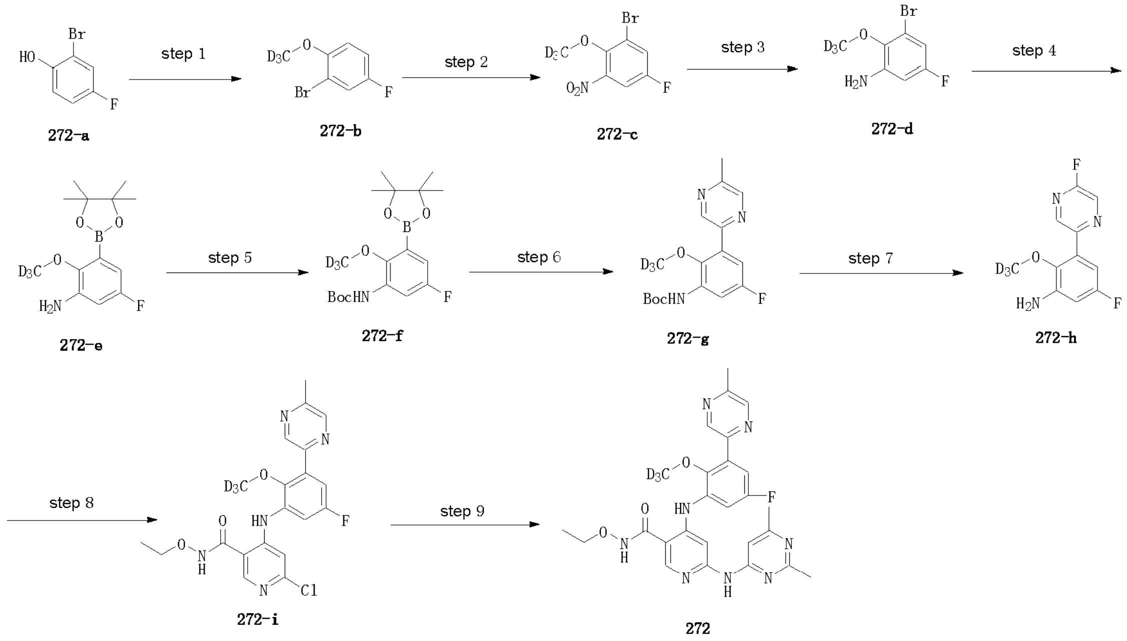


Step 1: To 30 mL of acetone were sequentially added 2-bromo-4-fluorophenol **(272-a,** 3.0 g, 15.71 mmol) and potassium carbonate (5.4g, 39.3 mmol), followed by deuterated iodomethane (2.73 g, 18.8 mmol). The mixture was heated to 60 °C with stirring for 6h. Upon TLC indicating a completed reaction, the filtrate was concentrated, and the residue was separated and purified by silica gel column chromatography (ethyl acetate:petroleum ether=1:10) to provide 2-bromo-4-fluoro-1-(methoxy-d3)benzene **(272-b,** 3.13 g, 15.2 mmol, 95.8% yield).

Step 2: 2-bromo-4-fluoro-1-(methoxy-d3)benzene **(272-b,** 3.13 g, 15.2 mmol) was dissolved in 10mL of concentrated sulfuric acid. The mixture was cooled in ice-water bath and slowly added with 1.83 mL of concentrated nitric acid dropwise. After the addition, the mixture was allowed to react at that temperature with continuous stirring for 3h. When TLC indicated a completed reaction, the reaction mixture was poured into ice-water, extracted with ethyl acetate, washed with saturated brine, dried over anhydrous sodium sulfate, and concentrated under reduced pressure. The residue was separated and purified by silica gel column chromatography (ethyl acetate:petroleum ether=1:5) to provide 1-bromo-5-fluoro-2-(methoxy-d3)-3-nitrobenzene **(272-c,** 2.1 g, 8.3 mmol, 55.7% yield).

Step 3: 1-bromo-5-fluoro-2-(methoxy-d3)-3-nitrobenzene **(272-c,** 1.9 g, 7.51 mmol), iron powder (2.1 g, 37.5 mmol) and ammonium chloride (2.4 g, 45.1 mmol) were sequentially added to 10 mL of mixed solvent of ethanol/water (4/1). The mixture was heated to 85 °C with stirring for 4h. Upon indication of completed reaction by TLC, the mixture was filtered by suction. The filter cake was washed with methanol and concentrated under reduced pressure. The residue was separated and purified by silica gel column chromatography (ethyl acetate:petroleum ether=1:1) to provide 3-bromo-5-fluoro-2-(methoxy-d3) aniline **(272-d,** 1.2 g, 5.4 mmol, 71.6% yield). MS Calcd: 223.1; MS Found: 264.0 ($[M+CH_3CN]^+$).

Step 4: 3-bromo-5-fluoro-2-(methoxy-d3) aniline **(272-d,** 1.17 g, 5.24 mmol), bis(pinacolato)diboron (2.0 g, 7.87 mmol), $Pd(dppf)Cl_2$ (0.5 g, 0.6 mmol) and potassium acetate (2.2 g, 22.6 mmol) were sequentially added to dioxane. After the atmosphere of the mixture was evacuated to vacuum and replaced with nitrogen three times, the mixture was heated to 110°C with continuous stirring for 20 hours. Upon indication of completed reaction by TLC, the reaction mixture was thoroughly concentrated, added with water (40 mL), and extracted with ethyl acetate (30 mL x 2). The organic phase was washed with saturated brine (30 mL x 2), dried over anhydrous sodium sulfate, and concentrated under reduced pressure. The residue was separated and purified by silica gel column chromatography (ethyl acetate:petroleum ether=1:4) to provide 5-fluoro-2-(methoxy-d3)-3-(4,4,5,5-tetramethyl-1,3,2-dioxaborolan-2-yl)aniline **(272-e,** 1.18 g, 4.4 mmol, 83.3% yield). MS Calcd: 270.1; MS Found: 272.2 ($[M+H]^+$).

Step 5: 5-fluoro-2-(methoxy-d3)-3-(4,4,5,5-tetramethyl-1,3,2-dioxaborolan-2-yl)aniline **(272-e,** 1.18 g, 4.4 mmol) and Boc anhydride(1.1 g, 5.2 mmol) were sequentially added to 10 mL of ethanol. The mixture was stirred overnight at room temperature. Upon TLC indicating a completed reaction, the mixture was filtered and the filtrate was concentrated. The residue was separated and purified by silica gel column chromatography (ethyl acetate:petroleum ether=1:4) to provide t-butyl (5-fluoro-2-(methoxy-d3)-3-(4,4,5,5-tetramethyl-1,3,2-dioxaborolan-2-yl)phenyl)carbamate **(272-f,** 1.1 g, 3.0 mmol, 68.8% yield). MS Calcd: 370.2; MS Found: 315.2 ($[M-56]^+$).

Step 6: t-butyl (5-fluoro-2-(methoxy-d3)-3-(4,4,5,5-tetramethyl-1,3,2-dioxaborolan-2-yl)phenyl)carbamate **(272-f,** 0.5 g, 1.35 mmol), 2-chloro-5-fluoropyrimidine (208mg, 1.6 mmol), potassium phosphate (0.86 g, 4.1 mmol) and $Pd(dppf)Cl_2$(139 mg, 0.2 mmol) were added to 10 mL of mixed solution of water and dioxane (6:1). After the

atmosphere of the mixture was evacuated to vacuum and replaced with nitrogen three times, the mixture was stirred at 105°C for 10 hours. Upon indication of completed reaction by TLC, the reaction mixture was thoroughly concentrated. The residue was separated and purified by silica gel column chromatography (ethyl acetate:petroleum ether=1:1) to provide t-butyl (5-fluoro-2-(methoxy-d3)-3-(5-methyl pyrazin-2-yl)phenyl)carbamate **(272-g,** 130 mg, 0.4 mmol, 28.6% yield). MS Calcd: 336.4; MS Found: 337.2 ($[M+H]^+$). Step 7: t-butyl (5-fluoro-2-(methoxy-d3)-3-(5-methyl pyrazin-2-yl)phenyl) carbamate **(272-g,** 130 mg, 0.4 mmol) was dissolved in dichloromethane, followed by adding trifluoroacetic acid (0.88 g, 7.7 mmol). The mixture was stirred at room temperature for 5h. Upon indication of completed reaction by TLC, the reaction mixture was thoroughly concentrated. The residue was added with saturated sodium bicarbonate aqueous solution, extracted with dichloromethane, washed with saturated brine, dried over anhydrous sodium sulfate, and concentrated under reduced pressure. The residue was separated and purified by silica gel column chromatography (ethyl acetate:petroleum ether=1:1) to provide 5-fluoro-3-(5-fluoropyrazin-2-yl)-2-(methoxy-d3) aniline **(272-h,** 80 mg, 0.3 mmol, 87.6% yield). MS Calcd: 236.3; MS Found: 237.1 ($[M+H]^+$).

Step 8: 5-fluoro-3-(5-fluoropyrazin-2-yl)-2-(methoxy-d3) aniline **(272-h,** 80 mg, 0.3 mmol), 4,6-dichloro-N-ethoxy nicotinamide (81 mg, 0.34 mmol) were added to 5 ml of anhydrous N,N-dimethylacetamide. The mixture was added at room temperature with a solution of LiHMDS in tetrahydrofuran (1.0 ml, 1.0 mmol), and stirred at room temperature for 3 hours. Upon indication of completed reaction by TLC, the mixture was adjusted with aqueous hydrochloride (4N) to pH 5, and extracted with ethyl acetate (10 ml x 3). The combined organic layers were dried, concentrated and then purified by column chromatography (DCM: MeOH =20:1), to provide 6-chloro-N-ethoxy-4-((5-fluoro-2-(methoxy-d3)-3-(5-methyl pyrazin-2-yl)phenyl)amino)nicotinamide **(272-i,** 140 mg, 0.3 mmol, 94.6% yield). MS Calcd: 434.9; MS Found: 435.1 ($[M+H]^+$).

Step 9: 6-chloro-N-ethoxy-4-((5-fluoro-2-(methoxy-d3)-3-(5-methyl pyrazin-2-yl)phenyl)amino)nicotinamide **(272-i,** 140 mg, 0.3 mmol), 4-amino-2,6-dimethyl pyrimidine (40 mg, 0.32 mmol), cesium carbonate (348 mg, 1.1 mmol), XantPhos(25mg, 0.04mmol) and $Pd_2(dba)_3$(27mg, 0.029 mmol) were added to anhydrous dioxane (2 ml). After the atmosphere of the mixture was evacuated to vacuum and replaced with nitrogen three times, the mixture was heated to 140°C and stirred in a microwave reactor for 3 hours, and filtered by suction. The filtrate was concentrated and purified by preparative TLC (MeOH:DCM=1:20) to provide the title compound: 6-((2,6-dimethyl pyrimidin-4-yl)amino)-N-ethoxy-4-((5-fluoro-2-(methoxy-d3)-3-(5-methyl pyrazin-2-yl)phenyl)amino)nicotinamide **(272,** 40mg, 0.1mmol, 23.8% yield). MS Calcd: 521.6; MS Found: 522.2 ($[M+H]^+$). $^1H$ NMR (400 MHz, DMSO-d6)δ11.77 (s, 1H), 10.51 (s, 1H), 10.18 (s, 1H), 9.02 (d, J = 1.6 Hz, 1H), 8.69 (d, J = 1.6 Hz, 1H), 8.43 (s, 1H), 8.19 (s, 1H), 7.58 - 7.52 (m, 1H), 7.26 (dd, J = 9.4, 3.2 Hz, 1H), 7.18 (s, 1H), 3.96 (q, J = 7.2 Hz, 2H), 2.57 (s, 3H), 2.41 (s, 3H), 2.30 (s, 3H), 1.23 (t, J = 7.2 Hz, 3H).

Example 273

6-((2,6-dimethyl pyrimidin-4-yl)amino)-N-ethoxy-4-((3-(5-fluoropyrimidin-2-yl)-2-(methoxy-d3)phenyl)amino)nicotinamide

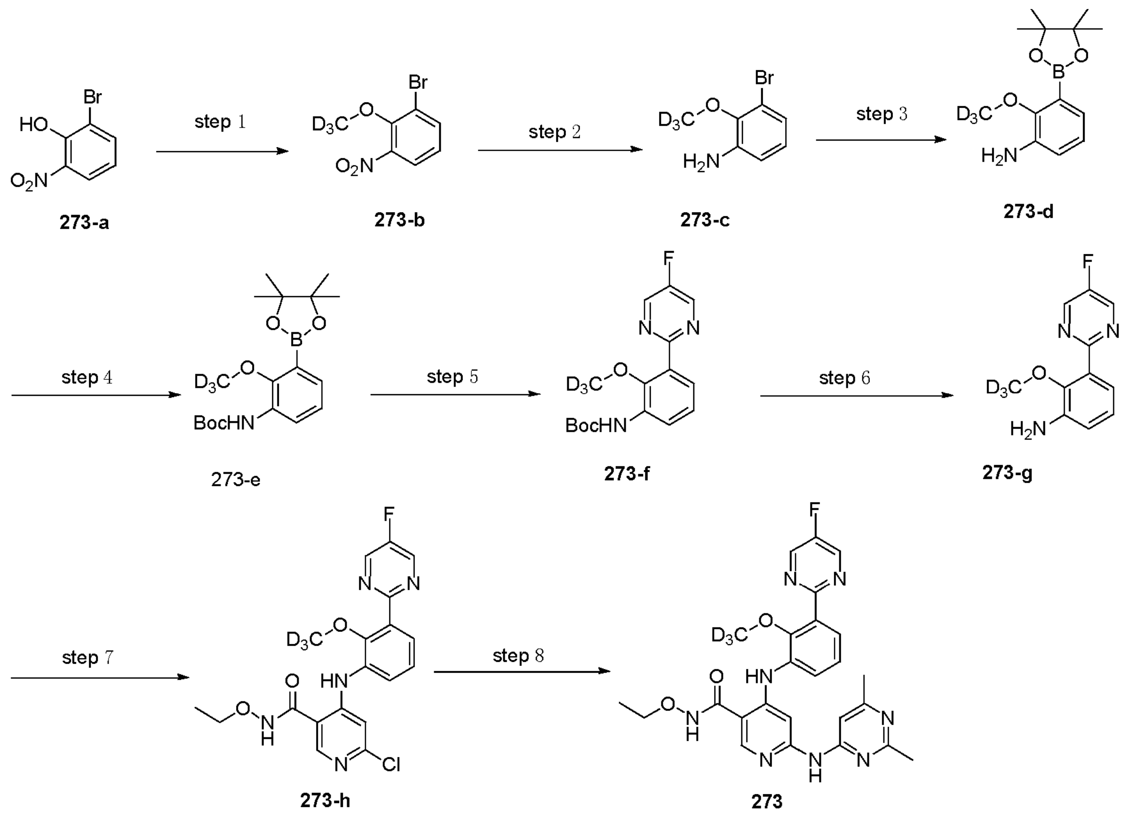


273-a 273-b 273-c 273-d 273-e 273-f 273-g 273-h 273

Step 1: 2-bromo-6-nitrophenol **(273-a,** 2.0 g, 9.17 mmol) and potassium carbonate (3.17 g, 22.9 mmol) were sequentially added to 10 mL of acetone, followed by adding deuterated iodomethane (1.6 g, 11.0 mmol). The mixture was heated to 60 °C with stirring for 6h. Upon TLC indicating a completed reaction, the filtrate was concentrated, and the residue was separated and purified by silica gel column chromatography (EA:PE=1:10) to provide 1-bromo-2-(methoxy-d3)-3-nitrobenzene **(273-b,** 1.53 g, 6.5 mmol, 70.9% yield).

Step 2: 1-bromo-2-(methoxy-d3)-3-nitrobenzene **(273-b,** 1.53 g, 6.5 mmol), iron powder (1.8 g, 32.5 mmol) and ammonium chloride (2.1 g, 39.0 mmol) were sequentially added to 10 mL of mixed solution of ethanol/water (4/1). The mixture was heated to 85 °C and stirred for 4h. Upon indication of completed reaction by TLC, the mixture was filtered by suction. The filter cake was washed with methanol, and concentrated under reduced pressure. The residue was separated and purified by silica gel column chromatography (EA:PE=1:1) to provide 3-bromo-2-(methoxy-d3) aniline **(273-c,** 1.16 g, 5.7 mmol, 86.9% yield). MS Calcd: 204.00; MS Found: 228.3 ($[M+Na]^+$).

Step 3: 3-bromo-2-(methoxy-d3) aniline **(273-c,** 1.16 g, 5.7 mmol), bis(pinacolato)diboron (2.2 g, 8.48 mmol), Pd(dppf)$Cl_2$ (0.5 g, 0.6 mmol) and potassium acetate (2.2 g, 22.6 mmol) were sequentially added to dioxane. After the atmosphere of the mixture was evacuated to vacuum and replaced with nitrogen three times, the mixture was heated to 110°C with continuous stirring for 20 hours. Upon indication of completed reaction by TLC, the reaction mixture was thoroughly concentrated, added with water (40 mL), and extracted with ethyl acetate (30 mL x 2). The organic phase was washed with saturated brine (30 mL x 2), dried over anhydrous sodium sulfate, and concentrated under reduced pressure. The residue was separated and purified by silica gel column chromatography (EA:PE=1:4) to provide 2-(methoxy-d3)-3-(4,4,5,5-tetramethyl-1,3,2-dioxaborolan-2-yl)aniline **(273-d,** 0.72 g, 2.9 mmol, 50.5% yield).

Step 4: 2-(methoxy-d3)-3-(4,4,5,5-tetramethyl-1,3,2-dioxaborolan-2-yl)aniline **(273-d,** 0.72 g, 2.9 mmol) and Boc anhydride(0.9 g, 4.3 mmol) were sequentially added to 10 mL of ethanol. The mixture was stirred overnight at room temperature. Upon TLC indicating a completed reaction, the mixture was filtered and the filtrate was concentrated. The residue was separated and purified by silica gel column chromatography (EA:PE=1:4) to provide t-butyl (2-(methoxy-d3)-3-(4,4,5,5-tetramethyl-1,3,2-dioxaborolan-2-yl)phenyl)carbamate **(273-e,** 0.7 g, 2 mmol, 70% yield). MS Calcd: 352.22; MS Found: 297.1 ($[M-56]^+$).

Step 5: t-butyl (2-(methoxy-d3)-3-(4,4,5,5-tetramethyl-1,3,2-dioxaborolan-2-yl)phenyl)carbamate **(273-e,** 600 mg, 1.7 mmol), 2-chloro-5-fluoropyrimidine (225mg, 1.7 mmol), potassium phosphate (1.08 g, 5.1 mmol) and Pd(dppf)$Cl_2$ (139 mg, 0.2 mmol) were added to 10 mL of mixed solution of water and dioxane (6:1). After the atmosphere of the mixture was evacuated to vacuum and replaced with nitrogen three times, the mixture was stirred at 105°C for 10 hours. Upon indication of completed reaction by TLC, the reaction mixture was thoroughly concentrated, and the residue was separated and purified by silica gel column chromatography (EA:PE=1:1) to provide the product t-butyl (3-(5-fluoropyrimidin-2-yl)-2-(methoxy-d3)phenyl)carbamate **(273-f,** 250 mg, 0.8 mmol, 45.5% yield). MS Calcd: 322.15; MS Found: 267.1 ([M-56]$^+$).

Step 6: t-butyl (3-(5-fluoropyrimidin-2-yl)-2-(methoxy-d3)phenyl)carbamate **(273-f,** 250 mg, 0.8 mmol) was dissolved in dichloromethane, followed by adding trifluoroacetic acid (1.76 g, 15.5 mmol). The mixture was stirred at room temperature for 5h. Upon indication of completed reaction by TLC, the reaction mixture was thoroughly concentrated. The residue was added with saturated sodium bicarbonate aqueous solution, and extracted with dichloromethane. The organic phase was washed with saturated brine, dried over anhydrous sodium sulfate, and concentrated under reduced pressure. The residue was separated and purified by silica gel column chromatography (EA:PE=1:1) to provide the product 3-(5-fluoropyrimidin-2-yl)-2-(methoxy-d3) aniline **(273-g,** 150 mg, 0.7 mmol, 87.1% yield). MS Calcd: 2; MS Found: 223.12 ([M+H]$^+$).

Step 7: 3-(5-fluoropyrimidin-2-yl)-2-(methoxy-d3) aniline **(273-g,** 150 mg, 0.7 mmol) and 4,6-dichloro-N-ethoxy nicotinamide (150 mg, 0.68 mmol) were added to 5 ml of anhydrous N,N-dimethylacetamide. The mixture was added at room temperature with a solution of LiHMDS in tetrahydrofuran (1.91 ml, 1.91 mmol), and stirred at room temperature for 3 hours. Upon indication of completed reaction by TLC, the mixture was adjusted with aqueous hydrochloride (4N) to pH 5, and extracted with ethyl acetate (20 ml x 3). The combined organic layers were dried, concentrated and then purified by column chromatography (DCM: MeOH =20:1) to provide compound 6-chloro-N-ethoxy-4-((3-(5-fluoropyrimidin-2-yl)-2-(methoxy-d3)phenyl)amino)nicotinamide **(273-h,** 160 mg, 0.4 mmol, 59.6% yield). MS Calcd: 420.12; MS Found: 421.13 ([M+H]$^+$).

Step 8: 6-chloro-N-ethoxy-4-((3-(5-fluoropyrimidin-2-yl)-2-(methoxy-d3)phenyl)amino)nicotinamide **(273-h,** 150 mg, 0.36 mmol), 4-amino-2,6-dimethyl pyrimidine (44 mg, 0.36 mmol), cesium carbonate (348 mg, 1.1 mmol), XantPhos(25mg, 0.04mmol) and $Pd_2(dba)_3$(27mg, 0.029 mmol) were added to anhydrous dioxane (2 ml). After the atmosphere of the mixture was evacuated to vacuum and replaced with nitrogen three times, the mixture was heated to 140°C and stirred in a microwave reactor for 3 hours, and filtered by suction. The filtrate was concentrated and purified by preparative TLC (MeOH:DCM=1:20) to provide the title compound: 6-((2,6-dimethyl pyrimidin-4-yl)amino)-N-ethoxy-4-((3-(5-fluoropyrimidin-2-yl)-2-(methoxy-d3)phenyl)amino)nicotinamide **(273,** 40mg, 0.1mmol, 22% yield). MS Calcd: 507.22; MS Found: 508.5 ([M+H]$^+$). $^1$H NMR (400 MHz, DMSO-d6)δ11.72 (s, 1H), 10.23 (s, 1H), 10.12 (s, 1H), 9.05 (d, J = 0.8 Hz, 2H), 8.40 (s, 1H), 8.18 (s, 1H), 7.74 (dd, J = 8.0, 1.6 Hz, 1H), 7.46 (dd, J = 8.0, 1.6 Hz, 1H), 7.32 (t, J = 8.0 Hz, 1H), 7.09 (s, 1H), 3.96 (q, J = 7.2 Hz, 2H), 2.39 (s, 3H), 2.29 (s, 3H), 1.23 (t, J = 7.2 Hz, 3H).

Example **274**

N-ethoxy-4-((2-methoxy-3-(5-methyl pyrimidin-2-yl)phenyl)amino)-6-(pyrimidin-2-ylamino)nicotinamide

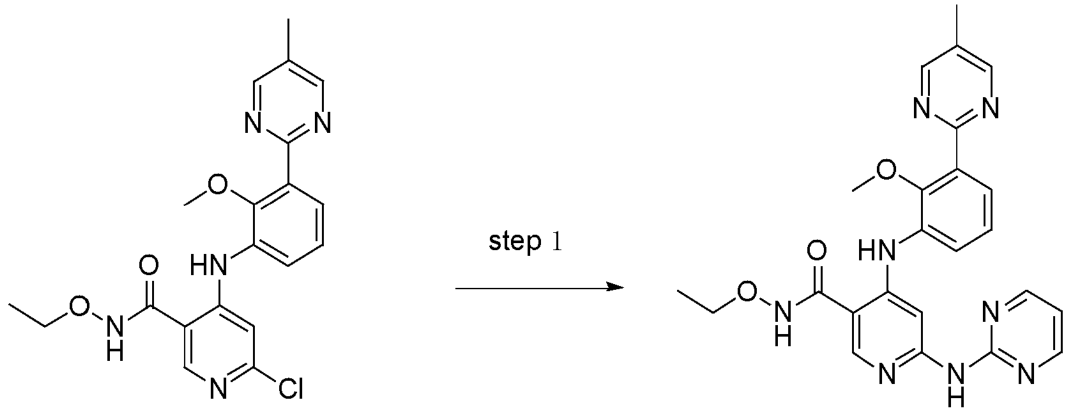


238-c

274

Step 1: To a 10ml microwave tube were sequentially added 6-chloro-N-ethoxy-4-((2-methoxy-3-(5-methyl pyrimidin-2-yl)phenyl)amino)nicotinamide **(238-c,** 100 mg, 0.24mmol), 2-aminopyrimidine (34mg, 0.36mmol), $Pd_2(dba)_3$ (21mg, 0.023mmol), XantPhos (28mg, 0.046mmol), $Cs_2CO_3$ (224mg, 0.69mmol) and dioxane(2ml). After atmosphere replacement with nitrogen three times, the mixture was heated to 140°C with microwave and stirred for 3 hours under nitrogen protection. When LCMS indicated a completed reaction, the reaction mixture was cooled to room temperature, diluted with 5ml of methanol, and filtered through diatomaceous earth. The filter cake was washed with 3*5ml of methanol. The combined filtrate was subjected to rotary evaporation under reduced pressure to dryness, and the

crude material was purified by Prep-TLC (DCM: MeOH =20:1) to provide the title compound: N-ethoxy-4-((2-methoxy-3-(5-methyl pyrimidin-2-yl)phenyl)amino)-6-(pyrimidin-2-ylamino)nicotinamide **(274,** 60mg, 51% yield), MS Calcd: 472.20; MS Found: 473.2([M+H]+). 1H NMR (400 MHz, DMSO-d6)δ11.75 (s, 1H), 10.26 (s, 1H), 10.05 (s, 1H), 8.80 (d, J = 0.8 Hz, 2H), 8.55 (d, J = 4.8 Hz, 2H), 8.43 (d, J = 6.8 Hz, 2H), 7.75 (dd, J = 7.8, 1.6 Hz, 1H), 7.41 (dd, J = 7.8, 1.6 Hz, 1H), 7.35 (t, J = 7.8 Hz, 1H), 6.98 (t, J = 4.8 Hz, 1H), 3.97 (q, J = 7.2 Hz, 2H), 3.70 (s, 3H), 2.35 (s, 3H), 1.23 (t, J = 7.2 Hz, 3H).

Example **275**

N-ethoxy-4-((2-methoxy-3-(5-methyl pyrimidin-2-yl)phenyl)amino)-6-(pyrimidin-2-ylamino)pyridazine-3-carboxamide

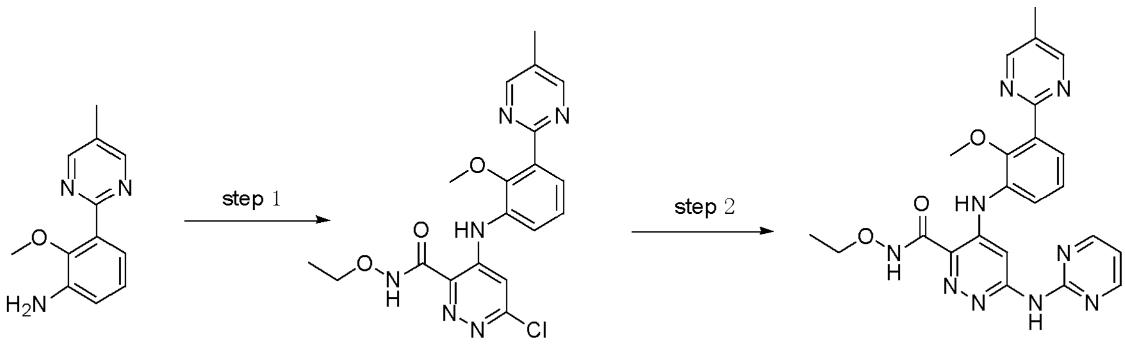


238-b 275-a 275

Step 1: To a 100ml flask were sequentially added 2-methoxy-3-(5-methyl pyrimidin-2-yl)aniline **(238-b,** 150mg, 0.7mmol), 4,6-dichloro-N-ethoxypyridazine-3-carboxamide(213mg, 0.9mmol) and THF(5ml).The mixture was cooled in ice bath and added with LiHMDS(2.1ml, 2.1 mol) (1M in THF). The reaction mixture was removed from ice bath and stirred at room temperature for 2 hours. When LCMS monitoring indicated that no raw material unreacted, the reaction mixture was cooled in ice bath and added with 3ml of diluted HCl solution (1M) to quench the reaction, diluted with 30ml of water, and extracted with 3*30ml of EA. The organic phases were combined, washed with 3*20ml of saturated NaCl aqueous solution. The organic phases were collected, dried over anhydrous $NaSO_4$, and subjected to rotary evaporation under reduced pressure to dryness. The crude material was purified by flash column chromatography (EA:PE = 0 to 60% in 30min), to provide 6-chloro-N-ethoxy-4-((2-methoxy-3-(5-methyl pyrimidin-2-yl)phenyl)amino)pyridazine-3-carboxamide(**275-a**, 200mg, 0.5 mmol, 69% yield)MS Calcd: 414.12; MS Found: 415.27([M+H]+).

Step 2: To a 10ml microwave tube were sequentially added 6-chloro-N-ethoxy-4-((2-methoxy-3-(5-methyl pyrimidin-2-yl)phenyl)amino)pyridazine-3-carboxamide(**275-a**, 100 mg, 0.24mmol), 2-aminopyrimidine (34mg, 0.36mmol), $Pd_2(dba)_3$ (21mg, 0.023mmol), Xantphos (28mg, 0.046mmol), $Cs_2CO_3$ (224mg, 0.69mmol) and dioxane(2ml). After atmosphere replacement with nitrogen three times, the mixture was heated to 140°C with microwave and stirred for 3 hours under nitrogen protection. When LCMS indicated a completed reaction, the reaction mixture was cooled to room temperature, diluted with 5ml of methanol, and filtered through diatomaceous earth. The filter cake was washed with 3*5ml of methanol. The combined filtrate was subjected to rotary evaporation under reduced pressure to dryness, and the crude material was purified by Prep-TLC (DCM: MeOH =20:1) to provide the title compound: N-ethoxy-4-((2-methoxy-3-(5-methyl pyrimidin-2-yl)phenyl)amino)-6-(pyrimidin-2-ylamino)pyridazine-3-carboxamide **(275,** 40mg, 35% yield), MS Calcd: 473.19; MS Found: 474.18([M+H]). 1H NMR (400 MHz, DMSO-d6)δ12.23 (s, 1H), 10.59 (d, J = 5.6 Hz, 2H), 8.80 (d, J = 0.8 Hz, 2H), 8.57 (d, J = 4.8 Hz, 2H), 8.53 (s, 1H), 7.73 (dd, J = 8.0, 1.6 Hz, 1H), 7.49 (dd, J = 7.8, 1.6 Hz, 1H), 7.37 (t, J = 8.0 Hz, 1H), 7.03 (t, J = 4.8 Hz, 1H), 4.00 (q, J = 7.2 Hz, 2H), 3.69 (s, 3H), 2.35 (s, 3H), 1.24 (t, J = 7.2 Hz, 3H).

Example **276**

6-((2,6-dimethyl pyrimidin-4-yl)amino)-N-ethoxy-4-((2-methoxy-3-(5-methyl pyrimidin-2-yl)phenyl)amino)pyridazine-3-carboxamide

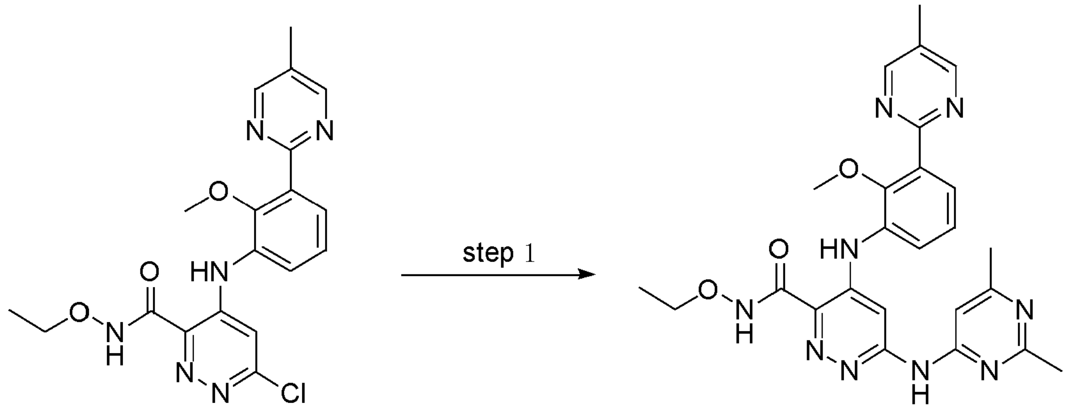


275-a

276

Step 1: 6-chloro-N-ethoxy-4-((2-methoxy-3-(5-methyl pyrimidin-2-yl)phenyl)amino)pyridazine-3-carboxamide **(275-a,** 100mg, 0.24mmol), 2,6-dimethyl pyrimidin-4-ylamine (60mg, 0.48mmol), cesium carbonate (234mg, 0.72mmol), XantPhos(23mg, 0.04mmol) and $Pd_2(dba)_3$(18mg, 0.02mmol) were added to anhydrous dioxane (2ml). After the atmosphere of the mixture was evacuated to vacuum and refilled with nitrogen, the mixture was heated in microwave reactor to 140°C, followed by stirring for 3 hours, and filtered by suction. The filtrate was concentrated and purified by high performance preparative thin layer chromatography (MeOH:DCM=1:10) to provide the title compound: 6-((2,6-dimethyl pyrimidin-4-yl)amino)-N-ethoxy-4-((2-methoxy-3-(5-methyl pyrimidin-2-yl)phenyl)amino)pyridazine-3-carboxamide **(276,** 22mg, 0.040mmol, 17% yield). MS Calcd: 501.55; MS Found: 502.55 ($[M+H]^+$). $^1H$ NMR (400 MHz, DMSO-$d_6$) δ12.276(s, 1H), 10.59 (s, 1H), 10.52 (s, 1H), 8.80 (d, *J* = 0.8Hz, 2H), 8.38 (s, 1H), 7.71 (dd, *J* = 8.0, 1.6Hz, 1H), 7.52 (dd, *J* = 7.8, 1.6 Hz, 1H), 7.34 (t, *J* = 7.9 Hz, 1H), 7.15 (s, 1H), 3.99 (q, *J* = 7.2 Hz, 2H), 3.69 (s, 3H), 2.39 (s, 3H), 2.35 (s, 3H), 2.31 (s, 3H), 1.23 (t, *J* = 7.2 Hz, 3H).

Example **277**

4-((4-cyclopropyl-2-(N-methyl methanesulfonamido)phenyl)amino)-6-(2,2-difluorocyclopropyl-1-carboxamido)-N-ethoxy nicotinamide

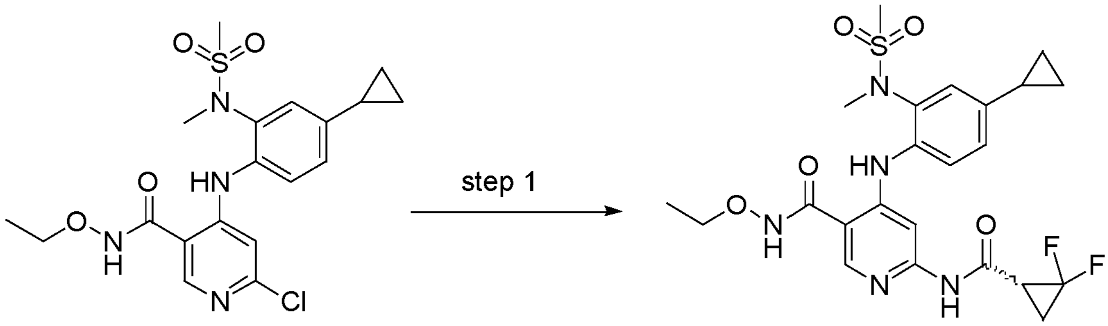


20-a

277

Step 1: 6-chloro-4-((4-cyclopropyl-2-(N-methyl sulfonamido)phenyl)amino)-N-ethoxy nicotinamide **(20-a,** 117mg, 0.27mmol), 2,2-difluorocyclopropyl-1-carboxamide (65mg, 0.54mmol), cesium carbonate (263mg, 0.81mmol), Xphos(34mg, 0.06mmol) and $Pd_2(dba)_3$(27mg, 0.03mmol) were added to anhydrous dioxane (3ml). After the atmosphere of the mixture was evacuated to vacuum and refilled with nitrogen, the reaction mixture was heated to 120°C with stirring for 8 hours, and filtered by suction. The filtrate was concentrated and purified by preparative TLC (MeOH:DCM=1:20) to provide the title compound: 4-((4-cyclopropyl-2-(N-methyl methanesulfonamido)phenyl)amino)-6-(2,2-difluorocyclopropyl-1-carboxamido)-N-ethoxy nicotinamide **(277,** 32mg, 0.061mmol, 22.7% yield). MS Calcd: 523.17; MS Found: 524.2 ($[M+H]^+$). $^1H$ NMR (400 MHz, DMSO-$d_6$)δ11.67 (s, 1H), 10.93 (s, 1H), 9.88 (s, 1H), 8.34 (s, 1H), 7.76 (s, 1H), 7.34 (d, *J* = 8.4 Hz, 1H), 7.27 (d, *J* = 2.0 Hz, 1H), 7.11 (dd, *J* = 8.4, 2.0 Hz, 1H), 3.94 (q, *J* = 7.2 Hz, 2H), 3.12 (s, 3H), 3.07 (s, 3H), 3.01 - 2.88 (m, 1H), 2.03 - 1.90 (m, 3H), 1.22 (t, *J* = 7.2 Hz, 3H), 1.02 - 0.94 (m, 2H), 0.79 - 0.69 (m, 2H).

Example **278**

6-[(3,5-difluoropyridin-2-yl)amino]-N-ethoxy-4-((5-fluoro-3-(5-fluoropyrimidin-2-yl)-2-methoxy phenyl)amino)pyridine-3-carboxamide

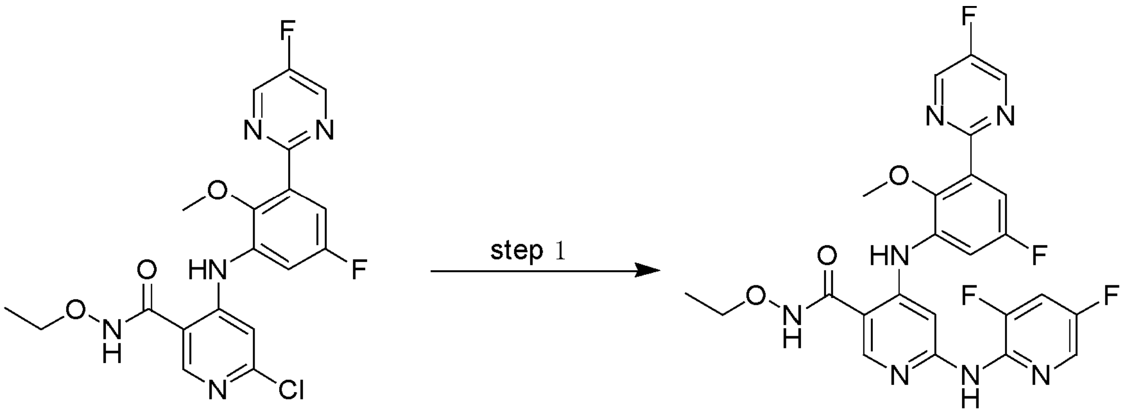


**233-f** **278**

Step 1: 6-chloro-N-ethoxy-4-{[5-fluoro-3-(5-fluoropyrimidin-2-yl)-2-methoxy phenyl]amino}pyridine-3-carboxamide(**233-f**, 70mg, 0.16mmol), 3,5-difluoropyridin-2-ylamine (31.3mg, 0.24mmol), pd2(dba)3 (29.4mg, 0.032mmol), xantphos(37.2mg, 0.064mmol), and $Cs_2CO_3$(157mg, 0.48mmol) were mixed and added with 1,4-dioxane (1ml). After atmosphere replacement with nitrogen three times, the mixture was heated with microwave to 140°C and allowed to react for 3.5 hours. When TLC indicated a completed reaction, the mixture was filtered by suction, and the filtrate was concentrated under reduced pressure at 40°C, separated and purified by plate chromatography, eluted with DCM:MeOH=20:1, to provide the title compound: 6-[(3,5-difluoropyridin-2-yl)amino]-N-ethoxy-4-((5-fluoro-3-(5-fluoropyrimidin-2-yl)-2-methoxy phenyl)amino)pyridine-3-carboxamide(**278**, 58mg, 0.109mmol, 68.2% yield). MS Calcd.: 529.15; MS Found: 530.18 ($[M+H]^+$). $^1H$ NMR (400 MHz, DMSO-$d_6$)δ11.72 (s, 1H), 10.40 (s, 1H), 9.47 (s, 1H), 9.07 (d, *J* = 0.8 Hz, 2H), 8.36 (s, 1H), 8.10 (d, *J* = 2.8 Hz, 1H), 7.97-7.92 (m, 1H), 7.76 (s, 1H), 7.58 (dd, *J* = 10.4, 3.2 Hz, 1H), 7.20 (dd, *J* = 9.2, 3.2 Hz, 1H), 3.95 (q, *J* = 7.2 Hz, 2H), 3.70 (s, 3H), 1.22 (t, *J* = 7.2 Hz, 3H).

Example **279**

6-((2,6-dimethyl pyrimidin-4-yl)amino)-N-ethoxy-4-((2-(methoxy-d3)-3-(pyrimidin-2-yl)phenyl)amino)nicotinamide

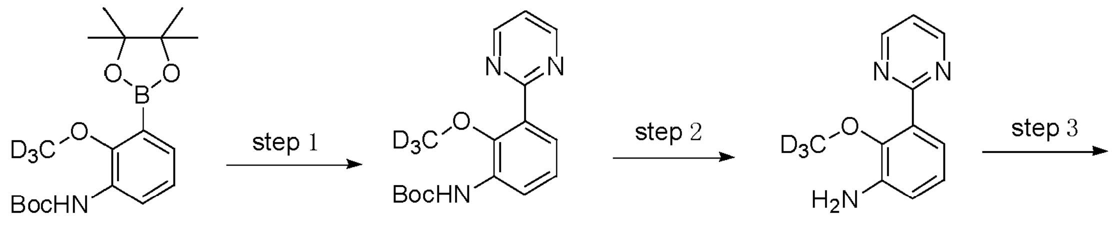


273-e 279-a 279-b

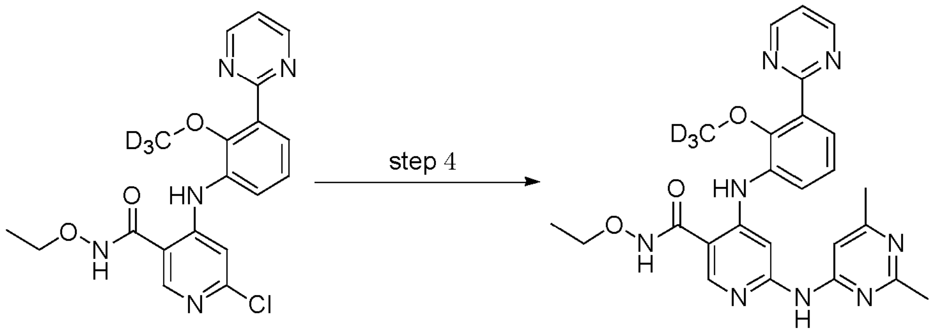


279-c 279

Step 1: t-butyl (2-(methoxy-d3)-3-(4,4,5,5-tetramethyl-1,3,2-dioxaborolan-2-yl)phenyl)carbamate **(273-e,** 600 mg, 1.7 mmol), 2-bromopyrimidine (270mg, 1.7 mmol), potassium phosphate (1.08 g, 5.1 mmol) and Pd(dppf)$Cl_2$(139 mg, 0.2 mmol) were added to 10 mL of mixed solution of water and dioxane (6:1). After the atmosphere of the mixture was evacuated to vacuum and replaced with nitrogen three times, the mixture was stirred at 105°C for 10 hours. Upon indication of completed reaction by TLC, the reaction mixture was thoroughly concentrated, and the residue was separated and purified by silica gel column chromatography (ethyl acetate:petroleum ether=1:1) to provide t-butyl (2-(methoxy-d3)-3-(pyrimidin-2-yl)phenyl)carbamate **(279-a,** 300 mg, 0.98 mmol, 57.9% yield). MS Calcd: 304.16.15; MS Found: 305.25 ($[M-H]^+$).

Step 2: t-butyl (2-(methoxy-d3)-3-(pyrimidin-2-yl)phenyl)carbamate **(279-a,** 300 mg, 0.98 mmol) was dissolved in dichloromethane, followed by adding trifluoroacetic acid (1.14 g, 10.2 mmol). The mixture was stirred at room temperature for 5h. Upon indication of completed reaction by TLC, the reaction mixture was thoroughly concentrated. The residue was added with saturated sodium bicarbonate aqueous solution, extracted with dichloromethane, washed with saturated brine, dried over anhydrous sodium sulfate, and concentrated under reduced pressure. The residue was separated and purified by silica gel column chromatography (ethyl acetate:petroleum ether=1:1) to provide 2-(methoxy-d3)-3-(pyrimidin-2-yl)aniline **(279-b,** 140 mg, 0.60 mmol, 70% yield). MS Calcd: 204.11; MS Found: 205.2 ($[M+H]^+$).

Step 3: 2-(methoxy-d3)-3-(pyrimidin-2-yl)aniline **(279-b,** 140 mg, 0.60 mmol) and 4,6-dichloro-N-ethoxy nicotinamide (170 mg, 0.72 mmol) were added to 3 ml of anhydrous N,N-dimethylacetamide. The mixture was added at room temperature with a solution of LiHMDS in tetrahydrofuran (2.1 ml, 2.1 mmol), and stirred at room temperature for 3 hours. Upon indication of completed reaction by TLC, the mixture was adjusted with aqueous hydrochloride (4N) to pH 5, and extracted with ethyl acetate (20 ml x 3). The combined organic layers were dried, concentrated and then purified by column chromatography (DCM: MeOH =20:1) to provide compound 6-chloro-N-ethoxy-4-((2-(methoxy-d3)-3-(pyrimidin-2-yl) phenyl)amino)nicotinamide **(279-c,** 150 mg, 0.37 mmol, 62.2% yield). MS Calcd: 402.13; MS Found: 403.2 ($[M+H]^+$).

Step 4: 6-chloro-N-ethoxy-4-((2-(methoxy-d3)-3-(pyrimidin-2-yl)phenyl)amino)nicotinamide **(279-c,** 130 mg, 0.32 mmol), 4-amino-2,6-dimethyl pyrimidine (43.7 mg, 0.35 mmol), cesium carbonate (348 mg, 1.1 mmol), Xant-Phos(25mg, 0.04mmol) and $Pd_2(dba)_3$(27mg, 0.029 mmol) were added to anhydrous dioxane (2 ml). After the atmosphere of the mixture was evacuated to vacuum and replaced with nitrogen three times, the mixture was heated to 140°C and stirred in a microwave reactor for 3 hours, and filtered by suction. The filtrate was concentrated and purified by preparative TLC (MeOH:DCM=1:20) to provide the title compound: 6-((2,6-dimethyl pyrimidin-4-yl)amino)-N-ethoxy-4-((2-(methoxy-d3)-3-(pyrimidin-2-yl)phenyl)amino)nicotinamide **(279,** 41mg, 0.08mmol, 25.6% yield). MS Calcd: 489.23; MS Found: 490.3 ($[M+H]^+$). $^1$H NMR (400 MHz, DMSO-$d_6$)δ11.71 (s, 1H), 10.23 (s, 1H), 10.11 (s, 1H), 8.96 (d, *J* = 4.8 Hz, 2H), 8.40 (s, 1H), 8.17 (s, 1H), 7.73 (dd, *J* = 8.0, 1.6 Hz, 1H), 7.52 (t, *J* = 4.8 Hz, 1H), 7.47 (dd, *J* = 8.0, 1.6 Hz, 1H), 7.32 (t, *J* = 8.0 Hz, 1H), 7.10 (s, 1H), 3.96 (q, *J* = 7.2 Hz, 2H), 2.40 (s, 3H), 2.29 (s, 3H), 1.23 (t, *J* = 7.2 Hz, 3H).

**Testing Example 280: Half inhibition dose of compound of the present invention on IL-10-induced JAK1/TYK2 signal pathway**

**Main principle of the experiment:**

Cytokine IL-10 is one of the main members of IL-10 family. The receptor that cytokine IL-10 binds to consists of two kinds of subunits, i.e., IL-10R1 and IL-10R2, which respectively couple with JAK1 and TYK2. After the coupling of IL-10 with its receptor, interaction of phosphorylation occurs between JAK1 and TYK2 to form STAT3 docking site for phosphorylation of STAT3. The phosphorylated STAT3 enters the nucleus and drives the transcription and expression of downstream genes.

The reporter gene plasmid STAT3-Luc Reporter used in the present invention has luciferase expression sequence as the reporter gene, upstream of which is a promoter sequence containing multiple STAT3 binding sites. When phosphorylated STAT3 induced by IL-10 signal pathway binds to the promoter of STAT3-Luc Reporter plasmid, luciferase is transcribed and expressed. The excitation fluorescence catalyzed by Luciferase can be detected after the addition of fluorescein substrate, whose intensity can quantitively reflect the activation level of the signal pathway. In the test of the present invention, when the compound added has JAK1 or TYK2 inhibition activity so as to inhibit the transduction of phosphorylating signal to STAT3, the expression of luciferase induced by IL-10 is inhibited, and the corresponding fluorescence detected shows weakened intensity, thereby reflecting the inhibition strength of the compound on JAK1 or TYK2.

**Experimental materials and equipments:**

Human osteosarcoma cell line U2OS was commercially available from American Type Culture Collection (ATCC), and relevant culture conditions referred to those from ATCC. It was known that such cell line could express IL-10R2, JAK1, TYK2 and STAT3, therefore, by exogenous expression of IL-10R1 gene, a complete IL-10 signal pathway could be established. The human IL-10R1 subunit overexpression plasmid pCMV3-IL 10RA-EGFP was constructed by Shanghai Genechem Co., Ltd., and capable of simultaneously expressing enhanced green fluorescent protein. In the subsequent detection and calculation, the signal of green fluorescent protein in the tested sample was used as a reference for transfection efficiency calibration of the luciferase reporter gene signal. Reporter plasmid STAT3-Luc Reporter was commercially available from Ji Man Biotech (Shanghai) Co., Ltd.

X-tremeGENE 9 DNA Transfection Reagent (Roche) was used for the transfection of plasmid DNA into cells, Cell Culture Lysis 5× reagent (Promega) and Luciferase Assay System (Promega) were used in cell lysate preparation and enzyme-substrate reaction for luciferase reporter gene detection. Green fluorescent protein signal and luciferase reporter gene signal were both detected by Tecan Spark™ 10M multimode microplate reader. The inhibition rate of the tested compound on the IL-10 signal pathway reporter gene signal and the half inhibition dose $IC_{50}$ were both calculated using Microsoft Excel and GraphPad Prism 7.00 software.

**Principal experimental procedure:**

U2OS cell line was inoculated in 96-well plate with flat bottom at 5000 cells per well, and cultured overnight at 37°C and 5% $CO_2$ followed by plasmid transfection. Following the instructions and using X-tremeGENE 9 DNA Transfection Reagent, pCMV3-IL10RA-EGFP and STAT3-Luc Reporter plasmids were co-transfected into the inoculated U2OS cells. After 24 hours of transfection, the tested compound was added respectively for a final concentration of 0, 0.1, 0.3, 1, 3, 10, 30, 100, or 300 nM. After 6 hours of culture at 37 °C and 5% $CO_2$, IL-10 cytokine (Human IL-10 PEPROTECH 200-10-10UG) was added to each well for a final concentration of 10 ng/mL, the well in which neither compound nor cytokine was added was designated as 0% control well. The culture was continued for 24 hours before testing.

Cell lysate was prepared by using Cell Culture Lysis reagent and transferred to 96-well plate with half-well clear bottom. The green fluorescent protein signal value was detected at Ex485/Em520 using Tecan microplate reader, and recorded. After adding to each well the luciferase substrate from Luciferase Assay System, the signal value of luciferase-substrate reaction was immediately detected at 560 nm using Tecan microplate reader, and recorded.

Using Microsoft Excel software, the signal value of luciferase-substrate reaction of each well was calculated and calibrated based on the signal value of green fluorescent protein of each well. The signal value of every wells, after subtracting of signal value of the enzyme-substrate reaction in 0% control well, was compared to that of the 100% control well in which cytokine was added but the final concentration of compound was 0, to calculate the relative reduction percentage of value of the enzyme-substrate reaction signal in the well, thereby obtaining the inhibition rate of the tested compound at different doses on the reporter gene signal pathway. The inhibition rate data were input into GraphPad Prism 7.00 software and processed by relevant calculation scheme to provide the half inhibition dose ($IC_{50}$) of each compound on the reporter gene signal pathway. The results were shown in following Table 1:

**Table 1: Half inhibition dose ($IC_{50}$) of compounds of the present invention on IL-10-induced JAK1/TYK2 signal pathway**

| Compound # | $IC_{50}$ (nM) | Compound # | $IC_{50}$ (nM) | Compound # | $IC_{50}$ (nM) | Compound # | $IC_{50}$ (nM) | Compound # | $IC_{50}$ (nM) |
|---|---|---|---|---|---|---|---|---|---|
| **BM-S-986165** | 3.026 | **2** | 1.58 | **4** | 0.7045 | **10** | 1.184 | **12** | 0.9613 |
| **13** | 1.102 | **14** | 0.8735 | **16** | 1.273 | **18** | 0.7837 | **19** | 1.06 |
| **20** | 0.9545 | **21** | 0.4215 | **22** | 0.5701 | **29** | 2.33 | **36** | 0.686 |
| **38** | 0.5056 | **41** | 1.697 | **42** | 1.509 | **43** | 1.181 | **44** | 1.043 |
| **45** | 0.855 | **46** | 1.087 | **47** | 1.34 | **60** | 1.147 | **61** | 1.702 |
| **62** | 1.291 | **63** | 0.865 | **68** | 1.314 | **69** | 2.78 | **70** | 2.442 |
| **71** | 161.3 | **79** | 0.8893 | **80** | 0.9613 | **81** | 1.499 | **90** | 1.397 |
| **(control)** | | | | | | | | | |
| **93 (control)** | 278.5 | **95** | 2.275 | **97** | 1.333 | **98** | 1.513 | **100 (control)** | 17.04 |
| **101** | 1.467 | **102** | 0.6226 | **106** | 1.808 | **108** | 1.874 | **109** | 0.7327 |
| **110** | 1.51 | **111** | 1.093 | **112** | 0.711 | **113** | 0.4144 | **114** | 0.3203 |
| **115** | 0.2611 | **116** | 0.4709 | **118** | 1.282 | **119** | 1.535 | **125** | 0.9462 |
| **126** | 0.7017 | **127** | 0.7756 | **128** | 0.6975 | **129** | 1.082 | **130** | 0.8024 |
| **131** | 0.5715 | **132** | 0.9769 | **133** | 0.6183 | **134** | 2.767 | **136** | 2.486 |
| **138** | 0.6011 | **139** | 1.401 | **141** | 0.4888 | **142** | 0.4098 | **143** | 0.4962 |
| **144** | 0.9686 | **145** | 0.3007 | **146** | 2.522 | **151** | 2.067 | **153** | 1.508 |
| **154** | 1.522 | **155** | 1.792 | **156** | 1.005 | **157** | 1.494 | **159** | 0.9075 |
| **160** | 0.4423 | **162** | 0.4377 | **166** | 1.506 | **167** | 1.506 | **170** | 1.499 |
| **171** | 1.536 | **173** | 0.4242 | **174** | 0.7838 | **175** | 0.8393 | **176** | 2.321 |
| **177** | 1.596 | **178** | 1.278 | **179** | 1.308 | **180** | 1.132 | **181** | 0.5604 |
| **182** | 1.594 | **183** | 0.7959 | **185** | 0.8210 | **186** | 0.2759 | **187** | 0.9063 |
| **188** | 1.267 | **189** | 0.8246 | **190** | 2.732 | **191** | 0.6527 | **192** | 0.4584 |
| **193** | 0.6470 | **194** | 0.6004 | **197** | 0.7312 | **198** | 0.6502 | **200** | 0.4203 |
| **201** | 0.5406 | **202** | 0.4116 | **203** | 0.383 | **204** | 0.5342 | **205** | 1.791 |
| **207** | 1.077 | **208** | 0.8890 | **209** | 1.778 | **210** | 0.9579 | **211** | 1.983 |
| **212** | 0.2709 | **213** | 0.1457 | **214** | 0.1598 | **215** | 0.1024 | **217** | 1.048 |
| **218** | 0.1705 | **219** | 1.092 | **220** | 1.318 | **222** | 1.093 | **223** | 0.1720 |
| **224** | 0.1770 | **225** | 0.3600 | **226** | 0.5384 | **229** | 0.4243 | **230** | 0.8501 |
| **234** | 0.3680 | **235** | 0.7216 | **236** | 0.5872 | **237** | 0.3945 | **238** | 0.1683 |
| **239** | 0.2895 | **240** | 0.2579 | **242** | 2.123 | **243** | 0.5074 | **244** | 2.578 |
| **245** | 1.918 | **248** | 1.036 | **253 (control)** | 9.747 | **258** | 2.151 | **268** | 0.3147 |
| **277** | 0.4659 | **279** | 0.5295 | **273** | 2.190 | | | | |

Conclusion: The results showed that compounds of the present invention had strong inhibition activity on the IL-10-induced JAK1/TYK2 signal pathway.

**Testing Example 281: Inhibition activity of compounds of the present invention on IL-2-induced JAK1/JAK3 signal**

**Main principle of the experiment:**

The main physiological function of cytokine IL-2 is to stimulate and maintain the proliferation and differentiation of T cells. Generally, IL-2 dependent T cells cannot survive in *in-vitro* culture for a long time, but the addition of IL-2 can make them continue to proliferate. The signal pathway induced by IL-2 coupling with its receptor is mediated by JAK1/JAK3-STAT5, so the inhibitor of JAK1 and/or JAK3 can inhibit the proliferation of IL-2 dependent cells.
It is known that the proliferation of human natural killer cell lymphoma cell line KHYG-1 relies on IL-2, and meanwhile, the selective inhibitor of JAK1/JAK3, tofacitinib (CP-690550), can effectively inhibit the proliferation of this cell line.

**Experimental materials and equipments:**

Human natural killer cell lymphoma cell line KHYG-1 was commercially available from Health Science Research Resources Bank (HSRRB), and relevant culture conditions referred to those from HSRRB.
AlamarBlue™ Cell Viability Reagent (Invitrogen™) was used the fluorescent color reaction for demonstrating cell activities, with fluorescent signal detected using Tecan Spark™ 10M multimode microplate reader. The inhibition rate of the tested compound on IL-2-induced cell proliferation was calculated using Microsoft Excel.

**Principal experimental procedure:**

KHYG-1 cell line was inoculated in 96-well plate with flat bottom at 15000 cells per well, each well was added with 10 ng/mL of human recombinant IL-2(Human IL-2 PEPROTECH 200-02-10UG), together with testing compound respectively for a final concentration of 0, 100, 1000, or 10000 nM. After culture at 37°C and 5% $CO_2$ for 72 hours, alamarBlue™ Cell Viability Reagent was added to each well following the instructions. After incubation at 37 °C for color reaction, the fluorescence value at Ex530/Em590 was detected using Tecan Spark™ 10M multimode microplate reader.
The well in which neither compound nor cytokine was added was designated as 0% control well, and the fluorescence value in the wells in which the final concentration of compound was 0 was designated as 100% control. The inhibition activity $IC_{50}$ of the tested compound on JAK1/JAK3 could be calculated from the inhibition rate of the tested compound on IL-2-induced cell proliferation at different doses. The results were shown in following Table 2:

**Table 2: Inhibition activity of compounds of the present invention on IL-2-induced JAK1/JAK3 signal pathway**

| **Compound #** | $IC_{50}$ (μM) | **Compound #** | $IC_{50}$ (μM) | **Compound #** | $IC_{50}$ (μM) | **Compound #** | $IC_{50}$ (μM) | **Compound #** | $IC_{50}$ (μM) |
|---|---|---|---|---|---|---|---|---|---|
| **tofacitinib** | <0.1 | **1** | >10 | **2** | >10 | **3** | >10 | **4** | >10 |
| **5** | >10 | **6** | >10 | **7** | >10 | **8** | >10 | **9** | >10 |
| **10** | >10 | **11** | >10 | **12** | >10 | **13** | >10 | **14** | >10 |
| **15** | >10 | **16** | >10 | **17** | >10 | **18** | >10 | **19** | >10 |
| **20** | >10 | **21** | >10 | **22** | >10 | **23** | >10 | **24** | >10 |
| **25** | >10 | **26** | >10 | **27** | >10 | **28** | >10 | **29** | >10 |
| **30** | >10 | **31** | >10 | **32** | >10 | **33** | >10 | **34** | >10 |
| **35** | >10 | **36** | >10 | **37** | >10 | **38** | >10 | **39** | >10 |
| **40** | >10 | **41** | >10 | **42** | >10 | **43** | >10 | **44** | >10 |
| **45** | >10 | **46** | >10 | **47** | >10 | **48** | >10 | **49** | >10 |
| **50** | >10 | **51** | >10 | **52** | >10 | **53** | >10 | **54** | >10 |
| **55** | >10 | **56** | >10 | **57** | >10 | **58** | >10 | **59** | >10 |
| **60** | >10 | **61** | >10 | **62** | >10 | **63** | >10 | **64** | >10 |
| **65** | >10 | **66** | >10 | **67** | >10 | **68** | >10 | **69** | >10 |
| **70** | >10 | **71** | >10 | **72** | >10 | **73** | >10 | **74** | >10 |

(continued)

| Compound # | $IC_{50}$ (μM) | Compound # | $IC_{50}$ (μM) | Compound # | $IC_{50}$ (μM) | Compound # | $IC_{50}$ (μM) | Compound # | $IC_{50}$ (μM) |
|---|---|---|---|---|---|---|---|---|---|
| **75** | >10 | **76** | >10 | **77** | >10 | **78** | >10 | **79** | >10 |
| **80** | >10 | **81** | >10 | **82** | >10 | **83** | >10 | **84** | >10 |
| **85** | >10 | **86** | >10 | **87** | >10 | **88** | >10 | **89** | >10 |
| **90** | >10 | **91** | >10 | **92** | >10 | **93** | >10 | **94** | >10 |
| **95** | >10 | **96** | >10 | **97** | >10 | **98** | >10 | **99** | >10 |
| **100** | >10 | **101** | >10 | **102** | >10 | **103** | >10 | **104** | >10 |
| **105** | >10 | **106** | >10 | **107** | >10 | **108** | >10 | **109** | >10 |
| **110** | >10 | **111** | >10 | **112** | >10 | **113** | >10 | **114** | >10 |
| **115** | >10 | **116** | >10 | **117** | >10 | **118** | >10 | **119** | >10 |
| **120** | >10 | **121** | >10 | **122** | >10 | **123** | >10 | **124** | >10 |
| **125** | >10 | **126** | >10 | **127** | >10 | **128** | >10 | **129** | >10 |
| **130** | >10 | **131** | >10 | **132** | >10 | **133** | >10 | **134** | >10 |
| **135** | >10 | **136** | >10 | **137** | >10 | **138** | >10 | **139** | >10 |
| **140** | >10 | **141** | >10 | **142** | >10 | **143** | >10 | **144** | >10 |
| **145** | >10 | **146** | >10 | **147** | >10 | **148** | >10 | **149** | >10 |
| **150** | >10 | **151** | >10 | **152** | >10 | **153** | >10 | **154** | >10 |
| **155** | >10 | **156** | >10 | **157** | >10 | **158** | >10 | **159** | >10 |
| **160** | >10 | **161** | >10 | **162** | >10 | **163** | >10 | **164** | >10 |
| **165** | >10 | **166** | >10 | **167** | >10 | **168** | >10 | 169 | >10 |
| **170** | >10 | **171** | >10 | **172** | >10 | **173** | >10 | **174** | >10 |
| **175** | >10 | **176** | >10 | **177** | >10 | **178** | >10 | **179** | >10 |
| **180** | >10 | **181** | >10 | **182** | >10 | **183** | >10 | **184** | >10 |
| **185** | >10 | **186** | >10 | **187** | >10 | **188** | >10 | **189** | >10 |
| **190** | >10 | **191** | >10 | **192** | >10 | **193** | >10 | **194** | >10 |
| **197** | >10 | **198** | >10 | **200** | >10 | **201** | >10 | **202** | >10 |
| **203** | >10 | **204** | >10 | **205** | >10 | **207** | >10 | **208** | >10 |
| **209** | >10 | **210** | >10 | **211** | >10 | **212** | >10 | **213** | >10 |
| **214** | >10 | **215** | >10 | **217** | >10 | **218** | >10 | **219** | >10 |
| **220** | >10 | **221** | >10 | **222** | >10 | **223** | >10 | **224** | >10 |
| **225** | >10 | **226** | >10 | **228** | >10 | **229** | >10 | **230** | >10 |
| **234** | >10 | **235** | >10 | **236** | >10 | **237** | >10 | **238** | >10 |
| **239** | >10 | **240** | >10 | **242** | >10 | **243** | >10 | **244** | >10 |
| **245** | >10 | **248** | >10 | **258** | >10 | **268** | >10 | **273** | >10 |
| **276** | >10 | **277** | >10 | **279** | >10 | | | | |

Conclusion: The results showed that compounds of the present invention had weak inhibition activity on IL-2-induced JAK1/JAK3 signal pathway.

**Testing Example 282: Inhibition activity of compounds of the present invention on EPO-induced JAK2 signal**

**Main principle of the experiment:**

The cytokine EPO, i.e. erythropoietin, can promote the proliferation and division of erythroid progenitor cells, and the signal pathway induced by EPO coupling with its receptor is mediated by JAK2-STAT5. It is known that the proliferation of human promegakaryocytic leukemia cell line UT-7 relies heavily on EPO, and meanwhile, the selective inhibitor of JAK1/JAK2, ruxolitinib (INCB 18424), can effectively inhibit the proliferation of this cell line.

**Experimental materials and equipments:**

Human promegakaryocytic leukemia cell line UT-7 was commercially available from Cell Resource Center, Institute of Basic Medicine, Chinese Academy of Medical Sciences. The culture medium was RPIM1640 containing 10% FBS, and EPO was added for a final concentration of 10 ng/mL.

AlamarBlue™ Cell Viability Reagent (Invitrogen™) was used the fluorescent color reaction for demonstrating cell activities, with fluorescent signal detected using Tecan Spark™ 10M multimode microplate reader. The inhibition rate of the tested compound on EPO-induced cell proliferation was calculated using Microsoft Excel.

**Principal experimental procedure:**

UT-7 cell line was inoculated in 96-well plate with flat bottom at 15000 cells per well, each well was added with 10 ng/mL of human recombinant EPO (Human EPO PEPROTECH 100-64-10UG), along with tested compound respectively for a final concentration of 0, 100, 1000 or 10000 nM. After culture at 37°C and 5% $CO_2$ for 72 hours, alamarBlue™ Cell Viability Reagent was added to each well following the instructions. After incubation at 37 °C for color reaction, the fluorescence value at Ex530/Em590 was detected using Tecan Spark™ 10M multimode microplate reader.

The well in which neither compound nor cytokine was added was designated as 0% control well, and the fluorescence value in the wells in which the final concentration of compound was 0 was designated as 100% control. The inhibition activity $IC_{50}$ of the tested compound on JAK2 could be calculated from the inhibition rate of the tested compound on EPO-induced cell proliferation at different doses. The results were shown in following Table 3:

**Table 3: Inhibition activity of compounds of the present invention on EPO-induced JAK2 signal pathway**

| Compound # | $IC_{50}$ (μM) | Compound # | $IC_{50}$ (μM) | Compound # | $IC_{50}$ (μM) | Compound # | $IC_{50}$ (μM) | Compound # | $IC_{50}$ (μM) |
|---|---|---|---|---|---|---|---|---|---|
| **ruxolitinib** | <0.1 | **1** | >10 | **2** | >10 | **3** | >10 | **4** | >10 |
| **5** | >10 | **6** | >10 | **7** | >10 | **8** | >10 | **9** | >10 |
| **10** | >10 | **11** | >10 | **12** | >10 | **13** | >10 | **14** | >10 |
| **15** | >10 | **16** | >10 | **17** | >10 | **18** | >10 | **19** | >10 |
| **20** | >10 | **21** | >10 | **22** | >10 | **23** | >10 | **24** | >10 |
| **25** | >10 | **26** | >10 | **27** | >10 | **28** | >10 | **29** | >10 |
| **30** | >10 | **31** | >10 | **32** | >10 | **33** | >10 | **34** | >10 |
| **35** | >10 | **36** | >10 | **37** | >10 | **38** | >10 | **39** | >10 |
| **40** | >10 | **41** | >10 | **42** | >10 | **43** | >10 | **44** | >10 |
| **45** | >10 | **46** | >10 | **47** | >10 | **48** | >10 | **49** | >10 |
| **50** | >10 | **51** | >10 | **52** | >10 | **53** | >10 | **54** | >10 |
| **55** | >10 | **56** | >10 | **57** | >10 | **58** | >10 | **59** | >10 |
| **60** | >10 | **61** | >10 | **62** | >10 | **63** | >10 | **64** | >10 |
| **65** | >10 | **66** | >10 | **67** | >10 | **68** | >10 | **69** | >10 |
| **70** | >10 | **71** | >10 | **72** | >10 | **73** | >10 | **74** | >10 |
| **75** | >10 | **76** | >10 | **77** | >10 | **78** | >10 | **79** | >10 |
| **80** | >10 | **81** | >10 | **82** | >10 | **83** | >10 | **84** | >10 |
| **85** | >10 | **86** | >10 | **87** | >10 | **88** | >10 | **89** | >10 |
| **90** | >10 | **91** | >10 | **92** | >10 | **93** | >10 | **94** | >10 |

(continued)

| Compound # | $IC_{50}$ (μM) | Compound # | $IC_{50}$ (μM) | Compound # | $IC_{50}$ (μM) | Compound # | $IC_{50}$ (μM) | Compound # | $IC_{50}$ (μM) |
|---|---|---|---|---|---|---|---|---|---|
| **95** | >10 | **96** | >10 | **97** | >10 | **98** | >10 | **99** | >10 |
| **100** | >10 | **101** | >10 | **102** | >10 | **103** | >10 | **104** | >10 |
| **105** | >10 | **106** | >10 | **107** | >10 | **108** | >10 | **109** | >10 |
| **110** | >10 | **111** | >10 | **112** | >10 | **113** | >10 | **114** | >10 |
| **115** | >10 | **116** | >10 | **117** | >10 | **118** | >10 | **119** | >10 |
| **120** | >10 | **121** | >10 | **122** | >10 | **123** | >10 | **124** | >10 |
| **125** | >10 | **126** | >10 | **127** | >10 | **128** | >10 | **129** | >10 |
| **130** | >10 | **131** | >10 | **132** | >10 | **133** | >10 | **134** | >10 |
| **135** | >10 | **136** | >10 | **137** | >10 | **138** | >10 | **139** | >10 |
| **140** | >10 | **141** | >10 | **142** | >10 | **143** | >10 | **144** | >10 |
| **145** | >10 | **146** | >10 | **147** | >10 | **148** | >10 | **149** | >10 |
| **150** | >10 | **151** | >10 | **152** | >10 | **153** | >10 | **154** | >10 |
| **155** | >10 | **156** | >10 | **157** | >10 | **158** | >10 | **159** | >10 |
| **160** | >10 | **161** | >10 | **162** | >10 | **163** | >10 | **164** | >10 |
| **165** | >10 | **166** | >10 | **167** | >10 | **168** | >10 | **169** | >10 |
| **170** | >10 | **171** | >10 | **172** | >10 | **173** | >10 | **174** | >10 |
| **175** | >10 | **176** | >10 | **177** | >10 | **178** | >10 | **179** | >10 |
| **180** | >10 | **181** | >10 | **182** | >10 | **183** | >10 | **184** | >10 |
| **185** | >10 | **186** | >10 | **187** | >10 | **188** | >10 | **189** | >10 |
| **190** | >10 | **191** | >10 | **192** | >10 | **193** | >10 | **194** | >10 |
| **197** | >10 | **198** | >10 | **200** | >10 | **201** | >10 | **202** | >10 |
| **203** | >10 | **204** | >10 | **205** | >10 | **207** | >10 | **208** | >10 |
| **209** | >10 | **210** | >10 | **211** | >10 | **212** | >10 | **213** | >10 |
| **214** | >10 | **215** | >10 | **217** | >10 | **218** | >10 | **219** | >10 |
| **220** | >10 | **221** | >10 | **222** | >10 | **223** | >10 | **224** | >10 |
| **225** | >10 | **226** | >10 | **228** | >10 | **229** | >10 | **230** | >10 |
| **234** | >10 | **235** | >10 | **236** | >10 | **237** | >10 | **238** | >10 |
| **239** | >10 | **240** | >10 | **242** | >10 | **243** | >10 | **244** | >10 |
| **245** | >10 | **248** | >10 | **258** | >10 | **268** | >10 | **273** | >10 |
| **276** | >10 | **277** | >10 | **279** | >10 | | | | |

Conclusion: The results showed that compounds of the present invention had weak inhibition activity on EPO-induced JAK2 signal pathway.

IL-10 signal pathway reporter gene system was used to evaluate the target inhibition activity of compounds of the present invention at the cell level, aiming to evaluate the inhibition intensity of different compounds on IL-10-induced JAK1/TYK2-STAT3 signal pathway, thereby indirectly reflecting the inhibition activity of compound on TYK2.

Human natural killer cell lymphoma cell line KHYG-1, whose proliferation relied on IL-2, was used for testing the effect of compounds of the present invention on IL-2-induced JAK1/JAK3-STAT5 pathway at the cell level; and human promegakaryocytic leukemia cell line UT-7, whose proliferation relied on EPO, was used for testing the effect of compounds of the present invention on EPO-induced JAK2-STAT5 pathway at the cell level.

Based on the evaluation results of the above three cell models, it could be determined that compounds of the present invention showed selectivity to various subtypes of the JAK family, i.e., compounds of the present invention

showed strong inhibition activity on the TYK2-mediated signal pathway.

**Example 283: Analysis of inhibition activity of compounds of the present invention on TYK2 JH2 domain**

**Experimental protocol:**

Compound of the present invention formulated into different concentrations (series 3 times dilutions, started from 60 μ M, 11 concentrations in total) was added to 384-well plate, followed by 5 μL of enzyme (i.e., TYK2 JH2 domain), Tb antibody (Cisbio) and tracer. The mixture was incubated at 25°C for 60 min and left at 4 °C overnight. Based on the fluorescence signals of the reaction system at 665 nm and 615 nm read using Envision in FRET mode, inhibition rate was calculated with the following formula:

$$\%\ inhibition = 1 - \left[\frac{Ratio_{cmpd} - \overline{Ratio}_{low}}{\overline{Ratio}_{High} - \overline{Ratio}_{Low}}\right] * 100\%$$

wherein: Ratio meant the ratio of fluorescence signals (channal 665/channal 615); High meant high control, i.e., the fluorescence signal of the wells detected by the method which was operated similarly but no compound of the present invention was added; Low meant Low control, i.e., the fluorescence signal of the wells detected by the method which was operated similarly but neither enzyme nor compound of the present invention was added.

Using nonlinear regression analysis, data were fit to a S-shaped response (variable slope) curve, and the resulting $IC_{50}$ values were as follows:

**Table 4: Inhibition activity of compounds of the present invention on TYK2 JH2 domain**

| **Compound #** | $IC_{50}$ (nM) | **Compound#** | $IC_{50}$ (nM) | **Compound#** | $IC_{50}$ (nM) | **Compound#** | $IC_{50}$ (nM) |
|---|---|---|---|---|---|---|---|
| **BMS-986165** | 0.14 | **21** | 0.05 | **98** | 0.09 | **115** | 0.07 |
| **181** | 0.08 | **186** | 0.07 | **192** | 0.08 | **193** | 0.06 |
| **194** | 0.08 | **198** | 0.08 | **200** | 0.06 | **201** | 0.06 |
| **202** | 0.06 | **204** | 0.06 | **212** | 0.05 | **214** | 0.09 |
| **215** | 0.07 | **219** | 0.13 | **229** | 0.05 | **277** | 0.12 |

Conclusion: The results showed that compounds of the present invention had strong inhibition activity on TYK2 JH2 domain.

**Example 284: Inhibition experiment of compounds of the present invention on INF-α-induced pSTAT5 in human PBMC**

**Experimental protocol:**

hPBMC (available from Shanghai AoNeng Biotechnology Co., Ltd.) was inoculated in 96-well plate, added with compound of the present invention (at concentrations of 40nM and 200nM), incubated for 60 min (compound in concentrations of 2nM, 10nM respectively during the incubation); added with 30 ng/ml of stimulating factor IFN-α (BioLegend) together with anti-CD3 antibody (Biolegend) and then continued to be incubated for 30 min. After the incubation, each well was added with 1 mL of fixing solution (BD Biosciences), the obtained mixture was incubated for 10 min followed by centrifugation, added with 400 μL/well of Perm III (BD Biosciences), followed by further incubation at 4 °C for 30min. The obtained mixture was added with mouse anti-human pSTATS antibody (Alexa Fluor® 647 Conjugate), followed by incubation at room temperature for 40 min. Fluorescence intensity of pSTATS in CD3 positive cells was detected by FACS.

Inhibition rate at individual compound concentration was calculated with the following formula:

Inhibition rate = (MFI value (IFN-α-stimulated control)-MFI value (2/10 nM compound treated group) / MFI value (IFN-α-stimulated control) *100%

wherein: MFI value meant fluorescence intensity value; IFN-α-stimulated control meant control group which was operated

similarly to the experimental groups but no compound of the present invention was added.

**Table 5: Experimental results of inhibition activity of compounds of the present invention on INF-α-induced pSTAT5 in human PBMC**

| **Compound #** | $IC_{50}$ (nM) | **Compound #** | $IC_{50}$ (nM) | **Compound #** | $IC_{50}$ (nM) | **Compound #** | $IC_{50}$ (nM) |
|---|---|---|---|---|---|---|---|
| **BMS-986165** | 2.004 | 202 | <2 | **205** | <2 | **211** | <2 |
| **212** | <2 | **215** | <2 | **219** | <2 | **225** | <2 |
| **238** | <2 | **248** | <2 | | | | |

Conclusion: The results showed that compounds of the present invention had strong inhibition activity on INF-α-induced pSTAT5 in human PBMC.

**Example 285: *In-vivo* pharmacokinetic test in mice**

**Main principle of the experiment:**

LC-MS/MS method is used to determine the drug concentration in the plasma of mice at different times after intragastric administration of the compound of the example. Relevant pharmacokinetic parameters are calculated to study the pharmacokinetic behavior of the compound of the present invention in mice, thereby evaluating the pharmacokinetic characteristics thereof.

**Experimental materials, protocols and result analysis:**

Test animal was healthy adult BALB/c female mouse (provided by Chengdu Yaokang Biotechnology Co., Ltd.); Compound administration and sample collection: compound was administered to BALB/c female mice by gavage (10 mg/kg). At 0.25, 0.5, 1, 2, 4, 6, 8, and 24 hours after administration, 60μL of whole blood of the mouse fundus venous plexus were collected and centrifuged at 4000 rpm for 10 min to provide plasma.

Sample analysis: 10 μL of mouse plasma samples were respectively added with 290μL of acetonitrile solution containing internal standard to precipitate proteins. After swirling for 10 min and centrifuging at 4000 rpm for 10 min, 200 μL of supernatant was added to 96-well plate, and analyzed by LC-MS/MS at an injection volume of 1 μL.

Under the same dose and administration mode, compounds of the present invention resulted in the pharmacokinetic parameters in mice as shown in following Table 6:

Table 6: *In-vivo* pharmacokinetic parameters of compounds in mice

| **Example #** | **BMS-986165** | **208** | **214** | **212** | **238** | **215** | **213** | **219** |
|---|---|---|---|---|---|---|---|---|
| **AUC(μM*h)** | 10.1 | 34.3 | 41.9 | 16.3 | 39.9 | 25.8 | 49.3 | 16.8 |
| **$C_{max}$(μM)** | 4.27 | 16.45 | 9.21 | 14.96 | 15.12 | 13.57 | 18.01 | 10.34 |

Conclusion: The results showed that the compounds of the present invention were obviously superior to the positive control, i.e. BMS-986165, in terms of properties such as drug plasma concentration.

## Claims

**1.** A compound represented by formula (I) or a pharmaceutically acceptable salt thereof:

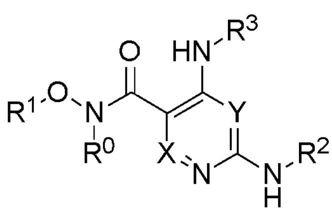

(I)

wherein:

X is $CR^a$;
Y is CH;
$R^a$ is hydrogen;
$R^0$ is hydrogen;
$R^1$ is selected from the group consisting of $C_{1-6}$ alkyl optionally substituted by from one to seven $R^{1a}$ groups;
$R^{1a}$ is selected from the group consisting of deuterium, F and Cl;
$R^2$ is selected from the group consisting of $C_{3-6}$ cycloalkyl-C(O)-, $C_{3-6}$ heterocyclyl-C(O)- wherein the $C_{3-6}$ heterocyclyl contains from one to two heteroatoms selected from N, O and S, phenyl, and 5-6 membered heteroaryl containing from one to two heteroatoms selected from N, O and S, wherein the $C_{3-6}$ cycloalkyl, $C_{3-6}$ heterocyclyl containing from one to two heteroatoms selected from N, O and S, phenyl, and 5-6 membered heteroaryl containing from one to two heteroatoms selected from N, O and S are respectively and optionally substituted by from one to two $R^{2a}$ groups;
$R^{2a}$ is selected from the group consisting of $C_{1-6}$ alkyl, halogen, $C_{1-6}$ alkoxy, $C_{3-6}$ cycloalkyl, cyano, halo $C_{1-6}$ alkyl, $C_{1-6}$ alkyl substituted by hydroxyl group, and $C_{1-6}$ alkyl-$S(O)_2$-;
$R^3$ is selected from the group consisting of phenyl, pyridinyl, 2-pyridinonyl and indazolyl, wherein the phenyl, pyridinyl, 2-pyridinonyl and indazolyl are optionally substituted by from one to three $R^{3a}$ groups;
$R^{3a}$ is selected from the group consisting of F, Cl, Br, I, CN, $-NO_2$, $-OR^b$, $-SR^b$,$-C(O)OR^b$, $-C(O)NR^bR^c$, $-NR^bC(O)R^c$, $-NR^bC(O)OR^c$, $-NRbC(O)NR^cR^c$, $-NR^bS(O)_2R^c$, $-S(O)_2R^c$, $-S(O)_2NR^cR^c$, $-P(O)R^bR^c$, $C_{1-6}$ alkyl optionally substituted by from one to three $R^d$ groups, $C_{2-6}$ alkenyl, $C_{2-6}$ alkynyl, $C_{3-6}$ cycloalkyl, 5-9 membered heteroaryl containing from one to three heteroatoms selected from N, O and S and optionally substituted by from one to three $R^d$ groups, and 5-6 membered saturated heterocyclyl containing from one to two heteroatoms selected from N, O and S;
$R^b$ is selected from the group consisting of hydrogen, $C_{1-6}$ alkyl optionally substituted by from one to three $R^d$ groups, and 4-6 membered saturated heterocyclyl containing from one to two oxygen atoms;
$R^c$ is selected from the group consisting of hydrogen, $C_{1-6}$ alkyl and $C_{3-6}$ cycloalkyl;
$R^d$ is selected from the group consisting of hydrogen, deuterium, F, Cl, Br, I,$-OCF_3$, $-CF_3$, $-(CH_2)_r-CN$, $-NO_2$, $-OR^e$, $-(CH_2)_r-COR^c$, $-NR^eR^e$, $-NR^eC(O)OR^c$, $C_1$-$C_6$ alkyl, $C_{3-6}$ cycloalkyl, and $-(CH_2)_r$-phenyl;
$R^e$ is hydrogen;
p is 0, 1 or 2, and
r is 0, 1, 2, 3 or 4.

**2.** The compound or a pharmaceutically acceptable salt thereof as claimed in claim 1, wherein:

X is $CR^a$;
$R^a$ is hydrogen;
$R^0$ is hydrogen;
$R^1$ is selected from the group consisting of methyl, ethyl, isopropyl, t-butyl, trideuterated methyl and 2,2,2-trifluoroethyl;
$R^2$ is selected from the group consisting of cyclopropylcarbonyl,

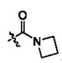,

phenyl, pyrazolyl, thiazolyl, pyridinyl, pyrazinyl, pyrimidinyl and pyridazinyl, wherein the cyclopropylcarbonyl,

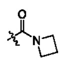,

phenyl, pyrazolyl, thiazolyl, pyridinyl, pyrazinyl, pyrimidinyl and pyridazinyl are optionally substituted by from one to two $R^{2a}$ groups;
$R^{2a}$ is selected from the group consisting of methyl, F, methoxy, cyclopropyl, cyano, $-CF_3$, hydroxymethyl and methanesulfonyl;
$R^3$ is selected from the group consisting of phenyl, pyridinyl, 2-pyridinonyl and indazolyl, wherein the phenyl, pyridinyl, 2-pyridinonyl and indazolyl are optionally substituted by from one to three $R^{3a}$ groups;
$R^{3a}$ is selected from the group consisting of F, Cl, Br, I, CN, $-OR^b$, $-C(O)NR^bR^c$,$-NR^bS(O)_2R^c$, $-S(O)_2R^c$, $-S(O)_2NR^cR^c$, $-P(O)R^bR^c$, $C_{1-6}$ alkyl optionally substituted by from one to three $R^d$ groups, $C_{2-6}$ alkynyl, $C_{3-6}$

cycloalkyl, 5-9 membered heteroaryl containing from one to three heteroatoms selected from N, O and S and optionally substituted by from one to three $R^d$ groups, and 5-6 membered saturated heterocyclyl containing from one to two heteroatoms selected from N, O and S;

$R^b$ is selected from the group consisting of hydrogen; methyl, ethyl and isopropyl, each optionally substituted by from one to three $R^d$ groups; and

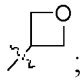 ;

$R^c$ is selected from the group consisting of hydrogen, methyl, and cyclopropyl;

$R^d$ is selected from the group consisting of deuterium, F, Cl, hydroxyl, methyl, cyclopropyl and isopropyl.

**3.** The compound or a pharmaceutically acceptable salt thereof as claimed in claim 1 or 2, wherein:

X is CH;

$R^1$ is selected from the group consisting of methyl, ethyl, isopropyl, t-butyl, trideuterated methyl and 2,2,2-trifluoroethyl;

$R^{2a}$ is selected from the group consisting of methyl, F, methoxy, cyclopropyl, cyano, $-CF_3$, hydroxymethyl and methanesulfonyl;

$R^{3a}$ is selected from the group consisting of F, Cl, Br, I, CN, $-OR^b$, $-C(O)NR^bR^c$,$-NR^bS(O)_2R^c$, $-S(O)_2R^c$, $-S(O)_2NR^cR^c$, $-P(O)R^bR^c$, $C_{1-3}$ alkyl optionally substituted by from one to three $R^d$ groups, $C_{2-4}$ alkynyl, $C_{3-5}$ cycloalkyl, 5-9 membered heteroaryl containing from one to three heteroatoms selected from N, O and S and optionally substituted by $R^d$ group, and 5-6 membered saturated heterocyclyl containing from one to two heteroatoms selected from N, O and S, wherein the 5-9 membered heteroaryl containing from one to three heteroatoms selected from N, O and S is selected from the group consisting of thiazolyl, benzimidazolyl, pyrazolyl, oxadiazolyl, triazolyl, pyridinyl, pyrimidinyl and pyrazinyl, wherein the 5-6 membered saturated heterocyclyl containing from one to two heteroatoms selected from N, O and S is selected from the group consisting of morpholinyl and piperidinyl;

$R^b$ is selected from the group consisting of hydrogen; methyl, ethyl and isopropyl, each optionally substituted by from one to three $R^d$ groups; and

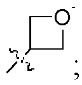 ;

$R^c$ is selected from the group consisting of hydrogen, methyl and cyclopropyl;

$R^d$ is selected from the group consisting of deuterium, F, Cl, hydroxyl, methyl, cyclopropyl and isopropyl.

**4.** The compound or a pharmaceutically acceptable salt thereof as claimed in claim 1 or 2, wherein:
$R^2$ is selected from the group consisting of

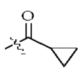, 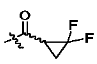, 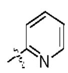, 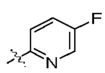,

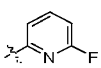, 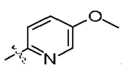, 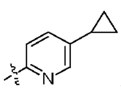, 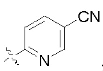, 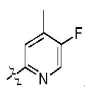, 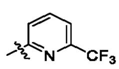, 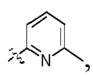, 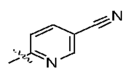, 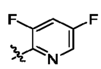,

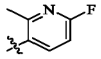, 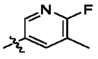, 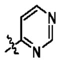, 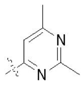, 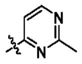, 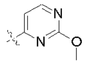, 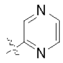, 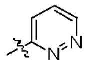, 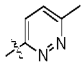, 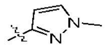,

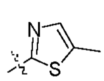 and 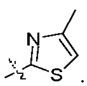.

**5.** The compound or a pharmaceutically acceptable salt thereof as claimed in claim 1 or 2, wherein:

$R^3$ is selected from the group consisting of:

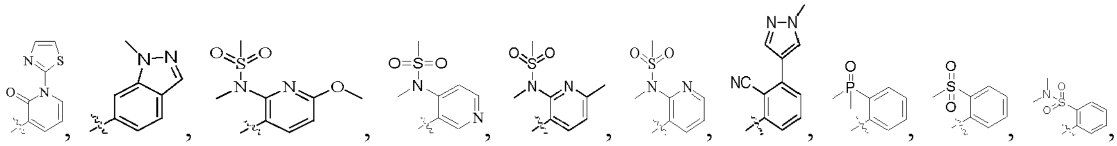, , , , , , , , , ,

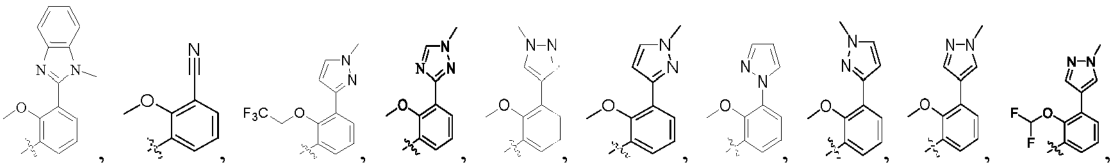, , , , , , , , , ,

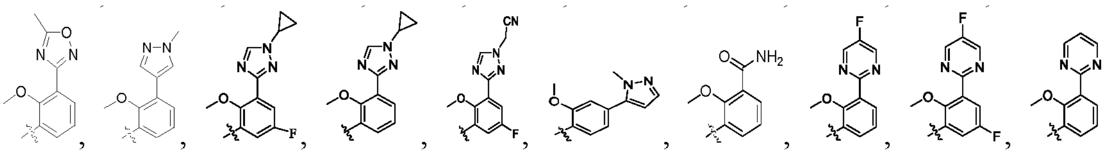, , , , , , , , , ,

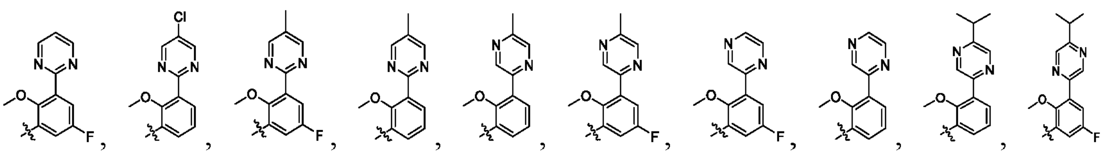, , , , , , , , , ,

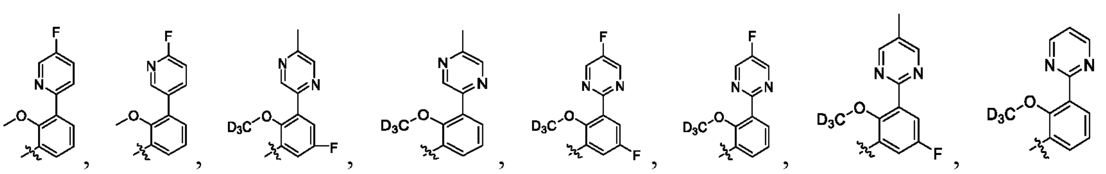, , , , , , , ,

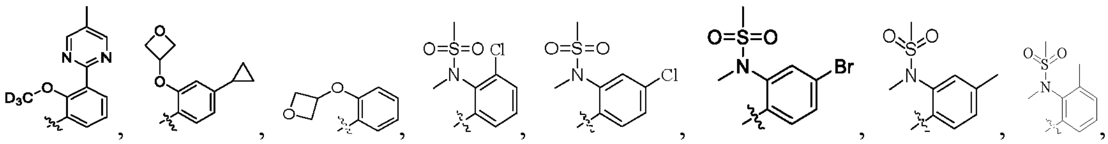, , , , , , , ,

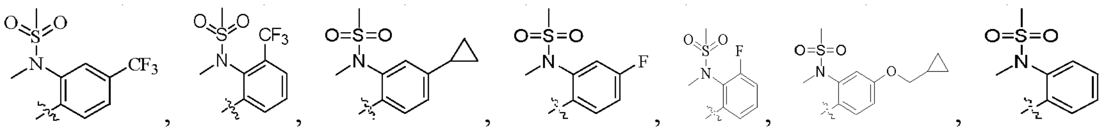, , , , , , ,

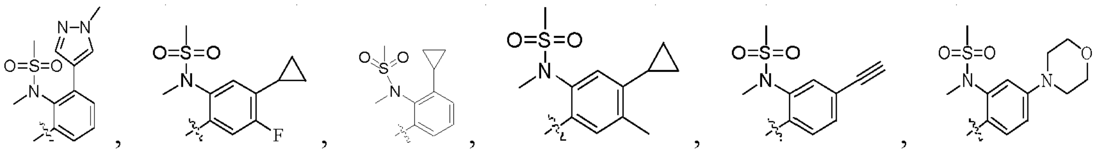, , , , , ,

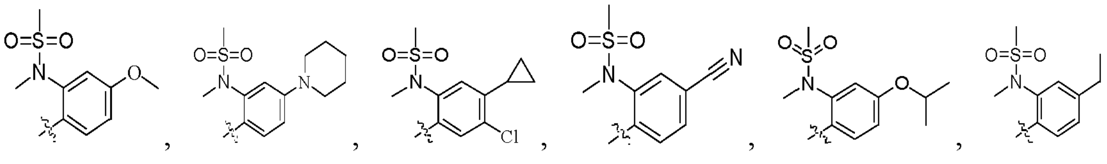, , , , , ,

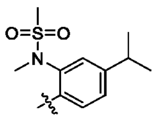, 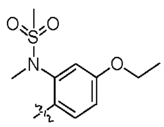, 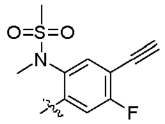, 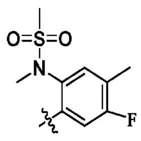, 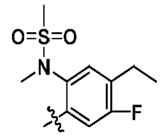, 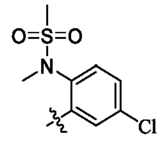,

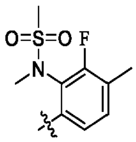, 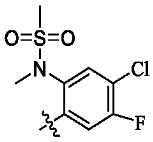, 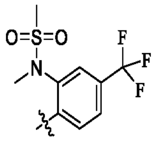, 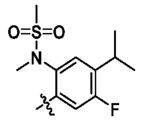, 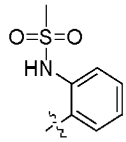, 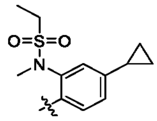,

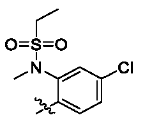, 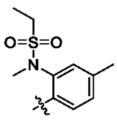, 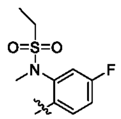, 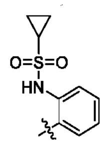, 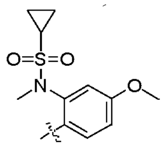, 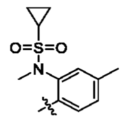, 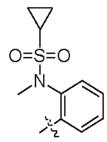,

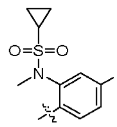, 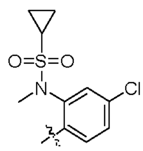, 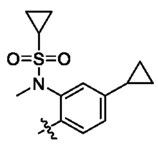 and 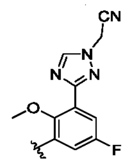.

**6.** The compound or a pharmaceutically acceptable salt thereof as claimed in claim 1, wherein:

X is CH;
Y is CH;
$R^0$ is hydrogen;
$R^1$ is ethyl;
$R^2$ is selected from the group consisting of pyridinyl, pyrimidinyl, -C(O)-$C_{3-6}$ cycloalkyl and -C(O)-$C_{3-6}$ heterocyclyl, wherein the $C_{3-6}$ heterocyclyl contains from one to two heteroatoms selected from N, O and S; wherein the $C_{3-6}$ cycloalkyl and $C_{3-6}$ heterocyclyl are respectively and optionally substituted by from one to three $R^{2a}$ groups;
$R^{2a}$ is selected from the group consisting of $C_{1-6}$ alkyl, halogen, $C_{1-6}$ alkoxy, $C_{3-6}$ cycloalkyl, cyano, halo $C_{1-6}$ alkyl, $C_{1-6}$ alkyl substituted by hydroxyl group, and $C_{1-6}$ alkyl-$S(O)_2$-;
$R^3$ is phenyl optionally substituted by from one to three $R^{3a}$ groups;
$R^{3a}$ is selected from the group consisting of $-(CH_2)_r-OR^b$, $-NR^bS(O)_pR^c$, F, Cl, $C_{1-6}$ alkyl, $C_{3-6}$ cycloalkyl, triazolyl, pyrazolyl, pyrimidinyl and pyrazinyl; wherein the $C_{1-6}$ alkyl, $C_{3-6}$ cycloalkyl, triazolyl, pyrazolyl, pyrimidinyl and pyrazinyl are, respectively and independently, optionally substituted by from one to three $R^d$ groups;
$R^b$ is selected from the group consisting of hydrogen, $C_{1-6}$ alkyl optionally substituted by from one to three $R^d$ groups, and 4-6 membered saturated heterocyclyl containing from one to two oxygen atoms;
$R^c$ is selected from the group consisting of hydrogen, $C_{1-6}$ alkyl and $C_{3-6}$ cycloalkyl;
$R^d$ is selected from the group consisting of hydrogen, deuterium, F, Cl, Br, I, $OCF_3$, $CF_3$, $-(CH_2)_r-CN$, $NO_2$, $OR^e$, $-(CH_2)_r-COR^c$, $-NR^eR^e$, $-NR^eC(O)OR^c$, $C_1$-$C_6$ alkyl, $C_{3-6}$ cycloalkyl, and $-(CH_2)_r$-phenyl;
$R^e$ is hydrogen;
p is 0, 1 or 2, and
r is 0, 1, 2, 3 or 4.

**7.** The compound or a pharmaceutically acceptable salt thereof as claimed in claim 6, wherein, $R^{2a}$ is selected from the group consisting of methyl, F, methoxy, cyclopropyl, cyano, $-CF_3$, hydroxymethyl and methanesulfonyl.

**8.** The compound or a pharmaceutically acceptable salt thereof as claimed in claim 6, wherein, $R^2$ is selected from the following structures:

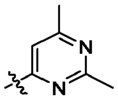, 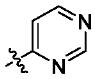, 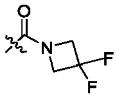, 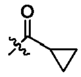, 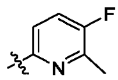, 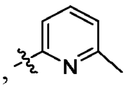, 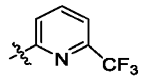, 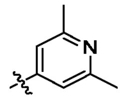,

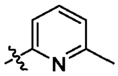, 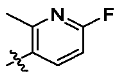, 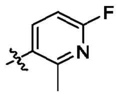, 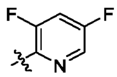, 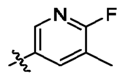, 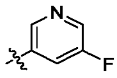, 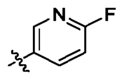, 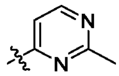,

and

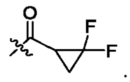.

**9.** The compound or a pharmaceutically acceptable salt thereof as claimed in claim 6, wherein, $R^b$ is selected from the group consisting of hydrogen; methyl, ethyl, and isopropyl, each optionally substituted by from one to three $R^d$ groups; and

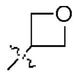.

**10.** The compound or a pharmaceutically acceptable salt thereof as claimed in claim 6, wherein, $R^c$ is selected from the group consisting of hydrogen, methyl, ethyl and cyclopropyl.

**11.** The compound or a pharmaceutically acceptable salt thereof as claimed in claim 6, wherein, $R^d$ is selected from the group consisting of F, Cl, hydroxyl, methyl, cyclopropyl and $-CH_2CN$.

**12.** The compound or a pharmaceutically acceptable salt thereof as claimed in claim 6, wherein, $R^3$ is selected from the following structures:

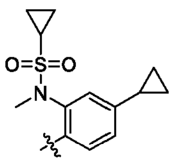, 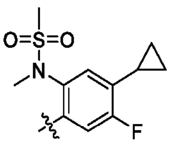, 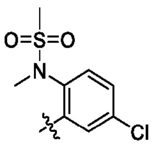, 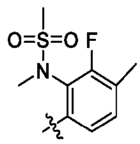, 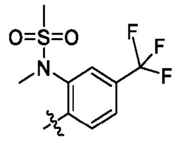, 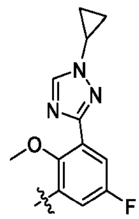,

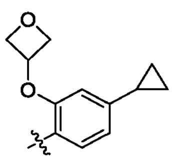, 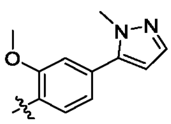, 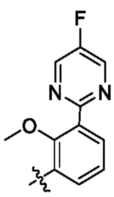, 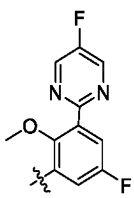, 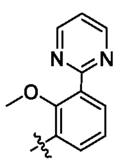, 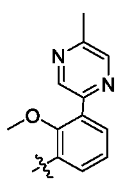, 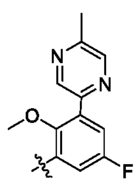,

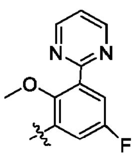, 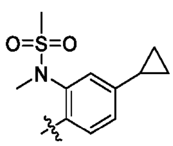, 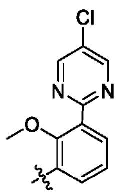, 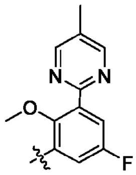, 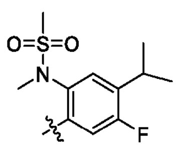, 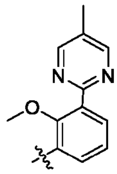, 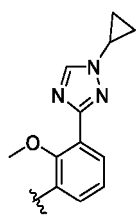,

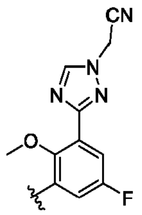, 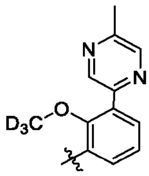, 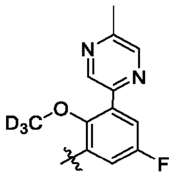, 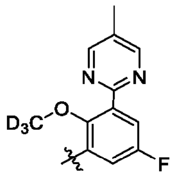, 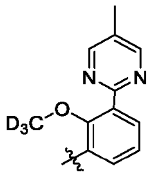, 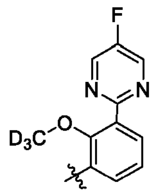,

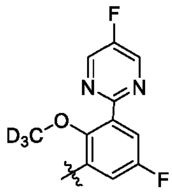, 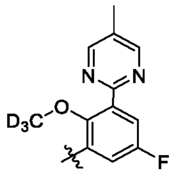, and 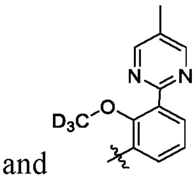.

**13.** The compound or a pharmaceutically acceptable salt thereof as claimed in claim 1, wherein the compound is selected from the group consisting of:

| Compound # | Structure | Compound # | Structure |
|---|---|---|---|
| 2 | 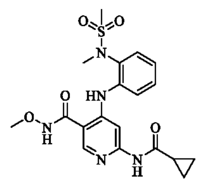 | 157 | 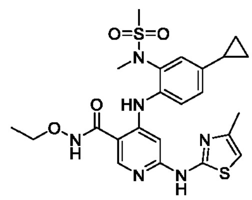 |
| 4 | 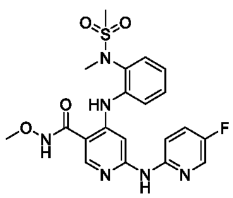 | 159 | 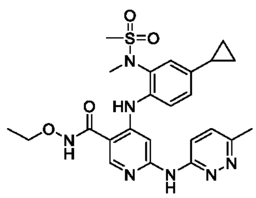 |
| 10 | 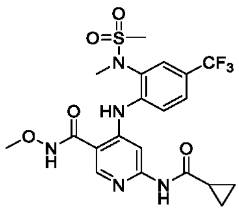 | 160 | 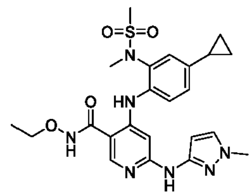 |
| 12 | 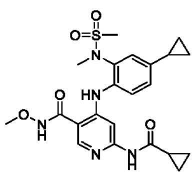 | 162 | 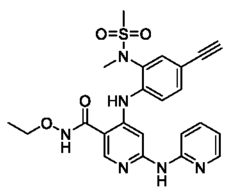 |
| 13 | 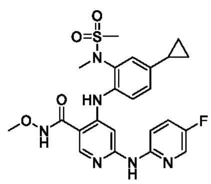 | 166 | 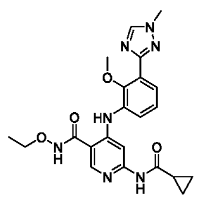 |

(continued)

| Compound # | Structure | Compound # | Structure |
|---|---|---|---|
| 14 | 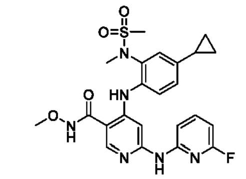 | 167 | 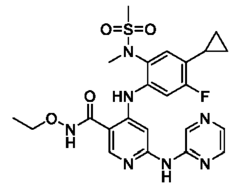 |
| 16 | 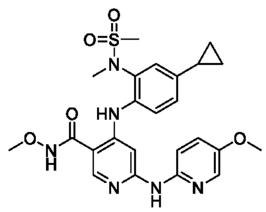 | 170 | 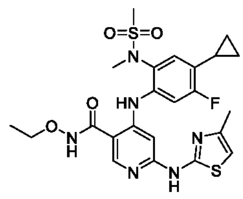 |
| 18 | 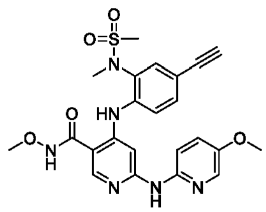 | 171 | 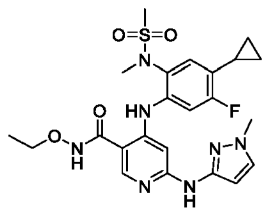 |
| 19 | 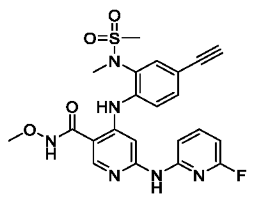 | 173 | 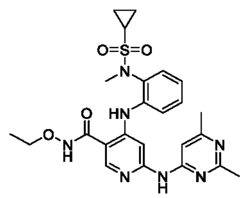 |
| 20 | 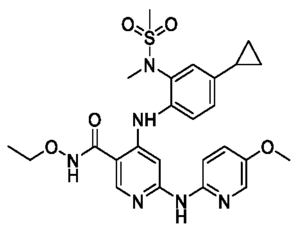 | 174 | 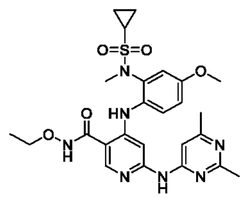 |
| 21 | 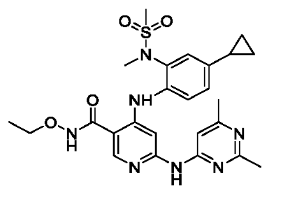 | 175 | 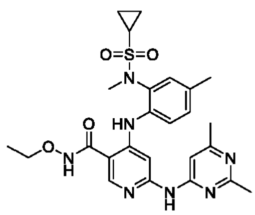 |
| 22 | 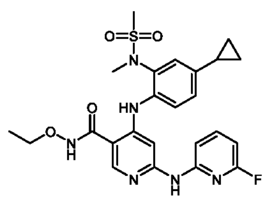 | 176 | 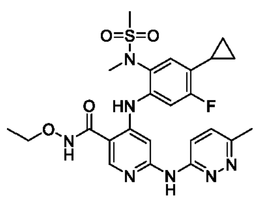 |

(continued)

| Compound # | Structure | Compound # | Structure |
|---|---|---|---|
| 29 | 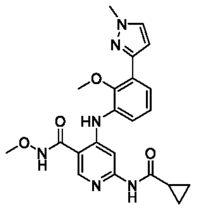 | 177 | 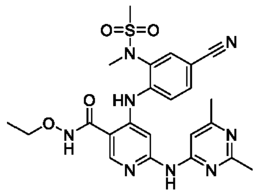 |
| 36 | 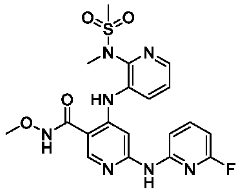 | 178 | 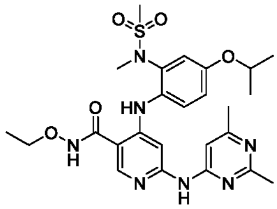 |
| 38 | 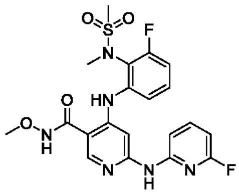 | 179 | 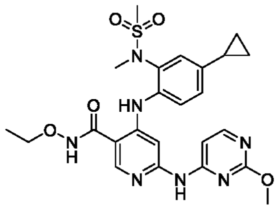 |
| 41 | 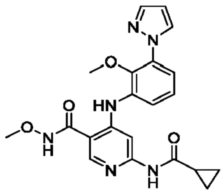 | 180 | 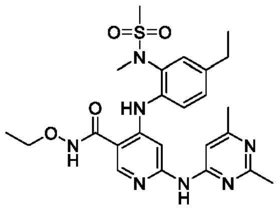 |
| 42 | 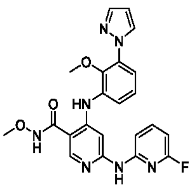 | 181 | 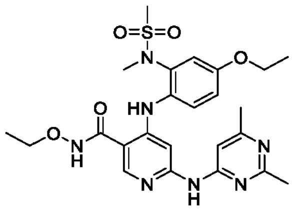 |
| 43 | 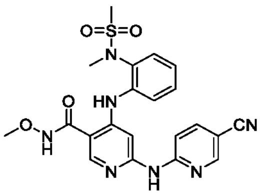 | 182 | 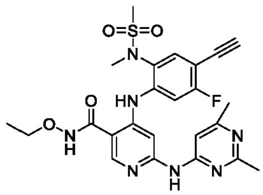 |
| 44 | 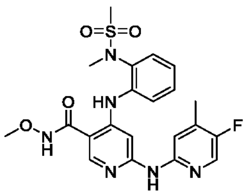 | 183 | 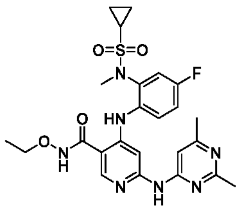 |

(continued)

| Compound # | Structure | Compound # | Structure |
|---|---|---|---|
| 45 | 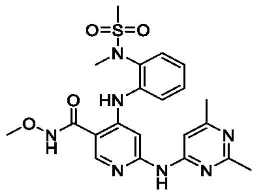 | 185 | 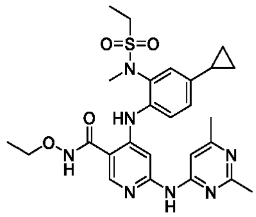 |
| 46 | 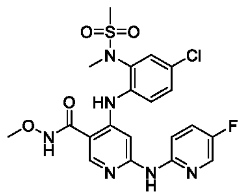 | 186 | 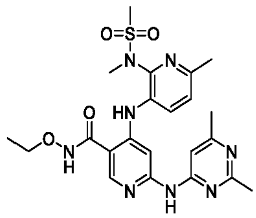 |
| 47 | 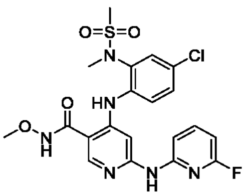 | 187 | 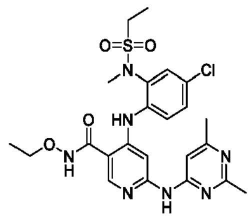 |
| 60 | 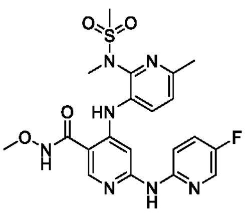 | 188 | 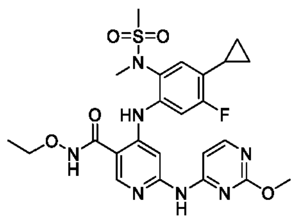 |
| 61 | 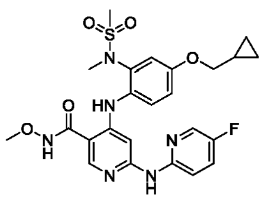 | 189 | 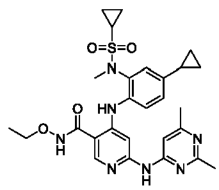 |
| 62 | 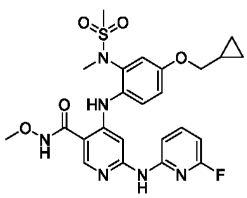 | 190 | 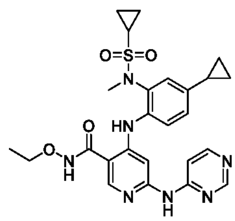 |
| 63 | 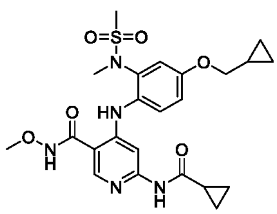 | 197 | 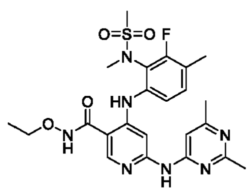 |

(continued)

| Compound # | Structure | Compound # | Structure |
|---|---|---|---|
| 68 | 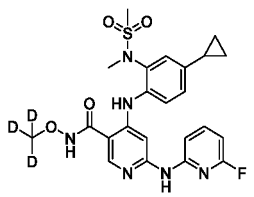 | 198 | 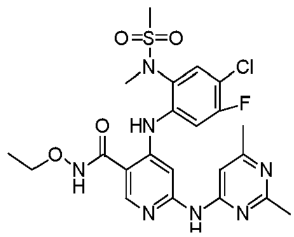 |
| 69 | 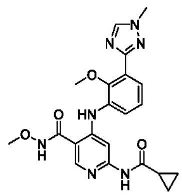 | 200 | 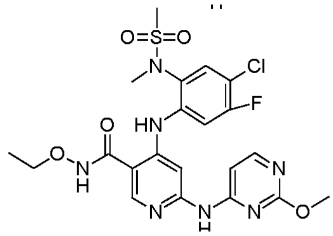 |
| 70 | 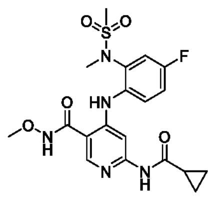 | 202 | 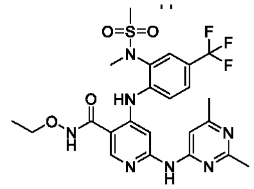 |
| 79 | 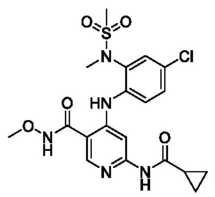 | 204 | 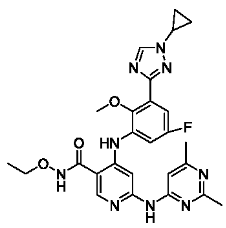 |
| 80 | 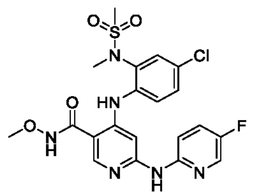 | 194 | 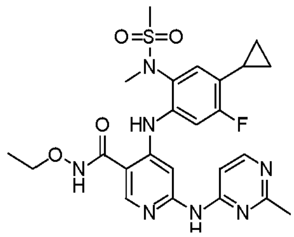 |
| 81 | 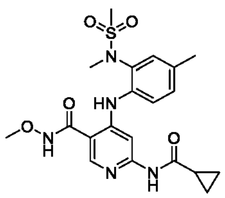 | 192 | 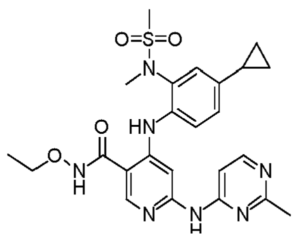 |
| 90 | 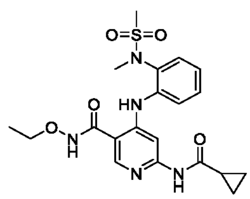 | 193 | 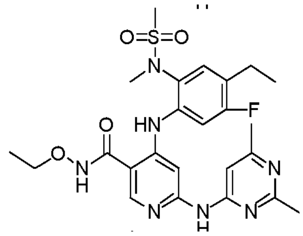 |

(continued)

| Compound # | Structure | Compound # | Structure |
|---|---|---|---|
| 95 | 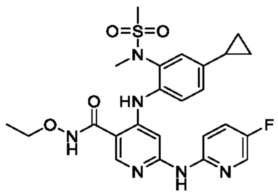 | 203 | 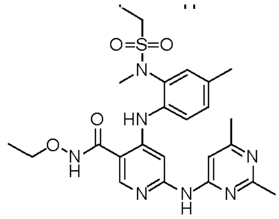 |
| 97 | 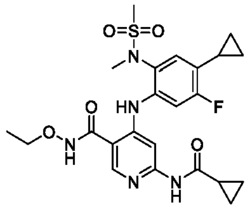 | 230 | 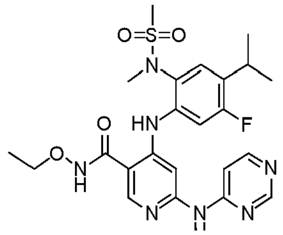 |
| 98 | 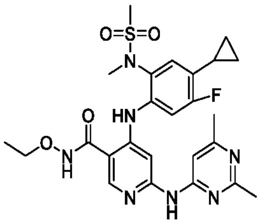 | 229 | 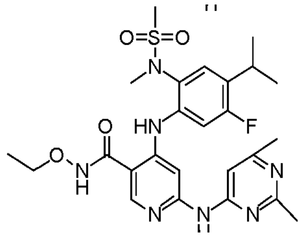 |
| 101 | 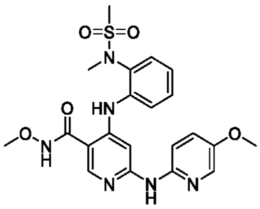 | 201 | 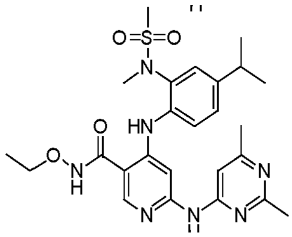 |
| 102 | 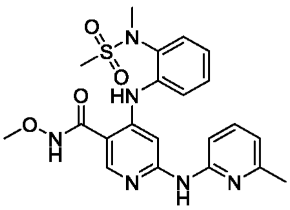 | 191 | 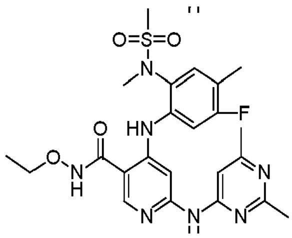 |
| 106 | 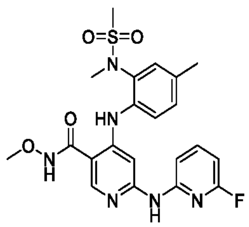 | 205 | 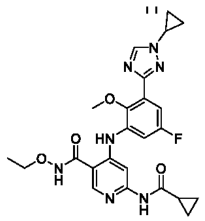 |
| 108 | 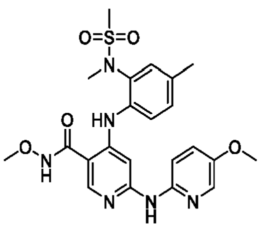 | 207 | 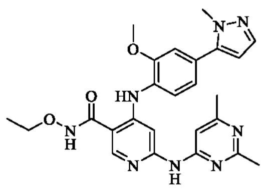 |

(continued)

| Compound # | Structure | Compound # | Structure |
|---|---|---|---|
| 109 | 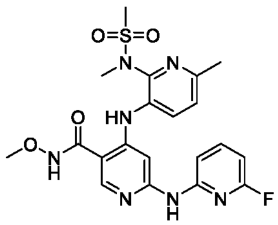 | 208 | 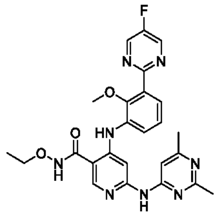 |
| 110 | 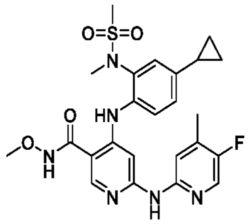 | 209 | 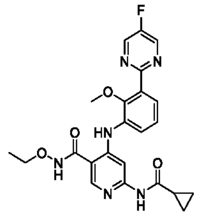 |
| 111 | 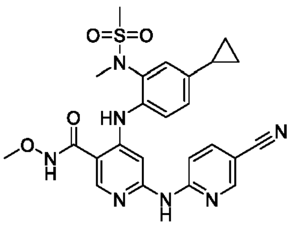 | 210 | 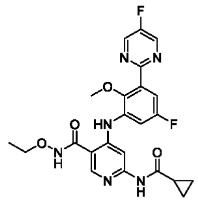 |
| 112 | 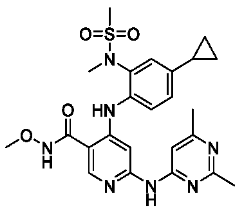 | 211 | 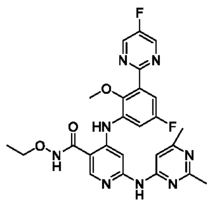 |
| 113 | 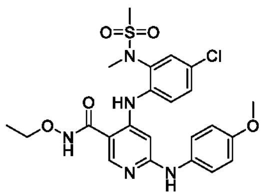 | 212 | 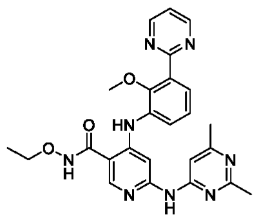 |
| 114 | 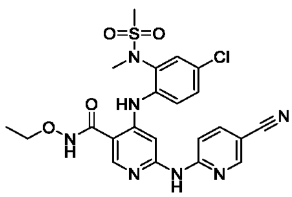 | 213 | 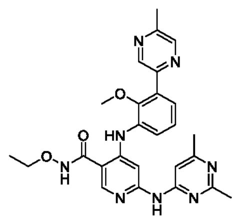 |
| 115 | 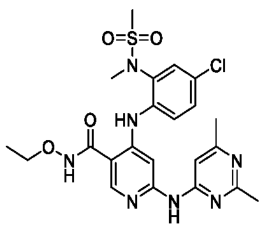 | 214 | 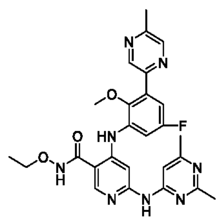 |

(continued)

| Compound # | Structure | Compound # | Structure |
|---|---|---|---|
| 116 | 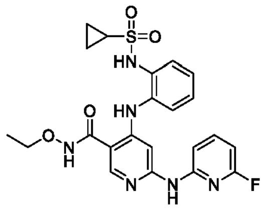 | 215 | 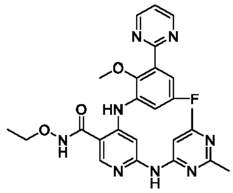 |
| 118 | 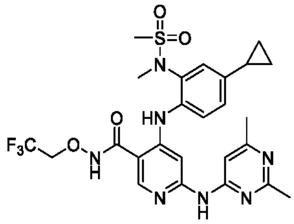 | 217 | 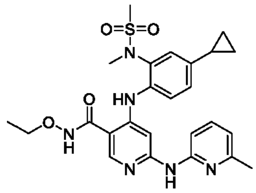 |
| 119 | 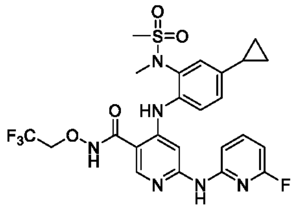 | 218 | 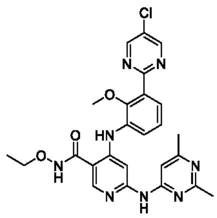 |
| 125 | 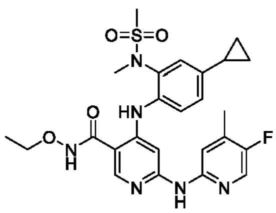 | 219 | 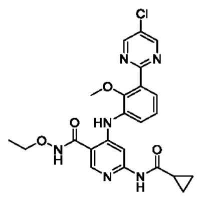 |
| 126 | 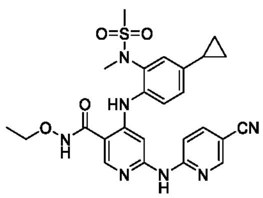 | 220 | 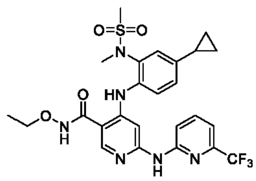 |
| 127 | 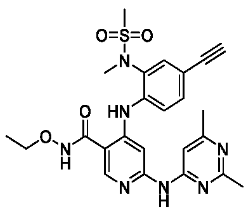 | 222 | 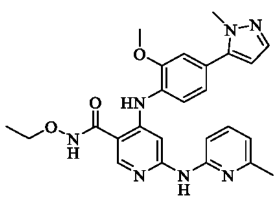 |
| 128 | 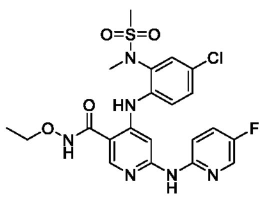 | 223 | 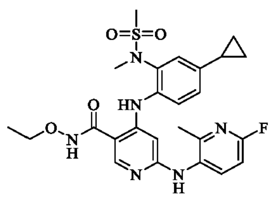 |

(continued)

| Compound # | Structure | Compound # | Structure |
|---|---|---|---|
| 129 | 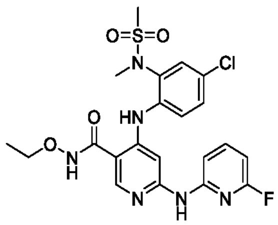 | 224 | 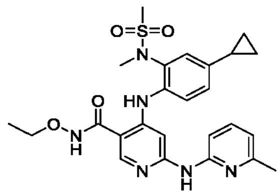 |
| 130 | 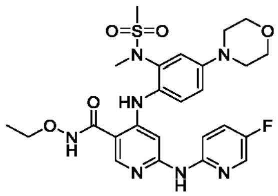 | 225 | 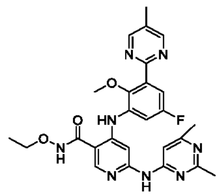 |
| 131 | 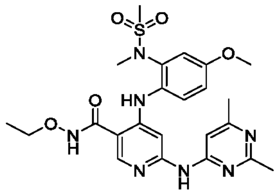 | 226 | 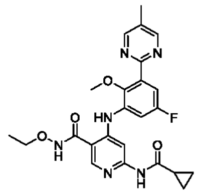 |
| 132 | 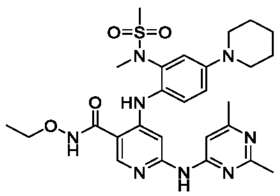 | 234 | 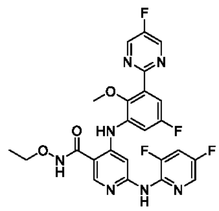 |
| 133 | 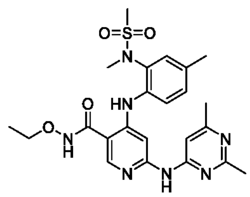 | 235 | 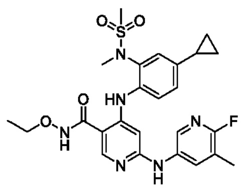 |
| 134 | 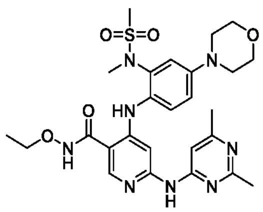 | 236 | 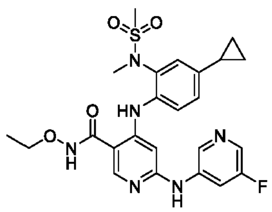 |
| 136 | 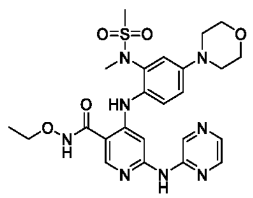 | 237 | 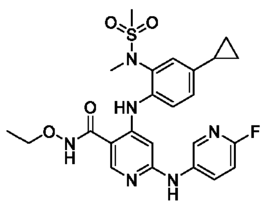 |

(continued)

| Compound # | Structure | Compound # | Structure |
|---|---|---|---|
| 138 | 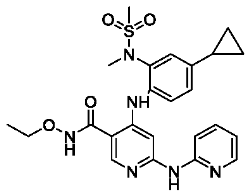 | 238 | 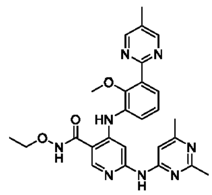 |
| 139 | 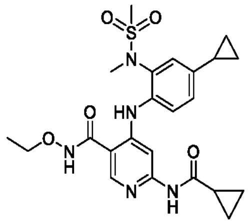 | 239 | 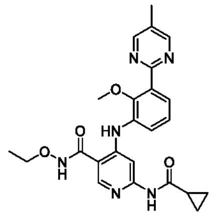 |
| 141 | 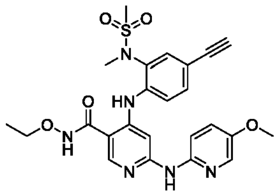 | 240 | 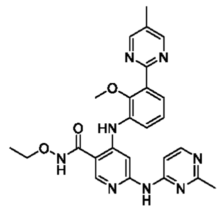 |
| 142 | 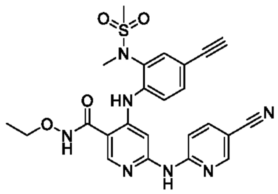 | 242 | 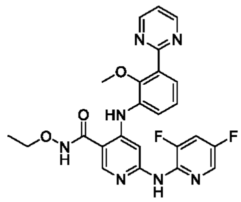 |
| 143 | 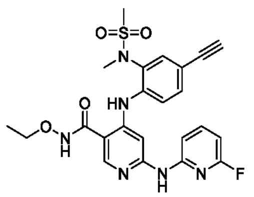 | 243 | 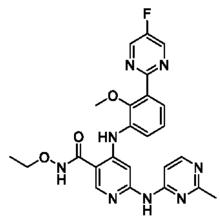 |
| 144 | 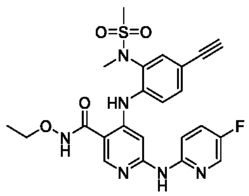 | 244 | 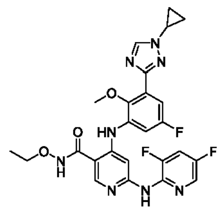 |
| 145 | 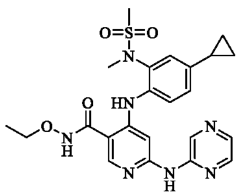 | 245 | 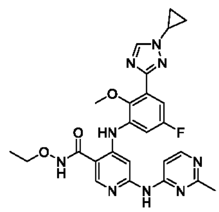 |

(continued)

| Compound # | Structure | Compound # | Structure |
|---|---|---|---|
| 146 | 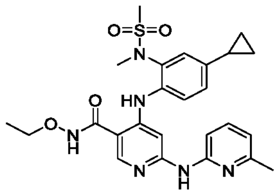 | 248 | 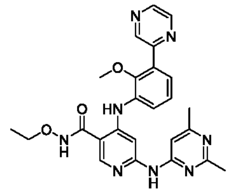 |
| 151 | 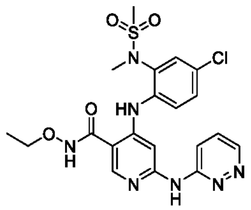 | 258 | 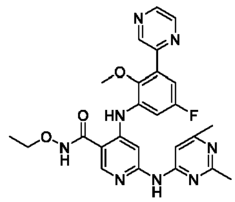 |
| 153 | 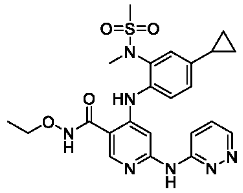 | 268 | 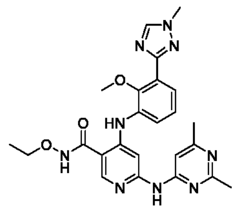 |
| 154 | 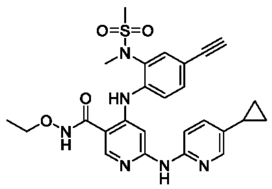 | 273 | 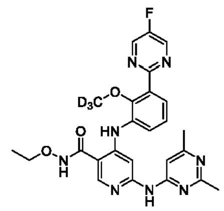 |
| 155 | 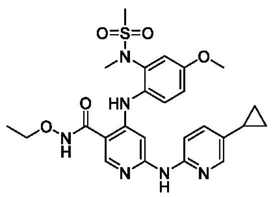 | 277 | 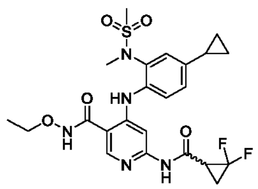 |
| 156 | 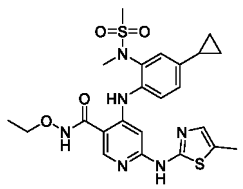 | 279 | 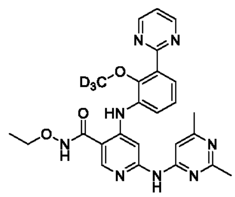 |

**14.** A pharmaceutical composition, comprising the compound or a pharmaceutically acceptable salt thereof as claimed in any one of claims 1-13, and optionally comprising, a pharmaceutical acceptable carrier and/or adjuvant and/or diluent.

**15.** The compound or a pharmaceutically acceptable salt thereof as claimed in any one of claims 1-13 or the pharmaceutical composition as claimed in claim 14, for use in treating and/or preventing a related disease mediated by TYK2; preferably, the related disease mediated by TYK2 includes inflammatory disease, autoimmune disease and cancer.

**Patentansprüche**

**1.** Verbindung, die durch Formel (I) dargestellt ist, oder ein pharmazeutisch akzeptables Salz davon:

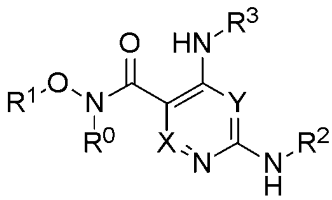

(I)

wobei:

X $CR^a$ ist;
Y CH ist;
$R^a$ Wasserstoff ist;
$R^0$ Wasserstoff ist;
$R^1$ ausgewählt ist aus der Gruppe bestehend aus $C_{1-6}$-Alkyl, optional substituiert durch eine bis sieben $R^{1a}$-Gruppen;
$R^{1a}$ ausgewählt ist aus der Gruppe bestehend aus Deuterium, F und Cl;
$R^2$ ausgewählt ist aus der Gruppe bestehend aus $C_{3-6}$ Cycloalkyl-C(O)-, $C_{3-6}$-Heterocyclyl-C(O)-, wobei das $C_{3-6}$-Heterocyclyl ein bis zwei Heteroatome enthält, die ausgewählt sind aus N, O und S, Phenyl und 5-6-gliedrigem Heteroaryl, das ein bis zwei Heteroatome enthält, die ausgewählt sind aus N, O und S, wobei das $C_{3-6}$-Cycloalkyl, das $C_{3-6}$ Heterocyclyl, das ein bis zwei Heteroatome enthält, die ausgewählt sind aus N, O und S, das Phenyl und das 5-6 gliedrige Heteroaryl, das ein bis zwei Heteroatome enthält, die ausgewählt sind aus N, O und S, jeweils und optional durch ein bis zwei $R^{2a}$-Gruppen substituiert sind;
$R^{2a}$ ausgewählt ist aus der Gruppe bestehend aus $C_{1-6}$-Alkyl, Halogen, $C_{1-6}$-Alkoxy, $C_{3-6}$-Cycloalkyl, Cyano, Halo-$C_{1-6}$-Alkyl, $C_{1-6}$-Alkyl, das durch eine Hydroxylgruppe substituiert ist, und $C_{1-6}$-Alkyl-$S(O)_2$-;
$R^3$ ausgewählt ist aus der Gruppe bestehend aus Phenyl, Pyridinyl, 2-Pyridinonyl und Indazolyl, wobei das Phenyl, das Pyridinyl, das 2-Pyridinonyl und das Indazolyl optional durch ein bis drei $R^{3a}$-Gruppen substituiert sind;
$R^{3a}$ ausgewählt ist aus der Gruppe bestehend aus F, Cl, Br, I, CN, $-NO_2$, $-OR^b$, $-SR^b$, $-C(O)OR^b$, $-C(O)NR^bR^c$, $-NR^bC(O)R^c$, $-NR^bC(O)OR^c$, $-NR^bC(O)NR^cR^c$, $-NR^bS(O)_2R^c$,$-S(O)_2R^c$, $-S(O)_2NR^cR^c$, $-P(O)R^bR^c$, $C_{1-6}$-Alkyl, das optional durch eine bis drei $R^d$-Gruppen substituiert ist, $C_{2-6}$-Alkenyl, $C_{2-6}$-Alkynyl, $C_{3-6}$-Cycloalkyl, 5-9-gliedriges Heteroaryl, das ein bis drei Heteroatome enthält, die ausgewählt sind aus N, O und S und optional durch eine bis drei $R^d$-Gruppen susbstituiert sind, und 5-6-gliedriges gesättigtes Heterocyclyl, das ein bis zwei Heteroatome enthält, die ausgewählt sind aus N, O und S;
$R^b$ ausgewählt ist aus der Gruppe bestehend aus Wasserstoff, $C_{1-6}$-Alkyl, das optional durch eine bis drei $R^d$-Gruppen substituiert ist, und 4-6-gliedriges gesättigtes Heterocyclyl, das ein bis zwei Sauerstoffatome enthält;
$R^c$ ausgewählt ist aus der Gruppe bestehend aus Wasserstoff, $C_{1-6}$-Alkyl und $C_{3-6}$-Cycloalkyl;
$R^d$ ausgewählt ist aus der Gruppe bestehend aus Wasserstoff, Deuterium, F, Cl, Br, I, $-OCF_3$, $-CF_3$, $-(CH_2)_r-CN$, $-NO_2$, $-OR^e$, $-(CH_2)_r-COR^c$, $-NR^eR^e$, $-NR^eC(O)OR^c$, $C_1$-$C_6$-Alkyl, $C_{3-6}$-Cycloalkyl und $-(CH_2)_r$-Phenyl;
$R^e$ Wasserstoff ist;
p 0, 1 oder 2 ist, und
r 0, 1, 2, 3 oder 4 ist.

**2.** Verbindung oder ein pharmazeutisch akzeptables Salz davon, wie in Anspruch 1 beansprucht, wobei:

X $CR^a$ ist;
$R^a$ Wasserstoff ist;
$R^0$ Wasserstoff ist;
$R^1$ ausgewählt ist aus der Gruppe bestehend aus Methyl, Ethyl, Isopropyl, t-Butyl, trideuteriertes Methyl und 2,2,2-Trifluorethyl;
$R^2$ ausgewählt ist aus der Gruppe bestehend aus Cyclopropylcarbonyl,

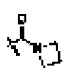,

Phenyl, Pyrazolyl, Thiazolyl, Pyridinyl, Pyrazinyl, Pyrimidinyl und Pyridazinyl, wobei das Cyclopropylcarbonyl,

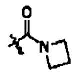,

das Phenyl, das Pyrazolyl, das Thiazolyl, das Pyridinyl, das Pyrazinyl, das Pyrimidinyl und das Pyridazinyl optional durch eine bis zwei $R^{2a}$-Gruppen substituiert sind;
$R^{2a}$ ausgewählt ist aus der Gruppe bestehend aus Methyl, F, Methoxy, Cyclopropyl, Cyano, $-CF_3$, Hydroxymethyl und Methansulfonyl;
$R^3$ ausgewählt ist aus der Gruppe aus Phenyl, Pyridinyl, 2-Pyridinonyl und Indazolyl, wobei das Phenyl, das Pyridinyl, das 2-Pyridinonyl und das Indazolyl optional durch ein bis drei $R^{3a}$-Gruppen substituiert sind;
$R^{3a}$ ausgewählt ist aus der Gruppe bestehend aus F, Cl, Br, I, CN, $-OR^b$, $-C(O)NR^bR^c$, $-NR^bS(O)_2R^c$, $-S(O)_2R^c$, $-S(O)_2NR^cR^c$, $-P(O)R^bR^c$, $C_{1-6}$-Alkyl, das optional durch eine bis drei $R^d$-Gruppen substituiert ist, $C_{2-6}$-Alkynyl, $C_{3-6}$-Cycloalkyl, 5-9-gliedriges Heteroaryl, das ein bis drei Heteroatome enthält, die ausgewählt sind aus N, O und S und optional durch eine bis drei $R^d$-Gruppen substituiert sind, und 5-6 gliedriges gesättigtes Heterocyclyl, das ein bis zwei Heteroatome enthält, die ausgewählt sind aus N, O und S;
$R^b$ ausgewählt ist aus der Gruppe bestehend aus Wasserstoff; Methyl, Ethyl und Isopropyl, wobei jedes optional durch eine bis drei $R^d$-Gruppen substituiert ist; und

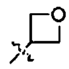;

$R^c$ ausgewählt ist aus der Gruppe bestehend aus Wasserstoff, Methyl und Cyclopropyl;
$R^d$ ausgewählt ist aus der Gruppe bestehend aus Deuterium, F, Cl, Hydroxyl, Methyl, Cyclopropyl und Isopropyl.

**3.** Verbindung oder ein pharmazeutisch akzeptables Salz davon, wie in Anspruch 1 oder 2 beansprucht, wobei:

X CH ist;
$R^1$ ausgewählt ist aus der Gruppe bestehend aus Methyl, Ethyl, Isopropyl, t-Butyl, trideuteriertes Methyl und 2,2,2-Trifluorethyl;
$R^{2a}$ ausgewählt ist aus der Gruppe bestehend aus Methyl, F, Methoxy, Cyclopropyl, Cyano, $-CF_3$, Hydroxymethyl und Methansulfonyl;
$R^{3a}$ ausgewählt ist aus der Gruppe bestehend aus F, Cl, Br, I, CN, $-OR^b$, $-C(O)NR^bR^c$, $-NR^bS(O)_2R^c$, $-S(O)_2R^c$, $-S(O)_2NR^cR^c$, $-P(O)R^bR^c$, $C_{1-3}$-Alkyl, das optional durch eine bis drei $R^d$-Gruppen substituiert ist, $C_{2-4}$-Alkynyl, $C_{3-5}$-Cycloalkyl, 5-9-gliedriges Heteroaryl, das ein bis drei Heteroatome enthält, die ausgewählt sind aus N, O und S und optional durch eine $R^d$-Gruppe substituiert sind, und 5-6-gliedriges gesättigtes Heterocyclyl, das ein bis zwei Heteroatome enthält, die ausgewählt sind aus N, O und S, wobei das 5-9-gliedrige Heteroaryl, das ein bis drei Heteroatome enthält, die ausgewählt sind aus N, O und S, ausgewählt ist aus der Gruppe bestehend aus Thiazolyl, Benzimidazolyl, Pyrazolyl, Oxadiazolyl, Triazolyl, Pyridinyl, Pyrimidinyl und Pyrazinyl, wobei das 5-6-gliedrige gesättigte Heterocyclyl, das ein bis zwei Heteroatome enthält, die ausgewählt sind aus N, O und S, ausgewählt ist aus der Gruppe bestehend aus Morpholinyl und Piperidinyl;
$R^b$ ausgewählt ist aus der Gruppe bestehend aus Wasserstoff; Methyl, Ethyl und Isopropyl, wobei jedes optional durch eine bis drei $R^d$-Gruppen substituiert ist; und

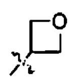;

$R^c$ ausgewählt ist aus der Gruppe bestehend aus Wasserstoff, Methyl und Cyclopropyl;
$R^d$ ausgewählt ist aus der Gruppe bestehend aus Deuterium, F, Cl, Hydroxyl, Methyl, Cyclopropyl und Isopropyl.

**4.** Verbindung oder ein pharmazeutisch akzeptables Salz davon, wie in Anspruch 1 oder 2 beansprucht, wobei:
$R^2$ ausgewählt ist aus der Gruppe bestehend aus

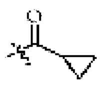, 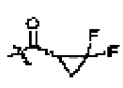, 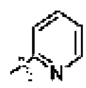, 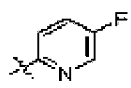,

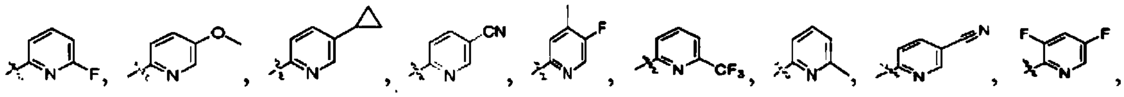

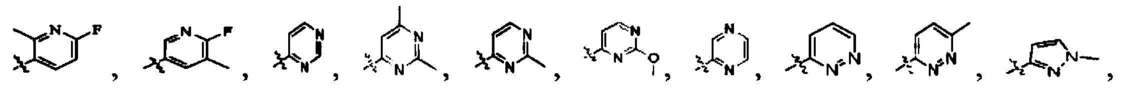

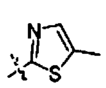 und 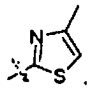.

**5.** Verbindung oder ein pharmazeutisch akzeptables Salz davon, wie in Anspruch 1 oder 2 beansprucht, wobei: $R^3$ ausgewählt ist aus der Gruppe bestehend aus:

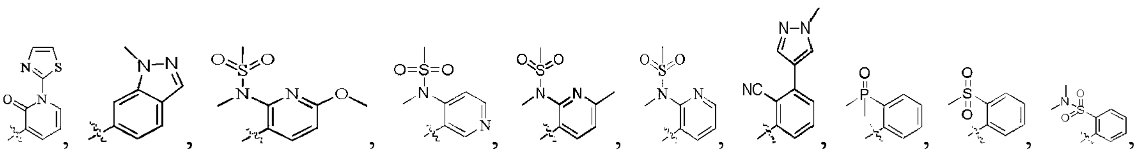

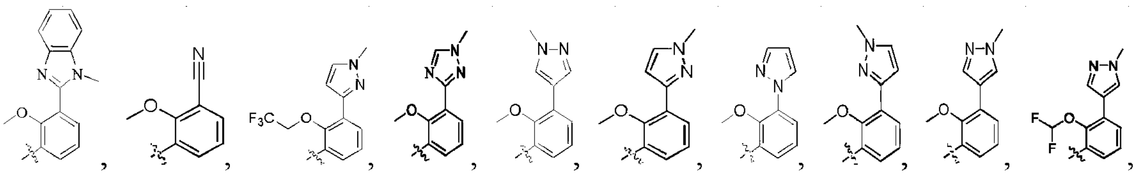

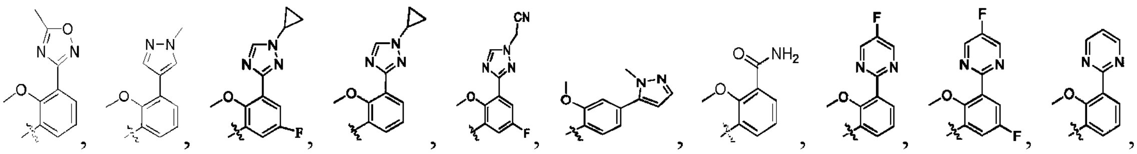

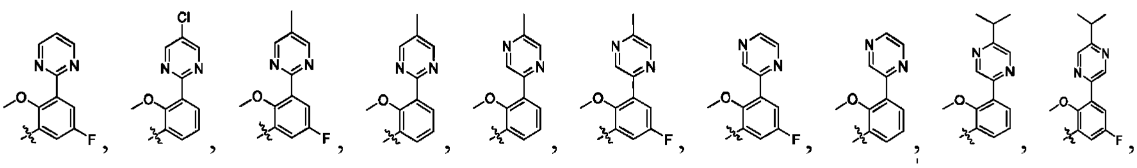

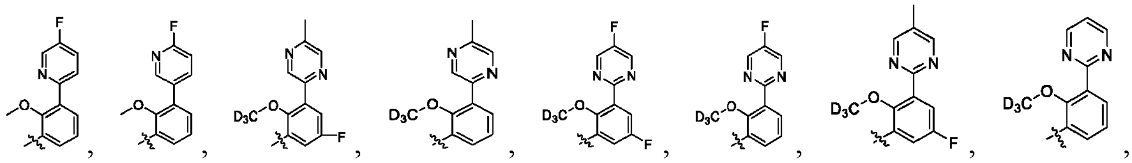

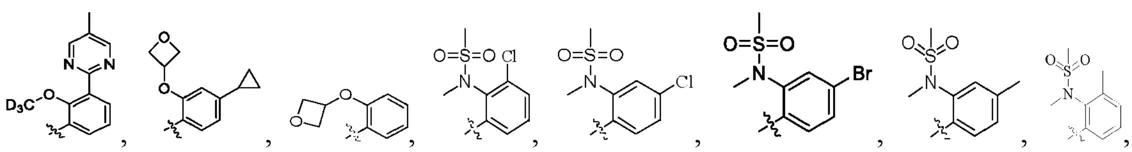

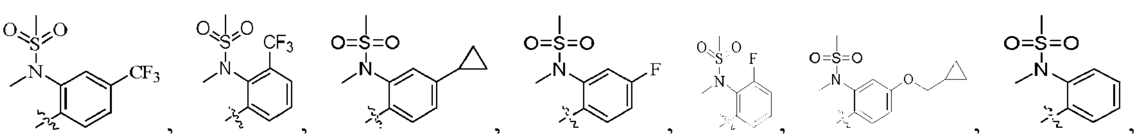

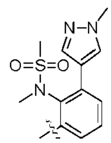, 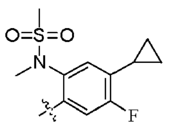, 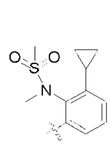, 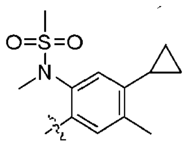, 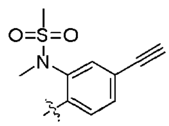, 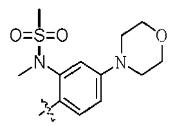,

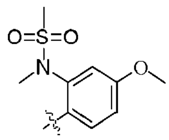, 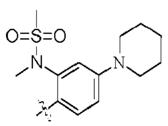, 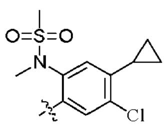, 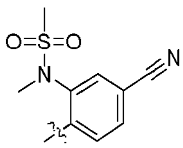, 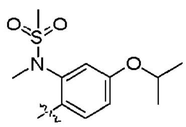, 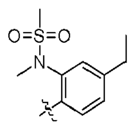,

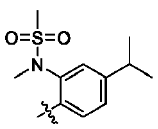, 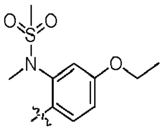, 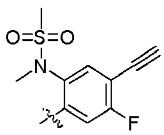, 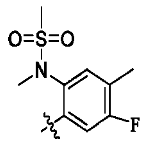, 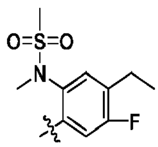, 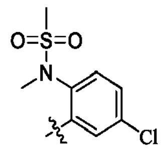,

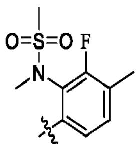, 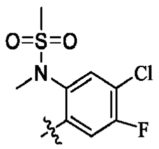, 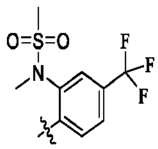, 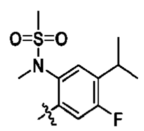, 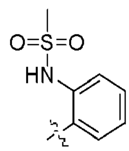, 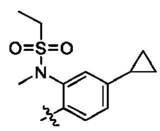,

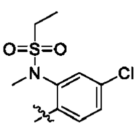, 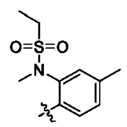, 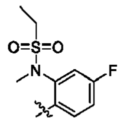, 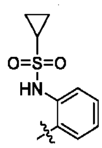, 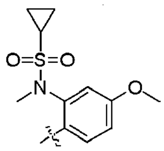, 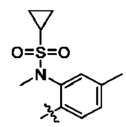, 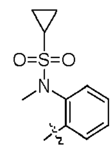,

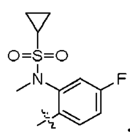, 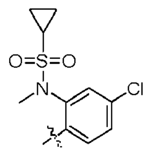, 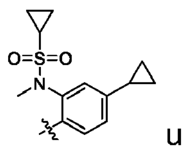 und 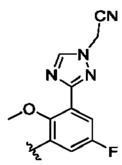.

**6.** Verbindung oder ein pharmazeutisch akzeptables Salz davon, wie in Anspruch 1 beansprucht, wobei:

X CH ist;
Y CH ist;
$R^0$ Wasserstoff ist;
$R^1$ Ethyl ist;
$R^2$ ausgewählt ist aus der Gruppe bestehend aus Pyridinyl, Pyrimidinyl, -C(O)-$C_{3-6}$-Cycloalkyl und -C(O)-$C_{3-6}$-Heterocyclyl, wobei das $C_{3-6}$-Heterocyclyl ein bis zwei Heteroatome enthält, die ausgewählt sind aus N, O und S; wobei das $C_{3-6}$-Cycloalkyl und das $C_{3-6}$-Heterocyclyl jeweils und optional substituiert sind durch ein bis drei $R^{2a}$-Gruppen;
$R^{2a}$ ausgewählt ist aus der Gruppe bestehend aus $C_{1-6}$-Alkyl, Halogen, $C_{1-6}$-Alkoxy, $C_{3-6}$-Cycloalkyl, Cyano, Halo-$C_{1-6}$-Alkyl, $C_{1-6}$-Alkyl, das durch eine Hydroxylgruppe substituiert ist, und $C_{1-6}$-Alkyl-$S(O)_2$-;
$R^3$ Phenyl ist, das optional durch eine bis drei $R^{3a}$-Gruppen substituiert ist;
$R^{3a}$ ausgewählt ist aus der Gruppe bestehend aus $-(CH_2)_r-OR^b$, $-NR^bS(O)_pR^c$, F, Cl, $C_{1-6}$-Alkyl, $C_{3-6}$-Cycloalkyl, Triazolyl, Pyrazolyl, Pyrimidinyl und Pyrazinyl; wobei das $C_{1-6}$-Alkyl, das $C_{3-6}$-Cycloalkyl, das Triazolyl, das Pyrazolyl, das Pyrimidinyl und das Pyrazinyl jeweils und unabhängig optional durch eine bis drei $R^d$-Gruppen substituiert sind;
$R^b$ ausgewählt ist aus der Gruppe bestehend aus Wasserstoff, $C_{1-6}$-Alkyl, das optional durch eine bis drei $R^d$-Gruppen substituiert ist, und 4-6-gliedriges gesättigtes Heterocyclyl, das ein bis zwei Sauerstoffatome enthält;

$R^c$ ausgewählt ist aus der Gruppe bestehend aus Wasserstoff, $C_{1-6}$-Alkyl und $C_{3-6}$-Cycloalkyl;
$R^d$ ausgewählt ist aus der Gruppe bestehend aus Wasserstoff, Deuterium, F, Cl, Br, I, $OCF_3$, $CF_3$, $-(CH_2)_r$-CN, $NO_2$, $OR^e$, $-(CH_2)_r$-$COR^c$, $-NR^eR^e$, $-NR^eC(O)OR^c$, $C_1$-$C_6$-Alkyl, $C_{3-6}$-Cycloalkyl und $-(CH_2)_r$-Phenyl;
$R^e$ Wasserstoff ist;
p 0, 1 oder 2 ist, und
r 0, 1, 2, 3 oder 4 ist.

**7.** Verbindung oder ein pharmazeutisch akzeptables Salz davon, wie in Anspruch 6 beansprucht, wobei $R^{2a}$ ausgewählt ist aus der Gruppe bestehend aus Methyl, F, Methoxy, Cyclopropyl, Cyano, $-CF_3$, Hydroxymethyl und Methansulfonyl.

**8.** Verbindung oder ein pharmazeutisch akzeptables Salz davon, wie in Anspruch 6 beansprucht, wobei $R^2$ aus den folgenden Strukturen ausgewählt ist:

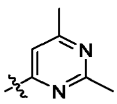, 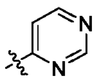, 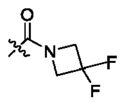, 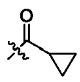, 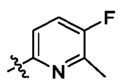, 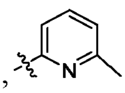, 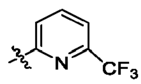, 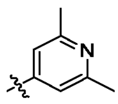,

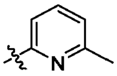, 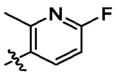, 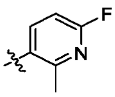, 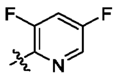, 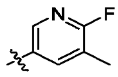, 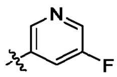, 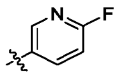, 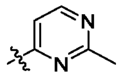,

und

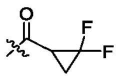.

**9.** Verbindung oder ein pharmazeutisch akzeptables Salz davon, wie in Anspruch 6 beansprucht, wobei $R^b$ ausgewählt ist aus der Gruppe bestehend aus Wasserstoff; Methyl, Ethyl und Isopropyl, die jeweils optional durch eine bis drei $R^d$-Gruppen substituiert sind; und

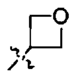.

**10.** Verbindung oder ein pharmazeutisch akzeptables Salz davon, wie in Anspruch 6 beansprucht, wobei $R^c$ ausgewählt ist aus der Gruppe bestehend aus Wasserstoff, Methyl, Ethyl und Cyclopropyl.

**11.** Verbindung oder ein pharmazeutisch akzeptables Salz davon, wie in Anspruch 6 beansprucht, wobei $R^d$ ausgewählt ist aus der Gruppe bestehend aus F, Cl, Hydroxyl, Methyl, Cyclopropyl und $-CH_2CN$.

**12.** Verbindung oder ein pharmazeutisch akzeptables Salz davon, wie in Anspruch 6 beansprucht, wobei $R^3$ aus den folgenden Strukturen ausgewählt ist:

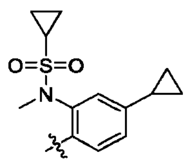, 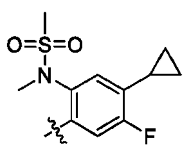, 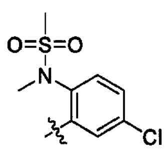, 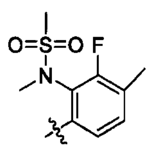, 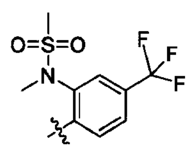, 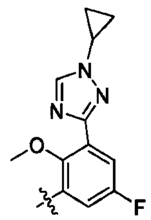,

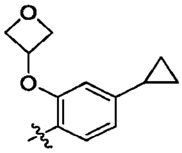, 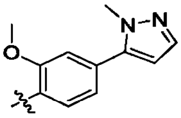, 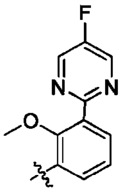, 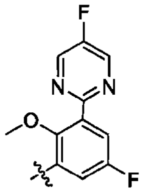, 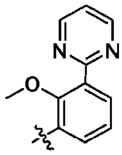, 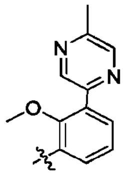, 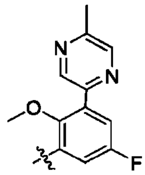,

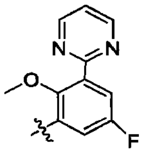, 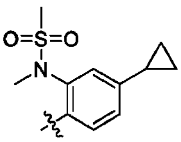, 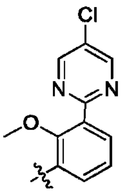, 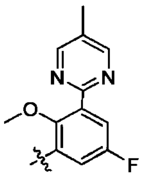, 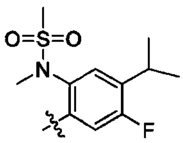, 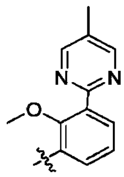, 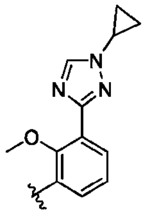,

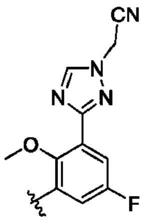, 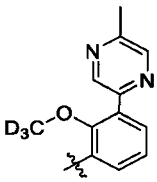, 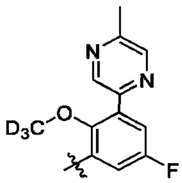, 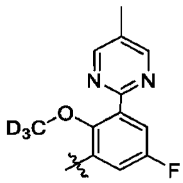, 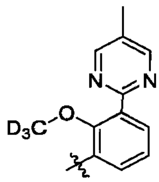, 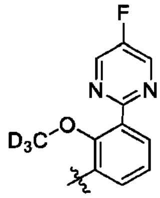,

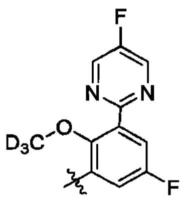, 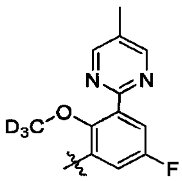, und 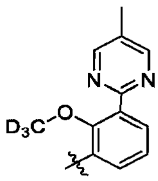.

13. Verbindung oder ein pharmazeutisch akzeptables Salz davon, wie in Anspruch 1 beansprucht, wobei die Verbindung ausgewählt ist aus der Gruppe bestehend aus:

| Verbindung # | Struktur | Verbindung # | Struktur |
|---|---|---|---|
| 2 | 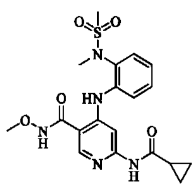 | 157 | 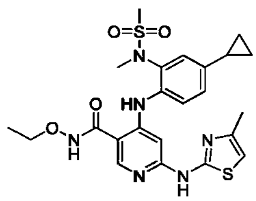 |
| 4 | 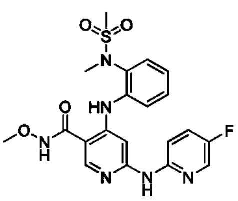 | 159 | 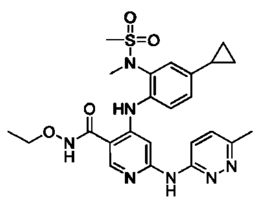 |

(continued)

| Verbindung # | Struktur | Verbindung # | Struktur |
|---|---|---|---|
| 10 | 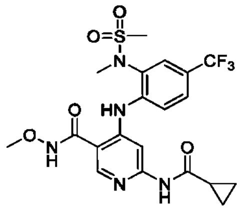 | 160 | 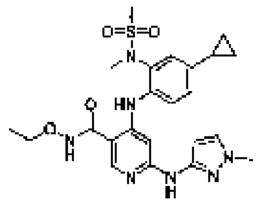 |
| 12 | 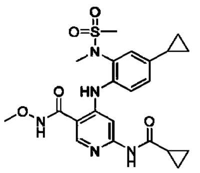 | 162 | 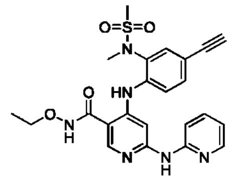 |
| 13 | 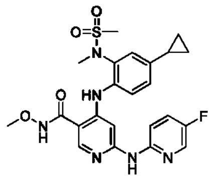 | 166 | 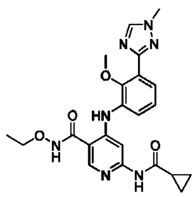 |
| 14 | 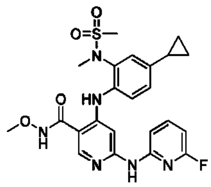 | 167 | 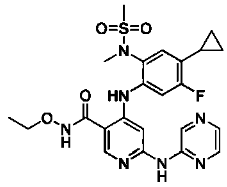 |
| 16 | 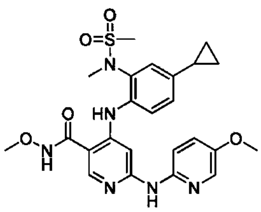 | 170 | 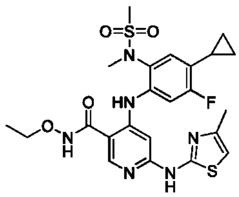 |
| 18 | 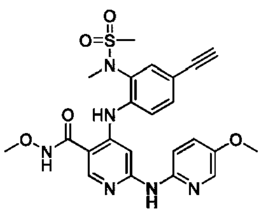 | 171 | 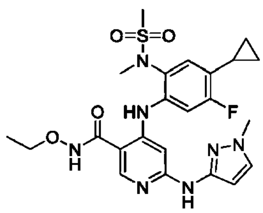 |
| 19 | 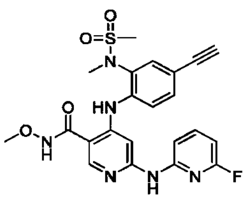 | 173 | 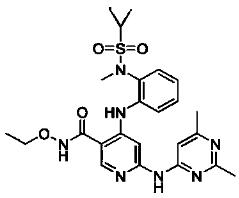 |

(continued)

| Verbindung # | Struktur | Verbindung # | Struktur |
|---|---|---|---|
| 20 | 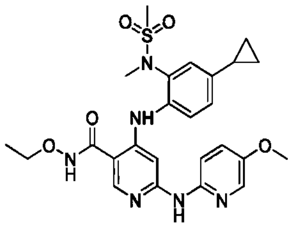 | 174 | 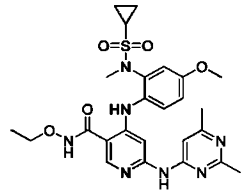 |
| 21 | 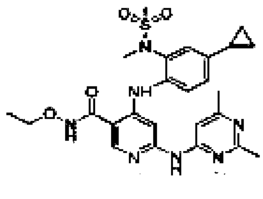 | 175 | 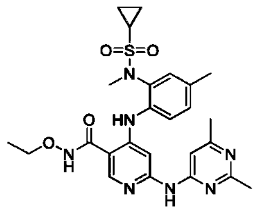 |
| 22 | 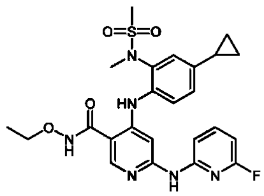 | 176 | 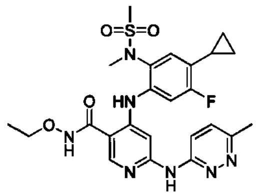 |
| 29 | 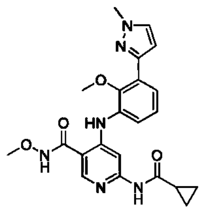 | 177 | 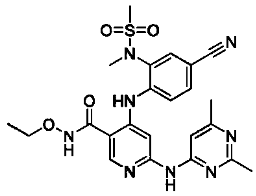 |
| 36 | 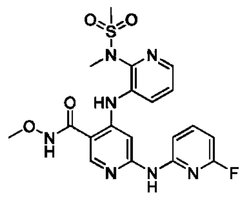 | 178 | 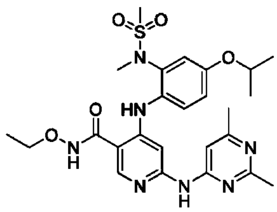 |
| 38 | 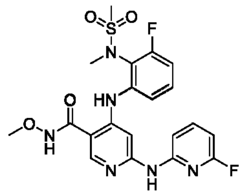 | 179 | 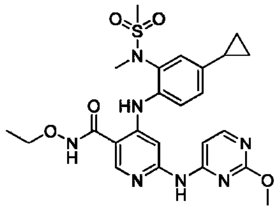 |
| 41 | 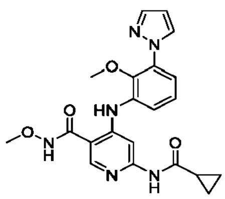 | 180 | 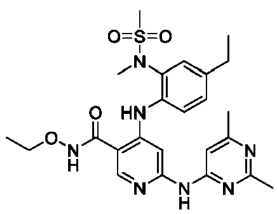 |

(continued)

| Verbindung # | Struktur | Verbindung # | Struktur |
|---|---|---|---|
| 42 | 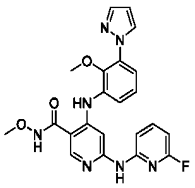 | 181 | 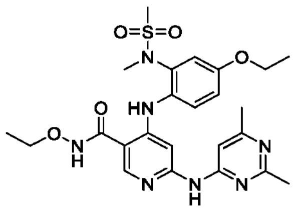 |
| 43 | 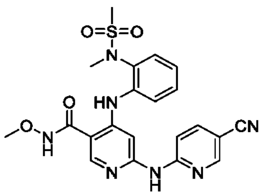 | 182 | 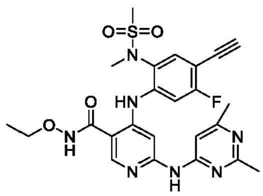 |
| 44 | 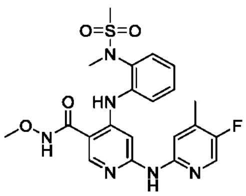 | 183 | 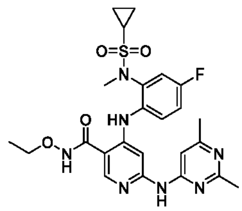 |
| 45 | 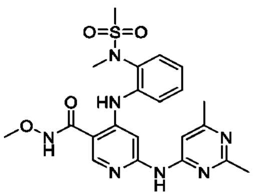 | 185 | 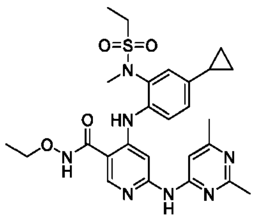 |
| 46 | 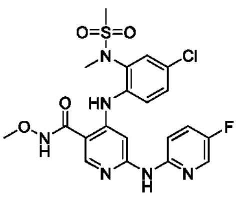 | 186 | 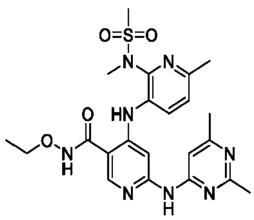 |
| 47 | 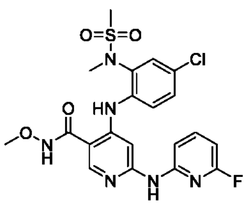 | 187 | 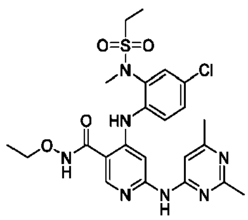 |
| 60 | 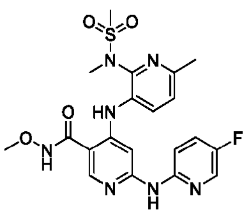 | 188 | 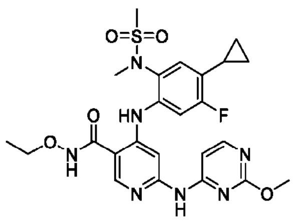 |

(continued)

| Verbindung # | Struktur | Verbindung # | Struktur |
|---|---|---|---|
| 61 | 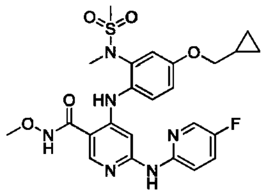 | 189 | 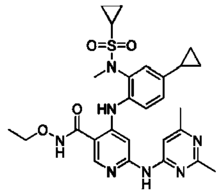 |
| 62 | 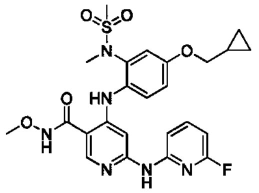 | 190 | 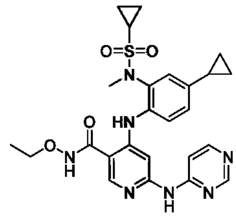 |
| 63 | 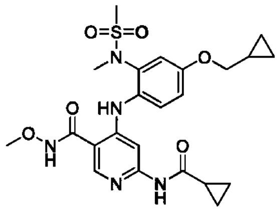 | 197 | 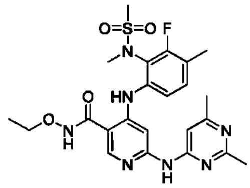 |
| 68 | 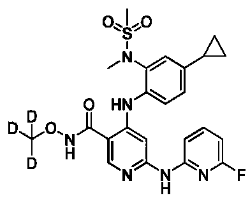 | 198 | 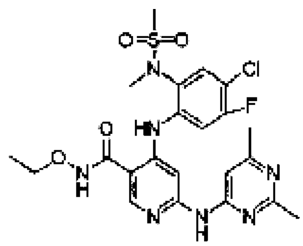 |
| 69 | 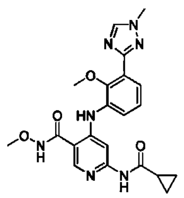 | 200 | 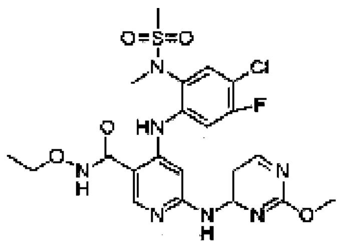 |
| 70 | 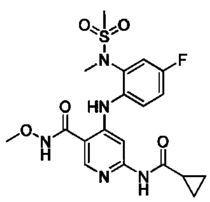 | 202 | 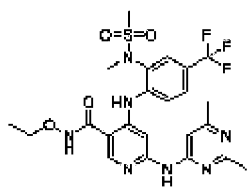 |
| 79 | 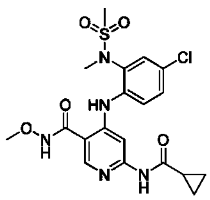 | 204 | 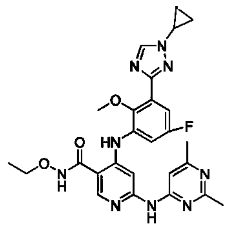 |

(continued)

| Verbindung # | Struktur | Verbindung # | Struktur |
|---|---|---|---|
| 80 | 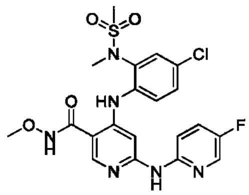 | 194 | 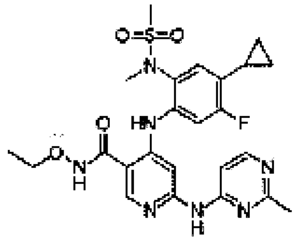 |
| 81 | 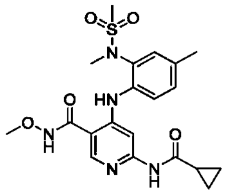 | 192 | 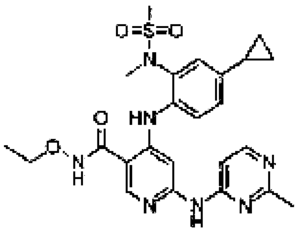 |
| 90 | 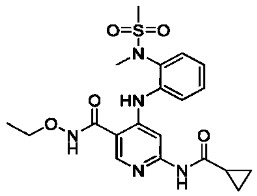 | 193 | 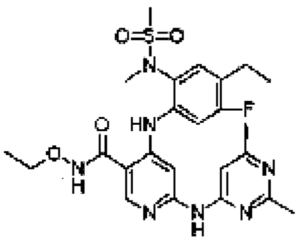 |
| 95 | 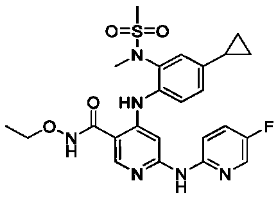 | 203 | 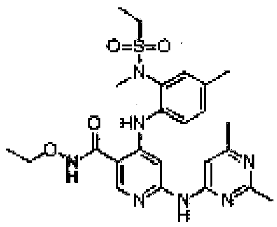 |
| 97 | 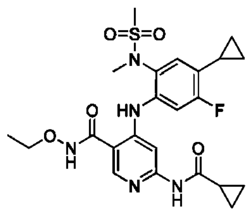 | 230 | 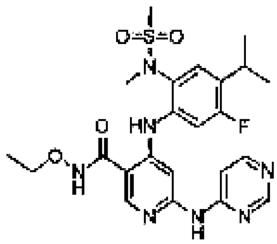 |
| 98 | 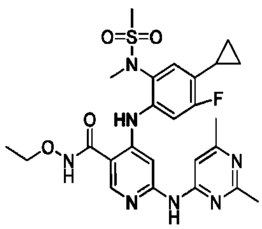 | 229 | 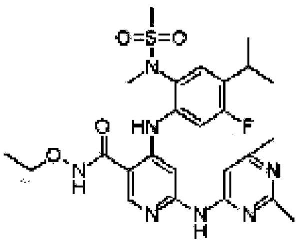 |

(continued)

| Verbindung # | Struktur | Verbindung # | Struktur |
|---|---|---|---|
| 101 | 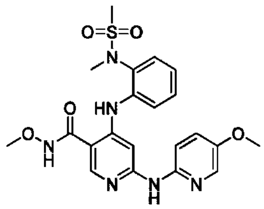 | 201 | 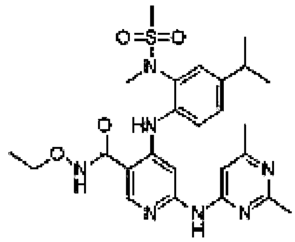 |
| 102 | 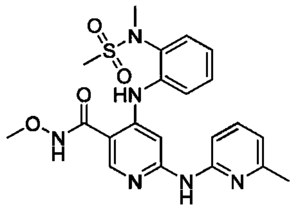 | 191 | 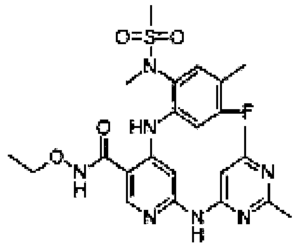 |
| 106 | 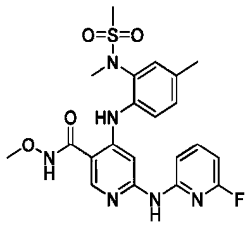 | 205 | 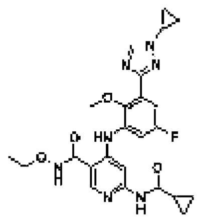 |
| 108 | 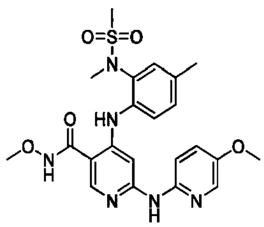 | 207 | 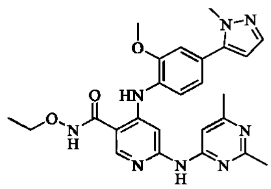 |
| 109 | 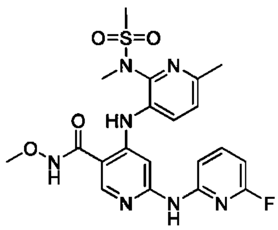 | 208 | 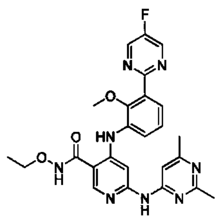 |
| 110 | 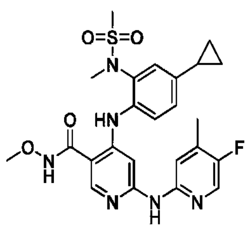 | 209 | 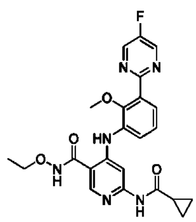 |

(continued)

| Verbindung # | Struktur | Verbindung # | Struktur |
|---|---|---|---|
| 111 | 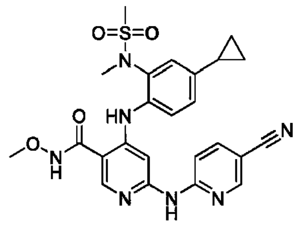 | 210 | 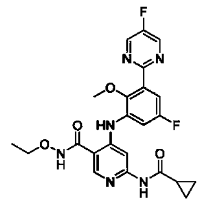 |
| 112 | 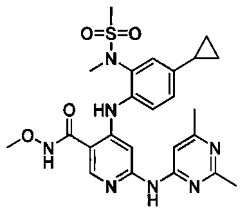 | 211 | 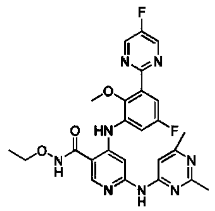 |
| 113 | 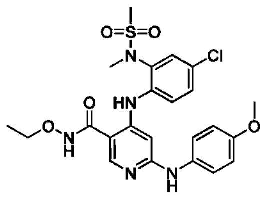 | 212 | 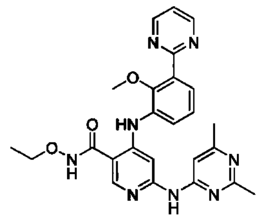 |
| 114 | 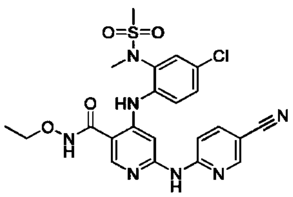 | 213 | 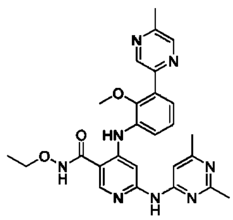 |
| 115 | 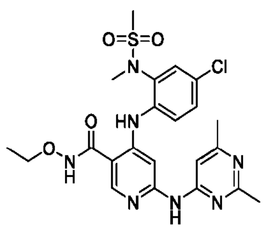 | 214 | 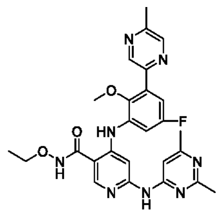 |
| 116 | 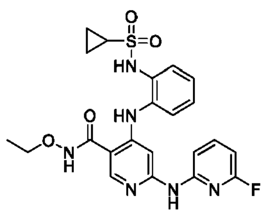 | 215 | 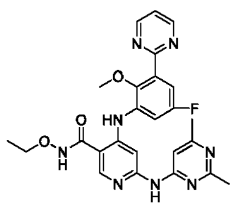 |
| 118 | 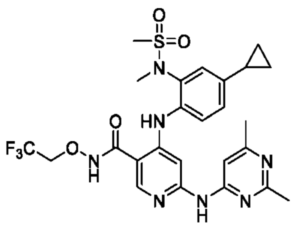 | 217 | 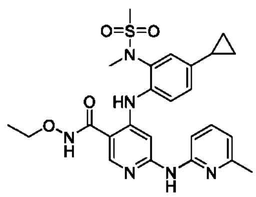 |

(continued)

| Verbindung # | Struktur | Verbindung # | Struktur |
|---|---|---|---|
| 119 | 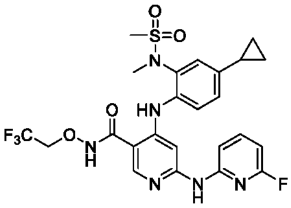 | 218 | 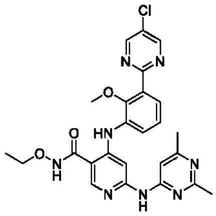 |
| 125 | 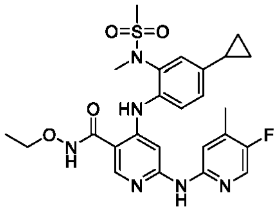 | 219 | 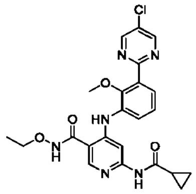 |
| 126 | 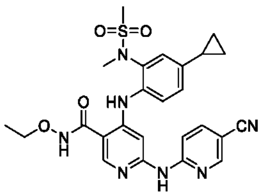 | 220 | 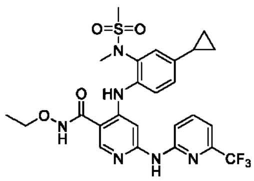 |
| 127 | 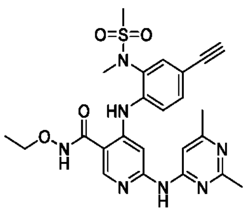 | 222 | 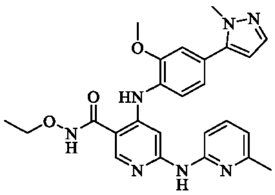 |
| 128 | 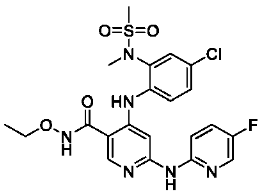 | 223 | 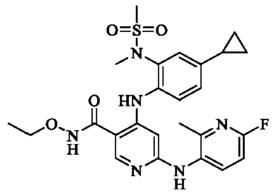 |
| 129 | 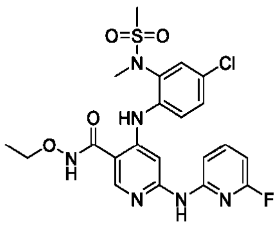 | 224 | 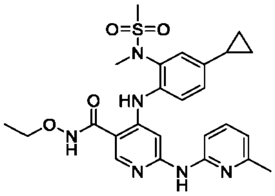 |
| 130 | 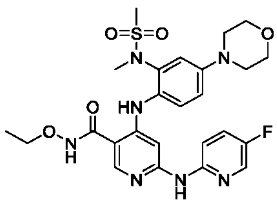 | 225 | 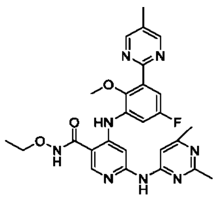 |

(continued)

| Verbindung # | Struktur | Verbindung # | Struktur |
|---|---|---|---|
| 131 | 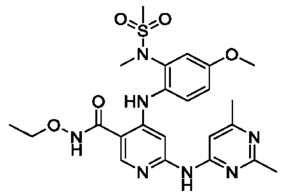 | 226 | 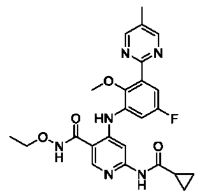 |
| 132 | 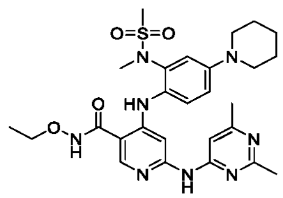 | 234 | 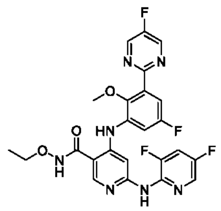 |
| 133 | 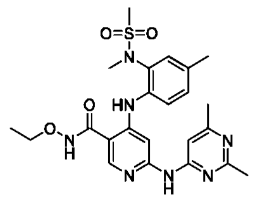 | 235 | 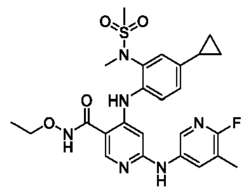 |
| 134 | 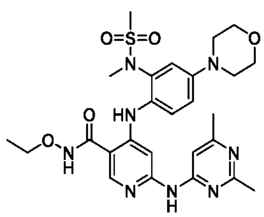 | 236 | 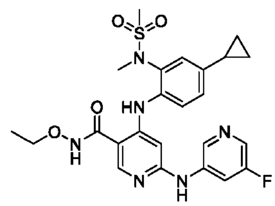 |
| 136 | 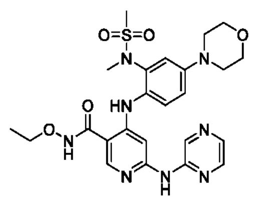 | 237 | 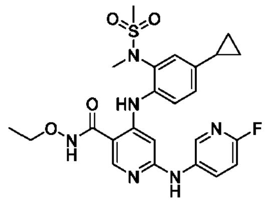 |
| 138 | 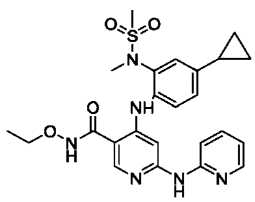 | 238 | 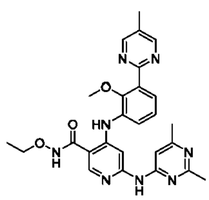 |
| 139 | 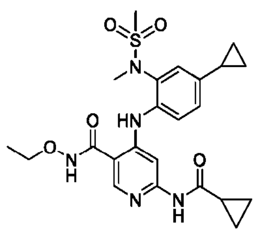 | 239 | 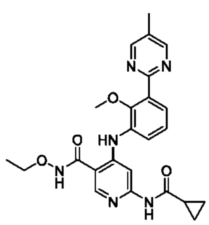 |

(continued)

| Verbindung # | Struktur | Verbindung # | Struktur |
|---|---|---|---|
| 141 | 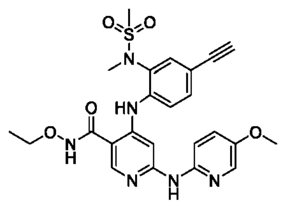 | 240 | 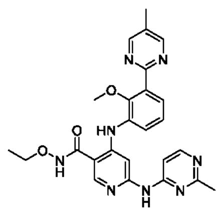 |
| 142 | 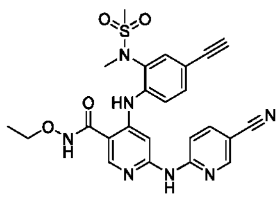 | 242 | 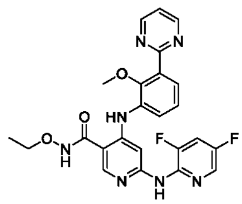 |
| 143 | 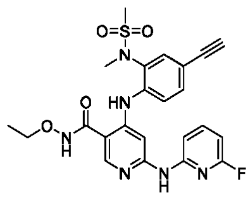 | 243 | 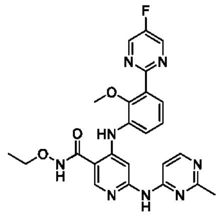 |
| 144 | 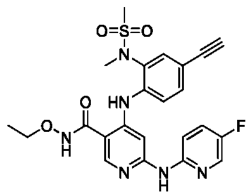 | 244 | 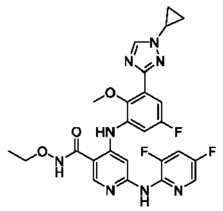 |
| 145 | 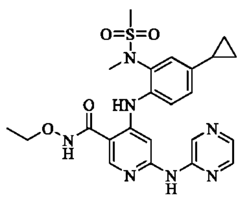 | 245 | 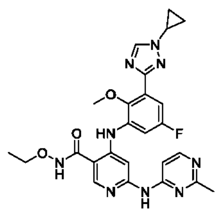 |
| 146 | 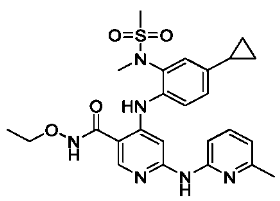 | 248 | 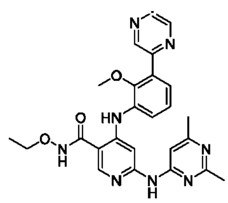 |
| 151 | 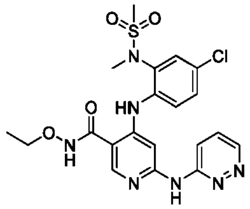 | 258 | 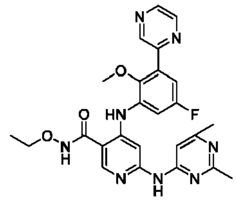 |

(continued)

| Verbindung # | Struktur | Verbindung # | Struktur |
|---|---|---|---|
| 153 | 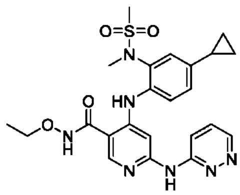 | 268 | 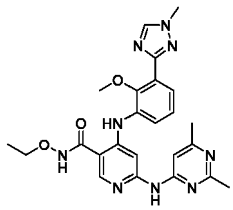 |
| 154 | 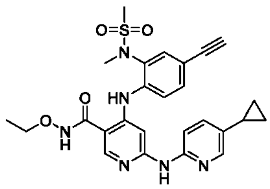 | 273 | 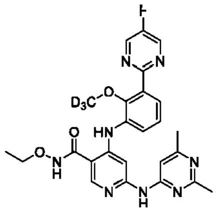 |
| 155 | 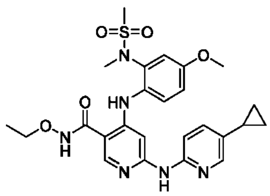 | 277 | 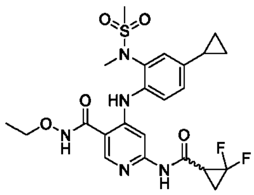 |
| 156 | 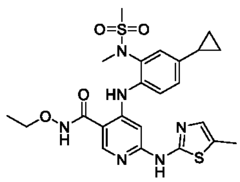 | 279 | 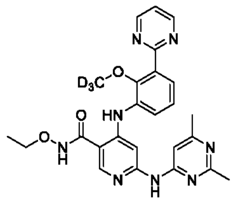 |

**14.** Pharmazeutische Zusammensetzung, umfassend eine Verbindung oder ein pharmazeutisch akzeptables Salz davon, wie in einem der Ansprüche 1 bis 13 beansprucht, und optional umfassend einen pharmazeutisch akzeptablen Träger und/oder Hilfsstoff und/oder Verdünner.

**15.** Verbindung oder ein pharmazeutisch akzeptables Salz davon wie in einem der Ansprüche 1 bis 13 beansprucht oder die pharmazeutische Zusammensetzung wie in Anspruch 14 beansprucht, zur Verwendung beim Behandeln und/oder Verhindern einer verwandten durch TYK2 vermittelten Erkrankung;
wobei bevorzugt die verwandte durch TYK2 vermittelte Erkrankung eine entzündliche Erkrankung, eine Autoimmunerkrankung und Krebs beinhaltet.

## Revendications

**1.** Composé représenté par la formule (I) ou un sel pharmaceutiquement acceptable de celui-ci :

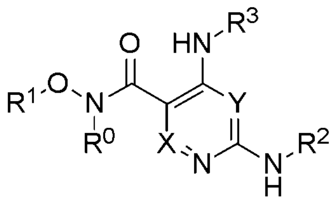

(I)

dans lequel :

X est $CR^a$ ;
Y est CH;
$R^a$ est hydrogène ;
$R^0$ est hydrogène ;
$R^1$ est choisi dans le groupe consistant en alkyle en $C_{1-6}$ éventuellement substitué par de un à sept groupes $R^{1a}$ ;
$R^{1a}$ est choisi dans le groupe consistant en deutérium, F et Cl ;
$R^2$ est choisi dans le groupe consistant en cycloalkyle en $C_{3-6}$-C(O)-, hétérocyclyle en $C_{3-6}$-C(O)- dans lequel l'hétérocyclyle en $C_{3-6}$ contient de un à deux hétéroatomes choisis parmi N, O et S, phényle, et hétéroaryle à 5-6 chaînons contenant de un à deux hétéroatomes choisis parmi N, O et S, dans lequel les cycloalkyle en $C_{3-6}$, hétérocyclyle en $C_{3-6}$ contenant de un à deux hétéroatomes choisis parmi N, O et S, phényle et hétéroaryle à 5-6 chaînons contenant de un à deux hétéroatomes choisis parmi N, O et S sont respectivement et éventuellement substitués par de un à deux groupes $R^{2a}$ ;
$R^{2a}$ est choisi dans le groupe consistant en alkyle en $C_{1-6}$, halogène, alcoxy en $C_{1-6}$, cycloalkyle en $C_{3-6}$, cyano, halo alkyle en $C_{1-6}$, alkyle en $C_{1-6}$ substitué par un groupe hydroxyle et alkyle en $C_{1-6}$-$S(O)_2$- ;
$R^3$ est choisi dans le groupe consistant en phényle, pyridinyle, 2-pyridinonyle et indazolyle, dans lequel les phényle, pyridinyle, 2-pyridinonyle et indazolyle sont éventuellement substitués par de un à trois groupes $R^{3a}$ ;
$R^{3a}$ est choisi dans le groupe consistant en F, Cl, Br, I, CN, $-NO_2$, $-OR^b$, $-SR^b$,$-C(O)OR^b$, $-C(O)NR^bR^c$, $-NR^bC(O)R^c$, $-NR^bC(O)OR^c$, $-NR^bC(O)NR^cR^c$, $-NR^bS(O)_2R^c$,$-S(O)_2R^c$, $-S(O)_2NR^cR^c$, $-P(O)R^bR^c$, alkyle en $C_{1-6}$ éventuellement substitué par de un à trois groupes $R^d$, alcényle en $C_{2-6}$, alcynyle en $C_{2-6}$, cycloalkyle en $C_{3-6}$, hétéroaryle à 5-9 chaînons contenant de un à trois hétéroatomes choisis parmi N, O et S et éventuellement substitué par de un à trois groupes $R^d$, et hétérocyclyle saturé à 5-6 chaînons contenant de un à deux hétéroatomes choisis parmi N, O et S ;
$R^b$ est choisi dans le groupe consistant en hydrogène, alkyle en $C_{1-6}$ éventuellement substitué par de un à trois groupes $R^d$, et hétérocyclyle saturé à 4-6 chaînons contenant de un à deux atomes d'oxygène ;
$R^c$ est choisi dans le groupe consistant en hydrogène, alkyle en $C_{1-6}$ et cycloalkyle en C3-6 ;
$R^d$ est choisi dans le groupe consistant en hydrogène, deutérium, F, Cl, Br, I, $-OCF_3$, $-CF_3$, $-(CH_2)_r$-CN, $-NO_2$, $-OR^e$, $-(CH_2)_r-COR^c$, $-NR^eR^e$, $-NR^eC(O)OR^c$, alkyle en $C_1$-$C_6$, cycloalkyle en $C_{3-6}$ et $-(CH_2)_r$-phényle ;
$R^e$ est hydrogène ;
p est 0, 1 ou 2, et
r est 0, 1, 2, 3 ou 4.

**2.** Composé ou un sel pharmaceutiquement acceptable de celui-ci selon la revendication 1, dans lequel :

X est $CR^a$ ;
$R^a$ est hydrogène ;
$R^0$ est hydrogène ;
$R^1$ est choisi dans le groupe consistant en méthyle, éthyle, isopropyle, t-butyle, méthyle tridéutéré et 2,2,2-trifluoroéthyle ;
$R^2$ est choisi dans le groupe consistant en cyclopropylcarbonyle,

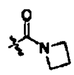,

phényle, pyrazolyle, thiazolyle, pyridinyle, pyrazinyle, pyrimidinyle et pyridazinyle, dans lequel les cyclopropylcarbonyle,

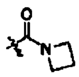,

phényle, pyrazolyle, thiazolyle, pyridinyle, pyrazinyle, pyrimidinyle et pyridazinyle sont éventuellement substitués par de un à deux groupes $R^{2a}$ ;
$R^{2a}$ est choisi dans le groupe consistant en méthyle, F, méthoxy, cyclopropyle, cyano, $-CF_3$, hydroxyméthyle et méthanesulfonyle ;
$R^3$ est choisi dans le groupe consistant en phényle, pyridinyle, 2-pyridinonyle et indazolyle, dans lequel les phényle, pyridinyle, 2-pyridinonyle et indazolyle sont éventuellement substitués par de un à trois groupes $R^{3a}$ ;
$R^{3a}$ est choisi dans le groupe consistant en F, Cl, Br, I, CN, $-OR^b$, $-C(O)NR^bR^c$,$-NR^bS(O)_2R^c$, $-S(O)_2R^c$,

$-S(O)_2NR^cR^c$, $-P(O)R^bR^c$, alkyle en $C_{1-6}$ éventuellement substitué par de un à trois groupes $R^d$, alcynyle en $C_{2-6}$, cycloalkyle en $C_{3-6}$, hétéroaryle à 5-9 chaînons contenant de un à trois hétéroatomes choisis parmi N, O et S et éventuellement substitué par de un à trois groupes $R^d$, et hétérocyclyle saturé à 5-6 chaînons contenant de un à deux hétéroatomes choisis parmi N, O et S ;

$R^b$ est choisi dans le groupe consistant en hydrogène ; méthyle, éthyle et isopropyle, chacun étant éventuellement substitué par de un à trois groupes $R^d$ ; et

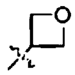 ;

$R^c$ est choisi dans le groupe consistant en hydrogène, méthyle et cyclopropyle ;

$R^d$ est choisi dans le groupe consistant en deutérium, F, Cl, hydroxyle, méthyle, cyclopropyle et isopropyle.

**3.** Composé ou un sel pharmaceutiquement acceptable de celui-ci selon la revendication 1 ou 2, dans lequel :

X est CH ;

$R^1$ est choisi dans le groupe consistant en méthyle, éthyle, isopropyle, t-butyle, méthyle tridéutéré et 2,2,2-trifluoroéthyle ;

$R^{2a}$ est choisi dans le groupe consistant en méthyle, F, méthoxy, cyclopropyle, cyano, $-CF_3$, hydroxyméthyle et méthanesulfonyle ;

$R^{3a}$ est choisi dans le groupe consistant en F, Cl, Br, I, CN, $-OR^b$, $-C(O)NR^bR^c$,$-NR^bS(O)_2R^c$, $-S(O)_2R^c$, $-S(O)_2NR^cR^c$, $-P(O)R^bR^c$, alkyle en $C_{1-3}$ éventuellement substitué par de un à trois groupes $R^d$, alcynyle en $C_{2-4}$, cycloalkyle en $C_{3-5}$, hétéroaryle à 5-9 chaînons contenant de un à trois hétéroatomes choisis parmi N, O et S et éventuellement substitué par un groupe $R^d$, et hétérocyclyle saturé à 5-6 chaînons contenant de un à deux hétéroatomes choisis parmi N, O et S, dans lequel l'hétéroaryle à 5-9 chaînons contenant de un à trois hétéroatomes choisis parmi N, O et S est choisi dans le groupe consistant en thiazolyle, benzimidazolyle, pyrazolyle, oxadiazolyle, triazolyle, pyridinyle, pyrimidinyle et pyrazinyle, dans lequel l'hétérocyclyle saturé à 5-6 chaînons contenant de un à deux hétéroatomes choisis parmi N, O et S est choisi dans le groupe consistant en morpholinyle et pipéridinyle ;

$R^b$ est choisi dans le groupe consistant en hydrogène ; méthyle, éthyle et isopropyle, chacun étant éventuellement substitué par de un à trois groupes $R^d$ ; et

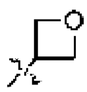 ;

$R^c$ est choisi dans le groupe consistant en hydrogène, méthyle et cyclopropyle ;

$R^d$ est choisi dans le groupe consistant en deutérium, F, Cl, hydroxyle, méthyle, cyclopropyle et isopropyle.

**4.** Composé ou un sel pharmaceutiquement acceptable de celui-ci selon la revendication 1 ou 2, dans lequel : $R^2$ est choisi dans le groupe consistant en

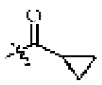, 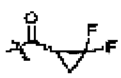, 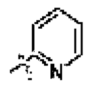, 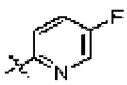,

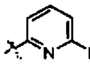, 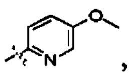, 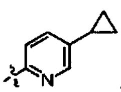, 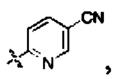, 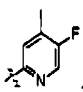, 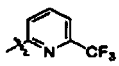, 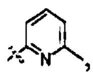, 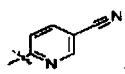, 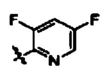,

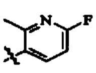, 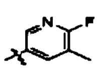, 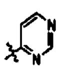, 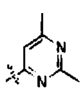, 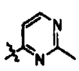, 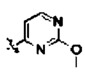, 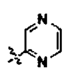, 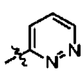, 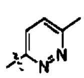, 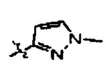,

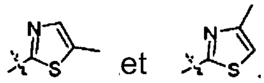
et .

**5.** Composé ou un sel pharmaceutiquement acceptable de celui-ci selon la revendication 1 ou 2, dans lequel : $R^3$ est choisi dans le groupe consistant en :

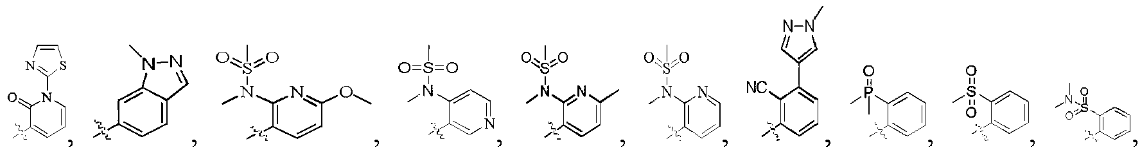
, , , , , , , , , ,

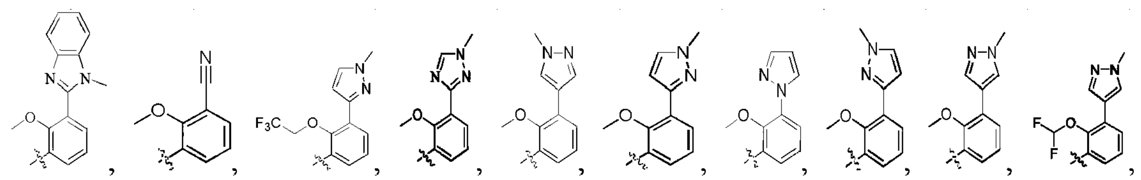
, , , , , , , , , ,

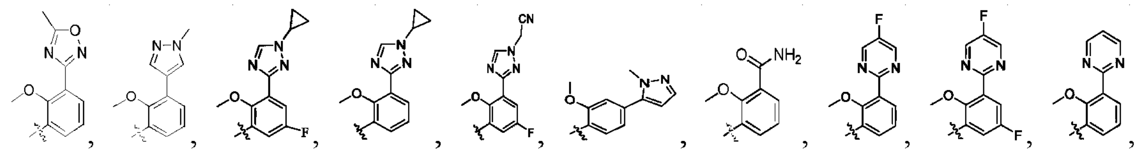
, , , , , , , , , ,

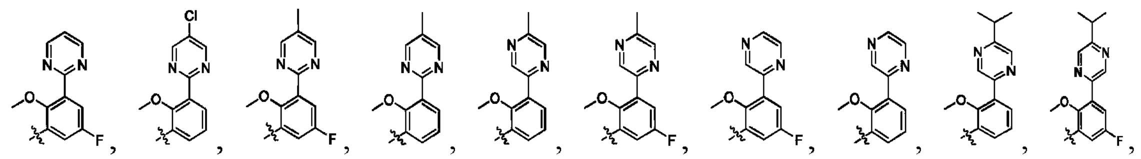
, , , , , , , , , ,

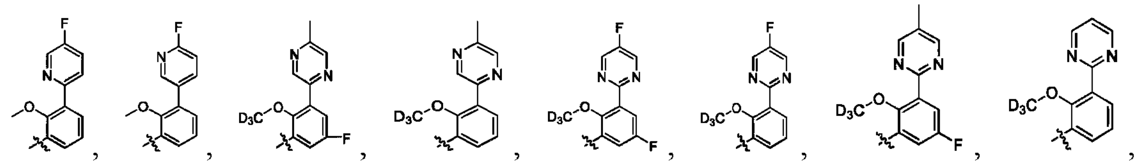
, , , , , , , ,

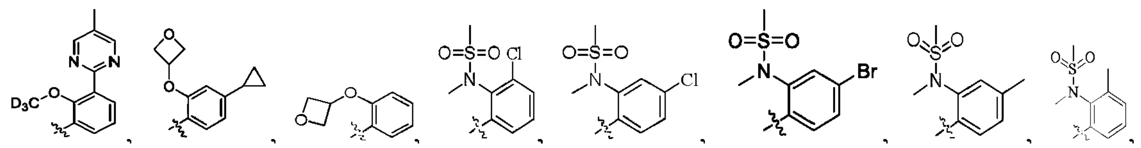
, , , , , , , , ,

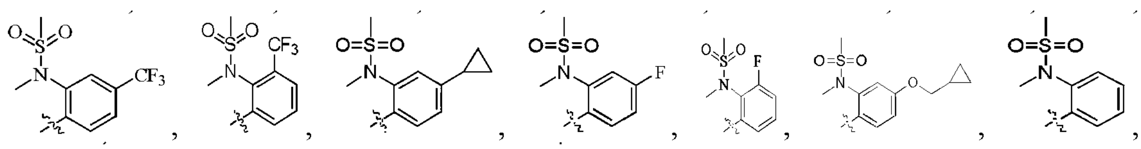
, , , , , , , ,

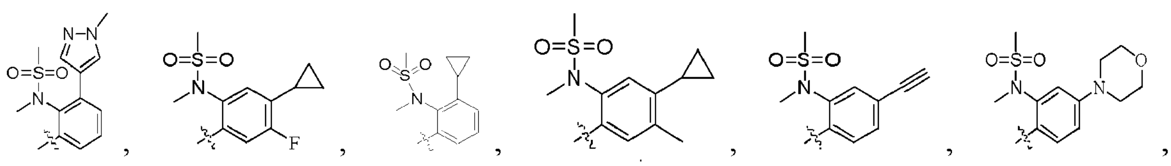
, , , , , ,

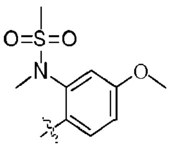, 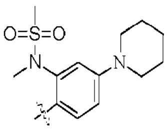, 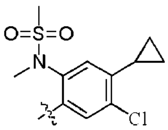, 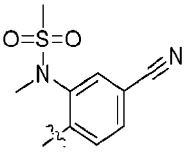, 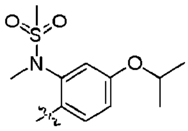, 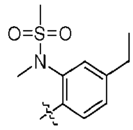,

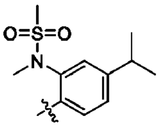, 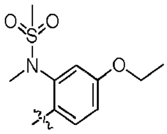, 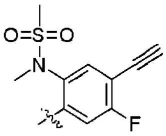, 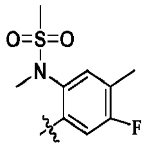, 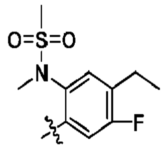, 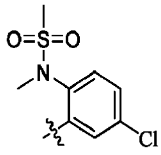,

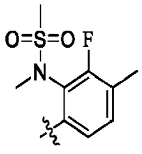, 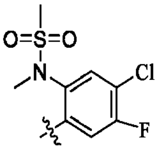, 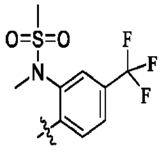, 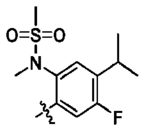, 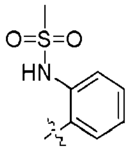, 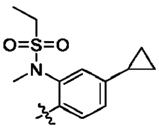,

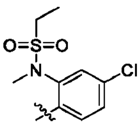, 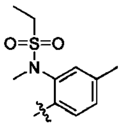, 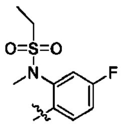, 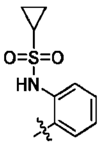, 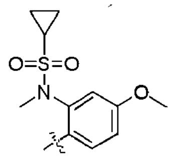, 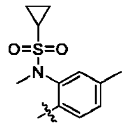, 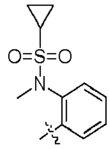,

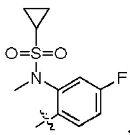, 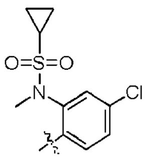, 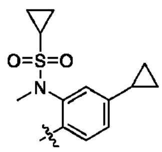 et 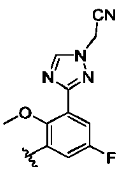.

**6.** Composé ou un sel pharmaceutiquement acceptable de celui-ci selon la revendication 1, dans lequel :

X est CH ;
Y est CH;
$R^0$ est hydrogène ;
$R^1$ est éthyle ;
$R^2$ est choisi dans le groupe consistant en pyridinyle, pyrimidinyle, -C(O)-cycloalkyle en $C_{3-6}$ et -C(O)-hétérocyclyle en $C_{3-6}$, dans lequel l'hétérocyclyle en $C_{3-6}$ contient de un à deux hétéroatomes choisis parmi N, O et S ; dans lequel le cycloalkyle en $C_{3-6}$ et l'hétérocyclyle en $C_{3-6}$ sont respectivement et éventuellement substitués par de un à trois groupes R2a ;
$R^{2a}$ est choisi dans le groupe consistant en alkyle en $C_{1-6}$, halogène, alcoxy en $C_{1-6}$, cycloalkyle en $C_{3-6}$, cyano, halo alkyle en $C_{1-6}$, alkyle en $C_{1-6}$ substitué par un groupe hydroxyle et alkyle en $C_{1-6}$-$S(O)_2$- ;
$R^3$ est phényle éventuellement substitué par de un à trois groupes $R^{3a}$ ;
$R^{3a}$ est choisi dans le groupe consistant en -$(CH_2)_r$-$OR^b$, -$NR^bS(O)_pR^c$, F, Cl, alkyle en $C_{1-6}$, cycloalkyle en $C_{3-6}$, triazolyle, pyrazolyle, pyrimidinyle et pyrazinyle ; dans lequel les alkyle en $C_{1-6}$, cycloalkyle en $C_{3-6}$, triazolyle, pyrazolyle, pyrimidinyle et pyrazinyle sont, respectivement et indépendamment, éventuellement substitués par de un à trois groupes $R^d$ ;
$R^b$ est choisi dans le groupe consistant en hydrogène, alkyle en $C_{1-6}$ éventuellement substitué par de un à trois groupes $R^d$, et hétérocyclyle saturé à 4-6 chaînons contenant de un à deux atomes d'oxygène ;
$R^c$ est choisi dans le groupe consistant en hydrogène, alkyle en $C_{1-6}$ et cycloalkyle en C3-6 ;
$R^d$ est choisi dans le groupe consistant en hydrogène, deutérium, F, Cl, Br, I, $OCF_3$, $CF_3$, -$(CH_2)_r$-CN, $NO_2$, $OR^e$, -$(CH_2)_r$-$COR^c$, -$NR^eR^e$, -$NR^eC(O)OR^c$, alkyle en $C_1$-$C_6$, cycloalkyle en $C_{3-6}$ et -$(CH_2)_r$-phényle ;
$R^e$ est hydrogène ;
p est 0, 1 ou 2, et
r est 0, 1, 2, 3 ou 4.

**7.** Composé ou un sel pharmaceutiquement acceptable de celui-ci selon la revendication 6, dans lequel $R^{2a}$ est choisi dans le groupe consistant en méthyle, F, méthoxy, cyclopropyle, cyano, $-CF_3$, hydroxyméthyle et méthanesulfonyle.

**8.** Composé ou un sel pharmaceutiquement acceptable de celui-ci selon la revendication 6, dans lequel $R^2$ est choisi parmi les structures suivantes :

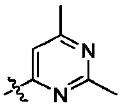, 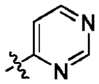, 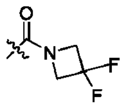, 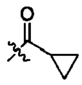, 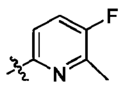, 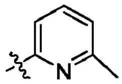, 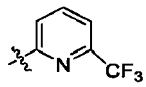, 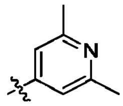,

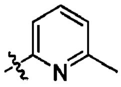, 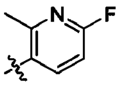, 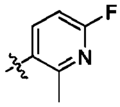, 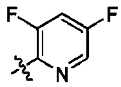, 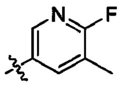, 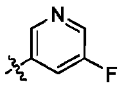, 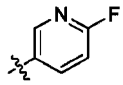, 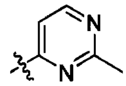,

et

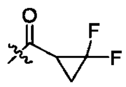.

**9.** Composé ou un sel pharmaceutiquement acceptable de celui-ci selon la revendication 6, dans lequel $R^b$ est choisi dans le groupe consistant en hydrogène ; méthyle, éthyle et isopropyle, chacun étant éventuellement substitué par de un à trois groupes $R^d$ ; et

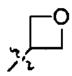.

**10.** Composé ou un sel pharmaceutiquement acceptable de celui-ci selon la revendication 6, dans lequel $R^c$ est choisi dans le groupe consistant en hydrogène, méthyle, éthyle et cyclopropyle.

**11.** Composé ou un sel pharmaceutiquement acceptable de celui-ci selon la revendication 6, dans lequel $R^d$ est choisi dans le groupe consistant en F, Cl, hydroxyle, méthyle, cyclopropyle et $-CH_2CN$.

**12.** Composé ou un sel pharmaceutiquement acceptable de celui-ci selon la revendication 6, dans lequel $R^3$ est choisi parmi les structures suivantes :

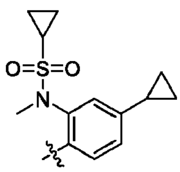, 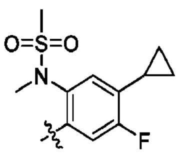, 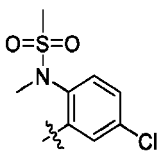, 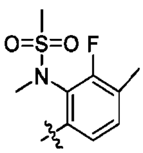, 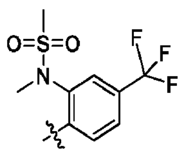, 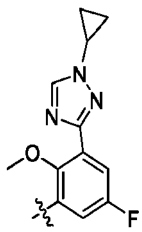,

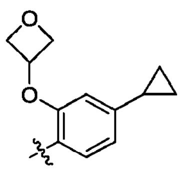, 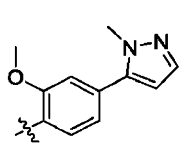, 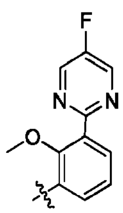, 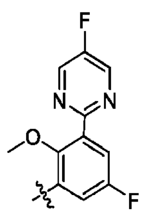, 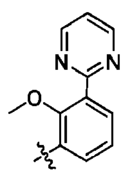, 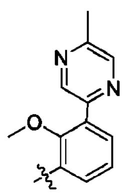, 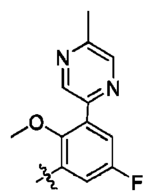,

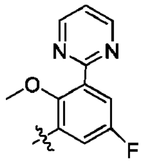, 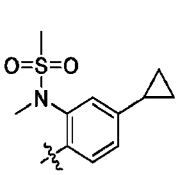, 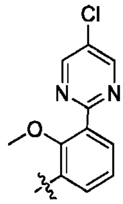, 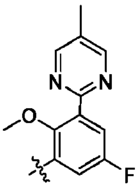, 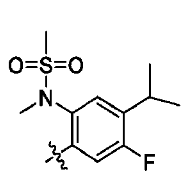, 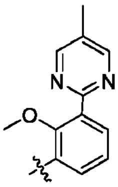, 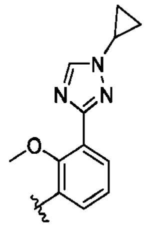,

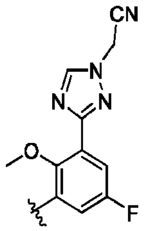, 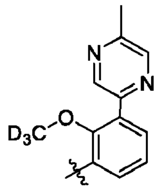, 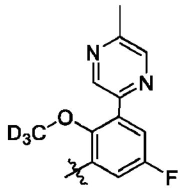, 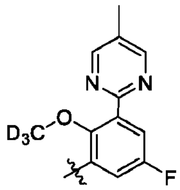, 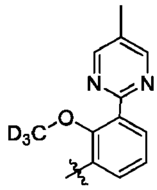, 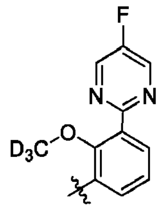,

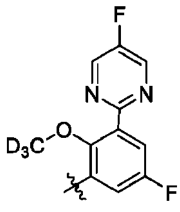, 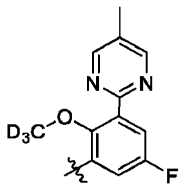, et 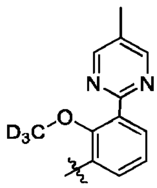.

13. Composé ou un sel pharmaceutiquement acceptable de celui-ci selon la revendication 1, dans lequel le composé est choisi dans le groupe consistant en :

| N° composé | Structure | N° composé | Structure |
|---|---|---|---|
| 2 | 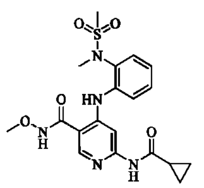 | 157 | 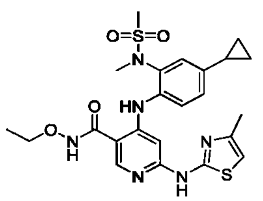 |
| 4 | 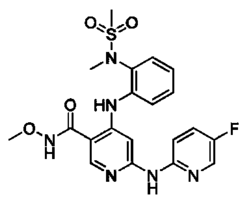 | 159 | 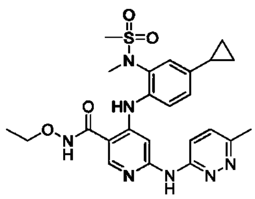 |
| 10 | 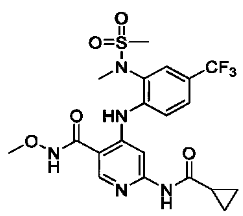 | 160 | 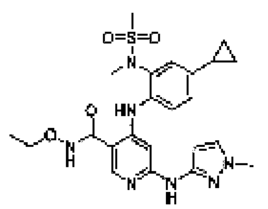 |
| 12 | 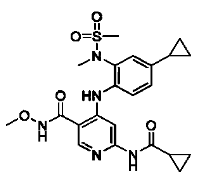 | 162 | 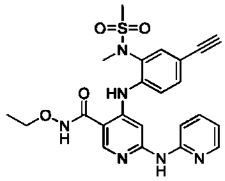 |

(continued)

| N° composé | Structure | N° composé | Structure |
|---|---|---|---|
| 13 | 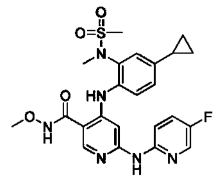 | 166 | 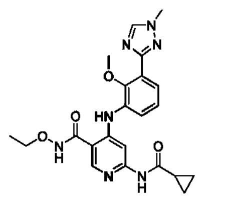 |
| 14 | 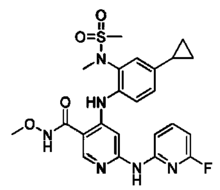 | 167 | 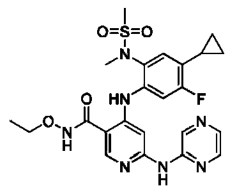 |
| 16 | 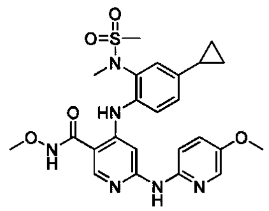 | 170 | 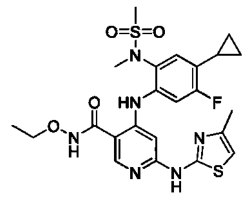 |
| 18 | 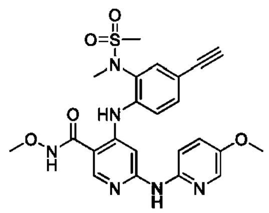 | 171 | 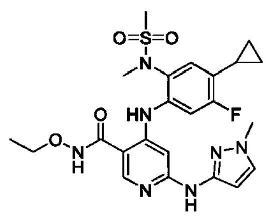 |
| 19 | 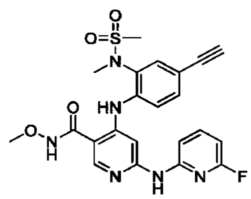 | 173 | 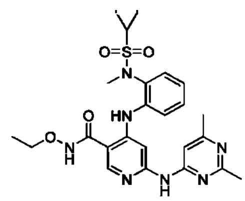 |
| 20 | 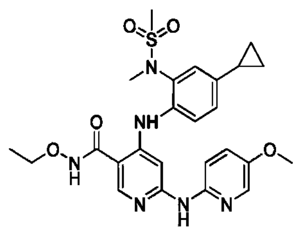 | 174 | 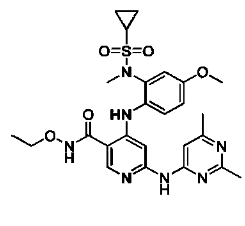 |
| 21 | 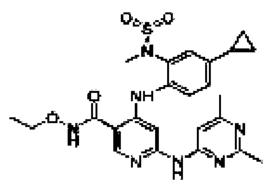 | 175 | 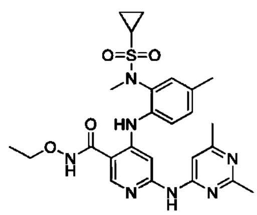 |

(continued)

| N° composé | Structure | N° composé | Structure |
|---|---|---|---|
| 22 | 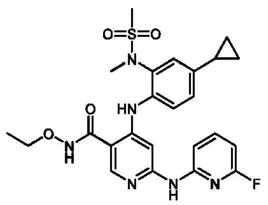 | 176 | 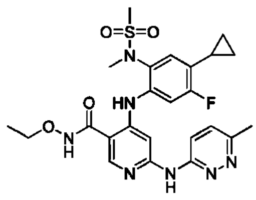 |
| 29 | 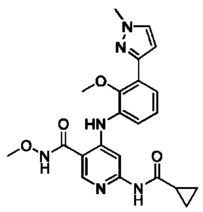 | 177 | 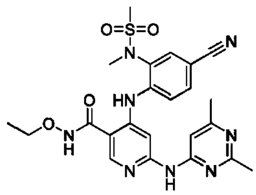 |
| 36 | 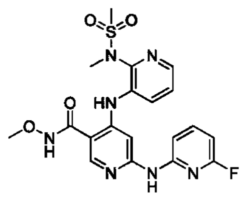 | 178 | 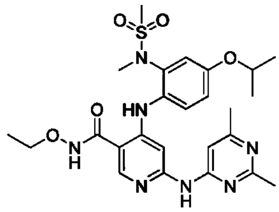 |
| 38 | 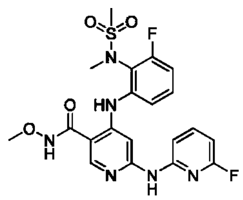 | 179 | 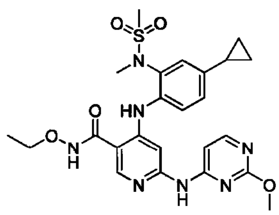 |
| 41 | 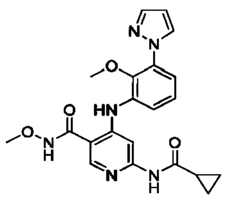 | 180 | 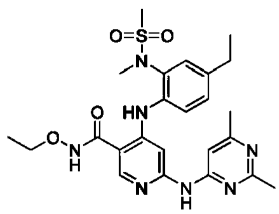 |
| 42 | 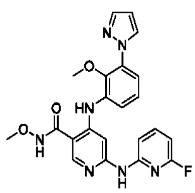 | 181 | 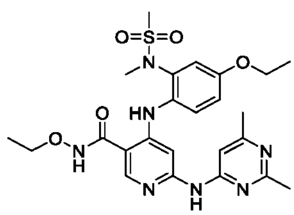 |
| 43 | 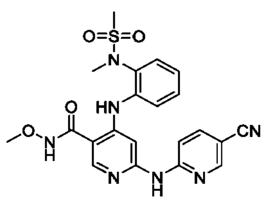 | 182 | 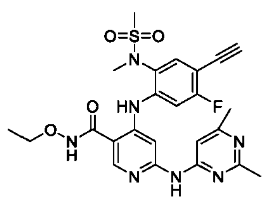 |

(continued)

| N° composé | Structure | N° composé | Structure |
|---|---|---|---|
| 44 | 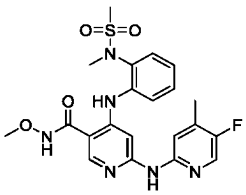 | 183 | 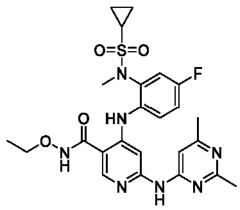 |
| 45 | 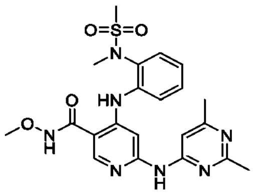 | 185 | 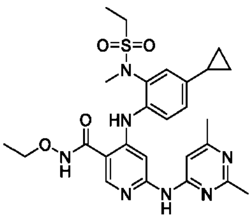 |
| 46 | 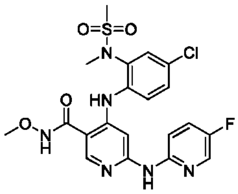 | 186 | 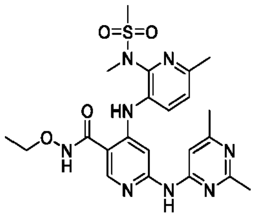 |
| 47 | 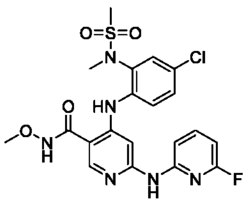 | 187 | 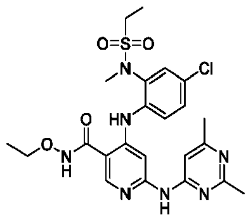 |
| 60 | 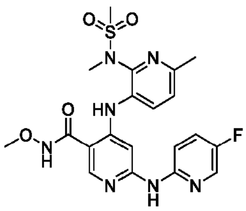 | 188 | 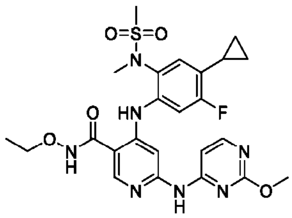 |
| 61 | 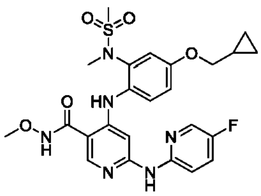 | 189 | 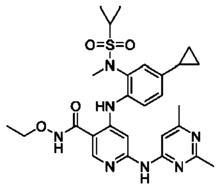 |
| 62 | 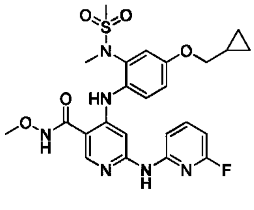 | 190 | 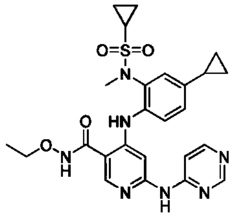 |

(continued)

| N° composé | Structure | N° composé | Structure |
|---|---|---|---|
| 63 | 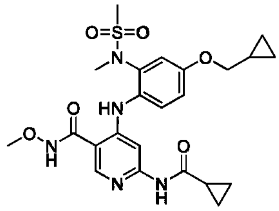 | 197 | 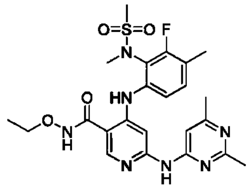 |
| 68 | 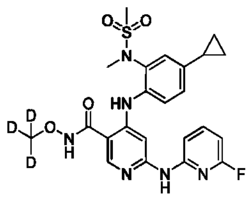 | 198 | 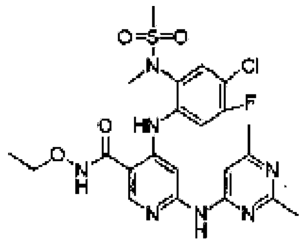 |
| 69 | 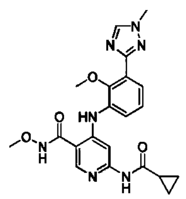 | 200 | 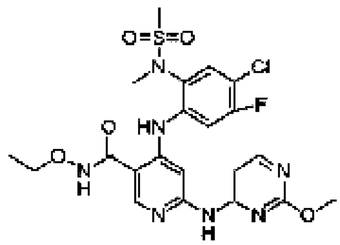 |
| 70 | 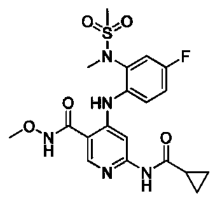 | 202 | 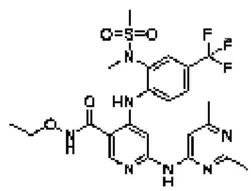 |
| 79 | 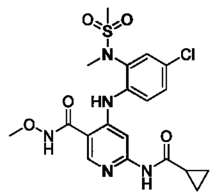 | 204 | 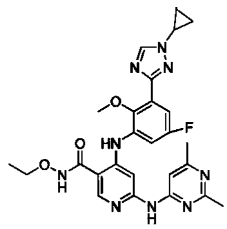 |
| 80 | 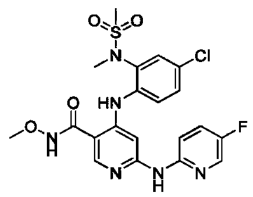 | 194 | 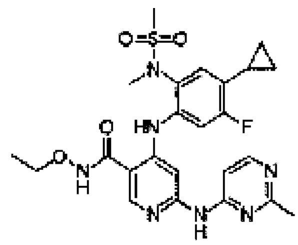 |
| 81 | 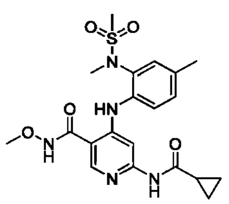 | 192 | 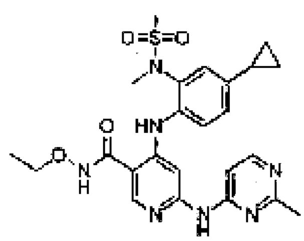 |

(continued)

| N° composé | Structure | N° composé | Structure |
|---|---|---|---|
| 90 | 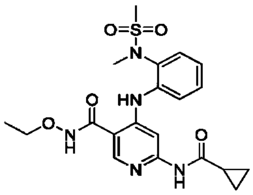 | 193 | 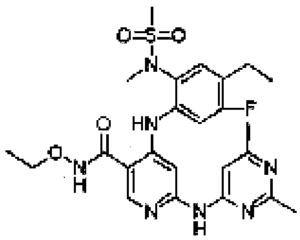 |
| 95 | 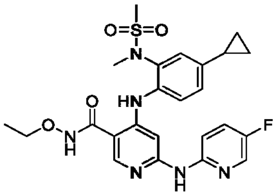 | 203 | 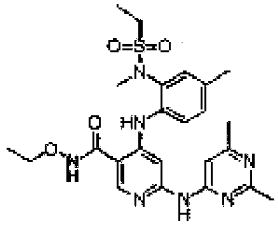 |
| 97 | 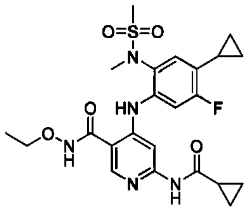 | 230 | 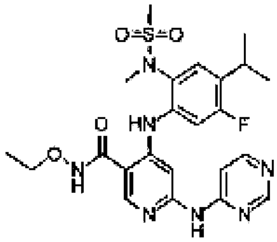 |
| 98 | 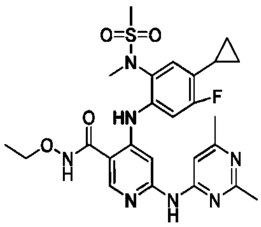 | 229 | 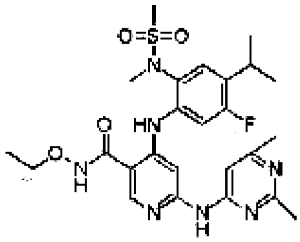 |
| 101 | 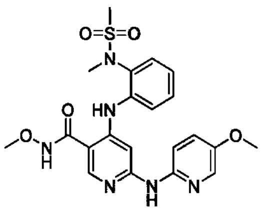 | 201 | 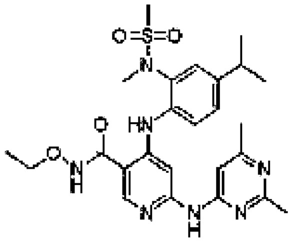 |
| 102 | 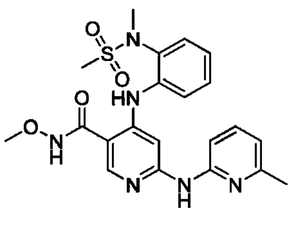 | 191 | 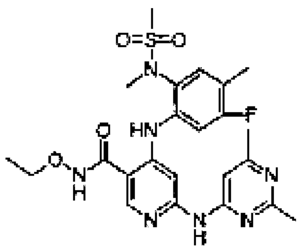 |

(continued)

| N° composé | Structure | N° composé | Structure |
|---|---|---|---|
| 106 | 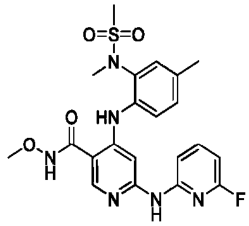 | 205 | 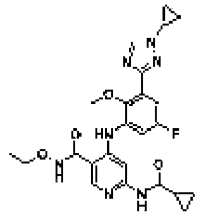 |
| 108 | 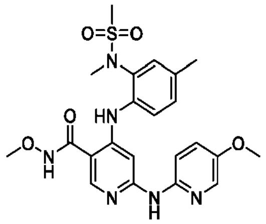 | 207 | 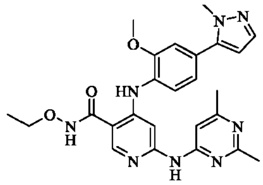 |
| 109 | 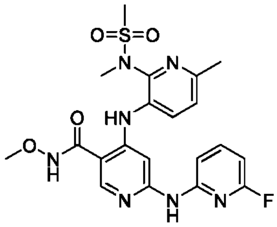 | 208 | 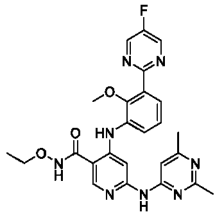 |
| 110 | 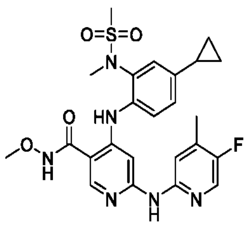 | 209 | 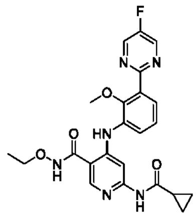 |
| 111 | 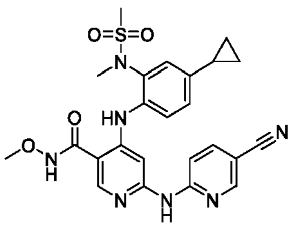 | 210 | 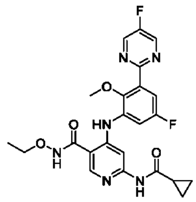 |
| 112 | 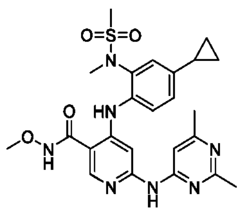 | 211 | 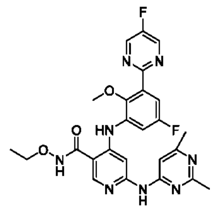 |
| 113 | 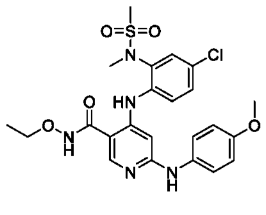 | 212 | 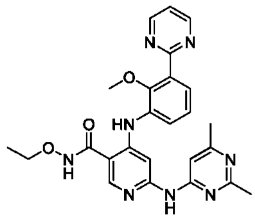 |

(continued)

| N° composé | Structure | N° composé | Structure |
|---|---|---|---|
| 114 | 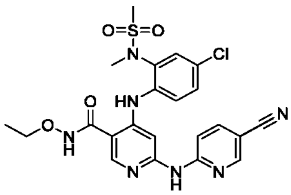 | 213 | 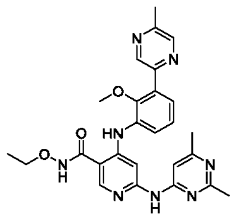 |
| 115 | 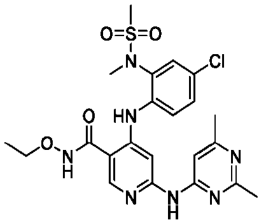 | 214 | 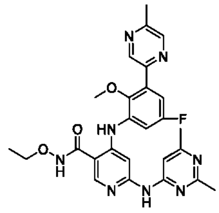 |
| 116 | 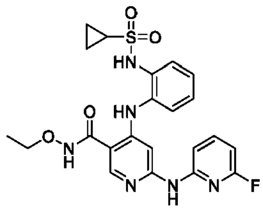 | 215 | 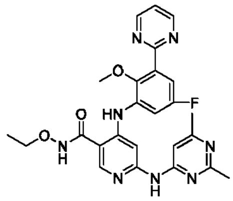 |
| 118 | 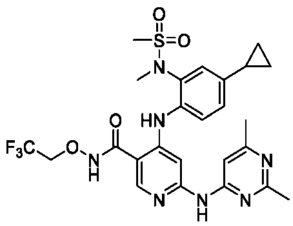 | 217 | 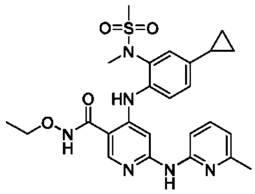 |
| 119 | 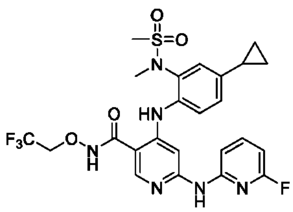 | 218 | 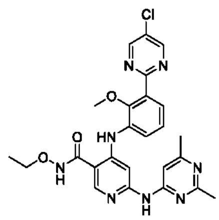 |
| 125 | 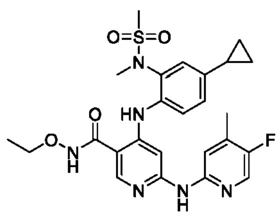 | 219 | 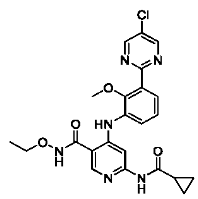 |
| 126 | 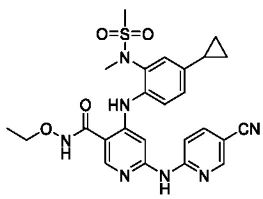 | 220 | 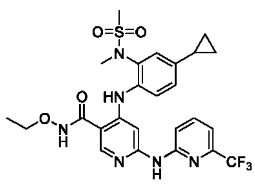 |

(continued)

| N° composé | Structure | N° composé | Structure |
|---|---|---|---|
| 127 | 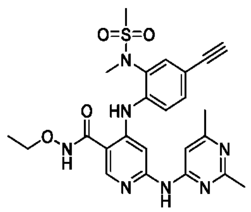 | 222 | 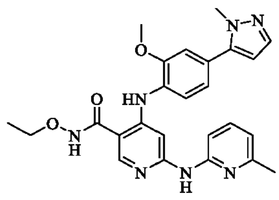 |
| 128 | 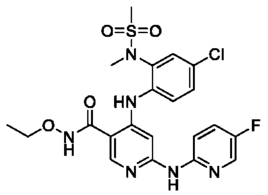 | 223 | 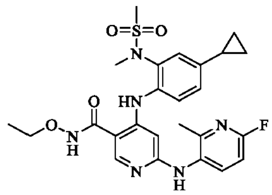 |
| 129 | 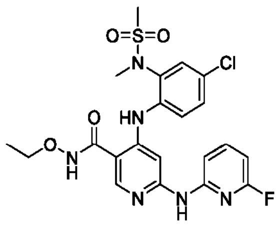 | 224 | 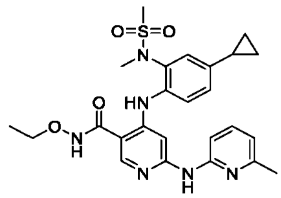 |
| 130 | 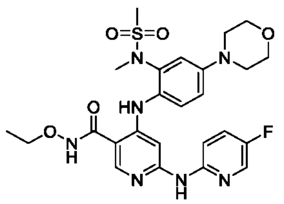 | 225 | 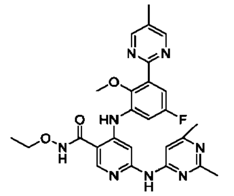 |
| 131 | 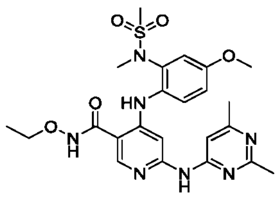 | 226 | 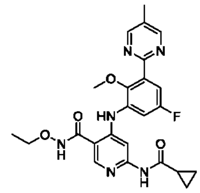 |
| 132 | 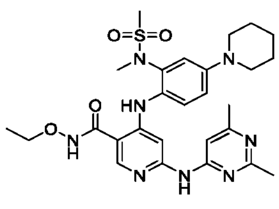 | 234 | 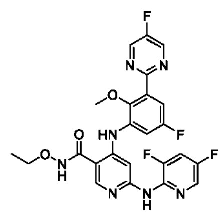 |
| 133 | 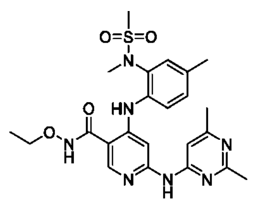 | 235 | 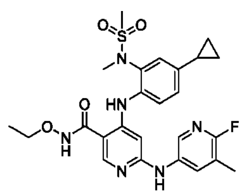 |

(continued)

| N° composé | Structure | N° composé | Structure |
|---|---|---|---|
| 134 | 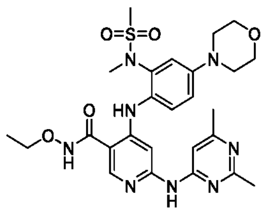 | 236 | 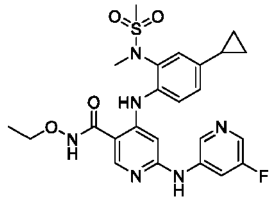 |
| 136 | 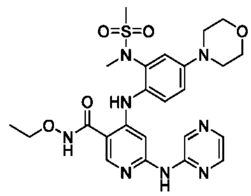 | 237 | 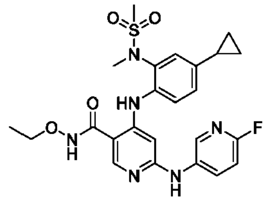 |
| 138 | 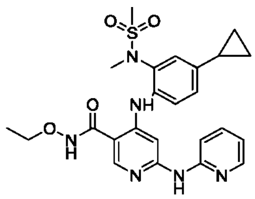 | 238 | 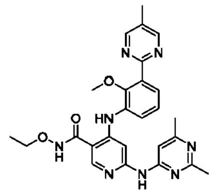 |
| 139 | 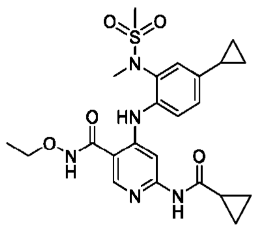 | 239 | 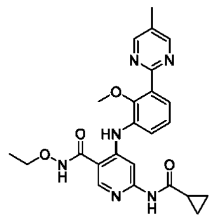 |
| 141 | 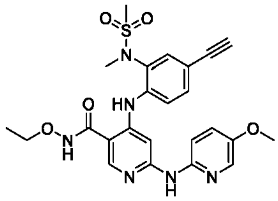 | 240 | 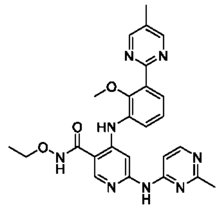 |
| 142 | 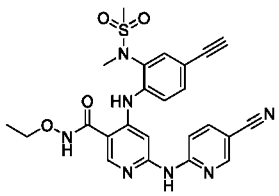 | 242 | 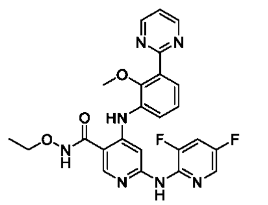 |
| 143 | 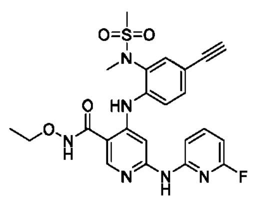 | 243 | 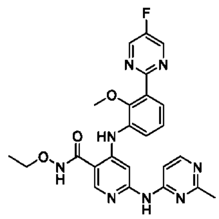 |

(continued)

| N° composé | Structure | N° composé | Structure |
|---|---|---|---|
| 144 | 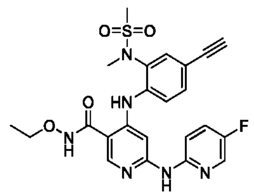 | 244 | 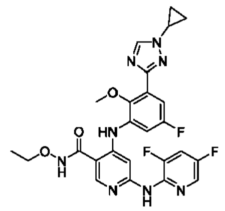 |
| 145 | 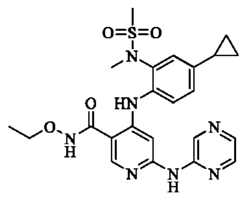 | 245 | 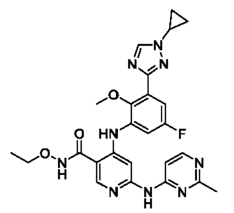 |
| 146 | 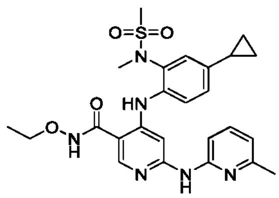 | 248 | 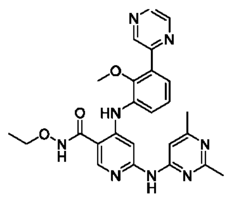 |
| 151 | 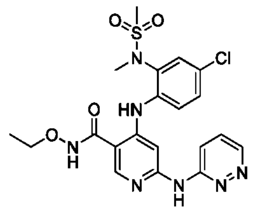 | 258 | 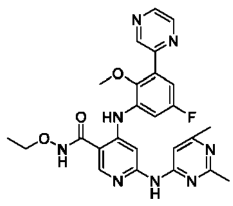 |
| 153 | 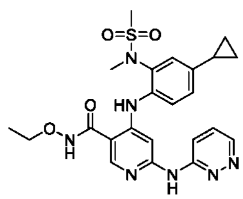 | 268 | 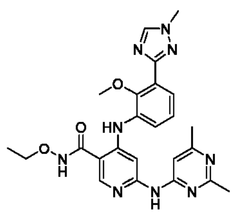 |
| 154 | 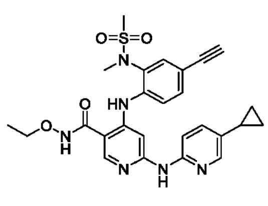 | 273 | 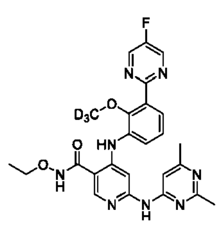 |
| 155 | 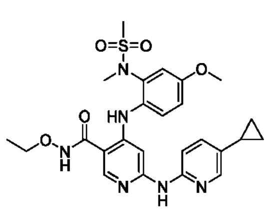 | 277 | 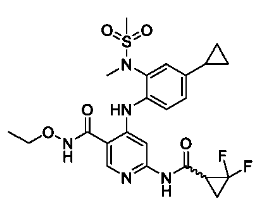 |

(continued)

| N° composé | Structure | N° composé | Structure |
|---|---|---|---|
| 156 | 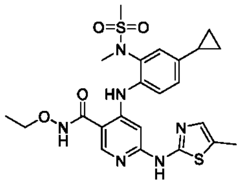 | 279 | 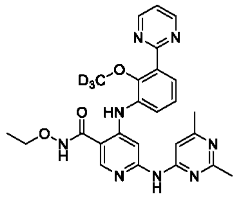 |

**14.** Composition pharmaceutique, comprenant le composé ou un sel pharmaceutiquement acceptable de celui-ci selon l'une quelconque des revendications 1 à 13, et comprenant éventuellement un support et/ou un adjuvant et/ou un diluant pharmaceutiquement acceptable.

**15.** Composé ou un sel pharmaceutiquement acceptable de celui-ci selon l'une quelconque des revendications 1 à 13 ou composition pharmaceutique selon la revendication 14, destiné(e) à être utilisé(e) dans le traitement et/ou la prévention d'une maladie associée médiée par TYK2 ;
de préférence, la maladie associée médiée par TYK2 inclut une maladie inflammatoire, une maladie auto-immune et un cancer.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020086616 A


### Non-patent literature cited in the description


- REMINGTON'S PHARMACEUTICAL SCIENCES. Mack Publishing Company, 1995
- *Tetrahedron Letters*, 2017, vol. 58, 1681